(19) Europäisches Patentamt
European Patent Office
Office européen des brevets

(11) EP 2 978 756 B1

(12) **EUROPÄISCHE PATENTSCHRIFT**

(45) Veröffentlichungstag und Bekanntmachung des Hinweises auf die Patenterteilung:
**27.03.2019 Patentblatt 2019/13**

(21) Anmeldenummer: **14713452.2**

(22) Anmeldetag: **27.03.2014**

(51) Int Cl.:
*C07D 413/14* (2006.01) *C07D 213/85* (2006.01)
*C07D 401/12* (2006.01) *C07D 405/06* (2006.01)
*C07D 409/06* (2006.01) *C07D 413/12* (2006.01)
*C07D 417/12* (2006.01) *C07D 213/64* (2006.01)
*C07D 213/69* (2006.01) *A61K 31/4427* (2006.01)
*A61K 31/4412* (2006.01) *A61K 31/4418* (2006.01)
*A61P 7/00* (2006.01) *A61P 9/00* (2006.01)

(86) Internationale Anmeldenummer:
**PCT/EP2014/056135**

(87) Internationale Veröffentlichungsnummer:
**WO 2014/154794 (02.10.2014 Gazette 2014/40)**

(54) **SUBSTITUIERTE OXOPYRIDIN-DERIVATE UND IHRE VERWENDUNG BEI DER BEHANDLUNG VON HERZ-KREISLAUF-ERKRANKUNGEN**

SUBSTITUTED OXOPYRIDINE DERIVATIVES AND THEIR USE IN THE TREATMENT OF CARDIOVASCULAR DISEASES

DÉRIVÉS D'OXOPYRIDINE ET LEUR UTILISATION DANS LE TRAITEMENT DE MALADIES CARDIOVASCULAIRES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **28.03.2013 EP 13161588**
**30.10.2013 EP 13190944**

(43) Veröffentlichungstag der Anmeldung:
**03.02.2016 Patentblatt 2016/05**

(73) Patentinhaber: **Bayer Pharma Aktiengesellschaft**
**13353 Berlin (DE)**

(72) Erfinder:
- **RÖHRIG, Susanne**
**40724 Hilden (DE)**
- **HILLISCH, Alexander**
**42651 Solingen (DE)**
- **STRAßBURGER, Julia**
**42115 Wuppertal (DE)**
- **HEITMEIER, Stefan**
**42489 Wülfrath (DE)**
- **SCHMIDT, Martina Victoria**
**51063 Köln (DE)**
- **SCHLEMMER, Karl-Heinz**
**42113 Wuppertal (DE)**
- **TERSTEEGEN, Adrian**
**42111 Wuppertal (DE)**
- **BUCHMÜLLER, Anja**
**45259 Essen (DE)**
- **GERDES, Christoph**
**51063 Köln (DE)**
- **SCHÄFER, Martina**
**13465 Berlin (DE)**
- **KINZEL, Tom**
**40489 Düsseldorf (DE)**
- **TELLER, Henrik**
**18258 Schwaan (DE)**
- **SCHIROK, Hartmut**
**40764 Langenfeld (DE)**
- **KLAR, Jürgen**
**42109 Wuppertal (DE)**
- **JIMENEZ NUNEZ, Eloisa**
**42117 Wuppertal (DE)**

(74) Vertreter: **BIP Patents**
**c/o Bayer Intellectual Property GmbH**
**Alfred-Nobel-Straße 10**
**40789 Monheim am Rhein (DE)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

(Forts. nächste Seite)

(56) Entgegenhaltungen:
**WO-A1-2006/030032** **WO-A1-2007/131179**
**WO-A2-2008/079787** **WO-A2-2014/160592**

**Beschreibung**

Die Erfindung betrifft substituierte Oxopyridin-Derivate und Verfahren zu ihrer Herstellung sowie ihre Verwendung zur Herstellung von Arzneimitteln zur Behandlung und/oder Prophylaxe von Krankheiten, insbesondere von Herz-Kreislauf-Erkrankungen, vorzugsweise von thrombotischen beziehungsweise thromboembolischen Erkrankungen sowie von Ödemen, als auch von ophthalmologischen Erkrankungen.

Die Blutgerinnung ist ein Schutzmechanismus des Organismus, mit dessen Hilfe Defekte in der Gefäßwand rasch und zuverlässig "abgedichtet" werden können. So kann ein Blutverlust vermieden beziehungsweise minimiert werden. Die Blutstillung nach Gefäßverletzung erfolgt im Wesentlichen durch das Gerinnungssystem, bei dem eine enzymatische Kaskade komplexer Reaktionen von Plasmaproteinen ausgelöst wird. Hierbei sind zahlreiche Blutgerinnungsfaktoren beteiligt, von denen jeder, sobald aktiviert, die jeweils nächste inaktive Vorstufe in ihre aktive Form überführt. Am Ende der Kaskade steht die Umwandlung des löslichen Fibrinogens in das unlösliche Fibrin, so dass es zu einem Blutgerinnsel kommt. Traditionell unterscheidet man bei der Blutgerinnung zwischen dem intrinsischen und dem extrinsischen System, die in einem abschließenden gemeinsamen Reaktionsweg münden. Hierbei kommen den Faktoren Xa und IIa (Thrombin) Schlüsselrollen zu: Faktor Xa bündelt die Signale der beiden Gerinnungswege, da er sowohl über Faktor VIIa/Tissue Factor (extrinsischer Weg) wie auch den Tenase Komplex (intrisischer Weg) durch Umsetzung von Faktor X entsteht. Die aktivierte Serinprotease Xa spaltet Prothrombin zu Thrombin, das über eine Reihe von Umsetzungen die Impulse aus der Kaskade auf den Gerinnungsstatus des Blutes überträgt.

In der jüngeren Vergangenheit ist die traditionelle Theorie der zwei getrennten Bereiche der Koagulationkaskade (extrinsischer beziehungsweise intrinsischer Pfad) aufgrund neuer Erkenntnisse modifiziert worden: In diesen Modellen wird die Koagulation durch Bindung von aktiviertem Faktor VIIa an Tissue Faktor (TF) initiiert. Der entstandene Komplex aktiviert Faktor X, was wiederum zur Thrombin-Generierung mit anschließender Herstellung von Fibrin und Thrombozyten-Aktivierung (via PAR-1) als verletzungsverschließende Endprodukte der Hämostase führt. Im Vergleich zur anschließenden Amplifikations-/Propagationsphase ist die Geschwindigkeit der Thrombin-Herstellung klein und durch das Auftreten von TFPI als Hemmer des TF-FVIIa-FX-Komplexes zeitlich begrenzt.

Ein zentraler Bestandteil des Übergangs von der Initiation zur Amplifikation und Propagation der Koagulation ist Faktor XIa. Thrombin aktiviert in positiven Rückkopplungsschleifen neben Faktor V und Faktor VIII auch Faktor XI zu Faktor XIa, was Faktor IX zu Faktor IXa umsetzt und über den so generierten Faktor IXa/ Faktor VIIIa-Komplex schnell größere Mengen Faktor Xa produziert. Dies löst die Produktion großer Mengen Thrombin aus, die zu starkem Thrombuswachstum führt und den Thrombus stabilisiert.

Daneben kann die Aktivierung des Gerinnungssystems auch an insbesondere negativ geladenen Oberflächen erfolgen, zu denen auch artifizielle Oberflächen wie Gefäßprothesen, Stents und extrakorporale Kreisläufe gehören. Auf der Oberfläche findet zunächst die Aktivierung von Faktor XII zu Faktor XIIa statt, der im Folgenden über Kininogen oder Glycoprotein Ib an Zelloberflächen gebundenen Faktor XI aktiviert. Dieser führt zur weiteren Aktivierung der Gerinnungskaskade.

Daneben aktiviert Faktor XIIa ebenfalls gebundenes Plasmaprokallikrein zu Plasma-Kallikrein. Plasma-Kallikrein führt im Rahmen einer Potentierungsschleife wiederum zu weiterer Faktor XII-Aktivierung, was insgesamt eine Verstärkung der Initiation der Gerinnungskaskade zur Folge hat. Zusätzlich stellt Plasma-Kallikrein eine wichtige Bradikinin-freisetzende Protease dar, die somit unter anderem zum Anstieg der endothelialen Permeabilität führt. Als weitere Substrate wurden Prorenin und Prourokinase beschrieben, deren Aktivierung die regulatorischen Prozesse des Renin-Angiotensin-Systems und der Fibrinolyse auslösen kann.

Eine unkontrollierte Aktivierung des Gerinnungssystems oder defekte Hemmung der Aktivierungsprozesse kann die Bildung von lokalen Thrombosen oder Embolien in Gefäßen (Arterien, Venen, Lymphgefäßen) oder Herzhöhlen bewirken. Dies kann zu schwerwiegenden thrombotischen oder thromboembolischen Erkrankungen führen. Darüber hinaus kann eine systemische Hyperkoagulabilität zu einer Verbrauchskoagulopathie im Rahmen einer disseminierten intravasalen Gerinnung führen.

Im Verlauf vieler Herzkreislauf- und Stoffwechselerkrankungen kommt es infolge systemischer Faktoren, wie z.B. Hyperlipidämie, Diabetes oder Rauchen, infolge von Blutflußveränderungen mit Stase, wie z.B. beim Vorhofflimmern, oder infolge pathologischer Gefäßwandveränderungen, z.B. endothelialer Dysfunktionen oder Atherosklerose, zu einer erhöhten Neigung von Gerinnungs- und Thrombozytenaktivierung. Diese unerwünschte und überschießende Hämostase kann durch Bildung fibrin- und plättchenreicher Thromben zu thromboembolischen Erkrankungen und thrombotischen Komplikationen mit lebensbedrohlichen Zuständen führen. Hierbei können auch entzündliche Prozesse involviert sein.

Thromboembolische Erkrankungen sind die häufigste Ursache von Morbidität und Mortalität in den meisten industrialisierten Ländern [Heart Disease: A Textbook of Cardiovascular Medicine, Eugene Braunwald, 5. Auflage, 1997, W.B. Saunders Company, Philadelphia].

Die aus dem Stand der Technik bekannten Antikoagulantien, d.h. Stoffe zur Hemmung oder Verhinderung der Blutgerinnung, weisen verschiedene, oftmals gravierende Nachteile auf. Eine effiziente Behandlungsmethode beziehungsweise Prophylaxe von thrombotischen/thromboembolischen Erkrankungen erweist sich in der Praxis deshalb als

sehr schwierig und unbefriedigend.

In der Therapie und Prophylaxe von thromboembolischen Erkrankungen findet zum einen Heparin Verwendung, das parenteral oder subkutan appliziert wird. Aufgrund günstigerer pharmakokinetischer Eigenschaften wird zwar heutzutage zunehmend niedermolekulares Heparin bevorzugt; allerdings können auch hierdurch die im Folgenden geschilderten bekannten Nachteile nicht vermieden werden, die bei der Therapierung mit Heparin bestehen. So ist Heparin oral unwirksam und besitzt nur eine vergleichsweise geringe Halbwertszeit. Darüber hinaus besteht ein hohes Blutungsrisiko, insbesondere können Hirnblutungen und Blutungen im Gastrointestinaltrakt auftreten, und es kann zu Thrombopenie, Alopecia medicomentosa oder Osteoporose kommen [Pschyrembel, Klinisches Wörterbuch, 257. Auflage, 1994, Walter de Gruyter Verlag, Seite 610, Stichwort "Heparin"; Römpp Lexikon Chemie, Version 1.5, 1998, Georg Thieme Verlag Stuttgart, Stichwort "Heparin"]. Niedermolekulare Heparine besitzen zwar eine geringere Wahrscheinlichkeit zur Ausbildung einer Heparin-induzierten Thrombocytopenie, sind aber auch nur subkutan applizierbar. Dies gilt auch für Fondaparinux, einem synthetisch hergestellten, selektiven Faktor Xa Inhibitor mit einer langen Halbwertszeit.

Eine zweite Klasse von Antikoagulantien stellen die Vitamin K-Antagonisten dar. Hierzu gehören beispielsweise 1,3-Indandione, vor allem aber Verbindungen wie Warfarin, Phenprocoumon, Dicumarol und andere Cumarin-Derivate, die unselektiv die Synthese verschiedener Produkte bestimmter Vitamin K-abhängiger Gerinnungsfaktoren in der Leber hemmen. Durch den Wirkmechanismus bedingt, setzt die Wirkung nur sehr langsam ein (Latenzzeit bis zum Wirkeintritt 36 bis 48 Stunden). Die Verbindungen können zwar oral appliziert werden, aufgrund des hohen Blutungsrisikos und des engen therapeutischen Indexes ist aber eine aufwendige individuelle Einstellung und Beobachtung des Patienten notwendig [J. Hirsh, J. Dalen, D.R. Anderson et al., "Oral anticoagulants: Mechanism of action, clinical effectiveness, and optimal therapeutic range" Chest 2001, 119, 8S-21S; J. Ansell, J. Hirsh, J. Dalen et al., "Managing oral anticoagulant therapy" Chest 2001, 119, 22S-38S; P.S. Wells, A.M. Holbrook, N.R. Crowther et al., "Interactions of warfarin with drugs and food" Ann. Intern. Med. 1994, 121, 676-683]. Darüber hinaus sind weitere Nebenwirkungen wie gastrointestinale Störungen, Haarausfall und Hautnekrosen beschrieben.

Neuere Ansätze für orale Antikoagulantien befinden sich in verschiedenen Phasen der klinischen Erprobung beziehungsweise im klinischen Einsatz, haben jedoch auch Nachteile gezeigt, wie z.B. hochvariable Bioverfügbarkeit, Leberschädigung und Blutungskomplikationen.

Bei antithrombotischen Arzneimitteln ist die therapeutische Breite von zentraler Bedeutung: Der Abstand zwischen der therapeutisch wirksamen Dosis zur Gerinnungshemmung und der Dosis, bei der Blutungen auftreten können, sollte möglichst groß sein, so dass eine maximale therapeutische Wirksamkeit bei minimalem Risikoprofil erreicht wird.

In verschiedenen in-vivo Modellen mit beispielsweise Antikörpern als Faktor XIa Inhibitoren, aber auch in Faktor XIa-Knock-out-Modellen, wurde der anti-thrombotische Effekt bei geringer/keiner Verlängerung der Blutungszeit oder Vergrößerung des Blutvolumens belegt. In klinischen Studien waren erhöhte Faktor XIa-Spiegel mit einer gesteigerten Ereignisrate assoziiert. Dagegen führte Faktor XI-Defizienz (Hämophilie C) im Gegensatz zu Faktor VIIIa- oder Faktor IXa- (Hämophilie A beziehungsweise B) nicht zu spontanen Blutungen und fiel nur im Rahmen von Operationen und Traumen auf. Stattdessen zeigte sich eine Protektion gegenüber bestimmten thromboembolischen Ereignissen.

Weiterhin kann auch die Kombination von antithrombotischen und antiinflammtorischen Prinzipien für viele Erkrankungen besonders attraktiv sein, um die wechselseitige Verstärkung von Koagulation und Inflammation zu unterbinden.

Plasma-Kallikrein ist assoziert mit Erkrankungen, die mit einer erhöhten Gefäßpermeabilität einhergehen, wie es z.B. bei der diabetischen Retinopathie und dem Makulaödem der Fall ist.

Die diabetische Retinopathie, eine gut charakterisierte chronische Augenerkrankung, ist die häufigste mikrovaskuläre Folgeerkrankung des Diabetes mellitus Typ1 und Typ2. Sie wird in zwei Formen unterteilt, die nicht-proliferative Retinopathie und die proliferative Retinopathie, welche wiederum nach ihrem Schweregrad unterteilt sind.

Der diabetischen Retinopathie liegt in erster Linie eine Mikrogefäßschwäche zu Grunde. Es kommt zunächst zu einer Verdickung der Basalmembran der Gefäße und zum Verlust von gefäßummantelnden Perizyten, später folgt ein Verschluss von Gefäßen und eine retinale Ischämie. Die weitere Entwicklung wird dann durch die hervorgerufene retinale Hypoxie gesteuert, welche eine präretinale Neovaskularisation und eine verstärkte Gefäßpermeabilität mit nachfolgender Ausbildung eines Makulaödems zur Folge hat. Dies alles führt letztendlich zur Erblindung des Patienten.

Aus tierexperimentellen Ansätzen gibt es Hinweise, dass die Inhibition von Plasma-Kallikrein die erhöhte Gefäßpermeabilität inhibiert und somit die Ausbildung eines Makulaödems beziehungsweis der diabetischen Retinopathie verhindert kann.

Eine Aufgabe der vorliegenden Erfindung ist daher die Bereitstellung neuer Verbindungen zur Behandlung von Herz-Kreislauf-Erkrankungen, insbesondere von thrombotischen beziehungsweise thromboembolischen sowie ödematösen Erkrankungen, und/oder ophthalmologischen Erkrankungen, insbesondere von diabetischer Retinopathie beziehungsweise des Makulaödems, bei Menschen und Tieren, die eine große therapeutische Bandbreite aufweisen.

WO 2006/030032 beschreibt unter anderem substituierte Pyridinone als allosterische Modulatoren des mGluR2 Rezeptors und WO 2008/079787 beschreibt substituierte Pyridin-2-one und ihre Verwendung als Glucokinase Aktivatoren. WO 2007/131179 beschreibt substituierte Imidazole als Faktor Xa Inhibtoren zur Behandlung von Thrombosen.

Gegenstand der Erfindung sind Verbindungen der Formel

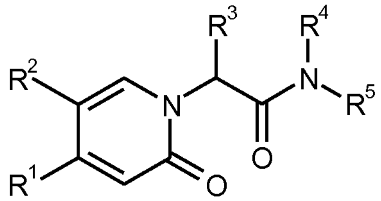

(I),

in welcher

$R^1$ für eine Gruppe der Formel

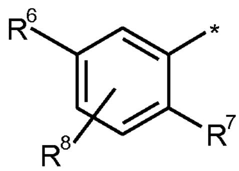

steht,
wobei * die Anknüpfstelle an den Oxopyridinring ist,
$R^6$ für Brom, Chlor, Fluor, Methyl, Difluormethyl, Trifluormethyl, Methoxy, Difluormethoxy oder Trifluormethoxy steht,
$R^7$ für Brom, Chlor, Fluor, Cyano, Nitro, Hydroxy, Methyl, Difluormethyl, Trifluormethyl, Methoxy, Ethoxy, Difluormethoxy, Trifluormethoxy, Ethinyl, 3,3,3-Trifluorprop-1-in-1-yl oder Cyclopropyl steht,
$R^8$ für Wasserstoff, Chlor oder Fluor steht,

$R^2$ für Chlor, Cyano, Methoxy, Ethoxy oder Difluormethoxy steht,

$R^3$ für Wasserstoff, $C_1$-$C_5$-Alkyl, $C_1$-$C_4$-Alkoxy, Difluormethyl, Trifluormethyl, 1,1-Difluorethyl, 1,1,2,2,2-Pentadeuteroethyl, 3,3,3-Trifluor-2-hydroxyprop-1-yl, 3,3,3-Trifluor-2-methoxyprop-1-yl, 3,3,3-Trifluor-2-ethoxyprop-1-yl, Prop-2-in-1-yl, Cyclopropyloxy oder Cyclobutyloxy steht,
wobei Alkyl substituiert sein kann mit einem Substituenten ausgewählt aus der Gruppe bestehend aus Fluor, Cyano, Hydroxy, Difluormethyl, Trifluormethyl, Methoxy, Ethoxy, Difluormethoxy, Trifluormethoxy, $C_3$-$C_6$-Cycloalkyl, 4- bis 6-gliedriges Oxo-Heterocyclyl, 4- bis 6-gliedriges Thio-Heterocyclyl, 1,4-Dioxanyl, Phenyl und Pyridyl,

worin Cycloalkyl substituiert sein kann mit 1 bis 2 Substituenten unabhängig voneinander ausgewählt aus der Gruppe bestehend aus Fluor, Hydroxy, Methyl, Ethyl, Methoxy, Ethoxy, Difluormethyl, Trifluormethyl, Difluormethoxy und Trifluormethoxy,
und
worin Oxo-Heterocyclyl und Thio-Heterocyclyl substituiert sein können mit 1 bis 2 Substituenten unabhängig voneinander ausgewählt aus der Gruppe bestehend aus Oxo, Fluor, Methyl, Ethyl, Difluormethyl und Trifluormethyl,

$R^4$ für Wasserstoff steht,

$R^5$ für eine Gruppe der Formel

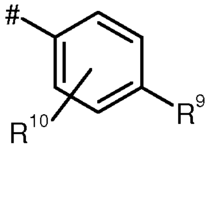

oder

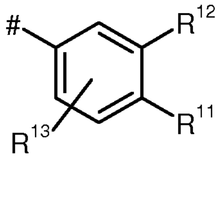

oder

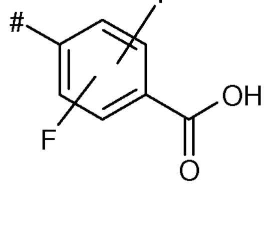

steht,
wobei # die Anknüpfstelle an das Stickstoffatom ist,
$R^9$ für Hydroxycarbonyl oder 5-gliedriges Heterocyclyl steht,
wobei Heterocyclyl substituiert sein kann mit 1 bis 2 Substituenten unabhängig voneinander ausgewählt aus der

Gruppe bestehend aus Oxo, Hydroxy, Thioxo, Sulfanyl, Methyl, Difluormethyl, Trifluormethyl, 2-Hydroxycarbonyl-1,1,2,2-tetrafluorethyl und 2-Methoxycarbonyl-1,1,2,2-tetrafluorethyl,
worin Methyl substituiert sein kann mit einem Substituenten Methoxy,
$R^{10}$ für Wasserstoff, Chlor, Fluor oder Methyl steht,
$R^{11}$ und $R^{12}$ zusammen mit den Kohlenstoffatomen an die sie gebunden sind einen 5-gliedrigen Heterocyclus bilden,
wobei der Heterocyclus substituiert sein kann mit 1 bis 2 Substituenten unabhängig voneinander ausgewählt aus der Gruppe bestehend aus Oxo, Chlor, Hydroxy, Hydroxycarbonyl, Methyl, Difluormethyl, Trifluormethyl, 1,1,2,2,2-Pentafluorethyl, 2-Hydroxycarbonyl-1,1,2,2-tetrafluorethyl und 2-Methoxycarbonyl-1,1,2,2-tetrafluorethyl,
$R^{13}$ für Wasserstoff, Chlor, Fluor, Methyl oder Methoxy steht,und ihre Salze, ihre Solvate und die Solvate ihrer Salze.

Erfindungsgemäße Verbindungen sind die Verbindungen der Formel (I) und deren Salze, Solvate und Solvate der Salze, sowie die von Formel (I) umfassten, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler beziehungsweise chiraler Phase.

Sofern die erfindungsgemäßen Verbindungen in tautomeren Formen vorkommen können, umfasst die vorliegenden Erfindung sämtliche tautomere Formen.

Im Rahmen der vorliegenden Erfindung wird der Begriff "enantiomerenrein" dahingehend verstanden, dass die betreffende Verbindung hinsichtlich der Absolutkonfiguration des chiralen Zentrums in einem Enantiomerenüberschuss vom mehr als 95%, bevorzugt vom mehr als 97% vorliegt. Der Enantiomerenüberschuss (engl. *enantiomeric excess,* ee-Wert) wird hierbei durch Auswertung des entsprechenden HPLC-Chromatogramms an chiraler Phase mit Hilfe der folgenden Formel berechnet:

$$ee = [E^A\,(\text{Flächen\%}) - E^B\,(\text{Flächen\%})] \times 100\% \,/\, [E^A\,(\text{Flächen\%}) + E^B\,(\text{Flächen\%})]$$

($E^A$: Enantiomer im Überschuss, $E^B$: Enantiomer im Unterschuss)

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie $^{2}H$ (Deuterium), $^{3}H$ (Tritium), $^{13}C$, $^{14}C$, $^{15}N$, $^{17}O$, $^{18}O$, $^{32}P$, $^{33}P$, $^{33}S$, $^{34}S$, $^{35}S$, $^{36}S$, $^{18}F$, $^{36}Cl$, $^{82}Br$, $^{123}I$, $^{124}I$, $^{129}I$ und $^{131}I$. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit $^{3}H$- oder $^{14}C$-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von

Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin, N-Methylpiperidin und Cholin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Außerdem beschreibt die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Im Rahmen der vorliegenden Erfindung haben die Substituenten, soweit nicht anders spezifiziert, die folgende Bedeutung:

Alkyl steht für einen linearen oder verzweigten Alkylrest mit 1 bis 5 Kohlenstoffatomen, bevorzugt 1 bis 3 Kohlenstoffatomen, beispielhaft und vorzugsweise für Methyl, Ethyl, n-Propyl, iso-Propyl, 2-Methyl-prop-1-yl, n-Butyl, *tert.*-Butyl und 2,2-Dimethylprop-1-yl.

Alkoxy steht für einen linearen oder verzweigten Alkoxyrest mit 1 bis 4 Kohlenstoffatomen, bevorzugt 1 bis 3 Kohlenstoffatomen, beispielhaft und vorzugsweise für Methoxy, Ethoxy, n-Propoxy, iso-Propoxy, 2-Methyl-prop-1-oxy, n-Butoxy und *tert.*-Butoxy.

Cycloalkyl steht für eine monocyclische Cycloalkylgruppe mit 3 bis 6 Kohlenstoffatomen, beispielhaft und vorzugsweise für Cycloalkyl seien genannt Cyclopropyl, Cyclobutyl, Cyclopentyl und Cyclohexyl.

5-gliedriges Heterocyclyl in der Definition des Restes $R^9$ steht für einen gesättigten, teilweise ungesättigten oder aromatischen monocyclischen Rest mit 5 Ringatomen und bis zu 4 Heteroatomen aus der Reihe S, O und N, wobei ein Stickstoffatom auch ein N-Oxid bilden kann, beispielhaft und vorzugsweise für Thienyl, Furyl, Pyrrolyl, Thiazolyl, Oxazolyl, Isoxazolyl, Oxadiazolyl, Thiadiazolyl, Pyrazolyl, Imidazolyl, Triazolyl, Tetrazolyl, Dihydrooxazolyl und Dihydroimidazolyl.

5-gliedriger Heterocyclus in der Definition der Reste $R^{11}$ und $R^{12}$ steht für einen gesättigten, teilweise ungesättigten oder aromatischen monocyclischen Rest mit 5 Ringatomen und bis zu 2 Heteroatomen aus der Reihe S, O und N, wobei ein Stickstoffatom auch ein N-Oxid bilden kann. Dieser 5-gliedrige Heterocyclus steht zusammen mit dem Phenylring, an den er gebunden ist, beispielhaft und vorzugsweise für 2,3-Dihydro-1-benzothiophen-5-yl, 1,3-Dihydro-2-benzothiophen-5-yl, 2,3-Dihydro-1-benzofuran-5-yl, 1,3-Dihydro-2-benzofuran-5-yl, Indolin-5-yl, Isoindolin-5-yl, 2,3-Dihydro-1H-indazol-5-yl, 2,3-Dihydro-1H-benzimidazol-5-yl, 1,3-Dihydro-2,1-benzoxazol-5-yl, 2,3-Dihydro-1,3-benzoxazol-5-yl, 1,3-Dihydro-2,1-benzothiazol-5-yl, 2,3-Dihydro-1,3-benzothiazol-5-yl, 1H-Benzimidazol-5-yl, 1H-Indazol-5-yl, 1,2-Benzoxazol-5-yl, Indol-5-yl, Isoindol-5-yl, Benzofuran-5-yl, Benzothiophen-5-yl, 2,3-Dihydro-1-benzothiophen-6-yl, 1,3-Dihydro-2-benzothiophen-6-yl, 2,3-Dihydro-1-benzofuran-6-yl, 1,3-Dihydro-2-benzofuran-6-yl, Indolin-6-yl, Isoindolin-6-yl, 2,3-Dihydro-1H-indazol-6-yl, 2,3-Dihydro-1H-benzimidazol-6-yl, 1,3-Dihydro-2,1-benzoxazol-6-yl, 2,3-Dihydro-1,3-benzoxazol-6-yl, 1,3-Dihydro-2,1-benzothiazol-6-yl, 2,3-Dihydro-1,3-benzothiazol-6-yl, 1H-Benzimidazol-6-yl, 1H-Indazol-6-yl, 1,2-Benzoxazol-6-yl, Indol-6-yl, Isoindol-6-yl, Benzofuran-6-yl und Benzothiophen-6-yl.

4- bis 6-gliedriges Oxo-Heterocyclyl in der Definition des Restes $R^3$ steht für einen gesättigten monocyclischen Rest mit 4 bis 6 Ringatomen, in dem ein Ringatom ein Sauerstoffatom ist, beispielhaft und vorzugsweise für Oxetanyl, Tetrahydrofuranyl und Tetrahydro-2H-pyranyl.

4- bis 6-gliedriges Thio-Heterocyclyl in der Definition des Restes $R^3$ steht für einen gesättigten monocyclischen Rest mit 4 bis 6 Ringatomen, in dem ein Ringatom ein Schwefelatom ist, beispielhaft und vorzugsweise für Thientanyl, Tetrahydrothienyl und Tetrahydro-2H-thiopyranyl.

In den Formeln der Gruppe, die für $R^1$ stehen kann, steht der Endpunkt der Linie, neben der jeweils ein * steht, nicht für ein Kohlenstoffatom beziehungsweise eine CH2-Gruppe sondern ist Bestandteil der Bindung zu dem Atom, an das $R^1$ gebunden ist.

In den Formeln der Gruppe, die für $R^5$ stehen kann, steht der Endpunkt der Linie, neben der jeweils ein # steht, nicht für ein Kohlenstoffatom beziehungsweise eine $CH_2$-Gruppe sondern ist Bestandteil der Bindung zu dem Atom, an das $R^5$ gebunden ist.

Bevorzugt sind Verbindungen der Formel (I), in welcher

$R^1$ für eine Gruppe der Formel

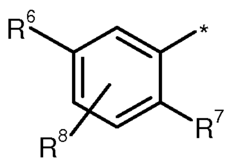

steht,
wobei * die Anknüpfstelle an den Oxopyridinring ist,
$R^6$ für Chlor steht,
$R^7$ für Brom, Chlor, Cyano, Nitro, Methyl, Difluormethyl, Trifluormethyl, Difluormethoxy, Trifluormethoxy, Ethinyl oder Cyclopropyl steht,
$R^8$ für Wasserstoff steht,

$R^2$ für Chlor, Cyano, Methoxy, Ethoxy oder Difluormethoxy steht,

$R^3$ für Wasserstoff, $C_1$-$C_5$-Alkyl, Ethoxy, 1,1,2,2,2-Pentadeuteroethyl oder Prop-2-in-1-yl steht,
wobei $C_1$-Alkyl substituiert sein kann mit einem Substituenten ausgewählt aus der Gruppe bestehend aus Difluormethyl, Trifluormethyl, Cyclopropyl, Cyclobutyl, Cyclohexyl, Oxetanyl, Tetrahydrofuranyl, Tetrahydro-2H-pyranyl, Tetrahydro-2H-thiopyranyl, 1,4-Dioxanyl, Phenyl und Pyridyl,

worin Cyclopropyl, Cyclobutyl, Cyclohexyl und Oxetanyl substituiert sein können mit 1 bis 2 Substituenten unabhängig voneinander ausgewählt aus der Gruppe bestehend aus Fluor, Hydroxy, Methyl, Ethyl, Methoxy und Trifluormethyl,
und
worin Tetrahydrofuranyl, Tetrahydro-2H-pyranyl und Tetrahydro-2H-thiopyranyl substituiert sein können mit 1 bis 2 Substituenten unabhängig voneinander ausgewählt aus der Gruppe bestehend aus Oxo, Methyl und Ethyl,

und
wobei $C_2$-$C_4$-Alkyl substituiert sein kann mit einem Substituenten ausgewählt aus der Gruppe bestehend aus Fluor, Hydroxy, Trifluormethyl, Methoxy und Trifluormethoxy,

$R^4$ für Wasserstoff steht,

$R^5$ für eine Gruppe der Formel

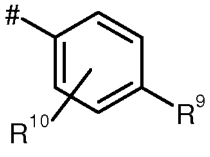

oder

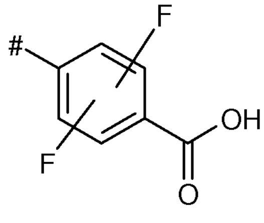

steht,
wobei # die Anknüpfstelle an das Stickstoffatom ist,
$R^9$ für Hydroxycarbonyl, Oxazolyl, Oxadiazolyl, Thiadiazolyl, Pyrazolyl, Imidazolyl, Triazolyl, Tetrazolyl oder Dihydrooxazolyl steht,
wobei Oxazolyl, Oxadiazolyl, Thiadiazolyl, Pyrazolyl, Imidazolyl, Triazolyl und Dihydrooxazolyl substituiert sein

können mit 1 bis 2 Substituenten unabhängig voneinander ausgewählt aus der Gruppe bestehend aus Oxo, Hydroxy, Thioxo, Sulfanyl, Methyl, Trifluormethyl und 2-Hydroxycarbonyl-1,1,2,2-tetrafluorethyl,
worin Methyl substituiert sein kann mit einem Substituenten Methoxy,
$R^{10}$ für Wasserstoff, Chlor, Fluor oder Methyl steht,oder

$R^5$ für 2,3-Dihydro-1H-indazol-6-yl, 1H-Benzimidazol-6-yl, Indol-6-yl, 2,3-Dihydro-1H-indazol-5-yl, 2,3-Dihydro-1H-benzimidazol-5-yl, Indol-5-yl, 1H-Indazol-6-yl oder 1H-Indazol-5-yl steht,
wobei der 5-gliedrige Heterocyclus in 2,3-Dihydro-1H-indazol-6-yl, 1H-Benzimidazol-6-yl, Indol-6-yl, 2,3-Dihydro-1H-indazol-5-yl, 2,3-Dihydro-1H-benzimidazol-5-yl, Indol-5-yl, 1H-Indazol-6-yl und 1H-Indazol-5-yl substituiert sein kann mit 1 bis 2 Substituenten unabhängig voneinander ausgewählt aus der Gruppe bestehend aus Oxo, Chlor, Hydroxycarbonyl, Methyl und Trifluormethyl,
und
wobei der Benzylring in 2,3-Dihydro-1H-indazol-6-yl, 1H-Benzimidazol-6-yl, Indol-6-yl, 2,3-Dihydro-1H-indazol-5-yl, 2,3-Dihydro-1H-benzimidazol-5-yl, Indol-5-yl, 1H-Indazol-6-yl und 1H-Indazol-5-yl substituiert sein kann mit einem Substituenten ausgewählt aus der Gruppe bestehend aus Fluor und Methoxy,

und ihre Salze, ihre Solvate und die Solvate ihrer Salze.

Bevorzugt sind auch Verbindungen der Formel (I), in welcher

$R^1$ für eine Gruppe der Formel

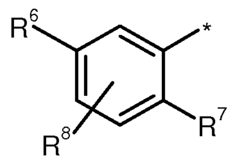

steht,
wobei * die Anknüpfstelle an den Oxopyridinring ist,
$R^6$ für Chlor steht,
$R^7$ für Cyano oder Difluormethoxy steht,
$R^8$ für Wasserstoff steht,

$R^2$ für Chlor, Cyano, Methoxy, Ethoxy oder Difluormethoxy steht,

$R^3$ für Methyl, Ethyl, n-Propyl, 2-Methyl-prop-1-yl oder n-Butyl steht,
wobei Methyl substituiert sein kann mit einem Substituenten ausgewählt aus der Gruppe bestehend aus Difluormethyl, Trifluormethyl, Cyclopropyl, Cyclobutyl, Cyclohexyl, Tetrahydrofuranyl, Tetrahydro-2H-pyranyl, 1,4-Dioxanyl, Phenyl und Pyridyl,
worin Cyclopropyl, Cyclobutyl und Cyclohexyl substituiert sein können mit 1 bis 2 Substituenten unabhängig voneinander ausgewählt aus der Gruppe bestehend aus Fluor, Hydroxy, Methyl, Methoxy und Trifluormethyl,
und
wobei Ethyl, n-Propyl und n-Butyl substituiert sein können mit einem Substituenten ausgewählt aus der Gruppe bestehend aus Fluor, Methoxy und Trifluormethoxy,

$R^4$ für Wasserstoff steht,

$R^5$ für eine Gruppe der Formel

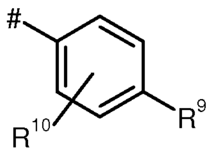

steht,
wobei # die Anknüpfstelle an das Stickstoffatom ist,
$R^9$ für Hydroxycarbonyl, Oxadiazolyl, Pyrazolyl, Triazolyl oder Tetrazolyl steht,

wobei Oxadiazolyl und Pyrazolyl substituiert sein können mit 1 bis 2 Substituenten unabhängig voneinander ausgewählt aus der Gruppe bestehend aus Oxo, Hydroxy und Trifluormethyl,
und
wobei Triazolyl substituiert sein kann mit einem Substituenten ausgewählt aus der Gruppe bestehend aus Trifluormethyl und 2-Hydroxycarbonyl-1,1,2,2-tetrafluorethyl,
$R^{10}$ für Wasserstoff oder Fluor steht,oder
$R^5$ für 2,3-Dihydro-1H-indazol-6-yl, 1H-Benzimidazol-6-yl, 2,3-Dihydro-1H-benzimidazol-5-yl oder 1H-Indazol-5-yl steht,
wobei der 5-gliedrige Heterocyclus in 2,3-Dihydro-1H-indazol-6-yl substituiert sein kann mit 1 bis 2 Substituenten unabhängig voneinander ausgewählt aus der Gruppe bestehend aus Oxo und Methyl,
und
wobei der Benzylring in 2,3-Dihydro-1H-indazol-6-yl substituiert sein kann mit einem Substituenten Fluor,
und
wobei der 5-gliedrige Heterocyclus in 1H-Benzimidazol-6-yl substituiert sein kann mit einem Substituenten Hydroxycarbonyl,
und
wobei der 5-gliedrige Heterocyclus in 2,3-Dihydro-1H-benzimidazol-5-yl substituiert sein kann mit einem Substituenten Oxo,
und
wobei der 5-gliedrige Heterocyclus in 1H-Indazol-5-yl substituiert sein kann mit einem Substituenten Chlor,

und ihre Salze, ihre Solvate und die Solvate ihrer Salze.

Bevorzugt sind auch Verbindungen der Formel (I), in welcher

$R^3$ für Wasserstoff, $C_1$-$C_5$-Alkyl, Ethoxy, 1,1,2,2,2-Pentadeuteroethyl oder Prop-2-in-1-yl steht,
wobei $C_1$-Alkyl substituiert sein kann mit einem Substituenten ausgewählt aus der Gruppe bestehend aus Difluormethyl, Trifluormethyl, Cyclopropyl, Cyclobutyl, Cyclohexyl, Oxetanyl, Tetrahydrofuranyl, Tetrahydro-2H-pyranyl, Tetrahydro-2H-thiopyranyl, 1,4-Dioxanyl, Phenyl und Pyridyl,

worin Cyclopropyl, Cyclobutyl, Cyclohexyl und Oxetanyl substituiert sein können mit 1 bis 2 Substituenten unabhängig voneinander ausgewählt aus der Gruppe bestehend aus Fluor, Hydroxy, Methyl, Ethyl, Methoxy und Trifluormethyl,
und

worin Tetrahydrofuranyl, Tetrahydro-2H-pyranyl und Tetrahydro-2H-thiopyranyl substituiert sein können mit 1 bis 2 Substituenten unabhängig voneinander ausgewählt aus der Gruppe bestehend aus Oxo, Methyl und Ethyl,

und
wobei $C_2$-$C_4$-Alkyl substituiert sein kann mit einem Substituenten ausgewählt aus der Gruppe bestehend aus Fluor, Hydroxy, Trifluormethyl, Methoxy und Trifluormethoxy.

Bevorzugt sind auch Verbindungen der Formel (I), in welcher

$R^3$ für Methyl, Ethyl, n-Propyl, 2-Methyl-prop-1-yl oder n-Butyl steht,
wobei Methyl substituiert sein kann mit einem Substituenten ausgewählt aus der Gruppe bestehend aus Difluormethyl, Trifluormethyl, Cyclopropyl, Cyclobutyl, Cyclohexyl, Tetrahydrofuranyl, Tetrahydro-2H-pyranyl, 1,4-Dioxanyl, Phenyl und Pyridyl,
worin Cyclopropyl, Cyclobutyl und Cyclohexyl substituiert sein können mit 1 bis 2 Substituenten unabhängig voneinander ausgewählt aus der Gruppe bestehend aus Fluor, Hydroxy, Methyl, Methoxy und Trifluormethyl,
und
wobei Ethyl, n-Propyl und n-Butyl substituiert sein können mit einem Substituenten ausgewählt aus der Gruppe bestehend aus Fluor, Methoxy und Trifluormethoxy.

Bevorzugt sind auch Verbindungen der Formel (I), in welcher

$R^5$ für eine Gruppe der Formel

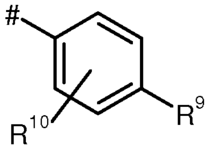

oder

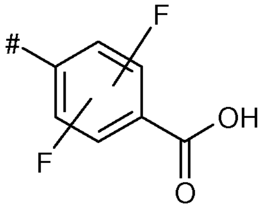

steht,
wobei # die Anknüpfstelle an das Stickstoffatom ist,
$R^9$ für Hydroxycarbonyl, Oxazolyl, Oxadiazolyl, Thiadiazolyl, Pyrazolyl, Imidazolyl, Triazolyl, Tetrazolyl oder Dihydrooxazolyl steht,
wobei Oxazolyl, Oxadiazolyl, Thiadiazolyl, Pyrazolyl, Imidazolyl, Triazolyl und Dihydrooxazolyl substituiert sein können mit 1 bis 2 Substituenten unabhängig voneinander ausgewählt aus der Gruppe bestehend aus Oxo, Hydroxy, Thioxo, Sulfanyl, Methyl, Trifluormethyl und 2-Hydroxycarbonyl-1,1,2,2-tetrafluorethyl,
worin Methyl substituiert sein kann mit einem Substituenten Methoxy,
$R^{10}$ für Wasserstoff, Chlor, Fluor oder Methyl steht.

Bevorzugt sind auch Verbindungen der Formel (I), in welcher

$R^5$ für eine Gruppe der Formel

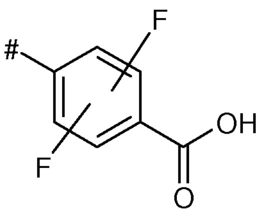

steht,
wobei # die Anknüpfstelle an das Stickstoffatom ist.

Bevorzugt sind auch Verbindungen der Formel (I), in welcher

$R^5$ für 2,3-Dihydro-1H-indazol-6-yl, 1H-Benzimidazol-6-yl, Indol-6-yl, 2,3-Dihydro-1H-indazol-5-yl, 2,3-Dihydro-1H-benzimidazol-5-yl, Indol-5-yl, 1H-Indazol-6-yl oder 1H-Indazol-5-yl steht,
wobei der 5-gliedrige Heterocyclus in 2,3-Dihydro-1H-indazol-6-yl, 1H-Benzimidazol-6-yl, Indol-6-yl, 2,3-Dihydro-1H-indazol-5-yl, 2,3-Dihydro-1H-benzimidazol-5-yl, Indol-5-yl, 1H-Indazol-6-yl und 1H-Indazol-5-yl substituiert sein kann mit 1 bis 2 Substituenten unabhängig voneinander ausgewählt aus der Gruppe bestehend aus Oxo, Chlor, Hydroxycarbonyl, Methyl und Trifluormethyl,
und
wobei der Benzylring in 2,3-Dihydro-1H-indazol-6-yl, 1H-Benzimidazol-6-yl, Indol-6-yl, 2,3-Dihydro-1H-indazol-5-yl, 2,3-Dihydro-1H-benzimidazol-5-yl, Indol-5-yl, 1H-Indazol-6-yl und 1H-Indazol-5-yl substituiert sein kann mit einem Substituenten ausgewählt aus der Gruppe bestehend aus Fluor und Methoxy.

Bevorzugt sind auch Verbindungen der Formel (I), in welcher

$R^5$ für 2,3-Dihydro-1H-indazol-6-yl, 1H-Benzimidazol-6-yl, 2,3-Dihydro-1H-benzimidazol-5-yl oder 1H-Indazol-5-yl steht,
wobei der 5-gliedrige Heterocyclus in 2,3-Dihydro-1H-indazol-6-yl substituiert sein kann mit 1 bis 2 Substituenten unabhängig voneinander ausgewählt aus der Gruppe bestehend aus Oxo und Methyl,
und
wobei der Benzylring in 2,3-Dihydro-1H-indazol-6-yl substituiert sein kann mit einem Substituenten Fluor,
und
wobei der 5-gliedrige Heterocyclus in 1H-Benzimidazol-6-yl substituiert sein kann mit einem Substituenten Hydroxycarbonyl,
und

wobei der 5-gliedrige Heterocyclus in 2,3-Dihydro-1H-benzimidazol-5-yl substituiert sein kann mit einem Substituenten Oxo,
und
wobei der 5-gliedrige Heterocyclus in 1H-Indazol-5-yl substituiert sein kann mit einem Substituenten Chlor.

Bevorzugt sind auch Verbindungen der Formel (I), in welcher

$R^5$ für 2,3-Dihydro-1H-indazol-6-yl, 1H-Benzimidazol-6-yl, Indol-6-yl, 2,3-Dihydro-1H-indazol-5-yl, 2,3-Dihydro-1H-benzimidazol-5-yl oder Indol-5-yl steht, wobei der 5-gliedrige Heterocyclus in 2,3-Dihydro-1H-indazol-6-yl, 1H-Benzimidazol-6-yl, Indol-6-yl, 2,3-Dihydro-1H-indazol-5-yl, 2,3-Dihydro-1H-benzimidazol-5-yl und Indol-5-yl substituiert sein kann mit 1 bis 2 Substituenten unabhängig voneinander ausgewählt aus der Gruppe bestehend aus Oxo, Hydroxycarbonyl, Methyl und Trifluormethyl.

Bevorzugt sind auch Verbindungen der Formel (I), in welcher

$R^1$ für eine Gruppe der Formel

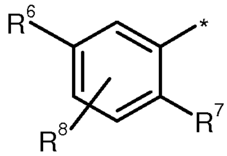

steht,
wobei * die Anknüpfstelle an den Oxopyridinring ist,
$R^6$ für Brom, Chlor, Fluor, Methyl, Difluormethyl, Trifluormethyl, Methoxy, Difluormethoxy oder Trifluormethoxy steht,
$R^7$ für Brom, Chlor, Fluor, Cyano, Nitro, Hydroxy, Methyl, Difluormethyl, Trifluormethyl, Methoxy, Ethoxy, Difluormethoxy, Trifluormethoxy, Ethinyl, 3,3,3-Trifluorprop-1-in-1-yl oder Cyclopropyl steht,
$R^8$ für Wasserstoff, Chlor oder Fluor steht,

$R^2$ für Chlor, Cyano, Methoxy, Ethoxy oder Difluormethoxy steht,

$R^3$ für Wasserstoff, $C_1$-$C_5$-Alkyl, $C_1$-$C_4$-Alkoxy, Difluormethyl, Trifluormethyl, 1,1-Difluorethyl, 3,3,3-Trifluor-2-hydroxyprop-1-yl, 3,3,3-Trifluor-2-methoxyprop-1-yl, 3,3,3-Trifluor-2-ethoxyprop-1-yl, Prop-2-in-1-yl, Cyclopropyloxy oder Cyclobutyloxy steht,
wobei Alkyl substituiert sein kann mit einem Substituenten ausgewählt aus der Gruppe bestehend aus Fluor, Cyano, Hydroxy, Difluormethyl, Trifluormethyl, Methoxy, Ethoxy, Difluormethoxy, Trifluormethoxy, $C_3$-$C_6$-Cycloalkyl, 4- bis 6-gliedriges Oxo-Heterocyclyl, 1,4-Dioxanyl, Phenyl und Pyridyl,
worin Cycloalkyl substituiert sein kann mit 1 bis 2 Substituenten unabhängig voneinander ausgewählt aus der Gruppe bestehend aus Fluor, Hydroxy, Methyl, Ethyl, Methoxy, Ethoxy, Difluormethyl, Trifluormethyl, Difluormethoxy und Trifluormethoxy,

$R^4$ für Wasserstoff steht,

$R^5$ für eine Gruppe der Formel

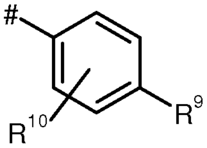

oder

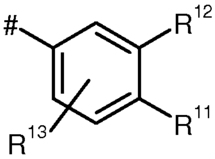

steht,
wobei # die Anknüpfstelle an das Stickstoffatom ist,

$R^9$ für Hydroxycarbonyl oder 5-gliedriges Heterocyclyl steht, wobei Heterocyclyl substituiert sein kann mit 1 bis 2 Substituenten unabhängig voneinander ausgewählt aus der Gruppe bestehend aus Oxo, Hydroxy, Methyl, Difluormethyl, Trifluormethyl, 2-Hydroxycarbonyl-1,1,2,2-tetrafluorethyl und 2-Methoxycarbonyl-1,1,2,2-tetrafluorethyl, worin Methyl substituiert sein kann mit einem Substituenten Methoxy,

$R^{10}$ für Wasserstoff, Chlor, Fluor oder Methyl steht,

$R^{11}$ und $R^{12}$ zusammen mit den Kohlenstoffatomen an die sie gebunden sind einen 5-gliedrigen Heterocyclus bilden, wobei der Heterocyclus substituiert sein kann mit 1 bis 2 Substituenten unabhängig voneinander ausgewählt aus der Gruppe bestehend aus Oxo, Chlor, Hydroxy, Hydroxycarbonyl, Methyl, Difluormethyl, Trifluormethyl, 1,1,2,2,2-Pentafluorethyl, 2-Hydroxycarbonyl-1,1,2,2-tetrafluorethyl und 2-Methoxycarbonyl-1,1,2,2-tetrafluorethyl,

$R^{13}$ für Wasserstoff, Chlor, Fluor oder Methyl steht,

und ihre Salze, ihre Solvate und die Solvate ihrer Salze.

Bevorzugt sind auch Verbindungen der Formel (I), in welcher

$R^1$ für eine Gruppe der Formel

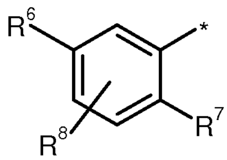

steht,
wobei * die Anknüpfstelle an den Oxopyridinring ist,
$R^6$ für Chlor steht,
$R^7$ für Brom, Chlor, Cyano, Nitro, Methyl, Difluormethyl, Trifluormethyl, Difluormethoxy, Trifluormethoxy, Ethinyl oder Cyclopropyl steht,
$R^8$ für Wasserstoff steht,

$R^2$ für Chlor, Cyano, Methoxy, Ethoxy oder Difluormethoxy steht,

$R^3$ für Wasserstoff, $C_1$-$C_5$-Alkyl, Ethoxy oder Prop-2-in-1-yl steht, wobei $C_1$-Alkyl substituiert sein kann mit einem Substituenten ausgewählt aus der Gruppe bestehend aus Difluormethyl, Trifluormethyl, Cyclopropyl, Cyclobutyl, Cyclohexyl, Oxetanyl, Tetrahydrofuranyl, Tetrahydro-2H-pyranyl, 1,4-Dioxanyl, Phenyl und Pyridyl,
worin Cyclopropyl, Cyclobutyl, Cyclohexyl und Oxetanyl substituiert sein können mit 1 bis 2 Substituenten unabhängig voneinander ausgewählt aus der Gruppe bestehend aus Fluor, Hydroxy, Methyl und Ethyl,
und
wobei $C_2$-$C_4$-Alkyl substituiert sein kann mit einem Substituenten ausgewählt aus der Gruppe bestehend aus Fluor, Hydroxy, Trifluormethyl, Methoxy und Trifluormethoxy,

$R^4$ für Wasserstoff steht,

$R^5$ für eine Gruppe der Formel

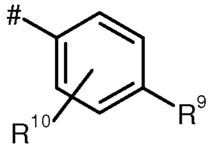

steht,
wobei # die Anknüpfstelle an das Stickstoffatom ist,
$R^9$ für Hydroxycarbonyl, Oxadiazolyl, Pyrazolyl, Imidazolyl, Triazolyl, Tetrazolyl oder Dihydrooxazolyl steht,

wobei Oxadiazolyl, Pyrazolyl, Imidazolyl, Triazolyl und Dihydrooxazolyl substituiert sein können mit 1 bis 2 Substituenten unabhängig voneinander ausgewählt aus der Gruppe bestehend aus Oxo, Hydroxy, Methyl, Trifluormethyl und 2-Hydroxycarbonyl-1,1,2,2-tetrafluorethyl,
worin Methyl substituiert sein kann mit einem Substituenten Methoxy,
$R^{10}$ für Wasserstoff, Chlor, Fluor oder Methyl steht,

oder

$R^5$ für 2,3-Dihydro-1H-indazol-6-yl, 1H-Benzimidazol-6-yl, Indol-6-yl, 2,3-Dihydro-1H-indazol-5-yl, 2,3-Dihydro-1H-benzimidazol-5-yl oder Indol-5-yl steht,
wobei 2,3-Dihydro-1H-indazol-6-yl, 1H-Benzimidazol-6-yl, Indol-6-yl, 2,3-Dihydro-1H-indazol-5-yl, 2,3-Dihydro-1H-benzimidazol-5-yl und Indol-5-yl substituiert sein können mit 1 bis 2 Substituenten unabhängig voneinander ausgewählt aus der Gruppe bestehend aus Oxo, Hydroxycarbonyl, Methyl und Trifluormethyl,

und ihre Salze, ihre Solvate und die Solvate ihrer Salze.

Bevorzugt sind auch Verbindungen der Formel (I), in welcher

$R^1$ für eine Gruppe der Formel

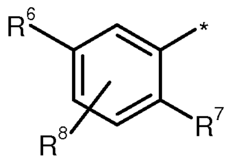

steht,
wobei * die Anknüpfstelle an den Oxopyridinring ist,
$R^6$ für Chlor steht,
$R^7$ für Cyano oder Difluormethoxy steht,
$R^8$ für Wasserstoff steht,
$R^2$ für Chlor, Cyano, Methoxy, Ethoxy oder Difluormethoxy steht,
$R^3$ für Methyl, Ethyl, n-Propyl, 2-Methyl-prop-1-yl oder n-Butyl steht,
wobei Methyl substituiert sein kann mit einem Substituenten ausgewählt aus der Gruppe bestehend aus Difluormethyl, Trifluormethyl, Cyclopropyl, Cyclobutyl, Cyclohexyl, Tetrahydrofuranyl, Tetrahydro-2H-pyranyl, 1,4-Dioxanyl, Phenyl und Pyridyl,
worin Cyclopropyl, Cyclobutyl und Cyclohexyl substituiert sein können mit 1 bis 2 Substituenten unabhängig voneinander ausgewählt aus der Gruppe bestehend aus Fluor, Hydroxy und Methyl,
und
wobei Ethyl, n-Propyl und n-Butyl substituiert sein können mit einem Substituenten ausgewählt aus der Gruppe bestehend aus Fluor, Methoxy und Trifluormethoxy,
$R^4$ für Wasserstoff steht,
$R^5$ für eine Gruppe der Formel

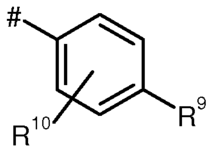

steht,
wobei # die Anknüpfstelle an das Stickstoffatom ist,

$R^9$ für Hydroxycarbonyl, Oxadiazolyl, Pyrazolyl, Triazolyl oder Tetrazolyl steht,
wobei Oxadiazolyl und Pyrazolyl substituiert sein können mit 1 bis 2 Substituenten unabhängig voneinander ausgewählt aus der Gruppe bestehend aus Oxo, Hydroxy und Trifluormethyl,
und
wobei Triazolyl substituiert sein kann mit einem Substituenten ausgewählt aus der Gruppe bestehend aus Trifluormethyl und 2-Hydroxycarbonyl-1,1,2,2-tetrafluorethyl,

$R^{10}$ für Wasserstoff oder Fluor steht,

oder

$R^5$ für 2,3-Dihydro-1H-indazol-6-yl, 1H-Benzimidazol-6-yl oder 2,3-Dihydro-1H-benzimidazol-5-yl steht, wobei 2,3-Dihydro-1H-indazol-6-yl substituiert sein kann mit 1 bis 2 Substituenten unabhängig voneinander ausgewählt aus der Gruppe bestehend aus Oxo und Methyl,
und
wobei 1H-Benzimidazol-6-yl substituiert sein kann mit einem Substituenten Hydroxycarbonyl,
und
wobei 2,3-Dihydro-1H-benzimidazol-5-yl substituiert sein kann mit einem Substituenten Oxo,

und ihre Salze, ihre Solvate und die Solvate ihrer Salze.

Bevorzugt sind auch Verbindungen der Formel (I), in welcher

$R^1$ für eine Gruppe der Formel

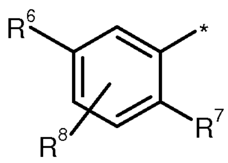

steht,
wobei * die Anknüpfstelle an den Oxopyridinring ist,
$R^6$ für Chlor steht,
$R^7$ für Cyano oder Difluormethoxy steht,
$R^8$ für Wasserstoff steht.

Bevorzugt sind auch Verbindungen der Formel (I), in welcher $R^2$ für Chlor, Cyano, Methoxy, Ethoxy oder Difluormethoxy steht.

Bevorzugt sind auch Verbindungen der Formel (I), in welcher

$R^3$ für $C_1$-$C_5$-Alkyl, $C_1$-$C_4$-Alkoxy, Difluormethyl, Trifluormethyl, 1,1-Difluorethyl, 3,3,3-Trifluor-2-hydroxyprop-1-yl, 3,3,3-Trifluor-2-methoxyprop-1-yl, 3,3,3-Trifluor-2-ethoxyprop-1-yl, Prop-2-in-1-yl, Cyclopropyloxy oder Cyclobutyloxy steht,
wobei Alkyl substituiert sein kann mit einem Substituenten ausgewählt aus der Gruppe bestehend aus Fluor, Cyano, Hydroxy, Difluormethyl, Trifluormethyl, Methoxy, Ethoxy, Difluormethoxy, Trifluormethoxy, $C_3$-$C_6$-Cycloalkyl, 4- bis 6-gliedriges Oxo-Heterocyclyl, 1,4-Dioxanyl, Phenyl und Pyridyl,
worin Cycloalkyl substituiert sein kann mit 1 bis 2 Substituenten unabhängig voneinander ausgewählt aus der Gruppe bestehend aus Fluor, Hydroxy, Methyl, Ethyl, Methoxy, Ethoxy, Difluormethyl, Trifluormethyl, Difluormethoxy und Trifluormethoxy.

Bevorzugt sind auch Verbindungen der Formel (I), in welcher

$R^3$ für $C_1$-$C_5$-Alkyl, Ethoxy oder Prop-2-in-1-yl steht,
wobei $C_1$-Alkyl substituiert sein kann mit einem Substituenten ausgewählt aus der Gruppe bestehend aus Difluormethyl, Trifluormethyl, Cyclopropyl, Cyclobutyl, Cyclohexyl, Oxetanyl, Tetrahydrofuranyl, Tetrahydro-2H-pyranyl, 1,4-Dioxanyl, Phenyl und Pyridyl,
worin Cyclopropyl, Cyclobutyl, Cyclohexyl und Oxetanyl substituiert sein können mit 1 bis 2 Substituenten unabhängig voneinander ausgewählt aus der Gruppe bestehend aus Fluor, Hydroxy, Methyl und Ethyl,
und
wobei $C_2$-$C_4$-Alkyl substituiert sein kann mit einem Substituenten ausgewählt aus der Gruppe bestehend aus Fluor, Hydroxy, Trifluormethyl, Methoxy und Trifluormethoxy.

Bevorzugt sind auch Verbindungen der Formel (I), in welcher

$R^3$ für Methyl, Ethyl, n-Propyl, 2-Methyl-prop-1-yl oder n-Butyl steht,
wobei Methyl substituiert sein kann mit einem Substituenten ausgewählt aus der Gruppe bestehend aus Difluormethyl, Trifluormethyl, Cyclopropyl, Cyclobutyl, Cyclohexyl, Tetrahydrofuranyl, Tetrahydro-2H-pyranyl, 1,4-Dioxanyl, Phenyl und Pyridyl,
worin Cyclopropyl, Cyclobutyl und Cyclohexyl substituiert sein können mit 1 bis 2 Substituenten unabhängig voneinander ausgewählt aus der Gruppe bestehend aus Fluor, Hydroxy und Methyl,
und
wobei Ethyl, n-Propyl und n-Butyl substituiert sein können mit einem Substituenten ausgewählt aus der Gruppe bestehend aus Fluor, Methoxy und Trifluormethoxy.

Bevorzugt sind auch Verbindungen der Formel (I), in welcher

$R^5$ für eine Gruppe der Formel

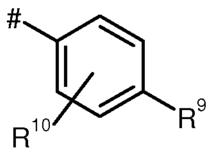

steht,
wobei # die Anknüpfstelle an das Stickstoffatom ist,

$R^9$ für Hydroxycarbonyl, Oxadiazolyl, Pyrazolyl, Triazolyl oder Tetrazolyl steht,
wobei Oxadiazolyl und Pyrazolyl substituiert sein können mit 1 bis 2 Substituenten unabhängig voneinander ausgewählt aus der Gruppe bestehend aus Oxo, Hydroxy und Trifluormethyl,
und
wobei Triazolyl substituiert sein kann mit einem Substituenten ausgewählt aus der Gruppe bestehend aus Trifluormethyl und 2-Hydroxycarbonyl-1,1,2,2-tetrafluorethyl,

$R^{10}$ für Wasserstoff oder Fluor steht.

Bevorzugt sind auch Verbindungen der Formel (I), in welcher

$R^5$ für 2,3-Dihydro-1H-indazol-6-yl, 1H-Benzimidazol-6-yl oder 2,3-Dihydro-1H-benzimidazol-5-yl steht,
wobei 2,3-Dihydro-1H-indazol-6-yl substituiert sein kann mit 1 bis 2 Substituenten unabhängig voneinander ausgewählt aus der Gruppe bestehend aus Oxo und Methyl,
und
wobei 1H-Benzimidazol-6-yl substituiert sein kann mit einem Substituenten Hydroxycarbonyl,
und
wobei 2,3-Dihydro-1H-benzimidazol-5-yl substituiert sein kann mit einem Substituenten Oxo.

Bevorzugt sind auch Verbindungen der Formel (I), in welcher

$R^5$ für 2,3-Dihydro-1H-indazol-6-yl, 1H-Benzimidazol-6-yl oder 2,3-Dihydro-1H-benzimidazol-5-yl steht,
wobei der 5-gliedrige Heterocyclus in 2,3-Dihydro-1H-indazol-6-yl substituiert sein kann mit 1 bis 2 Substituenten unabhängig voneinander ausgewählt aus der Gruppe bestehend aus Oxo und Methyl,
wobei der 5-gliedrige Heterocyclus in 1H-Benzimidazol-6-yl substituiert sein kann mit einem Substituenten Hydroxycarbonyl,
und
wobei der 5-gliedrige Heterocyclus in 2,3-Dihydro-1H-benzimidazol-5-yl substituiert sein kann mit einem Substituenten Oxo.

Bevorzugt sind auch Verbindungen der Formel (I), in welcher

$R^1$ für eine Gruppe der Formel

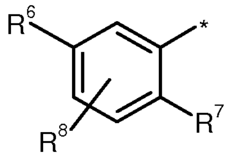

steht,
wobei * die Anknüpfstelle an den Oxopyridinring ist,
$R^6$ für Brom, Chlor, Fluor, Methyl, Difluormethyl, Trifluormethyl, Methoxy, Difluormethoxy oder Trifluormethoxy steht,
$R^7$ für Brom, Chlor, Fluor, Cyano, Nitro, Hydroxy, Methyl, Difluormethyl, Trifluormethyl, Methoxy, Difluormethoxy, Trifluormethoxy, Ethinyl, 3,3,3-Trifluorprop-1-in-1-yl oder Cyclopropyl steht,
$R^8$ für Wasserstoff, Chlor oder Fluor steht,

$R^2$ für Chlor, Cyano, Methoxy, Ethoxy oder Difluormethoxy steht,

$R^3$ für $C_1$-$C_5$-Alkyl, Difluormethyl, Trifluormethyl, 1,1-Difluorethyl oder Prop-2-in-1-yl steht, wobei Alkyl substituiert sein kann mit einem Substituenten ausgewählt aus der Gruppe bestehend aus Fluor, Cyano, Hydroxy, Difluormethyl, Trifluormethyl, Methoxy, Difluormethoxy, Trifluormethoxy, $C_3$-$C_6$-Cycloalkyl, 4- bis 6-gliedriges Oxo-Heterocyclyl, Phenyl und Pyridyl,

$R^4$ für Wasserstoff steht,

$R^5$ für eine Gruppe der Formel

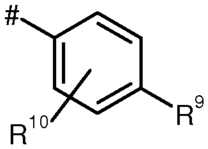

oder

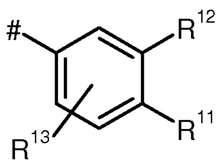

steht,
wobei # die Anknüpfstelle an das Stickstoffatom ist,
$R^9$ für Hydroxycarbonyl oder 5-gliedriges Heterocyclyl steht,
wobei Heterocyclyl substituiert sein kann mit 1 bis 2 Substituenten ausgewählt aus der Gruppe bestehend aus Oxo, Hydroxy, Methyl, Difluormethyl und Trifluormethyl,
worin Methyl substituiert sein kann mit einem Substituenten Methoxy,
$R^{10}$ für Wasserstoff, Chlor, Fluor oder Methyl steht,
$R^{11}$ und $R^{12}$ zusammen mit den Kohlenstoffatomen an die sie gebunden sind einen 5-gliedrigen Heterocyclus bilden,
wobei der Heterocyclus substituiert sein kann mit 1 bis 2 Substituenten ausgewählt aus der Gruppe bestehend aus Oxo, Hydroxy, Methyl, Difluormethyl, Trifluormethyl und 1,1,2,2,2-Pentafluorethyl,
$R^{13}$ für Wasserstoff, Chlor, Fluor oder Methyl steht,

und ihre Salze, ihre Solvate und die Solvate ihrer Salze

Bevorzugt sind auch Verbindungen der Formel (I), in welcher

$R^1$ für eine Gruppe der Formel

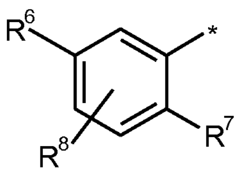

steht,
wobei * die Anknüpfstelle an den Oxopyridinring ist,
$R^6$ für Chlor steht,
$R^7$ für Brom, Chlor, Cyano, Nitro, Difluormethyl, Trifluormethyl, Trifluormethoxy, Ethinyl oder Cyclopropyl steht,
$R^8$ für Wasserstoff steht,

$R^2$ für Chlor, Cyano, Methoxy oder Ethoxy steht,

$R^3$ für $C_1$-$C_5$-Alkyl oder Prop-2-in-1-yl steht,
wobei $C_1$-Alkyl substituiert sein kann mit einem Substituenten ausgewählt aus der Gruppe bestehend aus Cyclopropyl, Phenyl und Pyridyl,

$R^4$ für Wasserstoff steht,

$R^5$ für eine Gruppe der Formel

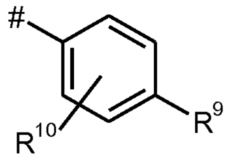

steht,
wobei # die Anknüpfstelle an das Stickstoffatom ist,
$R^9$ für Hydroxycarbonyl, Oxadiazolyl, Pyrazolyl, Imidazolyl, Triazolyl oder Tetrazolyl steht,
wobei Oxadiazolyl, Pyrazolyl, Imidazolyl und Triazolyl substituiert sein können mit 1 bis 2 Substituenten ausgewählt aus der Gruppe bestehend aus Oxo, Hydroxy, Methyl und Trifluormethyl,
worin Methyl substituiert sein kann mit einem Substituenten Methoxy,
$R^{10}$ für Wasserstoff, Chlor, Fluor oder Methyl steht,

oder

$R^5$ für 2,3-Dihydro-1H-indazol-6-yl, 1H-Benzimidazol-6-yl oder 2,3-Dihydro-1H-indazol-5-yl steht,
wobei 2,3-Dihydro-1H-indazol-6-yl, 1H-Benzimidazol-6-yl und 2,3-Dihydro-1H-indazol-5-yl substituiert sein können mit 1 bis 2 Substituenten ausgewählt aus der Gruppe bestehend aus Oxo, Methyl und Trifluormethyl,

und ihre Salze, ihre Solvate und die Solvate ihrer Salze.

Bevorzugt sind auch Verbindungen der Formel (I), in welcher

$R^1$ für eine Gruppe der Formel

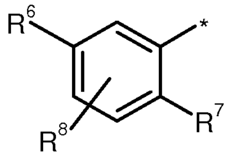

steht,
wobei * die Anknüpfstelle an den Oxopyridinring ist,
$R^6$ für Chlor steht,
$R^7$ für Cyano oder Trifluormethyl steht,
$R^8$ für Wasserstoff steht,

$R^2$ für Chlor, Fluor, Cyano, Methoxy oder Ethoxy steht,

$R^3$ für Methyl, Ethyl oder 2-Methyl-prop-1-yl steht,

wobei Methyl substituiert sein kann mit einem Substituenten Cyclopropyl,

$R^4$ für Wasserstoff steht,

$R^5$ für eine Gruppe der Formel

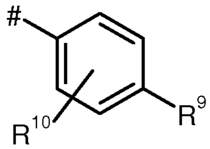

steht,
wobei # die Anknüpfstelle an das Stickstoffatom ist,
$R^9$ für Hydroxycarbonyl, Oxadiazolyl, Pyrazolyl, Imidazolyl, Triazolyl oder Tetrazolyl steht,
wobei Oxadiazolyl, Pyrazolyl, Imidazolyl und Triazolyl substituiert sein können mit 1 bis 2 Substituenten ausgewählt aus der Gruppe bestehend aus Oxo, Hydroxy, Methyl und Trifluormethyl,
worin Methyl substituiert sein kann mit einem Substituenten Methoxy,
$R^{10}$ für Wasserstoff, Chlor, Fluor oder Methyl steht,

oder

$R^5$ für 2,3-Dihydro-1H-indazol-6-yl oder 1H-Benzimidazol-6-yl steht,
wobei 2,3-Dihydro-1H-indazol-6-yl und 1H-Benzimidazol-6-yl substituiert sein können mit 1 bis 2 Substituenten ausgewählt aus der Gruppe bestehend aus Oxo, Methyl und Trifluormethyl,

und ihre Salze, ihre Solvate und die Solvate ihrer Salze.

Bevorzugt sind auch Verbindungen der Formel (I), in welcher

$R^1$ für eine Gruppe der Formel

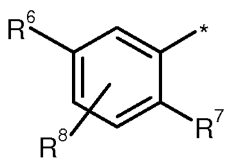

steht,
wobei * die Anknüpfstelle an den Oxopyridinring ist,
$R^6$ für Chlor steht,
$R^7$ für Cyano oder Trifluormethoxy steht,
$R^8$ für Wasserstoff steht.

Bevorzugt sind auch Verbindungen der Formel (I), in welcher $R^2$ für Chlor, Cyano, Methoxy oder Ethoxy steht.

Bevorzugt sind auch Verbindungen der Formel (I), in welcher $R^3$ für Methyl, Ethyl oder 2-Methyl-prop-1-yl steht, wobei Methyl substituiert sein kann mit einem Substituenten Cyclopropyl.

Bevorzugt sind auch Verbindungen der Formel (I), in welcher

$R^5$ für eine Gruppe der Formel

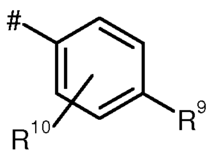

steht,

wobei # die Anknüpfstelle an das Stickstoffatom ist,
$R^9$ für Hydroxycarbonyl, Oxadiazolyl, Pyrazolyl, Imidazolyl, Triazolyl oder Tetrazolyl steht,
wobei Oxadiazolyl, Pyrazolyl, Imidazolyl und Triazolyl substituiert sein können mit 1 bis 2 Substituenten ausgewählt aus der Gruppe bestehend aus Oxo, Hydroxy, Methyl und Trifluormethyl,
worin Methyl substituiert sein kann mit einem Substituenten Methoxy,
$R^{10}$ für Wasserstoff, Chlor, Fluor oder Methyl steht.

Bevorzugt sind auch Verbindungen der Formel (I), in welcher

$R^5$ für 2,3-Dihydro-1H-indazol-6-yl, 1H-Benzimidazol-6-yl oder 2,3-Dihydro-1H-indazol-5-yl steht,
wobei der 5-gliedrige Heterocyclus in 2,3-Dihydro-1H-indazol-6-yl, 1H-Benzimidazol-6-yl und 2,3-Dihydro-1H-indazol-5-yl substituiert sein kann mit 1 bis 2 Substituenten ausgewählt aus der Gruppe bestehend aus Oxo, Methyl und Trifluormethyl.

Bevorzugt sind auch Verbindungen der Formel (I), in welcher $R^5$ für 2,3-Dihydro-1H-indazol-6-yl oder 1H-Benzimidazol-6-yl steht, wobei 2,3-Dihydro-1H-indazol-6-yl und 1H-Benzimidazol-6-yl substituiert sein können mit 1 bis 2 Substituenten ausgewählt aus der Gruppe bestehend aus Oxo, Methyl und Trifluormethyl.

Bevorzugt sind auch Verbindungen der Formel (I), in welcher $R^5$ für 2,3-Dihydro-1H-indazol-6-yl oder 1H-Benzimidazol-6-yl steht, wobei der 5-gliedrige Heterocyclus in 2,3-Dihydro-1H-indazol-6-yl und 1H-Benzimidazol-6-yl substituiert sein kann mit 1 bis 2 Substituenten ausgewählt aus der Gruppe bestehend aus Oxo, Methyl und Trifluormethyl.

Bevorzugt sind auch Verbindungen, welche die Formel (Ia) aufweisen

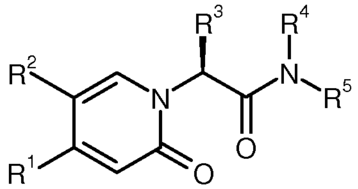 (Ia),

worin $R^1$, $R^2$, $R^3$, $R^4$ und $R^5$ wie oben definiert sind.

Gegenstand der Erfindung ist weiterhin ein Verfahren zur Herstellung der Verbindungen der Formel (I), oder ihrer Salze, ihrer Solvate oder der Solvate ihrer Salze, wobei

[A] die Verbindungen der Formel

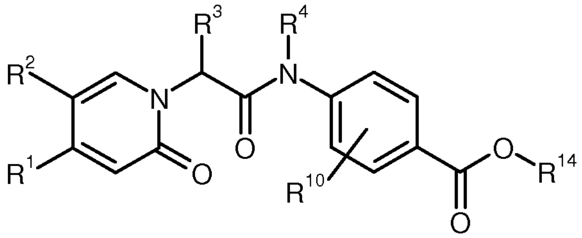 (IIa),

in welcher

$R^1$, $R^2$, $R^3$, $R^4$ und $R^{10}$ die oben angegebene Bedeutung haben, und

$R^{14}$ für tert-Butyl steht,

mit einer Säure zu Verbindungen der Formel

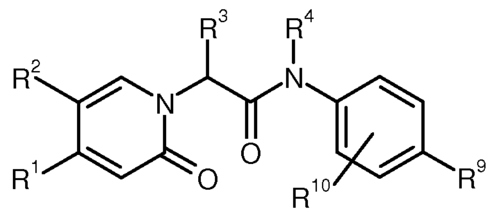

(Ib),

in welcher

$R^1$, $R^2$, $R^3$, $R^4$ und $R^{10}$ die oben angegebene Bedeutung haben, und
$R^9$ für Hydroxycarbonyl steht,

umgesetzt werden,
oder

[B] die Verbindungen der Formel

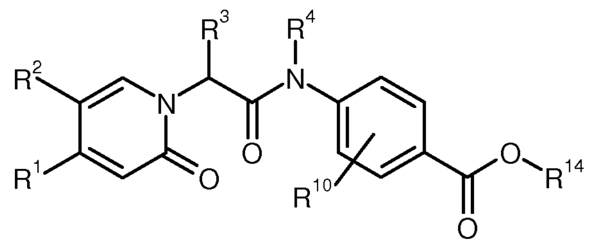

(IIb),

in welcher

$R^1$, $R^2$, $R^3$, $R^4$ und $R^{10}$ die oben angegebene Bedeutung haben, und
$R^{14}$ für Methyl oder Ethyl steht,

mit einer Base zu Verbindungen der Formel

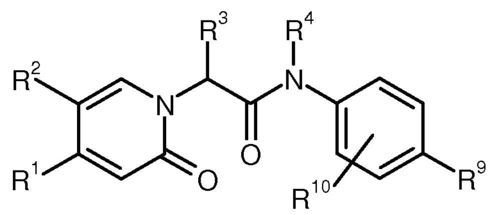

(Ib),

in welcher

$R^1$, $R^2$, $R^3$, $R^4$ und $R^{10}$ die oben angegebene Bedeutung haben, und
$R^9$ für Hydroxycarbonyl steht,

umgesetzt werden,
oder

[C] die Verbindungen der Formel

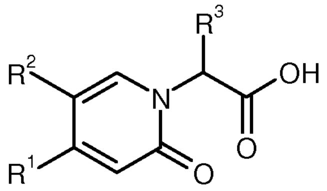

(III),

in welcher
$R^1$, $R^2$ und $R^3$ die oben angegebene Bedeutung haben,
mit Verbindungen der Formel

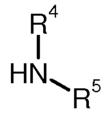

(IV),

in welcher
$R^4$ und $R^5$ die oben angegebene Bedeutung haben,
in Gegenwart eines Dehydratisierungsreagenzes zu Verbindungen der Formel (I) umgesetzt werden,
oder

[D] die Verbindungen der Formel

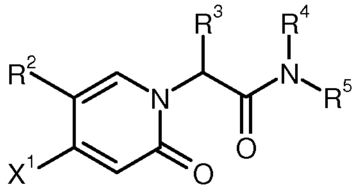

(V),

in welcher

$R^2$, $R^3$, $R^4$ und $R^5$ die oben angegebene Bedeutung haben, und

$X^1$ für Chlor, Brom oder Iod steht,

mit Verbindungen der Formel

$R^1$-Q (VI),

in welcher

$R^1$ die oben angegebene Bedeutung hat, und

Q für $-B(OH)_2$, einen Boronsäure-Ester, bevorzugt Boronsäurepinakolester, oder $-BF_3^-K^+$ steht,

unter Suzuki-Kupplungsbedingungen zu Verbindungen der Formel (I) umgesetzt werden.

Die Verbindungen der Formel (Ib) sind eine Teilmenge der Verbindungen der Formel (I).

Die Verbindungen der Formeln (IIa) und (IIb) bilden zusammen die Menge der Verbindungen der Formel (II).

Die Umsetzung nach Verfahren [A] erfolgt im Allgemeinen in inerten Lösungsmitteln, bevorzugt in einem Temperaturbereich von Raumtemperatur bis 60°C bei Normaldruck.

Inerte Lösungsmittel sind beispielsweise Halogenkohlenwasserstoffe wie Dichlormethan, Trichlormethan, Tetrachlormethan oder 1,2-Dichlorethan, oder Ether wie Tetrahydrofuran oder Dioxan, bevorzugt ist Dichlormethan.

Säuren sind beispielsweise Trifluoressigsäure oder Chlorwasserstoff in Dioxan, bevorzugt ist Trifluoressigsäure.

Die Umsetzung nach Verfahren [B] erfolgt im Allgemeinen in inerten Lösungsmitteln, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist ein Gemisch aus Tetrahydrofuran und Wasser oder ein Gemisch aus Methanol und Wasser.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, oder Alkoholate wie Kalium- oder Natrium-tert-butylat, bevorzugt ist Lithiumhydroxid oder Cäsiumcarbonat.

Die Umsetzung nach Verfahren [C] erfolgt im Allgemeinen in inerten Lösungsmitteln, gegebenenfalls in Gegenwart einer Base, bevorzugt in einem Temperaturbereich von 0°C bis Raumtemperatur bei Normaldruck.

Als Dehydratisierungsreagenzien eignen sich hierbei beispielsweise Carbodiimide wie z.B. *N,N'*-Diethyl-, *N,N'*-Dipropyl-, *N,N'*-Diisopropyl-, *N,N'*-Dicyclohexylcarbodiimid, *N*-(3-Dimethylamino-isopropyl)-*N'*-ethylcarbodiimid-Hydrochlorid (EDC) (gegebenenfalls in Gegenwart von Pentafluorphenol (PFP)), *N*-Cyclohexylcarbodiimid-*N'*-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder *O*-(Benzotriazol-1-yl)-*N,N,N',N'*-tetra-methyluronium-hexafluorophosphat (HBTU), 2-(2-Oxo-1-(2H)-pyridyl)-1,1,3,3-tetramethyluroniumtetrafluoroborat (TPTU), (Benzotriazol-1-yloxy)bisdimethylamino-methyliumfluoroborat (TBTU) oder *O*-(7-Azabenzotriazol-1-yl)-*N,N,N',N'*-tetramethyl-uronium-hexafluorophosphat (HATU), oder 1-Hydroxybenztriazol (HOBt), oder Benzotriazol-1-yloxy-tris(dimethylamino)-phosphoniumhexafluorophosphat (BOP), oder Mischungen aus diesen, mit Basen. Vorzugsweise wird die Kondensation mit HATU durchgeführt.

Basen sind beispielsweise Alkalicarbonate, wie z.B. Natrium- oder Kaliumcarbonat, oder -hydrogencarbonat, oder organische Basen wie Trialkylamine, z.B. Triethylamin, N-Methylmorpholin, N-Methylpiperidin, 4-Dimethylaminopyridin oder Diisopropylethylamin. Vorzugsweise wird die Kondensation mit Diisopropylethylamin durchgeführt.

Inerte Lösungsmittel sind beispielsweise Halogenkohlenwasserstoffe wie Dichlormethan oder Trichlormethan, Kohlenwasserstoffe wie Benzol, oder andere Lösungsmittel wie Nitromethan, Dioxan, Dimethylformamid, Dimethylsulfoxid oder Acetonitril. Ebenso ist es möglich, Gemische der Lösemittel einzusetzen. Besonders bevorzugt ist Dimethylformamid.

Die Umsetzung nach Verfahren [D] erfolgt im Allgemeinen in inerten Lösungsmitteln, in Gegenwart eines Katalysators, gegebenenfalls in Gegenwart eines Zusatzreagenzes, gegebenenfalls in einer Mikrowelle, bevorzugt in einem Temperaturbereich von Raumtemperatur bis 150°C bei Normaldruck bis 3 bar.

Katalysatoren sind beispielsweise für Suzuki-Reaktionsbedingungen übliche Palladium-Katalysatoren, bevorzugt sind Katalysatoren wie z.B. Dichlorbis(triphenylphosphin)-palladium, Tetrakistriphenylphosphinpalladium(0), Palladium(II)acetat/Triscyclohexylphosphin, Tris(dibenzylidenaceton)dipalladium, Bis-(diphenylphosphanferrocenyl)-palladium-(II)-chlorid, 1,3-Bis(2,6-diisopropylphenyl)imidazol-2-yliden(1,4-napthtochinon)palladiumdimer, Allyl(chlor)-(1,3-dimesityl-1,3-dihydro-2H-imidazol-2-yliden)palladium, Palladium(II)acetat/Dicyclohexyl-(2',4',6'-triisopropyl-biphenyl-2-yl)-phosphin, [1,1-Bis-(diphenylphosphino)-ferrocen]-palladium(II)chlorid-Monodichlormethan-addukt oder XPhos Prekatalysator [(2'-Aminobiphenyl-2-yl)(chlor)palladium-Dicyclohexyl(2',4',6'-triisopropylbiphenyl-2-yl)phosphan (1:1)], bevorzugt ist Tetrakistriphenylphosphin-palladium(0), [1,1-Bis-(diphenylphosphino)-ferrocen]-palladium(II)chlorid-Monodichlormethan-addukt oder XPhos Prekatalysator [(2'-Aminobiphenyl-2-yl)(chlor)palladium-Dicyclohexyl(2',4',6'-triisopropylbiphenyl-2-yl)phosphan (1:1)].

Zusatzreagenzien sind beispielsweise Kaliumacetat, Cäsium-, Kalium- oder Natriumcarbonat, Kalium-tert.-butylat, Cäsiumfluorid oder Kaliumphosphat, wobei diese in wässriger Lösung vorliegen können, bevorzugt sind Zusatzreagenzien wie Kaliumcarbonat oder wässrige Kaliumphosphat-Lösung.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, oder Carbonsäureamide wie Dimethylformamid oder Dimethylacetamid, Alkylsulfoxide wie Dimethylsulfoxid, oder N-Methylpyrrolidon oder Acetonitril, oder Gemische der Lösungsmittel mit Alkoholen wie Methanol oder Ethanol und/oder Wasser, bevorzugt ist Tetrahydrofuran, Dioxan oder Acetonitril.

Die Verbindungen der Formel (IV) sind bekannt, lassen sich nach bekannten Verfahren aus den entsprechenden Ausgangsverbindungen synthetisieren oder können analog den im Beispielteil beschriebenen Verfahren hergestellt werden.

Die Verbindungen der Formel (VI) sind bekannt oder lassen sich nach bekannten Verfahren aus den entsprechenden Ausgangsverbindungen synthetisieren.

Die Verbindungen der Formel (II) sind bekannt oder können hergestellt werden, indem Verbindungen der Formel

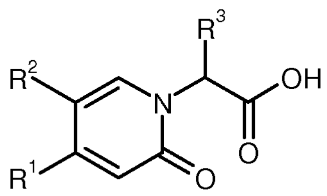

(III),

in welcher $R^1$, $R^2$ und $R^3$ die oben angegebene Bedeutung haben, mit Verbindungen der Formel

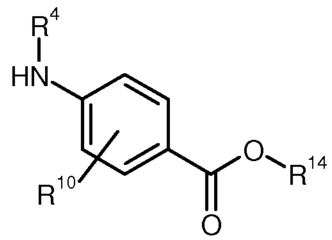

(VII),

in welcher

$R^4$ und $R^{10}$ die oben angegebene Bedeutung haben, und
$R^{14}$ für Methyl, Ethyl oder tert-Butyl steht,

in Gegenwart eines Dehydratisierungsreagenzes umgesetzt werden.

Die Umsetzung erfolgt wie für Verfahren [C] beschrieben.

Die Verbindungen der Formel (VII) sind bekannt, lassen sich nach bekannten Verfahren aus den entsprechenden Ausgangsverbindungen synthetisieren oder können analog den im Beispielteil beschriebenen Verfahren hergestellt werden.

Die Verbindungen der Formel (III) sind bekannt oder können hergestellt werden, indem

[E] Verbindungen der Formel

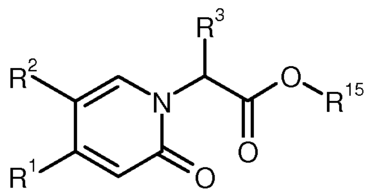

(VIIIa),

in welcher

$R^1$, $R^2$ und $R^3$ die oben angegebene Bedeutung haben, und
$R^{15}$ für tert-Butyl steht,

mit einer Säure umgesetzt werden,
oder

[F] Verbindungen der Formel

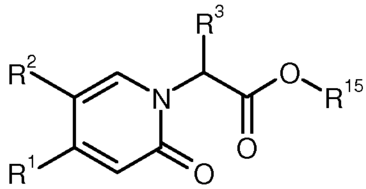

(VIIIb),

in welcher

$R^1$, $R^2$ und $R^3$ die oben angegebene Bedeutung haben, und
$R^{15}$ für Methyl, Ethyl oder Benzyl steht,

mit einer Base umgesetzt werden.

Die Verbindungen der Formeln (VIIIa) und (VIIIb) bilden zusammen die Menge der Verbindungen der Formel (VIII).

Die Umsetzung nach Verfahren [E] erfolgt wie für Verfahren [A] beschrieben.

Die Umsetzung nach Verfahren [F] erfolgt wie für Verfahren [B] beschrieben.

Die Verbindungen der Formel (VIII) sind bekannt oder können hergestellt werden, indem

[G] Verbindungen der Formel

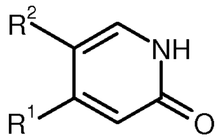

(IX),

in welcher
$R^1$ und $R^2$ die oben angegebene Bedeutung haben,
mit Verbindungen der Formel

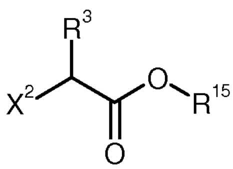

(X),

in welcher

$R^3$ die oben angegebene Bedeutung hat,
$R^{15}$ für Methyl, Ethyl, Benzyl oder tert-Butyl steht, und
$X^2$ für Chlor, Brom, Iod, Methansulfonyloxy oder Trifluormethansulfonyloxy steht,

umgesetzt werden,
oder

[H] Verbindungen der Formel

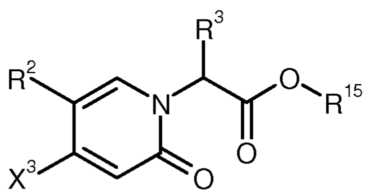

(XI),

in welcher

$R^2$ und $R^3$ die oben angegebene Bedeutung haben,
$R^{15}$ für Methyl, Ethyl, Benzyl oder tert-Butyl steht, und
$X^3$ für Chlor, Brom oder Iod steht,

mit Verbindungen der Formel (VI) unter Suzuki-Kupplungsbedingungen umgesetzt werden.

Die Umsetzung nach Verfahren [G] erfolgt im Allgemeinen in inerten Lösungsmitteln, gegebenenfalls in Gegenwart einer Base, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist Dimethylformamid.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, oder Kalium- oder Natrium-tert-butylat, Natriumhydrid oder eine Mischung aus diesen Basen oder eine Mischung aus Natriumhydrid und Lithiumbromid, bevorzugt ist Kaliumcarbonat oder Natriumhydrid.

Die Verbindungen der Formel (X) sind bekannt oder lassen sich nach bekannten Verfahren aus den entsprechenden Ausgangsverbindungen synthetisieren.

Die Umsetzung nach Verfahren [H] erfolgt wie für Verfahren [D] beschrieben.

Weitere Verfahren, wie die Verbindungen der Formel (VIII) hergestellt werden können, sind unter den Ausgangsverbindungen in den Beispielen 32.1A-C, 41.1A-C, 43.1B, 43.1C, 44.1B und 44.1C zu finden.

Die Verbindungen der Formel (IX) sind bekannt oder können hergestellt werden, indem Verbindungen der Formel

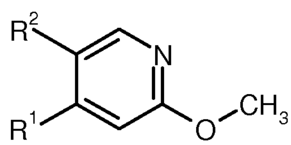 (XII),

in welcher
$R^1$ und $R^2$ die oben angegebene Bedeutung haben,
mit Pyridinium-Hydrochlorid oder Pyridinium-Hydrobromid umgesetzt werden.

Die Umsetzung erfolgt im Allgemeinen in inerten Lösungsmitteln, bevorzugt in einem Temperaturbereich von 80°C bis 120°C bei Normaldruck.

Inerte Lösungsmittel sind beispielsweise Kohlenwasserstoffe wie Benzol, oder andere Lösungsmittel wie Nitromethan, Dioxan, Dimethylformamid, Dimethylsulfoxid oder Acetonitril. Ebenso ist es möglich, Gemische der Lösemittel einzusetzen. Besonders bevorzugt ist Dimethylformamid.

Die Verbindungen der Formel (XII) sind bekannt oder können hergestellt werden, indem Verbindungen der Formel

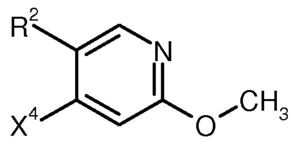 (XIII),

in welcher

$R^2$ die oben angegebene Bedeutung hat, und

$X^4$ für Chlor, Brom oder Iod steht,

mit Verbindungen der Formel (VI) unter Suzuki-Kupplungsbedingungen umgesetzt werden.

Die Umsetzung erfolgt wie für Verfahren [D] beschrieben.

Die Verbindungen der Formel (XIII) sind bekannt oder lassen sich nach bekannten Verfahren aus den entsprechenden Ausgangsverbindungen synthetisieren.

Die Verbindungen der Formel (XI) sind bekannt oder können hergestellt werden, indem Verbindungen der Formel

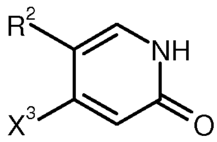 (XIV),

in welcher

$R^2$ die oben angegebene Bedeutung hat, und

$X^3$ für Chlor, Brom oder Iod steht,

mit Verbindungen der Formel (X) umgesetzt werden.

Die Umsetzung erfolgt wie für Verfahren [G] beschrieben.

Die Verbindungen der Formel (XIV) sind bekannt oder lassen sich nach bekannten Verfahren aus den entsprechenden Ausgangsverbindungen synthetisieren.

Die Verbindungen der Formel (V) sind bekannt oder können hergestellt werden, indem Verbindungen der Formel

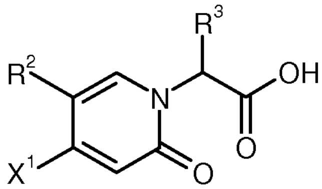 (XV),

in welcher

$R^2$ und $R^3$ die oben angegebene Bedeutung haben, und
$X^1$ für Chlor, Brom oder Iod steht,

mit Verbindungen der Formel (IV) in Gegenwart eines Dehydratisierungsreagenzes umgesetzt werden.

Die Umsetzung erfolgt wie für Verfahren [C] beschrieben.

Die Verbindungen der Formel (XV) sind bekannt oder können hergestellt werden, indem

[I] Verbindungen der Formel

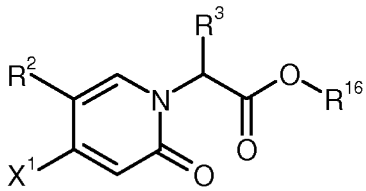 (XVIa),

in welcher

$R^2$ und $R^3$ die oben angegebene Bedeutung haben,
$R^{16}$ für tert-Butyl steht, und
$X^1$ für Chlor, Brom oder Iod steht,

mit einer Säure umgesetzt werden,

oder

[J] Verbindungen der Formel

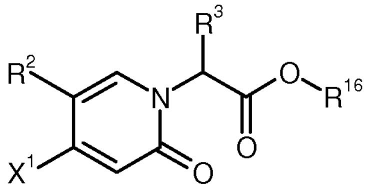

(XVIb),

in welcher

$R^2$ und $R^3$ die oben angegebene Bedeutung haben,
$R^{16}$ für Methyl, Ethyl oder Benzyl steht, und
$X^1$ für Chlor, Brom oder Iod steht,

mit einer Base umgesetzt werden.

Die Verbindungen der Formeln (XVIa) und (XVIb) bilden zusammen die Menge der Verbindungen der Formel (XVI).
Die Umsetzung nach Verfahren [I] erfolgt wie für Verfahren [A] beschrieben.
Die Umsetzung nach Verfahren [J] erfolgt wie für Verfahren [B] beschrieben.
Die Verbindungen der Formel (XVI) sind bekannt oder können hergestellt werden, indem Verbindungen der Formel

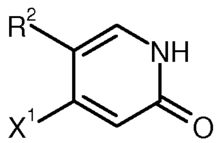

(XVII),

in welcher

$R^2$ die oben angegebene Bedeutung habt, und
$X^1$ für Chlor, Brom oder Iod steht,

mit Verbindungen der Formel

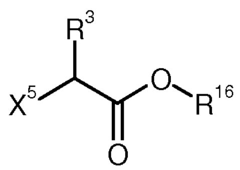

(XVIII),

in welcher

$R^3$ die oben angegebene Bedeutung hat,
$R^{16}$ für Methyl, Ethyl, Benzyl oder tert-Butyl steht, und
$X^5$ für Chlor, Brom, Iod, Methansulfonyloxy oder Trifluormethansulfonyloxy steht,

umgesetzt werden.
Die Umsetzung erfolgt wie für Verfahren [G] beschrieben.
Die Verbindungen der Formel (XVII) und (XVIII) sind bekannt oder lassen sich nach bekannten Verfahren aus den entsprechenden Ausgangsverbindungen synthetisieren.
In einem alternativen Verfahren können die Verbindungen der Formel (VIII) hergestellt werden, indem Verbin-

dungen der Formel

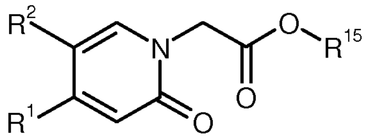 (XIX),

in welcher

$R^1$ und $R^2$ die oben angegebene Bedeutung haben, und
$R^{15}$ für Methyl, Ethyl, Benzyl oder tert-Butyl steht,

mit Verbindungen der Formel

$R^3$-$X^6$ (XX),

in welcher

$R^3$ die oben angegebene Bedeutung hat, und
$X^6$ für Chlor, Brom, Iod, Methansulfonyloxy, Trifluormethansulfonyloxy oder para-Toluolsulfonyloxy steht,

umgesetzt werden.

Die Umsetzung erfolgt im Allgemeinen in inerten Lösungsmitteln, gegebenenfalls in Gegenwart einer Base, bevorzugt in einem Temperaturbereich von -78°C bis Raumtemperatur bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist Tetrahydrofuran.

Basen sind beispielsweise Kalium- oder Natrium-tert-butylat, Natriumhydrid, N-Butyllithium oder Bis-(trimethylsilyl)-lithiumamid, bevorzugt ist Bis-(trimethylsilyl)-lithiumamid.

Die Verbindungen der Formel (XIX) sind bekannt oder lassen sich nach den oben beschriebenen Verfahren, z.B. Verfahren [G], aus den entsprechenden Ausgangsverbindungen synthetisieren.

Die Verbindungen der Formel (XX) sind bekannt oder lassen sich nach bekannten Verfahren aus den entsprechenden Ausgangsverbindungen synthetisieren.

In einem alternativen Verfahren können die Verbindungen der Formel (III) hergestellt werden, indem Verbindungen der Formel

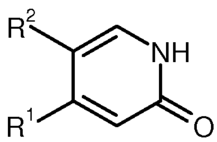 (IX),

in welcher
$R^1$ und $R^2$ die oben angegebene Bedeutung haben,
mit Verbindungen der Formel

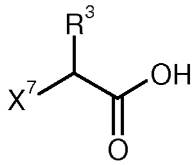 (XXI),

in welcher

$R^3$ die oben angegebene Bedeutung hat, und

$X^7$ für Chlor, Brom oder Iod steht,

umgesetzt werden.

Die Umsetzung erfolgt im Allgemeinen in inerten Lösungsmitteln, gegebenenfalls in Gegenwart einer Base, bevorzugt in einem Temperaturbereich von -10°C bis 90°C bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist Tetrahydrofuran.

Basen sind beispielsweise Kalium- oder Natrium-tert-butylat, Natriumhydrid oder Bis-(trimethylsilyl)-lithiumamid oder ein Gemisch aus Magnesiumdi-tert-butylat und Kalium-tert-butylat, bevorzugt ist ein Gemisch aus Magnesiumdi-tert-butylat und Kalium-tert-butylat.

Die Verbindungen der Formel (XXI) sind bekannt oder lassen sich nach bekannten Verfahren aus den entsprechenden Ausgangsverbindungen synthetisieren.

In einem alternativen Verfahren können die Verbindungen der Formel (XV) hergestellt werden, indem Verbindungen der Formel

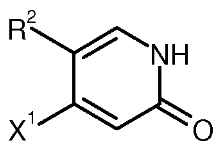 (XVII),

in welcher

$R^2$ die oben angegebene Bedeutung habt, und
$X^1$ für Chlor, Brom oder Iod steht,

mit Verbindungen der Formel

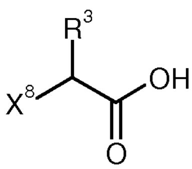 (XXII),

in welcher

$R^3$ die oben angegebene Bedeutung hat, und
$X^8$ für Chlor, Brom oder Iod steht,

umgesetzt werden.

Die Umsetzung erfolgt wie für die Umsetzung von Verbindungen der Formel (IX) mit Verbindungen der Formel (XXI) beschrieben.

Die Verbindungen der Formel (XXII) sind bekannt oder lassen sich nach bekannten Verfahren aus den entsprechenden Ausgangsverbindungen synthetisieren.

Die Herstellung der Ausgangsverbindungen und der Verbindungen der Formel (I) kann durch das folgende Syntheseschema verdeutlicht werden.

**Schema 1:**

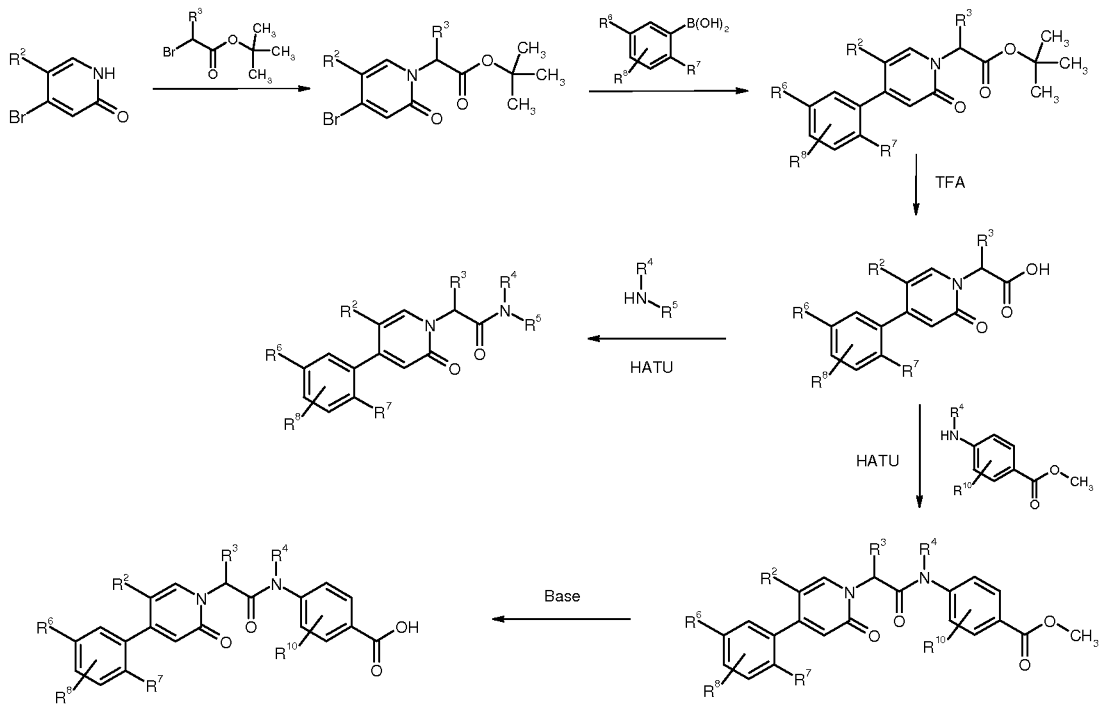


Die erfindungsgemäßen Verbindungen zeigen ein nicht vorhersehbares, wertvolles pharmakologisches und pharmakokinetisches Wirkspektrum. Es handelt sich dabei um Verbindungen, die die proteolytische Aktivität der Serinprotease FXIa beeinflussen. Die erfindungsgemäßen Verbindungen hemmen die enzymatische Spaltung von Substraten, die eine wesentliche Rolle bei der Aktivierung der Blutgerinnungskaskade und der Aggregation von Blutplättchen einnehmen. Weiterhin inhibieren einige der Verbindungen auch Plasma-Kallikrein.

Sie eigenen sich daher zur Verwendung als Arzneimittel zur Behandlung und/oder Prophylaxe von Krankheiten bei Menschen und Tieren.

Weiterer Gegenstand der vorliegenden Erfindung ist der Einsatz der erfindungsgemäßen Verbindungen zur Behandlung und/oder Prophylaxe von Erkrankungen, insbesondere von Herz-Kreislauf-Erkrankungen, vorzugsweise von thrombotischen beziehungsweise thromboembolischen Erkrankungen und/oder thrombotischen beziehungsweise thromboembolischen Komplikationen, und/oder ophthalmologischen Erkrankungen, insbesondere von diabetischer Retinopathie beziehungsweise des Makulaödems, und/oder entzündlichen Erkrankungen, insbesondere diejenigen, die mit übermäßiger Plasma-Kallikrein-Aktivität in Zusammenhang stehen.

Zu den "thromboembolischen Erkrankungen" im Sinne der vorliegenden Erfindung zählen insbesondere Erkrankungen wie das akute Koronarsyndrom (ACS), Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie, Stentimplantation oder aortokoronarem Bypass, periphere arterielle Verschlusskrankheiten, Lungenembolien, venöse Thrombosen, insbesondere in tiefen Beinvenen und Nierenvenen, transitorische ischämische Attacken sowie thrombotischer und thromboembolischer Hirnschlag.

Die erfindungsgemäßen Verbindungen eignen sich daher auch zur Prävention und Behandlung von kardiogenen Thromboembolien, wie beispielsweise Hirn-Ischämien, Schlaganfall und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, ferner bei Patienten mit Herzklappen-Erkrankungen oder mit künstlichen Herzklappen.

Darüber hinaus sind die erfindungsgemäßen Verbindungen zur Behandlung und Prävention einer disseminierten intravasalen Gerinnung (DIC) geeignet, die unter anderem im Rahmen einer Sepsis, aber auch infolge von Operationen, Tumorerkrankungen, Verbrennungen oder anderen Verletzungen auftreten und durch Mikrothrombosierungen zu schweren Organschäden führen kann.

Thromboembolische Komplikationen treten ferner auf bei mikroangiopathischen hämolytischen Anämien und durch Kontakt des Blutes mit körperfremden Oberflächen im Rahmen von extrakorporalen Blutkreisläufen, wie zum Beispiel Hämodialyse, ECMO ("extracorporal membrane oxygenation"), LVAD ("left ventricular assist device") und ähnlichen Verfahren, AV-Fisteln, Gefäß- sowie Herzklappenprothesen.

Außerdem kommen die erfindungsgemäßen Verbindungen auch zur Beeinflussung der Wundheilung, für die Prophylaxe und/oder Behandlung von atherosklerotischen Gefäßerkrankungen und entzündlichen Erkrankungen wie

rheumatische Erkrankungen des Bewegungsapparats, koronaren Herzkrankheiten, von Herzinsuffizienz, von Bluthochdruck, von entzündlichen Erkrankungen, wie z.B. Asthma, entzündlichen Lungenerkrankungen, Glomerulonephritis und entzündlichen Darmerkrankungen, wie zum Beispiel Morbus Crohn oder Colitis ulcerosa, in Betracht, darüber hinaus ebenso für die Prophylaxe und/oder Behandlung der von Demenzerkrankungen, wie z. B. der Alzheimer'schen Erkrankung. Außerdem können die erfindungsgemäßen Verbindungen zur Inhibition des Tumorwachstums und der Metastasenbildung, bei Mikroangiopathien, altersbedingter Makula-Degeneration, diabetischer Retinopathie, diabetischer Nephropathie und anderen mikrovaskulären Erkrankungen sowie zur Prävention und Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Die erfindungsgemäßen Verbindugen eignen sich ebenfalls zur Beeinflussung von Erkrankungen die eine starke Gefäßpermeabilität und Inflammation hervorrufen, wie z.B. das Hereditäre Angioödem (HAE), bei dem eine Dysregulation der Gefäßpermeabilität, ausgelöst durch eine übermäßige Plasma-Kallikrein-Aktivierung, grundlegend ist.

Ferner eignen sich die erfindungsgemäßen Verbindungen insbesondere mit Plasma-Kallikrein Wirkung zur Anwendung bei Lungentransplantationen, orthotopischen Lebertransplantationen, Komplikationen im Rahmen von CABG (coronary artery bypass graft) Operationen. Ferner eignen sich die erfindungsgemäßen Verbindungen zum Schutz von Organen im Rahmen einer Transplantation.

Außerdem kommen die erfindungsgemäßen Verbindungen auch für die Prophylaxe und/oder Behandlung von pulmonaler Hypertonie in Betracht.

Der Begriff "pulmonale Hypertonie" umfasst bestimmte Formen der pulmonalen Hypertonie, wie sie z.B. von der Weltgesundheitsorganisation (WHO) festgelegt worden sind. Als Beispiele seien genannt, die pulmonale arterielle Hypertonie, die pulmonale Hypertonie bei Erkrankungen des linken Herzens, die pulmonale Hypertonie bei Lungenerkrankung und/oder Hypoxie und die Pulmonale Hypertonie aufgrund chronischer Thrombembolien (CTEPH).

Die "pulmonale arterielle Hypertonie" beinhaltet die Idiopathische Pulmonale Arterielle Hypertonie (IPAH, früher auch als primäre pulmonale Hypertonie bezeichnet), die Familiär bedingte Pulmonale Arterielle Hypertonie (FPAH) und die Assoziierte Pulmonal-Arterielle Hypertonie (APAH), die assoziiert ist mit Kollagenosen, kongenitalen systemisch-pulmonalen Shuntvitien, portaler Hypertension, HIV-Infektionen, der Einnahme bestimmter Drogen und Medikamente, mit anderen Erkrankungen (Schilddrüsenerkrankungen, Glykogenspeicherkrankheiten, Morbus Gaucher, hereditäre Teleangiektasie, Hämoglobinopathien, myeloproliferative Erkrankungen, Splenektomie), mit Erkrankungen mit einer signifikanten venösen/kapillären Beteiligung wie der pulmonal-venookklusiven Erkrankung und der pulmonal-kapillären Hämangiomatose, sowie die persistierende pulmonale Hypertonie der Neugeborenen.

Die pulmonale Hypertonie bei Erkrankungen des linken Herzens beinhaltet die Erkrankung des linken Vorhofes oder Ventrikels und Mitral- oder Aortenklappenfehler.

Die pulmonale Hypertonie bei Lungenerkrankung und/oder Hypoxie beinhaltet chronisch obstruktive Lungenerkrankungen, interstitielle Lungenerkrankung, Schlafapnoe-Syndrom, alveolärer Hypoventilation, chronische Höhenkrankheit und anlagebedingte Fehlbildungen.

Die Pulmonale Hypertonie aufgrund chronischer Thrombembolien (CTEPH) beinhaltet den thrombembolischen Verschluss proximaler Lungenarterien, den thrombembolischen Verschluss distaler Lungenarterien und nicht-thrombotische Lungenembolien (Tumor, Parasiten, Fremdkörper).

Weiterer Gegenstand der vorliegenden Erfindung ist die Verwendung der erfindungsgemäßen Verbindungen zur Herstellung von Arzneimitteln zur Behandlung und/oder Prophylaxe von pulmonaler Hypertonie bei Sarkoidose, Histiozytose X und Lymphangiomatosis.

Außerdem kommen die erfindungsgemäßen Substanzen auch zur Behandlung von pulmonalen und hepatischen Fibrosen in Betracht.

Außerdem kommen die erfindungsgemäßen Verbindungen auch für die Behandlung und/oder Prophylaxe von Sepsis (oder Septikämie), Systemic Inflammatory Syndrome (SIRS), septische Organdysfunktion, septisches Organversagen und Muliorganversagen, Acute Respiratory Distress Syndrome (ARDS), Acute Lung Injury (ALI), Septischer Schock, DIC ("Disseminated Intravascular coagulation", oder "Verbrauchskoagulopathie") und/oder des septischen Organversagens in Betracht.

"Sepsis" wird definiert als das Vorliegen einer Infektion und eines "systemic inflammatory response syndrome" (nachfolgend mit "SIRS" bezeichnet). SIRS tritt im Rahmen von Infekten, aber auch von anderen Zuständen wie Verletzungen, Verbrennungen, Schock, Operationen, Ischämie, Pankreatitis, Reanimation oder Tumoren auf. Nach der Definition des ACCP/SCCM Consensus Conference Committee von 1992 (Crit Care Med 1992;20:864-874) werden die zur Diagnose "SIRS" erforderlichen Symptome zur Diagnose und Meßparameter beschrieben (u.a. veränderte Körpertemperatur, erhöhte Herzfrequenz, Atemschwierigkeiten und verändertes Blutbild). In der späteren (2001) SCCM/ESICM/ACCP/ATS/SIS International Sepsis Definitions Conference wurden die Kriterien im Wesentlichen beibehalten, in Details jedoch verfeinert (Levy et al., Crit Care Med 2003;31:1250-1256).

Im Verlauf einer Sepsis kann es zur generalisierten Aktivierung des Gerinnungssystems kommen ("Disseminated Intravascular coagulation", oder "Verbrauchskoagulopathie", nachfolgend als "DIC" bezeichnet) mit Mikrothrombosierung in verschiedenen Organen und sekundärer Blutungskomplikationen. Außerdem kann es es zur endothelialen Schädigung mit Erhöhung der Gefäßpermeabilität und Austritt von Flüssigkeit und Proteinen in den Extravasalraum kommen. Im weiteren Verlauf kann ein Versagen eines Organs (z.B. Nierenversagen, Leberversagen, Atemversagen, zentralnervöse Defizite und Herz-/Kreislaufversagen) oder zum Multiorganversagen kommen. "Septischer Schock" bezeichnet das Auftreten einer behandlungspflichtigen Blutdruckerniedrigung, die eine weitere Organschädigung begünstigt und mit einer Verschlechterung der Prognose einhergeht.

Krankheitserreger können Bakterien (gram-negativ und gram-positiv), Pilze, Viren und/oder Eukaryonten sein. Eintrittspforte beziehungsweise Primärinfektion können z.B. Pneumonie, Harnwegsinfekt, Peritonitis sein. Die Infektion kann, muß aber nicht zwingend, mit einer Bakteriämie einhergehen.

DIC und/oder SIRS können im Rahmen einer Sepsis, aber auch infolge von Operationen, Tumorerkrankungen, Verbrennungen oder anderen Verletzungen auftreten. Bei der DIC kommt es an der Oberfäche von geschädigten Endothelzellen, Fremdkörperoberflächen oder veretztem extravaskulärem Gewebe zur massiven Aktivierung des Gerinnungssystems. Als Folge kommt es zur Gerinnung in kleinen Gefäßen verschiedener Organe mit Hypoxie und anschließender Organdysfunktion. Sekundär kommt es zum Verbrauch von Gerinnungsfaktoren (z.B. Faktor X, Prothrombin und Fibrinogen) und Plättchen, wodurch die Gerinnungsfähigkeit des Blutes herabgesetzt wird und schwere Blutungen auftreten können.

Die Therapie der Sepsis besteht einerseits in der konsequenten Beseitigung der infektiösen Ursache, z.B. durch operative Herdsanierung und Antibiose. Andererseits besteht sie in der temporären intensivmedizinischen Unterstützung der beeinträchtigten Organsysteme. Therapien der verschiedenen Stadien dieser Erkrankung sind z.B. in folgender Publikation beschrieben (Dellinger et al., Crit Care Med 2004;32:858-873). Für die DIC existieren keine erwiesenermaßen effektive Therapien.

Zu den "ophthalmologischen Erkrankungen" im Sinne der vorliegenden Erfindung zählen insbesondere Erkrankungen wie diabetische Retinopathie, diabetisches Makulaödem (diabetic macular edema, DME), Makulaödem, Makulaödem assoziiert mit retinalem Venenverschluss, altersbedingte Makula-Degeneration (AMD), choroidale Neovaskularisierung (CNV), choroidale neovaskuläre Membranen (CNVM), zystoides Makulaödem (cystoid macula edema, CME), epiretinale Membranen (ERM) und Makula-Perforationen, Myopie-assoziierte choroidale Neovaskularisierung, angioide beziehungsweise vaskuläre Streifen (angioid streaks, vascular streaks), Retina-Ablösung, atrophische Veränderungen des retinalen Pigmentepithels, hypertrophische Veränderungen des retinalen Pigmentepithels, retinaler Venenverschluss, choroidaler retinaler Venenverschluss, Retinitis pigmentosa, Stargardt'sche Erkrankung, Frühgeborenen-Retinopathie, Glaukom, entzündliche Erkrankungen des Auges wie z.B. Uveitis, Skleritis oder Endophthalmitis, Katarakt, Refraktionsanomalien wie z.B. Myopie, Hyperopie oder Astigmatismus und Keratokonus, Erkrankungen des vorderen Auges wie z.B. korneale Angiogenese als Folge von z.B. Keratitis, Hornhaut-Transplantation oder Keratoplastie, korneale Angiogenese als Folge von Hypoxie (z.B. durch zu langes Tragen von Kontaktlinsen), Pterygium conjunctivae, subkorneales Ödem und intrakorneales Ödem.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:

- Antibiotische Therapie
  Verschiedene Antibiotika oder antifungale Medikamenten-Kombinationen kommen in Frage, entweder als kalkulierte Therapie (vor Vorliegen des mikrobiellen Befundes) oder als spezifische Therapie.

- Flüssigkeitstherapie
  z.B. Kristalloide oder kolloidale Flüssigkeiten.

- Vasopressoren
  z.B. Norepinephrine, Dopamine oder Vasopressin

- Inotrope Therapie
  z.B. Dobutamin

- Kortikosteroide
  z.B. Hydrokortison, oder Fludrokortison

- rekombinantes humanes aktivierte Protein C
  Xigris

- Blutprodukte

z.B. Erythrozytenkonzentrate, Thrombozytenkonzentrate, Erythropietin oder Fresh Frozen Plasma

- Maschinelle Beatmung bei sepsis-induziertem Acute Lung Injury (ALI) beziehungsweise Acute Respiratory Distress Syndrome (ARDS)
  z.B. Permissive Hyperkapnie, niedrigere Tidalvolumina

- Sedierung, Analgesierung und neuromuskuläre Blockade
  Sedierung: z.B. Diazepam, Lorazepam, Midazolam oder Propofol. Opioide: z.B. Fentanyl, Hydromorphon, Morphin, Meperidin oder Remifentanil. NSAIDs: z.B. Ketorolac, Ibuprofen oder Acetaminophen. Neuromuskuläre Blockade: z.B. Pancuronium

- Glukose-Kontrolle
  z.B. Insulin, Glukose

- Nierenersatzverfahren
  z.B. kontinuierliche veno-venöse-Hämofiltration oder intermittierende Hämodialyse. Dopamin niedrig-dosiert zur renalen Protektion.

- Antikoagulantien
  z.B. zur Thromboseprophylaxe oder bei Nierenersatzverfahren, z.B. unfraktionierte Heparine, low molecular weight Heparine, Heparinoide, Hirudin, Bivalirudin oder Argatroban.

- Bikarbonat-Therapie

- Streßulkusprophylaxe
  z.B. H2-Rezeptorinhibitoren, Antazida.

Die erfindungsgemäßen Verbindungen können darüber hinaus auch zur Verhinderung von Koagulation *ex vivo* eingesetzt werden, z.B. zur Konservierung von Blut- und Plasmaprodukten, zur Reinigung/Vorbehandlung von Kathetern und anderen medizinischen Hilfsmitteln und Geräten, zur Beschichtung künstlicher Oberflächen von *in vivo* oder *ex vivo* eingesetzten medizinischen Hilfsmitteln und Geräten oder bei biologischen Proben, die Faktor XIa und/oder Plasma-Kallikrein enthalten könnten.

Weiterer Gegenstand der vorliegenden Erfindung sind Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist eine Verbindung zur Behandlung und/oder Prophylaxe von Erkrankungen, insbesondere der zuvor genannten Erkrankungen, unter Verwendung einer therapeutisch wirksamen Menge einer erfindungsgemäßen Verbindung.

Weiterer Gegenstand der vorliegenden Erfindung sind Arzneimittel, enthaltend eine erfindungsgemäße Verbindung und einen oder mehrere weitere Wirkstoffe.

Weiterer Gegenstand der vorliegenden Erfindung ist ein Verfahren zur Verhinderung der Blutkoagulation *in vitro,* insbesondere bei Blutkonserven oder biologischen Proben, die Faktor XIa und/oder Plasma-Kallikrein enthalten könnten, das dadurch gekennzeichnet ist, dass eine antikoagulatorisch wirksame Menge der erfindungsgemäßen Verbindung zugegeben wird.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:

- Lipidsenker, insbesondere HMG-CoA-(3-Hydroxy-3-methylglutaryl-Coenzym A)-Reduktase-Inhibitoren wie beispielsweise Lovastatin (Mevacor), Simvastatin (Zocor), Pravastatin (Pravachol), Fluvastatin (Lescol) und Atorvastatin (Lipitor);

- Koronartherapeutika/Vasodilatatoren, insbesondere ACE-(Angiotensin-Converting-Enzyme)-Inhibitoren wie beispielsweise Captopril, Lisinopril, Enalapril, Ramipril, Cilazapril, Benazepril, Fosinopril, Quinapril und Perindopril, oder AII-(Angiotensin II)-Rezeptor-Antagonisten wie beispielsweise Embusartan, Losartan, Valsartan, Irbesartan, Candesartan, Eprosartan und Temisarta, oder β-Adrenozeptor-Antagonisten wie beispielsweise Carvedilol, Alprenolol, Bisoprolol, Acebutolol, Atenolol, Betaxolol, Carteolol, Metoprolol, Nadolol, Penbutolol, Pindolol, Propanolol und Timolol, oder alpha-1-Adrenozeptor-Antagonisten wie beispielsweise Prazosin, Bunazosin, Doxazosin und Terazosin, oder Diuretika wie beispielsweise Hydrochlorothiazid, Furosemid, Bumetanid, Piretanid, Torasemid, Amilorid und Dihydralazin, oder Calciumkanal-Blocker wie beispielsweise Verapamil und Diltiazem, oder Dihydropyridin-Derivate wie beispielsweise Nifedipin (Adalat) und Nitrendipin (Bayotensin), oder Nitropräparate wie beispielsweise Isosorbid-5-mononitrat, Isosorbiddinitrat und Glyceroltrinitrat, oder Substanzen, die eine Erhöhung von cyclischem Guanosinmonophosphat (cGMP) bewirken, wie beispielsweise Stimulatoren der löslichen Guanylatcyclase, wie beispielsweise Riociguat;

- Plasminogen-Aktivatoren (Thrombolytika/Fibrinolytika) und die Thrombolyse/Fibrinolyse steigernde Verbindungen wie Inhibitoren des Plasminogen-Aktivator-Inhibitors (PAI-Inhibitoren) oder Inhibitoren des Thrombin-aktivierten Fibrinolyse-Inhibitors (TAFI-Inhibitoren) wie beispielsweise Gewebsplasminogen-Aktivator (t-PA), Streptokinase, Reteplase und Urokinase oder Plasminogen-modulierende Substanzen, die zu verstärkter Plasmin-Bildung führen;

- antikoagulatorisch wirksame Substanzen (Antikoagulantien) wie beispielsweise Heparin (UFH), niedermolekulare Heparine (NMH) wie beispielsweise Tinzaparin, Certoparin, Parnaparin, Nadroparin, Ardeparin, Enoxaparin, Reviparin, Dalteparin, Danaparoid, Semuloparin (AVE 5026), Adomiparin (M118) und EP-42675/ORG42675,

- direkte Thrombin Inhibitoren (DTI) wie beispielsweise Pradaxa (Dabigatran), Atecegatran (AZD-0837), DP-4088 und SSR-182289A,

- direkte Faktor Xa-Inhibitoren wie beispielsweise Rivaroxaban, Apixaban, Edoxaban (DU-176b), Betrixaban (PRT-54021), R-1663, Darexaban (YM-150), Otamixaban (FXV-673/RPR-130673), Letaxaban (TAK-442), Razaxaban (DPC-906), DX-9065a, LY-517717, Tanogitran (BIBT-986, prodrug: BIBT-1011), Idraparinux und Fondaparinux,

- plättchenaggregationshemmende Substanzen (Plättchenaggregationshemmer, Thrombozytenaggregationshemmer) wie beispielsweise Acetylsalicylsäure (wie beispielsweise Aspirin), P2Y12-Antagonisten wie beispielsweise Ticlopidin (Ticlid), Clopidogrel (Plavix), Prasugrel, Ticagrelor, Cangrelor, Elinogrel, PAR-1-Antagonisten wie beispielsweise Vorapaxar, PAR-4 Antagonisten, EP3-Antagonisten wie beispielsweise DG041;

- Plättchenadhäsionshemmern wie GPVI- und/oder GPIb-Antagonisten wie beispielsweise Revacept oder Caplacizumab;

- Fibrinogen-Rezeptor-Antagonisten (Glycoprotein-IIb/IIIa-Antagonisten) wie beispielsweise Abciximab, Eptifibatide, Tirofiban, Lamifiban, Lefradafiban und Fradafiban;

- sowie Antiarrhythmika;

- Inhibitoren der VEGF- und/oder PDGF Signalwege wie beispielsweise Aflibercept, Ranibizumab, Bevacizumab, KH-902, Pegaptanib, Ramucirumab, Squalamin oder Bevasiranib, Apatinib, Axitinib, Brivanib, Cediranib, Dovitinib, Lenvatinib, Linifanib, Motesanib, Pazopanib, Regorafenib, Sorafenib, Sunitinib, Tivozanib, Vandetanib, Vatalanib, Vargatef und E-10030;

- Hemmer der Angiopoietin-Tie Signalwege wie beispielsweise AMG386;

- Inhibitoren der Tie2 Rezeptortyrosinkinase;

- Hemmer der Integrin-Signalwege wie beispielsweise Volociximab, Cilengitid und ALG1001;

- Hemmer der PI3K-Akt-mTor Signalwege wie beispielsweise XL-147, Perifosin, MK2206, Sirolimus, Temsirolimus und Everolimus;

- Corticosteroid wie beispielsweise Anecortave, Betamethason, Dexamethason, Triamcinolon, Fluocinolon und Fluocinolonacetonid;

- Inhibitoren des ALK1-Smad1/5 Signalweges wie beispielsweise ACE041;

- Cyclooxygenaseinhibitoren wie beispielsweise Bromfenac und Nepafenac;

- Hemmer des Kallikrein-Kinin-Systems wie beispielsweise Safotibant und Ecallantid;
- Inhibitoren der Sphingosin-1-phosphat-Signalwege wie beispielsweise Sonepcizumab;
- Inhibitoren des Komplement-C5a Rezeptors wie beispielsweise Eculizumab;
- Inhibitoren des 5HT1a Rezeptors wie beispielsweise Tandospiron;
- Hemmer des Ras-Raf-Mek-Erk Signalwegs; Hemmer der MAPK Signalwege; Hemmer der FGF-Signalwege; Hemmer der Endothelzellproliferation; Apoptose-induzierende Wirkstoffe;
- Photodynamische Therapie, bestehend aus einem Wirkstoff und der Einwirkung von Licht, wobei der Wirkstoff beispielsweise Verteporfin ist.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, extraocular, intraocular, otisch oder als Implantat beziehungsweise Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die extraoculare (topische) Applikation eignen sich nach dem Stand der Technik funktionierende schnell und/oder modifiziert beziehungsweise kontrolliert den Wirkstoff abgebende Applikationsformen, die den Wirkstoff in kristalliner und/oder amorphisierter und/oder gelöster Form enthalten, wie z.B. Augentropfen, Sprays und Lotionen (z.B. Lösungen, Suspensionen, vesikuläre/kolloidale Systeme, Emulsionen, Aerosole), Pulver für Augentropfen, Sprays und Lotionen (z.B. gemahlener Wirkstoff, Mischungen, Lyophilisate, gefällter Wirkstoff), halbfeste Augenzubereitungen (z.B. Hydrogele, in-situ Hydrogele, Cremes und Salben), Augeninserte (feste und halbfeste Zubereitungen, z.B. Bioadhesives, Filme/Wafer, Tabletten, Kontaktlinsen).

Die intraoculare Applikation umfasst z.B. die intravitreale subretinale, subsclerale, intrachoroidale, subconjunctivale, retrobulbare und subtenone Applikation. Für die intraoculare Applikation eignen sich nach dem Stand der Technik funktionierende schnell und/oder modifiziert beziehungsweise kontrolliert den Wirkstoff abgebende Applikationsformen, die den Wirkstoff in kristalliner und/oder amorphisierter und/oder gelöster Form enthalten, wie z.B. Injektionen und Konzentrate für Injektionen (z.B. Lösungen, Suspensionen, vesikuläre/kolloidale Systeme, Emulsionen, Pulver für Injektionen (z.B. gemahlener Wirkstoff, Mischungen, Lyophilisate, gefällter Wirkstoff), Gele für Injektionen (halbfeste Zubereitungen, z.B. Hydrogele, in-situ Hydrogele) und Implantate (feste Zubereitungen, z.B. bioabbaubare und nicht-bioabbaubare Implantate, implantierbare Pumpen).

Bevorzugt ist die orale Applikation beziehungsweise bei ophthalmologischen Erkrankungen die extraokulare und die intraokulare Applikation.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, vorzugsweise zusammen mit einem oder mehreren inerten nichttoxischen, pharmazeutisch geeigneten Hilfsstoff enthalten, sowie deren Verwendung zu den zuvor genannten Zwecken.

Im Allgemeinen hat es sich als vorteilhaft erwiesen, bei parenteraler Applikation Mengen von etwa 5 bis 250 mg je 24 Stunden zur Erzielung wirksamer Ergebnisse zu verabreichen. Bei oraler Applikation beträgt die Menge etwa 5 bis 500 mg je 24 Stunden.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt beziehungsweise Intervall, zu welchem die Applikation erfolgt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10% w/v": 100 ml Lösung oder Suspension enthalten 10 g Substanz.

## A) Beispiele

### Abkürzungen:

| | |
|---|---|
| ca. | circa |
| CDI | Carbonyldiimidazol |
| d | Tag(e), Dublett (bei NMR) |
| DAD | Dioden Array Detektor |
| DC | Dünnschicht-Chromatographie |
| DCM | Dichlormethan |
| DCI | direkte chemische Ionisation (bei MS) |
| dd | Doppeltes Dublett (bei NMR) |
| DIC | *N,N'*-Diisopropylcarbodiimid |
| DIEA | *N,N*-Diisopropylethylamin |
| DMAP | 4-Dimethylaminopyridin |
| DMF | *N,N*-Dimethylformamid |
| DMSO | Dimethylsulfoxid |
| d. Th. | der Theorie (bei Ausbeute) |
| eq. | Äquivalent(e) |
| ESI | Elektrospray-Ionisation (bei MS) |
| h | Stunde(n) |
| HATU | *O*-(7-Azabenzotriazol-1-yl)-*N,N,N',N'*-tetramethyluronium-Hexafluorphosphat |
| HPLC | Hochdruck-, Hochleistungsflüssigchromatographie |
| HV | Hochvakuum |
| LC-MS | Flüssigchromatographie-gekoppelte Massenspektroskopie |
| LDA | Lithiumdiisopropylamid |
| m | Multiplett (bei NMR) |
| min | Minute(n) |
| MS | Massenspektroskopie |
| NMR | Kernresonanzspektroskopie |
| Oxima | Hydroxyiminocyanoessigsaeureethylester |
| quant. | quantitativ |
| RP | reverse phase (bei HPLC) |
| RT | Raumtemperatur |
| $R_t$ | Retentionszeit (bei HPLC) |
| s | Singulett (bei NMR) |
| SFC | superkritische Flüssigkeitschromatographie (mit überkritischem Kohlendioxid als mobile Phase) |
| THF | Tetrahydrofuran |
| TFA | Trifluoressigsäure |
| T3P | 2,4,6-Tripropyl-1,3,5,2,4,6-trioxatriphosphinan-2,4,6-trioxid |

| | |
|---|---|
| Xantphos | 4,5-Bis(diphenylphosphino)-9,9-dimethylxanthen |
| XPhos Prekatalysator | [(2'-Aminobiphenyl-2-yl)(chlor)palladium-Dicyclohexyl(2',4',6'-triisopropylbiphenyl-2-yl)phosphan (1:1)], J. Am. Chem. Soc. 2010, 132, 14073-14075 |

**HPLC-. LC/MS- und GC-Methoden:**

Methode 1: Instrument: Waters ACQUITY SQD UPLC System; Säule: Waters Acquity UPLC HSS T3 1.8 μ 50x1 mm; Eluent A: 1 1 Wasser + 0.25 ml 99%ige Ameisensäure, Eluent B: 1 1 Acetonitril + 0.25 ml 99%ige Ameisensäure; Gradient: 0.0 min 90% A → 1.2 min 5% A → 2.0 min 5% A; Ofen: 50°C; Fluss: 0.40 ml/min; UV-Detektion: 208-400 nm.

Methode 2: Instrument: Waters ACQUITY SQD UPLC System; Säule: Waters Acquity UPLC HSS T3 1.8 μ 50x1 mm; Eluent A: 1 1 Wasser + 0.25 ml 99%ige Ameisensäure, Eluent B: 1 1 Acetonitril + 0.25 ml 99%ige Ameisensäure; Gradient: 0.0 min 95% A → 6.0 min 5% A → 7.5 min 5% A; Ofen: 50°C; Fluss: 0.35 ml/min; UV-Detektion: 210-400 nm.

Methode 3: Instrument: Micromass Quattro Premier mit Waters UPLC Acquity; Säule: Thermo Hypersil GOLD 1.9 μ 50x1 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 97% A → 0.5 min 97% A → 3.2 min 5% A → 4.0 min 5% A; Ofen: 50°C; Fluss: 0.3 ml/min; UV-Detektion: 210 nm.

Methode 4: Instrument MS: Waters (Micromass) Quattro Micro; Instrument HPLC: Agilent 1100 Serie; Säule: YMC-Triart C18 3μ 50x3 mm; Eluent A: 1 1 Wasser + 0.01 mol Ammoniumcarbonat, Eluent B: 11 Acetonitril; Gradient: 0.0 min 100% A → 2.75 min 5% A → 4.5 min 5% A; Ofen: 40°C; Fluss: 1.25 ml/min; UV-Detektion: 210 nm.

Methode 5: Instrument MS: Waters (Micromass) QM; Instrument HPLC: Agilent 1100 Serie; Säule: Agient ZORBAX Extend-C18 3.0x50 mm 3.5-Micron; Eluent A: 1 1 Wasser + 0.01 mol Ammoniumcarbonat, Eluent B: 1 1 Acetonitril; Gradient: 0.0 min 98% A → 0.2 min 98% A → 3.0 min 5% A → 4.5 min 5% A; Ofen: 40°C; Fluss: 1.75 ml/min; UV-Detektion: 210 nm.

Methode 6: Instrument MS: Waters (Micromass) ZQ; Instrument HPLC: Agilent 1100 Serie; Säule: Agient ZORBAX Extend-C18 3.0x50 mm 3.5-Micron; Eluent A: 1 1 Wasser + 0.01 mol Ammoniumcarbonat, Eluent B: 1 1 Acetonitril; Gradient: 0.0 min 98% A → 0.2 min 98% A → 3.0 min 5% A→ 4.5 min 5% A; Ofen: 40°C; Fluss: 1.75 ml/min; UV-Detektion: 210 nm.

Methode 7: Instrument: Thermo DFS, Trace GC Ultra; Säule: Restek RTX-35, 15 m x 200 μm x 0.33 μm; konstanter Fluss mit Helium: 1.20 ml/min; Ofen: 60°C; Inlet: 220°C; Gradient: 60°C, 30°C/min → 300°C (3.33 min halten).

Methode 8: Instrument: Agilent MS Quad 6150; HPLC: Agilent 1290; Säule: Waters Acquity UPLC HSS T3 1.8 μ 50 mm x 2.1 mm; Eluent A: 1 1 Wasser + 0.25 ml 99%ige Ameisensäure, Eluent B: 1 1 Acetonitril + 0.25 ml 99%ige Ameisensäure; Gradient: 0.0 min 90% A → 0.3 min 90% A → 1.7 min 5% A → 3.0 min 5% A; Ofen: 50°C; Fluss: 1.20 ml/min; UV-Detektion: 205-305 nm.

Methode 9: Instrument: Thermo Scientific DSQII, Thermo Scientific Trace GC Ultra; Säule: Restek RTX-35MS, 15 m x 200 μm x 0.33 μm; konstanter Fluss mit Helium: 1.20 ml/min; Ofen: 60°C; Inlet: 220°C; Gradient: 60°C, 30°C/min → 300°C (3.33 min halten).

Methode 10: Instrument MS: Waters SQD; Instrument HPLC: Waters UPLC; Säule: Zorbax SB-Aq (Agilent), 50 mm x 2.1 mm, 1.8 μm; Eluent A: Wasser + 0.025% Ameisensäure, Eluent B: Acetonitril (ULC) + 0.025% Ameisensaeure; Gradient: 0.0 min 98% A - 0.9 min 25% A - 1.0 min 5% A - 1.4 min 5% A - 1.41 min 98% A - 1.5 min 98% A; Ofen: 40°C; Fluss: 0.600 ml/min; UV-Detektion: DAD, 210 nm.

Methode 11: Gerätetyp MS: Waters Synapt G2S; Gerätetyp UPLC: Waters Acquity I-CLASS; Säule: Waters, HSST3, 2.1 mm x 50 mm, C18 1.8 μm; Eluent A: 1 1 Wasser + 0.01% Ameisensäure; Eluent B: 1 1 Acetonitril + 0.01% Ameisensäure; Gradient: 0.0 min 10% B → 0.3 min 10% B → 1.7 min 95% B → 2.5 min 95% B; Ofen: 50°C; Fluss: 1.20 ml/min; UV-Detektion: 210 nm.

Mikrowelle: Als Mikrowellenreaktor wurde ein "single mode" Gerät vom Typ Emrys™ Optimizer verwendet.

## Ausgangsverbindungen

### Allgemeine Methode 1A: Herstellung einer Boronsäure

Eine Lösung des entsprechenden Pyridinderivates in THF (3 ml/mmol) wurde bei -78°C mit LDA (2 molar in THF/Heptan/Ethylbenzol) versetzt, 2-4 h gerührt und anschließend zügig mit Triisopropylborat versetzt. Das Reaktionsgemisch wurde für weitere 2-3 h bei -78°C gehalten und anschließend langsam über Nacht auf RT aufgetaut. Nach Zugabe von Wasser wurde das THF im Vakuum entfernt und die wässrige Phase zweimal mit Ethylacetat extrahiert. Die wässrige Phase wurde mit 2M Salzsäure angesäuert, wobei in der Regel ein Niederschlag ausfiel, der filtriert, mit Wasser gewaschen und getrocknet wurde. Die wässrige Phase wurde dreimal mit Ethylacetat extrahiert. Die vereinigten, organischen Phasen wurden getrocknet (Natriumsulfat), filtriert und im Vakuum eingeengt.

### Allgemeine Methode 2A: Suzuki-Kupplung

In einem ausgeheizten, mit Argon gespülten Kolben wurden 1.0 eq. der entsprechenden Boronsäuren, 1.0 eq. des Arylbromides oder Aryliodides, 3.0 eq. Kaliumcarbonat und 0.1 eq. [1,1-Bis-(diphenylphosphino)-ferrocen]-palladium(II)chlorid-Monodichlormethan-addukt oder Tetrakis(triphenylphosphin)palladium(0) vorgelegt. Der Kolben wurde anschließend dreimal evakuiert und immer wieder mit Argon belüftet. Das Reaktionsgemisch wurde mit Dioxan (6 ml/mmol) versetzt und für mehrere Stunden bis zur weitgehend vollständigen Umsetzung bei 110°C gerührt. Das Reaktionsgemisch wurde anschließend über Celite filtriert, das Filtrat im Vakuum eingeengt. Der Rückstand wurde mit Wasser versetzt. Nach Zugabe von Ethylacetat und Phasentrennung wurde die organische Phase einmal mit Wasser und einmal mit gesättigter, wässriger Natriumchlorid-Lösung gewaschen, getrocknet (Magnesiumsulfat), filtriert und im Vakuum eingeengt. Das Rohprodukt wurde anschließend entweder mittels Flash-Chromatographie (Kieselgel-60, Eluent: Cyclohexan-Ethylacetat-Gemische oder Dichlormethan-Methanol-Gemische) oder präparativer HPLC (Reprosil C18, Wasser-Acetonitril-Gradient oder Wasser-Methanol-Gradient) aufgereinigt.

### Allgemeine Methode 2B: Suzuki-Kupplung

In einem ausgeheizten, mit Argon gespülten Kolben wurden 1.0 eq. der entsprechenden Boronsäure, 1.0 eq. des Arylbromides oder Aryliodides und 0.05 eq. XPhos Prekatalysator [(2'-Aminobiphenyl-2-yl)(chlor)palladium-Dicyclohexyl(2',4',6'-triisopropylbiphenyl-2-yl)phosphan (1:1)], J. Am. Chem. Soc. 2010, 132, 14073-14075] vorgelegt. Der Kolben wurde anschließend dreimal evakuiert und immer wieder mit Argon belüftet. Das Reaktionsgemisch wurde mit im Ultraschallbad entgastem THF (ca. 12 ml/mmol) und 3.0 eq. wässriger Kaliumphosphat-Lösung (0.5 molar) versetzt und bei 60°C gerührt. Anschließend wurde das Reaktionsgemisch mit Wasser und Ethylacetat versetzt. Nach Phasentrennung wurde die wässrige Phase einmal mit Ethylacetat extrahiert. Die vereinigten, organischen Phasen wurden getrocknet (Natriumsulfat), filtriert und im Vakuum eingeengt. Das Rohprodukt wurde anschließend entweder mittels Flash-Chromatographie (Kieselgel-60, Eluent: Cyclohexan-Ethylacetat-Gemische oder Dichlormethan-Methanol-Gemische) oder präparativer HPLC (Reprosil C18, Wasser-Acetonitril-Gradient oder Wasser-Methanol-Gradient) aufgereinigt.

### Allgemeine Methode 3A: Methoxypyridin Spaltung

Eine Lösung des entsprechenden Methoxypyridins in DMF (12.5 ml/mmol) wurde mit 20 eq. Pyridinium-Hydrochlorid oder Pyridinium-Hydrobromid versetzt und bei 100°C für mehrere Stunden bis Tage gerührt, eventuell mit weiterem Pyridinium-Hydrochlorid oder Pyridinium-Hydrobromid versetzt, bis zur weitgehend vollständigen Umsetzung. Anschließend wurde die Reaktionslösung im Vakuum eingeengt und der Rückstand mit Wasser verrührt. Der anfallende Niederschlag wurde filtriert, mit Wasser gewaschen und im Vakuum getrocknet.

### Allgemeine Methode 4A: *N*-Alkylierung von 2-Pyridinon-Derivaten mit den entsprechenden 2-Brom- oder 2-Chlorpropansäurederivaten

Eine Suspension von 1.0 eq. des entsprechenden 2-Pyridinon-Derivates, 2.0 eq. Magnesiumdi-*tert*.-butylat und 1.05 eq. Kalium-*tert*.-butylat in THF (5-10 ml/mmol) wurde unter Argon 10-20 min bei RT gerührt. Das Reaktionsgemisch wurde im Eisbad gekühlt und mit 1.5 eq. des entsprechenden 2-Brom- oder 2-Chlorpropansäurederivates versetzt. Anschließend wurde das Reaktionsgemisch zunächst 2.5 h bei RT und anschließend über Nacht bei 35-90°C nachgerührt und mit 6 N Salzsäure gequencht. Nach Zugabe von Ethylacetat und Phasentrennung wurde die organische Phase einmal mit Wasser und einmal mit gesättigter, wässriger Natriumchlorid-Lösung gewaschen, getrocknet (Magnesiumsulfat), filtriert und im Vakuum eingeengt. Das Rohprodukt wurde anschließend entweder mittels Flash-Chromatographie (Kieselgel-60, Eluent: Cyclohexan-Ethylacetat-Gemische oder Dichlormethan-Methanol-Gemische) oder präparativer HPLC (Reprosil C18, Wasser-Acetonitril-Gradient oder Wasser-Methanol-Gradient) aufgereinigt.

**Allgemeine Methode 4B: N-Alkylierung von 2-Pyridinon-Derivaten mit den entsprechenden 2-Brom- oder 2-Chlorpropansäureesterderivaten in Gegenwart von Kaliumcarbonat**

Eine Lösung von 1.0 eq. des entsprechenden 2-Pyridinon-Derivates in Dimethylformamid (5-10 ml/mmol) wurde unter Argon bei RT mit 1.2 eq. des entsprechenden 2-Brom- oder 2-Chlorpropansäureesterderivates und 1.5 eq. Kaliumcarbonat versetzt und bei 100°C gerührt. Nach Entfernen des DMF und Zugabe von Wasser/Ethylacetat und Phasentrennung wurde die organische Phase mit Wasser und mit gesättigter, wässriger Natriumchlorid-Lösung gewaschen, getrocknet (Natriumsulfat), filtriert und im Vakuum eingeengt. Das Rohprodukt wurde anschließend entweder mittels Flash-Chromatographie (Kieselgel-60, Eluent: Cyclohexan-Ethylacetat-Gemische oder Dichlormethan-Methanol-Gemische) oder präparativer HPLC (Reprosil C18, Wasser-Acetonitril-Gradient oder Wasser-Methanol-Gradient) aufgereinigt.

**Allgemeine Methode 4C: N-Alkylierung von 2-Pyridinon-Derivaten mit den entsprechenden 2-Brom- oder 2-Chlorpropansäureesterderivaten in Gegenwart von Natriumhydrid/ Lithiumbromid**

Eine Lösung von 1.0 eq. des entsprechenden 2-Pyridinon-Derivates in Dimethylformamid (5-10 ml/mmol) wurde unter Argon bei 0°C mit 1.25 eq. Natriumhydrid (60%ig in Mineralöl) versetzt und 10-20 min bei 0°C gerührt. Das Reaktionsgemisch wurde anschließend mit 2.0 eq. Lithiumbromid versetzt, 15 min bei RT gerührt, mit 1.25 eq. des entsprechenden 2-Brom- oder 2-Chlorpropansäureesterderivates versetzt und bei 65°C gerührt. Nach Entfernen des DMF und Zugabe von Wasser/Ethylacetat und Phasentrennung wurde die organische Phase mit Wasser gewaschen, getrocknet (Natriumsulfat), filtriert und im Vakuum eingeengt. Das Rohprodukt wurde anschließend entweder mittels Flash-Chromatographie (Kieselgel-60, Eluent: Cyclohexan-Ethylacetat-Gemische oder Dichlormethan-Methanol-Gemische) oder präparativer HPLC (Reprosil C18, Wasser-Acetonitril-Gradient oder Wasser-Methanol-Gradient) aufgereinigt.

**Allgemeine Methode 4D: *N*-Alkylierung von 2-Pyridinon-Derivaten mit den entsprechenden 2-Brom- oder 2-Chlorpropansäureester-Derivaten in Gegenwart von Natriumhydrid**

Eine Suspension von Natriumhydrid (1.2 eq.) in Dimethylformamid (5-10 ml/mmol) wurde unter Argon bei RT mit dem entsprechenden 2-Pyridinon-Derivat versetzt. Das Reaktionsgemisch wurde 30-90 min bei RT gerührt, auf 0°C gekühlt, mit dem entsprechenden 2-Brom- oder 2-Chlorpropansäureesterderivat (1.2 eq.) versetzt und 2-5 h bei RT gerührt. Nach Zugabe von Wasser und Phasentrennung wurde die wässrige Phase mit Essigsäureethylester extrahiert. Die vereinigten, organischen Phasen wurden getrocknet (Natriumsulfat oder Magnesiumsulfat), filtriert und im Vakuum eingeengt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

**Allgemeine Methode 4E: N-Alkylierung von 2-Pyridinon-Derivaten mit den entsprechenden Triflaten in Gegenwart von Natriumhydrid**

Eine Lösung des entsprechenden 2-Pyridinon-Derivates (1 eq.) in Tetrahydrofuran (0.05-0.2M) wurde unter Argon bei RT mit Natriumhydrid (1.1-1.5 eq.) versetzt und 30-90 min gerührt. Anschließend wurde das entsprechende Triflat (1.0-2.0 eq.) als Reinsubstanz oder Lösung in THF hinzugegeben. Die resultierende Reaktionsmischung wurde 1-5 h bei RT nachgerührt. Das Reaktionsgemisch wurde mit gesättigter, wässriger Ammoniumchlorid-Lösung versetzt. Nach Phasentrennung wurde die wässrige Phase mit Essigsäureethylester extrahiert. Die vereinigten, organischen Phasen wurden getrocknet (Natriumsulfat oder Magnesiumsulfat), filtriert und unter reduziertem Druck eingeengt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

**Allgemeine Methode 5A: Amid-Kupplung mit HATU/DIEA**

Eine Lösung der entsprechenden Carbonsäure (1.0 eq.) in Dimethylformamid (7-15 ml/mmol) wurde unter Argon bei RT mit dem Amin (l.leq.), mit *N,N*-Diisopropylethylamin (2.2 eq.) und einer Lösung von HATU (1.2 eq.) in etwas DMF versetzt. Das Reaktionsgemisch wurde bei RT gerührt. Nach Zugabe von Wasser/Ethylacetat und Phasentrennung wurde die organische Phase mit Wasser und mit gesättigter, wässriger Natriumchlorid-Lösung gewaschen, getrocknet (Natriumsulfat), filtriert und im Vakuum eingeengt. Das Rohprodukt wurde anschließend entweder mittels Flash-Chromatographie (Kieselgel-60, Eluent: Cyclohexan-Ethylacetat-Gemische oder Dichlormethan-Methanol-Gemische) oder präparativer HPLC (Reprosil C18, Wasser-Acetonitril-Gradient oder Wasser-Methanol-Gradient) aufgereinigt.

**Allgemeine Methode 5B: Amid-Kupplung mit OXIMA/DIC**

Zu einer entgasten Lösung der entsprechenden Carbonsäure (1 eq.), Anilin (1 eq.) und Hydroxyiminocyanoessigsäureethylester (Oxima) (1 eq.) in Dimethylformamid (0.1M) wurde tropfenweise *N,N'*-Diisopropylcarbodiimid (DIC) (1 eq.) gegeben und die resultierende Reaktionslösung für 8-24 h bei RT-40°C gerührt. Das Lösungsmittel wurde unter reduziertem Druck entfernt. Der Rückstand wurde entweder mit Wasser versetzt und das gewünschte Produkt filtriert oder mittels Normalphasen-Chromatographie (Cyclohexan/Essigsäureethylester-Gradient) oder präparativer RP-HPLC (Wasser-Acetonitril-Gradient oder Wasser-Methanol-Gradient) aufgereinigt.

**Allgemeine Methode 5C: Amid-Kupplung mit T3P/Pyridin**

Eine Lösung der Carbonsäure (1 eq.) und des entsprechenden Amins (1.5 eq.) in Pyridin (0.15-0.05M) wurde unter Argon bei 0°C tropfenweise mit Propylphosphonsäureanhydrid (T3P, 50%ig in Essigsäureethylester, 4 eq.) versetzt. Dieses Gemisch wurde auf 90°C erhitzt und 1-20 h bei 90°C gerührt. Das Reaktionsgemisch wurde auf RT gekühlt und mit Wasser und Essigsäureethylester versetzt. Nach Phasentrennung wurde die wässrige Phase mit Essigsäureethylester extrahiert. Die vereinigten, organischen Phasen wurden mit wässriger PufferLösung (pH 5), mit gesättigter, wässriger Natriumhydrogencarbonat-Lösung und mit gesättigter, wässriger Natriumchlorid-Lösung gewaschen, getrocknet (Natriumsulfat oder Magnesiumsulfat), filtriert und im Vakuum eingeengt. Das Rohprodukt wurde gegebenenfalls anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

**Allgemeine Methode 6A: Verseifung eines *tert.*-Butylesters mittels TFA**

Eine Lösung von 1.0 eq. des entsprechenden *tert*.-Butylesterderivates in Dichlormethan (ca. 7 ml/mmol) wurde bei RT mit 20 eq. TFA versetzt und bei RT für 1-8 h gerührt. Anschließend wurde das Reaktionsgemisch im Vakuum eingeengt, der Rückstand mehrmals mit Dichlormethan und/oder Toluol coevaporiert und im Vakuum getrocknet. Das Rohprodukt wurde gegebenenfalls anschließend entweder mittels Flash-Chromatographie (Kieselgel-60, Eluent: Cyclohexan-Ethylacetat-Gemische oder Dichlormethan-Methanol-Gemische) oder präparativer HPLC (Reprosil C18, Wasser-Acetonitril-Gradient oder Wasser-Methanol-Gradient) aufgereinigt.

**Allgemeine Methode 6B: Verseifung eines Methyl-/Ethyl- oder *tert.*-Butylesters mit Lithiumhydroxid**

Eine Lösung von 1.0 eq. des entsprechenden Methyl- oder Ethylesters in Tetrahydrofuran/Wasser (3:1, ca. 10 ml/mmol) wurde bei RT mit 3.0 eq. Lithiumhydroxid versetzt. Das Reaktionsgemisch wurde bei RT bis 60°C gerührt und anschließend mit wässriger, 1 N Salzsäure-Lösung auf pH 1 gestellt. Nach Zugabe von Wasser/Ethylacetat und Phasentrennung wurde die wässrige Phase dreimal mit Ethylacetat extrahiert. Die vereinigten, organischen Phasen wurden getrocknet (Natriumsulfat), filtriert und im Vakuum eingeengt. Das Rohprodukt wurde anschließend entweder mittels Flash-Chromatographie (Kieselgel-60, Eluent: Cyclohexan-Ethylacetat-Gemische oder Dichlormethan-Methanol-Gemische) oder präparativer HPLC (Reprosil C18, Wasser-Acetonitril-Gradient oder Wasser-Methanol-Gradient) aufgereinigt.

**Allgemeine Methode 6C: Verseifung eines Methyl- oder Ethylesters mit Cäsiumcarbonat**

Zu einer Lösung des entsprechenden Methyl- oder Ethylesters (1 eq.) in einer Mischung aus Methanol/Wasser (4/1, 0.05-0.2M) wurde Cäsiumcarbonat (2 eq.) gegeben und die resultierende Suspension 3-8 h bei RT-60°C gerührt. Das Reaktionsgemisch wurde gegebenenfalls auf RT abgekühlt und mit wässriger Salzsäure (1N) auf pH 3 eingestellt. Methanol wurde bei 30°C im Vakuum entfernt. Die wässrige Phase wurde mit Essigsäureethylester extrahiert. Die vereinigten, organischen Phasen wurden getrocknet (Natriumsulfat oder Magnesiumsulfat), filtriert und unter reduziertem Druck eingeengt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

**Allgemeine Methode 7A: Alkylierung von Essigsäureestern mit Halogeniden**

Eine Lösung des entsprechenden Essigsäureesters in THF (ca. 10 ml/mmol) wurde unter Argon bei -78°C mit 1.1 eq. Bis-(trimethylsilyl)-lithiumamid (1.0M in THF) versetzt und 10 min bei -78°C gerührt. Anschließend wurde das

Reaktionsgemisch mit einer Lösung des entsprechenden Iodids/Bromids/Chlorids in THF versetzt, 10 min bei -78°C und weiter im Eisbad gerührt und anschließend mit Wasser gequencht. Nach Zugabe von Ethylacetat und Phasentrennung wurde die wässrige Phase zweimal mit Ethylacetat extrahiert. Die vereinigten, organischen Phasen wurden getrocknet (Natriumsulfat), filtriert und im Vakuum eingeengt. Das Rohprodukt wurde anschließend entweder mittels Flash-Chromatographie (Kieselgel-60, Eluent: Cyclohexan-Ethylacetat-Gemische oder Dichlormethan-Methanol-Gemische) oder präparativer HPLC (Reprosil C18, Wasser-Acetonitril-Gradient oder Wasser-Methanol-Gradient) aufgereinigt.

**Allgemeine Methode 7B: Alkylierung von Essigsäureestern mit Triflaten**

Eine Lösung des entsprechenden Essigsäureesters (1 eq.) in Tetrahydrofuran (0.1-0.2M) wurde unter Argon bei -78°C tropfenweise mit Bis-(trimethylsilyl)-lithiumamid (1.0M in THF, 1.1-1.3 eq.) versetzt und 15 min gerührt. Anschließend wurde das entsprechende Alkyltriflat (1.5-2.0 eq.) als Reinsubstanz oder Lösung in THF hinzugegeben. Die resultierende Reaktionsmischung wurde 15 min bei -78°C und 1 h bei RT nachgerührt. Das Reaktionsgemisch wurde mit gesättigter, wässriger Ammoniumchlorid-Lösung versetzt. Nach Phasentrennung wurde die wässrige Phase mit Essigsäureethylester extrahiert. Die vereinigten, organischen Phasen wurden getrocknet (Natriumsulfat oder Magnesiumsulfat), filtriert und unter reduziertem Druck eingeengt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

**Allgemeine Methode 8A: Darstellung von Triflaten**

Eine Lösung des entsprechenden Alkohols (1 eq.) wurde in Dichlormethan (0.1M) vorgelegt und bei -20°C nacheinander mit Lutidin (1.1-1.5 eq.) oder Triethylamin (1.1-1.5 eq.) und mit Trifluormethansulfonsäureanhydrid (1.05-1.5 eq.) versetzt. Das Reaktionsgemisch wurde für 1 h bei -20°C nachgerührt und anschließend mit der dreifachen Menge (bezogen aufs Reaktionsvolumen) Methyl-*tert*.-butylether verdünnt. Die organische Phase wurde dreimal mit einer 3:1-Mischung aus gesättigter, wässriger Natriumchlorid-Lösung/1N Salzsäure und abschließend mit gesättigter, wässriger Natriumhydrogencarbonat-Lösung gewaschen, getrocknet (Natrium- oder Magnesiumsulfat), filtriert und das Lösungsmittel unter reduziertem Druck entfernt. Das Rohprodukt wurde ohne weitere Aufreinigung im nächsten Schritt eingesetzt.

**Allgemeine Methode 9A: Nitro-Reduktion mit Eisen/Ammoniumchlorid**

10 eq. Ammoniumchlorid wurden in einem Ethanol/Wasser-Gemisch (2:1) gelöst (ca. 2M), auf 95°C erhitzt und mit der Nitroaryl-Verbindung (1 eq.) versetzt. 3 eq. Eisenpulver wurden in kleinen Portionen über 1 h dazugegeben. Das Reaktionsgemisch wurde anschließend 30 min bei 95°C gerührt und heiß über Kieselgur filtriert. Der Filterkuchen wurde mit Ethanol gewaschen und das Filtrat im Vakuum von Ethanol befreit. Die verbleibende wässrige Phase wurde dreimal mit Diethylether extrahiert. Die vereinigten, organischen Phasen wurden mit gesättigter, wässriger Natriumchlorid-Lösung gewaschen, getrocknet (Natriumsulfat), filtriert und im Vakuum eingeengt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

**Allgemeine Methode 10A: Darstellung von *tert.*-Butylestern**

Eine Lösung der entsprechenden Carbonsäure (1 eq.) in Toluol (0.15-0.05M) wurde auf 60-100°C erhitzt und tropfenweise mit *N,N*-Dimethylformamid-di-*tert*.-butylacetal (4 eq.) versetzt. Das Reaktionsgemisch wurde 1-5 h bei 60-100°C gerührt, auf RT gekühlt und mit Essigsäureethylester versetzt. Die organische Phase wurde mit gesättigter, wässriger Natriumhydrogencarbonat-Lösung und mit gesättigter, wässriger Natriumchlorid-Lösung gewaschen, getrocknet (Natriumsulfat), filtriert und im Vakuum eingeengt. Das Rohprodukt wurde ohne Reinigung in der nächsten Stufe eingesetzt.

**Beispiel 1.1A**

4-Nitrobenzolcarboximidohydrazid

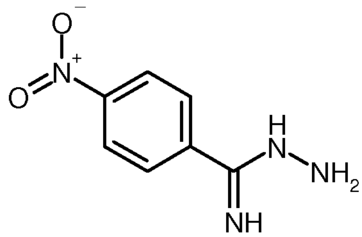

Eine Lösung von 2.0 g (9.92 mmol) 4-Nitrobenzolcarboximidamid-Monohydrochlorid in 20 ml Methanol wurde bei 0°C mit 5.2 ml (29.8 mmol, 3 eq.) *N,N*-Diisopropylethylamin und 0.62 g (80% Reinheit, 9.92 mmol, 1.0 eq.) Hydrazin-Monohydrat versetzt und 64 h bei RT gerührt. Anschließend wurde das Reaktionsgemisch auf 10%ige Natriumchlorid-Lösung gegeben und nach Zugabe von Ethylacetat und Phasentrennung zweimal mit Ethylacetat extrahiert. Die vereinigten, organischen Phasen wurden über Natriumsulfat getrocknet, filtriert und im Vakuum eingeengt. Ausbeute: 1.7 g (93% d. Th.)
LC/MS [Methode 4]: $R_t$ = 1.77 min; MS (ESIpos): m/z = 181 $(M+H)^+$

**Beispiel 1.1B**

5-(4-Nitrophenyl)-3-(trifluormethyl)-1*H*-1,2,4-triazol

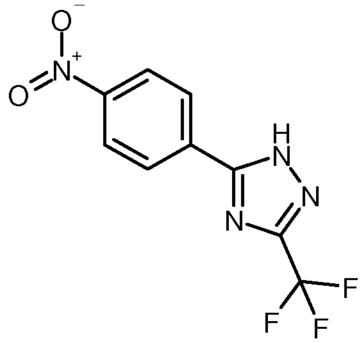

Eine Lösung von 1.7 g (9.3 mmol) 4-Nitrobenzolcarboximidohydrazid in 50 ml Dichlormethan wurde bei 0°C mit 1.95 g (9.3 mmol, 1 eq.) Trifluoressigsäureanhydrid versetzt und bei RT gerührt, wobei nach 20 min 50 ml Acetonitril für eine bessere Löslichkeit des Reaktionsgemisches zugegeben wurden. Das Reaktionsgemisch wurde 3 h bei 50°C gerührt und anschließend im Vakuum eingeengt. Der Rückstand wurde dreimal mit Dichlormethan coevaporiert und im Vakuum getrocknet. Ausbeute: 2.7 g (quant.)
LC/MS [Methode 1]: $R_t$ = 0.94 min; MS (ESIpos): m/z = 259 $(M+H)^+$

**Beispiel 1.1C**

4-[3-(Trifluormethyl)-1*H*-1,2,4-triazol-5-yl]anilin

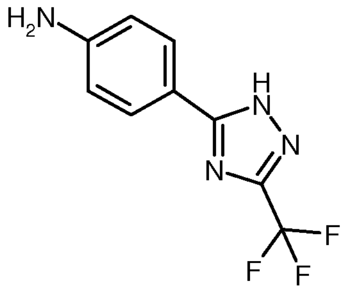

Eine Lösung von 2.7 g (9.9 mmol) 5-(4-Nitrophenyl)-3-(trifluormethyl)-1*H*-1,2,4-triazol in 110 ml Ethanol wurde mit 8.9 g (39.7 mmol, 4 eq.) Zinn(II)chlorid-dihydrat versetzt und 1 h bei 70°C gerührt. Das Reaktionsgemisch wurde auf Eiswasser gegossen und vorsichtig mit Natriumhydrogencarbonat bis zu einem pH-Wert von 8 versetzt. Das Gemisch wurde über eine Filterschicht filtriert und der Rückstand mit Ethylacetat nachgewaschen. Nach Phasentrennung wurde die wässrige Phase zweimal mit Ethylacetat gewaschen. Die vereinigten, organischen Phasen wurden mit wässriger Natriumchlorid-Lösung gewaschen, getrocknet (Magnesiumsulfat), filtriert und im Vakuum eingeengt. Ausbeute: 1.9 g (79% d. Th.)
LC/MS [Methode 4]: $R_t$ = 1.66 min; MS (ESIpos): m/z = 229 $(M+H)^+$

**Beispiel 1.2A**

4-(*H*-Imidazol-2-yl)anilin

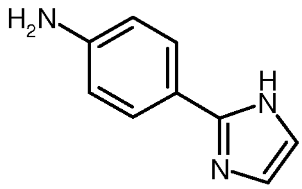

Eine Lösung von 95 mg (0.5 mmol) 2-(4-Nitrophenyl)-1*H*-imidazol in 3 ml Ethanol wurde in Gegenwart von 20 mg Palladium (10%ig auf Aktivkohle) bei RT und Normaldruck hydriert. Anschließend wurde das Reaktionsgemisch über Celite filtriert, das Filtrat im Vakuum eingeengt und getrocknet. Ausbeute: 91 mg (quant.)
LC/MS [Methode 5]: $R_t$ = 1.06 min; MS (ESIpos): m/z = 160 $(M+H)^+$

**Beispiel 1.3A**

5-(4-Nitrophenyl)-2-(trifluormethyl)-1*H*-imidazol

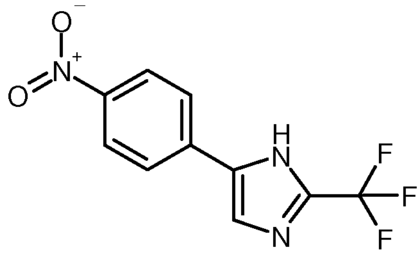

Eine Suspension von 200 mg (0.82 mmol) 2-Brom-1-(4-nitrophenyl)ethanon und 500 mg Natriumsulfat in 10 ml Acetonitril wurde mit 324 mg (85% Reinheit, 2.5 mmol, 3 eq.) 2,2,2-Trifluorethanimidamid versetzt, 1 h im Ultraschallbad behandelt und bei RT nachgerührt. Anschließend wurde das Natriumsulfat abfiltriert und das Filtrat im Vakuum eingeengt. Der Rückstand wurde mittels präparativer HPLC (Reprosil C18, Wasser-Methanol-Gradient) aufgereinigt.

Ausbeute: 104 mg (49% d. Th.)
LC/MS [Methode 1]: $R_t$ = 0.96 min; MS (ESIpos): m/z = 258 $(M+H)^+$

**Beispiel 1.3B**

4-[2-(Trifluormethyl)-1*H*-imidazol-5-yl]anilin

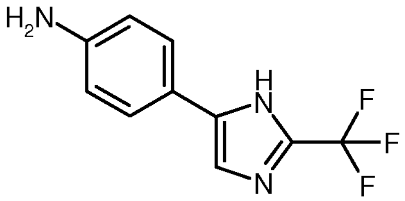

Eine Lösung von 104 mg (0.4 mmol) 5-(4-Nitrophenyl)-2-(trifluormethyl)-1*H*-imidazol in 10 ml Ethanol wurde in Gegenwart von 15 mg Palladium (10%ig auf Aktivkohle) bei RT und Normaldruck hydriert. Anschließend wurde das Reaktionsgemisch über Celite filtriert, das Filtrat im Vakuum eingeengt und getrocknet. Ausbeute: 98 mg (quant.)
LC/MS [Methode 1]: $R_t$ = 0.47 min; MS (ESIpos): m/z = 228 $(M+H)^+$

**Beispiel 1.4A**

5-(4-Nitrophenyl)-3-oxo-2,3-dihydro-1*H*-pyrazol-1-carbonsäure-*tert*.-butylester

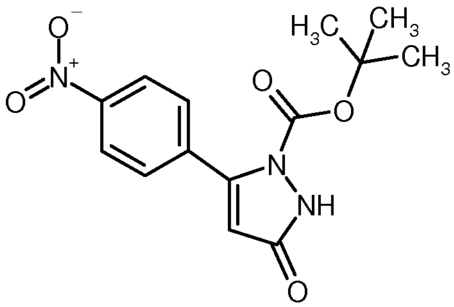

Eine Lösung von 2.5 g (12.2 mmol) 5-(4-Nitrophenyl)-1,2-dihydro-3*H*-pyrazol-3-on in 50 ml Dichlormethan wurde bei RT mit 2.7 g (12.2 mmol, 1.0 eq.) Di-*tert*.-butyldicarbonat und 1.7 ml (12.2 mmol, 1.0 eq.) Triethylamin versetzt und 4 h bei RT gerührt. Das Reaktionsgemisch wurde mit Dichlormethan und Wasser verdünnt. Nach Phasentrennung wurde die organische Phase getrocknet (Natriumsulfat), filtriert und im Vakuum eingeengt. Das Rohprodukt wurde mittels Flash-Chromatographie (Kieselgel-60, Eluent: Dichlormethan-Methanol-Gemische) aufgereinigt. Ausbeute: 2.23 g (58% d. Th.).
LC/MS [Methode 1]: $R_t$ = 1.07 min; MS (ESIpos): m/z = 306 $(M+H)^+$

**Beispiel 1.4B**

5-(4-Aminophenyl)-3-oxo-2,3-dihydro-1*H*-pyrazol-1-carbonsäure-*tert*.-butylester

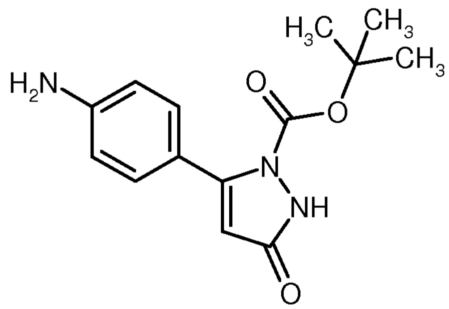

Eine Lösung von 2.2 g (7.1 mmol) 5-(4-Nitrophenyl)-3-oxo-2,3-dihydro-1*H*-pyrazol-1-carbonsäure-*tert*.-butylester in 100 ml Ethanol wurde in Gegenwart von 253 mg Palladium (10%ig auf Aktivkohle) bei RT und Normaldruck hydriert. Anschließend wurde das Reaktionsgemisch über Celite filtriert und das Filtrat im Vakuum eingeengt und getrocknet. Ausbeute: 1.99 g (90% Reinheit, 92% d. Th.)
LC/MS [Methode 6]: $R_t$ = 2.06 min; MS (ESIpos): m/z = 276 $(M+H)^+$

## Beispiel 1.5A

3-(4-Aminophenyl)-1,2,4-oxadiazol-5(4*H*)-on

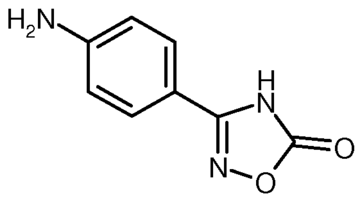

Eine Lösung von 1.5 g (7.2 mmol) 3-(4-Nitrophenyl)-1,2,4-oxadiazol-5(4*H*)-on in 75 ml Ethanol wurde mit 6.5 g (29 mmol, 4 eq.) Zinn(II)chlorid-dihydrat versetzt und 1 h bei 70°C gerührt. Das Reaktionsgemisch wurde auf Eiswasser gegossen und vorsichtig mit Natriumhydrogencarbonat bis zu einem pH-Wert von 8 versetzt. Das Gemisch wurde über eine Filterschicht filtriert und der Rückstand mit Ethylacetat nachgewaschen. Die vereinigten Filtrate wurden im Vakuum eingeengt. Der Rückstand wurde mit Dichlormethan und Methanol verrührt, 10 min im Ultraschallbad behandelt und anschließend filtriert. Das Filtrat wurde im Vakuum eingeengt und getrocknet. Ausbeute: 1.4 g (quant.)
LC/MS [Methode 1]: $R_t$ = 0.44 min; MS (ESIpos): m/z = 178 $(M+H)^+$
$^1H$-NMR (400 MHz, DMSO-$d_6$): $\delta$ [ppm] = 7.42 (d, 2H), 6.51 (d, 2H), 5.23 (s, 2H), 4.13 (br. s, 1H).

## Beispiel 1.6A

1-Methylhydrazin-1,2-dicarbonsäure-1-benzyl-2-*tert*.-butylester

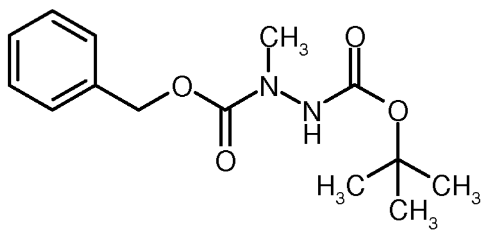

Eine Lösung von 14.2 g (78.8 mmol) 1-Methylhydrazincarbonsäurebenzylester in 100 ml Propan-2-ol wurde bei RT mit 20.6 g (94.6 mmol, 1.2 eq.) Di-*tert*.-butyldicarbonat in 42 ml Dichlormethan versetzt und 24 h bei RT gerührt. Das Reaktionsgemisch wurde mit Dichlormethan und Wasser verdünnt. Nach Phasentrennung wurde die organische

Phase getrocknet (Natriumsulfat), filtriert und im Vakuum eingeengt. Das Rohprodukt wurde mittels Flash-Chromatographie (Kieselgel-60, Eluent: Cyclohexan-Ethylacetat-Gemische) aufgereinigt. Ausbeute: 24.8 g (80% Reinheit, 90% d. Th.)
LC/MS [Methode 1]: $R_t$ = 1.00 min; MS (ESIneg): m/z = 279 $(M-H)^-$

**Beispiel 1.6B**

2-Methylhydrazincarbonsäure-*tert*.-butylester

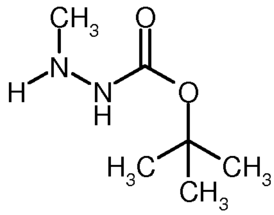

Eine Lösung von 24.8 g (70.8 mmol) 1-Methylhydrazin-1,2-dicarbonsäure-1-benzyl-2-*tert*.-butylester in 500 ml Ethanol wurde in Gegenwart von 1.24 g Palladium (10%ig auf Aktivkohle) bei RT und Normaldruck hydriert. Anschließend wurde das Reaktionsgemisch über Celite filtriert und das Filtrat im Vakuum eingeengt und getrocknet. Ausbeute: 12.3 g (48% Reinheit, 57% d. Th.)

**Beispiel 1.6C**

2-(2-Fluor-4-nitrobenzoyl)-2-methylhydrazincarbonsäure-*tert*.-butylester

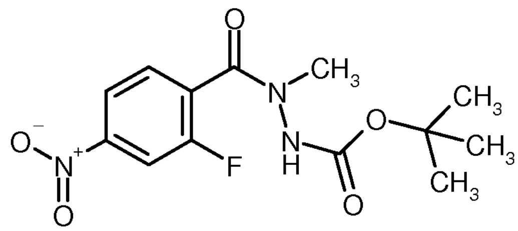

Eine Lösung von 9.1 g (49.3 mmol, 1.2 eq.) 2-Fluor-4-nitrobenzoesäure in 200 ml DMF wurde unter Argon bei RT mit 17.1g (53.4 mmol, 1.3 eq.) (Benzotriazol-l-yloxy)bisdimethylaminomethylium-fluorborat und 21.4 ml (123.1 mol, 3.0 eq.) *N,N*-Diisopropylethylamin versetzt und 20 min bei RT gerührt. Eine Lösung von 12.5 g (48% Reinheit, 41 mmol) 2-Methylhydrazincarbonsäure-*tert*.-butylester in 50 ml DMF wurde dazugegeben und das Reaktionsgemisch 6 h bei RT gerührt. Nach Entfernen des DMF im Vakuum und Zugabe von Wasser/Ethylacetat und Phasentrennung wurde die organische Phase mit 10% wässriger Zitronensäure und mit gesättigter, wässriger Natriumchlorid-Lösung gewaschen, getrocknet (Natriumsulfat), filtriert und im Vakuum eingeengt. Das Rohprodukt wurde mittels Flash-Chromatographie (Kieselgel-60, Eluent: Cyclohexan-Ethylacetat-Gemische) aufgereinigt. Ausbeute: 8.35 g (65% d. Th.)
LC/MS [Methode 1]: $R_t$ = 0.91 min; MS (ESIneg): m/z = 312 $(M-H)^-$
$^1$H-NMR (400 MHz, DMSO-$d_6$): $\delta$ [ppm] = 9.78 (s, 1H), 8.20 (d, 1H), 8.11 (d, 1H), 7.59 (t, 1H), 3.13 (s, 3H), 1.24 (s, 9H).

**Beispiel 1.6D**

2-Fluor-*N*-methyl-4-nitrobenzohydrazid

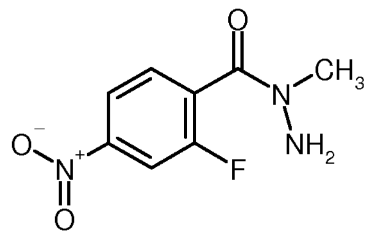

Eine Lösung von 3.6 g (11.5 mmol) 2-(2-Fluor-4-nitrobenzoyl)-2-methylhydrazincarbonsäure-*tert*.-butylester in 57 ml 4 molarer Salzsäure/Dioxan-Lösung wurde 3 h bei RT gerührt. Das Reaktionsgemisch wurde im Vakuum eingeengt, der Rückstand in Ethylacetat aufgenommen und mit gesättigter, wässriger Natriumhydrogencarbonat-Lösung gewaschen. Die organische Phase wurde getrocknet (Natriumsulfat), filtriert und im Vakuum eingeengt. Ausbeute: 2.0 g (81% d. Th.)
LC/MS [Methode 1]: $R_t$ = 0.50 min; MS (ESIpos): m/z = 214 $(M+H)^+$
$^1$H-NMR (400 MHz, DMSO-$d_6$): δ [ppm] = 8.09 (m, 2H), 7.61 (dd, 1H), 3.2 (s, 3H).

**Beispiel 1.6E**

2-Methyl-6-nitro-1,2-dihydro-3*H*-indazol-3-on

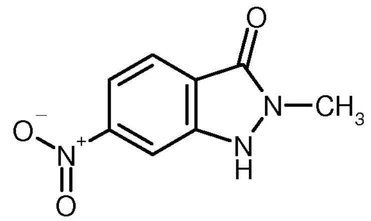

Eine Lösung von 2.4 g (10.9 mmol) 2-Fluor-*N*-methyl-4-nitrobenzohydrazid in 25 ml DMF wurde bei RT mit 6.6 ml (37.9 mmol, 3.5 eq.) *N,N*-Diisopropylethylamin versetzt und über Nacht bei 80°C gerührt. Das Reaktionsgemisch wurde im Vakuum eingeengt und der Rückstand in Ethylacetat aufgenommen. Der ausfallende Feststoff wurde filtriert und im Vakuum getrocknet. Ausbeute: 595 mg (28% d. Th.)
LC/MS [Methode 1]: $R_t$ = 0.49 min; MS (ESIpos): m/z = 194 $(M+H)^+$
$^1$H-NMR (400 MHz, DMSO-$d_6$): δ [ppm] = 8.14 (s, 1H), 7.85 (d, 1H), 7.78 (dd, 1H), 3.48 (s. 3H).

Alternativsynthese:

1.66 g (9.27 mmol) 6-Nitro-1,2-dihydro-3*H*-indazol-3-on wurden in 20 ml DMF vorgelegt und 1.75 ml (18.5 mmol, 2.0 eq.) Dimethylsulfat hinzugesetzt. Man erwärmte das Reaktionsgemisch 8 h auf 60°C, verdünnte dann mit Dichlormethan und schüttelte mit gesättigter, wässriger Natriumcarbonat-Lösung. Die wässrige Phase wurde je dreimal mit Dichlormethan und mit Essigsäureethylester gewaschen, und die organischen Fraktionen wurden verworfen. Darauffolgend wurde die wässrige Phase mit 4N wässriger Salzsäure vorsichtig auf pH 4.5 gebracht und dreimal mit Essigsäureethylester extrahiert. Diese vereinten organischen Phasen wurden über Magnesiumsulfat getrocknet und im Vakuum eingeengt. Das Rohprodukt wurde durch Flash-Chromatographie (Kieselgel-50, Eluens: Gradient Cyclohexan/Essigsäureethylester 1:1 bis Essigsäureethylester/Methanol 15:1) gereinigt und ergab die Titelverbindung. Ausbeute: 770 mg (43% d. Th.)
LC/MS [Methode 1]: $R_t$ = 0.50 min; MS (ESIpos): m/z = 194 $(M+H)^+$,
$^1$H-NMR (400 MHz, DMSO-$d_6$): δ [ppm] = 11.2 (br. s., 1H), 8.15 (d, 1H), 7.88 (d, 1H), 7.82 (dd, 1H), 3.48 (s, 3H).

**Beispiel 1.6F**

2-Methyl-6-nitro-3-oxo-2,3-dihydro-1*H*-indazol-1-carbonsäure-*tert*.-butylester

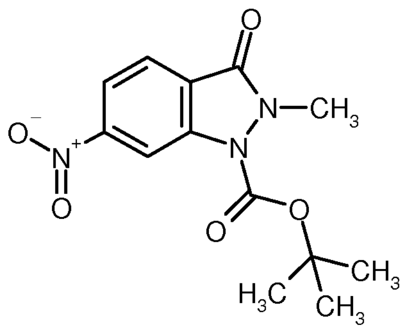

Eine Lösung von 595 mg (3.0 mmol) 2-Methyl-6-nitro-1,2-dihydro-3*H*-indazol-3-on in 25 ml Propan-2-ol wurde bei RT mit einer Lösung von 0.8 g (3.7 mmol, 1.2 eq.) Di-*tert*.-butyldicarbonat in 6 ml Dichlormethan versetzt und 12 h bei RT gerührt. Zur besseren Löslichkeit des Reaktionsgemisches wurden 6 ml DMF zugegeben. Das Reaktionsgemisch wurde mit weiteren 4 eq. Di-*tert*.-butyldicarbonat versetzt, 24 h bei RT gerührt und anschließend mit Dichlormethan und Wasser verdünnt. Nach Phasentrennung wurde die organische Phase getrocknet (Natriumsulfat), filtriert und im Vakuum eingeengt. Das Rohprodukt wurde mittels Flash-Chromatographie (Kieselgel-60, Eluent: Cyclohexan-Ethylacetat-Gemische) aufgereinigt. Ausbeute: 720 mg (80% d. Th.)
LC/MS [Methode 1]: $R_t$ = 1.04 min; MS (ESIpos): m/z = 194 $(M+H-Boc)^+$
$^1$H-NMR (400 MHz, DMSO-$d_6$): δ [ppm] = 8.62 (d, 1H), 8.17 (dd, 1H), 8.05 (d, 1H), 3.58 (s. 3H), 1.63 (s, 9H).

**Beispiel 1.6G**

6-Amino-2-methyl-3-oxo-2,3-dihydro-1*H*-indazol-1-carbonsäure-*tert*.-butylester

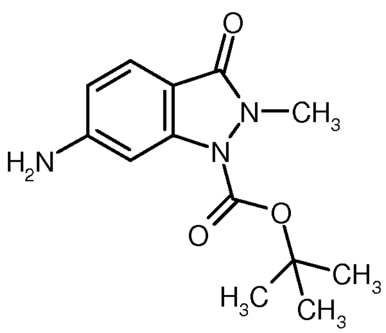

Eine Lösung von 715 mg (2.4 mmol) 2-Methyl-6-nitro-3-oxo-2,3-dihydro-1*H*-indazol-1-carbonsäure-*tert*.-butylester in 30 ml Ethanol wurde in Gegenwart von 52 mg Palladium (10%ig auf Aktivkohle) bei RT und Normaldruck hydriert. Anschließend wurde das Reaktionsgemisch über Celite filtriert und das Filtrat im Vakuum eingeengt und getrocknet. Ausbeute: 668 mg (100% d. Th.)
LC/MS [Methode 1]: $R_t$ = 0.79 min; MS (ESIpos): m/z = 264 $(M+H)^+$
$^1$H-NMR (400 MHz, DMSO-$d_6$): δ [ppm] = 7.36 (d, 1H), 6.94 (d, 1H), 6.53 (dd, 1H), 6.21 (s, 2H), 1.58 (s, 9H).

**Beispiel 1.6H**

6-Nitro-1,2-dihydro-3*H*-indazol-3-on

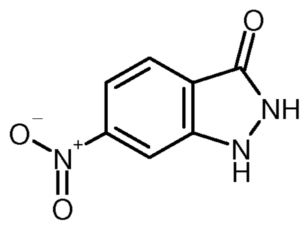

In zwei gleich großen Portionen wurden insgesamt 2.00 g (10.0 mmol) 2-Fluor-4-nitrobenzoesäuremethylester und 2.51 g (50.2 mmol) Hydrazin-Monohydrat in 36 ml Ethanol im Mikrowellenreaktor 2 h auf 120°C erhitzt. Die vereinigten Reaktionslösungen wurden mit Essigsäureethylester verdünnt und mit Wasser gewaschen. Die wässrige Phase wurde dreimal mit Essigsäureethylester extrahiert. Die vereinten organischen Phasen wurden über Magnesiumsulfat getrocknet und im Vakuum eingeengt. Ausbeute: 1.66 g (95% Reinheit, 88% d. Th.)
LC/MS [Methode 11]: $R_t$ = 0.73 min; MS (ESIpos): m/z = 180 $(M+H)^+$,
$^1$H-NMR (400 MHz, DMSO-$d_6$): δ [ppm] = 12.4 (s, 1H), 11.0 (br. s, 1H), 8.21 (d, 1H), 7.86 (d, 1H), 7.78 (dd, 1H).

**Beispiel 1.7A**

1,3-Thiazolidin-2,4-dion-Kaliumsalz

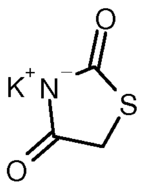

Eine Lösung von 2.0 g (17.1 mmol) 1,3-Thiazolidin-2,4-dion in 7 ml Ethanol wurde bei 50°C mit einer Lösung von 1.05 g (18.8 mmol, 1.1 eq.) Kaliumhydroxid in 3 ml Ethanol versetzt und 2 h bei RT gerührt. Der entstandene Niederschlag wurde filtriert, mit Ethanol gewaschen und im Vakuum getrocknet. Ausbeute: 2.3 g (87% d. Th.)

**Beispiel 1.7B**

3-[2-(4-Nitrophenyl)-2-oxoethyl]-1,3-thiazolidin-2,4-dion

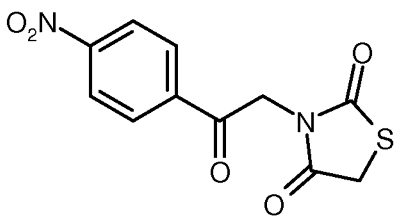

Eine Lösung von 2.0 g (8.2 mmol) 2-Brom-1-(4-nitrophenyl)ethanon in 80 ml Aceton wurde portionsweise mit 1.3 g (8.4 mmol) 1,3-Thiazolidin-2,4-dion-Kaliumsalz versetzt und 1 h bei 60°C gerührt. Das Reaktionsgemisch wurde im Vakuum eingeengt und der Rückstand in Wasser/Dichlormethan gelöst. Nach Phasentrennung wurde die organische Phase getrocknet (Natriumsulfat), filtriert und im Vakuum eingeengt und getrocknet. Ausbeute: 2.3 g (98% d. Th.)
LC/MS [Methode 3]: $R_t$ = 1.81 min; MS (ESIpos): m/z = 281 $(M+H)^+$.

**Beispiel 1.7C**

5-(4-Nitrophenyl)-1,3-oxazol-2(3*H*)-on

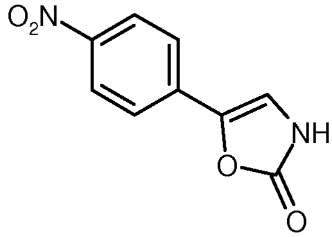

Eine Lösung von 2.3 g (8.0 mmol) 3-[2-(4-Nitrophenyl)-2-oxoethyl]-1,3-thiazolidin-2,4-dion in 80 ml Ethanol wurde mit 2.8 ml (20.1 mmol, 2.5 eq.) Triethylamin versetzt und 14 h unter Rückfluß gerührt. Das Reaktionsgemisch wurde im Vakuum eingeengt und der Rückstand in Wasser/Essigsäureethylester gelöst. Nach Phasentrennung wurde die organische Phase getrocknet (Natriumsulfat), filtriert und im Vakuum eingeengt und getrocknet. Der Rückstand wurde in Dichlormethan verrührt und der Niederschlag filtriert und im Vakuum getrocknet. Ausbeute: 1.1g (67% d. Th.)
LC/MS [Methode 1]: $R_t$ = 0.71 min; MS (ESIneg): m/z = 205 $(M-H)^-$.

**Beispiel 1.7D**

5-(4-Aminophenyl)-1,3-oxazol-2(3*H*)-on

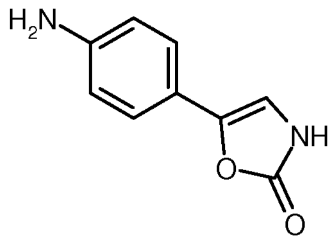

Eine Lösung von 1.1 g (5.4 mmol) 5-(4-Nitrophenyl)-1,3-oxazol-2(3*H*)-on in 40 ml Ethanol wurde in Gegenwart von 111 mg Palladium (10%ig auf Aktivkohle) 5 d bei RT und Normaldruck hydriert. Anschließend wurde das Reaktionsgemisch über Celite filtriert und der Rückstand mit Ethanol gewaschen. Die vereinigten Filtrate wurden im Vakuum eingeengt. Der Rückstand wurde ohne weitere Reinigung in der nächsten Stufe eingesetzt. Ausbeute: 626 mg (88% Reinheit, 58% d. Th.)
LC/MS [Methode 5]: $R_t$ = 1.23 min; MS (ESIpos): m/z = 177 $(M+H)^+$.

**Beispiel 1.8A**

6-Nitro-2-(trichlormethyl)-1*H*-benzimidazol

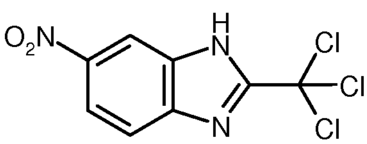

Eine Lösung von 5.0 g (32.7 mmol) 4-Nitrobenzol-1,2-diamin in 150 ml Eisessig wurde tropfenweise bei 0°C mit 6.3 g (35.9 mmol, 1.1 eq.) Methyl-2,2,2-trichlorethanimidoat versetzt. Das Reaktionsgemisch wurde 3 h bei RT

gerührt, anschließend auf 400 ml Wasser gegeben und mit 300 ml Essigsäureethylester versetzt. Nach Phasentrennung wurde die wässrige Phase zweimal mit Essigsäureethylester extrahiert. Die vereinigten, organischen Phasen wurden zweimal mit je 130 ml gesättigter, wässriger Natriumhydrogencarbonat-Lösung und einmal mit gesättigter, wässriger Natriumchlorid-Lösung gewaschen, getrocknet (Natriumsulfat), filtriert und im Vakuum eingeengt und getrocknet. Das Rohprodukt wurde mit Pentan verrieben und über Nacht stehen gelassen. Der Feststoff wurde anschließend filtriert, mit Pentan gewaschen und im Vakuum getrocknet. Ausbeute: 10.1 g (77% Reinheit, 85% d. Th.)
LC/MS [Methode 1]: $R_t$ = 0.91 min; MS (ESIpos): m/z = 280 $(M+H)^+$.

**Beispiel 1.8B**

6-Nitro-1*H*-benzimidazol-2-carbonsäureethylester

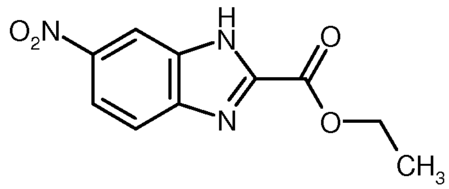

Eine Lösung von 10.0 g (77% Reinheit, 27.5 mmol) 6-Nitro-2-(trichlormethyl)-1*H*-benzimidazol in 100 ml Ethanol wurde mit 15.4 g (90.7 mmol, 3.3 eq.) Silber(I)nitrat versetzt, 15 h unter Rückfluß gerührt, auf RT abgekühlt und im Vakuum eingeengt. Der Rückstand wurde in eine Mischung aus 250 ml Salzsäure (1N) und 220 ml Essigsäureethylester aufgenommen, 1 h gerührt und über Kieselgel filtriert. Nach Phasentrennung wurde die organische Phase getrocknet (Natriumsulfat), filtriert und im Vakuum eingeengt und getrocknet. Der Rückstand wurde in 30 ml Diisopropylether verrührt, filtriert, mit Diisopropylether und Petrolether gewaschen und im Vakuum getrocknet. Ausbeute: 1.9 g (29% d. Th.)
LC/MS [Methode 1]: $R_t$ = 0.70 min; MS (ESIpos): m/z = 236 $(M+H)^+$.

**Beispiel 1.8C**

6-Amino-1*H*-benzimidazol-2-carbonsäureethylester

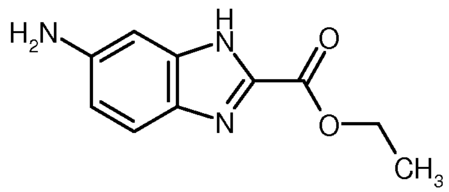

Eine Lösung von 1.9 g (8.1 mmol) 6-Nitro-1*H*-benzimidazol-2-carbonsäureethylester in 30 ml Ethanol wurde in Gegenwart von 190 mg Palladium (10%ig auf Aktivkohle) 3 h bei RT und Normaldruck hydriert. Anschließend wurde das Reaktionsgemisch über Kieselgur filtriert und der Rückstand mit Ethanol gewaschen. Die vereinigten Filtrate wurden im Vakuum eingeengt. Der Rückstand wurde im Vakuum getrocknet und anschließend mittels Flash-Chromatographie (Kieselgel-50, Eluent: Dichlormethan/Methanol 3-5%) aufgereinigt. Ausbeute: 640 mg (39% d. Th.)
LC/MS [Methode 5]: $R_t$ = 1.34 min; MS (ESIpos): m/z = 206 $(M+H)^+$
$^1$H-NMR (400 MHz, DMSO-$d_6$): $\delta$ [ppm] = 12.71 (s, 1H), 7.38 (d, 1H), 6.63 (dd, 1H), 6.58 (s, 1H), 5.29 (s, 2H), 4.34 (q, 2H), 1.34 (t, 3H).

**Beispiel 1.9A**

5-Amino-3-chlor-1*H*-indazol

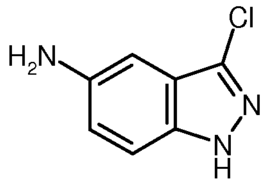

1.00 g (5.06 mmol) 3-Chlor-5-nitro-1*H*-indazol wurde in 50 ml Ethanol suspendiert und mit 5.71 g (25.3 mmol) Zinn(II)chlorid-Dihydrat versetzt. Man ließ über Nacht unter Rückfluss rühren, versetzte dann mit gesättigter, wässriger Natriumhydrogencarbonat-Lösung und extrahierte dreimal mit Essigsäureethylester. Die vereinten organischen Phasen wurden über Magnesiumsulfat getrocknet, und das Lösungsmittel wurde im Vakuum entfernt. Man verrührte mit *tert*-Butylmethylether und saugte den Feststoff ab. Ausbeute: 544 mg (90% Reinheit, 58% d. Th.).
LC/MS [Methode 5]: $R_t$ = 1.50 min; MS (ESIpos): m/z = 168 $(M+H)^+$,
$^1$H-NMR (400 MHz, DMSO-$d_6$): δ [ppm] = 12.8 (s, 1H), 7.28 (d, 1H), 6.89 (dd, 1H), 6.66 (m, 1H), 5.46 (br. s, 2H).

**Beispiel 1.10A**

2-Fluor-4-nitrobenzoesäure-*tert*.-butylester

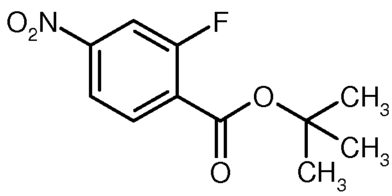

Eine Lösung von 500 mg (2.7 mmol) 2-Fluor-4-nitrobenzoesäure und 1.03 g (5.4 mmol, 2.0 eq.) para-Toluolsulfonsäurechlorid in 5.4 ml Pyridin wurde bei 0°C mit 0.258 ml (2.7 mmol, 1.0 eq.) tert.-Butanol versetzt, 60 min gerührt und mit weiteren 0.258 ml (2.7 mmol, 1.0 eq.) tert.-Butanol versetzt. Das Reaktionsgemisch wurde 18 h nachgerührt und im Vakuum eingeengt. Der Rückstand wurde mit gesättigter, wässriger Natriumhydrogencarbonat-Lösung und Essigsäureethylester versetzt. Nach Phasentrennung wurde die wässrige Phase mit Essigsäureethylester extrahiert. Die vereinigten, organischen Phasen wurden mit gesättigter, wässriger Natriumchlorid-Lösung gewaschen, getrocknet (Natriumsulfat), filtriert und im Vakuum eingeengt. Das Rohprodukt wurde anschließend mittels Normalphasen-Chromatographie (Eluent: Cyclohexan-Essigsäureethylester 14%-20% Gemische) aufgereinigt. Ausbeute: 524 mg (75% d. Th.).
LC/MS [Methode 1]: $R_t$ = 1.16 min; MS (ESIneg): m/z = 226 $(M-CH_3)^-$,
$^1$H-NMR (400 MHz, DMSO-$d_6$): δ [ppm] = 8.21 (dd, 1H), 8.16-8.12 (m, 1H), 8.06 (dd, 1H), 1.56 (s, 9H).

**Beispiel 1.10B**

4-Amino-2-fluorbenzoesäure-*tert*.-butylester

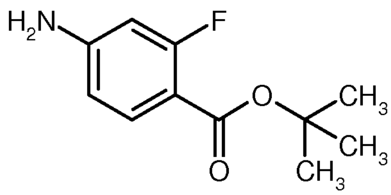

Eine Lösung von 1.109 g (20.73 mmol, 10 eq.) Ammoniumchlorid in 6.25 ml Ethanol und 3.125 ml Wasser wurde auf 95°C erhitzt und mit 500 mg (2.07 mmol) 2-Fluor-4-nitrobenzoesäure-*tert*.-butylester versetzt. 347 mg (6.22 mmol, 3 eq.) Eisenpulver wurden in kleinen Portionen über 1 h dazugegeben. Das Reaktionsgemisch wurde anschließend

30 min bei 95°C gerührt und heiß über Kieselgur filtriert. Der Filterkuchen wurde mit Ethanol gewaschen und das Filtrat im Vakuum von Ethanol befreit. Die wässrige Phase wurde dreimal mit jeweils 20 ml Diethylether extrahiert. Die vereinigten, organischen Phasen wurden mit gesättigter, wässriger Natriumchlorid-Lösung gewaschen, getrocknet (Natriumsulfat), filtriert und im Vakuum eingeengt. Das Rohprodukt wurde mittels Normalphasen-Chromatographie (Eluent: Cyclohexan-Essigsäureethylester 30-50% Gemische) aufgereinigt. Ausbeute: 280 mg (51% d. Th.).
LC/MS [Methode 8]: $R_t$ = 1.18 min; MS (ESIneg): m/z = 210 $(M-H)^-$,
$^1$H-NMR (400 MHz, DMSO-$d_6$): $\delta$ [ppm] = 7.49 (t, 1H), 6.36 (dd, 1H), 6.25 (dd, 1H), 6.15 (s, 2H), 1.48 (s, 9H).

**Beispiel 1.11A**

4-Amino-3-fluorbenzoesäure-*tert.*-butylester

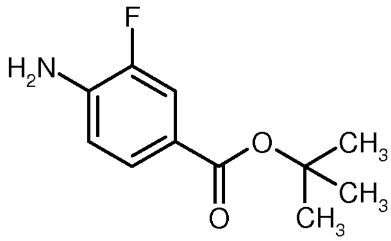

Nach der allgemeinen Methode 9A wurden 400 mg (1.66 mmol) 3-Fluor-4-nitrobenzoesäure-*tert.*-butylester umgesetzt. Das Rohprodukt wurde mittels Normalphasen-Chromatographie (Eluent: Cyclohexan-Essigsäureethylester 15-20% Gemische) aufgereinigt. Ausbeute: 295 mg (82% d. Th.)
LC/MS [Methode 1]: $R_t$ = 1.00 min; MS (ESIpos): m/z = 212 $(M+H)^+$,
$^1$H-NMR (400 MHz, DMSO-$d_6$): $\delta$ [ppm] = 7.48-7.38 (m, 2H), 6.75 (t, 1H), 5.95 (br. s, 2H), 1.50 (s, 9H).

**Beispiel 1.12A**

2,5-Difluor-4-nitrobenzoesäure-*tert.*-butylester

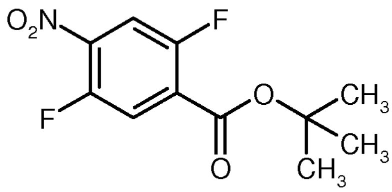

Nach der allgemeinen Methode 10A wurden 700 mg (3.45 mmol) 2,5-Difluor-4-nitrobenzoesäure umgesetzt. Das Rohprodukt wurde ohne weitere Reinigung in der nächsten Stufe eingesetzt. Ausbeute: 1000 mg (73% Reinheit, 82% d. Th.)
HPLC [Methode 3]: $R_t$ = 2.45 min,
$^1$H-NMR (400 MHz, DMSO-$d_6$): $\delta$ [ppm] = 8.24 (dd, 1H), 7.99 (dd, 1H), 1.56 (s, 9H).

**Beispiel 1.12B**

4-Amino-2,5-difluorbenzoesäure-*tert.*-butylester

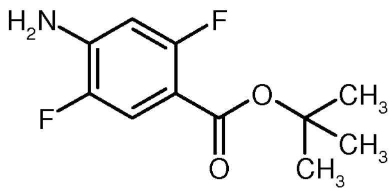

Eine Lösung von 1000 mg (2.82 mmol) 2,5-Difluor-4-nitrobenzoesäure-*tert.*-butylester in 8 ml Tetrahydrofuran und 8 ml Essigsäureethylester wurde in Gegenwart von 65.6 mg Palladium (10%ig auf Aktivkohle) bei RT und Normaldruck hydriert. Anschließend wurde das Reaktionsgemisch über Celite filtriert, das Filtrat im Vakuum eingeengt und getrocknet. Das Rohprodukt wurde mittels Normalphasen-Chromatographie (Eluent: Cyclohexan-Essigsäureethylester 15-20% Gemische) aufgereinigt. Ausbeute: 155 mg (85% Reinheit, 20% d. Th.)
LC/MS [Methode 8]: $R_t$ = 1.27 min; MS (ESIpos): m/z = 230 $(M+H)^+$,
$^1$H-NMR (400 MHz, DMSO-$d_6$): δ [ppm] = 7.35 (dd, 1H), 6.47 (dd, 1H), 6.27 (s, 2H), 1.49 (s, 9H).

**Beispiel 1.13A**

4-[(*tert.*-Butoxycarbonyl)amino]-2,6-difluorbenzoesäuremethylester

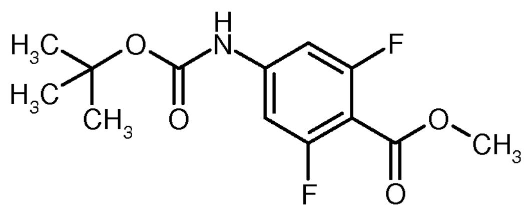

Ein Mikrowellengefäß wurde unter Argon mit 54 mg (0.22 mmol) 4-Brom-2,6-difluorbenzoesäuremethylester, 118 mg (1.01 mmol, 4.7 eq.) *tert.*-Butylcarbamat, 4.6 mg (0.02 mmol, 0.1 eq.) Palladium(II)acetat, 15 mg (0.026 mmol, 0.13 eq.) Xantphos, 137 mg (0.42 mmol, 2 eq.) Cäsiumcarbonat und mit 2 ml 1,4-Dioxan beladen. Ein Argonstrom wurde 2 min lang durch die Suspension geleitet. Das Reaktionsgemisch wurde 20 min in der Mikrowelle bei 140°C erhitzt. Nach Filtrieren über Kieselgur wurde das Filtrat im Vakuum eingeengt. Das Rohprodukt wurde mittels Normalphasen-Chromatographie (Eluent: Dichlormethan-Methanol 10-50% Gemische) aufgereinigt. Ausbeute: 37 mg (60% d. Th.)
LC/MS [Methode 8]: $R_t$ = 1.35 min; MS (ESIneg): m/z = 286 $(M-H)^-$,
$^1$H-NMR (400 MHz, DMSO-$d_6$): δ [ppm] = 10.10 (s, 1H), 7.28-7.22 (m, 2H), 3.83 (s, 3H), 1.49 (s, 9H).

**Beispiel 1.13B**

4-Amino-2,6-difluorbenzoesäuremethylester

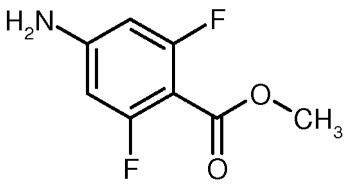

Eine Lösung von 36 mg (0.125 mmol) 4-[(*tert.*-Butoxycarbonyl)amino]-2,6-difluorbenzoe-säuremethylester in 1 ml Dichlormethan wurde bei RT mit 0.5 ml TFA versetzt und bei RT für 30 min gerührt. Anschließend wurde das Reaktionsgemisch im Vakuum eingeengt, der Rückstand mehrmals mit Dichlormethan und Toluol coevaporiert und im Vakuum getrocknet. Das Rohprodukt wurde ohne Reinigung in der nächsten Stufe eingesetzt. Ausbeute: 24 mg (quant.)
LC/MS [Methode 3]: $R_t$ = 1.56 min; MS (ESIpos): m/z = 188 $(M+H)^+$,
$^1$H-NMR (400 MHz, DMSO-$d_6$): δ [ppm] = 6.44 (s, 2H), 6.24-6.15 (m, 2H), 3.73(s, 3H).

**Beispiel 1.14A**

*tert.* -Butyl- [4-(*N*'-hydroxycarbamimidoyl)phenyl]carbamat

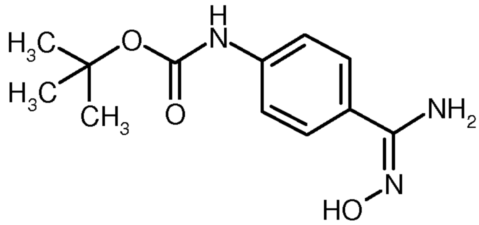

Eine Lösung von 2.0 g (9.16 mmol) *tert.*-Butyl-(4-cyanophenyl)carbamat in 45 ml Ethanol wurde bei RT mit 1.40 g (20.16 mmol, 2.2 eq.) Hydroxylammoniumchlorid und 2.81 ml (20.16 mmol, 2.2 eq.) Triethylamin versetzt. Das Reaktionsgemisch wurde 4 h unter Rückfluss erhitzt und im Vakuum eingeengt. Der Rückstand wurde 1 h bei RT mit 100 ml Wasser gerührt. Das Reaktionsgemisch wurde filtriert und der Filterkuchen mit Wasser gewaschen. Der Rückstand wurde in Essigsäureethylester gelöst. Die verbleibende wässrige Phase wurde abgetrennt und die organische Phase wurde getrocknet (Natriumsulfat), filtriert und im Vakuum eingeengt. Das Rohprodukt wurde ohne Reinigung in der nächsten Stufe eingesetzt. Ausbeute: 2.10 g (95% Reinheit, 87% d. Th.).
LC/MS [Methode 8]: Rt = 0.68 min; MS (ESIpos): m/z = 252 $(M+H)^+$,
$^1$H-NMR (400 MHz, DMSO-$d_6$): $\delta$ [ppm] = 9.45 (s, 1H), 9.42 (br. s, 1H), 7.57-7.52 (m, 2H), 7.46-7.40 (m, 2H), 5.69 (s, 2H), 1.48 (s, 9H).

**Beispiel 1.14B**

*tert.*-Butyl-[4-(5-oxo-4,5-dihydro-1,2,4-thiadiazol-3-yl)phenyl]carbamat

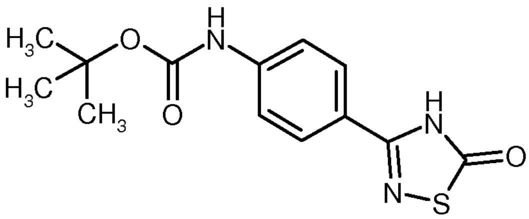

Eine Lösung von 500 mg (1.99 mmol) *tert*-Butyl-[4-(*N*'-hydroxycarbamimidoyl)phenyl]-carbamat in 16 ml Tetrahydrofuran wurde bei RT mit 560 mg (2.98 mmol, 1.5 eq.) 1,1'-Thiocarbonylimidazol versetzt und 30 min bei RT gerührt. Anschließend wurde das Reaktionsgemisch mit Wasser ersetzt. Nach Phasentrennung wurde die wässrige Phase mit Essigsäureethylester extrahiert. Die vereinigten, organischen Phasen wurden mit gesättigter, wässriger Natriumchlorid-Lösung gewaschen, getrocknet (Natriumsulfat), filtriert und im Vakuum eingeengt. Das Rückstand wurde in 8 ml Tetrahydrofuran gelöst und mit 0.76 ml (5.97 mmol, 3.0 eq.) Bortrifluorid-Diethylether-Komplex ersetzt. Das Reaktionsgemisch wurde 1 h bei RT gerührt. Nach Zugabe von Wasser/Essigsäureethylester und Phasentrennung wurde die wässrige Phase dreimal mit Essigsäureethylester extrahiert. Die vereinigten, organischen Phasen wurden mit gesättigter, wässriger Natriumchlorid-Lösung gewaschen, getrocknet (Natriumsulfat), filtriert und im Vakuum eingeengt. Das Rohprodukt wurde ohne Reinigung in der nächsten Stufe eingesetzt. Ausbeute: 130 mg (70% Reinheit, 15% d. Th.)
LC/MS [Methode 8]: Rt = 1.18 min; MS (ESIpos): m/z = 294 $(M+H)^+$,
$^1$H-NMR (400 MHz, DMSO-$d_6$): $\delta$ [ppm] = 13.24 (br. s, 1H), 9.70 (s, 1H), 7.87-7.80 (m, 2H), 7.61-7.54 (m, 2H), 1.49 (s, 9H).

**Beispiel 1.14C**

3-(4-Aminophenyl)-1,2,4-thiadiazol-5(4*H*)-on

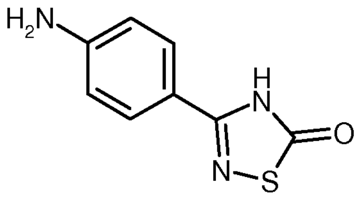

Eine Lösung von 129 mg (70% Reinheit, 0.44 mmol) *tert.*-Butyl-[4-(5-oxo-4,5-dihydro-1,2,4-thiadiazol-3-yl)phenyl]carbamat in 4 ml Dichlormethan wurde bei 0°C mit 0.8 ml TFA versetzt und 40 min bei RT gerührt. Anschließend wurde das Reaktionsgemisch im Vakuum eingeengt und das Rohprodukt mittels präparativer HPLC (Wasser-Acetonitril-0.1 % Ameisensäure-Gradient) aufgereinigt. Ausbeute: 54 mg (90% Reinheit, 57% d. Th.)
LC/MS [Methode 8]: Rt = 0.68 min; MS (ESIpos): m/z = 194 $(M+H)^+$,
$^1$H-NMR (400 MHz, DMSO-$d_6$): δ [ppm] = 12.93 (br. s, 1H), 7.65-7.60 (m, 2H), 6.64-6.58 (m, 2H), 5.58 (br. s, 2H).

**Beispiel 1.15A**

*tert.*-Butyl-[4-(5-thioxo-4,5-dihydro-1,2,4-oxadiazol-3-yl)phenyl]carbamat

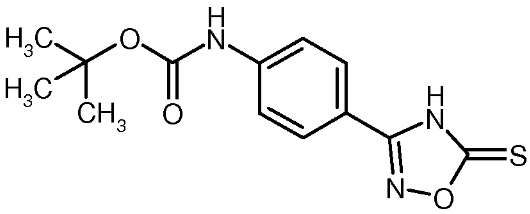

Eine Lösung von 500 mg (1.99 mmol) *tert*-Butyl-[4-(*N*'-hydroxycarbamimidoyl)phenyl]-carbamat in 20 ml Acetonitril wurde bei RT mit 821 mg (4.37 mmol, 2.2 eq.) 1,1'-Thiocarbonylimidazol und 1.19 ml (7.96 mmol, 4.0 eq.) 1,8-Diazabicyclo(5.4.0)undec-7-en versetzt und 24 h bei RT gerührt. Anschließend wurde das Reaktionsgemisch im Vakuum eingeengt und der Rückstand in Essigsäureethylester gelöst. Die organische Phase wurden mit Wasser und mit einer Kaliumcitrat/Zitronensäure-Lösung (pH 5) gewaschen. Anschließend wurde die organische Phase mit gesättigter, wässriger Natriumchlorid-Lösung gewaschen, getrocknet (Natriumsulfat), filtriert und im Vakuum eingeengt. Das Rohprodukt wurde mittels Normalphasen-Chromatographie (Eluent: Dichlormethan-Methanol 0-10% Gemische) aufgereinigt. Ausbeute: 120 mg (20% d. Th.)
LC/MS [Methode 8]: Rt = 1.17 min; MS (ESIneg): m/z = 292 $(M-H)^-$,
$^1$H-NMR (400 MHz, DMSO-$d_6$): δ [ppm] = 9.80 (s, 1H), 7.81-7.76 (m, 2H), 7.67-7.62 (m, 2H), 1.49 (s, 9H).

**Beispiel 1.15B**

3-(4-Aminophenyl)-1,2,4-oxadiazol-5(4*H*)-thion

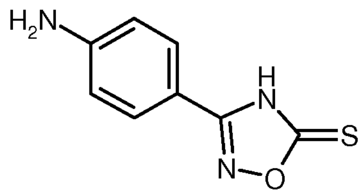

Eine Lösung von 119 mg (0.40 mmol) *tert*.-Butyl-[4-(5-thioxo-4,5-dihydro-1,2,4-oxadiazol-3-yl)phenyl]carbamat in 4 ml Dichlormethan wurde bei 0°C mit 0.8 ml TFA versetzt und 40 min bei RT gerührt. Anschließend wurde das Reaktionsgemisch im Vakuum eingeengt. Der Rückstand wurde mit Essigsäureethylester und einer gesättigten, wässrigen Natriumhydrogencarbonat-Lösung versetzt. Nach Phasentrennung wurde die wässrige Phase mit Essigsäureethylester extrahiert. Die wässrige Phase wurde eingeengt und das Rohprodukt mittels präparativer HPLC (Wasser-Acetonitril-0.1% Ameisensäure-Gradient) aufgereinigt. Ausbeute: 30 mg (38% d. Th.)
LC/MS [Methode 8]: Rt = 0.71 min; MS (ESIpos): m/z = 194 $(M+H)^+$.

**Beispiel 1.16A**

4-(1,3-Oxazol-2-yl)anilin

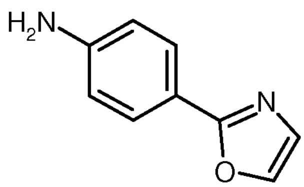

Nach der allgemeinen Methode 9A wurden 250 mg (1.31 mmol) 2-(4-Nitrophenyl)-1,3-oxazol umgesetzt. Das Rohprodukt wurde ohne Reinigung in der nächsten Stufe eingesetzt. Ausbeute: 220 mg (99% d. Th.)
LC/MS [Methode 1]: Rt = 0.55 min; MS (ESIpos): m/z = 161 $(M+H)^+$,
$^1$H-NMR (400 MHz, DMSO-$d_6$): $\delta$ [ppm] = 8.00 (d, 1H), 7.66-7.60 (m, 2H), 7.20 (d, 1H), 6.66-6.59 (m, 2H), 5.67 (br. s, 2H).

**Beispiel 1.17A**

3-[5-(4-Aminophenyl)-4*H*-1,2,4-triazol-3-yl]-2,2,3,3-tetrafluorpropansäuremethylester

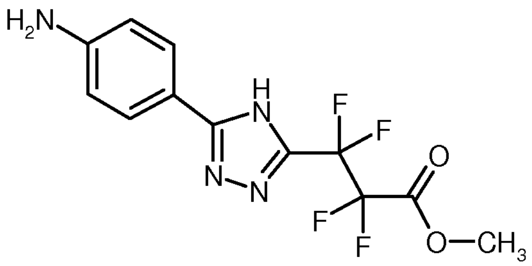

Ein Gemisch aus 9.7 g (48.5 mmol) 4-Nitrobenzolcarboximidohydrazid in 150 ml Dichlormethan wurde mit 15.0 g (87.2 mmol) 3,3,4,4-Tetrafluordihydrofuran-2,5-dion 2 min bei RT gerührt, die Suspension mit 150 ml Acetonitril versetzt und die erhaltene Lösung 16 h bei RT gerührt. Das Reaktionsgemisch wurde auf Kieselgel gezogen und mittels Flash-Chromatographie aufgetrennt (Dichlormethan-Methanol-Gemische). Die produkthaltigen Fraktionen wurden vereinigt und im Vakuum eingeengt. Der Rückstand wurde mit wenig Methanol verrührt, filtriert und im Vakuum getrocknet.

Der Rückstand wurde in Methanol gelöst, mit 1 ml Schwefelsäure versetzt und 4 h bei 70°C gerührt. Methanol wurde im Vakuum aus dem Reaktionsgemisch entfernt. Der Rückstand wurde in Essigsäureethylester aufgenommen und mit gesättigter Natriumhydrogenlösung extrahiert. Die organische Phase wurde mit gesättigter Natriumchlorid-Lösung gewaschen, getrocknet (Natriumsulfat), filtriert und im Vakuum eingengt.

Der Rückstand wurde in 150 ml Ethanol gelöst, mit 43.2 g (191.8 mmol) Zinn(II)chlorid-Dihydrat versetzt und 1 h bei 70°C gerührt. Das Reaktionsgemisch wurde auf Eiswasser gegossen, mit festem Natriumhydrogencarbonat auf pH 8 eingestellt und zum Abtrennen der ausgefallenen Salze über Kieselgur filtriert. Das Filtrat wurde mit Essigsäureethylester extrahiert. Die vereinigten, organischen Phasen wurde mit gesättigter Natriumchlorid-Lösung gewaschen, getrocknet (Natriumsulfat), filtriert und im Vakuum eingengt. Der Rückstand wurde in 600 ml Methanol aufgenommen, mit 200 mg Natriummethylat versetzt und 2 d bei RT gerührt. Das Reaktionsgemisch wurde im Vakuum eingeengt und getrocknet. Ausbeute: 9.2 g (91% Reinheit, 99% d. Th.)
LC-MS (Methode 1): $R_t$ = 0.77 min; MS (ESIpos): m/z = 319 $[M+H]^+$.

**Beispiel 2.1A**

2-(4-Brom-2-oxopyridin-1(2*H*)-yl)propansäure-*tert*.-butylester (Racemat)

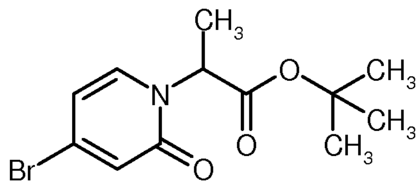

Nach der allgemeinen Methode 4B wurden 6.0 g (34.5 mmol) 4-Brompyridin-2(1*H*)-on mit 7.9 g (37.9 mmol) 2-Brompropansäure-*tert*.-butylester (Racemat) umgesetzt. Nach Entfernen des DMF wurde das gewünschte Produkt mit Wasser ausgefällt und anschließend mittels Flash-Chromatographie (Kieselgel-60, Eluent: Cyclohexan-Ethylacetat-Gemische) weiter aufgereinigt. Ausbeute: 7.4 g (69% d. Th.)
LC/MS [Methode 1]: $R_t$ = 0.94 min; MS (ESIpos): m/z = 302 $(M+H)^+$
$^1$H-NMR (400 MHz, DMSO-$d_6$): $\delta$ [ppm] = 7.66 (d, 1H), 6.75 (d, 1H), 6.51 (dd, 1H), 5.04 (q, 1H), 1.51 (d, 3H), 1.37 (s, 9H).

**Beispiel 2.2A**

2-[4-(5-Chlor-2-cyanophenyl)-2-oxopyridin-1(2*H*)-yl]propansäure-*tert*.-butylester (Racemat)

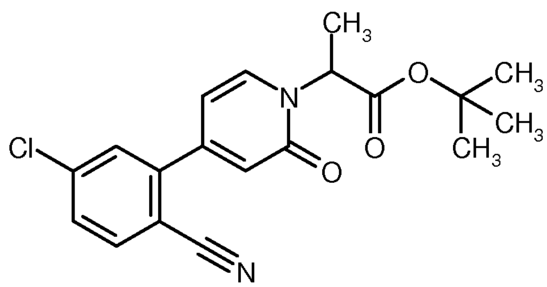

Nach der allgemeinen Methode 2A wurden 2.4 g (74% Reinheit, 5.9 mmol) 2-(4-Brom-2-oxopyridin-1(2*H*)-yl)propansäure-*tert*.-butylester (Racemat) mit 1.2 g (6.8 mmol) 5-Chlor-2-cyanophenylboronsäure in Gegenwart von Tetrakis(triphenylphosphin)palladium(0) umgesetzt. Ausbeute: 1.86 g (87% Reinheit, 77% d. Th.)
LC/MS [Methode 1]: $R_t$ = 1.07 min; MS (ESIpos): m/z = 359 $(M+H)^+$
$^1$H-NMR (400 MHz, DMSO-$d_6$): $\delta$ [ppm] = 8.03 (d, 1H), 7.84 (d, 1H), 7.81 (d, 1H), 7.75 (dd, 1H), 6.64 (d, 1H), 6.50 (dd, 1H), 5.14 (q, 1H), 1.58 (d, 3H), 1.40 (s, 9H).

**Beispiel 2.2B**

2-[4-(5-Chlor-2-cyanophenyl)-2-oxopyridin-1(2*H*)-yl]propansäure (Racemat)

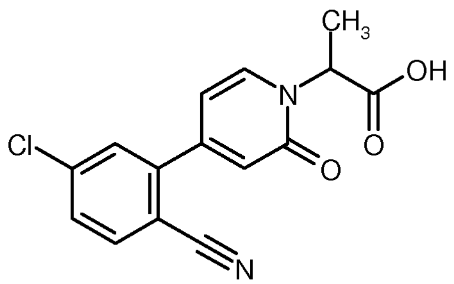

Nach der allgemeinen Methode 6A wurden 2.2 g (82% Reinheit, 5.0 mmol) 2-[4-(5-Chlor-2-cyanophenyl)-2-oxopyridin-1(2*H*)-yl]propansäure-*tert*.-butylester (Racemat) mit TFA verseift. Ausbeute: 1.5 g (94% d. Th.)
LC/MS [Methode 1]: $R_t$ = 0.80 min; MS (ESIpos): m/z = 303 $(M+H)^+$
$^1$H-NMR (400 MHz, DMSO-$d_6$): δ [ppm] = 13.04 (br. s, 1H), 8.04 (d, 1H), 7.88 (d, 1H), 7.82 (d, 1H), 7.76 (dd, 1H), 6.65 (d, 1H), 6.51 (dd,1H), 5.23 (q, 1H), 1.60 (d, 3H).

**Beispiel 2.2C**

4-({2-[4-(5-Chlor-2-cyanophenyl)-2-oxopyridin-1(2*H*)-yl]propanoyl}amino)benzosäure-*tert*.-butylester (Racemat)

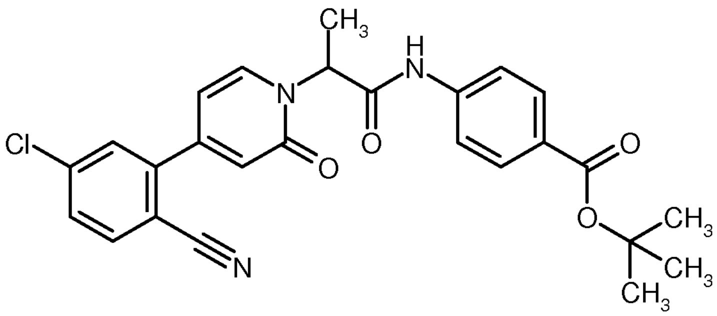

Nach der allgemeinen Methode 5A wurden 76 mg (83% Reinheit, 0.21 mmol) 2-[4-(5-Chlor-2-cyanophenyl)-2-oxopyridin-1(2*H*)-yl]propansäure (Racemat) mit 1.1 eq. 4-Aminobenzoesäure-*tert*.-butylester umgesetzt. Ausbeute: 43 mg (43% d. Th.)
LC/MS [Methode 1]: $R_t$ = 1.20 min; MS (ESIpos): m/z = 478 $(M+H)^+$

**Beispiel 2.3A**

5-[4-({2-[4-(5-Chlor-2-cyanophenyl)-2-oxopyridin-1(2*H*)-yl]propanoyl}amino)phenyl]-3-oxo-2,3-dihydro-1*H*-pyrazol-1-carbonsäure-*tert*.-butylester (Racemat)

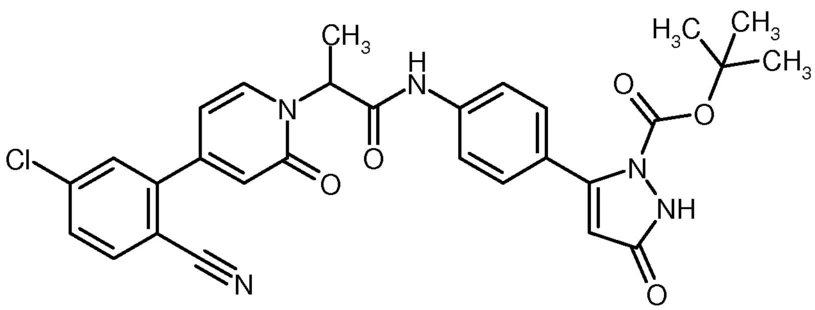

Nach der allgemeinen Methode 5A wurden 120 mg (93% Reinheit, 0.37 mmol) 2-[4-(5-Chlor-2-cyanophenyl)-2-oxopyridin-1(2*H*)-yl]propansäure (Racemat) mit 1.1 eq. 5-(4-Aminophenyl)-3-oxo-2,3-dihydro-1*H*-pyrazol-1-carbonsäure-*tert.*-butylester umgesetzt. Ausbeute: 110 mg (53% d. Th.)
LC/MS [Methode 1]: $R_t$ = 1.10 min; MS (ESIpos): m/z = 560 $(M+H)^+$
$^1$H-NMR (400 MHz, DMSO-$d_6$): $\delta$ [ppm] = 12.95 (s, 1H), 10.61 (s, 1H), 8.05 (d, 1H), 7.96 (d, 1H), 7.83 (d, 1H), 7.77 (dd, 1H), 7.70 (m, 4H), 6.67 (d, 1H), 6.56 (dd, 1H), 6.49 (d, 1H), 5.59 (q, 1H), 1.70 (d, 3H), 1.50 (s, 9H).

**Beispiel 2.4A**

4-({2-[4-(5-Chlor-2-cyanophenyl)-2-oxopyridin-1(2*H*)-yl]propanoyl}amino)-2-fluorbenzoesäuremethylester (Racemat)

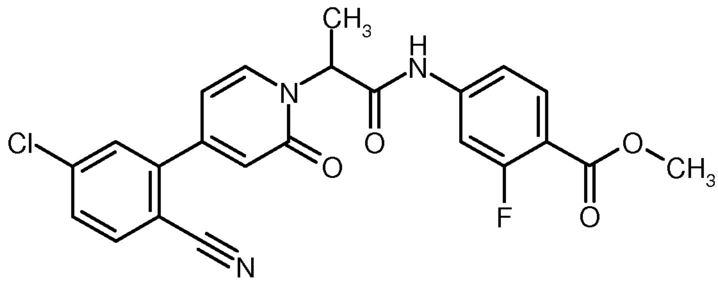

Nach der allgemeinen Methode 5A wurden 120 mg (0.39 mmol) 2-[4-(5-Chlor-2-cyanophenyl)-2-oxopyridin-1(2*H*)-yl]propansäure (Racemat) mit 1.1 eq. 4-Amino-2-fluorbenzoesäure-methylester umgesetzt. Ausbeute: 64 mg (36% d. Th.)
LC/MS [Methode 1]: $R_t$ = 1.03 min; MS (ESIpos): m/z = 454 $(M+H)^+$

**Beispiel 2.5A**

2-Chlor-4-({2-[4-(5-chlor-2-cyanophenyl)-2-oxopyridin-1(2*H*)-yl]propanoyl}amino)benzoesäuremethylester (Racemat)

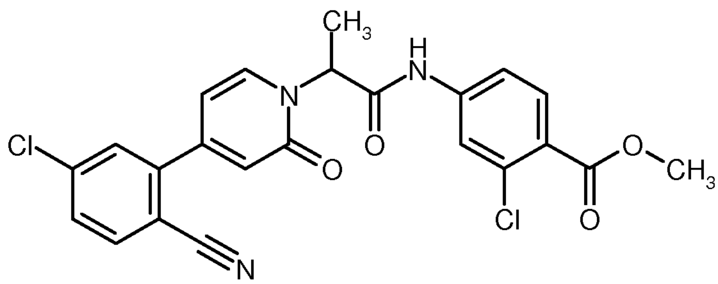

Nach der allgemeinen Methode 5A wurden 120 mg (0.39 mmol) 2-[4-(5-Chlor-2-cyanophenyl)-2-oxopyridin-1(2*H*)-yl]propansäure (Racemat) mit 1.1 eq. 4-Amino-2-chlorbenzoesäure-methylester umgesetzt. Nach der wässrigen Aufarbeitung wurde das gewünschte Produkt mit einem Gemisch aus etwas Wasser, Acetonitril und DMF ausgefällt. Ausbeute: 69 mg (38% d. Th.)
LC/MS [Methode 1]: $R_t$ = 1.07 min; MS (ESIpos): m/z = 470 $(M+H)^+$

**Beispiel 2.6A**

4-({2-[4-(5-Chlor-2-cyanophenyl)-2-oxopyridin-1(2*H*)-yl]propanoyl}amino)-2-methylbenzoesäuremethylester (Racemat)

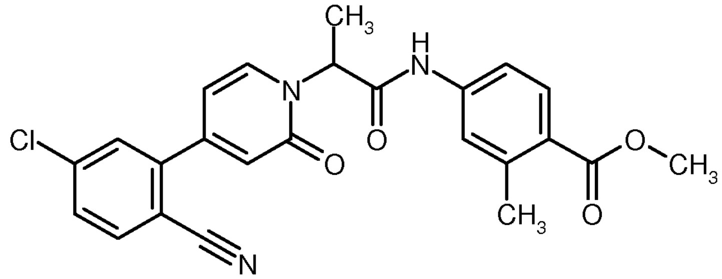

Nach der allgemeinen Methode 5A wurden 120 mg (0.39 mmol) 2-[4-(5-Chlor-2-cyanophenyl)-2-oxopyridin-1(2*H*)-yl]propansäure (Racemat) mit 1.1 eq. 4-Amino-2-methylbenzoesäuremethylester umgesetzt. Nach der wässrigen Aufarbeitung wurde das gewünschte Produkt mit einem Gemisch aus etwas Wasser, Acetonitril und DMF ausgefällt. Ausbeute: 120 mg (69% d. Th.)
LC/MS [Methode 1]: $R_t$ = 1.06 min; MS (ESIpos): m/z = 450 $(M+H)^+$
$^1$H-NMR (400 MHz, DMSO-$d_6$): δ [ppm] = 10.69 (s, 1H), 8.04 (d, 1H), 7.94 (d, 1H), 7.85 (d, 1H), 7.82 (d, 1H), 7.77 (dd, 1H), 7.57 (m, 2H), 6.66 (d, 1H), 6.56 (dd, 1H), 5.56 (q, 1H), 3.8 (s, 3H), 1.69 (d, 3H).

**Beispiel 2.7A**

6-({2-[4-(5-Chlor-2-cyanophenyl)-2-oxopyridni-1(2*H*)-yl]propanoyl}amino)-2-methyl-3-oxo-2,3-dihydro-1*H*-indazol-1-carbonsäure-*tert*.-butylester (Racemat)

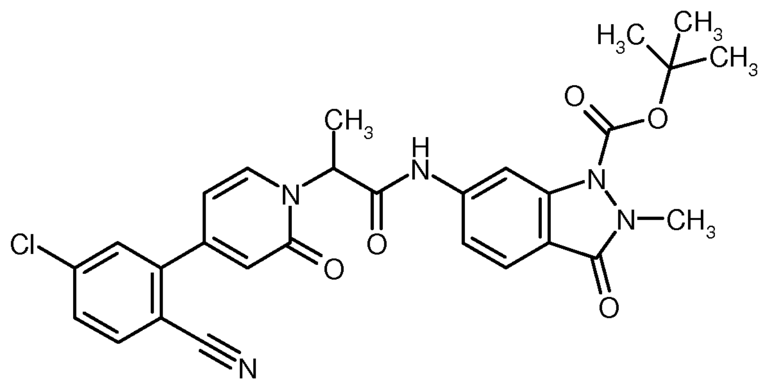

Nach der allgemeinen Methode 5A wurden 89 mg (83% Reinheit, 0.24 mmol) 2-[4-(5-Chlor-2-cyanophenyl)-2-oxopyridin-1(2*H*)-yl]propansäure (Racemat) mit 1.1 eq. 6-Amino-2-methyl-3-oxo-2,3-dihydro-1*H*-indazol-1-carbonsäure-*tert*.-butylester umgesetzt. Das Rohprodukt wurde mittels präparativer HPLC (Reprosil C18, Wasser-Methanol-Gradient) aufgereinigt. Ausbeute: 75 mg (56% d. Th.)
LC/MS [Methode 1]: $R_t$ = 1.04 min; MS (ESIpos): m/z = 548 $(M+H)^+$

**Beispiel 2.8A**

[(2-Brom-4-chlorphenyl)ethinyl](trimethyl)silan

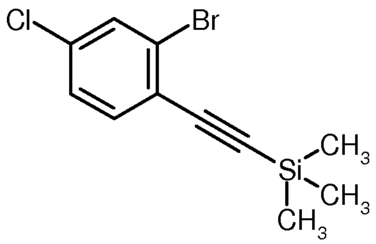

Eine Lösung von 73 mg (0.10 mmol, 0.025 eq.) Bis(triphenylphosphin)palladium(II)chlorid und 20 mg (0.10 mmol, 0.025 eq.) Kupfer(I)iodid in 27 ml THF wurde unter Argon nacheinander mit 2.89 ml (20.7 mmol, 5.0 eq.) Triethylamin, 2.99 g (4.1 mmol) 2-Brom-4-chlor-1-iodbenzol und 489 mg (4.97 mmol, 1.2 eq.) Ethinyl(trimethyl)silan versetzt und über Nacht bei RT gerührt. Das Reaktionsgemisch wurde anschließend mit Ethylacetat verdünnt, über Celite filtriert und das Filtrat im Vakuum eingeengt. Nach Zugabe von Ethylacetat/Wasser und Phasentrennung wurde die organische Phase im Vakuum eingeengt. Das Rohprodukt wurde mittels Flash-Chromatographie (Kieselgel-60, Eluent: Cyclohexan-Ethylacetat-Gemische) aufgereinigt. Ausbeute: 3.21 g (quant.)
$^1$H-NMR (400 MHz, DMSO-$d_6$): δ [ppm] = 7.70 (d, 1H), 7.40 (d, 1H), 7.29 (dd, 1H), 0.07 (s, 9H).

**Beispiel 2.8B**

4-{5-Chlor-2-[(trimethylsilyl)ethinyl]phenyl}-2-methoxypyridin

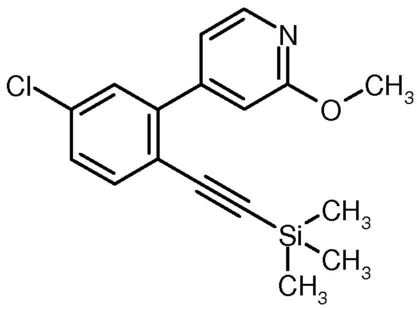

Eine Lösung von 333 mg (1.16 mmol) [(2-Brom-4-chlorphenyl)ethinyl](trimethyl)silan, 195 mg (1.28 mmol, 1.1 eq.) (2-Methoxypyridin-4-yl)boronsäure, 401 mg (2.9 mmol, 2.5 eq.) Kaliumcarbonat und 14 mg (0.02 mmol, 0.015 eq.) [1,1-Bis-(diphenylphosphino)-ferrocen]-palladium(II)chlorid-Monodichlormethanaddukt in 18 ml Dioxan wurde für 15 min bei 130°C in der Mikrowelle bestrahlt. Anschließend wurde das Reaktionsgemisch über Celite filtiert und der Rückstand mit Dioxan nachgewaschen. Die vereinigten Filtrate wurden im Vakuum eingeengt. Nach Zugabe von Wasser/Ethylacetat und Phasentrennung wurde die organische Phase im Vakuum eingeengt. Ausbeute: 550 mg (41% Reinheit, 62% d. Th.)
LC/MS [Methode 1]: $R_t$ = 1.50 min; MS (ESIpos): m/z = 316 $(M+H)^+$

**Beispiel 2.8C**

4-{5-Chlor-2-[(trimethylsilyl)ethinyl]phenyl}pyridin-2(1*H*)-on

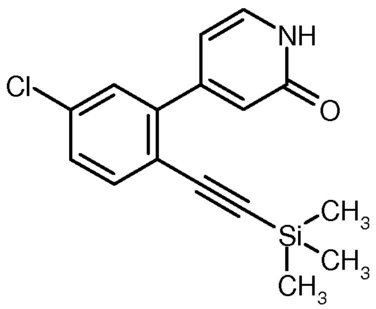

Nach der allgemeinen Methode 3A wurden 550 mg (41% Reinheit, 0.71 mmol) 4-{5-Chlor-2-[(trimethylsilyl)ethinyl]phenyl}-2-methoxypyridin mit 20 eq. Pyridinium-Hydrochlorid umgesetzt. Das Reaktionsgemisch wurde im Vakuum eingeengt und der Rückstand mit Wasser versetzt. Nach Zugabe von Ethylacetat und Phasentrennung wurde die organische Phase einmal mit Wasser gewaschen, getrocknet (Natriumsulfat), filtriert und im Vakuum eingeengt. Das Rohprodukt wurde mittels Flash-Chromatographie (Kieselgel-60, Cyclohexan-Ethylacetat- und Dichlormethan-Methanol-Gemische) aufgereinigt. Ausbeute: 141 mg (91% Reinheit, 59% d. Th.)
LC/MS [Methode 1]: $R_t$ = 1.11 min; MS (ESIpos): m/z = 302 $(M+H)^+$

**Beispiel 2.8D**

2-[4-{5-Chlor-2-[(trimethylsilyl)ethinyl]phenyl}-2-oxopyridin-1(2*H*)-yl]propansäure-*tert.*-butylester (Racemat)

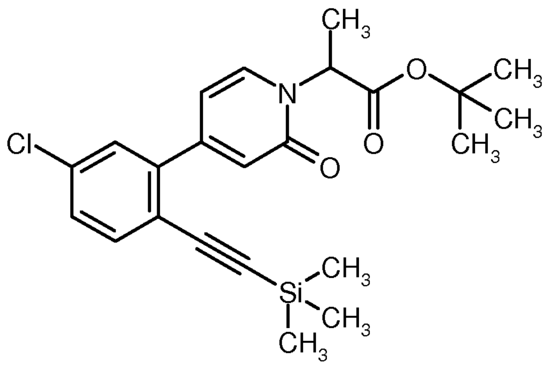

Nach der allgemeinen Methode 4B wurden 125 mg (91% Reinheit, 0.38 mmol) 4-{5-Chlor-2-[(trimethylsilyl)ethinyl]phenyl}pyridin-2(1*H*)-on mit 1.2 eq. 2-Brompropansäure-*tert.*-butylester (Racemat) bei 80°C umgesetzt. Ausbeute: 56 mg (89% Reinheit, 31% d. Th.)
LC/MS [Methode 1]: $R_t$ = 1.42 min; MS (ESIpos): m/z = 430 $(M+H)^+$

**Beispiel 2.8E**

2-[4-(5-Chlor-2-ethinylphenyl)-2-oxopyridin-1(2*H*)-yl]propansäure (Racemat)

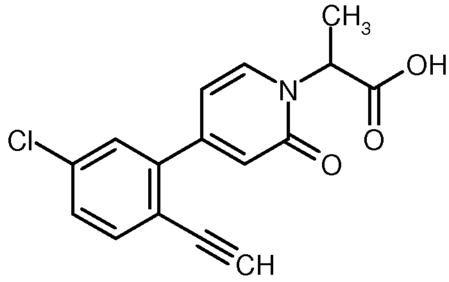

Nach der allgemeinen Methode 6A wurden 55 mg (89% Reinheit, 0.11 mmol) 2-[4-{5-Chlor-2-[(trimethylsilyl)ethinyl]phenyl}-2-oxopyridin-1(2*H*)-yl]propansäure-*tert*.-butylester (Racemat) mit TFA verseift. Ausbeute: 50 mg (82% Reinheit, quant.)
LC/MS [Methode 1]: $R_t$ = 0.90 min; MS (ESIpos): m/z = 301 $(M+H)^+$

**Beispiel 2.8F**

4-({2-[4-(5-Chlor-2-ethinylphenyl)-2-oxopyridin-1(2*H*)-yl]propanoyl}amino)benzoesäuremethylester (Racemat)

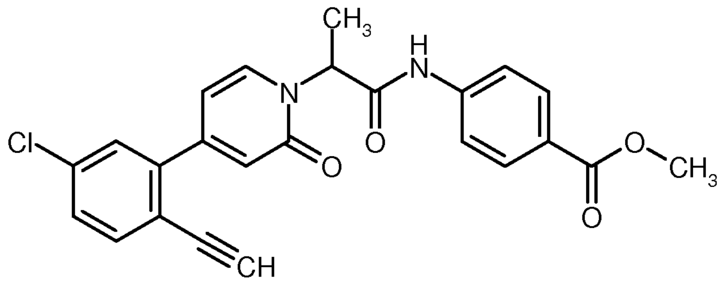

Nach der allgemeinen Methode 5A wurden 50 mg (82% Reinheit, 0.32 mmol) 2-[4-(5-Chlor-2-ethinylphenyl)-2-oxopyridin-l(2*H*)-yl]propansäure (Racemat) mit 1.2 eq. 4-Aminobenzoesäuremethylester umgesetzt. Ausbeute: 15 mg (25% d. Th.)
LC/MS [Methode 1]: $R_t$ = 1.11 min; MS (ESIpos): m/z = 435 $(M+H)^+$

**Beispiel 2.9A**

2-[4-(2,5-Dichlorphenyl)-2-oxopyridin-1(2*H*)-yl]propansäure-*tert*.-butylester (Racemat)

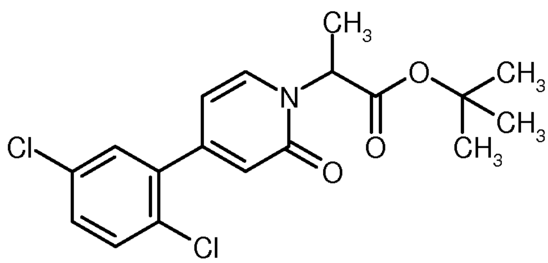

Nach der allgemeinen Methode 2A wurden 2.5 g (8.0 mmol) 2-(4-Brom-2-oxopyridin-1(2*H*)-yl)propansäure-*tert*.-butylester (Racemat) mit 1.76 g (9.2 mmol) 2,5-Dichlorphenylboronsäure in Gegenwart von Tetrakis(triphenylphosphin)palladium(0) umgesetzt. Ausbeute: 2.3 g (77% d. Th.)
LC/MS [Methode 1]: $R_t$ = 1.20 min; MS (ESIpos): m/z = 368 $(M+H)^+$

**Beispiel 2.9B**

2-[4-(2,5-Dichlorphenyl)-2-oxopyridin-1(2*H*)-yl]propansäure (Racemat)

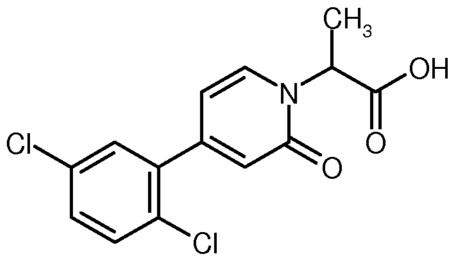

Eine Lösung von 2.3 g (6.2 mmol) 2-[4-(2,5-Dichlorphenyl)-2-oxopyridin-1(2*H*)-yl]propansäure-*tert*.-butylester (Racemat) in einer 4 molaren Salzsäure/Dioxan-Lösung wurde 7 h bei RT gerührt und anschließend im Vakuum eingeengt. Der Rückstand wurde dreimal mit Dichlormethan coevaporiert und im Vakuum getrocknet. Ausbeute: 2.0 g (99% d. Th.)
LC/MS [Methode 1]: $R_t$ = 0.88 min; MS (ESIpos): m/z = 312 $(M+H)^+$
$^1$H-NMR (400 MHz, DMSO-$d_6$): $\delta$ [ppm] = 13.03 (br. s, 1H), 7.78 (d, 1H), 7.63 (d, 1H), 7.57 (d, 1H), 7.54 (dd, 1H), 6.46 (d, 1H), 6.36 (dd,1 H), 5.21 (q, 1H), 1.58 (d, 3H).

**Beispiel 2.9C**

4-({2-[4-(2,5-Dichlorphenyl)-2-oxopyridin-1(2*H*)-yl]propanoyl}amino)benzoesäure-*tert*.-butylester (Racemat)

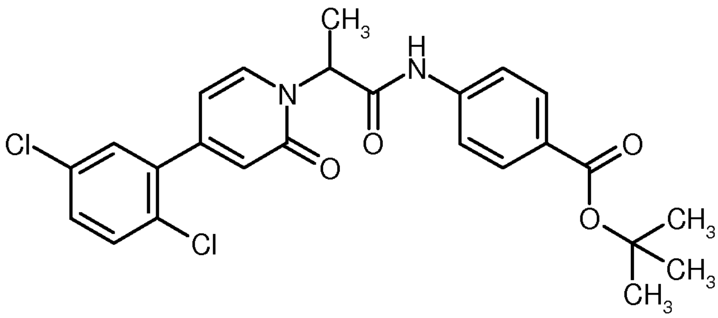

Nach der allgemeinen Methode 5A wurden 117 mg (0.36 mmol) 2-[4-(2,5-Dichlorphenyl)-2-oxopyridin-1(2*H*)-yl]propansäure (Racemat) mit 1.2 eq. 4-Aminobenzoesäure-*tert*.-butylester umgesetzt. Ausbeute: 83 mg (47% d. Th.)
LC/MS [Methode 1]: $R_t$ = 1.32 min; MS (ESIpos): m/z = 487 $(M+H)^+$

**Beispiel 2.10A**

2-[4-(2-Brom-5-chlorphenyl)-2-oxopyridin-1(2*H*)-yl]propansäure-*tert.*-butylester (Racemat)

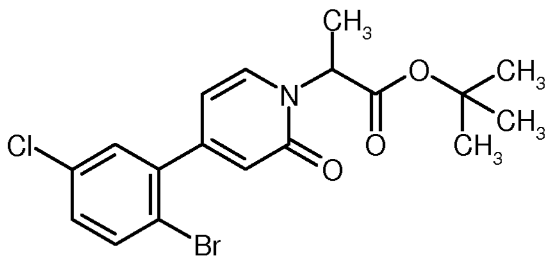

Nach der allgemeinen Methode 2A wurden 856 mg (2.75 mmol) 2-(4-Brom-2-oxopyridin-1(2*H*)-yl)propansäure-*tert.*-butylester (Racemat) mit 776 mg (3.3 mmol) 2-Brom-5-chlorphenylboronsäure in Gegenwart von Tetrakis(triphenylphosphin)palladium(0) umgesetzt. Ausbeute: 921 mg (80% d. Th.)
LC/MS [Methode 1]: $R_t$ = 1.21 min; MS (ESIpos): m/z = 412 $(M+H)^+$

**Beispiel 2.10B**

2-[4-(2-Brom-5-chlorphenyl)-2-oxopyridin-1(2*H*)-yl]propansäure (Racemat)

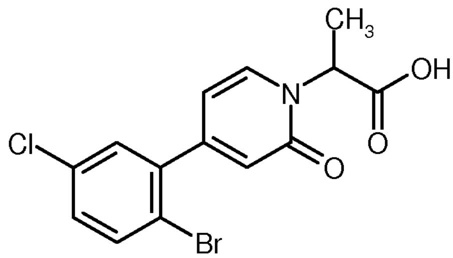

Nach der allgemeinen Methode 6A wurden 920 mg (2.2 mmol) 2-[4-(2-Brom-5-chlorphenyl)-2-oxopyridin-1(2*H*)-yl]propansäure-*tert.*-butylester (Racemat) mit TFA verseift. Ausbeute: 1110 mg (93% Reinheit, quant.)
LC/MS [Methode 1]: $R_t$ = 0.91 min; MS (ESIpos): m/z = 356 $(M+H)^+$

**Beispiel 2.10C**

4-({2-[4-(2-Brom-5-chlorphenyl)-2-oxopyridin-1(2*H*)-yl]propanoyl}amino)benzoesäure-*tert.*-butylester (Racemat)

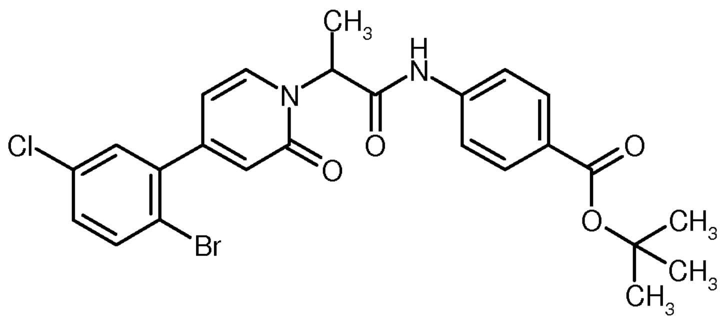

Nach der allgemeinen Methode 5A wurden 153 mg (93% Reinheit, 0.4 mmol) 2-[4-(2-Brom-5-chlorphenyl)-2-oxopyridin-1(2*H*)-yl]propansäure (Racemat) mit 1.2 eq. 4-Aminobenzoesäure-*tert.*-butylester umgesetzt. Ausbeute: 96 mg (44% d. Th.)
LC/MS [Methode 1]: $R_t$ = 1.32 min; MS (ESIpos): m/z = 531 $(M+H)^+$

**Beispiel 2.11A**

2-{4-[5-Chlor-2-(trifluormethyl)phenyl]-2-oxopyridin-1(2*H*)-yl}propansäure-*tert.*-butylester (Racemat)

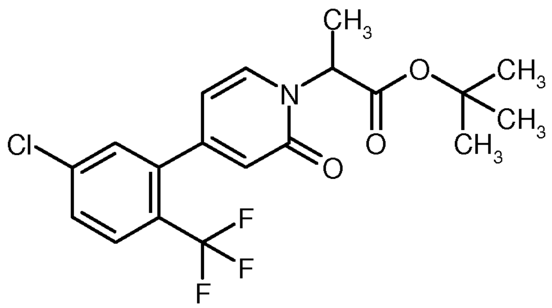

Nach der allgemeinen Methode 2A wurden 2.0 g (6.4 mmol) 2-(4-Brom-2-oxopyridin-1(2*H*)-yl)propansäure-*tert*.-butylester (Racemat) mit 1.7 g (7.7 mmol) 5-Chlor-2-(trifluormethyl)-phenylboronsäure in Gegenwart von Tetrakis(triphenylphosphin)palladium(0) umgesetzt. Ausbeute: 2.3 g (91% Reinheit, 82% d. Th.)
LC/MS [Methode 1]: $R_t$ = 1.22 min; MS (ESIpos): m/z = 402 $(M+H)^+$

**Beispiel 2.11B**

2-{4-[5-Chlor-2-(trifluormethyl)phenyl]-2-oxopyridin-1(2*H*)-yl}propansäure (Racemat)

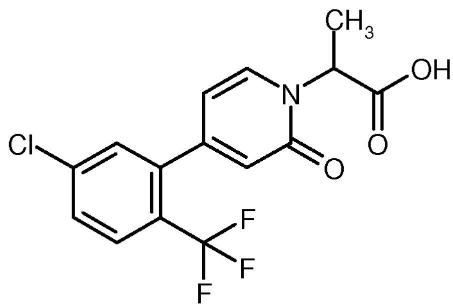

Nach der allgemeinen Methode 6A wurden 2.3 g (91% Reinheit, 5.2 mmol) 2-[4-(5-Chlor-2-cyanophenyl)-2-oxopyridin-1(2*H*)-yl]propansäure-*tert*.-butylester (Racemat) mit TFA verseift. Ausbeute: 2.6 g (93% Reinheit, quant.)
LC/MS [Methode 1]: $R_t$ = 0.91 min; MS (ESIpos): m/z = 346 $(M+H)^+$

**Beispiel 2.11C**

4-[(2-{4-[5-Chlor-2-(trifluormethyl)phenyl]-2-oxopyridin-1(2*H*)-yl}propanoyl)amino]benzoesäure-*tert*.-butylester (Racemat)

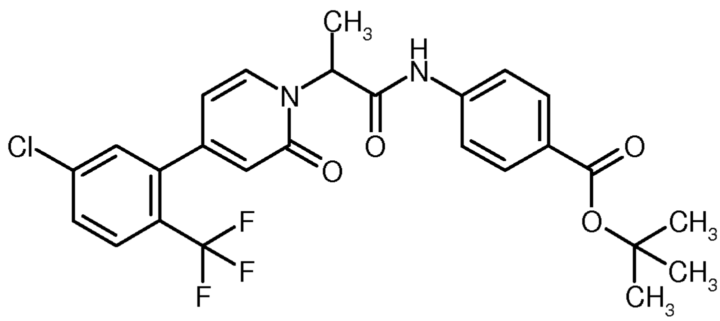

Nach der allgemeinen Methode 5A wurden 130 mg (93% Reinheit, 0.35 mmol) 2-{4-[5-Chlor-2-(trifluormethyl)phenyl]-2-oxopyridin-1(2*H*)-yl}propansäure (Racemat) mit 1.2 eq. 4-Aminobenzosäure-tert.-butylester umgesetzt.

Das Rohprodukt wurde mittels präparativer HPLC (Reprosil C18, Wasser-Methanol-Gradient) aufgereinigt. Ausbeute: 104 mg (56% d. Th.)
LC/MS [Methode 1]: $R_t$ = 1.33 min; MS (ESIpos): m/z = 521 $(M+H)^+$

**Beispiel 3.1A**

2-(4-Brom-2-oxopyridin-1(2*H*)-yl)butansäure-*tert*.-butylester (Racemat)

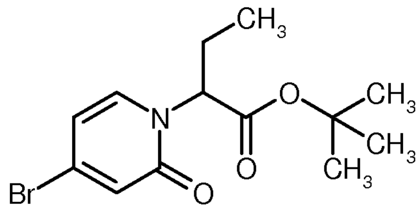

Nach der allgemeinen Methode 4B wurden 348 mg (2.0 mmol) 4-Brompyridin-2(1*H*)-on mit 1.2 eq. 2-Brombutansäure-*tert*.-butylester (Racemat) bei 120°C umgesetzt. Nach wässriger Aufarbeitung wurde das gewünschte Produkt als Rohprodukt weiter umgesetzt. Ausbeute: 608 mg (82% Reinheit, 79% d. Th.)
LC/MS [Methode 1]: $R_t$ = 0.99 min; MS (ESIpos): m/z = 316 $(M+H)^+$

**Beispiel 3.1B**

2-[4-(5-Chlor-2-cyanophenyl)-2-oxopyridin-1(2*H*)-yl]butansäure-*tert*.-butylester (Racemat)

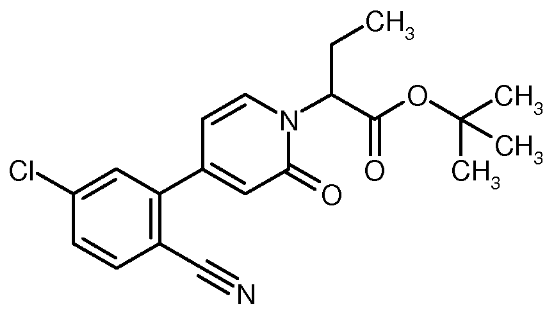

Nach der allgemeinen Methode 2A wurden 600 mg (82% Reinheit, 1.56 mmol) 2-(4-Brom-2-oxopyridin-1(2*H*)-yl)butansäure-*tert*.-butylester (Racemat) mit 325 mg (1.8 mmol) 5-Chlor-2-cyanophenylboronsäure in Gegenwart von Tetrakis(triphenylphosphin)palladium(0) umgesetzt. Ausbeute: 543 mg (59% Reinheit, 55% d. Th.)
LC/MS [Methode 1]: $R_t$ = 1.10 min; MS (ESIpos): m/z = 373 $(M+H)^+$

**Beispiel 3.1C**

2-[4-(5-Chlor-2-cyanophenyl)-2-oxopyridin-1(2*H*)-yl]butansäure (Racemat)

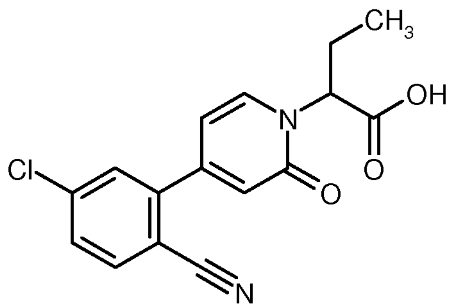

Nach der allgemeinen Methode 6A wurden 543 mg (59% Reinheit, 0.86 mmol) 2-[4-(5-Chlor-2-cyanophenyl)-2-oxopyridin-1(2*H*)-yl]butansäure-*tert*.-butylester (Racemat) mit 20 eq. TFA verseift. Ausbeute: 425 mg (60% Reinheit, 94% d. Th.)
LC/MS [Methode 1]: $R_t$ = 0.78 min; MS (ESIpos): m/z = 317 $(M+H)^+$

**Beispiel 4.1A**

2-(4-Brom-2-oxopyridin-1(2*H*)-yl)-3-methylbutansäureethylester (Racemat)

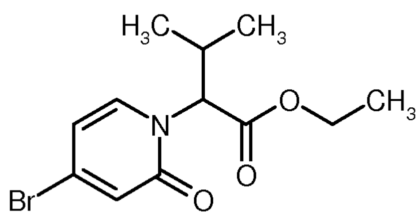

Nach der allgemeinen Methode 4C wurden 500 mg (2.9 mmol) 4-Brompyridin-2(1*H*)-on mit 841 mg (4.02 mmol) 2-Brom-3-methylbutansäureethylester (Racemat) in Gegenwart von 1.15 eq. Natriumhydrid und 2.3 eq. Lithiumbromid umgesetzt. Ausbeute: 260 mg (92% Reinheit, 28% d. Th.)
LC/MS [Methode 3]: $R_t$ = 2.05 min; MS (ESIpos): m/z = 302 $(M+H)^+$

**Beispiel 4.1B**

2-[4-(5-Chlor-2-cyanophenyl)-2-oxopyridin-1(2*H*)-yl]-3-methylbutansäureethylester (Racemat)

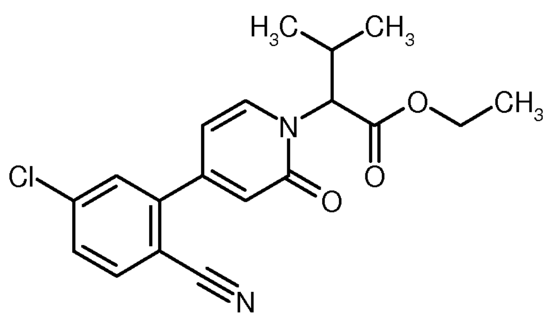

Nach der allgemeinen Methode 2A wurden 240 mg (92% Reinheit, 0.73 mmol) 2-(4-Brom-2-oxopyridin-1(2*H*)-yl)-3-methylbutansäureethylester (Racemat) mit 172 mg (0.95 mmol) 5-Chlor-2-cyanophenylboronsäure in Gegenwart von [1,1-Bis-(diphenylphosphino)-ferrocen]-palladium(II)chlorid-Monodichlormethanaddukt umgesetzt. Ausbeute: 117 mg (81% Reinheit, 36% d. Th.)
LC/MS [Methode 1]: $R_t$ = 1.10 min; MS (ESIpos): m/z = 359 $(M+H)^+$

**Beispiel 4.1C**

2-[4-(5-Chlor-2-cyanophenyl)-2-oxopyridin-1(2*H*)-yl]-3-methylbutansäure (Racemat)

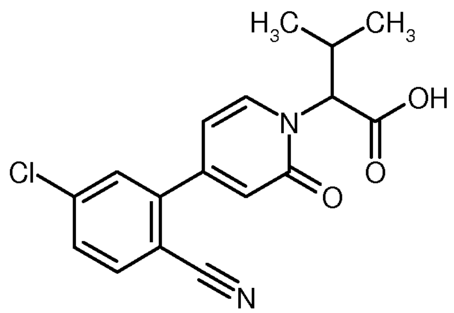

Nach der allgemeinen Methode 6B wurden 117 mg (81% Reinheit, 0.26 mmol) 2-[4-(5-Chlor-2-cyanophenyl)-2-oxopyridin-1(2*H*)-yl]-3-methylbutansäureethylester (Racemat) mit Lithiumhydroxid verseift. Ausbeute: 79 mg (86% Reinheit, 78% d. Th.)
LC/MS [Methode 1]: Rt = 0.89 min; MS (ESIpos): m/z = 331 $(M+H)^+$

**Beispiel 5.1A**

2-(4-Iod-2-oxopyridin-1(2*H*)-yl)hexansäureethylester (Racemat)

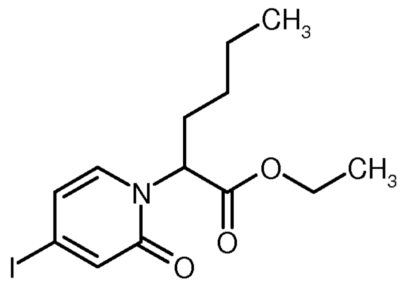

Nach der allgemeinen Methode 4C wurden 500 mg (2.3 mmol) 4-Iodpyridin-2(1*H*)-on mit 706 mg (3.2 mmol) 2-Bromhexansäureethylester (Racemat) in Gegenwart von 1.15 eq. Natriumhydrid und 2.3 eq. Lithiumbromid umgesetzt. Ausbeute: 352 mg (43% d. Th.)
LC/MS [Methode 1]: $R_t$ = 1.08 min; MS (ESIpos): m/z = 364 $(M+H)^+$
$^1$H-NMR (400 MHz, DMSO-$d_6$): $\delta$ [ppm] = 7.45 (d, 1H), 6.96 (d, 1H), 6.63 (dd, 1H), 5.10 (dd, 1H), 4.10 (q, 1H), 2.03 (m, 2H), 1.24 (m, 3H), 1.15 (t, 3H), 1.02 (m, 1H), 0.84 (t, 3H).

**Beispiel 5.1B**

2-[4-(5-Chlor-2-cyanophenyl)-2-oxopyridin-1(2*H*)-yl]hexansäureethylester (Racemat)

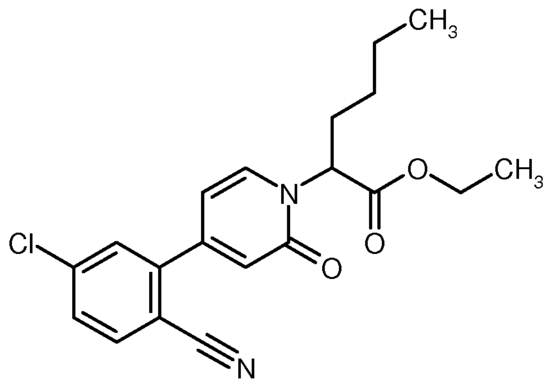

Nach der allgemeinen Methode 2A wurden 150 mg (0.41 mmol) 2-(4-Iod-2-oxopyridin-1(2*H*)-yl)hexansäureethylester (Racemat) mit 97 mg (0.53 mmol) 5-Chlor-2-cyanophenylboronsäure in Gegenwart von [1,1-Bis-(diphenylphosphino)-ferrocen]-palladium(II)chlorid-dichlormethan-Monoaddukt umgesetzt. Ausbeute: 114 mg (95% Reinheit, 70% d. Th.)
LC/MS [Methode 1]: $R_t$ = 1.15 min; MS (ESIpos): m/z = 373 $(M+H)^+$

**Beispiel 5.1C**

2-[4-(5-Chlor-2-cyanophenyl)-2-oxopyridin-1(2*H*)-yl]hexansäure (Racemat)

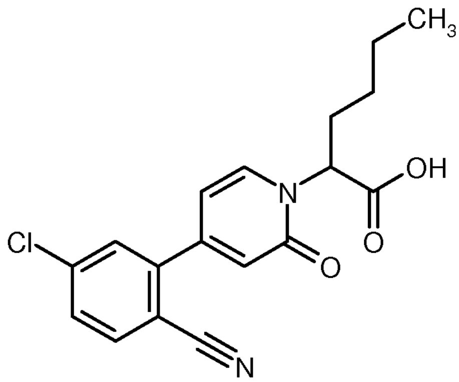

Nach der allgemeinen Methode 6B wurden 113 mg (95% Reinheit, 0.29 mmol) 2-[4-(5-Chlor-2-cyanophenyl)-2-oxopyridin-1(2*H*)-yl]hexansäureethylester (Racemat) mit Lithiumhydroxid verseift. Ausbeute: 64 mg (78% Reinheit, 50% d. Th.)
LC/MS [Methode 1]: $R_t$ = 0.98 min; MS (ESIpos): m/z = 345 $(M+H)^+$

**Beispiel 6.1A**

Brom(cyclopropylmethyl)magnesium

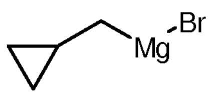

1.2 g (48.1 mmol) Magnesiumspäne wurden in 30 ml THF vorlegt, mit einer Spatelspitze Iod versetzt und eine Lösung von 6.5 g (48.1 mmol, 1.0 eq.) Brommethylcyclopropan in 15 ml THF langsam zugetropft. Anschließend wurde das Reaktionsgemisch 2 h unter Rückfluß gerührt. Nach dem Abkühlen auf RT wurde die Reaktionslösung von den

übrig gebliebenen Magnesiumspänen dekantiert und die Rohlösung weiter umgesetzt.

**Beispiel 6.1B**

3-Cyclopropyl-2-hydroxypropansäureethylester (Racemat)

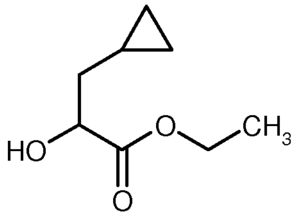

Eine Lösung von 5.4 g (50% Reinheit, 26.7 mmol) Ethyloxoacetat in 50 ml THF wurde unter Argon und Eiskühlung zügig zu 7.6 g (48 mmol, 1.8 eq.) Brom(cyclopropylmethyl)magnesium getropft. Das Reaktionsgemisch wurde 48 h nachgerührt, mit Ethylacetat verdünnt und mit Wasser gequencht. Das Reaktionsgemisch wurde mit Celite versetzt, 5 min gerührt und filtriert. Nach Phasentrennung wurde die organische Phase einmal mit Wasser gewaschen, getrocknet (Natriumsulfat), filtriert und im Vakuum eingeengt. Ausbeute: 2.6 g (60% d. Th.)
GC [Methode 7]: $R_t$ = 2.49 min; MS (EI): m/z = 158 $(M)^+$

**Beispiel 6.1C**

3-Cyclopropyl-2-[(methylsulfonyl)oxy]propansäureethylester (Racemat)

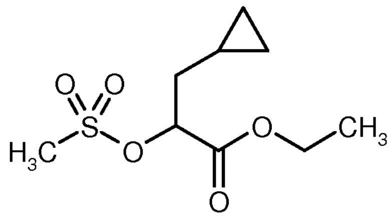

Eine Lösung von 1.9 g (40% Reinheit, 4.8 mmol) 3-Cyclopropyl-2-hydroxypropansäureethylester (Racemat) in 100 ml Dichlormethan wurde bei RT mit 2 ml (11.5 mmol, 2.4 eq.) *N*-Ethyl-*N*-(propan-2-yl)propan-2-amin und bei 0°C zügig mit 0.45 ml (5.8 mmol, 1.2 eq.) Methansulfonylsäurechlorid versetzt. Das Reaktionsgemisch wurde 2 h bei RT gerührt und mit Eis gequencht. Nach Phasentrennung wurde die organische Phase dreimal mit Wasser und einmal mit gesättigter Natriumchlorid-Lösung gewaschen. Die vereinigten, organischen Phasen wurden getrocknet (Natriumsulfat), filtriert und im Vakuum eingeengt. Das Rohprodukt wurde ohne weitere Reinigung in die nächste Stufe eingesetzt.

**Beispiel 6.1D**

2-(4-Brom-2-oxopyridin-1(2*H*)-yl)-3-cyclopropylpropansäureethylester (Racemat)

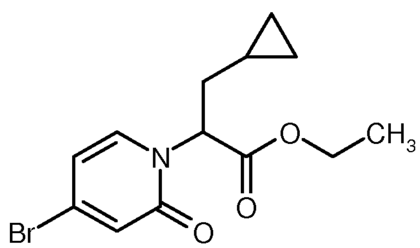

Nach der allgemeinen Methode 4C wurden 1.02 g (5.87 mmol) 4-Brompyridin-2(1*H*)-on mit 2.23 g (56% Reinheit, 5.28 mmol) 3-Cyclopropyl-2-[(methylsulfonyl)oxy]propansäureethylester (Racemat) in Gegenwart von 1.15 eq. Natriumhydrid und 2.3 eq. Lithiumbromid umgesetzt (über Nacht bei 65°C gerührt). Ausbeute: 246 mg (13% d. Th.)
$^1$H-NMR (400 MHz, DMSO-$d_6$): δ [ppm] = 7.67 (d, 1H), 6.76 (d, 1H), 6.54 (dd, 1H), 5.76 (m, 1H), 5.12 (dd, 1H), 4.97 (m, 2H), 4.11 (q, 2H), 2,15 (q, 2H), 1.92 (m, 2H), 1.15 (t, 3H).

**Beispiel 6.1E**

2-{4-[5-Chlor-2-(trifluormethyl)phenyl]-2-oxopyridin-1(2*H*)-yl}-3-cyclopropylpropansäureethylester (Racemat)

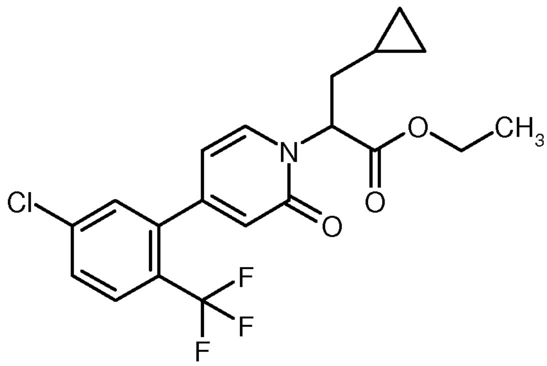

Nach der allgemeinen Methode 2A wurden 250 mg (0.8 mmol) 2-(4-Brom-2-oxopyridin-1(2*H*)-yl)-3-cyclopropylpropansäureethylester (Racemat) mit 214 mg (0.96 mmol) 5-Chlor-2-trifluormethylphenylboronsäure in Gegenwart von Tetrakis(triphenylphosphin)palladium(0) umgesetzt. Ausbeute: 35 mg (87% Reinheit, 9% d. Th.) der Titelverbindung und 70 mg (22% d. Th.) des bereits verseiften Produktes (Beispiel 6.1F)
LC/MS [Methode 1]: $R_t$ = 1.23 min; MS (ESIpos): m/z = 414 $(M+H)^+$

**Beispiel 6.1F**

2-{4-[5-Chlor-2-(trifluormethyl)phenyl]-2-oxopyridin-1(2*H*)-yl}-3-cyclopropylpropansäure (Racemat)

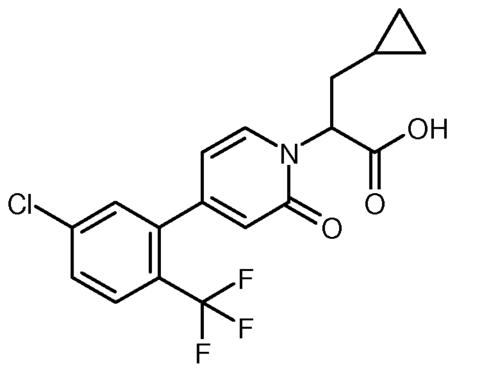

Nach der allgemeinen Methode 6B wurden 35 mg (87% Reinheit, 0.07 mmol) 2-{4-[5-Chlor-2-(trifluormethyl)phenyl]-2-oxopyridin-1(2*H*)-yl}-3-cyclopropylpropansäurethylester (Racemat) mit Lithiumhydroxid verseift. Ausbeute: 60 mg (88% Reinheit, quant.)
LC/MS [Methode 1]: $R_t$ = 1.05 min; MS (ESIpos): m/z = 386 $(M+H)^+$

**Beispiel 6.1G**

4-[(2-{4-[5-Chlor-2-(trifluormethyl)phenyl]-2-oxopyridin-1(2*H*)-yl}-3-cyclopropylpropanoyl)-amino] -benzoesäuremethylester (Racemat)

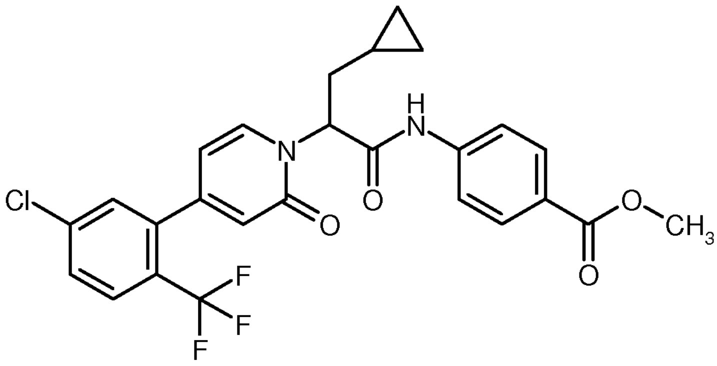

Nach der allgemeinen Methode 5A wurden 105 mg (94% Reinheit, 0.26 mmol) 2-{4-[5-Chlor-2-(trifluormethyl)phenyl]-2-oxopyridin-1(2*H*)-yl}-3-cyclopropylpropansäure (Racemat) mit 1.1 eq. 4-Aminobenzoesäuremethylester umgesetzt. Nach wässriger Aufarbeitung wurde das Rohprodukt ohne weitere Reinigung weiter umgesetzt. Ausbeute: 200 mg (48% Reinheit, 72% d. Th.)
LC/MS [Methode 1]: $R_t$ = 1.27 min; MS (ESIpos): m/z = 519 $(M+H)^+$

**Beispiel 6.2A**

2-(4-Brom-2-oxopyridin-1(2*H*)-yl)-3-cyclopropylpropansäure (Racemat)

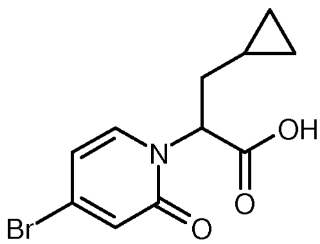

Nach der allgemeinen Methode 6B wurden 350 mg (1.1 mmol) 2-(4-Brom-2-oxopyridin-1(2*H*)-yl)-3-cyclopropylpropansäureethylester (Racemat) mit Lithiumhydroxid verseift. Ausbeute: 290 mg (94% Reinheit, 86% d. Th.)
LC/MS [Methode 1]: $R_t$ = 0.76 min; MS (ESIpos): m/z = 286 $(M+H)^+$
$^1$H-NMR (400 MHz, DMSO-$d_6$): $\delta$ [ppm] = 13.14 (s, 1H), 7.65 (d, 1H), 6.74 (d, 1H), 6.51 (dd, 1H), 5.75 (m, 1H), 5.11 (t, 1H), 4.98 (m, 2H), 2.15 (q, 2H), 1.91 (m, 2H).

**Beispiel 6.2B**

4-{[2-(4-Brom-2-oxopyridin-1(2*H*)-yl)-3-cyclopropylpropanoyl]amino}benzoesäure-*tert*.-butylester (Racemat)

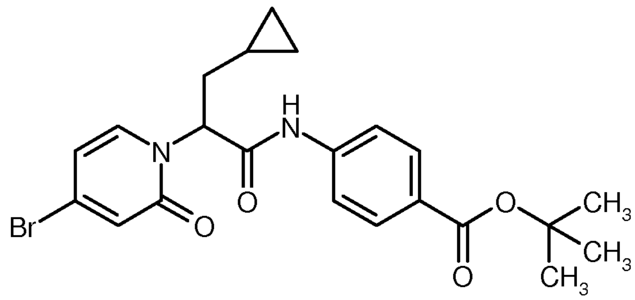

Nach der allgemeinen Methode 5A wurden 290 mg (94% Reinheit, 0.95 mmol) 2-(4-Brom-2-oxopyridin-1(2*H*)-yl)-3-cyclopropylpropansäure (Racemat) mit 1.1 eq. 4-Aminobenzoesäure-*tert*.-butylester umgesetzt. Ausbeute: 114 mg (80% Reinheit, 21% d. Th.)
LC/MS [Methode 1]: $R_t$ = 1.25 min; MS (ESIpos): m/z = 461 $(M+H)^+$

**Beispiel 6.2C**

4-[(2-{4-[5-Chlor-2-(trifluormethoxy)phenyl]-2-oxopyridin-1(2*H*)-yl}-3-cyclopropylpropanoyl)-amino] -benzoesäure-*tert*. -butylester (Racemat)

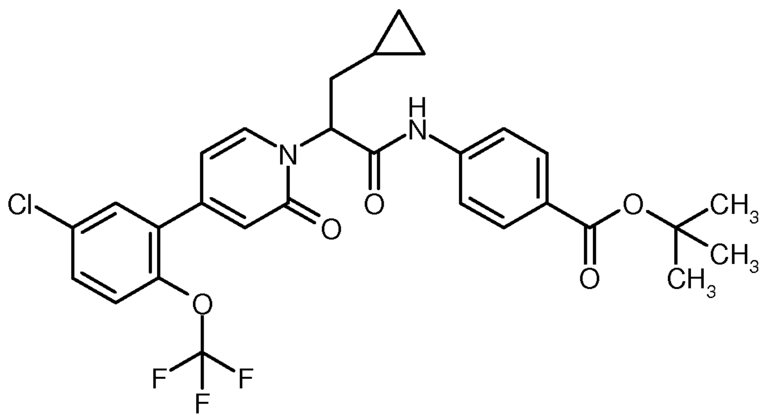

110 mg (80% Reinheit, 0.19 mmol) 4-{[2-(4-Brom-2-oxopyridin-1(2*H*)-yl)-3-cyclopropylpropanoyl]-amino}benzosäure-*tert*.-butylester (Racemat), 46 mg (0.19 mmol) 5-Chlor-2-trifluormethoxyphenylboronsäure und 22 mg (0.02 mmol) Tetrakis(triphenylphosphin)-palladium(0) wurden in 2.5 ml Dioxan und 2.5 ml gesättigter, wässriger Natriumcarbonat-Lösung aufgenommen und 12 min bei 130°C in der Mikrowelle bestrahlt. Das Rohprodukt wurde mittels Flash-Chromatographie (Kieselgel-60, Cyclohexan-Ethylacetat-Gemische) aufgereinigt. Ausbeute: 77 mg (92% Reinheit, 64% d. Th.)
LC/MS [Methode 1]: $R_t$ = 1.46 min; MS (ESIpos): m/z = 577 $(M+H)^+$

**Beispiel 7.1A**

2-(4-Brom-2-oxopyridin-1(2*H*)-yl)-3-phenylpropansäureethylester (Racemat)

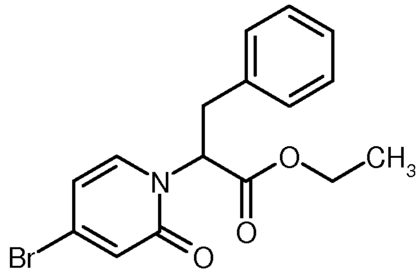

Nach der allgemeinen Methode 4C wurden 544 mg (3.13 mmol) 4-Brompyridin-2(1*H*)-on mit 845 mg (3.3 mmol) 2-Brom-3-phenylpropansäureethylester (Racemat) in Gegenwart von 1.15 eq. Natriumhydrid und 2.3 eq. Lithiumbromid umgesetzt (1.5 h bei 65°C gerührt). Ausbeute: 572 mg (51% d. Th.)
LC/MS [Methode 1]: $R_t$ = 1.03 min; MS (ESIpos): m/z = 350 $(M+H)^+$

**Beispiel 7.1B**

2-[4-(5-Chlor-2-cyanophenyl)-2-oxopyridin-1(2*H*)-yl]-3-phenylpropansäureethylester (Racemat)

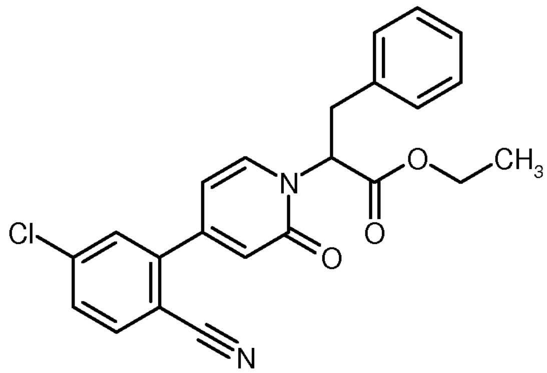

Nach der allgemeinen Methode 2A wurden 572 mg (1.6 mmol) 2-(4-Brom-2-oxopyridin-1(2*H*)-yl)-3-phenylpropanosäureethylester (Racemat) mit 330 mg (1.8 mmol) 5-Chlor-2-cyanophenylboronsäure in Gegenwart von Tetrakis(triphenylphosphin)palladium(0) umgesetzt. Ausbeute: 300 mg (94% Reinheit, 43% d. Th.)
LC/MS [Methode 1]: $R_t$ = 1.12 min; MS (ESIpos): m/z = 407 $(M+H)^+$

**Beispiel 7.1C**

2-[4-(5-Chlor-2-cyanophenyl)-2-oxopyridin-1(2*H*)-yl]-3-phenylpropansäure (Racemat)

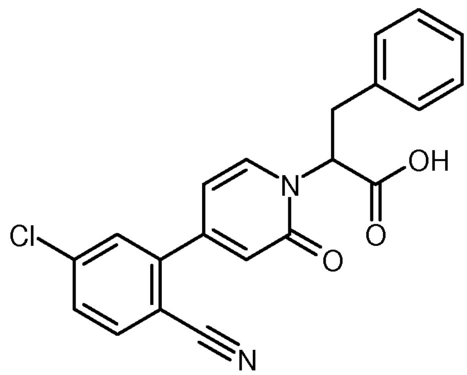

Nach der allgemeinen Methode 6B wurden 300 mg (94% Reinheit, 0.69 mmol) 2-[4-(5-Chlor-2-cyanophenyl)-2-oxopyridin-1(2*H*)-yl]-3-phenylpropansäureethylester (Racemat) mit Lithiumhydroxid verseift. Ausbeute: 129 mg (89% Reinheit, 43% d. Th.)
LC/MS [Methode 1]: $R_t$ = 0.96 min; MS (ESIpos): m/z = 379 $(M+H)^+$

**Beispiel 8.1A**

(4-Brom-2-oxopyridin-1(2*H*)-yl)essigsäureethylester

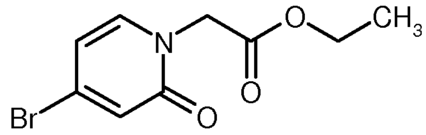

Nach der allgemeinen Methode 4B wurden 5.0 g (28.7 mmol) 4-Brompyridin-2(1*H*)-on mit 5.3 g (31.6 mmol) Bromessigsäureethylester umgesetzt. Ausbeute: 6.2 g (83% d. Th)
LC/MS [Methode 3]: $R_t$ = 1.57 min; MS (ESIpos): m/z = 260 $(M+H)^+$

**Beispiel 8.1B**

{4-[5-Chlor-2-(trifluormethyl)phenyl]-2-oxopyridin-1(2*H*)-yl}essigsäureethylester

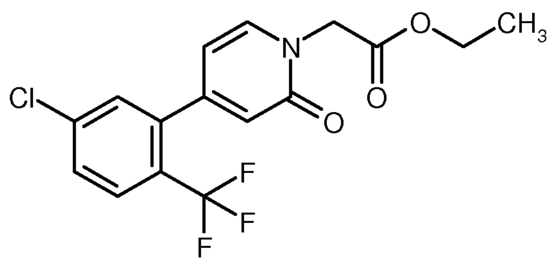

Nach der allgemeinen Methode 2A wurden 2.04 g (7.8 mmol) (4-Brom-2-oxopyridin-1(2*H*)-yl)essigsäureethylester mit 1.98 g (8.6 mmol) 5-Chlor-2-trifluormethylphenylboronsäure in Gegenwart von [1,1-Bis-(diphenylphosphino)-ferrocen] -palladium(II)chlorid-dichlormethan-Monoaddukt umgesetzt. Ausbeute: 2.89 g (quant.)
LC/MS [Methode 1]: $R_t$ = 1.05 min; MS (ESIpos): m/z = 360 $(M+H)^+$

**Beispiel 8.1C**

2-{4-[5-Chlor-2-(trifluormethyl)phenyl]-2-oxopyridin-1(2*H*)-yl}-3-(pyridin-2-yl)propansäureethylester (Racemat)

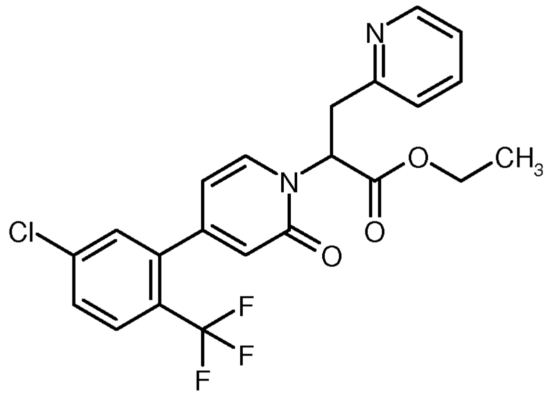

Nach der allgemeinen Methode 7A wurden 440 mg (1.22 mmol) {4-[5-Chlor-2-(trifluormethyl)phenyl]-2-oxopyridin-1(2*H*)-yl}essigsäureethylester mit 464 mg (1.84 mmol) 2-(Brommethyl)pyridin-monohydrobromid umgesetzt. Ausbeute: 371 mg (65% Reinheit, 44% d. Th.) der Titelverbindung und 270 mg (50% d.Th.) des bereits verseiften Produktes (Beispiel 8.1D)
LC/MS [Methode 2]: $R_t$ = 3.07 min; MS (ESIpos): m/z = 451 $(M+H)^+$

**Beispiel 8.1D**

2-{4-[5-Chlor-2-(trifluormethyl)phenyl]-2-oxopyridin-1(2*H*)-yl}-3-(pyridin-2-yl)propansäure (Racemat)

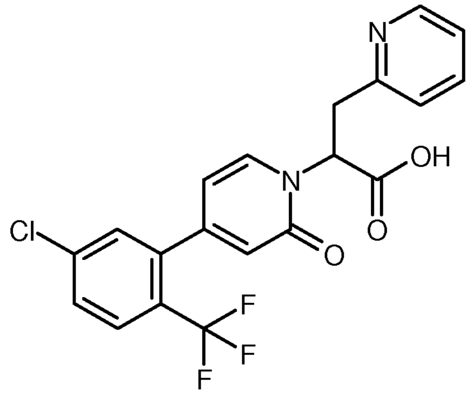

Nach der allgemeinen Methode 6B wurden 350 mg (65% Reinheit, 0.51 mmol) 2-{4-[5-Chlor-2-(trifluormethyl)phenyl] -2-oxopyridin-1(2*H*)-yl}-3-(pyridin-2-yl)propansäureethylester (Racemat) mit Lithiumhydroxid verseift. Ausbeute: 240 mg (80% Reinheit, 90% d. Th.)
LC/MS [Methode 1]: $R_t$ = 0.80 min; MS (ESIpos): m/z = 423 $(M+H)^+$

**Beispiel 8.1E**

4-{[2-{4-[5-Chlor-2-(trifluormethyl)phenyl]-2-oxopyridin-1(2*H*)-yl}-3-(pyridin-2-yl)propanoyl]-amino}benzoesäure-*tert*.-butylester (Racemat)

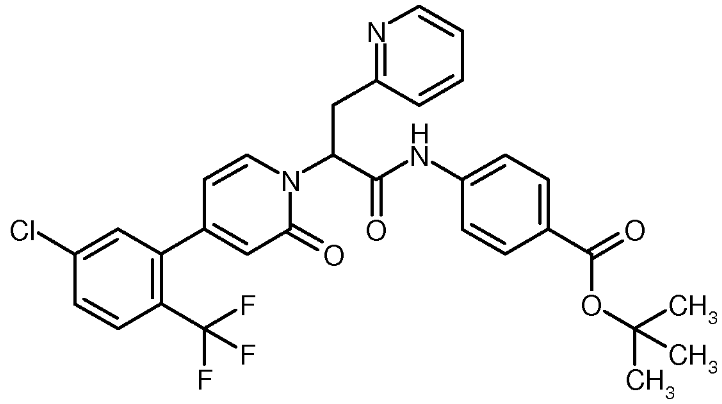

Eine Lösung von 470 mg (50% Reinheit, 0.56 mmol) 2-{4-[5-Chlor-2-(trifluormethyl)phenyl]-2-oxopyridin-1(2*H*)-yl}-3-(pyridin-2-yl)propansäure (Racemat) und 129 mg (0.67 mmol) 4-Aminobenzoesäure-*tert.*-butylester in 45 ml Ethylacetat wurde unter Argon bei 0°C mit 707 mg (50%ig in Ethylacetat, 1.11 mmol) T3P und 0.29 ml (1.67 mmol) *N,N*-Diisopropylethylamin versetzt. Das Reaktionsgemisch wurde 1 h bei 60°C gerührt, nochmals mit 353 mg (50%ig in Ethylacetat, 0.56 mmol) T3P und 0.1 ml (0.56 mmol) *N,N*-Diisopropylethylamin versetzt und 1 h bei 60°C gerührt. Nach Zugabe von Wasser/Ethylacetat und Phasentrennung wurde die wässrige Phase dreimal mit Ethylacetat extrahiert. Die vereinigten, organischen Phasen wurden getrocknet (Natriumsulfat), filtriert und im Vakuum eingeengt. Das Rohprodukt wurde mittels Flash-Chromatographie (Kieselgel-60, Dichlormethan-Methanol-Gemische) aufgereinigt. Ausbeute: 163 mg (93% Reinheit, 46% d. Th.)
LC/MS [Methode 1]: $R_t$ = 1.29 min; MS (ESIpos): m/z = 598 $(M+H)^+$

**Beispiel 9.1A**

2-{4-[5-Chlor-2-(trifluormethyl)phenyl]-2-oxopyridin-1(2*H*)-yl}-3-(pyridin-3-yl)propansäureethylester (Racemat)

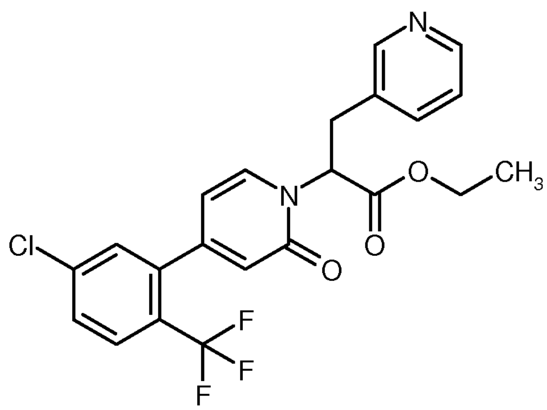

Nach der allgemeinen Methode 7A wurden 216 mg (0.6 mmol) {4-[5-Chlor-2-(trifluormethyl)phenyl]-2-oxopyridin-1(2*H*)-yl}essigsäureethylester mit 228 mg (0.9 mmol) 3-(Brommethyl)pyridin-Monohydrobromid umgesetzt. Ausbeute: 39 mg (14% d. Th.)
LC/MS [Methode 1]: $R_t$ = 0.94 min; MS (ESIpos): m/z = 451 $(M+H)^+$

**Beispiel 9.1B**

2-{4-[5-Chlor-2-(trifluormethyl)phenyl]-2-oxopyridin-1(2*H*)-yl}-3-(pyridin-3-yl)propansäure (Racemat)

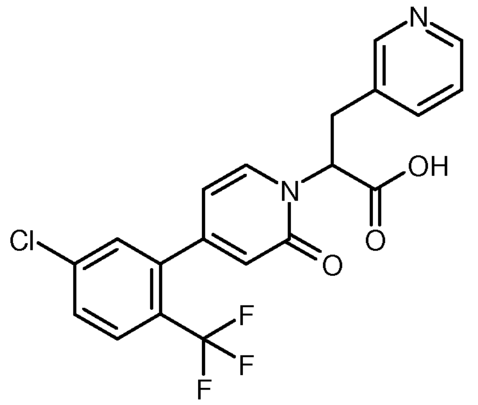

Nach der allgemeinen Methode 6B wurden 39 mg (0.09 mmol) 2-{4-[5-Chlor-2-(trifluormethyl)phenyl] -2-oxopyridin-1(2*H*)-yl}-3-(pyridin-3-yl)propansäureethylester (Racemat) mit Lithiumhydroxid verseift. Ausbeute: 28 mg (92% Reinheit, 70% d. Th.)
LC/MS [Methode 1]: $R_t$ = 0.74 min; MS (ESIpos): m/z = 423 $(M+H)^+$

**Beispiel 9.1C**

4-{[2-{4-[5-Chlor-2-(trifluormethyl)phenyl]-2-oxopyridin-1(2*H*)-yl}-3-(pyridin-3-yl)propanoyl]-amino }benzoesäure-*tert*.-butylester (Racemat)

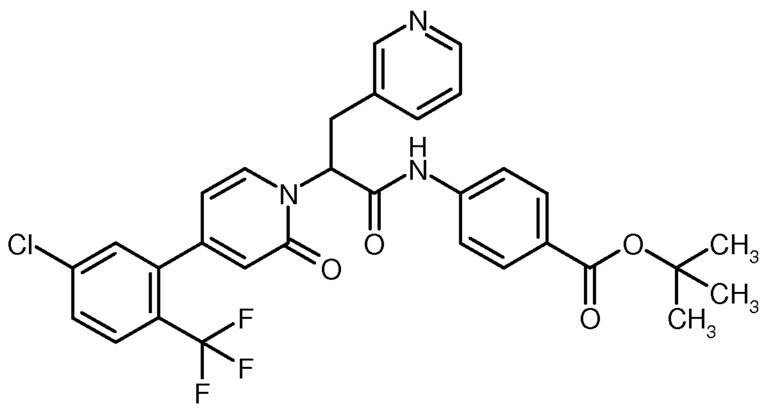

Nach der allgemeinen Methode 5A wurden 26 mg (92% Reinheit, 0.06 mmol) 2-{4-[5-Chlor-2-(trifluormethyl)phenyl]-2-oxopyridin-1(2*H*)-yl}-3-(pyridin-3-yl)propansäure (Racemat) mit 1.1 eq. 4-Aminobenzoesäure-*tert*.-butylester umgesetzt. Das Reaktionsgemisch wurde von DMF befreit und der Rückstand mit Eiswasser verrührt. Die erhaltenen Kristalle wurden abfiltriert, mit Wasser gewaschen und im Vakuum getrocknet. Ausbeute: 33 mg (94% Reinheit, 92% d. Th.)
LC/MS [Methode 1]: $R_t$ = 1.22 min; MS (ESIpos): m/z = 598 $(M+H)^+$

**Beispiel 9.2A**

(4-Brom-2-oxopyridin-1(2*H*)-yl)essigsäure-*tert*.-butylester

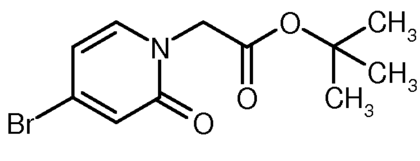

Nach der allgemeinen Methode 4B wurden 4.9 g (28.4 mmol) 4-Brompyridin-2(1*H*)-on mit 1.2 eq. Bromessigsäure-*tert*.-butylester bei 120°C umgesetzt. Nach wässriger Aufarbeitung wurde das Rohprodukt ohne weitere Reinigung weiter umgesetzt. Ausbeute: 8.6 g (91% Reinheit, 95% d. Th.)
LC/MS [Methode 1]: $R_t$ = 0.89 min; MS (ESIpos): m/z = 288 $(M+H)^+$

**Beispiel 9.2B**

[4-(2-Brom-5-chlorphenyl)-2-oxopyridin-1(2*H*)-yl]essigsäure-*tert*.-butylester

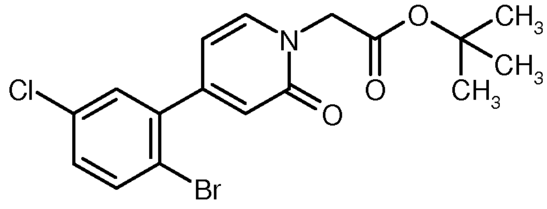

Nach der allgemeinen Methode 2A wurden 3.8 g (12 mmol) (4-Brom-2-oxopyridin-1(2*H*)-yl)essigsäure-*tert*.-butylester mit 3.4 g (14.4 mmol) 2-Brom-5-chlorphenylboronsäure in Gegenwart von Tetrakis(triphenylphosphin)palladium(0) umgesetzt. Ausbeute: 3.9 g (94% Reinheit, 76% d. Th.)
LC/MS [Methode 1]: $R_t$ = 1.15 min; MS (ESIpos): m/z = 398 $(M+H)^+$

**Beispiel 9.2C**

2-[4-(2-Brom-5-chlorphenyl)-2-oxopyridin-1(2*H*)-yl]-3-(pyridin-3-yl)propansäure-*tert*.-butylester (Racemat)

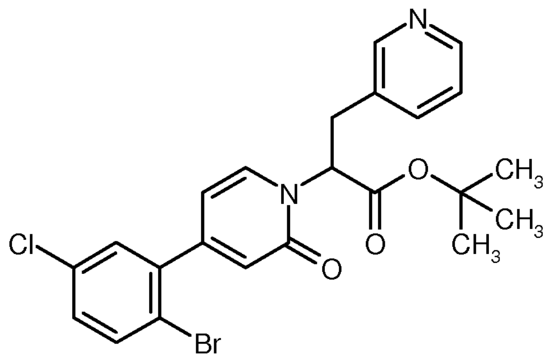

Nach der allgemeinen Methode 7A wurden 206 mg (94% Reinheit, 0.49 mmol) [4-(2-Brom-5-chlorphenyl)-2-oxopyridin-1(2*H*)-yl]essigsäure-*tert*.-butylester mit 184 mg (0.73 mmol) 3-(Brommethyl)pyridin-Monohydrobromid umgesetzt. Ausbeute: 274 mg (88% Reinheit, quant.)
LC/MS [Methode 1]: $R_t$ = 1.02 min; MS (ESIpos): m/z = 489 $(M+H)^+$

**Beispiel 9.2D**

2-[4-(2-Brom-5-chlorphenyl)-2-oxopyridin-1(2*H*)-yl]-3-(pyridin-3-yl)propansäure (Racemat)

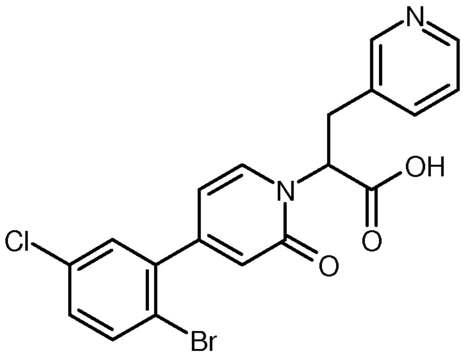

Nach der allgemeinen Methode 6A wurden 274 mg (88% Reinheit, 0.49 mmol) 2-[4-(2-Brom-5-chlorphenyl)-2-oxopyridin-1(2*H*)-yl]-3-(pyridin-3-yl)propansäure-*tert*.-butylester (Racemat) mit TFA verseift. Ausbeute: 244 mg (57% Reinheit, 65% d. Th.)

LC/MS [Methode 1]: $R_t$ = 0.73 min; MS (ESIpos): m/z = 433 $(M+H)^+$

**Beispiel 9.2E**

4-({2-[4-(2-Brom-5-chlorphenyl)-2-oxopyridin-1(2*H*)-yl]-3-(pyridin-3-yl)propanoyl}amino)-benzoesäuremethylester (Racemat)

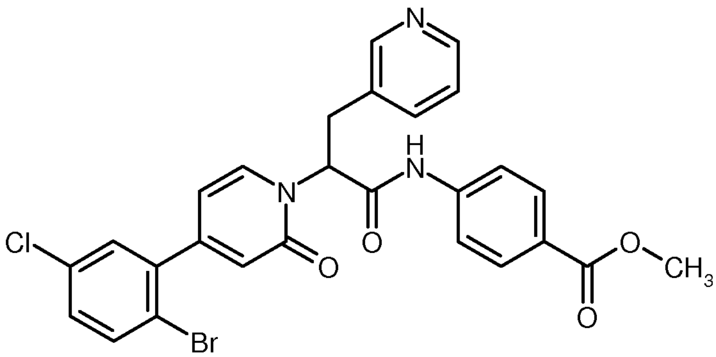

Nach der allgemeinen Methode 5A wurden 244 mg (57% Reinheit, 0.32 mmol) 2-[4-(2-Brom-5-chlorphenyl)-2-oxopyridin-1(2*H*)-yl]-3-(pyridin-3-yl)propansäure (Racemat) mit 1.2 eq. 4-Aminobenzoesäuremethylester umgesetzt. Ausbeute: 65 mg (85% Reinheit, 30% d. Th.)

LC/MS [Methode 1]: $R_t$ = 1.02 min; MS (ESIpos): m/z = 566 $(M+H)^+$

**Beispiel 10.1A**

2-{4-[5-Chlor-2-(trifluormethyl)phenyl]-2-oxopyridin-1(2*H*)-yl}-3-(pyridin-4-yl)propansäureethylester (Racemat)

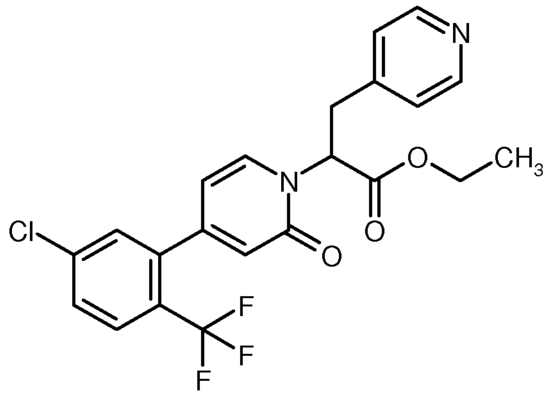

Nach der allgemeinen Methode 7A wurden 1.8 g (5.05 mmol) {4-[5-Chlor-2-(trifluormethyl)phenyl]-2-oxopyridin-1(2*H*)-yl}essigsäureethylester mit 1.9 g (7.6 mmol) 4-(Brommethyl)pyridin-Monohydrobromid umgesetzt. Ausbeute: 0.45 g (20% d. Th.)
LC/MS [Methode 2]: $R_t$ = 2.42 min; MS (ESIpos): m/z = 451 $(M+H)^+$

**Beispiel 10.1B**

2-{4-[5-Chlor-2-(trifluormethyl)phenyl]-2-oxopyridin-1(2*H*)-yl}-3-(pyridin-4-yl)propansäure (Racemat)

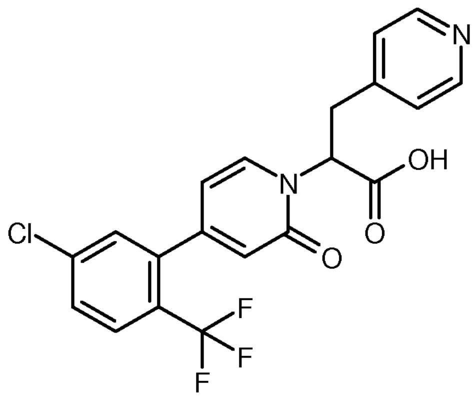

Nach der allgemeinen Methode 6B wurden 452 mg (1.0 mmol) 2-{4-[5-Chlor-2-(trifluormethyl)phenyl] -2-oxopyridin-1(2*H*)-yl}-3-(pyridin-4-yl)propansäureethylester (Racemat) mit Lithiumhydroxid verseift. Ausbeute: 289 mg (68% d. Th.)
LC/MS [Methode 1]: $R_t$ = 0.70 min; MS (ESIpos): m/z = 423 $(M+H)^+$

**Beispiel 10.1C**

4-{[2-{4-[5-Chlor-2-(trifluormethyl)phenyl]-2-oxopyridin-1(2*H*)-yl}-3-(pyridin-4-yl)propanoyl]-amino}benzoesäure-*tert.*-butylester (Racemat)

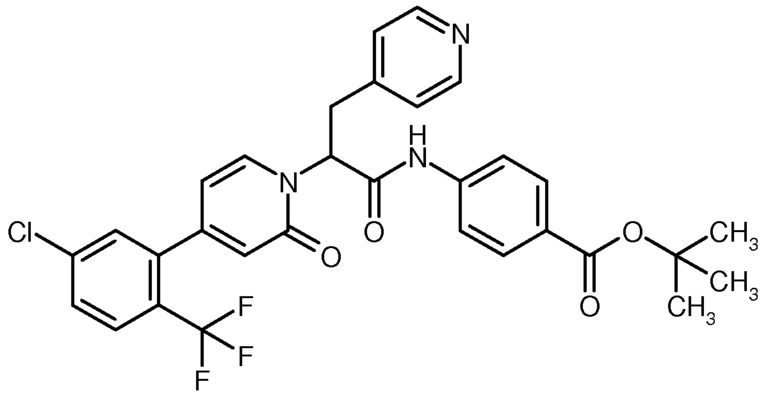

Nach der allgemeinen Methode 5A wurden 626 mg (50% Reinheit, 0.74 mmol) 2-{4-[5-Chlor-2-(trifluormethyl)phenyl]-2-oxopyridin-1(2*H*)-yl}-3-(pyridin-4-yl)propansäure (Racemat) mit 1.2 eq. 4-Aminobenzoesäure-*tert*.-butylester umgesetzt. Ausbeute: 155 mg (94% Reinheit, 33% d. Th.)
LC/MS [Methode 1]: $R_t$ = 1.17 min; MS (ESIpos): m/z = 598 $(M+H)^+$

## Beispiel 10.2A

2-[4-(2-Brom-5-chlorphenyl)-2-oxopyridin-1(2*H*)-yl]-3-(pyridin-4-yl)propansäure-*tert*.-butylester (Racemat)

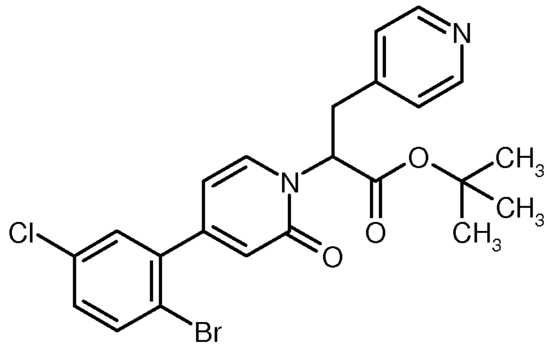

Nach der allgemeinen Methode 7A wurden 1.3 g (94% Reinheit, 3.0 mmol) [4-(2-Brom-5-chlorphenyl)-2-oxopyridin-1(2*H*)-yl]essigsäure-*tert*.-butylester mit 1.2 g (4.5 mmol) 4-(Brommethyl)pyridin-Monohydrobromid umgesetzt. Ausbeute: 1.7 g (89% Reinheit, quant.)
LC/MS [Methode 1]: $R_t$ = 0.98 min; MS (ESIpos): m/z = 489 $(M+H)^+$

## Beispiel 10.2B

2-[4-(2-Brom-5-chlorphenyl)-2-oxopyridin-1(2*H*)-yl]-3-(pyridin-4-yl)propansäure (Racemat)

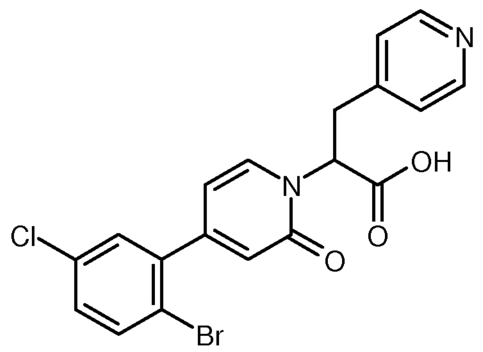

Nach der allgemeinen Methode 6A wurden 1.7 g (89% Reinheit, 3.2 mmol) 2-[4-(2-Brom-5-chlorphenyl)-2-oxopyridin-1(2*H*)-yl]-3-(pyridin-4-yl)propansäure-*tert.*-butylester (Racemat) mit TFA verseift. Nach der Aufarbeitung wurde der Rückstand mit Diethylether verrührt, der Feststoff filtriert und im Vakuum getrocknet. Ausbeute: 1.8 g (76% Reinheit, 99% d. Th.)
LC/MS [Methode 1]: $R_t$ = 0.64 min; MS (ESIpos): m/z = 433 $(M+H)^+$

**Beispiel 10.2C**

4-({2-[4-(2-Brom-5-chlorphenyl)-2-oxopyridin-1(2*H*)-yl]-3-(pyridin-4-yl)propanoyl}amino)-benzoesäure-*tert.*-butylester (Racemat)

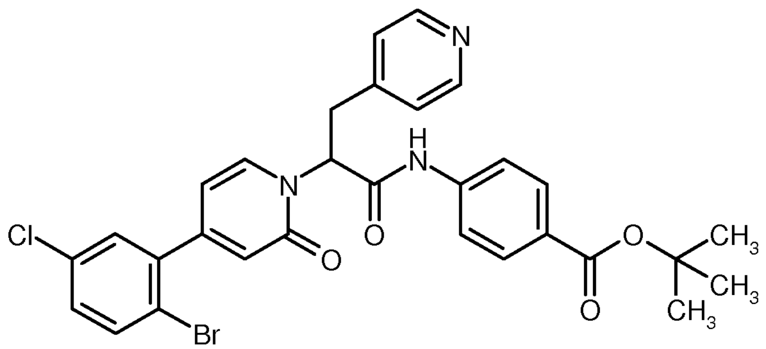

Nach der allgemeinen Methode 5A wurden 1.8 g (76% Reinheit, 3.2 mmol) 2-[4-(2-Brom-5-chlorphenyl)-2-oxopyridin-1(2*H*)-yl]-3-(pyridin-4-yl)propansäure (Racemat) mit 1.1 eq. 4-Aminobenzoesäure-*tert.*-butylester umgesetzt. Ausbeute: 734 mg (92% Reinheit, 35% d. Th.)
LC/MS [Methode 1]: Rt = 1.16 min; MS (ESIpos): m/z = 608 $(M+H)^+$

**Beispiel 11.1A**

4-Chlor-2-(5-fluor-2-methoxypyridin-4-yl)benzonitril

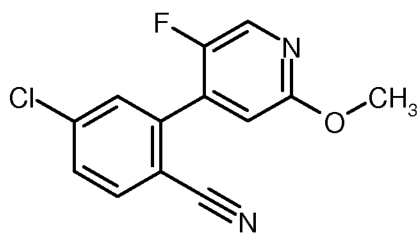

Nach der allgemeinen Methode 2A wurden 256 mg (1.5 mmol) 5-Fluor-2-methoxypyridin-4-ylboronsäure mit 295 mg (1.34 mmol) 2-Brom-4-chlorbenzonitril umgesetzt. Das Produkt wurde mit Wasser ausgefällt. Ausbeute: 146 mg (37% d. Th.)
LC/MS [Methode 1]: $R_t$ = 1.10 min; MS (ESIpos): m/z = 263 $(M+H)^+$

**Beispiel 11.1B**

4-Chlor-2-(5-fluor-2-oxo-1,2-dihydropyridin-4-yl)benzonitril

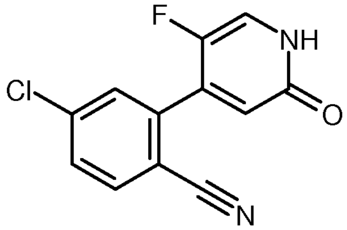

Nach der allgemeinen Methode 3A wurden 210 mg (0.77 mmol) 4-Chlor-2-(5-fluor-2-methoxypyridin-4-yl)benzonitril mit Pyridinium-Hydrochlorid umgesetzt. Ausbeute: 126 mg (66% d. Th.)
LC/MS [Methode 1]: $R_t$ = 0.76 min; MS (ESIpos): m/z = 249 $(M+H)^+$

**Beispiel 11.1C**

2-[4-(5-Chlor-2-cyanophenyl)-5-fluor-2-oxopyridin-1(2*H*)-yl]propansäure-*tert*.-butylester (Racemat)

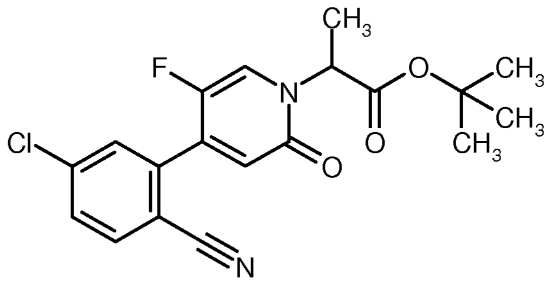

Nach der allgemeinen Methode 4B wurden 126 mg (0.51 mmol) 4-Chlor-2-(5-fluor-2-oxo-1,2-dihydropyridin-4-yl)benzonitril mit 1.05 eq. 2-Brompropansäure-*tert*.-butylester (Racemat) bei 100°C umgesetzt. Ausbeute: 48 mg (25% d. Th.)
LC/MS [Methode 1]: $R_t$ = 1.09 min; MS (ESIpos): m/z = 377 $(M+H)^+$

**Beispiel 11.1D**

2-[4-(5-Chlor-2-cyanophenyl)-5-fluor-2-oxopyridin-1(2*H*)-yl]propansäure (Racemat)

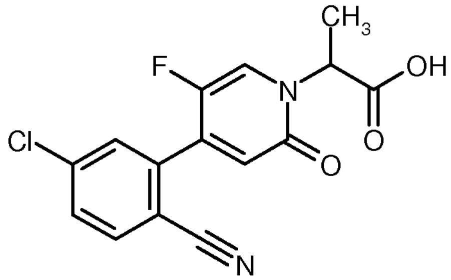

Nach der allgemeinen Methode 6A wurden 46 mg (0.12 mmol) 2-[4-(5-Chlor-2-cyanophenyl)-5-fluor-2-oxopyridin-1(2*H*)-yl]propansäure-*tert*.-butylester (Racemat) mit TFA verseift. Ausbeute: 54 mg (90% Reinheit, quant.)
LC/MS [Methode 1]: $R_t$ = 0.78 min; MS (ESIpos): m/z = 321 $(M+H)^+$

**Beispiel 11.1E**

4-({2-[4-(5-Chlor-2-cyanophenyl)-5-fluor-2-oxopyridin-1(2*H*)-yl]propanoyl}amino)benzoesäure-*tert*.-butylester (Racemat)

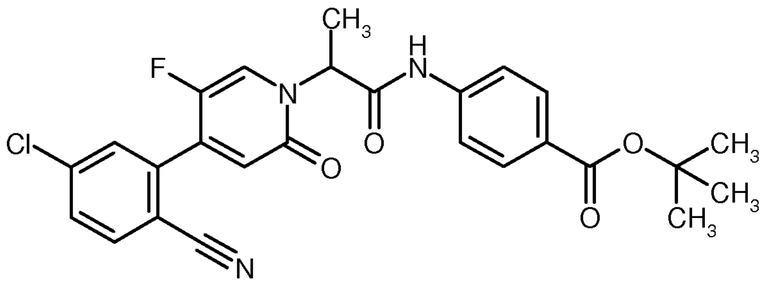

Nach der allgemeinen Methode 5A wurden 54 mg (90% Reinheit, 0.15 mmol) 2-[4-(5-Chlor-2-cyanophenyl)-5-fluor-2-oxopyridin-1(2*H*)-yl]propansäure (Racemat) mit 1.1 eq. 4-Aminobenzoesäure-*tert*.-butylester umgesetzt. Ausbeute: 51 mg (67% d. Th.)
LC/MS [Methode 1]: Rt = 1.23 min; MS (ESIpos): m/z = 496 $(M+H)^+$

**Beispiel 12.1A**

(5-Chlor-2-methoxypyridin-4-yl)boronsäure

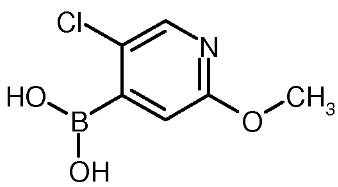

Nach der allgemeinen Methode 1A wurden 10.0 g (69.65 mmol) 5-Chlor-2-methoxypyridin umgesetzt. Das gewünschte Produkt fiel als Niederschlag beim Ansäuern mit Salzsäure (2N) aus. Ausbeute: 10.44 g (91% Reinheit, 73% d. Th.)
LC/MS [Methode 1]: $R_t$ = 0.50 min; MS (ESIpos): m/z = 188 $(M+H)^+$
$^1$H-NMR (400 MHz, DMSO-$d_6$): $\delta$ [ppm] = 8.64 (br. s, 2H), 8.12 (s, 1H), 6.81 (s, 1H), 3.82 (s, 3H).

**Beispiel 12.1B**

4-Chlor-2-(5-chlor-2-methoxypyridin-4-yl)benzonitril

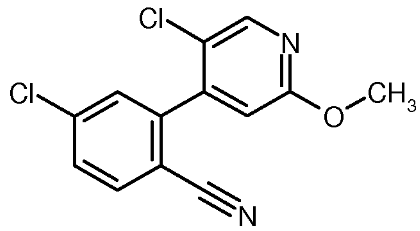

Nach der allgemeinen Methode 2A wurden 5.36 g (91% Reinheit, 26.03 mmol) 5-Chlor-2-methoxypyridin-4-ylboronsäure mit 5.12 g (23.66 mmol) 2-Brom-4-chlorbenzonitril in Gegenwart von [1,1-Bis-(diphenylphosphino)-ferrocen] -palladium(II)chlorid-dichlormethan-Monoaddukt umgesetzt. Nach Aufarbeitung wurde das Rohprodukt anschließend mittels Flash-Chromatographie (Kieselgel-60, Cyclohexan-Dichlormethan-Gemische) aufgereinigt. Ausbeute: 4.11 g (91% Reinheit, 52% d. Th.)
LC/MS [Methode 1]: $R_t$ = 1.17 min; MS (ESIpos): m/z = 279 $(M+H)^+$

**Beispiel 12.1C**

4-Chlor-2-(5-chlor-2-oxo-1,2-dihydropyridin-4-yl)benzonitril

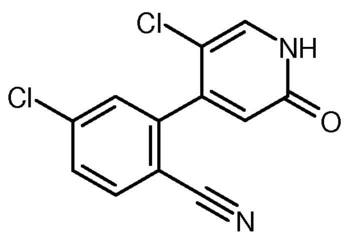

Nach der allgemeinen Methode 3A wurden 6.34 g (93% Reinheit, 21.12 mmol) 4-Chlor-2-(2,5-dimethoxypyridin-4-yl)benzonitril mit Pyridinium-Hydrochlorid umgesetzt. Ausbeute: 4.23 g (76% d. Th.)
LC/MS [Methode 1]: $R_t$ = 0.82 min; MS (ESIpos): m/z = 265 $(M+H)^+$

**Beispiel 12.1D**

2-[5-Chlor-4-(5-chlor-2-cyanophenyl)-2-oxopyridin-1(2*H*)-yl]propansäure (Racemat)

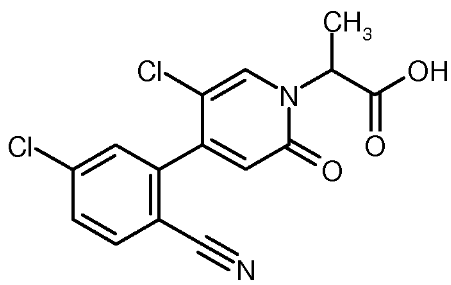

Nach der allgemeinen Methode 4A wurden 910 mg (57% Reinheit, 1.96 mmol) 4-Chlor-2-(5-chlor-2-oxo-1,2-dihydropyridin-4-yl)benzonitril mit 1.5 eq. 2-Brompropansäure (Racemat) zunächst 2.5 h bei RT und anschließend über Nacht bei 45°C umgesetzt. Das gewünschte Produkt wurde durch Ausfällung mit Salzsäure erhalten Ausbeute: 1.06 g (78% Reinheit, quant.)
LC/MS [Methode 1]: $R_t$ = 0.86 min; MS (ESIpos): m/z = 337 $(M+H)^+$

**Beispiel 12.1E**

4-({2-[5-Chlor-4-(5-chlor-2-cyanophenyl)-2-oxopyridin-1(2*H*)-yl]propanoyl}amino)benzoesäure-*tert*.-butylester (Racemat)

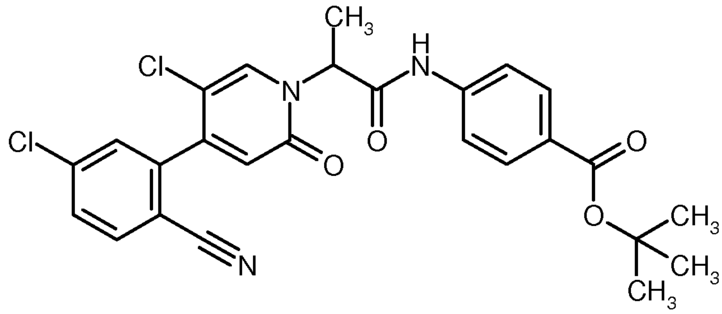

Nach der allgemeinen Methode 5A wurden 135 mg (93% Reinheit, 0.37 mmol) 2-[5-Chlor-4-(5-chlor-2-cyanophenyl)-2-oxopyridin-1(2*H*)-yl]propansäure (Racemat) mit 1.2 eq. 4-Aminobenzoesäure-*tert*.-butylester umgesetzt. Ausbeute: 281 mg (58% Reinheit, 85% d. Th.)
LC/MS [Methode 3]: $R_t$ = 2.69 min; MS (ESIpos): m/z = 512 $(M+H)^+$

**Beispiel 12.2A**

5-[4-({2-[5-Chlor-4-(5-chlor-2-cyanophenyl)-2-oxopyridin-1(2*H*)-yl]propanoyl}amino)phenyl]-3-oxo-2,3-dihydro-1*H*-pyrazol-1-carbonsäure-*tert*.-butylester (Racemat)

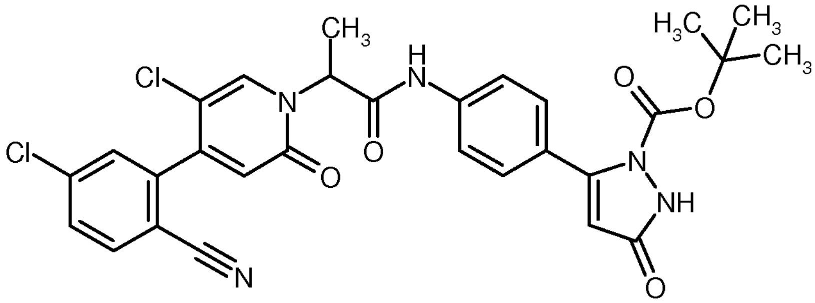

Nach der allgemeinen Methode 5A wurden 150 mg (82% Reinheit, 0.37 mmol) 2-[5-Chlor-4-(5-chlor-2-cyanophenyl)-2-oxopyridin-1(2*H*)-yl]propansäure (Racemat) mit 1.2 eq. 5-(4-Aminophenyl)-3-oxo-2,3-dihydro-1*H*-pyrazol-1-carbonsäure-*tert*.-butylester umgesetzt. Ausbeute: 17.8 mg (78% Reinheit, 6% d. Th.)
LC/MS [Methode 1]: $R_t$ = 1.17 min; MS (ESIpos): m/z = 594 $(M+H)^+$

**Beispiel 13.1A**

[5-Chlor-4-(5-chlor-2-cyanophenyl)-2-oxopyridin-1(2*H*)-yl]essigsäure-*tert*.-butylester

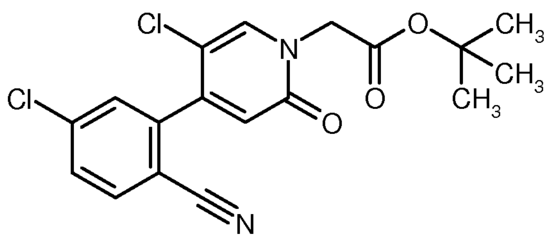

Nach der allgemeinen Methode 4B wurden 400 mg (91% Reinheit, 1.37 mmol) 4-Chlor-2-(5-chlor-2-oxo-1,2-dihydropyridin-4-yl)benzonitril mit 1.2 eq. Bromessigsäure-*tert*.-butylester bei 100°C umgesetzt. Ausbeute: 421 mg (80% d. Th.)
LC/MS [Methode 1]: $R_t$ = 1.11 min; MS (ESIneg): m/z = 377 $(M-H)^-$

**Beispiel 13.1B**

2-[5-Chlor-4-(5-chlor-2-cyanophenyl)-2-oxopyridin-1(2*H*)-yl]-3-cyclopropylpropansäure-*tert*.-butylester (Racemat)

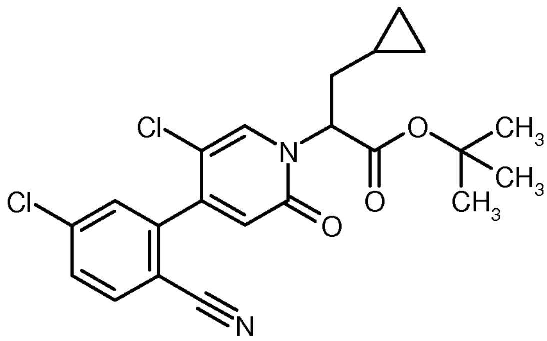

Nach der allgemeinen Methode 7A wurden 349 mg (0.91 mmol) [5-Chlor-4-(5-chlor-2-cyanophenyl)-2-oxopyridin-1(2*H*)-yl]essigsäure-*tert*.-butylester mit 216 mg (1.18 mmol) (Iodmethyl)cyclopropan umgesetzt. Ausbeute: 245 mg (62% d. Th.)
LC/MS [Methode 3]: $R_t$ = 2.75 min; MS (ESIpos): m/z = 433 $(M+H)^+$

**Beispiel 13.1C**

2-[5-Chlor-4-(5-chlor-2-cyanophenyl)-2-oxopyridin-1(2*H*)-yl]-3-cyclopropylpropansäure (Racemat)

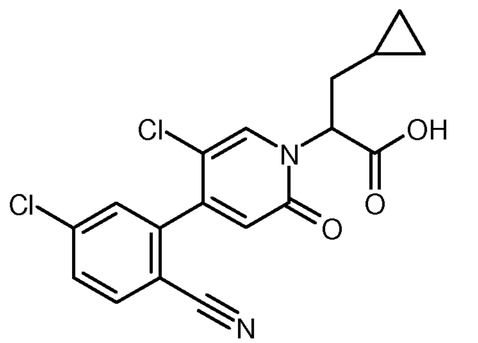

Nach der allgemeinen Methode 6A wurden 245 mg (0.57 mmol) 2-[5-Chlor-4-(5-chlor-2-cyanophenyl)-2-oxopyridin-1(2*H*)-yl]-3-cyclopropylpropansäure-*tert*.-butylester (Racemat) mit 20 eq. TFA verseift. Ausbeute: 268 mg (quant.)
LC/MS [Methode 3]: $R_t$ = 2.21 min; MS (ESIpos): m/z = 377 $(M+H)^+$

**Beispiel 13.1D**

4-({2-[5-Chlor-4-(5-chlor-2-cyanophenyl)-2-oxopyridin-1(2*H*)-yl]-3-cyclopropylpropanoyl}-amino)-benzoesäure-*tert*.-butylester (Racemat)

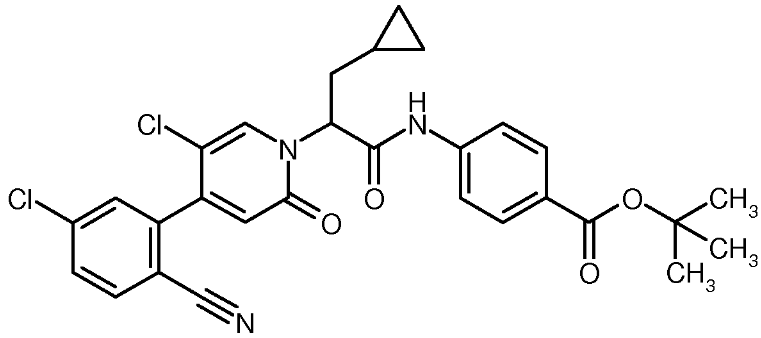

Nach der allgemeinen Methode 5A wurden 268 mg (0.68 mmol) 2-[5-Chlor-4-(5-chlor-2-cyanophenyl)-2-oxopyridin-1(2*H*)-yl]-3-cyclopropylpropansäure (Racemat) mit 1.2 eq. 4-Aminobenzoesäure-*tert*.-butylester umgesetzt. Ausbeute: 192 mg (51% d. Th.)
LC/MS [Methode 1]: $R_t$ = 1.40 min; MS (ESIpos): m/z = 552 $(M+H)^+$

**Beispiel 14.1A**

2-Brom-4-chlor-1-(difluormethyl)benzol

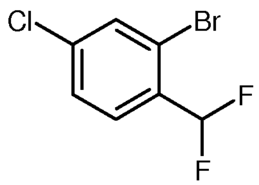

Eine Lösung von 1.0 g (4.56 mmol) 2-Brom-4-chlorbenzaldehyd in 12 ml Dichlormethan wurde bei 0°C mit 0.9 ml (6.83 mmol) Diethylaminoschwefeltrifluorid versetzt. Das Reaktionsgemisch wurde über Nacht bei RT gerührt und anschließend zu einer gesättigten Natriumhydrogencarbonat-Lösung getropft bis keine Kohlendioxid-Entwicklung mehr erkennbar war. Nach Zugabe von Ethylacetat und Phasentrennung wurde die wässrige Phase zweimal mit Ethylacetat extrahiert. Die vereinigten, organischen Phasen wurden mit gesättigter, wässriger Natriumchlorid-Lösung gewaschen, getrocknet (Natriumsulfat), filtriert, und kurz (!) im Vakuum eingeengt und getrocknet. Ausbeute: 872 mg (79% d. Th.)
GC/MS [Methode 7]: $R_t$ = 2.98 min; MS (EI): m/z = 240 $(M)^+$

**Beispiel 14.1B**

5-Chlor-4-[5-chlor-2-(difluormethyl)phenyl]-2-methoxypyridin

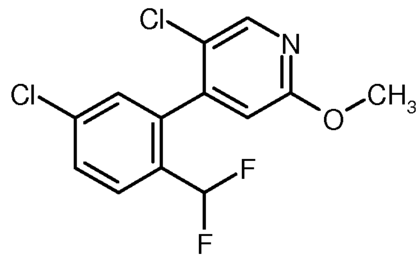

Nach der allgemeinen Methode 2A wurden 463 mg (93% Reinheit, 2.3 mmol) (5-Chlor-2-methoxy-pyridin-4-yl)boronsäure mit 515 mg (2.1 mmol) 2-Brom-4-chlor-1-(difluormethyl)benzol in Gegenwart von [1,1-Bis-(diphenylphosphino)-ferrocen]-palladium(II)chlorid-dichlormethan-Monoaddukt umgesetzt. Ausbeute: 305 mg (77% Reinheit, 34% d. Th.)
LC/MS [Methode 1]: $R_t$ = 1.30 min; MS (ESIpos): m/z = 304 $(M+H)^+$

**Beispiel 14.1C**

5-Chlor-4-[5-chlor-2-(difluormethyl)phenyl]pyridin-2(1*H*)-on

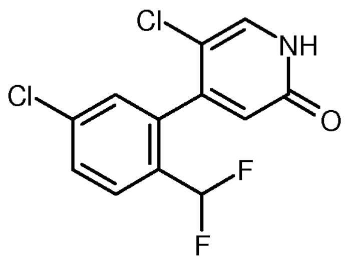

Nach der allgemeinen Methode 3A wurden 305 mg (77% Reinheit, 0.77 mmol) 5-Chlor-4-[5-chlor-2-(difluormethyl)phenyl]-2-methoxypyridin mit 20 eq. Pyridiniumchlorid umgesetzt. Nach Aufarbeitung wurde das Rohprodukt mittels präparativer HPLC (Reprosil C18, Wasser-Acetonitril-Gradient) aufgereinigt. Ausbeute: 179 mg (80% d. Th.)
LC/MS [Methode 1]: $R_t$ = 0.90 min; MS (ESIpos): m/z = 290 $(M+H)^+$

**Beispiel 14.1D**

2-{5-Chlor-4-[5-chlor-2-(difluormethyl)phenyl]-2-oxopyridin-1(2*H*)-yl}propansäure (Racemat)

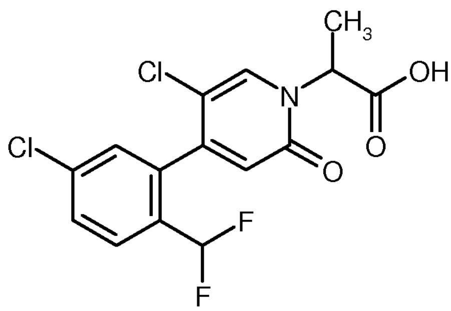

Nach der allgemeinen Methode 4A wurden 118 mg (0.41 mmol) 5-Chlor-4-[5-chlor-2-(difluormethyl)phenyl]pyridin-2(1*H*)-on mit 1.5 eq. 2-Brompropansäure (Racemat) bei 35°C umgesetzt. Ausbeute: 112 mg (76% d. Th.)
LC/MS [Methode 1]: $R_t$ = 0.96 min; MS (ESIpos): m/z = 362 $(M+H)^+$

**Beispiel 14.1E**

4-[(2-{5-Chlor-4-[5-chlor-2-(difluormethyl)phenyl]-2-oxopyridin-1(2*H*)-yl}propanoyl)amino]-benzoesäure-tert.-butylester (Racemat)

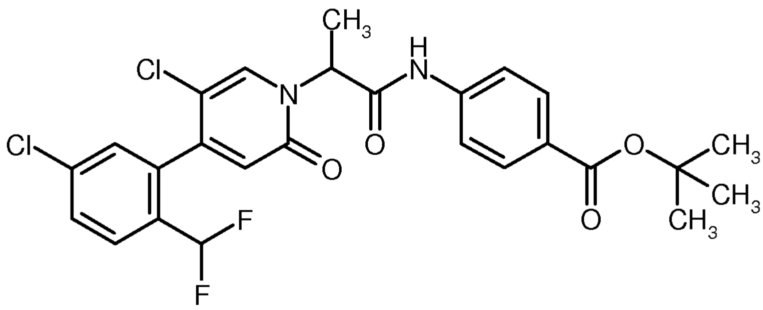

Nach der allgemeinen Methode 5A wurden 115 mg (0.32 mmol) 2-{5-Chlor-4-[5-chlor-2-(difluormethyl)phenyl]-2-oxopyridin-1(2*H*)-yl}propansäure (Racemat) mit 1.2 eq. 4-Aminobenzoesäure-*tert*.-butylester umgesetzt. Nach Aufarbeitung wurde das Rohprodukt mittels präparativer HPLC (Reprosil C18, Wasser-Acetonitril-Gradient) aufgereinigt. Ausbeute: 79 mg (46% d. Th.)
LC/MS [Methode 1]: $R_t$ = 1.31 min; MS (ESIpos): m/z = 537 $(M+H)^+$

**Beispiel 15.1A**

5-Chlor-4-[5-chlor-2-(trifluormethyl)phenyl]-2-methoxypyridin

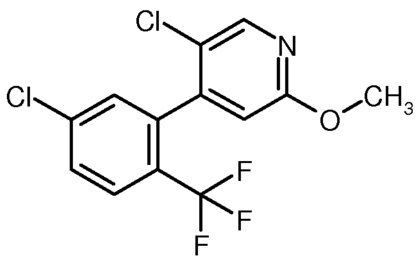

Nach der allgemeinen Methode 2B wurden 443 mg (2.20 mmol) 5-Chlor-2-methoxypyridin-4-ylboronsäure mit 571 mg (2.20 mmol) 2-Brom-4-chlor-1-(trifluormethyl)benzol in Gegenwart von XPhos Prekatalysator umgesetzt. Ausbeute: 193 mg (93% Reinheit, 25% d. Th.)
LC/MS [Methode 1]: $R_t$ = 1.36 min; MS (ESIpos): m/z = 322 $(M+H)^+$

**Beispiel 15.1B**

5-Chlor-4-[5-chlor-2-(trifluormethyl)phenyl]pyridin-2(1H)-on

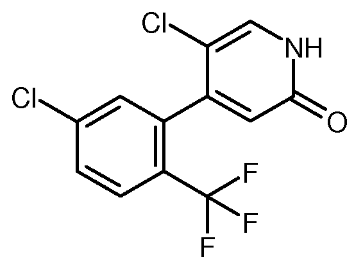

Nach der allgemeinen Methode 3A wurden 193 mg (93% Reinheit, 0.56 mmol) 5-Chlor-4-[5-chlor-2-(trifluormethyl)phenyl]-2-methoxypyridin mit Pyridinium-Hydrochlorid umgesetzt. Ausbeute: 123 mg (72% d. Th.)
LC/MS [Methode 1]: $R_t$ = 0.97 min; MS (ESIpos): m/z = 308 $(M+H)^+$

## Beispiel 15.1C

2-{5-Chlor-4-[5-chlor-2-(trifluormethyl)phenyl]-2-oxopyridin-1(2*H*)-yl}propansäure-*tert.*-butylester (Racemat)

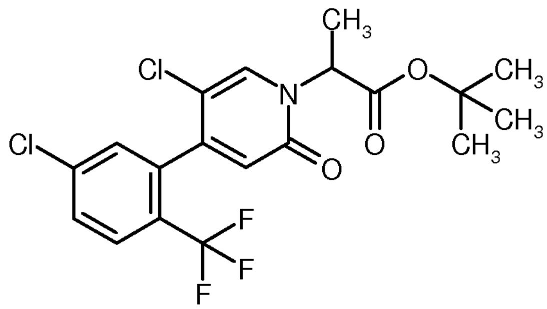

Nach der allgemeinen Methode 4B wurden 123 mg (0.4 mmol) 5-Chlor-4-[5-chlor-2-(trifluormethyl)phenyl]pyridin-2(1*H*)-on mit 1.05 eq. 2-Brompropansäure-*tert.*-butylester (Racemat) bei 100°C umgesetzt. Ausbeute: 81 mg (47% d. Th.)
LC/MS [Methode 1]: $R_t$ = 1.27 min; MS (ESIpos): m/z = 436 $(M+H)^+$

## Beispiel 15.1D

2-{5-Chlor-4-[5-chlor-2-(trifluormethyl)phenyl]-2-oxopyridin-1(2*H*)-yl}propansäure (Racemat)

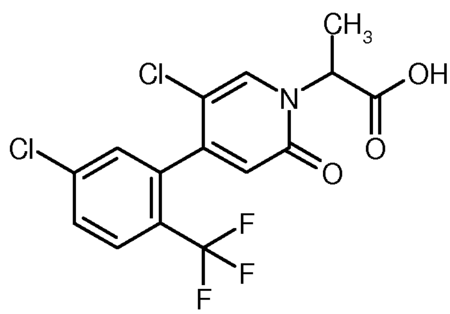

Nach der allgemeinen Methode 6A wurden 81 mg (0.19 mmol) 2-{5-Chlor-4-[5-chlor-2-(trifluormethyl)phenyl]-2-oxopyridin-1(2*H*)-yl}propansäure (Racemat) mit TFA verseift. Ausbeute: 78 mg (94% Reinheit, quant.)
LC/MS [Methode 1]: $R_t$ = 0.98 min; MS (ESIpos): m/z = 380 $(M+H)^+$

**Beispiel 15.1E**

2-{5-Chlor-4-[5-chlor-2-(trifluormethyl)phenyl]-2-oxopyridin-1(2*H*)-yl}propansäure (Racemat)

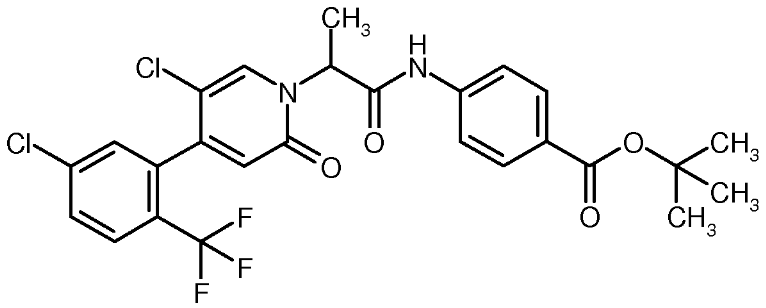

Nach der allgemeinen Methode 5A wurden 78 mg (94% Reinheit, 0.19 mmol) 2-{5-Chlor-4-[5-chlor-2-(trifluormethyl)phenyl]-2-oxopyridin-1(2*H*)-yl}propansäure (Racemat) mit 1.2 eq. 4-Aminobenzoesäure-*tert.*-butylester umgesetzt. Ausbeute: 75 mg (70% d. Th.)
LC/MS [Methode 1]: $R_t$ = 1.37 min; MS (ESIpos): m/z = 555 $(M+H)^+$

**Beispiel 16.1A**

(5-Cyano-2-methoxypyridin-4-yl)boronsäure

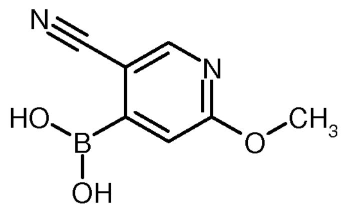

Nach der allgemeinen Methode 1A wurden 10.0 g (74.6 mmol) 6-Methoxypyridin-3-carbonitril umgesetzt. Ausbeute: 10.5 g (89% Reinheit, 70% d. Th.)
LC/MS [Methode 1]: $R_t$ = 0.51 min; MS (ESIpos): m/z = 179 $(M+H)^+$

**Beispiel 16.1B**

4-(5-Chlor-2-cyanophenyl)-6-methoxypyridin-3-carbonitril

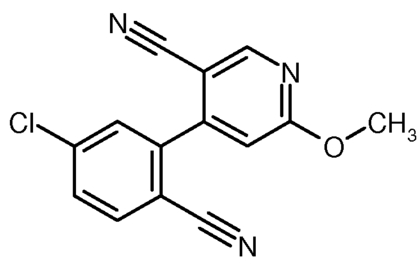

Nach der allgemeinen Methode 2A wurden 600 mg (89% Reinheit, 3.0 mmol) 5-Cyano-2-methoxypyridin-4-ylboronsäure mit 649 mg (3.0 mmol) 2-Brom-4-chlorbenzonitril in Gegenwart von [1,1-Bis-(diphenylphosphino)-ferrocen]

-palladium(II)chlorid-dichlormethan-Monoaddukt umgesetzt. Nach wässriger Aufarbeitung wurde das Rohprodukt mit Wasser und anschließend mit Cyclohexan/Ethylacetat (7:3) verrührt, der Feststoff abfiltriert und im Hochvakuum getrocknet. Ausbeute: 399 mg (94% Reinheit, 46% d. Th.)
LC/MS [Methode 1]: $R_t$ = 1.02 min; MS (ESIpos): m/z = 270 $(M+H)^+$

**Beispiel 16.1C**

4-(5-Chlor-2-cyanophenyl)-6-oxo-1,6-dihydropyridin-3-carbonitril

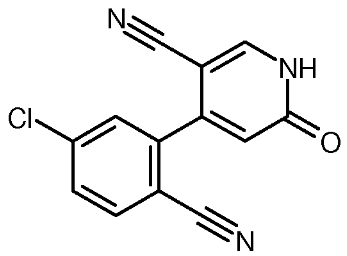

Nach der allgemeinen Methode 3A wurden 414 mg (94% Reinheit, 1.14 mmol) 4-(5-Chlor-2-cyanophenyl)-6-methoxypyridin-3-carbonitril mit Pyridinium-Hydrochlorid umgesetzt. Ausbeute: 312 mg (91% Reinheit, 77% d. Th.)
LC/MS [Methode 1]: $R_t$ = 0.71 min; MS (ESIpos): m/z = 256 $(M+H)^+$

**Beispiel 16.1D**

2-[4-(5-Chlor-2-cyanophenyl)-5-cyano-2-oxopyridin-1(2*H*)-yl]propansäure (Racemat)

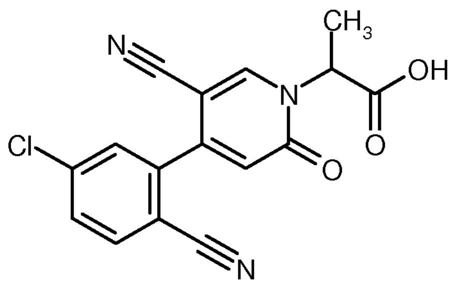

Nach der allgemeinen Methode 4A wurden 312 mg (91% Reinheit, 1.11 mmol) 4-(5-Chlor-2-cyanophenyl)-6-oxo-1,6-dihydropyridin-3-carbonitril mit 1.5 eq. 2-Brompropansäure (Racemat) bei 45°C umgesetzt. Ausbeute: 240 mg (85% Reinheit, 56% d. Th.)
LC/MS [Methode 1]: $R_t$ = 0.78 min; MS (ESIpos): m/z = 328 $(M+H)^+$

**Beispiel 16.1E**

4-({2-[4-(5-Chlor-2-cyanophenyl)-5-cyano-2-oxopyridin-1(2*H*)-yl]propanoyl}amino)benzoesäure-*tert.*-butylester (Racemat)

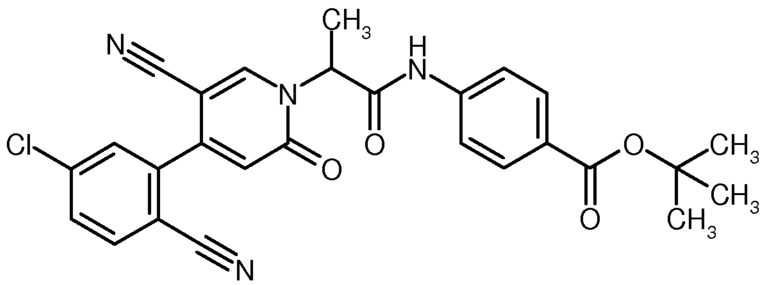

Nach der allgemeinen Methode 5A wurden 240 mg (85% Reinheit, 0.62 mmol) 2-[4-(5-Chlor-2-cyanophenyl)-5-cyano-2-oxopyridin-1(2*H*)-yl]propansäure (Racemat) mit 1.1 eq. 4-Aminobenzoesäure-*tert*.-butylester umgesetzt. Ausbeute: 93 mg (30% d. Th.)
LC/MS [Methode 1]: $R_t$ = 1.17 min; MS (ESIneg): m/z = 501 $(M-H)^-$

**Beispiel 17.1A**

4-[5-Chlor-2-(difluormethyl)phenyl]-6-methoxypyridin-3-carbonitril

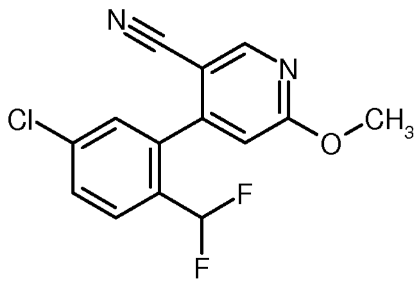

Nach der allgemeinen Methode 2B wurden 724 mg (3.0 mmol) 5-Chlor-2-methoxypyridin-4-ylboronsäure mit 600 mg (3.0 mmol) 2-Brom-4-chlor-1-(difluormethyl)benzol in Gegenwart von XPhos Prekatalysator umgesetzt. Ausbeute: 143 mg (65% Reinheit, 11% d. Th.)
LC/MS [Methode 1]: $R_t$ = 1.15 min; MS (ESIpos): m/z = 295 $(M+H)^+$

**Beispiel 17.1B**

4-[5-Chlor-2-(difluormethyl)phenyl]-6-oxo-1,6-dihydropyridin-3-carbonitril

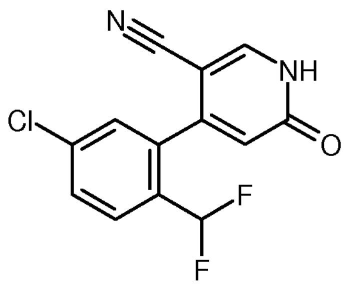

Nach der allgemeinen Methode 3A wurden 143 mg (65% Reinheit, 0.32 mmol) 4-[5-Chlor-2-(difluormethyl)phenyl]-6-methoxypyridin-3-carbonitril mit Pyridinium-Hydrochlorid umgesetzt. Ausbeute: 88 mg (99% d. Th.)
LC/MS [Methode 1]: $R_t$ = 0.82 min; MS (ESIpos): m/z = 281 $(M+H)^+$

**Beispiel 17.1C**

2-{4-[5-Chlor-2-(difluormethyl)phenyl]-5-cyano-2-oxopyridin-1(2*H*)-yl}propansäure (Racemat)

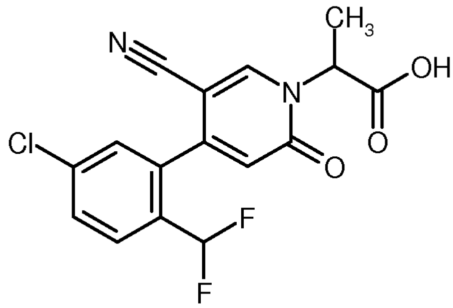

Nach der allgemeinen Methode 4A wurden 88 mg (0.31 mmol) 4-[5-Chlor-2-(difluormethyl)phenyl]-6-oxo-1,6-dihydropyridin-3-carbonitril mit 1.5 eq. 2-Brompropansäure (Racemat) bei 45°C umgesetzt. Ausbeute: 88 mg (92% Reinheit, 73% d. Th.)
LC/MS [Methode 1]: $R_t$ = 0.87 min; MS (ESIpos): m/z = 353 $(M+H)^+$

**Beispiel 17.1D**

4-[(2-{4-[5-Chlor-2-(difluormethyl)phenyl]-5-cyano-2-oxopyridin-1(2*H*)-yl}propanoyl)amino]-benzoesäure-*tert*.-butylester (Racemat)

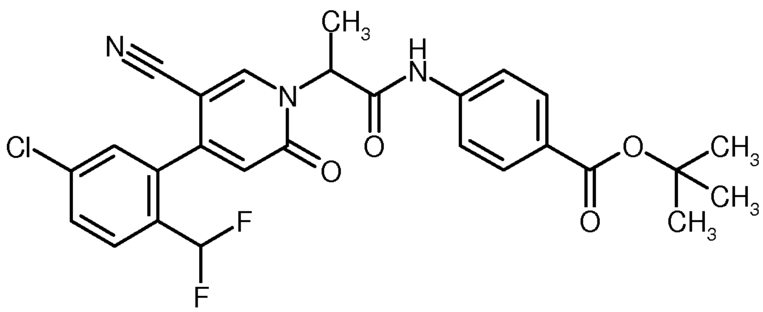

Nach der allgemeinen Methode 5A wurden 88 mg (92% Reinheit, 0.23 mmol) 2-{4-[5-Chlor-2-(difluormethyl)phenyl]-5-cyano-2-oxopyridin-1(2*H*)-yl}propansäure (Racemat) mit 1.1 eq. 4-Aminobenzoesäure-*tert*.-butylester umgesetzt. Ausbeute: 48 mg (36% d. Th.)
LC/MS [Methode 1]: $R_t$ = 1.24 min; MS (ESIpos): m/z = 528 $(M+H)^+$

**Beispiel 18.1A**

4-Iod-6-methoxypyridin-3-carbonitril

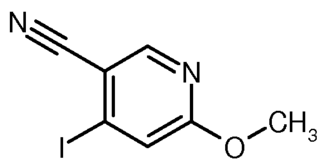

Eine Lösung von 4.0 g (29.8 mmol) 6-Methoxypyridin-3-carbonitril in 120 ml THF wurde bei -78°C mit 19.4 ml Lithiumdiisopropylamid (2 molar in THF/Heptan/Ethylbenzol, 1.3 eq.) versetzt und 1 h bei -78°C gerührt. Anschließend wurde das Reaktionsgemisch bei -78°C mit einer Lösung von 9.1 g (35.8 mmol) Iod in 20 ml THF versetzt, 1 h bei -78°C gerührt und vorsichtig auf gesättigte, wässrige Ammoniumchlorid-Lösung gegeben. Nach Zugabe von Ethylacetat wurde das Reaktionsgemisch dreimal mit Ethylacetat extrahiert. Die vereinigten, organischen Phasen wurden getrocknet (Magnesiumsulfat), filtriert und im Vakuum eingeengt. Das Rohprodukt wurde mittels Flash-Chromatographie (Kieselgel-60, Eluent: Cyclohexan-Ethylacetat-Gemische) aufgereinigt. Ausbeute: 4.0 g (92% Reinheit, 48% d. Th.)
GC [Methode 7]: $R_t$ = 5.08 min; MS (EI) m/z = 260 $(M)^+$
$^1$H-NMR (400 MHz, DMSO-$d_6$): δ [ppm] = 8.63 (s, 1H), 7.61 (s, 1H), 3.92 (s, 1H).

**Beispiel 18.1B**

4-Brom-6-oxo-1,6-dihydropyridin-3-carbonitril

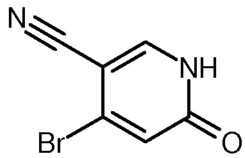

Nach der allgemeinen Methode 3A wurden 4.5 g (90% Reinheit, 15.6 mmol) 4-Iod-6-methoxypyridin-3-carbonitril mit 20 eq. Pyridinium-Hydrobromid umgesetzt. Nach wässriger Aufarbeitung wurde das Rohprodukt ohne weitere Reinigung weiter umgesetzt. Ausbeute: 1.99 g (77% Reinheit, 49% d. Th.) der Titelverbindung im Gemisch mit 11% der analogen Iodverbindung.
LC/MS [Methode 1]: $R_t$ = 0.48 min; MS (ESIpos): m/z = 199 $(M+H)^+$

**Beispiel 18.1C**

2-(4-Brom-5-cyano-2-oxopyrdin-1(2*H*)-yl)propansäure (Racemat)

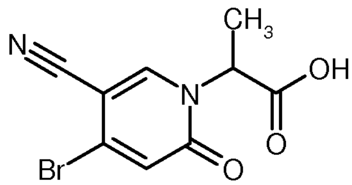

Nach der allgemeinen Methode 4A wurden 1.0 g (77% Reinheit, 3.87 mmol) 4-Brom-6-oxo-1,6-dihydropyridin-3-carbonitril mit 1.5 eq. 2-Brompropansäure (Racemat) bei 35-45°C umgesetzt. Nach wässriger Aufarbeitung wurde das Rohprodukt mit Cyclohexan/Dichlormethan verrührt, der Feststoff abfiltriert und im Hochvakuum getrocknet. Ausbeute: 648 mg (66% Reinheit, 41% d. Th.)
Das Filtrat wurde im Vakuum eingeengt und der Rückstand mittels präparativer HPLC (Reprosil C18, Wasser-Acetonitril-Gradient) aufgereinigt. Ausbeute: 110 mg (91% Reinheit, 10% d. Th.)
LC/MS [Methode 1]: $R_t$ = 0.58 min; MS (ESIpos): m/z = 271 $(M+H)^+$

**Beispiel 18.1D**

4-{[2-(4-Brom-5-cyano-2-oxopyridin-1(2*H*)-yl)propanoyl]amino}benzoesäure-*tert*.-butylester (Racemat)

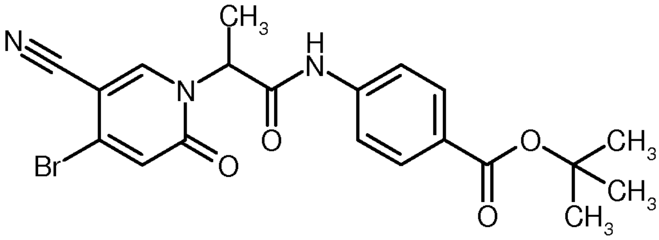

Nach der allgemeinen Methode 5A wurden 750 mg (70% Reinheit, 1.94 mmol) 2-(4-Brom-5-cyano-2-oxopyridin-1(2*H*)-yl)propansäure (Racemat) mit 1.2 eq. 4-Aminobenzoesäure-*tert*.-butylester umgesetzt. Ausbeute: 704 mg (70% Reinheit, 57% d. Th.)
LC/MS [Methode 1]: $R_t$ = 1.12 min; MS (ESIneg): m/z = 444 $(M-H)^-$

**Beispiel 18.1E**

4-[(2-{4-[5-Chlor-2-(trifluormethyl)phenyl]-5-cyano-2-oxopyridin-1(2*H*)-yl}propanoyl)amino]-benzoesäure-*tert*.-butylester (Racemat)

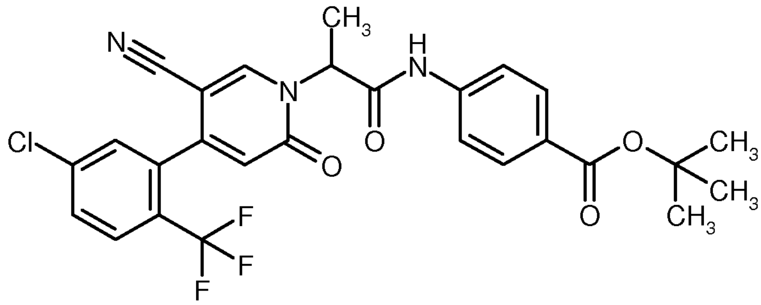

Nach der allgemeinen Methode 2A wurden 127 mg (70% Reinheit, 0.2 mmol) 4-{[2-(4-Brom-5-cyano-2-oxopyridin-1(2*H*)-yl)propanoyl]amino}benzoesäure-*tert*.-butylester (Racemat) mit 54 mg (0.24 mmol) 5-Chlor-2-trifluormethylphenylboronsäure in Gegenwart von [1,1-Bis-(diphenylphosphino)-ferrocen]-palladium(II)chlorid-dichlormethan-Monoaddukt umgesetzt. Nach wässriger Aufarbeitung wurde das Rohprodukt in Wasser verrührt, der Feststoff filtriert, getrocknet und mittels Flash-Chromatographie (Kieselgel-60, Eluent: Cyclohexan-Ethylacetat-Gemische) weiter aufgereinigt. Ausbeute: 124 mg (quant.)
LC/MS [Methode 1]: $R_t$ = 1.28 min; MS (ESIpos): m/z = 546 $(M+H)^+$
$^1$H-NMR (400 MHz, DMSO-$d_6$): $\delta$ [ppm] = 10.84 (d, 1H), 8.79 (s, 1H), 7.96 (dd, 1H), 7.89 (d, 2H), 7.84 (m, 2H), 7.71 (d, 2H), 6.58 (s, 1H), 5.57 (m, 1H), 1.76 (d, 3H), 1.54 (s, 9H).

**Beispiel 18.2A**

4-[(2-{4-[5-Chlor-2-(trifluormethoxy)phenyl]-5-cyano-2-oxopyridin-1(2*H*)-yl}propanoyl)amino] - benzoesäure-*tert*.-butylester (Racemat)

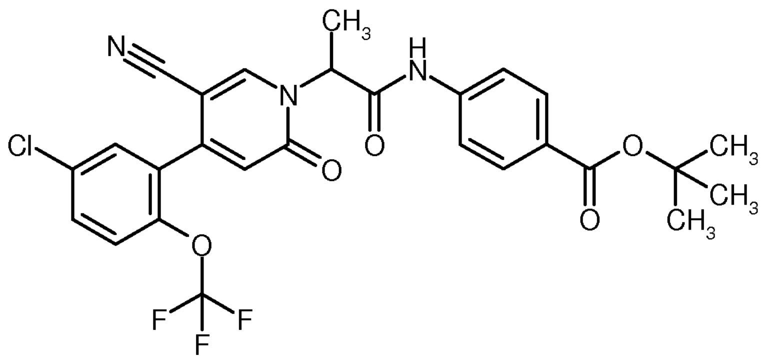

Nach der allgemeinen Methode 2A wurden 127 mg (70% Reinheit, 0.2 mmol) 4-{[2-(4-Brom-5-cyano-2-oxopyridin-1(2*H*)-yl)propanoyl]amino}benzoesäure-*tert.*-butylester (Racemat) mit 58 mg (0.24 mmol) 5-Chlor-2-trifluormethoxyphenylboronsäure in Gegenwart von [1,1-Bis-(diphenylphosphino)-ferrocen]-palladium(II)chlorid-dichlormethan-Monoaddukt umgesetzt. Ausbeute: 107 mg (94% Reinheit, 89% d. Th.)
LC/MS [Methode 1]: $R_t$ = 1.29 min; MS (ESIpos): m/z = 562 $(M+H)^+$

**Beispiel 18.3A**

4-({2-[4-(5-Chlor-2-cyclopropylphenyl)-5-cyano-2-oxopyridin-1(2*H*)-yl]propanoyl}amino)-benzoesäure-*tert.*-butylester (Racemat)

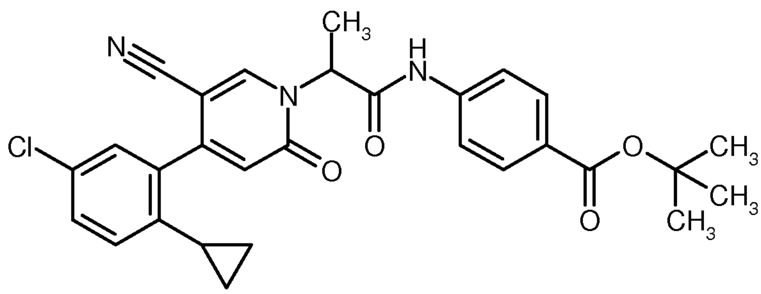

Nach der allgemeinen Methode 2A wurden 127 mg (70% Reinheit, 0.2 mmol) 4-{[2-(4-Brom-5-cyano-2-oxopyridin-1(2*H*)-yl)propanoyl]amino}benzoesäure-*tert.*-butylester (Racemat) mit 67 mg (0.24 mmol) 2-(5-Chlor-2-cyclopropylphenyl)-4,4,5,5-tetramethyl-1,3,2-dioxaborolan in Gegenwart von [1,1-Bis-(diphenylphosphino)-ferrocen] -palladium(II)chlorid-dichlormethan-Monoaddukt umgesetzt. Ausbeute: 66 mg (62% d. Th.)
LC/MS [Methode 1]: $R_t$ = 1.28 min; MS (ESIpos): m/z = 518 $(M+H)^+$

**Beispiel 19.1A**

4-Iod-6-methoxypyridin-3-carbaldehyd

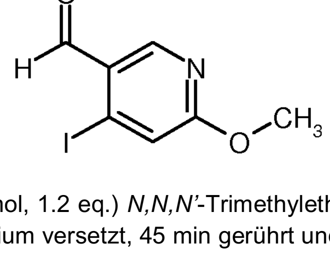

Eine Lösung von 5.7 ml (43.8 mmol, 1.2 eq.) *N,N,N'*-Trimethylethylendiamin in 135 ml THF wurde bei -78°C mit 25.1 ml (40.1 mmol, 1.1 eq.) *n*-Butyllithium versetzt, 45 min gerührt und mit 5.0 g (36.5 mmol) 6-Methoxypyridin-3-carbaldehyd versetzt. Das Reaktionsgemisch wurde nach 45 min bei -78°C mit weiteren 45.6 ml (72.9 mmol, 2.0 eq.) *n*-Butyllithium versetzt, 1 h gerührt und dabei die Temperatur auf -40°C auftauen lassen, weitere 1 h bei -40°C gerührt, wieder auf -78°C gekühlt und mit einer Lösung aus 18.5 g (72.9 mmol) Iod in 90 ml THF über einen Zeitraum von 50 min versetzt. Die Temperatur wurde weitere 4 h bei -78°C gehalten und anschließend langsam über Nacht auf RT kommen gelassen. Das Reaktionsgemisch wurde auf 300 ml gesättigte, wässrige Natriumchlorid-Lösung gegossen und nach Phasentrennung die wässrige Phase zweimal mit Ethylacetat extrahiert. Die vereinigten, organischen Phasen wurden getrocknet (Natriumsulfat), filtriert und im Vakuum eingeengt. Der Rückstand wurde mit Acetonitril verrührt, abfiltriert und der Niederschlag im HV getrocknet. Ausbeute: 647 mg (91% Reinheit, 6% d. Th.).

Weiterer Niederschlag aus der Mutterlauge wurde filtriert und im Vakuum getrocknet. Ausbeute: 1050 mg (70% Reinheit, 8% d. Th.).

Die vereinigten Mutterlaugen wurden im Vakuum eingeengt und der Rückstand mittels Flash-Chromatographie (Kieselgel-60, Eluent: Cyclohexan-Ethylacetat-Gemische) weiter aufgereinigt. Ausbeute: 1188 mg (75% Reinheit, 9% d. Th.).

LC/MS [Methode 1]: $R_t$ = 0.90 min; MS (ESIpos): m/z = 264 $(M+H)^+$

$^1$H-NMR (400 MHz, DMSO-$d_6$): $\delta$ [ppm] = 9.89 (s, 1H), 8.52 (s, 1H), 7.56 (s, 1H), 3.94 (s, 3H).

**Beispiel 19.1B**

5-(Difluormethyl)-4-iod-2-methoxypyridin

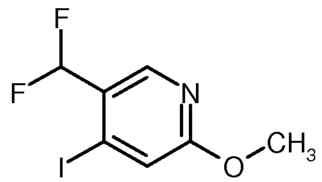

Eine Lösung von 1.0 g (91% Reinheit, 3.46 mmol) 4-Iod-6-methoxypyridin-3-carbaldehyd in 30 ml Dichlormethan wurde bei 0°C mit 0.7 ml (5.1 mmol, 1.5 eq.) Diethylaminoschwefeltrifluorid versetzt und über Nacht bei RT gerührt. Das Reaktionsgemisch wurde zu einer gesättigten Natriumhydrogencarbonat-Lösung getropft bis keine Kohlendioxid-Entwicklung mehr zu erkennen war. Nach Zugabe von Ethylacetat und Phasentrennung wurde die wässrige Phase zweimal mit Ethylacetat extrahiert. Die vereinigten, organischen Phasen wurden mit gesättigter, wässriger Natriumchlorid-Lösung gewaschen, getrocknet (Natriumsulfat), filtriert und kurz (!) im Vakuum eingeengt und getrocknet. Ausbeute: 616 mg (83% Reinheit, 52% d. Th.)

LC/MS [Methode 1]: $R_t$ = 1.04 min; MS (ESIpos): m/z = 286 $(M+H)^+$

**Beispiel 19.1C**

4-Chlor-2-[5-(difluormethyl)-2-methoxypyridin-4-yl]benzonitril

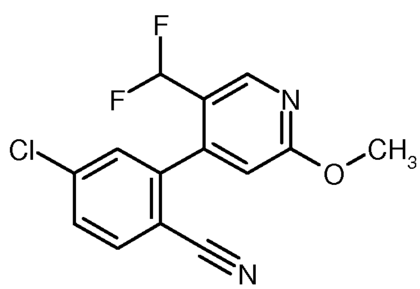

Nach der allgemeinen Methode 2A wurden 616 mg (83% Reinheit, 1.79 mmol) 5-(Difluormethyl)-4-iod-2-methoxypyridin mit 325 mg (1.79 mmol) 5-Chlor-2-cyanophenylboronsäure in Gegenwart von [1,1-Bis-(diphenylphosphino)-ferrocen]-palladium(II)chlorid-dichlormethan-Monoaddukt umgesetzt. Ausbeute: 223 mg (42% d. Th.)

LC/MS [Methode 1]: $R_t$ = 1.11 min; MS (ESIpos): m/z = 295 $(M+H)^+$

**Beispiel 19.1D**

4-Chlor-2-[5-(difluormethyl)-2-oxo-1,2-dihydropyridin-4-yl]benzonitril

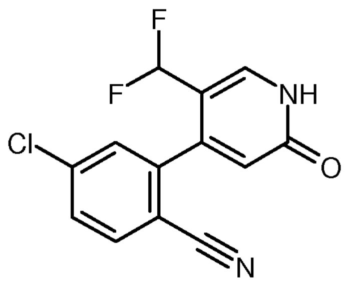

Nach der allgemeinen Methode 3A wurden 216 mg (0.73 mmol) 4-Chlor-2-[5-(difluormethyl)-2-methoxypyridin-4-yl]benzonitril mit Pyridinium-Hydrobromid umgesetzt. Ausbeute: 215 mg (64% Reinheit, 67% d. Th.)
LC/MS [Methode 1]: $R_t$ = 0.81 min; MS (ESIpos): m/z = 281 $(M+H)^+$

**Beispiel 19.1E**

2-[4-(5-Chlor-2-cyanophenyl)-5-(difluormethyl)-2-oxopyridin-1(2*H*)-yl]propansäure (Racemat)

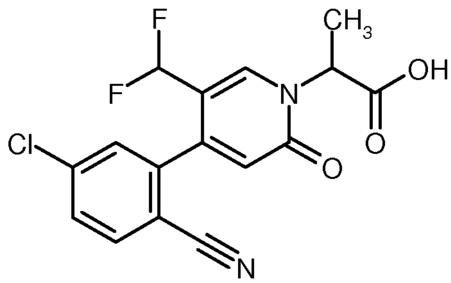

Nach der allgemeinen Methode 4A wurden 215 mg (64% Reinheit, 0.5 mmol) 4-Chlor-2-[5-(difluormethyl)-2-oxo-1,2-dihydropyridin-4-yl]benzonitril mit 1.5 eq. 2-Brompropansäure (Racemat) bei 50°C umgesetzt. Ausbeute: 256 mg Rohprodukt (64% Reinheit, 95% d. Th.)
LC/MS [Methode 1]: $R_t$ = 0.86 min; MS (ESIpos): m/z = 353 $(M+H)^+$

**Beispiel 19.1F**

4-({2-[4-(5-Chlor-2-cyanophenyl)-5-(difluormethyl)-2-oxopyridin-1(2*H*)-yl]propanoyl}amino)-benzoesäure-tert.-butylester (Racemat)

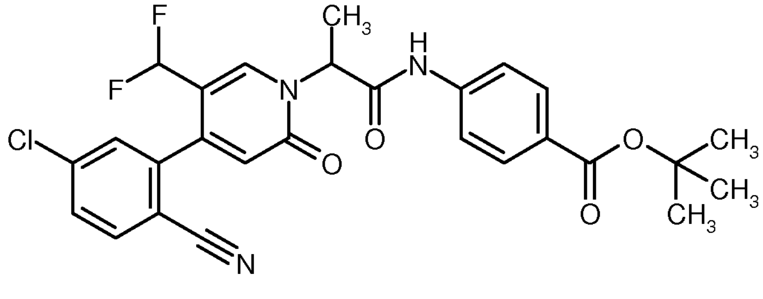

Nach der allgemeinen Methode 5A wurden 65 mg (88% Reinheit, 0.16 mmol) 2-[4-(5-Chlor-2-cyanophenyl)-5-(difluormethyl)-2-oxopyridin-1(2*H*)-yl]propansäure (Racemat) mit 1.2 eq. 4-Amino-benzoesäure-*tert*.-butylester umgesetzt. Ausbeute: 65 mg (76% d. Th.)
LC/MS [Methode 1]: $R_t$ = 1.23 min; MS (ESIpos): m/z = 528 $(M+H)^+$
$^1$H-NMR (400 MHz, DMSO-$d_6$): $\delta$ [ppm] = 10.85 (s, 1H), 8.30 (s, 1H), 8.02 (d, 1H), 7.88 (d, 2H), 7.74 (m, 4H), 6.85 (br. t, 1H), 6.56 (s, 1H), 5.58 (q, 1H), 1.74 (d, 3H), 1.55 (s, 9H).

**Beispiel 20.1A**

2-Methoxy-5-trifluormethylpyridin-4-ylboronsäure

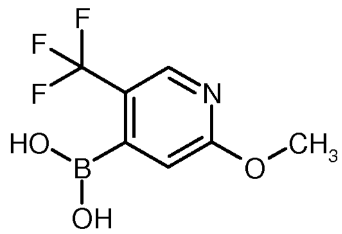

Nach der allgemeinen Methode 1A wurden 10 g (56.5 mmol) 2-Methoxy-5-(trifluormethyl)pyridin umgesetzt. Ausbeute: 4.4 g (34% d. Th.)
$^1$H-NMR (400 MHz, DMSO-$d_6$): $\delta$ [ppm] = 8.63 (br. s, 2H), 8.50 (s, 1H), 6.91 (s, 1H), 3.92 (s, 3H).

**Beispiel 20.1B**

4-Chlor-2-[2-methoxy-5-(trifluormethyl)pyridin-4-yl]benzonitril

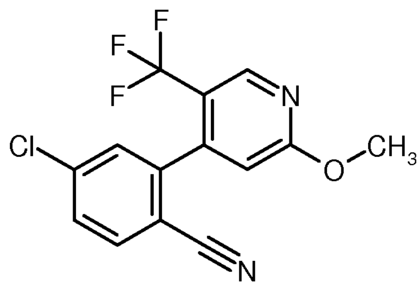

Nach der allgemeinen Methode 2A wurden 1.0 g (4.4 mmol) 2-Methoxy-5-trifluormethylpyridin-4-ylboronsäure mit 0.95 g (4.4 mmol) 2-Brom-4-chlorbenzonitril in Gegenwart von [1,1-Bis-(diphenylphosphino)-ferrocen]-palladium(II)chlorid-dichlormethan-Monoaddukt umgesetzt. Ausbeute: 351 mg (71% Reinheit, 18% d. Th.)
LC/MS [Methode 1]: $R_t$ = 1.19 min; MS (ESIpos): m/z = 313 $(M+H)^+$
$^1$H-NMR (400 MHz, DMSO-$d_6$): $\delta$ [ppm] = 8.76 (s, 1H), 8.07 (d, 1H), 7.81 (dd, 1H), 7.77 (s, 1H), 7.16 (s, 1H), 4.01 (s, 3H).

**Beispiel 20.1C**

4-Chlor-2-[2-oxo-5-(trifluormethyl)-1,2-dihydropyridin-4-yl]benzonitril

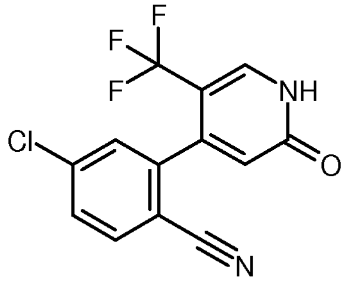

Nach der allgemeinen Methode 3A wurden 450 mg (71% Reinheit, 1.02 mmol) 4-Chlor-2-[2-methoxy-5-(trifluormethyl)pyridin-4-yl]benzonitril mit 20 eq. Pyridinium-Hydrochlorid umgesetzt. Nach wässriger Aufarbeitung wurde das Rohprodukt mittels Flash-Chromatographie (Kieselgel-60, Dichlormethan-Methanol-Gemische) aufgereinigt. Ausbeute: 456 mg (86% Reinheit, quant.)
LC/MS [Methode 1]: $R_t$ = 0.89 min; MS (ESIpos): m/z = 299 $(M+H)^+$
$^1$H-NMR (400 MHz, DMSO-$d_6$): δ [ppm] = 12.58 (br. s, 1H), 8.09 (s, 1H), 8.03 (d, 1H), 7.77 (dd, 1H), 7.74 (s, 1H), 6.51 (s, 1H).

**Beispiel 20.1D**

2-[4-(5-Chlor-2-cyanophenyl)-2-oxo-5-(trifluormethyl)pyridin-1(2*H*)-yl]propansäure (Racemat)

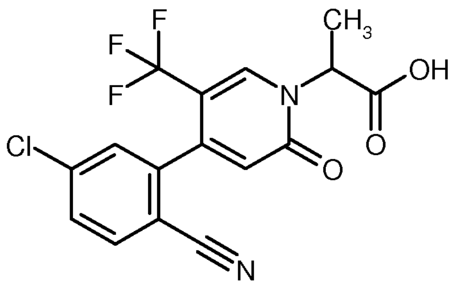

Nach der allgemeinen Methode 4A wurden 456 mg (86% Reinheit, 1.31 mmol) 4-Chlor-2-[2-oxo-5-(trifluormethyl)-1,2-dihydropyridin-4-yl]benzonitril mit 1.5 eq. 2-Brompropansäure (Racemat) bei 50°C umgesetzt. Ausbeute: 515 mg (51 % Reinheit, 54% d. Th.)
LC/MS [Methode 1]: $R_t$ = 0.91 min; MS (ESIpos): m/z = 371 $(M+H)^+$

**Beispiel 20.1E**

4-({2-[4-(5-Chlor-2-cyanophenyl)-2-oxo-5-(trifluormethyl)pyridin-1(2*H*)-yl]propanoyl}amino)-benzoesäure-*tert*.-butylester (Racemat)

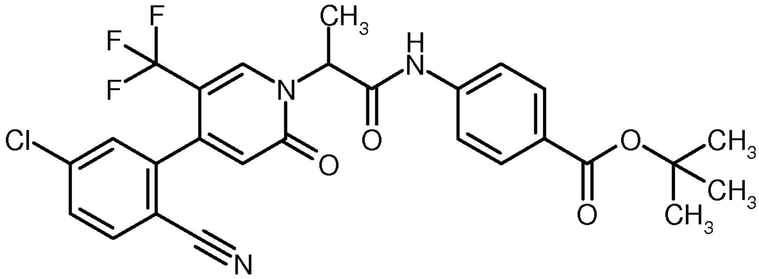

Nach der allgemeinen Methode 5A wurden 515 mg (51% Reinheit, 0.71 mmol) 2-[4-(5-Chlor-2-cyanophenyl)-2-oxo-5-(trifluormethyl)pyridin-1(2*H*)-yl]propansäure (Racemat) mit 1.2 eq. 4-Amino-benzoesäure-*tert*.-butylester umgesetzt. Ausbeute: 251 mg (79% Reinheit, 51% d. Th.)
LC/MS [Methode 1]: $R_t$ = 1.30 min; MS (ESIpos): m/z = 546 $(M+H)^+$

**Beispiel 21.1A**

2,5-Dimethoxypyridin-4-ylboronsäure

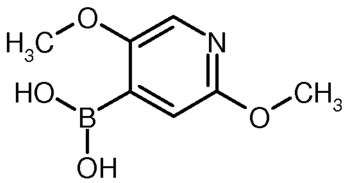

Nach der allgemeinen Methode 1A wurden 11.53 g (82.9 mmol) 2,5-Dimethoxypyridin umgesetzt. Nach Ansäuren der wässrigen Phase fiel das gewünschte Produkt als Niederschlag aus. Ausbeute: 9.53 g (61% d. Th.)
LC/MS [Methode 1]: $R_t$ = 0.47 min; MS (ESIpos): m/z = 184 $(M+H)^+$

**Beispiel 21.1B**

4-Chlor-2-(2,5-dimethoxypyridin-4-yl)benzonitril

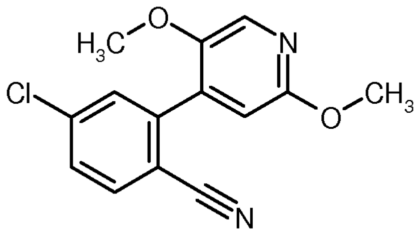

Nach der allgemeinen Methode 2A wurden 7.87 g (95% Reinheit, 40.86 mmol) 2,5-Dimethoxypyridin-4-ylboronsäure mit 8.85 g (40.86 mmol) 2-Brom-4-chlorbenzonitril in Gegenwart von [1,1-Bis-(diphenylphosphino)-ferrocen]-palladium(II)chlorid-dichlormethan-Monoaddukt umgesetzt. Ausbeute: 6.23 g (92% Reinheit, 51% d. Th.)
LC/MS [Methode 1]: $R_t$ = 1.08 min; MS (ESIpos): m/z = 275 $(M+H)^+$

**Beispiel 21.1C**

4-Chlor-2-(5-methoxy-2-oxo-1,2-dihydropyridin-4-yl)benzonitril

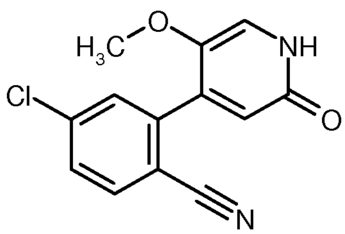

Nach der allgemeinen Methode 3A wurden 7.23 g (92% Reinheit, 24.21 mmol) 4-Chlor-2-(2,5-dimethoxypyridin-4-yl)benzonitril mit Pyridinium-Hydrochlorid umgesetzt. Ausbeute: 6.66 g (91% Reinheit, 96% d. Th.)
LC/MS [Methode 1]: $R_t$ = 0.76 min; MS (ESIpos): m/z = 261 $(M+H)^+$

$^1$H-NMR (400 MHz, DMSO-$d_6$): δ [ppm] = 11.45 (br. s, 1H), 7.98 (d, 1H), 7.75-7.67 (m, 2H), 7.29 (br. s, 1H), 6.43 (s, 1H), 3.64 (s, 3H).

**Beispiel 21.1D**

2-[4-(5-Chlor-2-cyanophenyl)-5-methoxy-2-oxopyridin-1(2*H*)-yl]propansäure (Racemat)

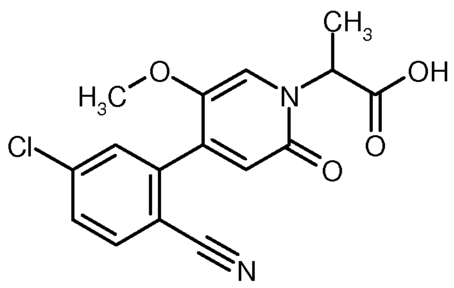

Nach der allgemeinen Methode 4A wurden 599 mg (87% Reinheit, 2.00 mmol) 4-Chlor-2-(5-methoxy-2-oxo-1,2-dihydropyridin-4-yl)benzonitril mit 1.5 eq. 2-Brompropansäure (Racemat) bei 90°C umgesetzt. Ausbeute: 716 mg (68% Reinheit, 73% d. Th.)
LC/MS [Methode 1]: $R_t$ = 0.80 min; MS (ESIpos): m/z = 333 $(M+H)^+$
$^1$H-NMR (400 MHz, DMSO-$d_6$): δ [ppm] = 7.99 (d, 1H), 7.73 (m, 2H), 7.48 (s, 1H), 6.50 (s, 1H), 5.17 (q, 1H), 3.65 (s, 3H), 1.61 (d, 3H).

**Beispiel 21.1E**

4-({2-[4-(5-Chlor-2-cyanophenyl)-5-methoxy-2-oxopyridin-1(2*H*)-yl]propanoyl}amino)-benzoesäure-*tert*. -butylester (Racemat)

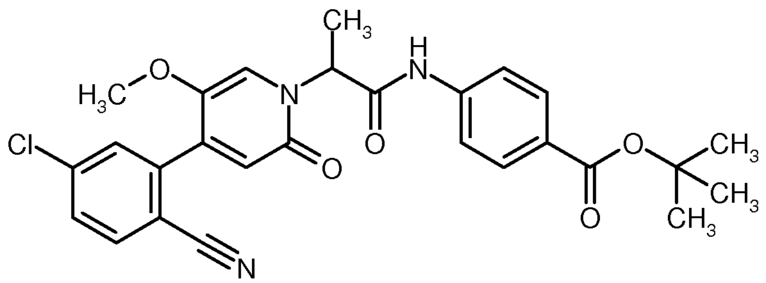

Nach der allgemeinen Methode 5A wurden 1.53 g (73% Reinheit, 3.35 mmol) 2-[4-(5-Chlor-2-cyanophenyl)-5-methoxy-2-oxopyridin-1(2*H*)-yl]propansäure (Racemat) mit 1.1 eq. 4-Aminobenzoesäure-*tert*.-butylester umgesetzt. Ausbeute: 1.52 g (93% Reinheit, 83% d. Th.)
LC/MS [Methode 1]: $R_t$ = 1.19 min; MS (ESIpos): m/z = 508 $(M+H)^+$
$^1$H-NMR (400 MHz, DMSO-$d_6$): δ [ppm] = 10.72 (s, 1H), 8.01 (d, 1H), 7.87 (d, 2H), 7.74 (m, 4H), 7.46 (s, 1H), 6.53 (s, 1H), 5.59 (q, 1H), 3.70 (s, 3H), 1.74 (d, 3H), 1.54 (s, 9H).

**Beispiel 21.2A**

4-(5-Chlor-2-nitrophenyl)-2,5-dimethoxypyridin

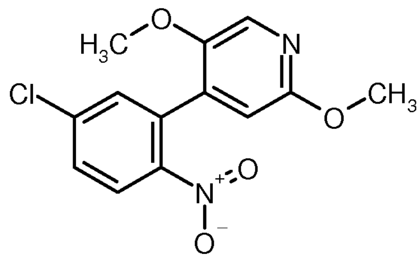

Nach der allgemeinen Methode 2B wurden 215 mg (85% Reinheit, 1.0 mmol) 2,5-Dimethoxypyridin-4-ylboronsäure mit 236 mg (1.0 mmol) 2-Brom-4-chlor-1-nitrobenzol in Gegenwart von XPhos Prekatalysator umgesetzt. Ausbeute: 124 mg (93% Reinheit, 39% d. Th.)
LC/MS [Methode 2]: $R_t$ = 3.22 min; MS (ESIpos): m/z = 295 $(M+H)^+$

**Beispiel 21.2B**

4-(5-Chlor-2-nitrophenyl)-5-methoxypyridin-2(1*H*)-on

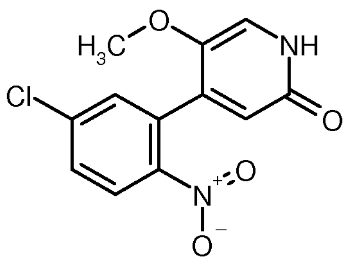

Nach der allgemeinen Methode 3A wurden 124 mg (93% Reinheit, 0.39 mmol) 4-(5-Chlor-2-nitrophenyl)-2,5-dimethoxypyridin mit Pyridinium-Hydrochlorid umgesetzt. Ausbeute: 115 mg (85% Reinheit, 89% d. Th.)
LC/MS [Methode 1]: $R_t$ = 0.74 min; MS (ESIpos): m/z = 281 $(M+H)^+$

**Beispiel 21.2C**

2-[4-(5-Chlor-2-nitrophenyl)-5-methoxy-2-oxopyridin-1(2*H*)-yl]propansäure (Racemat)

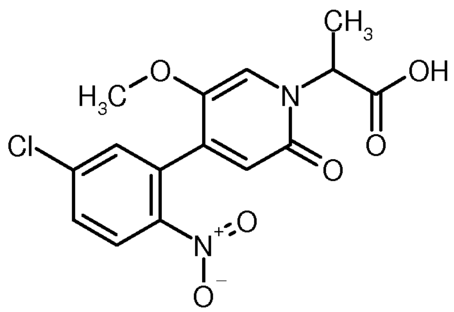

Nach der allgemeinen Methode 4A wurden 115 mg (85% Reinheit, 0.35 mmol) 4-(5-Chlor-2-nitrophenyl)-5-methoxypyridin-2(1*H*)-on mit 1.5 eq. 2-Brompropansäure (Racemat) bei 50°C umgesetzt. Ausbeute: 43 mg (35% d. Th.)
LC/MS [Methode 1]: $R_t$ = 0.80 min; MS (ESIpos): m/z = 353 $(M+H)^+$

**Beispiel 21.2D**

4-({2-[4-(5-Chlor-2-nitrophenyl)-5-methoxy-2-oxopyridin-1(2*H*)-yl]propanoyl}amino)-benzoesäure-*tert*.-butylester (Racemat)

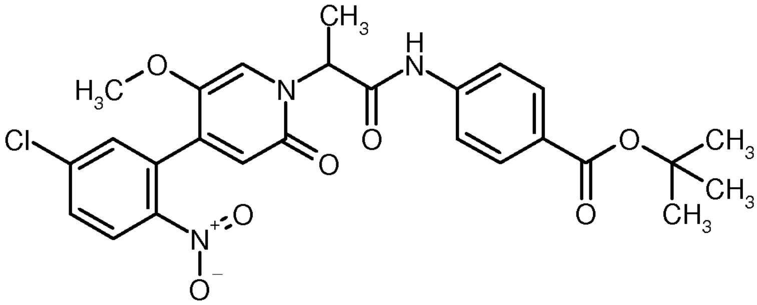

Eine Lösung von 30 mg (0.09 mmol) 2-[4-(5-Chlor-2-nitrophenyl)-5-methoxy-2-oxopyridin-1(2*H*)-yl]propansäure (Racemat) in 2 ml DMF wurde unter Argon bei RT mit 25 mg (0.13 mmol) 4-Aminobenzoesäure-*tert*.-butylester, 44 µl (0.26 mmol) *N,N*-Diisopropylethylamin und 74 µl (50%ig in DMF, 0.13 mmol) T3P versetzt und 2 h bei RT gerührt. Das Reaktionsgemisch wurde im Vakuum eingeengt und der Rückstand mittels präparativer HPLC (Reprosil C18, Wasser-Acetonitril-Gradient) gereinigt. Ausbeute: 32 mg (71% d. Th.)
LC/MS [Methode 1]: $R_t$ = 1.19 min; MS (ESIpos): m/z = 528 $(M+H)^+$

**Beispiel 22.1A**

[4-(5-Chlor-2-cyanophenyl)-5-methoxy-2-oxopyridin-1(2*H*)-yl]essigsäure-*tert*.-butylester

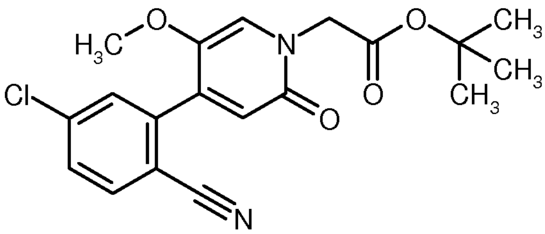

Nach der allgemeinen Methode 4B wurden 516 mg (91% Reinheit, 1.8 mmol) 4-Chlor-2-(5-methoxy-2-oxo-1,2-dihydropyridin-4-yl)benzonitril mit 1.2 eq. Bromessigsäure-*tert*.-butylester bei 100°C umgesetzt. Ausbeute: 464 mg (68% d. Th.)
LC/MS [Methode 1]: $R_t$ = 1.00 min; MS (ESIpos): m/z = 375 $(M+H)^+$

**Beispiel 22.1B**

2-[4-(5-Chlor-2-cyanophenyl)-5-methoxy-2-oxopyridin-1(2*H*)-yl]-3-cyclopropylpropansäure-*tert*.-butylester (Racemat)

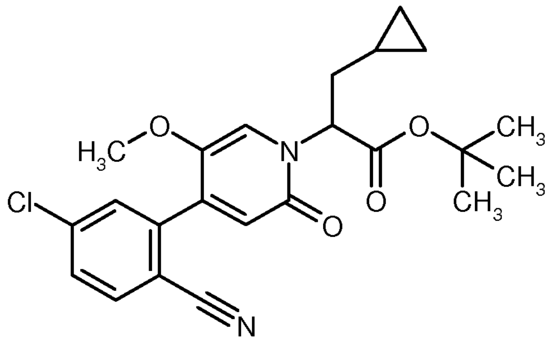

Nach der allgemeinen Methode 7A wurden 464 mg (1.24 mmol) [4-(5-Chlor-2-cyanophenyl)-5-methoxy-2-oxopyridin-1(2*H*)-yl]essigsäure-*tert*.-butylester mit 293 mg (1.61 mmol) (Iodmethyl)cyclopropan umgesetzt. Ausbeute: 379 mg (71% d. Th.)
LC/MS [Methode 1]: $R_t$ = 1.18 min; MS (ESIpos): m/z = 429 $(M+H)^+$

**Beispiel 22.1C**

2-[4-(5-Chlor-2-cyanophenyl)-5-methoxy-2-oxopyridin-1(2*H*)-yl]-3-cyclopropylpropansäure (Racemat)

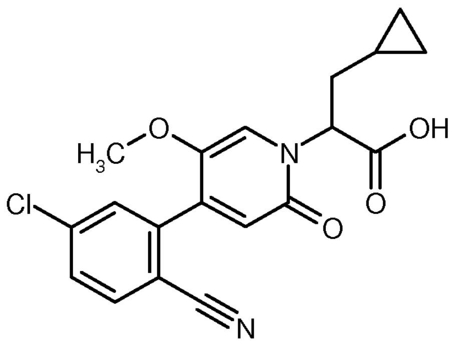

Nach der allgemeinen Methode 6A wurden 378 mg (0.88 mmol) 2-[4-(5-Chlor-2-cyanophenyl)-5-methoxy-2-oxopyridin-1(2*H*)-yl]-3-cyclopropylpropansäure-*tert*.-butylester (Racemat) mit 20 eq. TFA verseift. Ausbeute: 420 mg (92% Reinheit, quant.)
LC/MS [Methode 1]: $R_t$ = 0.90 min; MS (ESIpos): m/z = 373 $(M+H)^+$

**Beispiel 22.1D**

4-({2-[4-(5-Chlor-2-cyanophenyl)-5-methoxy-2-oxopyridin-1(2*H*)-yl]-3-cyclopropylpropanoyl}-amino)-benzoesäure-*tert*.-butylester (Racemat)

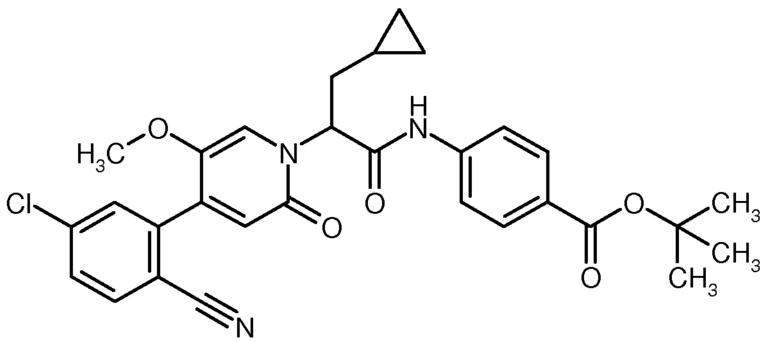

Nach der allgemeinen Methode 5A wurden 420 mg (92% Reinheit, 1.04 mmol) 2-[4-(5-Chlor-2-cyanophenyl)-5-methoxy-2-oxopyridin-1(2*H*)-yl]-3-cyclopropylpropansäure (Racemat) mit 1.2 eq. 4-Aminobenzoesäure-*tert*.-butylester umgesetzt. Ausbeute: 348 mg (61% d. Th.)
LC/MS [Methode 1]: $R_t$ = 1.29 min; MS (ESIpos): m/z = 548 $(M+H)^+$

**Beispiel 23.1A**

2-[4-(5-Chlor-2-cyanophenyl)-5-methoxy-2-oxopyridin-1(2*H*)-yl]pent-4-insäure-*tert*.-butylester (Racemat)

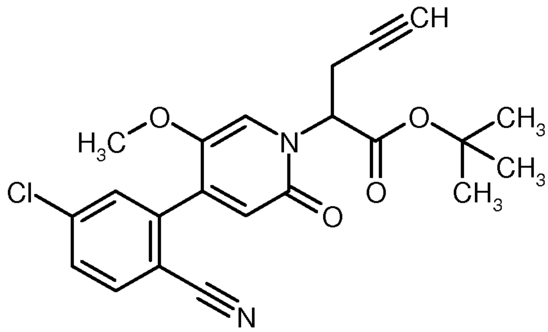

Nach der allgemeinen Methode 7A wurden 309 mg (0.8 mmol) [4-(5-Chlor-2-cyanophenyl)-5-methoxy-2-oxopyridin-1(2*H*)-yl]essigsäure-*tert*.-butylester mit 155 mg (1.04 mmol) 3-Bromprop-1-in umgesetzt. Ausbeute: 288 mg (87% d. Th.)
LC/MS [Methode 1]: $R_t$ = 1.08 min; MS (ESIpos): m/z = 413 $(M+H)^+$

**Beispiel 23.1B**

2-[4-(5-Chlor-2-cyanophenyl)-5-methoxy-2-oxopyridin-1(2*H*)-yl]pent-4-insäure (Racemat)

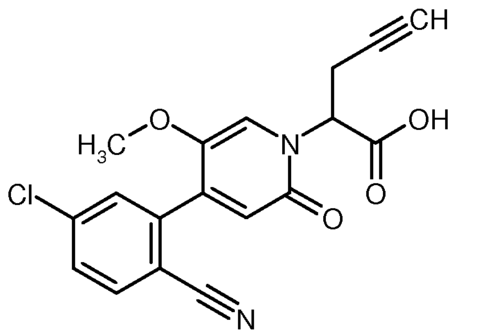

Nach der allgemeinen Methode 6A wurden 288 mg (0.7 mmol) 2-[4-(5-Chlor-2-cyanophenyl)-5-methoxy-2-oxopyridin-1(2*H*)-yl]pent-4-insäure-*tert*.-butylester (Racemat) mit TFA verseift. Ausbeute: 295 mg (85% Reinheit, quant.)
LC/MS [Methode 1]: $R_t$ = 0.81 min; MS (ESIpos): m/z = 357 $(M+H)^+$

**Beispiel 23.1C**

4-({2-[4-(5-Chlor-2-cyanophenyl)-5-methoxy-2-oxopyridin-1(2*H*)-yl]pent-4-inoyl}amino)-benzoesäure-*tert*.-butylester (Racemat)

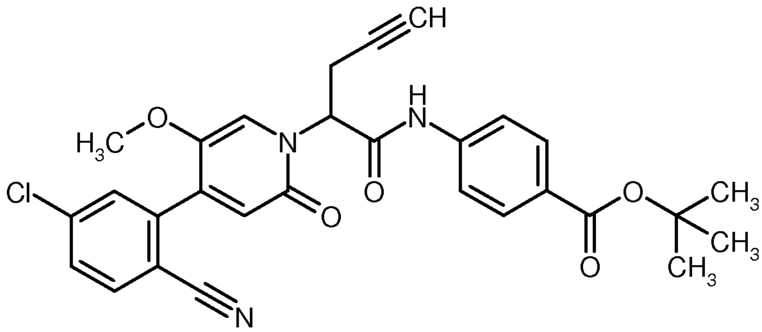

Nach der allgemeinen Methode 5A wurden 295 mg (85% Reinheit, 0.70 mmol) 2-[4-(5-Chlor-2-cyanophenyl)-5-methoxy-2-oxopyridin-1(2*H*)-yl]pent-4-insäure (Racemat) mit 1.1 eq. 4-Aminobenzoesäure-*tert*.-butylester umgesetzt. Ausbeute: 91 mg (24% d. Th.)
LC/MS [Methode 1]: $R_t$ = 1.23 min; MS (ESIpos): m/z = 532 $(M+H)^+$

**Beispiel 24.1A**

6-Methoxypyridin-3-ol

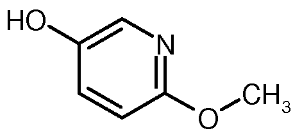

Eine Lösung von 46.0 g (392 mmol) *N*-Methylmorpholin-*N*-Oxid in 500 ml Dichlormethan wurde bei RT mit 50 g (327 mmol) 6-Methoxypyridin-3-ylboronsäure versetzt und 14 h bei 50°C gerührt. Es wurde noch zusätzliches *N*-Methylmorpholin-*N*-Oxid bis zur vollständigen Umsetzung zugegeben. Das Reaktionsgemisch wurde im Vakuum eingeengt und das Rohprodukt mittels Flash-Chromatographie (Kieselgel-60, Cyclohexan-Ethylacetat-Gemische) aufgereinigt. Ausbeute: 32.9 g (80% d. Th.)
LC/MS [Methode 1]: $R_t$ = 0.37 min; MS (ESIpos): m/z = 126 $(M+H)^+$
$^1$H-NMR (400 MHz, DMSO-$d_6$): $\delta$ [ppm] = 9.27 (s, 1H), 7.67 (d, 1H), 7.16 (dd, 1H), 6.66 (d, 1H), 3.74 (s, 3H).

**Beispiel 24.1B**

2-Methoxy-5-(tetrahydro-2*H*-pyran-2-yloxy)pyridin

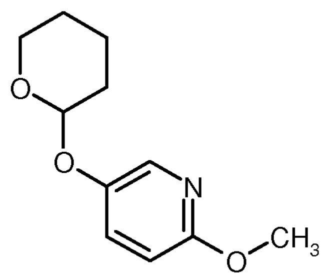

Eine Lösung von 10.0 g (79.9 mmol) 6-Methoxypyridin-3-ol in 150 ml Dichlormethan wurde mit 10.1 g (119.9 mmol, 1.5 eq.) 3,4-Dihydro-2*H*-pyran und 1.4 g (8.0 mmol, 0.1 eq.) 4-Toluolsulfonsäure versetzt und 5 d bei RT gerührt. Nach Zugabe von Wasser/Dichlormethan und Phasentrennung wurde die wässrige Phase mit Dichlormethan extrahiert. Die vereinigten, organischen Phasen wurden getrocknet (Natriumsulfat), filtriert und im Vakuum eingeengt. Ausbeute: 17.3 g (100% d. Th.)

LC/MS [Methode 1]: $R_t$ = 0.95 min; MS (ESIpos): m/z = 210 $(M+H)^+$

**Beispiel 24.1C**

4-Iod-2-methoxy-5-(tetrahydro-2*H*-pyran-2-yloxy)pyridin

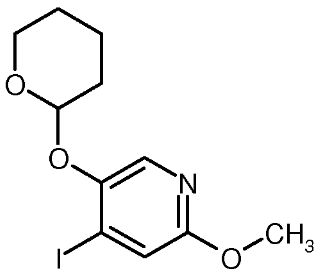

Eine Lösung von 16.2 g (75.1 mmol) 2-Methoxy-5-(tetrahydro-2*H*-pyran-2-yloxy)pyridin in 250 ml THF wurde bei -78°C mit 13.6 ml (90.1 mmol, 1.2 eq.) 1,2-Bis(dimethylamino)ethan und 54.0 ml (86.4 mmol, 1.15 eq.) *n*-Butyllithium versetzt und 1 h bei -78°C gerührt. Anschließend wurde das Reaktionsgemisch mit 24.8 g (97.6 mmol, 1.3 eq.) Iod versetzt, 1 h bei -78°C gerührt und dann über Nacht auf RT kommen gelassen. Das Reaktionsgemisch wurde mit Wasser gequencht und dreimal mit Ethylacetat extrahiert. Die vereinigten, organischen Phasen wurden mit gesättigter Natriumthiosulfat-Lösung gewaschen, getrocknet (Natriumsulfat), filtriert und im Vakuum eingeengt. Ausbeute: 25.1 g (82% Reinheit, 82% d. Th.)
LC/MS [Methode 1]: $R_t$ = 1.18 min; MS (ESIpos): m/z = 336 $(M+H)^+$

**Beispiel 24.1D**

4-Iod-6-methoxypyridin-3-ol

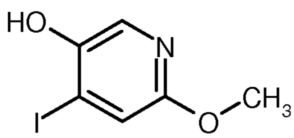

Eine Lösung von 25.1 g (82% Reinheit, 61.3 mmol) 4-Iod-2-methoxy-5-(tetrahydro-2*H*-pyran-2-yloxy)pyridin in 50 ml Dioxan und 50 ml Wasser wurde mit 50 ml (3 molar, 150 mmol) Salzsäure versetzt und 2 h bei RT gerührt. Anschließend wurde das Reaktionsgemisch filtriert, der Niederschlag mit Wasser nachgewachsen und am Hochvakuum getrocknet. Ausbeute: 13.5 g (93% Reinheit, 81% d. Th.)
LC/MS [Methode 1]: $R_t$ = 0.76 min; MS (ESIpos): m/z = 252 $(M+H)^+$
$^1$H-NMR (400 MHz, DMSO-$d_6$): $\delta$ [ppm] = 7.70 (s, 1H), 7.22 (s, 1H), 3.74 (s, 3H).

**Beispiel 24.1E**

4-Iod-5-isopropoxy-2-methoxypyridin

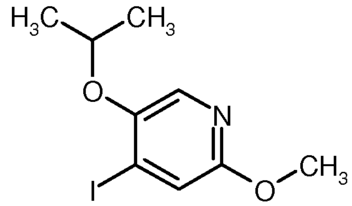

Eine Lösung von 861 mg (3.4 mmol) 4-Iod-6-methoxypyridin-3-ol in 15 ml Aceton wurden bei 0°C mit 758 mg (4.5 mmol) 2-Iodpropan und 948 mg (6.9 mmol, 2 eq.) Kaliumcarbonat versetzt, über Nacht bei 80°C gerührt und im Vakuum eingeengt. Nach Zugabe von Wasser/Ethylacetat und Phasentrennung wurde die wässrige Phase mit Ethylacetat extrahiert. Die vereinigten, organischen Phasen wurden getrocknet (Natriumsulfat), filtriert und im Vakuum eingeengt. Ausbeute: 741 mg (73% d. Th.)
LC/MS [Methode 1]: $R_t$ = 1.16 min; MS (ESIpos): m/z = 294 $(M+H)^+$

**Beispiel 24.1F**

4-Iod-5-isopropoxypyridin-2(1*H*)-on

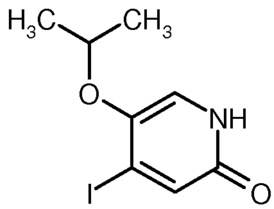

Nach der allgemeinen Methode 3A wurden 741 mg (2.53 mmol) 4-Iod-2-methoxy-5-(propan-2-yloxy)pyridin mit 20 eq. Pyridinium-Hydrobromid umgesetzt. Ausbeute: 413 mg (92% Reinheit) eines Gemisches (1.4: 1) aus der Iodverbindung 24.1F und der analogen Bromverbindung LC/MS [Methode 1]: Bromverbindung: $R_t$ = 0.71 min; MS (ESIpos): m/z = 232 $(M+H)^+$; Iodverbindung: $R_t$ = 0.74 min; MS (ESIpos): m/z = 280 $(M+H)^+$

**Beispiel 24.1G**

2-(4-Iod-5-isopropoxy-2-oxopyridin-1(2*H*)-yl)propansäure (Racemat)

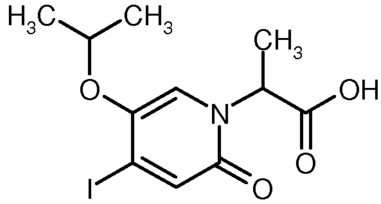

Nach der allgemeinen Methode 4A wurden 414 mg (92% Reinheit) eines Gemisches (1.4:1) aus 4-Iod-5-(propan-2-yloxy)pyridin-2(1*H*)-on und der analogen Bromverbindung mit 1.5 eq. 2-Brompropansäure (Racemat) bei 50°C umgesetzt. Ausbeute: 771 mg (90% Reinheit) eines Gemisches (1.6: 1) aus der Iodverbindung 24.1G und der analogen Bromverbindung
LC/MS [Methode 1]: Bromverbindung: $R_t$ = 0.78 min; MS (ESIpos): m/z = 304 $(M+H)^+$; Iodverbindung: $R_t$ = 0.80 min; MS (ESIpos): m/z = 352 $(M+H)^+$

**Beispiel 24.1H**

4-{[2-(4-Iod-5-isopropoxy-2-oxopyridin-1(2*H*)-yl)propanoyl]amino}benzoesäure-*tert*.-butylester (Racemat)

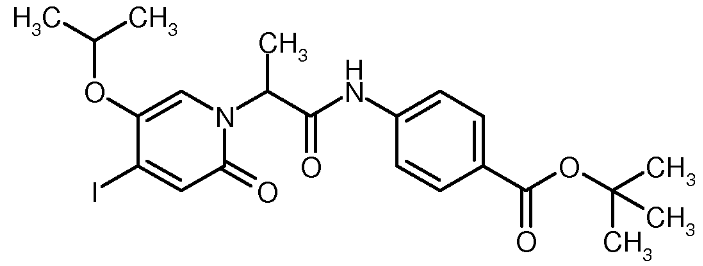

Nach der allgemeinen Methode 5A wurden 771 mg (90% Reinheit) eines Gemisches (1.6:1) aus 2-[4-Iod-2-oxo-5-(propan-2-yloxy)pyridin-1(2*H*)-yl]propansäure (Racemat) und der analogen Bromverbindung mit 1.2 eq. 4-Aminobenzoesäure-*tert*.-butylester umgesetzt. Ausbeute: 100 mg eines Gemisches (3:1) aus der Iodverbindung 24.1H und der analogen Bromverbindung
LC/MS [Methode 1]: Bromverbindung: $R_t$ = 1.22 min; MS (ESIpos): m/z = 479 $(M+H)^+$; Iodverbindung: $R_t$ = 1.24 min; MS (ESIpos): m/z = 527 $(M+H)^+$

**Beispiel 24.1I**

4-({2-[4-(5-Chlor-2-cyanophenyl)-5-isopropoxy-2-oxopyridin-1(2*H*)-yl]propanoyl}amino)-benzoesäure-*tert*.-butylester (Racemat)

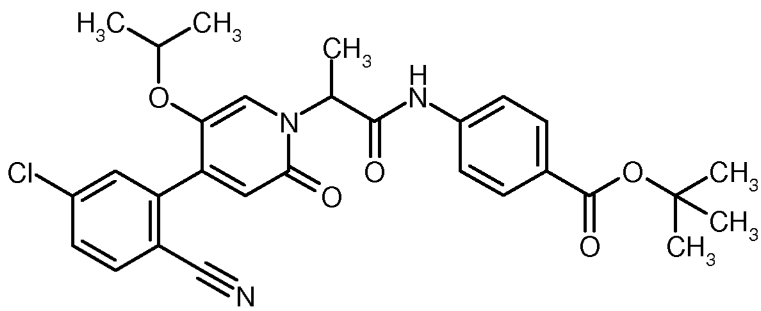

Nach der allgemeinen Methode 2A wurden 100 mg eines Gemisches (3:1) aus 4-({2-[4-Iod-2-oxo-5-(propan-2-yloxy)pyrdin-1(2*H*)-yl]propanoyl}amino)benzoesäure-*tert*.-butylester (Racemat) und der analogen Bromverbindung mit 41 mg (0.23 mmol) 5-Chlor-2-cyanophenylboronsäure in Gegenwart von [1,1-Bis-(diphenylphosphino)-ferrocen] -palladium(II)chlorid-dichlormethan-Monoaddukt umgesetzt. Ausbeute: 31 mg (29% d. Th.)
LC/MS [Methode 1]: $R_t$ = 1.31 min; MS (ESIpos): m/z = 536 $(M+H)^+$

**Beispiel 25.1A**

2-[4-(5-Chlor-2-cyanophenyl)-5-methoxy-2-oxopyridin-1(2*H*)-yl]-4-methylpentansäure-*tert*.-butylester (Racemat)

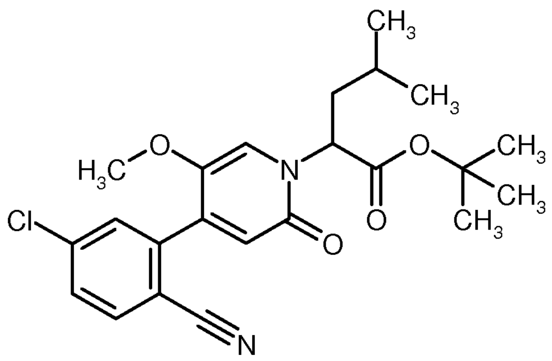

Nach der allgemeinen Methode 7A wurden 309 mg (0.80 mmol) [4-(5-Chlor-2-cyanophenyl)-5-methoxy-2-oxopyridin-1(2*H*)-yl]essigsäure-*tert*.-butylester mit 191 mg (1.04 mmol) Isobutyliodid umgesetzt. Ausbeute: 178 mg (92% Reinheit, 48% d. Th.) Produkt.
LC/MS [Methode 1]: $R_t$ = 1.25 min; MS (ESIpos): m/z = 431 $(M+H)^+$

**Beispiel 25.1B**

2-[4-(5-Chlor-2-cyanophenyl)-5-methoxy-2-oxopyridin-1(2*H*)-yl]-4-methylpentansäure (Racemat)

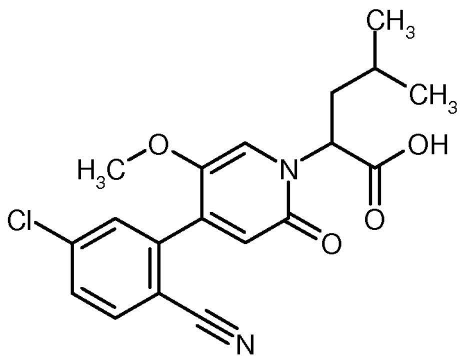

Nach der allgemeinen Methode 6A wurden 178 mg (92% Reinheit, 0.38 mmol) 2-[4-(5-Chlor-2-cyanophenyl)-5-methoxy-2-oxopyridin-1(2*H*)-yl]-4-methylpentansäure-*tert*.-butylester (Racemat) mit TFA verseift. Ausbeute: 165 mg (85% Reinheit, 98% d. Th.)
LC/MS [Methode 1]: $R_t$ = 0.95 min; MS (ESIpos): m/z = 375 $(M+H)^+$

**Beispiel 25.1C**

4-({2-[4-(5-Chlor-2-cyanophenyl)-5-methoxy-2-oxopyridin-1(2*H*)-yl]-4-methylpentanoyl}amino)-benzoesäure-*tert*.-butylester (Racemat)

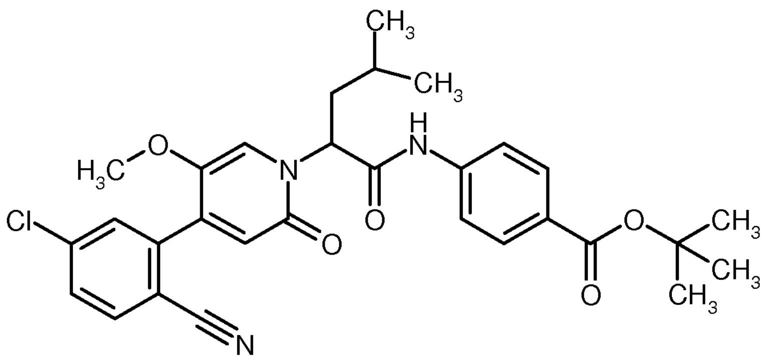

Nach der allgemeinen Methode 5A wurden 166 mg (85% Reinheit, 0.38 mmol) 2-[4-(5-Chlor-2-cyanophenyl)-5-methoxy-2-oxopyridin-1(2*H*)-yl]-4-methylpentansäure (Racemat) mit 1.1 eq. 4-Amino-benzoesäure-*tert*.-butylester umgesetzt. Ausbeute: 127 mg (60% d. Th.)
LC/MS [Methode 1]: $R_t$ = 1.33 min; MS (ESIpos): m/z = 550 $(M+H)^+$

**Beispiel 26.1A**

2-[4-(5-Chlor-2-cyanophenyl)-5-methoxy-2-oxopyridin-1(2*H*)-yl]butansäure (Racemat)

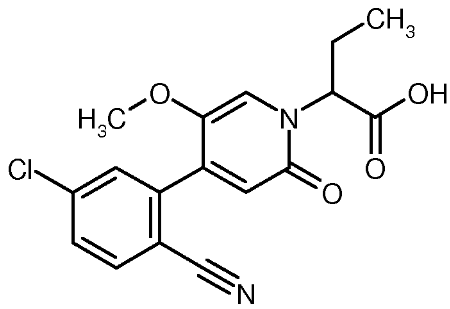

Nach der allgemeinen Methode 4A wurden 159 mg (82% Reinheit, 0.5 mmol) 4-Chlor-2-(5-methoxy-2-oxo-1,2-dihydropyridin-4-yl)benzonitril mit 1.5 eq. 2-Brombutansäure (Racemat) bei 50°C umgesetzt. Ausbeute: 55 mg (32% d. Th.)
LC/MS [Methode 1]: $R_t$ = 0.85 min; MS (ESIpos): m/z = 347 $(M+H)^+$

Alternativsynthese:

Eine Lösung von 4.1 g (10.2 mmol) 2-[4-(5-Chlor-2-cyanophenyl)-5-methoxy-2-oxopyridin-1(2*H*)-yl]butansäure-*tert*.-butylester (Racemat) in 40 ml Dichlormethan wurde unter Argon bei RT mit 7.8 ml (101.8 mmol, 10 eq.) Trifluoressigsäure versetzt, 1 h bei RT gerührt, mit weiteren 7.8 ml (101.8 mmol, 10 eq.) Trifluoressigsäure versetzt, 1 h bei RT gerührt, mit weiteren 7.8 ml (101.8 mmol, 10 eq.) Trifluoressigsäure versetzt und 1 h bei RT gerührt. Nach vollständiger Umsetzung wurde das Reaktionsgemisch im Vakuum eingeengt, der Rückstand jeweils dreimal mit Dichlormethan und einmal mit Toluol coevaporiert und im Vakuum getrocknet. Der Rückstand wurde in 100 ml Essigsäureethylester aufgenommen und mehrfach mit einer stark verdünnten, wässrigen Natriumhydrogencarbonat-Lösung gewaschen (wobei der pH-Wert des Waschwasser pH 3-4 nicht überschreiten sollte, da das Produkt ansonsten gut in Wasser löslich ist). Die organische Phase wurde anschließend getrocknet (Natriumsulfat), filtriert und im Vakuum eingeengt. Der Rückstand wurde in Methyl-*tert*.-butylether verrührt, filtriert, zweimal mit Methyl-*tert*.-butylether gewaschen und im Vakuum getrocknet. Ausbeute: 2.9 g (83% d. Th.)
LC/MS [Methode 1]: $R_t$ = 0.81 min; MS (ESIpos): m/z = 347 $(M+H)^+$,
$^1$H-NMR (400 MHz, DMSO-$d_6$): $\delta$ [ppm] = 12.97 (s, 1H), 7.99 (d, 1H), 7.77-7.70 (m, 2H), 7.41 (s, 1H), 6.49 (s, 1H), 5.09 (dd, 1H), 3.64 (s, 3H), 2.21-2.09 (m, 2H), 0.84 (t, 3H).

**Beispiel 26.1B**

4-({2-[4-(5-Chlor-2-cyanophenyl)-5-metoxy-2-oxopyridin-1(2*H*)-yl]butanoyl}amino)-benzoesäure-*tert*.-butylester (Racemat)

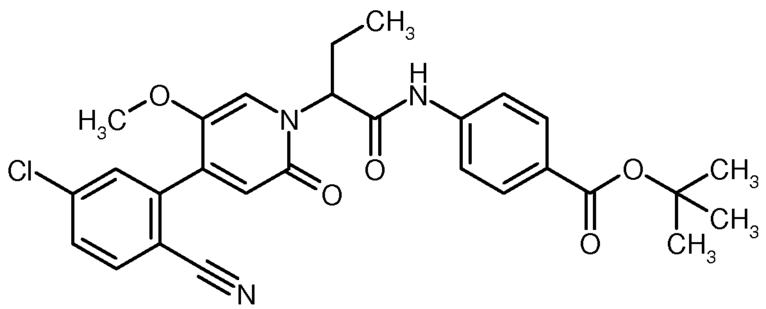

Nach der allgemeinen Methode 5A wurden 55 mg (0.16 mmol) 2-[4-(5-Chlor-2-cyanophenyl)-5-methoxy-2-oxopyridin-1(2*H*)-yl]butansäure (Racemat) mit 1.1 eq. 4-Aminobenzoesäure-*tert*.-butylester umgesetzt. Ausbeute: 68 mg (82% d. Th.)
LC/MS [Methode 1]: $R_t$ = 1.23 min; MS (ESIpos): m/z = 522 $(M+H)^+$

**Beispiel 26.2A**

5-[4-({2-[4-(5-Chlor-2-cyanophenyl)-5-methoxy-2-oxopyridin-1(2*H*)-yl]butanoyl}amino)phenyl]-3-oxo-2,3-dihydro-1*H*-pyrazol-1-carbonsäure-*tert*.-butylester (Racemat)

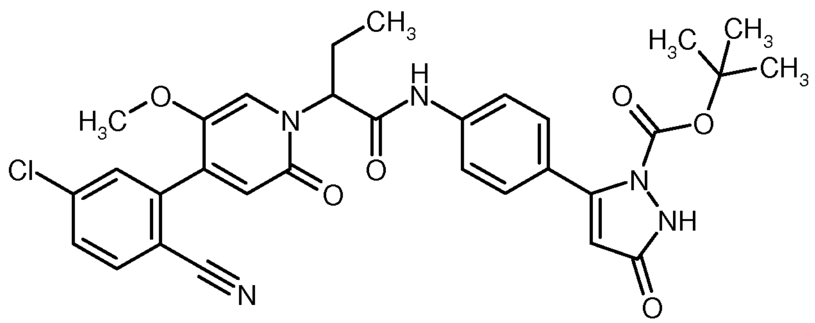

Nach der allgemeinen Methode 5A wurden 87 mg (0.25 mmol) 2-[4-(5-Chlor-2-cyanophenyl)-5-methoxy-2-oxopyridin-1(2*H*)-yl]butansäure (Racemat) mit 84 mg (90% Reinheit, 0.28 mmol, 1.1 eq.) 5-(4-Aminophenyl)-3-oxo-2,3-dihydro-1*H*-pyrazol-1-carbonsäure-*tert*.-butylester umgesetzt. Das Rohprodukt wurde mittels präparativer HPLC (Reprosil C18, Wasser-Acetonitril-Gradient) aufgereinigt. Ausbeute: 17 mg (70% Reinheit, 8% d. Th.)
LC/MS [Methode 1]: $R_t$ = 1.13 min; MS (ESIpos): m/z = 604 $(M+H)^+$.

**Beispiel 26.3A**

6-({2-[4-(5-Chlor-2-cyanophenyl)-5-methoxy-2-oxopyridin-1(2*H*)-yl]butanoyl}amino)-2-methyl-3-oxo-2,3-dihydro-1*H*-indazol-1-carbonsäure-*tert*.-butylester (Racemat)

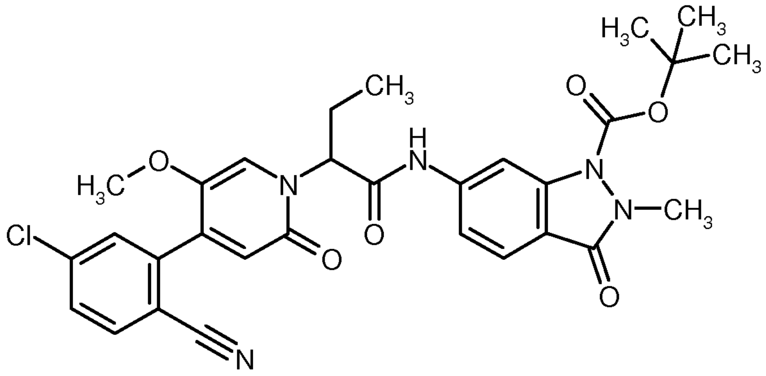

Nach der allgemeinen Methode 5A wurden 87 mg (0.25 mmol) 2-[4-(5-Chlor-2-cyanophenyl)-5-methoxy-2-oxopyridin-1(2*H*)-yl]butansäure (Racemat) mit 74 mg (0.27 mmol, 1.1 eq.) 6-Amino-2-methyl-3-oxo-2,3-dihydro-1*H*-indazol-1-carbonsäure-*tert*.-butylester umgesetzt. Das Rohprodukt wurde mittels präparativer HPLC (Reprosil C18, Wasser-Acetonitril-Gradient) aufgereinigt. Ausbeute: 112 mg (77% d. Th.)
LC/MS [Methode 1]: $R_t$ = 1.14 min; MS (ESIpos): m/z = 592 $(M+H)^+$.

**Beispiel 26.4A**

6-({2-[4-(5-Chlor-2-cyanophenyl)-5-methoxy-2-oxopyridin-1(2*H*)-yl]butanoyl}amino)-1*H*-benzimidazol-2-carbonsäureethylester (Racemat)

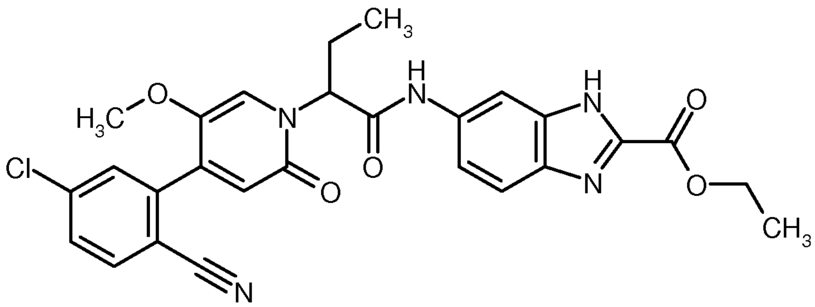

Nach der allgemeinen Methode 5A wurden 87 mg (0.25 mmol) 2-[4-(5-Chlor-2-cyanophenyl)-5-methoxy-2-oxopyridin-1(2*H*)-yl]butansäure (Racemat) mit 56 mg (0.28 mmol, 1.1 eq.) 6-Amino-1*H*-benzimidazol-2-carbonsäureethylester umgesetzt. Das Rohprodukt wurde mittels präparativer HPLC (Reprosil C18, Acetonitril/Wasser + 0.1% Ameisensäure-Gradient) aufgereinigt. Ausbeute: 86 mg (64% d. Th.)
LC/MS [Methode 1]: $R_t$ = 0.96 min; MS (ESIpos): m/z = 534 $(M+H)^+$.

**Beispiel 26.5A**

6-({2-[4-(5-Chlor-2-cyanophenyl)-5-methoxy-2-oxopyridin-1(2*H*)-yl]butanoyl}amino)-1*H*-indol-2-carbonsäureethylester (Racemat)

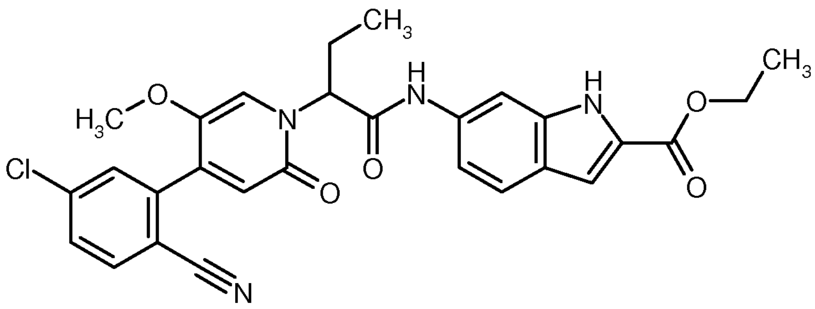

Nach der allgemeinen Methode 5A wurden 87 mg (0.25 mmol) 2-[4-(5-Chlor-2-cyanophenyl)-5-methoxy-2-oxopyridin-1(2*H*)-yl]butansäure (Racemat) mit 56 mg (0.28 mmol, 1.1 eq.) 6-Amino-1*H*-indol-2-carbonsäureethylester umgesetzt. Das Rohprodukt wurde mittels präparativer HPLC (Reprosil C18, Wasser-Acetonitril-Gradient) aufgereinigt. Ausbeute: 75 mg (55% d. Th.)
LC/MS [Methode 1]: $R_t$ = 1.09 min; MS (ESIpos): m/z = 533 $(M+H)^+$.

**Beispiel 26.6A**

5-({2-[4-(5-Chlor-2-cyanophenyl)-5-methoxy-2-oxopyridin-1(2*H*)-yl]butanoyl}amino)-1*H*-indol-2-carbonsäureethylester (Racemat)

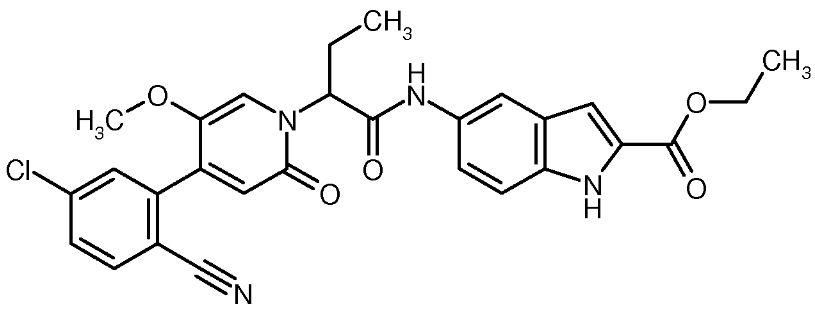

Nach der allgemeinen Methode 5A wurden 87 mg (0.25 mmol) 2-[4-(5-Chlor-2-cyanophenyl)-5-methoxy-2-oxopyridin-1(2*H*)-yl]butansäure (Racemat) mit 56 mg (0.28 mmol, 1.1 eq.) 5-Amino-1*H*-indol-2-carbonsäureethylester umgesetzt. Das Rohprodukt wurde mittels präparativer HPLC (Reprosil C18, Wasser-Acetonitril-Gradient) aufgereinigt. Ausbeute: 94 mg (70% d. Th.)
LC/MS [Methode 1]: $R_t$ = 1.09 min; MS (ESIpos): m/z = 533 $(M+H)^+$.

**Beispiel 27.1A**

1,3-Dithian-2-carbonsäure

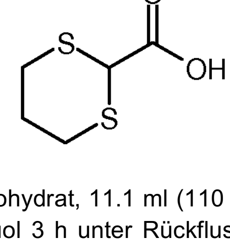

9.20 g (100 mmol) Glyoxalsäure-Monohydrat, 11.1 ml (110 mmol) 1,3-Propandithiol und 1.72 g (10.0 mmol) *para*-Toluolsulfonsäure wurden in 200 ml Toluol 3 h unter Rückfluss erhitzt. Das Reaktionsgemisch wurde auf RT abgekühlt und dreimal mit 100 ml gesättigter, wässriger Natriumhydrogencarbonat-Lösung extrahiert. Die vereinigten, wässrigen Phasen wurden mit 200 ml Diethylether gewaschen, mit wässriger Salzsäure (6N) angesäuert und viermal

mit 200 ml Essigsäureethylester extrahiert. Die vereinigten, organischen Phasen wurden über Magnesiumsulfat getrocknet, filtriert und das Lösungsmittel unter reduziertem Druck entfernt. Der Rückstand wurde ohne weitere Aufreinigung im nächsten Schritt eingesetzt. Ausbeute: 8.0 g (47% d. Th.)
LC/MS [Methode 4]: $R_t$ = 0.78 min; MS (ESIneg): m/z = 163 $(M-H)^-$ $^1$H-NMR (400 MHz, DMSO-$d_6$): δ [ppm] = 13.0 (br. s, 1H), 4.59 (s, 1H), 3.17-3.08 (m, 2H), 2.76-2.68 (m, 2H), 1.98-1.79 (m, 2H).

**Beispiel 27.1B**

1,3-Dithian-2-carbonsäure-*tert*.-butylester

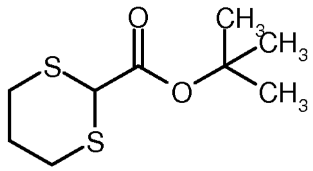

Eine Lösung von 7.54 mmol (45.9 mmol) 1,3-Dithian-2-carbonsäure in 28 ml THF/*tert*.-Butanol (1:1) wurde portionsweise mit 10.5 g (48.2 mmol) Di-tert.-butyldicarbonat und 1.68 g (13.8 mmol) Dimethylaminopyridin versetzt. Die resultierende Reaktionsmischung wurde über Nacht bei RT gerührt und mit 150 ml Essigsäureethylester verdünnt. Die organische Phase wurde nacheinander mit 100 ml gesättigter, wässriger Ammoniumchlorid-Lösung, 100 ml Wasser und 100 ml gesättigter, wässriger Natriumchlorid-Lösung gewaschen, über Natriumsulfat getrocknet, filtriert und das Lösungsmittel unter reduziertem Druck entfernt. Der Rückstand wurde mittels Flash-Chromatographie gereinigt und die Titelverbindung als kristalliner Feststoff erhalten. Ausbeute: 6.79 g (66% d. Th.)
LC/MS [Methode 4]: $R_t$ = 2.28 min; MS (ESIpos): m/z = 221 $(M+H)^+$
$^1$H-NMR (400 MHz, DMSO-$d_6$): δ [ppm] = 4.50 (s, 1H), 3.20-3.12 (m, 2H), 2.73-2.65 (m, 2H), 1.92-1.80 (m, 2H), 1.45 (s, 9H).

**Beispiel 27.1C**

1-Iod-2-methoxyethan

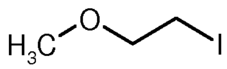

10.4 g (75.0 mmol) 1-Brom-2-methoxyethan und 13.5 g (90.0 mmol) Natriumiodid wurden in 75 ml Aceton 14 h bei RT gerührt. Anschließend wurde bei 25°C und 220 mbar das Lösungsmittel entfernt und der Rückstand in 100 ml Essigsäureethylester aufgenommen. Die organische Phase wurde zweimal mit 50 ml Wasser gewaschen, über Natriumsulfat getrocknet, filtriert und das Lösungsmittel unter reduziertem Druck entfernt. Das Rohprodukt wurde ohne weitere Aufreinigung im nächsten Schritt eingesetzt. Ausbeute: 12.5 g (90% d. Th.)
GC/MS [Methode 9]: $R_t$ = 1.56 min; MS: m/z = 186 $(M)^+$
$^1$H-NMR (400 MHz, $CDCl_3$): δ [ppm] = 3.66 (t, 2H), 3.41 (s, 3H), 3.26 (t, 2H).

**Beispiel 27.1D**

2-(2-Methoxyethyl)-1,3-dithian-2-carbonsäure-*tert*.-butylester

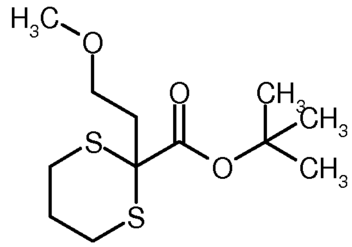

10.2 g (46.1 mmol) 1,3-Dithian-2-carbonsäure-*tert.*-butylester und 12.0 g (64.5 mmol) 1-Iod-2-methoxyethan wurden in 127 ml Dimethylformamid vorgelegt, auf 0°C abgekühlt und mit 6.21 g (55.3 mmol) Kalium-*tert.*-butanolat versetzt. Die resultierende Reaktionsmischung wurde 1 h bei 0°C und 16 h bei RT gerührt. Die Reaktionsmischung wurde auf 1.5 1 einer 1:2-Mischung aus Eis und gesättigter, wässriger Ammoniumchlorid-Lösung geben und dreimal mit 300 ml Diethylether extrahiert. Die vereinigten, organischen Phasen wurden über Magnesiumsulfat getrocknet, filtriert und das Lösungsmittel unter reduziertem Druck entfernt. Das Rohprodukt wurde ohne weitere Aufreinigung im nächsten Schritt eingesetzt. Ausbeute: 11.1 g (87% d. Th.)
LC/MS [Methode 1]: $R_t$ = 1.14 min; MS (ESIpos): m/z = 177 (M-COO-tert-Butyl)$^+$
$^1$H-NMR (400 MHz, DMSO-$d_6$): δ [ppm] = 3.46 (t, 2H), 3.19 (s, 3H), 3.13-3.06 (m, 2H), 2.78-2.72 (m, 2H), 2.15 (t, 2H), 2.08-1.98 (m, 1H), 1.75-1.64 (m, 1H), 1.45 (s, 9H).

**Beispiel 27.1E**

4-Methoxy-2-oxobutansäure-*tert.*-butylester

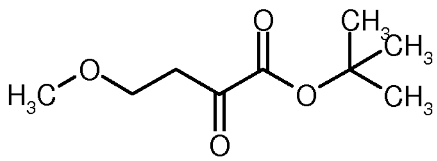

Zu einer auf -18°C gekühlten Lösung von 54.2 g (305 mmol) *N*-Bromsuccinimid in 365 ml Aceton und 18 ml Wasser wurde eine Lösung von 10.6 g (38.1 mmol) 2-(2-Methoxyethyl)-1,3-dithian-2-carbonsäure-*tert.*-butylester in 365 ml Aceton und 18 ml Wasser getropft, so dass die interne Temperatur -5°C nicht überstieg. Nach vollständiger Zugabe wurde 10 min nachgerührt und die Reaktion anschließend mit 630 ml Natriumsulfit-Lösung (1N) beendet. Die Reaktionsmischung wurde mit 420 ml *n*-Heptan versetzt und nach Phasentrennung dreimal mit 315 ml Essigsäureethylester extrahiert. Die vereinigten, organischen Phasen wurden über Natriumsulfat getrocknet, filtriert und das Lösungsmittel bei 25°C und 75 mbar entfernt. Die erhaltene Suspension wurde mit 100 ml *n*-Hexan verrührt und der Niederschlag filtriert. Das Lösungsmittel wurde unter reduziertem Druck entfernt und die Zielverbindung erhalten. Ausbeute: 5.28 g (59% d. Th.)
GC/MS [Methode 9]: $R_t$ = 3.20 min; MS: m/z = 188 (M)$^+$
$^1$H-NMR (400 MHz, $CDCl_3$): δ [ppm] = 3.70 (t, 2H), 3.34 (s, 3H), 3.04 (t, 2H), 1.55 (s, 9H).

**Beispiel 27.1F**

2-Hydroxy-4-methoxybutansäure-*tert.*-butylester (Racemat)

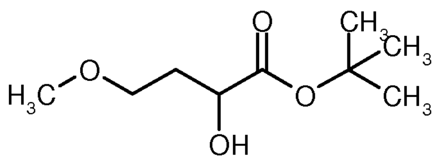

Zu einer Lösung von 5.20 g (27.6 mmol) 4-Methoxy-2-oxobutansäure-*tert.*-butylester in 68.5 ml Methanol wurden bei 0°C portionsweise 1.05 g (27.6 mmol) Natriumborhydrid gegeben. Das Reaktionsgemisch wurde 5 min nachgerührt, mit 5 ml Wasser versetzt und auf pH 6 mit wässriger Salzsäure (1N) eingestellt. Methanol wurde unter reduziertem Druck bei 30°C entfernt und die verbleibene wässrige Phase dreimal mit 50 ml Diethylether extrahiert. Die vereinigten, organischen Phasen wurden über Magnesiumsulfat getrocknet, filtriert und das Lösungsmittel unter reduziertem Druck (25°C, 70 mbar) entfernt. Ausbeute: 4.48 g (77% d. Th.)
GC/MS [Methode 9]: $R_t$ = 3.07 min; MS: m/z = 190 $(M)^+$
$^1$H-NMR (400 MHz, $CDCl_3$): δ [ppm] = 4.19-4.15 (m, 1H), 3.55 (t, 2H), 3.33 (s, 3H), 3.08 (d, 1H), 2.10-2.02 (m, 1H), 1.91-1.83 (m, 1H), 1.49 (s, 9H).

**Beispiel 27.1G**

4-Methoxy-2-{[(trifluormethyl)sulfonyl]oxy}butansäure-*tert*.-butylester (Racemat)

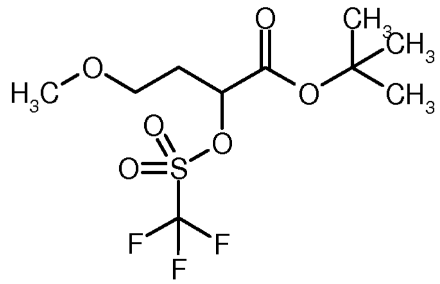

Gemäß allgemeiner Methode 8A wurden 3.15 g (16.6 mmol) 2-Hydroxy-4-methoxybutanosäure-*tert*.-butylester (Racemat) in 158 ml Dichlormethan mit 2.89 ml (24.8 mmol) Lutidin und 4.20 ml (24.8 mmol) Trifluormethansulfonsäureanhydrid umgesetzt. Ausbeute: 4.44 g (83% d. Th.)
$^1$H-NMR (400 MHz, $CDCl_3$): δ [ppm] = 5.18 (dd, 1H), 3.56-3.44 (m, 2H), 3.34 (s, 3H), 2.31-2.23 (m, 1H), 2.21-2.12 (m, 1H), 1.51 (s, 9H).

**Beispiel 27.1H**

2-[4-(5-Chlor-2-cyanophenyl)-5-methoxy-2-oxopyridin-1(2H)-yl]-4-methoxybutansäure-*tert*.-butylester (Racemat)

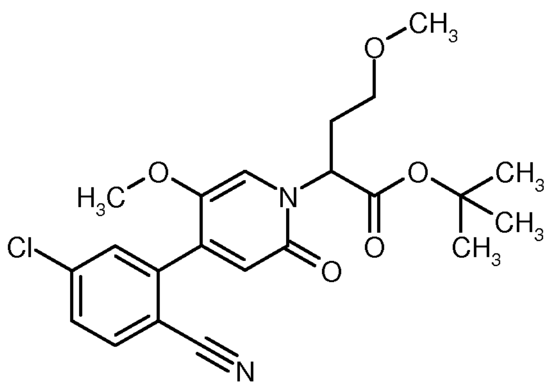

Eine Suspension von 2.4 g (9.2 mmol) 4-Chlor-2-(5-methoxy-2-oxo-1,2-dihydropyridin-4-yl)benzonitril in 70 ml Tetrahydrofuran wurde protionsweise mit 405 mg (10.1 mmol) Natriumhydrid (60%ig in Mineralöl) versetzt und 1 h bei RT nachgerührt. Zu der resultierenden Reaktionslösung wurden 4.45 g (13.8 mmol) 4-Methoxy-2-{[(trifluormethyl)sulfonyl]oxy}-butansäure-*tert*.-butylester (Racemat) als Lösung in 20 ml THF zügig zugetropft und nach vollständiger Zugabe 1.5 h bei RT nachgerührt. Die Reaktion wurde durch Zugabe von 150 ml gesättigter, wässriger Ammoniumchlorid-Lösung und 150 ml Methyl-*tert*.-butylether beendet. Die Phasen wurden getrennt und die wässrige Phase dreimal mit 130 ml Methyl-*tert*.-butylether extrahiert. Die vereinigten, organischen Phasen wurden über Magnesiumsulfat getrocknet, filtriert

und das Lösungsmittel unter reduziertem Druck entfernt. Das Rohprodukt wurde mittels Flash-Chromatographie (120 g Silica Kartusche, 85 ml/min, Cyclohexan/Essigsäureethylester-Gradient) aufgereinigt und die Titelverbindung erhalten. Ausbeute: 1.73 g (43% d. Th.)
LC/MS [Methode 1]: $R_t$ = 1.07 min; MS (ESIpos): m/z = 433 $(M+H)^+$
$^1$H-NMR (400 MHz, DMSO-$d_6$): δ [ppm] = 7.99 (d, 1H), 7.74 (s, 1H), 7.73 (dd, 1H), 7.38 (s, 1H), 6.49 (s, 1H), 5.11 (t, 1H), 3.64 (s, 3H), 3.41-3.35 (m, 1H), 3.23-3.13 (m, 1H), 3.20 (s, 3H), 2.36-2.31 (m, 2H), 1.40 (s, 9H).

**Beispiel 27.1I**

2-[4-(5-Chlor-2-cyanophenyl)-5-methoxy-2-oxopyridin-1(2*H*)-yl]-4-methoxybutansäure (Racemat)

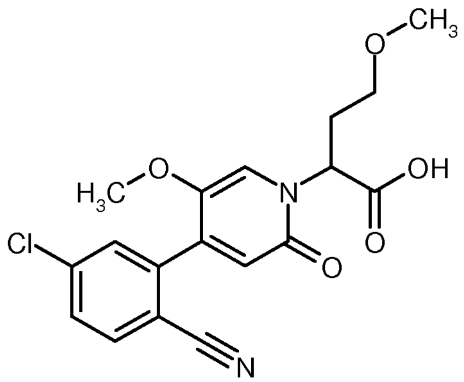

Gemäß allgemeiner Methode 6A wurden 1.99 g (4.60 mmol) 2-[4-(5-Chlor-2-cyanophenyl)-5-methoxy-2-oxopyridin-1(2*H*)-yl]-4-methoxybutansäure-*tert*.-butylester (Racemat) in 46 ml Dichlormethan mit 13.3 ml (172 mmol) TFA umgesetzt. Ausbeute: 1.58 g (91% d. Th.)
LC/MS [Methode 1]: $R_t$ = 0.82 min; MS (ESIneg): m/z = 374 $(M-H)^-$
$^1$H-NMR (400 MHz, DMSO-$d_6$): δ [ppm] = 13.0 (br. s, 1H), 7.99 (d, 1H), 7.75-7.72 (m, 2H), 7.42 (s, 1H), 6.48 (s, 1H), 5.13 (t, 1H), 3.63 (s, 3H), 3.41-3.31 (m, 1H), 3.19 (s, 3H), 3.15-3.10 (m, 1H), 2.38-2.33 (m, 2H).

**Beispiel 27.1J**

4-({2-[4-(5-Chlor-2-cyanophenyl)-5-methoxy-2-oxopyridin-1(2*H*)-yl]-4-methoxybutanoyl}-amino)benzoesäureethylester (Racemat)

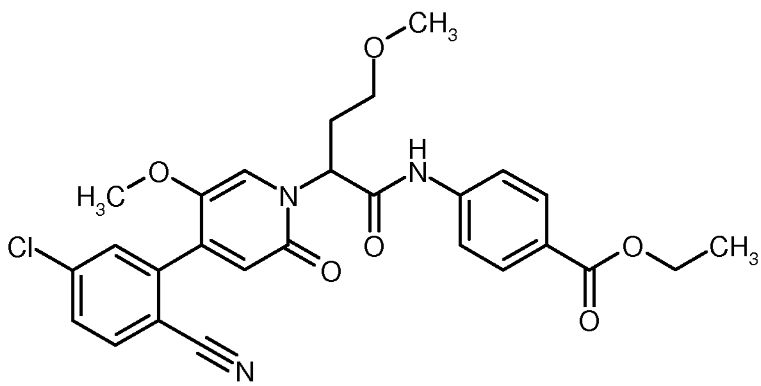

Gemäß allgemeiner Methode 5B wurden 1.50 g (3.98 mmol) 2-[4-(5-Chlor-2-cyanophenyl)-5-methoxy-2-oxopyridin-1(2*H*)-yl]-4-methoxybutansäure (Racemat), 658 mg (3.98 mmol) 4-Aminobenzoesäureethylester, 566 mg (3.98 mmol) Oxima und 620 µl (3.98 mmol) DIC in 39 ml Dimethylformamid zur Reaktion gebracht. Nach Filtration wurde die

Titelverbindung erhalten. Ausbeute: 1.87 g (85% d. Th.)
LC/MS [Methode 1]: $R_t$ = 1.10 min; MS (ESIpos): m/z = 524 $(M+H)^+$
$^1$H-NMR (400 MHz, DMSO-$d_6$): δ [ppm] = 10.8 (s, 1H), 8.00 (d, 1H), 7.94 (d, 2H), 7.79 (d, 2H), 7.65 (s, 1H), 7.74 (dd, 1H), 7.51 (s, 1H), 6.53 (s, 1H), 5.76 (t, 1H), 4.29 (q, 2H), 3.69 (s, 3H), 3.43-3.25 (m, 2H), 3.21 (s, 3H), 2.45-2.40 (m, 2H), 1.31 (t, 3H).

**Beispiel 28.1A**

(2*S*)-2-Methoxypropyltrifluormethansulfonat

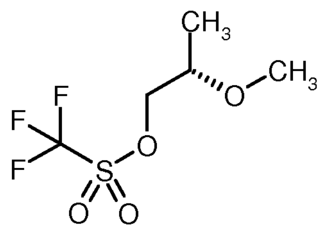

Nach der allgemeinen Methode 8A wurden 645 mg (7.16 mmol) (*S*)-(+)-2-Methoxypropanol mit 1.27 ml (7.52 mmol, 1.05 eq.) Trifluormethansulfonsäureanhydrid in Gegenwart von 917 µl (7.87 mmol, 1.1 eq.) 2,6-Dimethylpyridin umgesetzt. Das Rohprodukt wurde ohne weitere Reinigung in der nächsten Stufe umgesetzt.

**Beispiel 28.1B**

2-[4-(5-Chlor-2-cyanophenyl)-5-methoxy-2-oxopyridin-1(2*H*)-yl]-2,3,5-trideoxy-4-*O*-methyl-L-glycero-pentonsäure-*tert*.-butylester (Gemisch enantiomerenreiner Diastereomere 1 und 2)

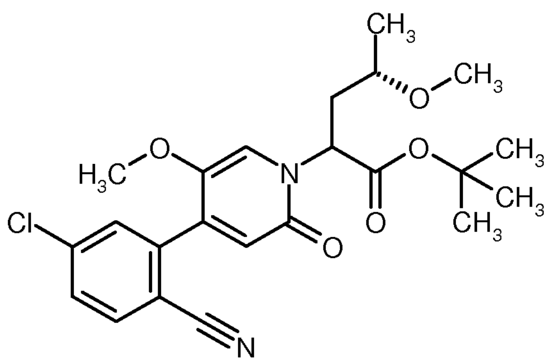

Nach der allgemeinen Methode 7B wurden 450 mg (1.15 mmol) [4-(5-Chlor-2-cyanophenyl)-5-methoxy-2-oxopyridin-1(2*H*)-yl]essigsäure-*tert*.-butylester in Gegenwart von 1.27 ml (1.27 mmol, 1.1 eq.) Bis-(trimethylsilyl)-lithiumamid (1M in THF) mit 384 mg (1.73 mmol, 1.5 eq.) (2S)-2-Methoxypropyltrifluormethansulfonat umgesetzt. Ausbeute: 375 mg (73% d. Th.)
LC/MS [Methode 1]: $R_t$ = 1.09 min; MS (ESIpos): m/z = 447 $(M+H)^+$.

**Beispiel 28.1C**

2-[4-(5-Chlor-2-cyanophenyl)-5-methoxy-2-oxopyridin-1(2*H*)-yl]-2,3,5-trideoxy-4-*O*-methyl-L-glycero-pentonsäure (Gemisch enantiomerenreiner Diastereomere 1 und 2)

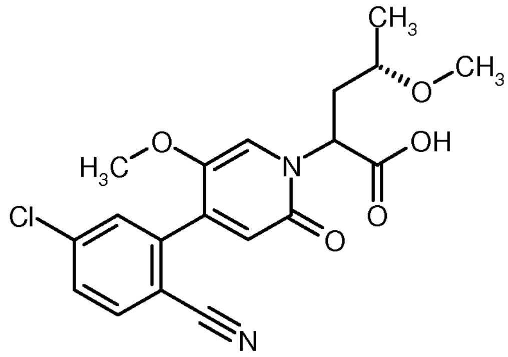

Nach der allgemeinen Methode 6A wurden 375 mg (0.84 mmol) 2-[4-(5-Chlor-2-cyanophenyl)-5-methoxy-2-oxopyridin-1(2*H*)-yl]-2,3,5-trideoxy-4-*O*-methyl-L-glycero-pentonsäure-*tert.*-butylester (Gemisch enantiomerenreiner Diastereomere 1 und 2) mit TFA verseift. Ausbeute: 391 mg (92% Reinheit, quant.)
LC/MS [Methode 2]: Diastereomer 1: $R_t$ = 2.28 min; MS (ESIpos): m/z = 391 $(M+H)^+$; Diastereomer 2: $R_t$ = 2.36 min; MS (ESIpos): m/z = 391 $(M+H)^+$.

**Beispiel 28.1D**

4-({(4*S*)-2-[4-(5-Chlor-2-cyanophenyl)-5-methoxy-2-oxopyridin-1(2*H*)-yl]-4-methoxypentanoyl}-amino)benzoesäure-*tert.*-butylester (Gemisch enantiomerenreiner Diastereomere 1 und 2)

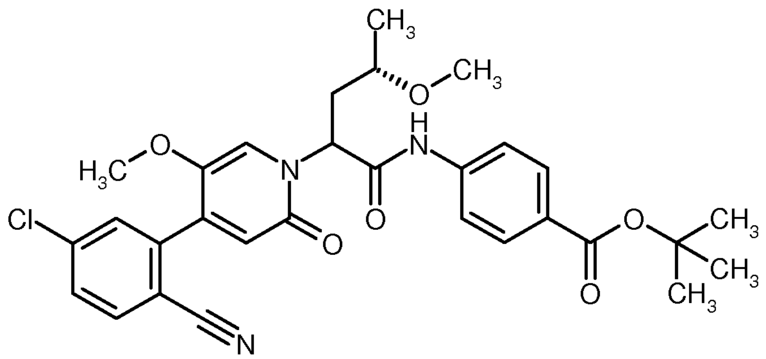

Nach der allgemeinen Methode 5A wurden 391 mg (92% Reinheit, 0.92 mmol) 2-[4-(5-Chlor-2-cyanophenyl)-5-methoxy-2-oxopyridin-1(2*H*)-yl]-2,3,5-trideoxy-4-*O*-methyl-L-glycero-pentonsäure (Gemisch enantiomerenreiner Diastereomere 1 und 2) mit 196 mg (1.01 mmol, 1.1 eq.) 4-Aminobenzoesäure-*tert.*-butylester umgesetzt. Ausbeute: 387 mg (71% d. Th.)
LC/MS [Methode 1]: Diastereomer 1: $R_t$ = 1.23 min; MS (ESIpos): m/z = 566 $(M+H)^+$; Diastereomer 2: $R_t$ = 1.24 min; MS (ESIpos): m/z = 566 $(M+H)^+$.

**Beispiel 29.1A**

(2*R*)-2-Methoxypropan-1-ol

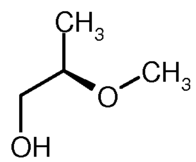

Eine Lösung von 500 mg (4.66 mmol) (*R*)-(+)-2-Methoxypropionsäure in 10 ml Dichlormethan wurde unter Argon bei 0°C tropfenweise mit 858 µl (8.39 mmol, 1.8 eq.) Boran-Dimethylsulfid-Komplex versetzt, das Reaktionsgemisch über Nacht bei RT gerührt und anschließend tropfenweise mit wässriger Natronlauge (2M) versetzt. Nach Phasentrennung wurde die wässrige Phase mit Dichlormethan extrahiert. Die vereinigten, organischen Phasen wurden getrocknet (Natriumsulfat), filtriert, im Vakuum (Wasserbad <20°C, Druck >300 mbar) eingeengt und getrocknet. Ausbeute: 490 mg (quant.)

$^1$H-NMR (400 MHz, DMSO-$d_6$): δ [ppm] = 4.55 (t, 1H), 3.40-3.31 (m, 1H), 3.30-3.20 (m, 2H), 3.24 (s, 3H), 1.02 (d, 3H).

**Beispiel 29.1B**

(2*R*)-2-Methoxypropyltrifluormethansulfonat

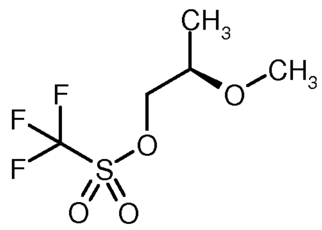

Nach der allgemeinen Methode 8A wurden 490 mg (5.44 mmol) (2*R*)-2-Methoxypropan-1-ol mit 1.01ml (5.98 mmol, 1.1 eq.) Trifluormethansulfonsäureanhydrid in Gegenwart von 834 µl (5.98 mmol, 1.1 eq.) Triethylamin umgesetzt. Das Rohprodukt wurde ohne weitere Reinigung in der nächsten Stufe umgesetzt.

$^1$H-NMR (400 MHz, DMSO-$d_6$): δ [ppm] = 4.39 (dd, 1H), 4.17 (dd, 1H), 3.66-3.58 (m, 1H), 3.33 (s, 3H), 1.09 (d, 3H).

**Beispiel 29.1C**

2-[4-(5-Chlor-2-cyanophenyl)-5-methoxy-2-oxopyridin-1(2*H*)-yl]-2,3,5-trideoxy-4-*O*-methyl-D-glycero-pentonsäure-*tert*.-butylester (Gemisch enantiomerenreiner Diastereomere 1 und 2)

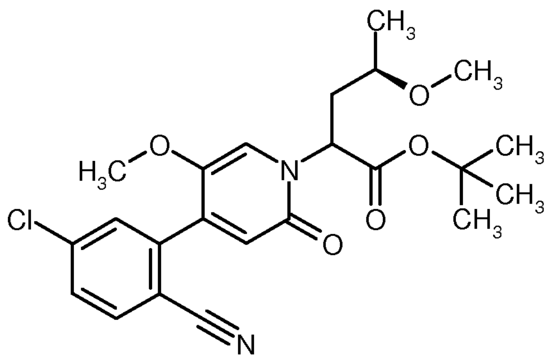

Nach der allgemeinen Methode 7B wurden 500 mg (1.24 mmol) [4-(5-Chlor-2-cyanophenyl)-5-methoxy-2-oxopyridin-1(2*H*)-yl]essigsäure-*tert*.-butylester in Gegenwart von 1.36 ml (1.36 mmol, 1.1 eq.) Bis-(trimethylsilyl)-lithiumamid (1M in THF) mit 861 mg (80% Reinheit, 3.1 mmol, 2.5 eq.) (2*R*)-2-Methoxypropyltrifluormethansulfonat umgesetzt. Ausbeute: 99 mg (19% d. Th.)

**Beispiel 29.1D**

2-[4-(5-Chlor-2-cyanophenyl)-5-methoxy-2-oxopyridin-1(2*H*)-yl]-2,3,5-trideoxy-4-*O*-methyl-D-glycero-pentonsäure (Gemisch enantiomerenreiner Diastereomere 1 und 2)

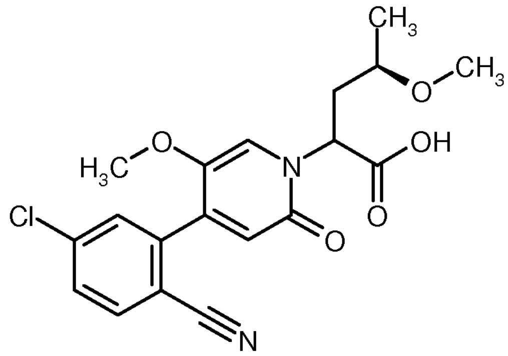

Nach der allgemeinen Methode 6A wurden 99 mg (0.22 mmol) 2-[4-(5-Chlor-2-cyanophenyl)-5-methoxy-2-oxopyridin-1(2*H*)-yl]-2,3,5-trideoxy-4-*O*-methyl-D-glycero-pentonsäure-*tert.*-butylester (Gemisch enantiomerenreiner Diastereomere 1 und 2) mit TFA verseift. Ausbeute: 88 mg (88% Reinheit, 91% d. Th.)
LC/MS [Methode 8]: Diastereomer 1: $R_t$ = 1.05 min; MS (ESIpos): m/z = 391 $(M+H)^+$; Diastereomer 2: $R_t$ = 1.07 min; MS (ESIpos): m/z = 391 $(M+H)^+$.

**Beispiel 29.1E**

4-({(4*R*)-2-[4-(5-Chlor-2-cyanophenyl)-5-methoxy-2-oxopyridin-1(2*H*)-yl]-4-methoxypentanoyl}-amino)benzoesäure-*tert.*-butylester (Gemisch enantiomerenreiner Diastereomere 1 und 2)

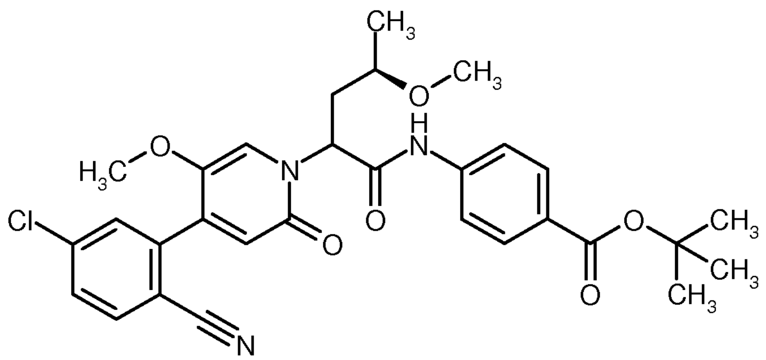

Nach der allgemeinen Methode 5A wurden 88 mg (88% Reinheit, 0.20 mmol) 2-[4-(5-Chlor-2-cyanophenyl)-5-methoxy-2-oxopyridin-1(2*H*)-yl]-2,3,5-trideoxy-4-*O*-methyl-D-glycero-pentonsäure (Gemisch enantiomerenreiner Diastereomere 1 und 2) mit 42 mg (0.22 mmol, 1.1 eq.) 4-Aminobenzoesäure-*tert.*-butylester umgesetzt. Ausbeute: 51 mg (46% d. Th.) Gemisch enantiomerenreiner Diastereomere 1 und 2 und 26 mg (23% d. Th.) Diastereomer 1.
LC/MS [Methode 8]: Diastereomer 1: $R_t$ = 1.51 min; MS (ESIneg): m/z = 564 $(M-H)^-$; Diastereomer 2: $R_t$ = 1.52 min; MS (ESIneg): m/z = 564 $(M-H)^-$.

**Beispiel 30.1A**

(2*R*)-Tetrahydrofuran-2-ylmethyltrifluormethansulfonat

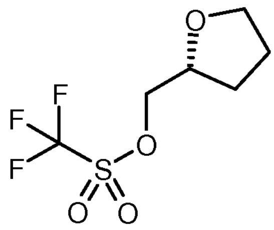

Nach der allgemeinen Methode 8A wurden 300 mg (2.9 mmol) (2*R*)-Tetrahydrofuran-2-ylmethanol mit 512 μl (3.0 mmol, 1.05 eq.) Trifluormethansulfonsäureanhydrid in Gegenwart von 369 μl (3.2 mmol, 1.1 eq.) 2,6-Dimethylpyridin umgesetzt. Das Rohprodukt wurde ohne weitere Reinigung in der nächsten Stufe umgesetzt.
$^1$H-NMR (400 MHz, DMSO-$d_6$): δ [ppm] = 4.35 (dd, 1H), 4.17 (dd, 1H), 4.09 (dq, 1H), 3.86-3.70 (m, 2H), 2.00-1.79 (m, 3H), 1.61-1.47 (m, 1H).

**Beispiel 30.1B**

2-[4-(5-Chlor-2-cyanophenyl)-5-methoxy-2-oxopyridin-1(2*H*)-yl]-3-[(2*R*)-tetrahydrofuran-2-yl]propansäure-*tert*.-butylester (Gemisch enantiomerenreiner Diastereomere 1 und 2)

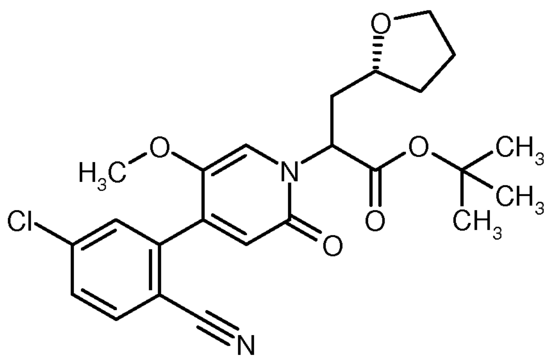

Nach der allgemeinen Methode 7B wurden 450 mg (94% Reinheit, 1.1 mmol) [4-(5-Chlor-2-cyanophenyl)-5-methoxy-2-oxopyridin-1(2*H*)-yl]essigsäure-*tert*.-butylester in Gegenwart von 1.35 ml (1.35 mmol, 1.2 eq.) Bis-(trimethylsilyl)-lithiumamid (1M in THF) mit 396 mg (1.7 mmol, 1.5 eq.) (2*R*)-Tetrahydrofuran-2-ylmethyltrifluormethansulfonat umgesetzt. Ausbeute: 625 mg (76% Reinheit, 92% d. Th.)
LC/MS [Methode 1]: $R_t$ = 1.09 min; MS (ESIpos): m/z = 459 $(M+H)^+$.

**Beispiel 30.1C**

2-[4-(5-Chlor-2-cyanophenyl)-5-methoxy-2-oxopyridin-1(2*H*)-yl]-3-[(2*R*)-tetrahydrofuran-2-yl]propansäure (Gemisch enantiomerenreiner Diastereomere 1 und 2)

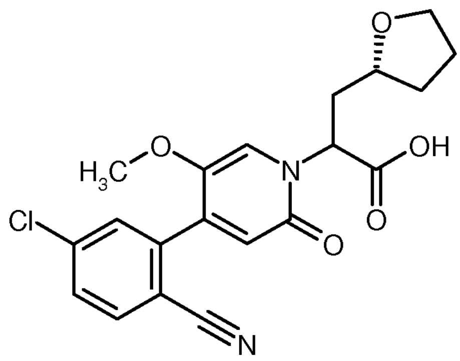

Nach der allgemeinen Methode 6A wurden 625 mg (76% Reinheit, 1.0 mmol) 2-[4-(5-Chlor-2-cyanophenyl)-5-methoxy-2-oxopyridin-1(2*H*)-yl]-3-[(2*R*)-tetrahydrofuran-2-yl]propansäure-*tert*.-butylester (Gemisch enantiomerenreiner Diastereomere 1 und 2) mit TFA verseift. Ausbeute: 585 mg (73% Reinheit, quant.)
LC/MS [Methode 1]: Diastereomer 1: $R_t$ = 2.33 min; MS (ESIpos): m/z = 403 $(M+H)^+$; Diastereomer 2: $R_t$ = 2.38 min; MS (ESIpos): m/z = 403 $(M+H)^+$.

**Beispiel 30.1D**

4-({2-[4-(5-Chlor-2-cyanophenyl)-5-methoxy-2-oxopyridin-1(2*H*)-yl]-3-[(2*R*)-tetrahydrofuran-2-yl]propanoyl}amino)benzoesäure-*tert*.-butylester (Gemisch enantiomerenreiner Diastereomere 1 und 2)

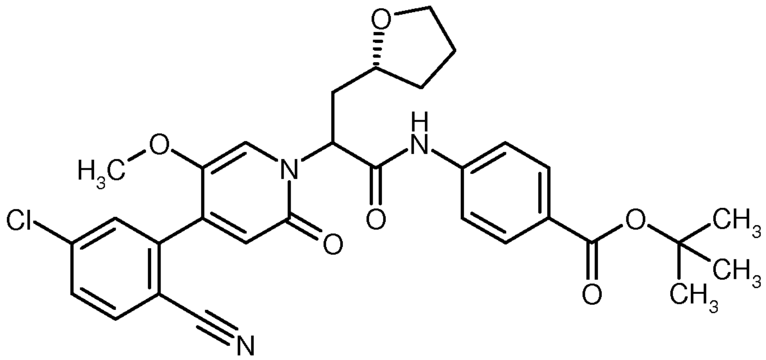

Nach der allgemeinen Methode 5A wurden 585 mg (73% Reinheit, 1.1 mmol) 2-[4-(5-Chlor-2-cyanophenyl)-5-methoxy-2-oxopyridin-1(2*H*)-yl]-3-[(2*R*)-tetrahydrofuran-2-yl]propansäure (Gemisch enantiomerenreiner Diastereomere 1 und 2) mit 225 mg (1.2 mmol, 1.1 eq.) 4-Aminobenzoesäure-*tert*.-butylester umgesetzt. Ausbeute: 327 mg (53% d. Th.)
LC/MS [Methode 1]: $R_t$ = 1.27 min; MS (ESIpos): m/z = 578 $(M+H)^+$.

**Beispiel 31.1A**

(2*S*)-Tetrahydrofuran-2-ylmethanol

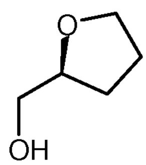

Eine Lösung von 2.13 g (18.0 mmol) (2S)-Tetrahydrofuran-2-carbonsäure in 35 ml Dichlormethan wurde unter

Argon bei 0°C tropfenweise mit 3.3 ml (32.4 mmol, 1.8 eq.) Boran-Dimethylsulfid-Komplex versetzt, das Reaktionsgemisch über Nacht bei RT gerührt und anschließend tropfenweise mit wässriger Natronlauge (2M) versetzt. Nach Phasentrennung wurde die wässrige Phase mit Dichlormethan extrahiert. Die vereinigten, organischen Phasen wurden getrocknet (Natriumsulfat), filtriert, im Vakuum eingeengt und getrocknet. Ausbeute: 2.19 g (angenommene Reinheit von 80%, quant.)

**Beispiel 31.1B**

(2*S*)-Tetrahydrofuran-2-ylmethyltrifluormethansulfonat

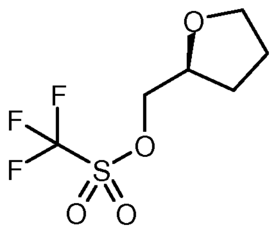

Nach der allgemeinen Methode 8A wurden 2.19 g (angenommene Reinheit von 80%, 17.2 mmol) (2*S*)-Tetrahydrofuran-2-ylmethanol mit 3.1 ml (18.0 mmol, 1.05 eq.) Trifluormethansulfonsäureanhydrid in Gegenwart von 2.2 ml (18.9 mmol, 1.1 eq.) 2,6-Dimethylpyridin umgesetzt. Das Rohprodukt wurde ohne weitere Reinigung in der nächsten Stufe umgesetzt.
$^1$H-NMR (400 MHz, DMSO-$d_6$): $\delta$ [ppm] = 4.35 (dd, 1H), 4.17 (dd, 1H), 4.09 (dq, 1H), 3.86-3.70 (m, 2H), 2.00-1.79 (m, 3H), 1.60-1.47 (m, 1H).

**Beispiel 31.1C**

2-[4-(5-Chlor-2-cyanophenyl)-5-methoxy-2-oxopyridin-1(2*H*)-yl]-3-[(2*S*)-tetrahydrofuran-2-yl]propansäure-*tert*.-butylester (Gemisch enantiomerenreiner Diastereomere 1 und 2)

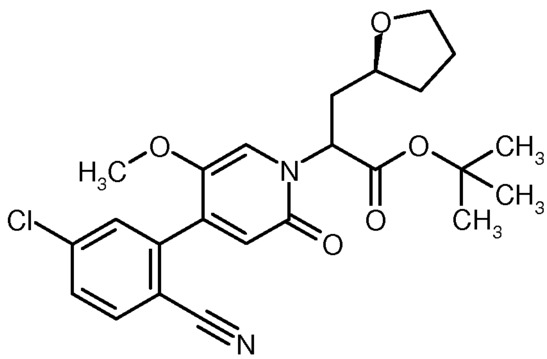

Nach der allgemeinen Methode 7B wurden 3.0 g (93% Reinheit, 7.4 mmol) [4-(5-Chlor-2-cyanophenyl)-5-methoxy-2-oxopyridin-1(2*H*)-yl]essigsäure-*tert*.-butylester in Gegenwart von 8.9 ml (8.9 mmol, 1.2 eq.) Bis-(trimethylsilyl)-lithiumamid (1M in THF) mit 3.5 g (angenommene Reinheit von 80%, 11.9 mmol, 1.6 eq.) (2*S*)-Tetrahydrofuran-2-ylmethyltrifluormethansulfonat umgesetzt. Ausbeute: 1.7 g (49% d. Th.)
LC/MS [Methode 8]: $R_t$ = 1.09 min; MS (ESIpos): m/z = 403 (M-*tert*.-Butyl+H)$^+$.

**Beispiel 31.1D**

2-[4-(5-Chlor-2-cyanophenyl)-5-methoxy-2-oxopyridin-1(2*H*)-yl]-3-[(2*S*)-tetrahydrofuran-2-yl]propansäure (Gemisch enantiomerenreiner Diastereomere 1 und 2)

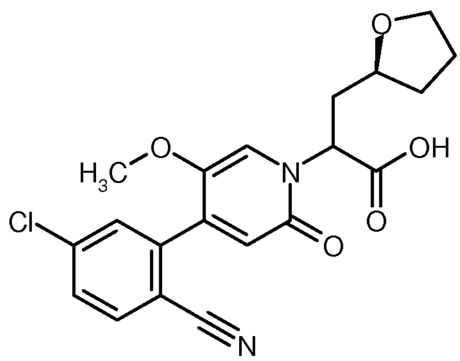

Nach der allgemeinen Methode 6A wurden 1.57 g (3.36 mmol) 2-[4-(5-Chlor-2-cyanophenyl)-5-methoxy-2-oxopyridin-1(2*H*)-yl]-3-[(2*S*)-tetrahydrofuran-2-yllpropansäure-*tert*.-butylester (Gemisch enantiomerenreiner Diastereomere 1 und 2) mit TFA verseift. Ausbeute: 1.41 g (92% Reinheit, 96% d. Th.)
LC/MS [Methode 8]: Diastereomer 1: $R_t$ = 1.09 min; MS (ESIpos): m/z = 403 $(M+H)^+$; Diastereomer 2: $R_t$ = 1.11 min; MS (ESIpos): m/z = 403 $(M+H)^+$.

**Beispiel 31.1E**

4-({2-[4-(5-Chlor-2-cyanophenyl)-5-methoxy-2-oxopyridin-1(2*H*)-yl]-3-[(2*S*)-tetrahydrofuran-2-yl]propanoyl}amino)benzoesäure-*tert*.-butylester (Gemisch enantiomerenreiner Diastereomere 1 und 2)

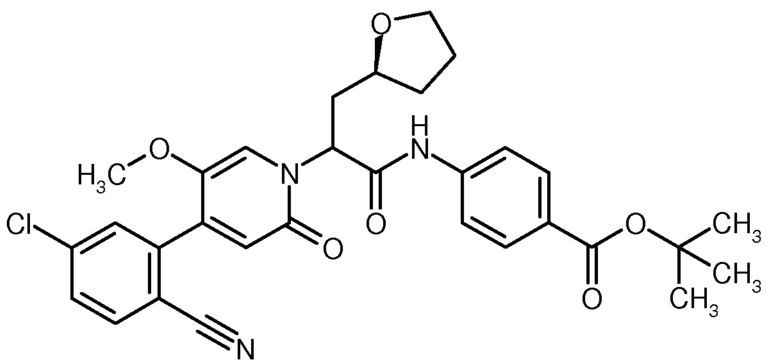

Nach der allgemeinen Methode 5A wurden 1.54 g (92% Reinheit, 3.52 mmol) 2-[4-(5-Chlor-2-cyanophenyl)-5-methoxy-2-oxopyridin-1(2*H*)-yl]-3-[(2*S*)-tetrahydrofuran-2-yl]propansäure (Gemisch enantiomerenreiner Diastereomere 1 und 2) mit 747 mg (3.87 mmol, 1.1 eq.) 4-Aminobenzoesäure-*tert*.-butylester umgesetzt. Ausbeute: 1.61 g (79% d. Th.)
LC/MS [Methode 1]: $R_t$ = 1.23 min; MS (ESIpos): m/z = 578 $(M+H)^+$.

**Beispiel 32.1A**

2-[4-(5-Chlor-2-cyanophenyl)-5-methoxy-2-oxopyridin-1(2*H*)-yl]-3-hydroxy-3-(tetrahydrofuran-3-yl)propansäure-*tert*.-butylester (Diastereomerengemisch)

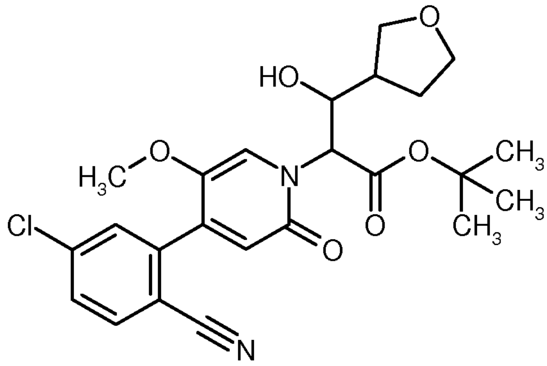

Eine Lösung von 2.00 g (5.34 mmol) [4-(5-Chlor-2-cyanophenyl)-5-methoxy-2-oxopyridin-1(2*H*)-yl]essigsäure-*tert.*-butylester in 50 ml Tetrahydrofuran wurde bei -70°C tropfenweise mit 6.94 ml (6.94 mmol, 1.3 eq.) Bis-(trimethylsilyl)-lithiumamid (1M in THF) versetzt, 10 min bei -70°C gerührt, mit einer Lösung von 801 mg (8.00 mmol, 1.5 eq.) Tetrahydrofuran-3-carbaldehyd in 4 ml Tetrahydrofuran versetzt und 90 min bei -70°C gerührt. Das Reaktionsgemisch wurde auf -20°C erwärmt und mit 25 ml halbgesättigter, wässriger Ammoniumchlorid-Lösung versetzt. Nach Phasentrennung wurde die wässrige Phase mit Diethylether extrahiert. Die vereinigten, organischen Phasen wurden mit gesättigter, wässriger Natriumchlorid-Lösung gewaschen, getrocknet (Natriumsulfat), filtriert und im Vakuum eingeengt und getrocknet. Das Rohprodukt wurde mittels Flash-Chromatographie (KP-SIL, Petrolether/Essigsäureethylester 33-75%) aufgereinigt. Ausbeute: 1.49 g (56% d. Th.)
LC/MS [Methode 1]: $R_t$ = 0.99 min; MS (ESIpos): m/z = 475 $(M+H)^+$.

## Beispiel 32.1B

2-[4-(5-Chlor-2-cyanophenyl)-5-methoxy-2-oxopyridin-1(2*H*)-yl]-3-(tetrahydrofuran-3-yl)acrylsäure-tert. -butylester (Diastereomerengemisch)

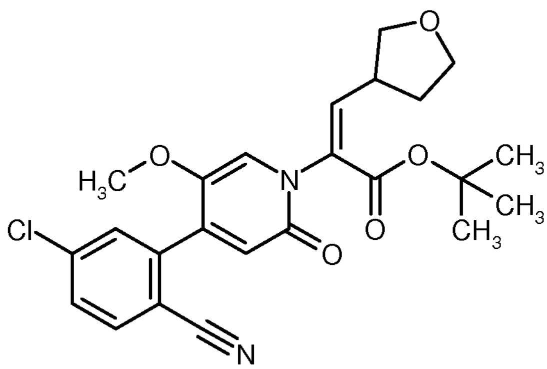

Eine Lösung von 1.55 mg (3.13 mmol) 2-[4-(5-Chlor-2-cyanophenyl)-5-methoxy-2-oxopyridin-1(2*H*)-yl]-3-hydroxy-3-(tetrahydrofuran-3-yl)propansäure-*tert.*-butylester (Diastereomerengemisch) in 48 ml Dichlormethan wurde bei RT tropfenweise mit 0.5 ml (3.8 mmol, 1.2 eq.) Diethylaminoschwefeltrifluorid versetzt, 90 min bei RT gerührt und anschließend mit 50 ml Dichlormethan und 50 ml gesättigter, wässriger Natriumhydrogencarbonat-Lösung versetzt. Nach Phasentrennung wurde die wässrige Phase mit Dichlormethan extrahiert. Die vereinigten, organischen Phasen wurden mit gesättigter, wässriger Natriumchlorid-Lösung gewaschen, getrocknet (Natriumsulfat), filtriert und im Vakuum eingeengt und getrocknet. Ausbeute: 1.38 g (93% d. Th.)
LC/MS [Methode 1]: $R_t$ = 1.03 min/1.05 min; MS (ESIpos): m/z = 457 $(M+H)^+$/457 $(M+H)^+$.

**Beispiel 32.1C**

2-[4-(5-Chlor-2-cyanophenyl)-5-methoxy-2-oxopyridin-1(2*H*)-yl]-3-(tetrahydrofuran-3-yl)propansäure-*tert*.-butylester (Gemisch racemischer Diastereomere)

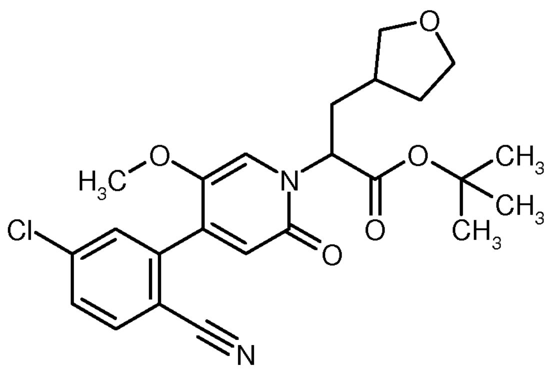

1.38 g (2.90 mmol) 2-[4-(5-Chlor-2-cyanophenyl)-5-methoxy-2-oxopyridin-1(2*H*)-yl]-3-(tetrahydrofuran-3-yl)acrylsäure-*tert*.-butylester (Diastereomerengemisch) wurden bei RT mit 100 ml einer "Hot Stryker's"-Reagenzlösung [B. A. Baker et al. Org. Lett. 2008, 10, 289-292] versetzt. Das Reaktionsgemisch wurde 6 h bei RT gerührt und anschließend im Vakuum eingeengt. Das Rohprodukt wurde viermal mit jeweils 50 ml Acetonitril verrührt und dekantiert. Die vereinigten, organischen Phasen wurden im Vakuum eingeengt. Der Rückstand wurde mittels Flash-Chromatographie (PF-50SIHC, Petrolether/Essigsäureethylester 40-66%) aufgereinigt. Ausbeute: 930 mg (70% d. Th.)
LC/MS [Methode 1]: $R_t$ = 1.06 min; MS (ESIpos): m/z = 459 $(M+H)^+$.

**Beispiel 32.1D**

2-[4-(5-Chlor-2-cyanophenyl)-5-methoxy-2-oxopyridin-1(2*H*)-yl]-3-(tetrahydrofuran-3-yl)propansäure (Gemisch racemischer Diastereomere)

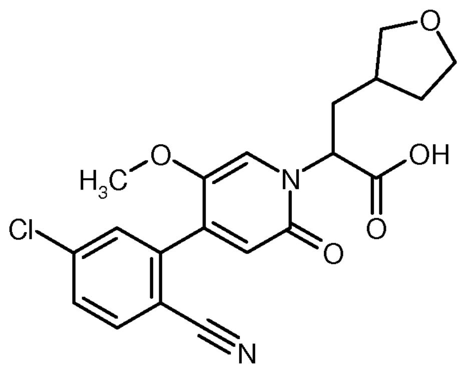

Nach der allgemeinen Methode 6A wurden 930 mg (2.0 mmol) 2-[4-(5-Chlor-2-cyanophenyl)-5-methoxy-2-oxopyridin-1(2*H*)-yl]-3-(tetrahydrofuran-3-yl)propansäure-*tert*.-butylester (Gemisch racemischer Diastereomere) mit TFA verseift. Ausbeute: 974 mg (94% Reinheit, quant.)
LC/MS [Methode 1]: $R_t$ = 0.77 min; MS (ESIpos): m/z = 403 $(M+H)^+$.

**Beispiel 32.1E**

4-([2-[4-(5-Chlor-2-cyanophenyl)-5-methoxy-2-oxopyridin-1(2*H*)-yl]-3-(tetrahydrofuran-3-yl)-propanoyl}amino)benzoesäure-*tert*.-butylester (Gemisch racemischer Diastereomere)

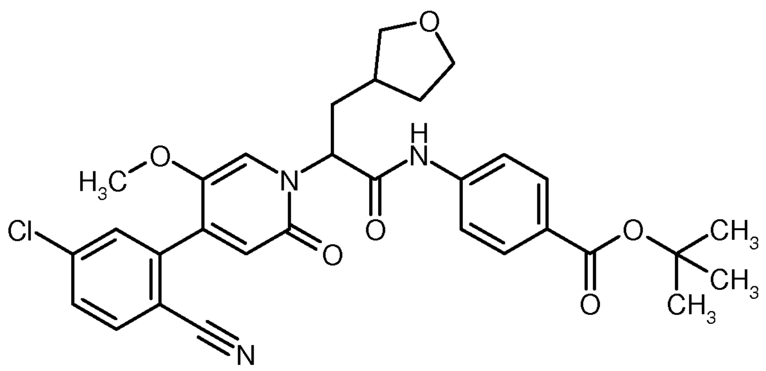

Nach der allgemeinen Methode 5A wurden 900 mg (94% Reinheit, 2.1 mmol) 2-[4-(5-Chlor-2-cyanophenyl)-5-methoxy-2-oxopyridin-1(2*H*)-yl]- 3-(tetrahydrofuran-3-yl)propansäure (Gemisch racemischer Diastereomere) mit 446 mg (2.3 mmol, 1.1 eq.) 4-Aminobenzoesäure-*tert*.-butylester umgesetzt. Ausbeute: 682 mg (97% Reinheit, 54% d. Th.) und 113 mg (92% Reinheit, 9% d. Th.)
LC/MS [Methode 1]: $R_t$ = 1.20 min; MS (ESIpos): m/z = 578 $(M+H)^+$.

**Beispiel 33.1A**

Tetrahydro-2*H*-pyran-4-ylmethyl-trifluormethansulfonat

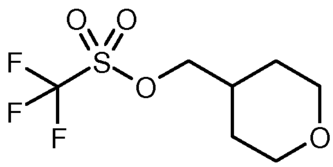

Gemäß allgemeiner Methode 8A wurden 5.00 g (43.0 mmol) Tetrahydro-2*H*-pyran-4-ylmethanol in 75 ml Dichlormethan mit 5.52 ml (47.3 mmol) Lutidin und 7.65 ml (45.2 mmol) Trifluormethansulfonsäureanhydrid umgesetzt. Das Rohprodukt wurde ohne weitere Aufreinigung im nächsten Schritt eingesetzt. Ausbeute: 12.4 g (quant.)
GC/MS [Methode 9]: $R_t$ = 3.15 min; MS: m/z = 248 $(M)^+$
$^1$H-NMR (400 MHz, $CDCl_3$): $\delta$ [ppm] = 4.37 (d, 2H), 4.03 (dd, 2H), 3.41 (dt, 2H), 2.16-2.02 (m, 1H), 1.72-1.65 (m, 2H), 1.48-1.37 (m, 2H).

**Beispiel 33.1B**

2-[4-(5-Chlor-2-cyanophenyl)-5-methoxy-2-oxopyridin-1(2*H*)-yl]-3-(tetrahydro-2*H*-pyran-4-yl)propansäure-*tert*.-butylester (Racemat)

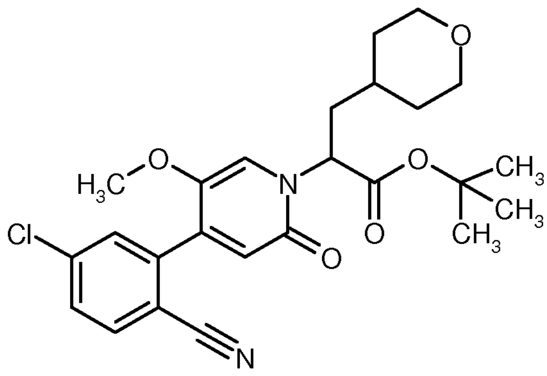

Gemäß allgemeiner Methode 7B wurden 1.65 g (4.41 mmol) [4-(5-Chlor-2-cyanophenyl)-5-methoxy-2-oxopyridin-1(2*H*)-yl]essigsäure-*tert*.-butylester, 1.64 g (6.61 mmol) Tetrahydro-2*H*-pyran-4-ylmethyl-trifluormethansulfonat und 5.73 ml (5.73 mmol) Bis-(trimethylsilyl)-lithiumamid (1M in THF) in 37 ml THF zur Reaktion gebracht. Nach säulenchromatographischer Reinigung (80 g Silica Kartusche, Fluss: 60 ml/min, Cyclohexan/Essigsäureethylester-Gradient) wurde die Titelverbindung erhalten. Ausbeute: 1.57 g (73% d. Th.)
LC/MS [Methode 1]: $R_t$ = 1.10 min; MS (ESIpos): m/z = 473 $(M+H)^+$
$^1$H-NMR (400 MHz, DMSO-$d_6$): $\delta$ [ppm] = 7.99 (d, 1H), 7.77-7.71 (m, 2H), 7.40 (s, 1H), 6.51 (s, 1H), 5.32-5.26 (m, 1H), 3.85-3.76 (m, 2H), 3.64 (s, 3H), 3.23-3.13 (m, 2H), 2.22-2.12 (m, 1H), 2.02-1.93 (m, 1H), 1.73-1.66 (m, 1H), 1.51-1.45 (m, 1H), 1.40 (s, 9H), 1.36-1.13 (m, 3H).

**Beispiel 33.1C**

2-[4-(5-Chlor-2-cyanophenyl)-5-methoxy-2-oxopyridin-1(2*H*)-yl]-3-(tetrahydro-2*H*-pyran-4-yl)propansäure (Racemat)

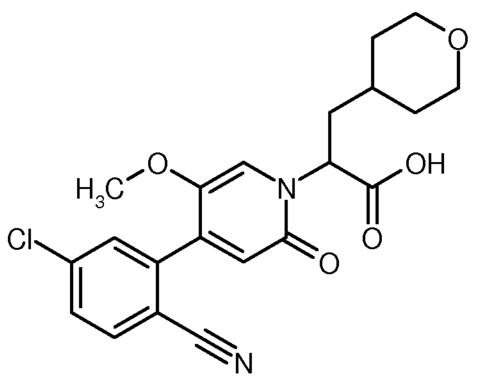

Gemäß allgemeiner Methode 6A wurden 1.57 g (3.32 mmol) 2-[4-(5-Chlor-2-cyanophenyl)-5-methoxy-2-oxopyridin-1(2*H*)-yl]-3-(tetrahydro-2*H*-pyran-4-yl)propansäure-*tert*.-butylester (Racemat) in 25 ml Dichlormethan mit 5.12 ml (66.4 mmol) TFA umgesetzt. Ausbeute: 1.60 g (quant.)
LC/MS [Methode 1]: $R_t$ = 0.80 min; MS (ESIpos): m/z = 417 $(M+H)^+$
$^1$H-NMR (400 MHz, DMSO-$d_6$): $\delta$ [ppm] = 13.1 (s, 1H), 7.99 (d, 1H), 7.77-7.71 (m, 2H), 7.45 (s, 1H), 6.50 (s, 1H), 5.38-5.30 (m, 1H), 3.85-3.74 (m, 2H), 3.63 (s, 3H), 3.22-3.12 (m, 2H), 2.26-2.16 (m, 1H), 2.05-1.96 (m, 1H), 1.73-1.65 (m, 1H), 1.48-1.40 (m, 1H), 1.36-1.11 (3H).

**Beispiel 33.1D**

4-({2-[4-(5-Chlor-2-cyanophenyl)-5-methoxy-2-oxopyridin-1(2*H*)-yl]-3-(tetrahydro-2*H*-pyran-4-yl)propanoyl}amino)benzoesäure (Racemat)

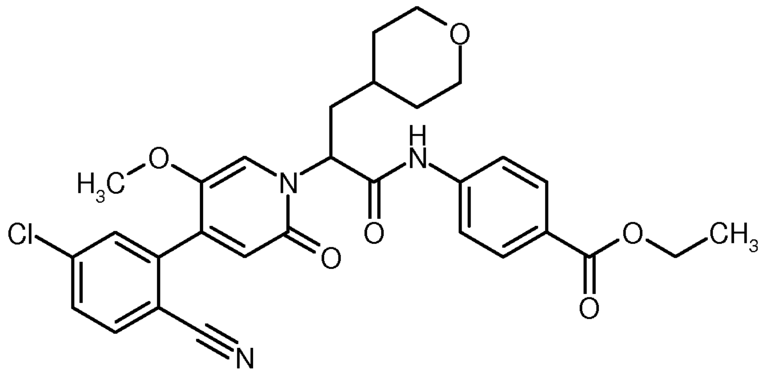

Gemäß allgemeiner Methode 5B wurden 1.38 g (3.31 mmol) 2-[4-(5-Chlor-2-cyanophenyl)-5-methoxy-2-oxopyridin-1(2*H*)-yl]-3-(tetrahydro-2*H*-pyran-4-yl)propansäure (Racemat), 547 mg (3.31 mmol) 4-Aminobenzoesäureethylester, 471 mg (3.31 mmol) Oxima und 516 µl (3.31 mmol) DIC in 33 ml Dimethylformamid zur Reaktion gebracht. Das Rohprodukt wurde mittels Flash-Chromatographie (120 g Kartusche, 85 ml/min, Cyclohexan/Essigsäureethylester-Gradient) gereinigt. Ausbeute: 1.10 g (58% d. Th.)
LC/MS [Methode 1]: $R_t$ = 1.14 min; MS (ESIpos): m/z = 564 $(M+H)^+$
$^1$H-NMR (400 MHz, DMSO-$d_6$): δ [ppm] = 10.9 (s, 1H), 8.00 (d, 1H), 7.94 (d, 2H), 7.81-7.72 (m, 4H), 7.52 (s, 1H), 6.54 (s, 1H), 5.92-5.85 (m, 1H), 4.33-4.25 (q, 2H), 3.87-3.77 (m, 2H), 3.69 (s, 3H), 3.25-3.11 (m, 2H), 2.31-2.21 (m, 1H), 2.03-1.94 (m, 1H), 1.65-1.57 (m, 2H), 1.39-1.19 (m, 3H), 1.32 (t, 3H).

**Beispiel 34.1A**

Tetrahydro-2*H*-pyran-3-ylmethyltrifluormethansulfonat (Racemat)

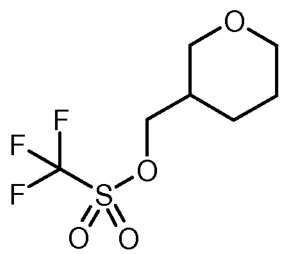

Nach der allgemeinen Methode 8A wurden 232 mg (2.00 mmol) Tetrahydro-2*H*-pyran-3-ylmethanol mit 355 µl (2.10 mmol, 1.05 eq.) Trifluormethansulfonsäureanhydrid in Gegenwart von 256 µl (2.20 mmol, 1.1 eq.) 2,6-Dimethylpyridin umgesetzt. Das Rohprodukt wurde ohne weitere Reinigung in der nächsten Stufe umgesetzt.
$^1$H-NMR (400 MHz, DMSO-$d_6$): δ [ppm] = 4.21-4.12 (m, 2H), 3.77 (dd, 1H), 3.74-3.66 (m, 1H), 3.40-3.30 (m, 1H), 3.20 (dd, 1H), 2.00-1.87 (m, 1H), 1.79-1.69 (m, 1H), 1.64-1.53 (m, 1H), 1.53-1.41 (m, 1H), 1.36-1.24 (m, 1H).

**Beispiel 34.1B**

2-[4-(5-Chlor-2-cyanophenyl)-5-methoxy-2-oxopyridin-1(2*H*)-yl]-3-(tetrahydro-2*H*-pyran-3-yl)propansäure-*tert*.-butylester (Gemisch racemischer Diastereomere)

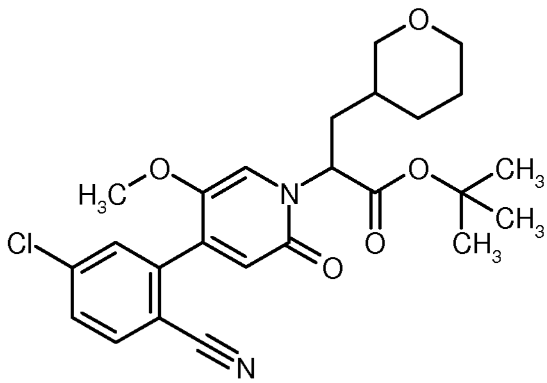

Nach der allgemeinen Methode 7B wurden 450 mg (94% Reinheit, 1.13 mmol) [4-(5-Chlor-2-cyanophenyl)-5-methoxy-2-oxopyridin-1(2*H*)-yl]essigsäure-*tert*.-butylester in Gegenwart von 1.24 ml (1.24 mmol, 1.1 eq.) Bis-(trimethylsilyl)-lithiumamid (1M in THF) mit 467 mg (1.69 mmol, 1.5 eq.) Tetrahydro-2*H*-pyran-3-ylmethyltrifluormethansulfonat (Racemat) umgesetzt. Ausbeute: 451 mg (82% Reinheit, 69% d. Th.)
LC/MS [Methode 1]: $R_t$ = 1.13 min; MS (ESIpos): m/z = 473 $(M+H)^+$.

**Beispiel 34.1C**

2-[4-(5-Chlor-2-cyanophenyl)-5-methoxy-2-oxopyridin-1(2*H*)-yl]-3-(tetrahydro-2*H*-pyran-3-yl)propansäure (Gemisch racemischer Diastereomere)

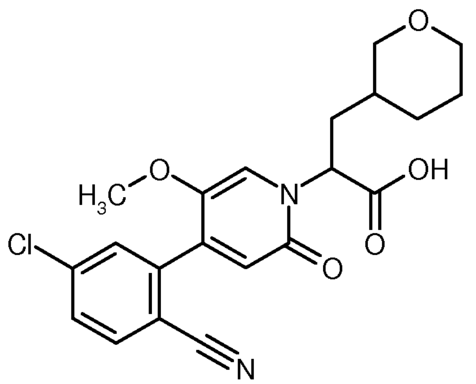

Nach der allgemeinen Methode 6A wurden 451 mg (82% Reinheit, 0.78 mmol) 2-[4-(5-Chlor-2-cyanophenyl)-5-methoxy-2-oxopyridin-1(2*H*)-yl]-3-(tetrahydro-2*H*-pyran-3-yl)propansäure-*tert*.-butylester (Gemisch racemischer Diastereomere) mit TFA verseift. Ausbeute: 440 mg (82% Reinheit, quant.)
LC/MS [Methode 1]: racemisches Diastereomer 1: $R_t$ = 0.84 min; MS (ESIpos): m/z = 417 $(M+H)^+$; racemisches Diastereomer 2: $R_t$ = 0.86 min; MS (ESIpos): m/z = 417 $(M+H)^+$.

**Beispiel 34.1D**

4-({2-[4-(5-Chlor-2-cyanophenyl)-5-methoxy-2-oxopyridin-1(2*H*)-yl]-3-(tetrahydro-2*H*-pyran-3-yl)propanoyl}amino)benzoesäure-*tert*.-butylester (Gemisch racemischer Diastereomere)

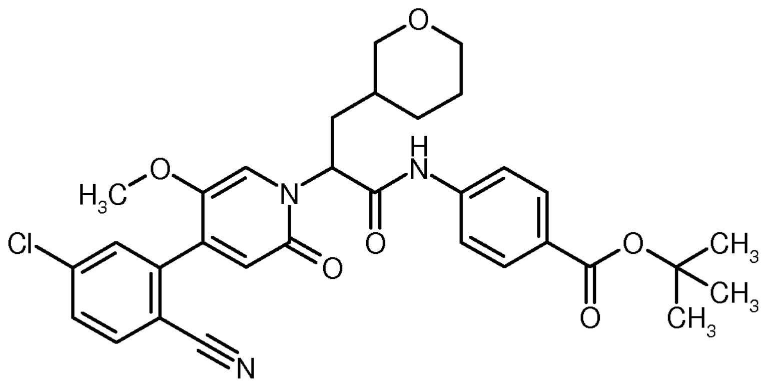

Nach der allgemeinen Methode 5A wurden 440 mg (82% Reinheit, 0.87 mmol) 2-[4-(5-Chlor-2-cyanophenyl)-5-methoxy-2-oxopyridin-1(2*H*)-yl]-3-(tetrahydro-2*H*-pyran-3-yl)propansäure (Gemisch racemischer Diastereomere) mit 184 mg (0.95 mmol, 1.1 eq.) 4-Aminobenzoesäure-*tert*.-butylester umgesetzt. Ausbeute: 742 mg (85% Reinheit, quant.) LC/MS [Methode 1]: Diastereomer 1: $R_t$ = 1.28 min; MS (ESIpos): m/z = 592 $(M+H)^+$; Diastereomer 2: $R_t$ = 1.29 min; MS (ESIpos): m/z = 592 $(M+H)^+$.

**Beispiel 35.1A**

Tetrahydro-2*H*-pyran-2-ylmethyl-trifluormethansulfonat (Racemat)

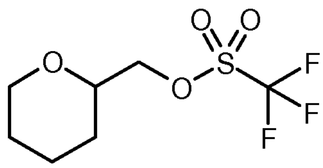

Gemäß allgemeiner Methode 8A wurden 5.85 g (50.4 mmol) Tetrahydro-2*H*-pyran-2-ylmethanol in 88 ml Dichlormethan mit 6.45 ml (55.4 mmol) Lutidin und 8.95 ml (52.9 mmol) Trifluormethansulfonsäureanhydrid umgesetzt. Das Rohprodukt wurde ohne weitere Aufreinigung im nächsten Schritt eingesetzt. Ausbeute: 14.8 g (quant.)
$^1$H-NMR (400 MHz, DMSO-$d_6$): δ [ppm] = 4.32 (dd, 1H), 4.18 (dd, 1H), 3.96-3.93 (m, 1H), 3.59-3.52 (m, 1H), 3.47-3.40 (m, 1H), 1.84-1.74 (m, 1H), 1.55-1.39 (m, 4H), 1.27-1.15 (m, 1H).

**Beispiel 35.1B**

2-[4-(5-Chlor-2-cyanophenyl)-5-methoxy-2-oxopyridin-1(2*H*)-yl]-3-(tetrahydro-2*H*-pyran-2-yl)propansäure-*tert*.-butylester (Gemisch racemischer Diastereomere)

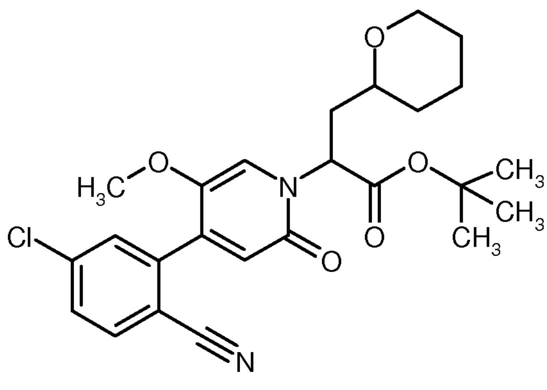

Gemäß allgemeiner Methode 7B wurden 4.20 g (11.2 mmol) [4-(5-Chlor-2-cyanophenyl)-5-methoxy-2-oxopyridin-1(2*H*)-yl]essigsäure-*tert*.-butylester, 4.17 g (16.8 mmol) Tetrahydro-2*H*-pyran-2-ylmethyl-trifluormethansulfonat (Racemat) und 11.8 ml (11.8 mmol) Bis-(trimethylsilyl)-lithiumamid (1M in THF) in 125 ml THF zur Reaktion gebracht. Nach säulenchromatographischer Reinigung (100 g Silica Kartusche, Fluss: 50 ml/min, Cyclohexan/Essigsäureethylester-Gradient) wurde die Titelverbindung erhalten. Ausbeute: 2.6 g (49% d. Th.)
LC/MS [Methode 1]: $R_t$ = 1.21 min; MS (ESIpos): m/z = 473 $(M+H)^+$.

**Beispiel 35.1C**

2-[4-(5-Chlor-2-cyanophenyl)-5-methoxy-2-oxopyridin-1(2*H*)-yl]-3-[tetrahydro-2*H*-pyran-2-yl]propansäure (Gemisch racemischer Diastereomere)

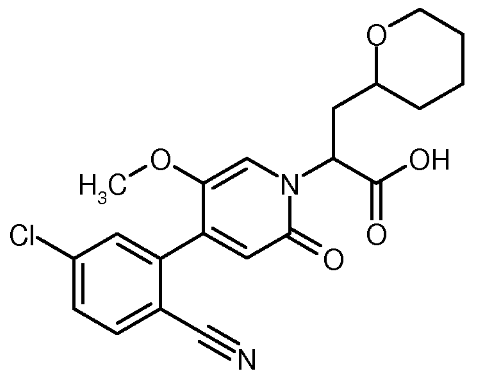

Gemäß allgemeiner Methode 6A wurden 2.50 g (5.29 mmol) 2-[4-(5-Chlor-2-cyanophenyl)-5-methoxy-2-oxopyridin-1(2*H*)-yl]-3-(tetrahydro-2*H*-pyran-2-yl)propansäure-*tert*.-butylester (Gemisch racemischer Diastereomere) in 60 ml Dichlormethan mit 15.3 ml (198 mmol) TFA umgesetzt. Ausbeute: 2.20 g (71% d. Th.)
LC/MS [Methode 1]: $R_t$ = 0.93-0.94 min; MS (ESIpos): m/z = 417 $(M+H)^+$.

**Beispiel 35.1D**

({2-[4-(5-Chlor-2-cyanophenyl)-5-methoxy-2-oxopyridin-1(2*H*)-yl]-3-[tetrahydro-2*H*-pyran-2-yl]propanoyl}amino)benzoesäuremethylester (Gemisch racemischer Diastereomere)

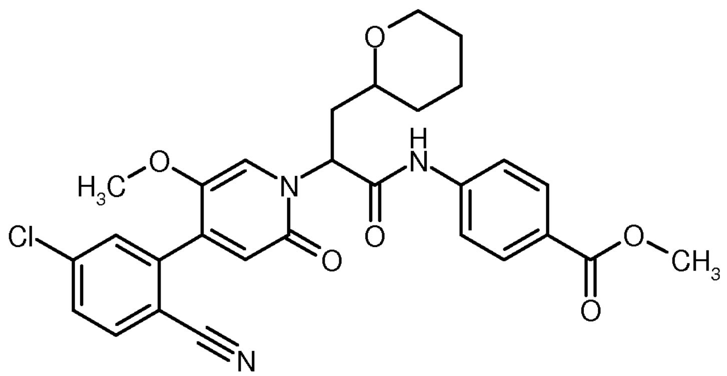

Gemäß allgemeiner Methode 5B wurden 2.20 g (5.28 mmol) 2-[4-(5-Chlor-2-cyanophenyl)-5-methoxy-2-oxopyridin-1(2*H*)-yl]-3-[tetrahydro-2*H*-pyran-2-yl]propansäure (Gemisch racemischer Diastereomere), 798 mg (5.28 mmol) 4-Aminobenzoesäuremethylester, 750 mg (5.28 mmol) Oxima und 822 µl (5.28 mmol) DIC in 110 ml Dimethylformamid zur Reaktion gebracht. Das Reaktionsgemisch wurde mittels Flash-Chromatographie (80 g Kartusche, 60 ml/min, Cyclohexan/Essigsäureethylester-Gradient) gereinigt. Ausbeute: 905 mg (31% d. Th.)
LC/MS [Methode 1]: $R_t$ = 1.14-1.16 min; MS (ESIpos): m/z = 550 $(M+H)^+$.

**Beispiel 36.1A**

1,4-Dioxan-2-ylmethyltrifluormethansulfonat (Racemat)

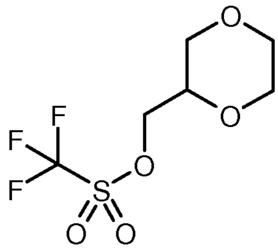

Nach der allgemeinen Methode 8A wurden 249 mg (2.00 mmol) 1,4-Dioxan-2-ylmethanol (Racemat) mit 355 µl (2.10 mmol, 1.05 eq.) Trifluormethansulfonsäureanhydrid in Gegenwart von 256 µl (2.20 mmol, 1.1 eq.) 2,6-Dimethylpyridin umgesetzt. Das Rohprodukt wurde ohne weitere Reinigung in der nächsten Stufe umgesetzt.
$^1$H-NMR (400 MHz, DMSO-$d_6$): δ [ppm] = 4.35 (dd, 1H), 4.27 (dd, 1H), 3.88-3.76 (m, 2H), 3.75-3.61 (m, 3H), 3.55-3.45 (m, 1H), 3.30 (t, 1H).

**Beispiel 36.1B**

2-[4-(5-Chlor-2-cyanophenyl)-5-methoxy-2-oxopyridin-1(2*H*)-yl]-3-(1,4-dioxan-2-yl)propansäure-*tert*.-butylester (Gemisch racemischer Diastereomere)

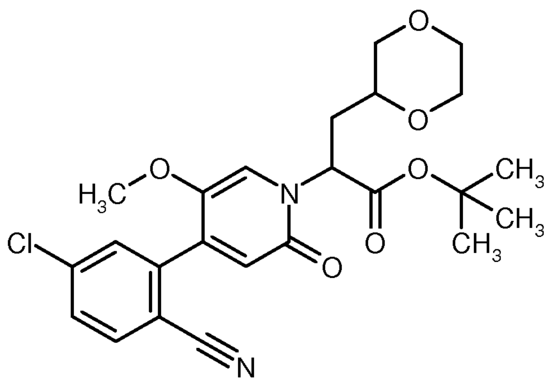

Nach der allgemeinen Methode 7B wurden 346 mg (93% Reinheit, 0.86 mmol) [4-(5-Chlor-2-cyanophenyl)-5-methoxy-2-oxopyridin-1(2*H*)-yl]essigsäure-*tert*.-butylester in Gegenwart von 0.95 ml (0.95 mmol, 1.1 eq.) Bis-(trimethylsilyl)-lithiumamid (1M in THF) mit 430 mg (90% Reinheit, 1.55 mmol, 1.8 eq.) 1,4-Dioxan-2-ylmethyltrifluormethansulfonat (Racemat) umgesetzt. Ausbeute: 133 mg (33% d. Th.)
LC/MS [Methode 1]: $R_t$ = 1.04 min; MS (ESIpos): m/z = 475 $(M+H)^+$.

**Beispiel 36.1C**

2-[4-(5-Chlor-2-cyanophenyl)-5-methoxy-2-oxopyridin-1(2*H*)-yl]-3-(1,4-dioxan-2-yl)propansäure (Gemisch racemischer Diastereomere)

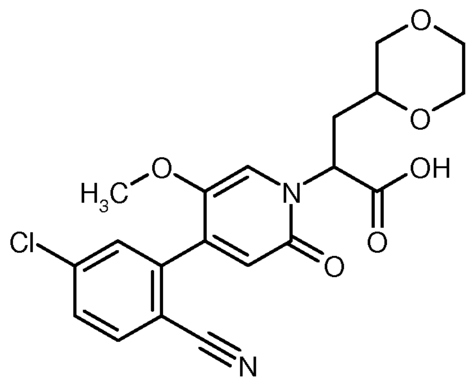

Nach der allgemeinen Methode 6A wurden 133 mg (0.28 mmol) 2-[4-(5-Chlor-2-cyanophenyl)-5-methoxy-2-oxopyridin-1(2*H*)-yl]-3-(1,4-dioxan-2-yl)propansäure-*tert*.-butylester (Gemisch racemischer Diastereomere) mit TFA verseift. Ausbeute: 132 mg (60% Reinheit, 68% d. Th.)
LC/MS [Methode 8]: $R_t$ = 0.99 min; MS (ESIpos): m/z = 419 $(M+H)^+$.

**Beispiel 36.1D**

4-({2-[4-(5-Chlor-2-cyanophenyl)-5-methoxy-2-oxopyridin-1(2*H*)-yl]-3-(1,4-dioxan-2-yl)-propanoyl}amino)benzoesäure-*tert*.-butylester (Gemisch racemischer Diastereomere)

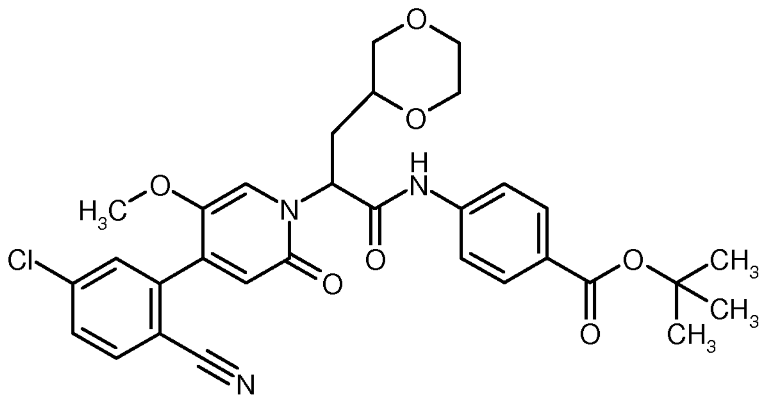

Nach der allgemeinen Methode 5A wurden 132 mg (60% Reinheit, 0.19 mmol) 2-[4-(5-Chlor-2-cyanophenyl)-5-methoxy-2-oxopyridin-1(2*H*)-yl]-3-(1,4-dioxan-2-yl)propansäure (Gemisch racemischer Diastereomere) mit 40 mg (0.21 mmol, 1.1 eq.) 4-Aminobenzoesäure-*tert*.-butylester umgesetzt. Ausbeute: 106 mg (94% d. Th.)
LC/MS [Methode 8]: racemisches Diastereomer 1: $R_t$ = 1.44 min; MS (ESIneg): m/z = 592 $(M-H)^-$; racemisches Diastereomer 2: $R_t$ = 1.46 min; MS (ESIneg): m/z = 592 $(M-H)^-$.

**Beispiel 37.1A**

2-Fluorethyl-trifluormethansulfonat

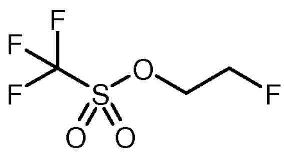

Zu 2.89 ml (17.2 mmol) Trifluormethansulfonsäureanhydrid in 5 ml Dichlormethan wurde bei -78°C eine Lösung aus 1.00 g (15.6 mmol) 2-Fluorethanol und 2.39 ml (17.2 mmol) Triethylamin in 5 ml Dichlormethan getropft, so dass die interne Temperatur -50°C nicht übersteigt. Es wurde 15 min bei -78°C nachgerührt und spontan auf RT erwärmt. Die Reaktionsmischung wurde mit 50 ml Methyl-*tert.*-butylether verdünnt, dreimal mit 25 ml einer Mischung aus gesättigter, wässriger Natriumchlorid/1N Salzsäure (3:1) gewaschen, über Magnesiumsulfat getrocknet, filtriert und bei 25°C und ≥100 mbar Druck eingeengt. Ausbeute: 2.3 g (75% d. Th.)
$^1$H-NMR (400 MHz, DMSO-$d_6$): δ [ppm] = 4.78-4.74 (m, 1H), 4.66-4.62 (m, 1H), 4.61-4.58 (m, 1H), 4.54-4.50 (m, 1H).

**Beispiel 37.1B**

2-[4-(5-Chlor-2-cyanophenyl)-5-methoxy-2-oxopyridin-1(2*H*)-yl]-4-fluorbutansäure-*tert.*-butylester (Racemat)

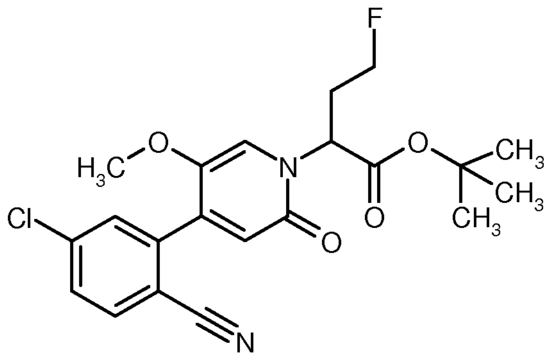

Gemäß allgemeiner Methode 7B wurden 500 mg (1.26 mmol) [4-(5-Chlor-2-cyanophenyl)-5-methoxy-2-oxopyridin-1(2*H*)-yl]essigsäure-*tert.*-butylester, 445 mg (2.27 mmol) 2-Fluorethyl-trifluormethansulfonat und 1.39 ml (1.39 mmol) Bis-(trimethylsilyl)-lithiumamid (1M in THF) in 10 ml THF zur Reaktion gebracht. Nach säulenchromatographischer Reinigung (120 g Silica Kartusche, Fluss: 80 ml/min, Cyclohexan/Essigsäureethylester-Gradient) wurde die Titelverbindung erhalten. Ausbeute: 360 mg (67% d. Th.)
LC/MS [Methode 1]: $R_t$ = 1.07 min; MS (ESIpos): m/z = 421 $(M+H)^+$
$^1$H-NMR (400 MHz, DMSO-$d_6$): δ [ppm] = 7.99 (d, 1H), 7.75-7.72 (m, 2H), 7.44 (s, 1H), 6.51 (s, 1H), 5.17 (dd, 1H), 4.66-4.49 (m, 1H), 4.44-4.27 (m, 1H), 3.63 (s, 3H), 2.62-2.40 (m, 2H), 1.40 (s, 9H).

**Beispiel 37.1C**

2-[4-(5-Chlor-2-cyanophenyl)-5-methoxy-2-oxopyridin-1(2*H*)-yl]-4-fluorbutansäure (Racemat)

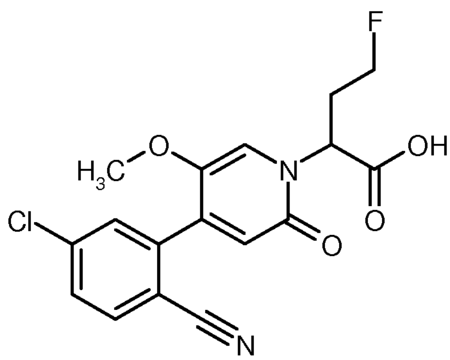

Gemäß allgemeiner Methode 6A wurden 359 mg (853 μmol) 2-[4-(5-Chlor-2-cyanophenyl)-5-methoxy-2-oxopyridin-1(2*H*)-yl]-4-fluorbutansäure-*tert.*-butylester (Racemat) in 8.5 ml Dichlormethan mit 2.5 ml (32 mmol) TFA umgesetzt. Ausbeute: 306 mg (96% d. Th.)

LC/MS [Methode 1]: $R_t$ = 0.80 min; MS (ESIneg): m/z = 363 $(M-H)^-$ $^1$H-NMR (400 MHz, DMSO-$d_6$): δ [ppm] = 13.1 (s, 1H), 7.99 (d, 1H), 7.74 (s, 1H), 7.73 (dd, 1H), 7.49 (s, 1H), 6.50 (s, 1H), 5.22 (dd, 1H), 4.66-4.48 (m, 1H), 4.42-4.24 (m, 1H), 3.63 (s, 3H), 2.65-2.42 (m, 2H).

**Beispiel 37.1D**

4-({2-[4-(5-Chlor-2-cyanophenyl)-5-methoxy-2-oxopyridin-1(2*H*)-yl]-4-fluorbutanoyl}amino)-benzoesäure-*tert*.-butylester (Racemat)

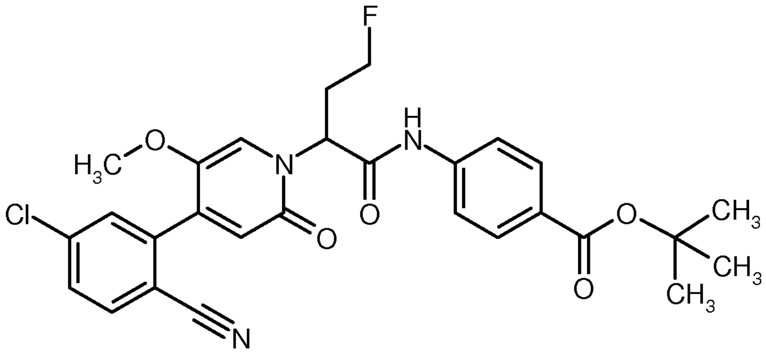

Gemäß allgemeiner Methode 5B wurden 100 mg (274 μmol) 2-[4-(5-Chlor-2-cyanophenyl)-5-methoxy-2-oxopyridin-1(2*H*)-yl]-4-fluorbutansäure (Racemat), 53.0 mg (274 μmol) 4-Aminobenzoesäure-*tert*.-butylester, 39.0 mg (274 μmol) Oxima und 43.0 μl (274 μmol) DIC in 5 ml Dimethylformamid zur Reaktion gebracht. Nach Filtration wurde die Titelverbindung erhalten. Ausbeute: 117 mg (78% d. Th.)
LC/MS [Methode 1]: $R_t$ = 1.20 min; MS (ESIpos): m/z = 540 $(M+H)^+$
$^1$H-NMR (400 MHz, DMSO-$d_6$): δ [ppm] = 10.8 (s, 1H), 8.00 (d, 1H), 7.87 (d, 2H), 7.77-7.73 (m, 4H), 7.51 (s, 1H), 6.55 (s, 1H), 5.85 (t, 1H), 4.67-4.49 (m, 1H), 4.47-4.28 (m, 1H), 3.69 (s, 3H), 2.69-2.55 (m, 2H), 1.54 (s, 9H).

**Beispiel 38.1A**

2,2-Difluorethyl-trifluormethansulfonat

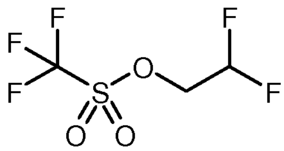

Zu 2.26 ml (13.4 mmol) Trifluormethansulfonsäureanhydrid in 5 ml Dichlormethan wurde bei -78°C eine Lösung aus 1.00 g (12.2 mmol) 2,2-Difluorethanol und 1.87 ml (13.4 mmol) Triethylamin in 5 ml Dichlormethan getropft, so dass die interne Temperatur -50°C nicht überstieg. Es wurde 15 min bei -78°C nachgerührt und spontan auf RT erwärmt. Die Reaktionsmischung wurde mit 50 ml Methyl-*tert*.-butylether verdünnt und dreimal mit 25 ml einer Mischung aus gesättigter, wässriger Natriumchlorid/1N Salzsäure (3:1) gewaschen, über Magnesiumsulfat getrocknet, filtriert und bei 25°C und ≥100 mbar Druck eingeengt. Ausbeute: 1.48 g (51% d. Th.)
$^1$H-NMR (400 MHz, $CDCl_3$): δ [ppm] = 6.05 (tt, 1H), 4.59 (dt, 2H).

**Beispiel 38.1B**

2-[4-(5-Chlor-2-cyanophenyl)-5-methoxy-2-oxopyridin-1(2*H*)-yl]-4,4-difluorbutansäure-*tert*.-butylester (Racemat)

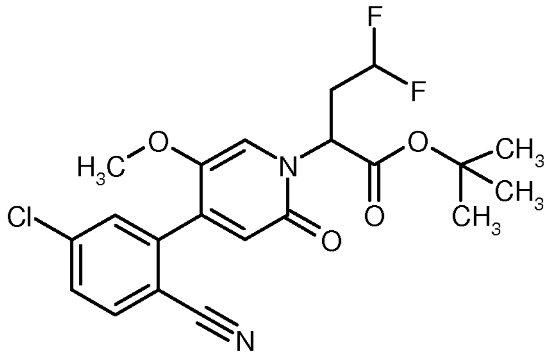

Gemäß allgemeiner Methode 7B wurden 150 mg (388 μmol) [4-(5-Chlor-2-cyanophenyl)-5-methoxy-2-oxopyridin-1(2*H*)-yl]essigsäure-*tert*.-butylester, 125 mg (582 μmol) 2,2-Difluorethyltrifluormethansulfonat und 427 μl (427 μmol) Bis-(trimethylsilyl)-lithiumamid (1M in THF) in 3 ml THF zur Reaktion gebracht. Nach säulenchromatographischer Reinigung (24 g Silica Kartusche, Fluss: 35 ml/min, Cyclohexan/Essigsäureethylester-Gradient) wurde die Titelverbindung erhalten. Ausbeute: 122 mg (71 % d. Th.)
LC/MS [Methode 1]: $R_t$ = 1.10 min; MS (ESIpos): m/z = 439 $(M+H)^+$
$^1$H-NMR (400 MHz, DMSO-$d_6$): δ[ppm] = 7.99 (d, 1H), 7.74-7.70 (m, 2H), 7.50 (s, 1H), 6.52 (s, 1H), 6.19 (tt, 1H), 5.29-5.20 (m, 1H), 3.64 (s, 3H), 2.83-2.65 (m, 2H), 1.39 (s, 9H).

**Beispiel 38.1C**

2-[4-(5-Chlor-2-cyanophenyl)-5-methoxy-2-oxopyridin-1(2*H*)-yl]-4,4-difluorbutansäure (Racemat)

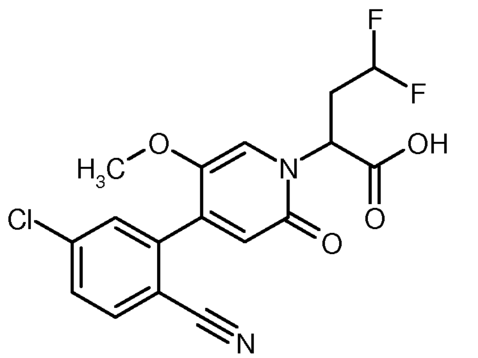

Gemäß allgemeiner Methode 6A wurden 114 mg (260 μmol) 2-[4-(5-Chlor-2-cyanophenyl)-5-methoxy-2-oxopyridin-1(2*H*)-yl]-4,4-difluorbutansäure-*tert*.-butylester (Racemat) in 8 ml Dichlormethan mit 400 μl (5.20 mmol) TFA umgesetzt. Ausbeute: 99 mg (91% d. Th.)
LC/MS [Methode 1]: $R_t$ = 0.85 min; MS (ESIneg): m/z = 381 $(M-H)^-$
$^1$H-NMR (400 MHz, DMSO-$d_6$): δ [ppm] = 13.4 (s, 1H), 7.99 (d, 1H), 7.73 (dd, 1H), 7.72 (s, 1H), 7.55 (s, 1H), 6.51 (s, 1H), 6.18 (tt, 1H), 5.31-5.25 (m, 1H), 3.63 (s, 3H), 2.83-2.65 (m, 2H).

**Beispiel 38.1D**

4-({2-[4-(5-Chlor-2-cyanophenyl)-5-methoxy-2-oxopyridin-1(2*H*)-yl]-4,4-difluorbutanoyl}amino)-benzoesäureethylester (Racemat)

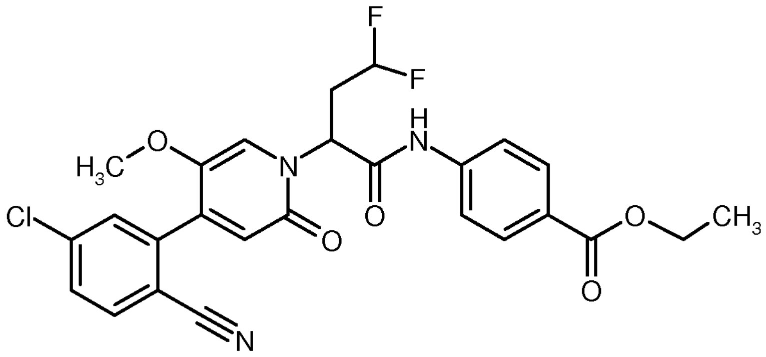

Gemäß allgemeiner Methode 5B wurden 97.0 mg (253 µmol) 2-[4-(5-Chlor-2-cyanophenyl)-5-methoxy-2-oxopyridin-1(2*H*)-yl]-4,4-difluorbutansäure (Racemat), 42.0 mg (253 µmol) 4-Aminobenzoesäureethylester, 36.0 mg (253 µmol) Oxima und 39.0 µl (253 µmol) DIC in 2.5 ml Dimethylformamid zur Reaktion gebracht. Das Rohprodukt wurde mittels präparativer HPLC [Säule: Chromatorex C18, 10 µm, 125x30 mm, Eluent: Acetonitril/0.05% Ameisensäure-Gradient (0 bis 3 min 10% Acetonitril, bis 35 min 90% Acetonitril und weitere 3 min 90% Acetonitril)] gereinigt. Ausbeute: 81.7 mg (60% d. Th.)
LC/MS [Methode 1]: $R_t$ = 1.12 min; MS (ESIpos): m/z = 530 $(M+H)^+$
$^1$H-NMR (400 MHz, DMSO-$d_6$): δ [ppm] = 10.8 (s, 1H), 8.00 (d, 1H), 7.94 (d, 2H), 7.76 (d, 2H), 7.75-7.71 (m, 2H), 7.56 (s, 1H), 6.55 (s, 1H), 6.15 (tt, 1H), 5.90 (dd, 1H), 4.29 (q, 2H), 3.69 (s, 3H), 2.97-2.78 (m, 2H), 1.31 (t, 3H).

**Beispiel 39.1A**

2,2,2-Trifluormethyl-trifluormethansulfonat

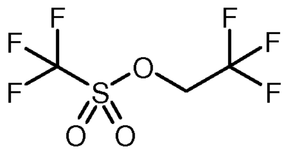

Zu 1.85 ml (11.0 mmol) Trifluormethansulfonsäureanhydrid in 5 ml Dichlormethan wurde bei -78°C eine Lösung aus 1.00 g (10.0 mmol) 2,2,2-Trifluorethanol und 1.53 ml (11.0 mmol) Triethylamin in 5 ml Dichlormethan getropft, so dass die interne Temperatur -50°C nicht überstieg. Es wurde 15 min bei -78°C nachgerührt und spontan auf RT erwärmt. Die Reaktionsmischung wurde mit 50 ml Methyl-*tert.*-butylether verdünnt und dreimal mit 25 ml einer Mischung aus gesättigter, wässriger Natriumchlorid/1N Salzsäure (3:1) gewaschen, über Magnesiumsulfat getrocknet, filtriert und bei 25°C und ≥100 mbar Druck eingeengt. Ausbeute: 1.0 g (43% d. Th.)
$^1$H-NMR (400 MHz, $CDCl_3$): δ [ppm] = 4.71 (q, 2H).

**Beispiel 39.1B**

2-[4-(5-Chlor-2-cyanophenyl)-5-methoxy-2-oxopyridin-1(2*H*)-yl]-4,4,4-trifluorbutansäure-*tert.*-butylester (Racemat)

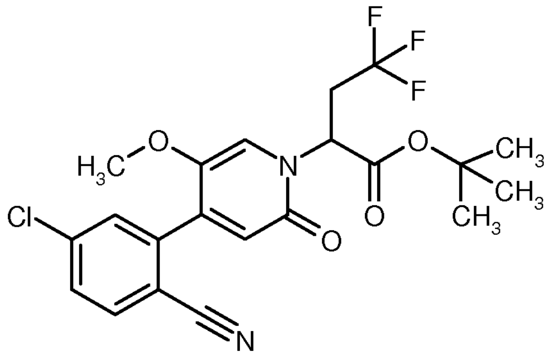

Gemäß allgemeiner Methode 7B wurden 500 mg (1.29 mmol) [4-(5-Chlor-2-cyanophenyl)-5-methoxy-2-oxopyridin-1(2*H*)-yl]essigsäure-tert.-butylester, 360 mg (1.55 mmol) 2,2,2-Trifluormethyl-trifluormethansulfonat und 1.42 ml (1.42 mmol) Bis-(trimethylsilyl)-lithiumamid (1M in THF) in 10 ml THF zur Reaktion gebracht. Nach säulenchromatographischer Reinigung (24 g Silica Kartusche, Fluss: 35 ml/min, Cyclohexan/Essigsäureethylester-Gradient) wurde die Titelverbindung erhalten. Ausbeute: 66 mg (11% d. Th.)
LC/MS [Methode 1]: $R_t$ = 1.12min; MS (ESIpos): m/z = 457 $(M+H)^+$.

**Beispiel 39.1C**

2-[4-(5-Chlor-2-cyanophenyl)-5-methoxy-2-oxopyridin-1(2*H*)-yl]-4,4,4-trifluorbutansäure (Racemat)

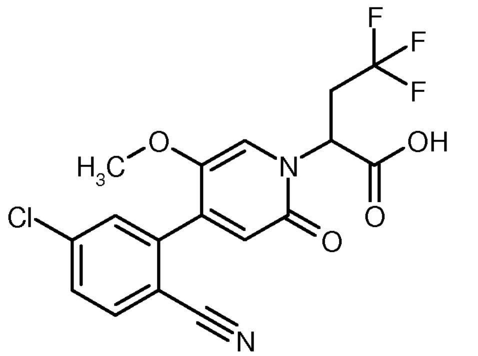

Gemäß allgemeiner Methode 6A wurden 65.0 mg (142 μmol) 2-[4-(5-Chlor-2-cyanophenyl)-5-methoxy-2-oxopyridin-1(2*H*)-yl]-4,4,4-trifluorbutansäure-*tert*.-butylester (Racemat) in 1.6 ml Dichlormethan mit 411 μl (5.34 mmol) TFA umgesetzt. Ausbeute: 53 mg (81% d. Th.)
LC/MS [Methode 1]: $R_t$ = 0.89 min; MS (ESIpos): m/z = 401 $(M+H)^+$
$^1$H-NMR (400 MHz, DMSO-$d_6$): δ [ppm] = 13.5 (br. s, 1H), 7.99 (d, 1H), 7.74-7.72 (m, 2H), 7.59 (s, 1H), 6.52 (s, 1H), 5.43-5.38 (m, 1H), 3.63 (s, 3H), 3.33-3.14 (m, 2H).

**Beispiel 39.1D**

4-({2-[4-(5-Chlor-2-cyanophenyl)-5-methoxy-2-oxopyridin-1(2*H*)-yl]-4,4,4-trifluorbutanoyl}-amino)benzoesäureethylester (Racemat)

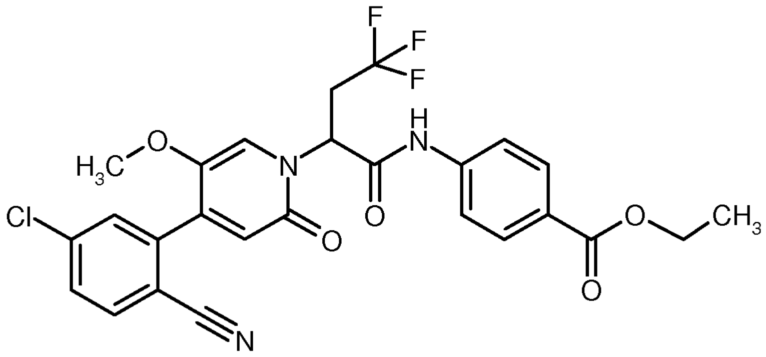

Gemäß allgemeiner Methode 5B wurden 63.0 mg (157 μmol) 2-[4-(5-Chlor-2-cyanophenyl)-5-methoxy-2-oxopyridin-1(2*H*)-yl]-4,4,4-trifluorbutansäure (Racemat), 26.0 mg (157 μmol) 4-Aminobenzoesäureethylester, 22.3 mg (157 μmol) Oxima und 24.0 μl (157 μmol) DIC in 1.6 ml Dimethylformamid zur Reaktion gebracht. Das Reaktionsprodukt wurde mittels präparativer HPLC [Säule: Chromatorex C18, 10 μm, 125x30 mm, Eluent: Acetonitril/0.05% Ameisensäure-Gradient (0 bis 3 min 10% Acetonitril, bis 35 min 90% Acetonitril und weitere 3 min 90% Acetonitril)] gereinigt. Ausbeute: 25.1 mg (28% d. Th.)
LC/MS [Methode 1]: $R_t$ = 1.15 min; MS (ESIpos): m/z = 548 $(M+H)^+$
$^1$H-NMR (400 MHz, DMSO-$d_6$): δ [ppm] = 10.9 (s, 1H), 8.00 (d, 1H), 7.95 (d, 2H), 7.78 (d, 2H), 7.74 (dd, 1H), 7.72 (s, 1H), 7.62 (s, 1H), 6.56 (s, 1H), 6.11-6.03 (m, 1H), 4.29 (q, 2H), 3.69 (s, 3H), 3.57-3.44 (m, 1H), 3.38-3.26 (m, 1H), 1.31 (t, 3H).

## Beispiel 40.1A

2-Fluorpropyltrifluormethansulfonat (Racemat)

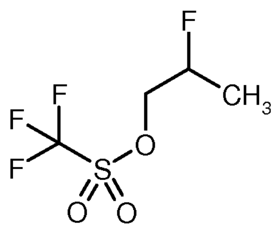

Nach der allgemeinen Methode 8A wurden 156 mg (1.94 mmol) 2-Fluorpropan-1-ol mit 361 μl (2.13 mmol, 1.1 eq.) Trifluormethansulfonsäureanhydrid in Gegenwart von 297 μl (2.13 mmol, 1.1 eq.) Triethylamin umgesetzt. Das Rohprodukt wurde ohne weitere Reinigung in der nächsten Stufe umgesetzt.

## Beispiel 40.1B

2-[4-(5-Chlor-2-cyanophenyl)-5-methoxy-2-oxopyridin-1(2*H*)-yl]-4-fluorpentansäure-*tert*.-butylester (Gemisch racemischer Diastereomere)

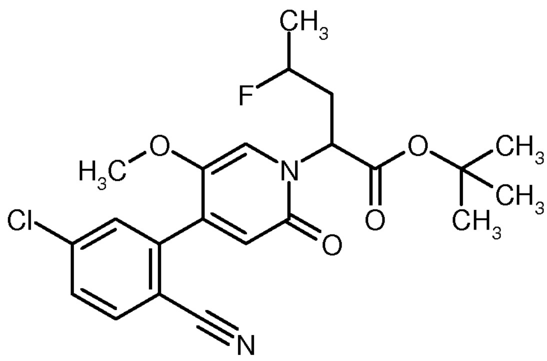

Nach der allgemeinen Methode 7B wurden 450 mg (94% Reinheit, 1.13 mmol) [4-(5-Chlor-2-cyanophenyl)-5-methoxy-2-oxopyridin-1(2*H*)-yl]essigsäure-*tert*.-butylester in Gegenwart von 1.24 ml (1.24 mmol, 1.1 eq.) Bis-(trimethylsilyl)-lithiumamid (1M in THF) mit 356 mg (1.69 mmol, 1.5 eq.) 2-Fluorpropyltrifluormethansulfonat (Racemat) umgesetzt. Ausbeute: 270 mg (52% d. Th.)
LC/MS [Methode 1]: $R_t$ = 1.09 min; MS (ESIpos): m/z = 435 $(M+H)^+$.

**Beispiel 40.1C**

2-[4-(5-Chlor-2-cyanophenyl)-5-methoxy-2-oxopyridin-1(2*H*)-yl]-4-fluorpentansäure (Gemisch racemischer Diastereomere)

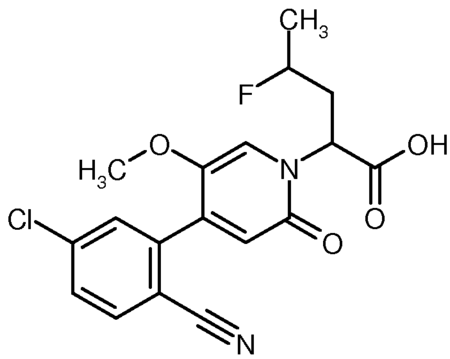

Nach der allgemeinen Methode 6A wurden 270 mg (0.59 mmol) 2-[4-(5-Chlor-2-cyanophenyl)-5-methoxy-2-oxopyridin-1(2*H*)-yl]-4-fluorpentansäure-*tert*.-butylester (Gemisch racemischer Diastereomere) mit TFA verseift. Ausbeute: 222 mg (85% Reinheit, 84% d. Th.)
LC/MS [Methode 1]: $R_t$ = 0.86 min; MS (ESIpos): m/z = 379 $(M+H)^+$.

**Beispiel 40.1D**

4-({2-[4-(5-Chlor-2-cyanophenyl)-5-methoxy-2-oxopyridin-1(2*H*)-yl]-4-fluorpentanoyl}-amino)benzoesäureethylester(Gemisch racemischer Diastereomere)

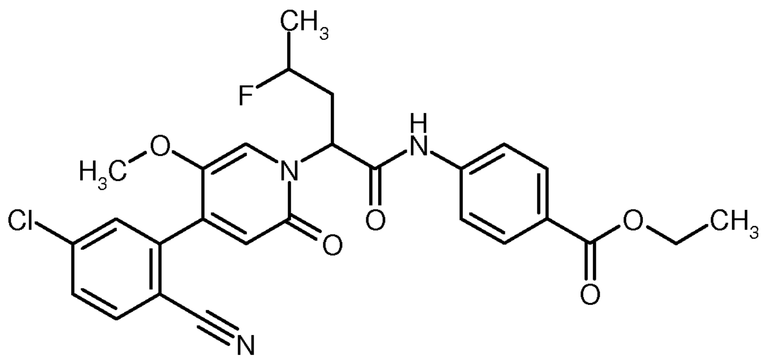

Nach der allgemeinen Methode 5A wurden 222 mg (85% Reinheit, 0.50 mmol) 2-[4-(5-Chlor-2-cyanophenyl)-5-methoxy-2-oxopyridin-1(2*H*)-yl]-4-fluorpentansäure (Gemisch racemischer Diastereomere) mit 91 mg (0.55 mmol, 1.1 eq.) 4-Aminobenzoesäureethylester umgesetzt. Ausbeute: 180 mg (91% Reinheit, 63% d. Th.)
LC/MS [Methode 1]: $R_t$ = 1.12 min; MS (ESIpos): m/z = 526 $(M+H)^+$.

**Beispiel 41.1A**

2-[4-(5-Chlor-2-cyanophenyl)-5-methoxy-2-oxopyridin-1(2*H*)-yl]-2,4,5-trideoxy-5,5,5-trifluor-4-methylpentonsäure-*tert*. -butylester (Diastereomerengemisch)

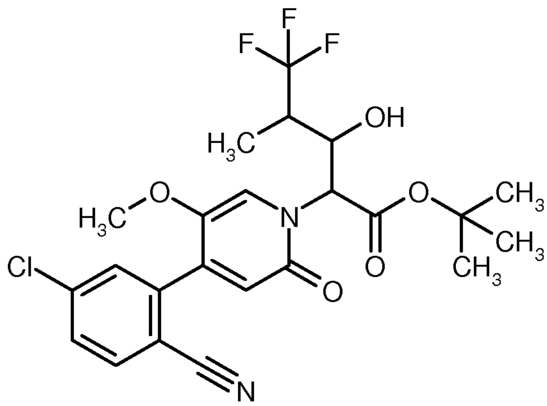

Eine Lösung von 400 mg (1.07 mmol) [4-(5-Chlor-2-cyanophenyl)-5-methoxy-2-oxopyridin-1(2*H*)-yl]essigsäure-*tert*.-butylester in 10.8 ml Tetrahydrofuran wurde bei -70°C tropfenweise mit 1.17 ml (1.17 mmol, 1.1 eq.) Bis-(trimethylsilyl)-lithiumamid (1M in THF) versetzt, 10 min bei -70°C gerührt, mit einer Lösung von 175 mg (1.39 mmol, 1.3 eq.) 2-(Trifluormethyl)propionaldehyd in 0.8 ml Tetrahydrofuran versetzt und 1 h bei -70°C gerührt. Das Reaktionsgemisch wurde auf RT erwärmt, weitere 30 min bei RT gerührt und mit 5 ml gesättigter, wässriger Ammoniumchlorid-Lösung versetzt. Nach Phasentrennung wurde die wässrige Phase zweimal mit Essigsäureethylester extrahiert. Die vereinigten, organischen Phasen wurden mit gesättigter, wässriger Natriumchlorid-Lösung gewaschen, getrocknet (Natriumsulfat), filtriert und im Vakuum eingeengt und getrocknet. Das Rohprodukt wurde mittels Flash-Chromatographie (KP-SIL, Essigsäureethylester/Cyclohexan 20-50%) aufgereinigt. Ausbeute: 274 mg (75% Reinheit, 38% d. Th.)
LC/MS [Methode 1]: $R_t$ = 1.15 min; MS (ESIpos): m/z = 501 $(M+H)^+$.

**Beispiel 41.1B**

2-[4-(5-Chlor-2-cyanophenyl)-5-methoxy-2-oxopyridin-1(2*H*)-yl]-5,5,5-trifluor-4-methylpent-2-ensäure-*tert*. -butylester (Diastereomerengemisch)

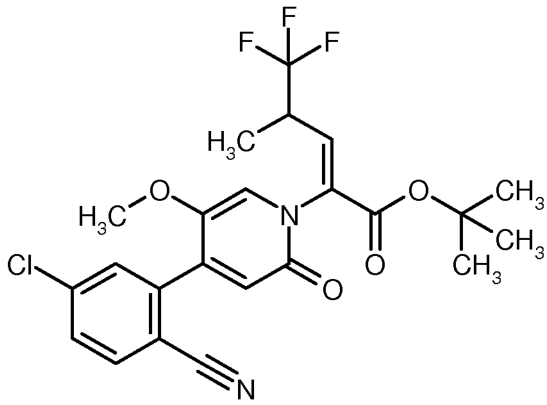

Eine Lösung von 270 mg (0.40 mmol) 2-[4-(5-Chlor-2-cyanophenyl)-5-methoxy-2-oxopyridin-1(2*H*)-yl]-2,4,5-trideoxy-5,5,5-trifluor-4-methylpentonsäure-*tert.*-butylester (Diastereomerengemisch) in 6 ml Dichlormethan wurde bei RT tropfenweise mit 64 μl (0.48 mmol, 1.2 eq.) Diethylaminoschwefeltrifluorid versetzt, 90 min bei RT gerührt und anschließend mit 3 ml Dichlormethan und 6 ml gesättigter, wässriger Natriumhydrogencarbonat-Lösung versetzt. Nach Phasentrennung wurde die wässrige Phase mit Essigsäureethylester extrahiert. Die vereinigten, organischen Phasen wurden mit gesättigter, wässriger Natriumchlorid-Lösung gewaschen, getrocknet (Natriumsulfat), filtriert und im Vakuum eingeengt und getrocknet. Ausbeute: 224 mg (72% Reinheit, 83% d. Th.)
LC/MS [Methode 2]: $R_t$= 3.78 min; MS (ESIpos): m/z = 483 $(M+H)^+$.

**Beispiel 41.1C**

2-[4-(5-Chlor-2-cyanophenyl)-5-methoxy-2-oxopyridin-1(2*H*)-yl]-5,5,5-trifluor-4-methylpentansäure-*tert*. -butylester (Gemisch racemischer Diastereomere)

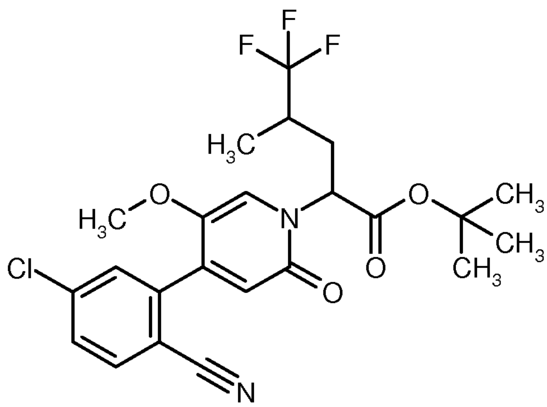

193 mg (72% Reinheit, 0.29 mmol) 2-[4-(5-Chlor-2-cyanophenyl)-5-methoxy-2-oxopyridin-1(2*H*)-yl]-5,5,5-trifluor-4-methylpent-2-ensäure-*tert.*-butylester (Diastereomerengemisch) wurden bei RT mit 10 ml einer "Hot Stryker's"-Reagenzlösung [B. A. Baker et al. Org. Lett. 2008, 10, 289-292] versetzt und das Reaktionsgemisch 6 h bei RT gerührt. Nach Zugabe weiterer 8 ml einer "Hot Stryker's"-Reagenzlösung wurde das Reaktionsgemisch über Nacht bei RT gerührt und anschließend im Vakuum eingeengt. Das Rohprodukt wurde dreimal mit jeweils 15 ml Acetonitril verrührt und dekantiert. Die vereinigten, organischen Phasen wurden im Vakuum eingeengt. Der Rückstand wurde mittels Flash-Chromatographie (KP-SIL, Essigsäureethylester/Cyclohexan 20-33%) aufgereinigt. Ausbeute: 169 mg (92% Reinheit, quant.)
LC/MS [Methode 1]: $R_t$ = 1.21 min; MS (ESIpos): m/z = 485 $(M+H)^+$.

**Beispiel 41.1D**

2-[4-(5-Chlor-2-cyanophenyl)-5-methoxy-2-oxopyridin-1(2*H*)-yl]-5,5,5-trifluor-4-methylpentansäure (Gemisch racemischer Diastereomere)

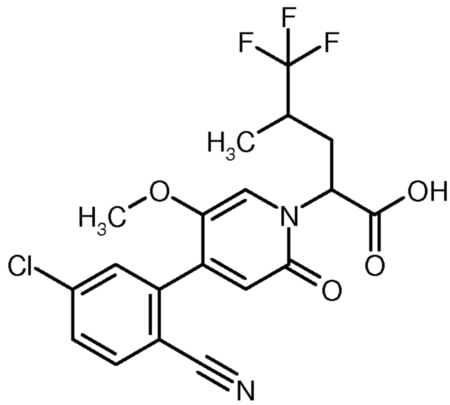

Nach der allgemeinen Methode 6A wurden 190 mg (92% Reinheit, 0.36 mmol) 2-[4-(5-Chlor-2-cyanophenyl)-5-methoxy-2-oxopyridin-1(2*H*)-yl]-5,5,5-trifluor-4-methylpentansäure-*tert.*-butylester (Gemisch racemischer Diastereomere) mit TFA verseift. Das Rohprodukt wurde ohne weitere Reinigung in der nächsten Stufe umgesetzt. Ausbeute: 129 mg

**Beispiel 41.1E**

4-({2-[4-(5-Chlor-2-cyanophenyl)-5-methoxy-2-oxopyridin-1(2*H*)-yl]-5,5,5-trifluor-4-methyl-pentanoyl}amino)benzoesäure-*tert.*-butylester (Gemisch racemischer Diastereomere)

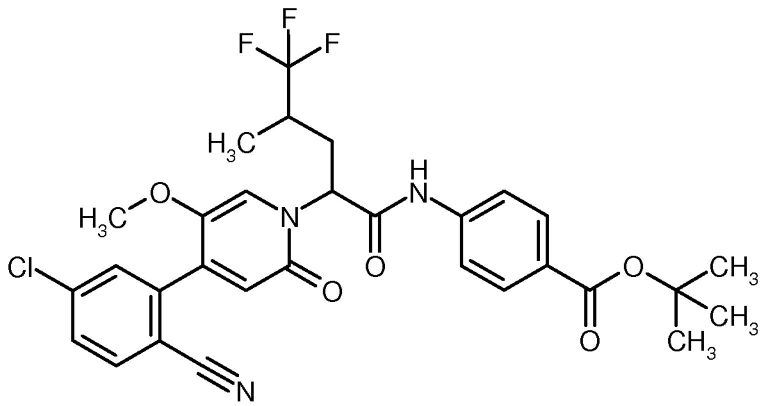

Nach der allgemeinen Methode 5A wurden 129 mg 2-[4-(5-Chlor-2-cyanophenyl)-5-methoxy-2-oxopyridin-1(2*H*)-yl]-5,5,5-trifluor-4-methylpentansäure (Gemisch racemischer Diastereomere) mit 59 mg (0.31 mmol, 1.1 eq.) 4-Aminobenzoesäure-*tert.*-butylester umgesetzt. Ausbeute: 51 mg (30% d. Th.)
LC/MS [Methode 1]: $R_t$ = 1.37 min; MS (ESIpos): m/z = 604 $(M+H)^+$.

**Beispiel 42.1A**

2-Hydroxy-4,4-dimethylpentansäure (Racemat)

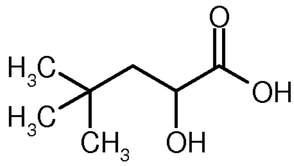

805 mg (5.54 mmol) 4-Methylleucin (Racemat) wurden in 11 ml Schwefelsäure (1M) vorgelegt und auf 0°C abgekühlt. Anschließend wurden 2.30 g (33.3 mmol) Natriumnitrit als Lösung in 6.5 ml Wasser über einen Zeitraum von 90 min langsam zugetropft. Die Lösung wurde 24 h bei RT nachgerührt. Es wurde vorsichtig mit 10 ml Wasser verdünnt und die wässrige Phase fünfmal mit 10 ml Methyl-*tert*.-butylether extrahiert. Die vereingten, organischen Phasen wurden mit 25 ml gesättigter, wässriger Natriumchlorid-Lösung gewaschen, über Natriumsulfat getrocknet, filtriert und das Lösungsmittel unter reduziertem Druck entfernt. Das Rohprodukt wurde ohne weitere Aufreinigung im nächsten Schritt eingesetzt. Ausbeute: 667 mg (82% d. Th.)
$^1$H-NMR (400 MHz, DMSO-$d_6$): δ[ppm] = 3.99 (dd, 1H), 1.56 (dd, 1H), 1.40 (dd, 1H), 0.93 (s, 9H).

**Beispiel 42.1B**

2-Hydroxy-4,4-dimethylpentansäurebenzylester (Racemat)

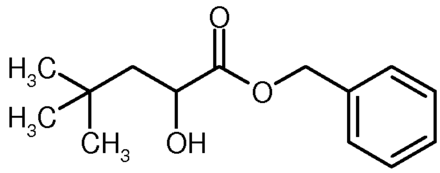

Zu einer Lösung von 667 mg (4.56 mmol) 2-Hydroxy-4,4-dimethylpentansäure (Racemat) in 8.7 ml Methanol und 1.7 ml Wasser wurden 743 mg (2.28 mmol) Cäsiumcarbonat gegeben. Die Reaktionsmischung wurde 60 min bei RT gerührt und anschließend das Lösungsmittel unter reduziertem Druck entfernt. Der Rückstand wurde im Hochvakuum getrocknet (4 h) und danach in 10 ml Dimethylformamid aufgenommen. Bei 0°C wurden 516 µl (4.33 mmol) Benzylbromid langsam zugetropft. Die Reaktionsmischung wurde 12 h bei RT gerührt, die Reaktion durch Zugabe von 25 ml Wasser beendet und die Reaktionsmischung dreimal mit 20 ml Essigsäureethylester extrahiert. Die vereinigten, organischen Phasen wurden über Magnesiumsulfat getrocknet, filtriert und das Lösungsmittel unter reduziertem Druck entfernt. Der Rückstand wurde mittels Flash-Chromatographie (40 g Silica Kartusche, 35 ml/min, Cyclohexan/Essigsäureethylester-Gradient) gereinigt. Ausbeute: 584 mg (54% d. Th.)
$^1$H-NMR (400 MHz, $CDCl_3$): δ [ppm] = 7.39-7.33 (m, 5H), 5.23 (d, 1H), 5.18 (d, 1H), 4.29 (ddd, 1H), 2.62 (d, 1H), 1.73 (dd, 1H), 1.49 (dd, 1H), 0.99 (s, 9H).

**Beispiel 42.1C**

4,4-Dimethyl-2-{[(trifluormethyl)sulfonyl]oxy}pentansäurebenzylester (Racemat)

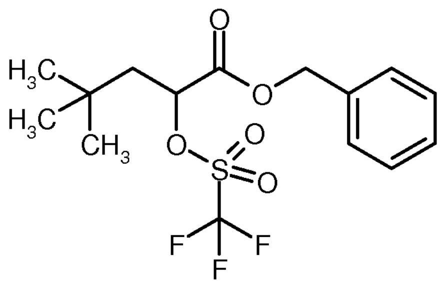

Gemäß allgemeiner Methode 8A wurden 236 mg (1.00 mmol) 2-Hydroxy-4,4-dimethylpentansäurebenzylester

(Racemat) in 10 ml Dichlormethan mit 175 µl (1.50 mmol) Lutidin und 254 µl (1.50 mmol) Trifluormethansulfonsäureanhydrid umgesetzt. Das Rohprodukt wurde ohne weitere Aufreinigung im nächsten Schritt eingesetzt. Ausbeute: 365 mg (99% d. Th.)

**Beispiel 42.1D**

2-[4-(5-Chlor-2-cyanophenyl)-5-methoxy-2-oxopyridin-1(2*H*)-yl]-4,4-dimethylpentansäurebenzylester (Racemat)

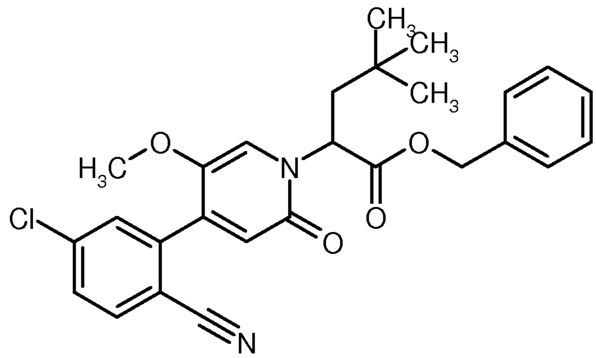

Eine Suspension von 261 mg (87% Reinheit, 870 µmol) 4-Chlor-2-(5-methoxy-2-oxo-1,2-dihydropyridin-4-yl)benzonitril in 10 ml THF wurde protionsweise mit 41.8 mg (1.04 mmol) Natriumhydrid (60%ig in Mineralöl) versetzt und 1 h bei RT nachgerührt. Zu der resultierenden Reaktionslösung wurden 481 mg (1.31 mmol) 4,4-Dimethyl-2-{[(trifluormethyl)sulfonyl]oxy}-pentansäurebenzylester (Racemat) als Lösung in 3 ml THF zügig getropft und nach vollständiger Zugabe 1.5 h bei RT nachgerührt. Die Reaktion wurde durch Zugabe von 10 ml gesättigter, wässriger Ammoniumchlorid-Lösung und 15 ml Methyl-*tert*.-butylether beendet. Die Phasen wurden getrennt und die wässrige Phase dreimal mit 10 ml Methyl-*tert*.-butylether extrahiert. Die vereinigten, organischen Phasen wurden über Magnesiumsulfat getrocknet, filtriert und das Lösungsmittel unter reduziertem Druck entfernt. Das Rohprodukt wurde mittels Flash-Chromatographie (80 g Silica Kartusche, 60 ml/min, Cyclohexan/Essigsäureethylester-Gradient) aufgereinigt. Ausbeute: 294 mg (71% d. Th.)

LC/MS [Methode 1]: $R_t$ = 1.29 min; MS (ESIpos): m/z = 479 $(M+H)^+$

$^1$H-NMR (400 MHz, DMSO-$d_6$): δ [ppm] = 7.99 (d, 1H), 7.74-7.70 (m, 2H), 7.55 (s, 1H), 7.39-7.30 (m, 5H), 6.53 (s, 1H), 5.56-5.50 (m, 1H), 5.18 (s, 2H), 3.63 (s, 3H), 2.19-2.10 (m, 2H), 0.87 (s, 9H).

**Beispiel 42.1E**

2-[4-(5-Chlor-2-cyanophenyl)-5-methoxy-2-oxopyridin-1(2*H*)-yl]-4,4-dimethylpentansäure (Racemat)

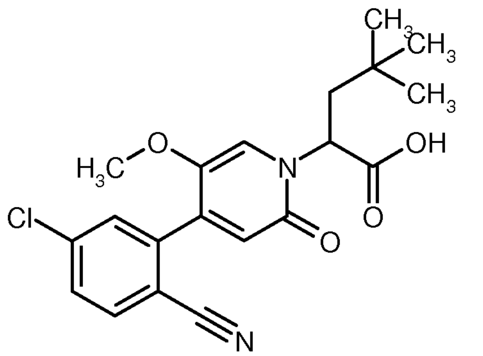

Zu einer Lösung von 140 mg (292 µmol) 2-[4-(5-Chlor-2-cyanophenyl)-5-methoxy-2-oxopyridin-1(2*H*)-yl]-4,4-dimethylpentansäurebenzylester (Racemat) in 5 ml THF (nicht trocken) wurden 17.5 mg (438 µmol, 60% in Mineralöl)

Natriumhydrid gegeben und 15 min nachgerührt. Die Reaktion wurde durch Zugabe von 5 ml gesättigter, wässriger Ammoniumchlorid-Lösung, 10 ml Dichlormethan und 0.5 ml Salzsäure (1N) beendet. Die Phasen wurden getrennt und die wässrige Phase dreimal mit 5 ml Dichlormethan extrahiert. Die vereinigten, organischen Phasen wurden über Natriumsulfat getrocknet, filtriert und das Lösungmittel unter reduziertem Druck entfernt. Der Rückstand entsprach der Titelverbindung und wurde ohne weitere Aufreinigung im nächsten Schritt eingesetzt. Ausbeute: 110 mg (83% d. Th., 86% Reinheit)

LC/MS [Methode 1]: $R_t$ = 0.99 min; MS (ESIneg): m/z = 387 $(M-H)^-$

$^1H$-NMR (400 MHz, DMSO-$d_6$): δ [ppm] = 13.1 (s, 1H), 7.97 (d, 1H), 7.73 (s, 1H), 7.72 (dd, 1H), 7.52 (s, 1H), 6.48 (s, 1H), 5.50-5.38 (br. s, 1H), 3.65 (s, 3H), 2.16-2.10 (m, 2H), 0.86 (s, 9H).

**Beispiel 42.1F**

4-({2-[4-(5-Chlor-2-cyanophenyl)-5-methoxy-2-oxopyridin-1(2*H*)-yl]-4,4-dimethyl-pentanoyl}amino)benzoesäure-*tert.* -butylester (Racemat)

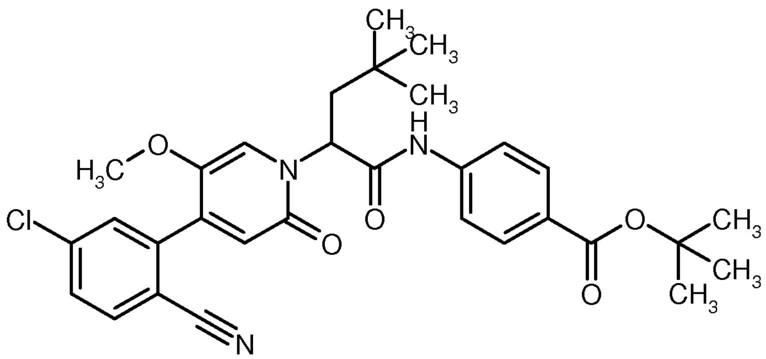

Gemäß allgemeiner Methode 5A wurden 110 mg (283 μmol) 2-[4-(5-Chlor-2-cyanophenyl)-5-methoxy-2-oxopyridin-1(2*H*)-yl]-4,4-dimethylpentansäure (Racemat), 65.6 mg (339 μmol) 4-Aminobenzoesäure-*tert.*-butylester, 129 mg (339 μmol) HATU und 148 μl (849 μmol) *N,N*-Diisopropylethylamin in 9 ml Dimethylformamid umgesetzt. Das Lösungsmittel wurde entfernt und der Rückstand mittels präparativer HPLC [Säule: Chromatorex C18, 10 μm, 125x30 mm, Eluent: Acetonitril/0.05% Ameisensäure-Gradient (0 bis 3 min 10% Acetonitril, bis 35 min 90% Acetonitril und weitere 3 min 90% Acetonitril)] aufgereinigt. Ausbeute: 100 mg (62% d. Th.)

LC/MS [Methode 4]: $R_t$ = 2.90 min; MS (ESIpos): m/z = 564 $(M+H)^+$

$^1H$-NMR (400 MHz, DMSO-$d_6$): δ [ppm] = 10.9 (s, 1H), 7.99 (d, 1H), 7.88 (d, 2H), 7.77 (d, 2H), 7.74-7.71 (m, 2H), 7.60 (s, 1H), 6.53 (s, 1H), 5.98 (dd, 1H), 3.70 (s, 3H), 2.14 (dd, 1H), 2.02 (dd, 1H), 1.54 (s, 9H), 0.92 (s, 9H).

**Beispiel 43.1A**

2,2-Difluorcyclopropancarbaldehyd

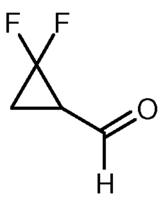

484 μl (5.55 mmol) Oxalylchlorid wurden mit 4Ä Molsieb in 5 ml Dichlormethan vorgelegt und auf -78°C abgekühlt. Bei -78°C wurden 410 μl (5.78 mmol) DMSO zugetropft und 5 min nachgerührt. Anschließend wurde eine Lösung von 500 mg (4.63 mmol) 2,2-Difluorcyclopropanmethanol in 5 ml Dichlormethan hinzugefügt und 30 min bei -78°C gerührt. Nach Zugabe von 1.93 ml (13.9 ml) Triethylamin wurde 10 min bei RT nachgerührt und die Reaktionslösung

mit 30 ml Wasser und 30 ml Dichlormethan verdünnt. Die Phasen wurden getrennt und die wässrige Phase zweimal mit 50 ml Dichlormethan extrahiert. Die vereinigten, organischen Phasen wurden mit gesättigter, wässriger Natriumchlorid-Lösung gewaschen, über Magnesiumsulfat getrocknet, filtriert und das Lösungsmittel unter reduziertem Druck entfernt. Das Rohprodukt wurde ohne weitere Reinigung im nächsten Schritt eingesetzt.

**Beispiel 43.1B**

2-[4-(5-Chlor-2-cyanophenyl)-5-methoxy-2-oxopyridin-l(2*H*)-yl]-3-(2,2-difluorcyclopropyl)prop-2-ensäure-tert. -butylester (Diastereomerengemisch)

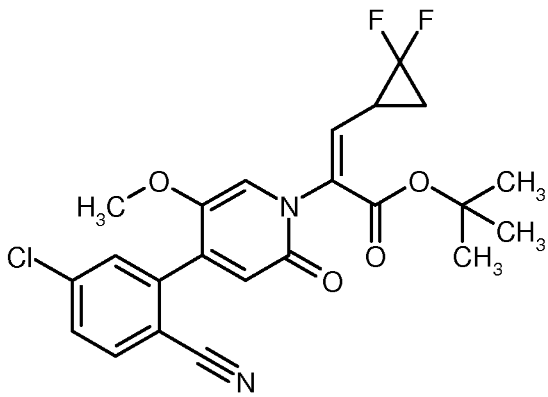

Zu einer Lösung von 500 mg (1.33 mmol) [4-(5-Chlor-2-cyanophenyl)-5-methoxy-2-oxopyridin-1(2*H*)-yl]essigsäure-*tert.*-butylester in 10 ml THF wurden bei -78°C 1.87 ml (1.87 mmol) Bis-(trimethylsilyl)-lithiumamid (1M in THF) getropft und für 10 min nachgerührt. Anschließend wurden 488 mg (4.60 mmol) 2,2-Difluorcyclopropancarbaldehyd zugegeben und nach weiteren 10 min auf -20°C erwärmt. Nach 3 h bei -20°C wurde die Reaktion durch Zugabe von 30 ml gesättigter, wässriger Ammoniumchlorid-Lösung beendet und die Reaktionsmischung dreimal mit 20 ml Essigsäureethylester extrahiert. Die vereinigten, organischen Phasen wurden mit gesättigter, wässriger Natriumchlorid-Lösung gewaschen, über Magnesiumsulfat getrocknet, filtriert und das Lösungsmittel unter reduziertem Druck entfernt. Der Rückstand wurde in wenig Dichlormethan aufgenommen und mittels Flash-Chromatographie (24 g Silica Kartusche, 35 ml/min, Cyclohexan/Essigsäureethylester-Gradient) gereinigt. Ausbeute: 240 mg (78% Reinheit, 30% d. Th.)
LC/MS [Methode 1]: $R_t$ = 1.12 min; MS (ESIpos): m/z = 463 $(M+H)^+$.

**Beispiel 43.1C**

2-[4-(5-Chlor-2-cyanophenyl)-5-methoxy-2-oxopyridm-1(2*H*)-yl]-3-[2,2-difluorcyclopropyl]-propansäure-*tert.*-butylester (Gemisch zweier racemischer Diastereomere)

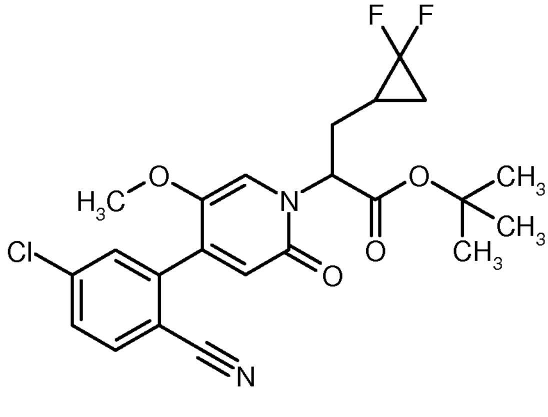

240 mg (78% Reinheit, 404 μmol) 2-[4-(5-Chlor-2-cyanophenyl)-5-methoxy-2-oxopyridin-1(2*H*)-yl]-3-(2,2-difluorcyclopropyl)prop-2-ensäure-*tert*.-butylester (Diastereomerengemisch) wurden bei RT mit 30 ml einer "Hot Stryker's"-Reagenzlösung [B. A. Baker et al. Org. Lett. 2008, 10, 289-292] versetzt. Das Reaktionsgemisch wurde 2 h bei RT gerührt und anschließend mit 20 ml gesättigter, wässriger Ammoniumchlorid-Lösung versetzt. Die Phasen wurden getrennt und die wässrige Phase dreimal mit 25 ml Essigsäureethylester extrahiert. Die vereinigten, organischen Phasen wurden über Magnesiumsulfat getrocknet, filtriert und das Lösungsmittel unter reduziertem Druck entfernt. Der Rückstand wurde mittels Flash-Chromatographie (40 g Silica Kartusche, 40 ml/min, Cyclohexan/Essigsäureethylester-Gradient) aufgereinigt. Ausbeute: 216 mg (quant.)
LC/MS [Methode 1]: $R_t$ = 1.14 min; MS (ESIpos): m/z = 465 $(M+H)^+$.

**Beispiel 43.1D**

2-[4-(5-Chlor-2-cyanophenyl)-5-methoxy-2-oxopyridin-1(2*H*)-yl]-3-[2,2-difluorcyclopropyl]-propansäure (Gemisch zweier racemischer Diastereomere)

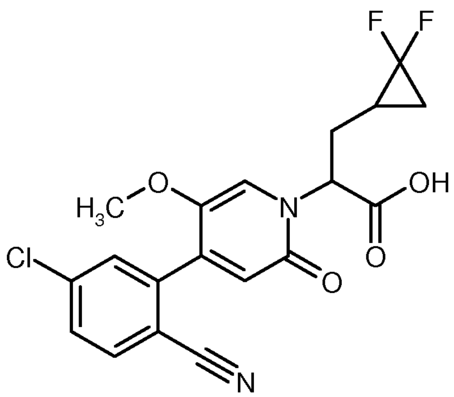

Gemäß allgemeiner Methode 6A wurden 216 mg (465 μmol) 2-[4-(5-Chlor-2-cyanophenyl)-5-methoxy-2-oxopyridin-1(2*H*)-yl] -3- [2,2-difluorcyclopropyl]-propansäure-*tert*.-butylester (Gemisch zweier racemischer Diastereomere) in 1 ml Dichlormethan mit 537 μl (6.97 mmol) TFA umgesetzt. Das Rohprodukt wurde mittels präparativer HPLC [Säule: Chromatorex C18, 10 μm, 125x30 mm, Eluent: Acetonitril/0.1% Ameisensäure-Gradient (0 bis 3 min 10% Acetonitril, bis 35 min 90% Acetonitril und weitere 3 min 90% Acetonitril)] aufgereinigt. Ausbeute: 88 mg (44% d. Th.)
LC/MS [Methode 1]: $R_t$ = 0.86/0.88 min; MS (ESIpos): m/z = 409 $(M+H)^+$.

**Beispiel 43.1E**

4-({2-[4-(5-Chlor-2-cyanophenyl)-5-methoxy-2-oxopyridin-1(2*H*)-yl]-3- [2,2-difluorcyclopropyl] - propanoyl}amino)benzoesäure-*tert*.-butylester (Gemisch zweier racemischer Diastereomere)

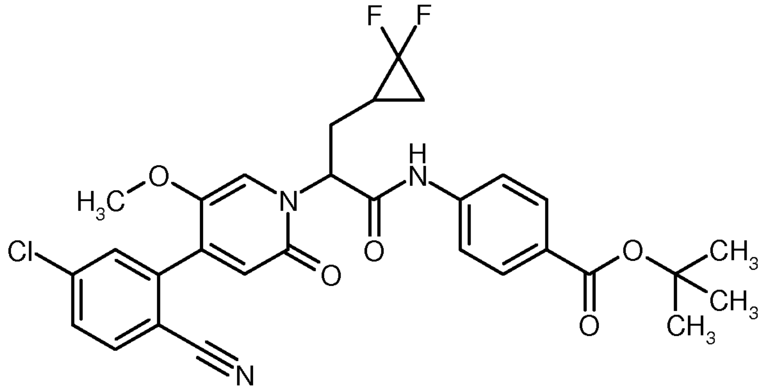

Gemäß allgemeiner Methode 5B wurden 88.0 mg (215 μmol) 2-[4-(5-Chlor-2-cyanophenyl)-5-methoxy-2-oxopyridin-1(2*H*)-yl]-3-[2,2-difluorcyclopropyl]propansäure (Gemisch zweier racemischer Diastereomere), 41.6 mg (215 μmol) 4-Aminobenzoesäure-*tert*.-butylester, 30.6 mg (215 μmol) Oxima und 34.0 μl (215 μmol) DIC in 2.1ml Dimethylformamid zur Reaktion gebracht. Ausbeute: 101 mg (66% d. Th.)
LC/MS [Methode 1]: $R_t$ = 1.25 min; MS (ESIpos): m/z = 584 $(M+H)^+$
$^1$H-NMR (400 MHz, DMSO-$d_6$): δ [ppm] = 10.8/10.7 (2x s, 1H), 8.00 (d, 1H), 7.90-7.85 (m, 2H), 7.77-7.71 (m, 4H), 7.53 (s, 1H), 6.55 (s, 1H), 5.80-5.69 (m, 1H), 3.70/3.69 (2x s, 3H), 2.63-2.38 (m, 1H), 2.34-2.07 (2x m, 1H), 1.71-1.46 (m, 2H), 1.54 (s, 9H), 1.35-1.04 (2x m, 1H).

**Beispiel 44.1A**

1-Methylcyclopropancarbaldehyd

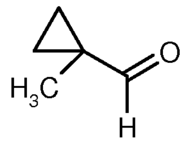

608 μl (6.97 mmol) Oxalylchlorid wurden mit 4Å Molsieb in 5 ml Dichlormethan vorgelegt und auf -78°C abgekühlt. Bei -78°C wurden 515 μl (7.26 mmol) DMSO zugetropft und 5 min nachgerührt. Anschließend wurde eine Lösung von 500 mg (5.81 mmol) (1-Methylcyclopropyl)methanol in 5 ml Dichlormethan hinzugefügt und 30 min bei -78°C gerührt. Nach Zugabe von 2.43 ml (17.4 ml) Triethylamin wurde 10 min bei RT nachgerührt und die Reaktionslösung mit 30 ml Wasser und 30 ml Dichlormethan verdünnt. Die Phasen wurden getrennt und die wässrige Phase zweimal mit 50 ml Dichlormethan extrahiert. Die vereinigten, organischen Phasen wurden mit gesättigter, wässriger Natriumchlorid-Lösung gewaschen, über Magnesiumsulfat getrocknet, filtriert und das Lösungsmittel unter reduziertem Druck entfernt. Das Rohprodukt wurde ohne weitere Reinigung im nächsten Schritt eingesetzt.

**Beispiel 44.1B**

2-[4-(5-Chlor-2-cyanophenyl)-5-methoxy-2-oxopyridin-1(2*H*)-yl]-3-(1-methylcyclopropyl)prop-2-ensäure-*tert*. -butylester (Isomerengemisch)

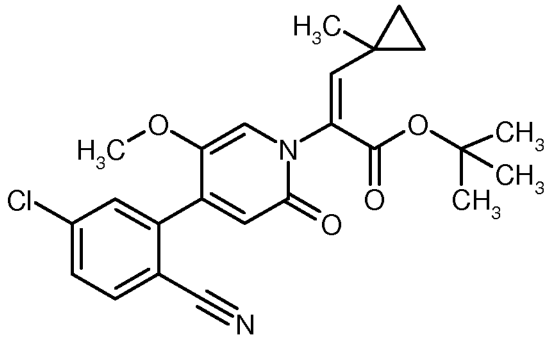

Zu einer Lösung von 500 mg (1.33 mmol) [4-(5-Chlor-2-cyanophenyl)-5-methoxy-2-oxopyridin-1(2*H*)-yl]essigsäure-*tert*.-butylester in 10 ml THF wurde bei -78°C 1.87 ml (1.87 mmol) Bis-(trimethylsilyl)-lithiumamid (1M in THF) getropft und für 10 min nachgerührt. Anschließend wurden 488 mg (5.80 mmol) 1-Methylcyclopropancarbaldehyd zugegeben und nach weiteren 10 min auf -20°C erwärmt. Nach 3 h bei -20°C wurde die Reaktion durch Zugabe von 30 ml gesättigter, wässriger Ammoniumchlorid-Lösung beendet und die Reaktionsmischung dreimal mit 20 ml Essigsäureethylester extrahiert. Die vereinigten, organischen Phasen wurden mit gesättigter, wässriger Natriumchlorid-Lösung gewaschen, über Magnesiumsulfat getrocknet, filtriert und das Lösungsmittel unter reduziertem Druck entfernt. Der Rückstand wurde in wenig Dichlormethan aufgenommen und mittels Flash-Chromatographie (24 g Silica Kartusche, 35 ml/min, Cyclohexan/Essigsäureethylester-Gradient) gereinigt. Ausbeute: 257 mg (44% d. Th.)

LC/MS [Methode 1]: $R_t$ = 1.15 min; MS (ESIpos): m/z = 441 $(M+H)^+$.

**Beispiel 44.1C**

2-[4-(5-Chlor-2-cyanophenyl)-5-methoxy-2-oxopyridin-1(2*H*)-yl]-3-(1-methylcyclopropyl)propansäure-*tert*.-butylester (Racemat)

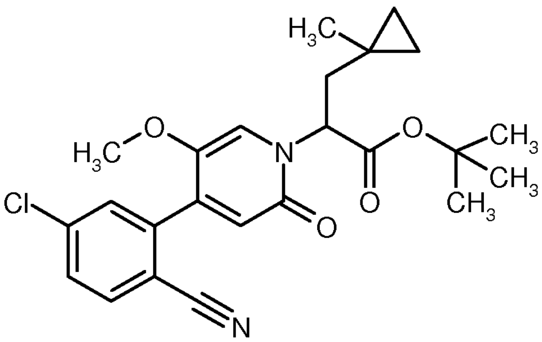

257 mg (583 μmol) 2-[4-(5-Chlor-2-cyanophenyl)-5-methoxy-2-oxopyridin-1(2*H*)-yl]-3-(1-methylcyclopropyl)prop-2-ensäure-*tert.*-butylester (Isomerengemisch) wurden bei RT mit 30 ml einer "Hot Stryker's"-Reagenzlösung [B. A. Baker et al. Org. Lett. 2008, 10, 289-292] versetzt. Das Reaktionsgemisch wurde 2 h bei RT gerührt und anschließend mit 20 ml gesättigter, wässriger Ammoniumchlorid-Lösung versetzt. Die Phasen wurden getrennt und die wässrige Phase dreimal mit 25 ml Essigsäureethylester extrahiert. Die vereinigten, organischen Phasen wurden über Magnesiumsulfat getrocknet, filtriert und das Lösungsmittel unter reduziertem Druck entfernt. Der Rückstand wurde mittels Flash-Chromatographie (40 g Silica Kartusche, 35 ml/min, Cyclohexan/Essigsäureethylester-Gradient) aufgereinigt. Ausbeute: 247 mg (96% d. Th.)
LC/MS [Methode 1]: $R_t$ = 1.21 min; MS (ESIpos): m/z = 443 $(M+H)^+$.

**Beispiel 44.1D**

2-[4-(5-Chlor-2-cyanophenyl)-5-methoxy-2-oxopyridin-1(2*H*)-yl]-3-(1-methylcyclopropyl)propansäure (Racemat)

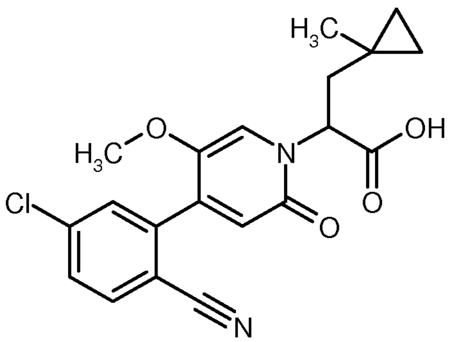

Gemäß allgemeiner Methode 6A wurden 247 mg (558 μmol) 2-[4-(5-Chlor-2-cyanophenyl)-5-methoxy-2-oxopyridin-1(2*H*)-yl]-3-(1-methylcyclopropyl)propansäure-*tert*. -butylester (Racemat) in 1 ml Dichlormethan mit 859 μl (11.2 mmol) TFA umgesetzt. Das Rohprodukt wurde mittels präparativer HPLC [Säule: Chromatorex C18, 10 μm, 125x30 mm, Eluent: Acetonitril/0.1% Ameisensäure-Gradient (0 bis 3 min 10% Acetonitril, bis 35 min 90% Acetonitril und weitere 3 min 90% Acetonitril)] aufgereinigt. Ausbeute: 95 mg (43% d. Th.)
LC/MS [Methode 2]: $R_t$ = 2.70 min; MS (ESIpos): m/z = 387 $(M+H)^+$.

**Beispiel 44.1E**

4-({2-[4-(5-Chlor-2-cyanophenyl)-5-methoxy-2-oxopyridin-1(2*H*)-yl]-3-(1-methylcyclopropyl)propanoyl}amino)benzoesäure-*tert*.-butylester (Racemat)

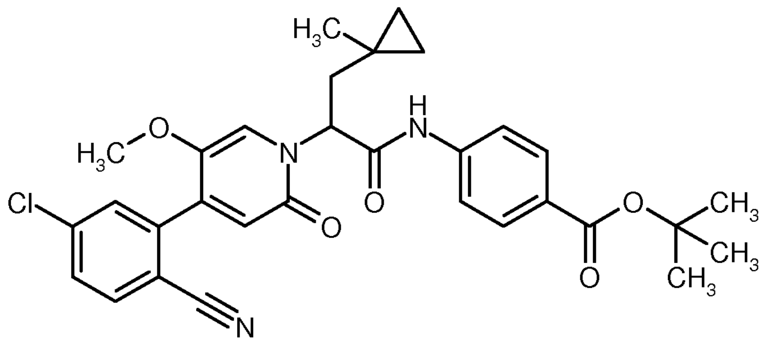

Gemäß allgemeiner Methode 5B wurden 95.0 mg (246 μmol) 2-[4-(5-Chlor-2-cyanophenyl)-5-methoxy-2-oxopyridin-1(2*H*)-yl]-3-(1-methylcyclopropyl)propansäure (Racemat), 47.5 mg (246 μmol) 4-Aminobenzoesäure-*tert*.-butylester, 34.9 mg (246 μmol) Oxima und 38.3 μl (246 μmol) DIC in 2.5 ml Dimethylformamid zur Reaktion gebracht. Ausbeute: 101 mg (66% d. Th.)
LC/MS [Methode 1]: $R_t$ = 1.32 min; MS (ESIpos): m/z = 562 $(M+H)^+$
$^1$H-NMR (400 MHz, DMSO-$d_6$): δ [ppm] = 10.8 (s, 1H), 8.00 (d, 1H), 7.88 (d, 2H), 7.77 (d, 2H), 7.75-7.72 (m, 2H), 7.54 (s, 1H), 6.53 (s, 1H), 5.97 (dd, 1H), 3.68 (s, 3H), 2.20 (dd, 1H), 2.05 (dd, 1H), 1.54 (s, 9H), 1.07 (s, 3H), 0.35-0.25 (m, 2H), 0.21-0.12 (m, 2H).

**Beispiel 45.1A**

3-Cyclobutyl-2-hydroxypropansäureethylester (Racemat)

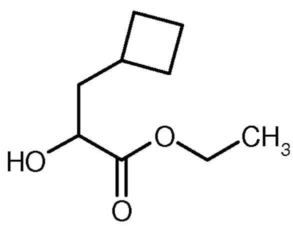

359 mg (14.8 mmol, 1.1 eq.) Magnesiumspäne wurden mit Diethylether überschichtet und durch Zugabe eines Krümels Iod 3-4 min lang angeätzt. Dieses Gemisch wurde unter Argon bei RT mit 5 ml einer Lösung von 2.0 g (13.4 mmol) (Brommethyl)cyclobutan in 30 ml Diethylether unter Rühren versetzt, 5 min lang gerührt (bis zum Anspringen der Reaktion) und innerhalb von weiteren 10 min tropfenweise mit der restlichen (Brommethyl)cyclobutan/Diethylether-Lösung versetzt. Das Reaktionsgemisch wurde 1 h unter Rückfluß gerührt, unter Argonstrom abgekühlt und unter Eiswasser-Kühlung tropfenweise zu einer Lösung von 2.4 ml (12.1 mmol, 0.9 eq.) Glyoxylsäureethylester (50%ig in Toluol) gegeben. Das Reaktionsgemisch wurde 1 h bei RT gerührt, mit 20 ml einer Kaliumcitrat/Zitronensäurelösung (pH 5) vorsichtig auf pH 7 gequencht und anschließend mit wässriger Salzsäure (1N) auf pH 4-5 gebracht. Nach Phasentrennung wurde die wässrige Phase mit Diethylether extrahiert. Die vereinigten, organischen Phasen wurden getrocknet (Natriumsulfat), filtriert und im Vakuum eingeengt. Der Rückstand wurde mittels Flash-Chromatograhpie (Kieselgel-50, Eluent: Cyclohexan-Essigsäureethylester 20-33%) aufgereinigt. Ausbeute: 110 mg (94% Reinheit, 5% d. Th.)
LC/MS [Methode 8]: $R_t$ = 3.37 min; MS (ESIpos): m/z = 172 $(M)^+$.

**Beispiel 45.1B**

3-Cyclobutyl-2-{[(trifluormethyl)sulfonyl]oxy}propansäureethylester (Racemat)

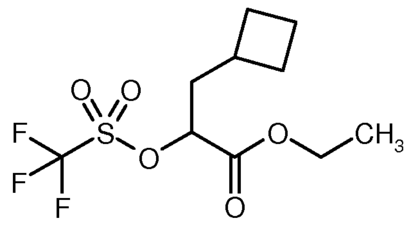

Nach der allgemeinen Methode 8A wurden 110 mg (94% Reinheit, 0.60 mmol) 3-Cyclobutyl-2-hydroxypropansäureethylester (Racemat) mit 142 µl (0.84 mmol, 1.4 eq.) Trifluormethansulfonsäureanhydrid in Gegenwart von 105 µl (0.90 mmol, 1.5 eq.) 2,6-Dimethylpyridin umgesetzt. Das Rohprodukt wurde ohne weitere Reinigung in der nächsten Stufe umgesetzt.

**Beispiel 45.1C**

2-[4-(5-Chlor-2-cyanophenyl)-5-methoxy-2-oxopyridin-1(2*H*)-yl]-3-cyclobutylpropansäure-ethylester (Racemat)

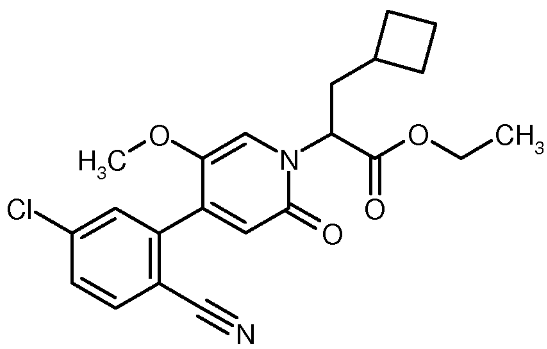

Nach der allgemeinen Methode 4E wurden 122 mg (87% Reinheit, 0.41 mmol) 4-Chlor-2-(5-methoxy-2-oxo-1,2-dihydropyridin-4-yl)benzonitril in Gegenwart von 1.3 eq. Natriumhydrid mit 161 mg (0.53 mmol, 1.3 eq.) 3-Cyclobutyl-2-{[(trifluormethyl)sulfonyl]oxy}propansäureethylester (Racemat) bei RT umgesetzt. Das Rohprodukt wurde mittels Flash-Chromatographie (KP-SIL, Cyclohexan/Essigsäureethylester 15-33%) aufgereinigt. Ausbeute: 140 mg (82% d. Th.)
LC/MS [Methode 1]: $R_t$ = 1.15 min; MS (ESIpos): m/z = 415 $(M+H)^+$
$^1H$-NMR (400 MHz, DMSO-$d_6$): δ [ppm] = 7.99 (d, 1H), 7.78-7.69 (m, 2H), 7.42 (s, 1H), 6.48 (s, 1H), 5.12 (dd, 1H), 4.21-4.07 (m, 2H), 3.64 (s, 3H), 2.38-2.24 (m, 1H), 2.23-2.11 (m, 2H), 2.05-1.93 (m, 1H), 1.89-1.61 (m, 4H), 1.60-1.47 (m, 1H), 1.18 (t, 3H).

**Beispiel 45.1D**

2-[4-(5-Chlor-2-cyanophenyl)-5-methoxy-2-oxopyridin-1(2*H*)-yl]-3-cyclobutylpropansäure (Racemat)

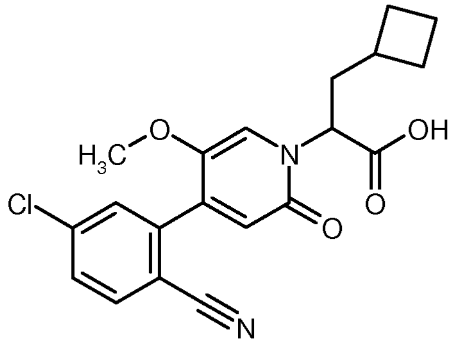

Nach der allgemeinen Methode 6B wurden 138 mg (0.33 mmol) 2-[4-(5-Chlor-2-cyanophenyl)-5-methoxy-2-oxopyridin-1(2*H*)-yl] -3-cyclobutylpropansäureethylester (Racemat) mit Lithiumhydroxid verseift. Ausbeute: 104 mg (82% d. Th.)
LC/MS [Methode 1]: $R_t$ = 0.95 min; MS (ESIpos): m/z = 387 $(M+H)^+$.

**Beispiel 45.1E**

4-({2-[4-(5-Chlor-2-cyanophenyl)-5-methoxy-2-oxopyridin-1(2*H*)-yl]-3-cyclobutylpropanoyl}-amino)benzoesäure-*tert*. -butylester (Racemat)

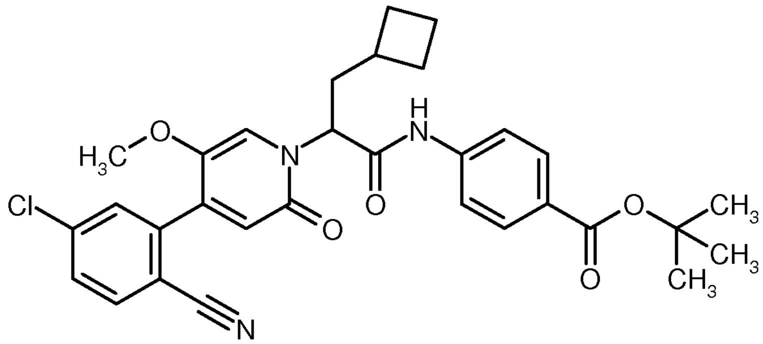

Nach der allgemeinen Methode 5A wurden 104 mg (0.27 mmol) 2-[4-(5-Chlor-2-cyanophenyl)-5-methoxy-2-oxopyridin-1(2*H*)-yl]-3-cyclobutylpropansäure (Racemat) mit 57 mg (0.30 mmol, 1.1eq.) 4-Aminobenzoesäure-*tert*.-butylester umgesetzt. Ausbeute: 66 mg (86% Reinheit, 38% d. Th.)
LC/MS [Methode 1]: $R_t$ = 1.38 min; MS (ESIpos): m/z = 562 $(M+H)^+$.

**Beispiel 46.1A**

[4-(5-Chlor-2-cyanophenyl)-5-methoxy-2-oxopyridin-1(2*H*)-yl](ethoxy)essigsäureethylester (Racemat)

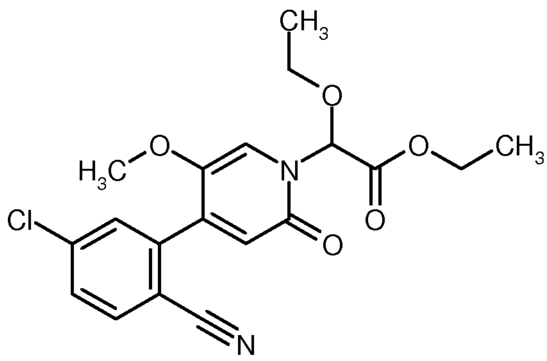

Eine Suspension von 53 mg (1.32 mmol, 1.2 eq.) Natriumhydrid (60%ig in Mineralöl) in 2.1 ml Dimethylformamid wurde unter Argon bei 0°C im Abstand von 10 min mit zwei Portionen von insgesamt 350 mg (82% Reinheit, 1.10 mmol) 4-Chlor-2-(5-methoxy-2-oxo-1,2-dihydropyridin-4-yl)benzonitril versetzt. Das Reaktionsgemisch wurde 60 min bei RT gerührt, wieder auf 0°C gekühlt, mit 245 mg (90% Reinheit, 1.32 mmol, 1.2 eq.) 2-Chlor-2-ethoxyessigsäureethylester versetzt und 2 h bei RT gerührt. Dieser Ansatz wurde mit einem analogen Testansatz mit 50 mg (82% Reinheit, 0.16 mmol) 4-Chlor-2-(5-methoxy-2-oxo-1,2-dihydropyridin-4-yl)benzonitril vereinigt. Nach Zugabe von 20 ml Wasser und Phasentrennung wurde die wässrige Phase zweimal mit Essigsäureethylester extrahiert. Die vereinigten, organischen Phasen wurden getrocknet (Natriumsulfat), filtriert und im Vakuum eingeengt. Der Rückstand wurde mittels Flash-Chromatographie (IR-50Si, Petrolether/Essigsäureethylester 15-50%) aufgereinigt. Ausbeute: 277 mg (55% d. Th. bezogen auf insgesamt 1.26 mmol eingesetztem 4-Chlor-2-(5-methoxy-2-oxo-1,2-dihydropyridin-4-yl)benzonitril)
LC/MS [Methode 1]: $R_t$ = 1.01 min; MS (ESIpos): m/z = 391 $(M+H)^+$.

**Beispiel 46.1B**

[4-(5-Chlor-2-cyanophenyl)-5-methoxy-2-oxopyridin-1(2*H*)-yl](ethoxy)essigsäure (Racemat)

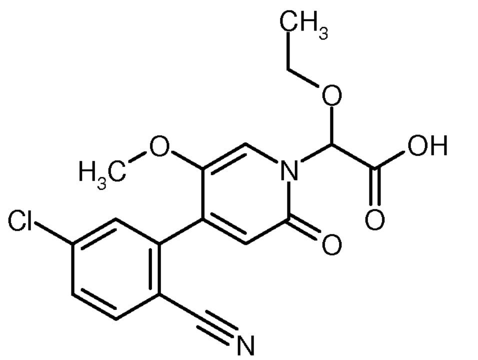

Nach der allgemeinen Methode 6B wurden 277 mg (0.69 mmol) [4-(5-Chlor-2-cyanophenyl)-5-methoxy-2-oxopyridin-1(2*H*)-yl](ethoxy)essigsäureethylester (Racemat) mit Lithiumhydroxid verseift. Ausbeute: 180 mg (71% d. Th.)
LC/MS [Methode 1]: $R_t$ = 0.80 min; MS (ESIpos): m/z = 363 $(M+H)^+$.

**Beispiel 46.1C**

4-({[4-(5-Chlor-2-cyanophenyl)-5-methoxy-2-oxopyridin-1(2*H*)-yl](ethoxy)acetyl}amino)-benzoesäure-*tert*.-butylester (Racemat)

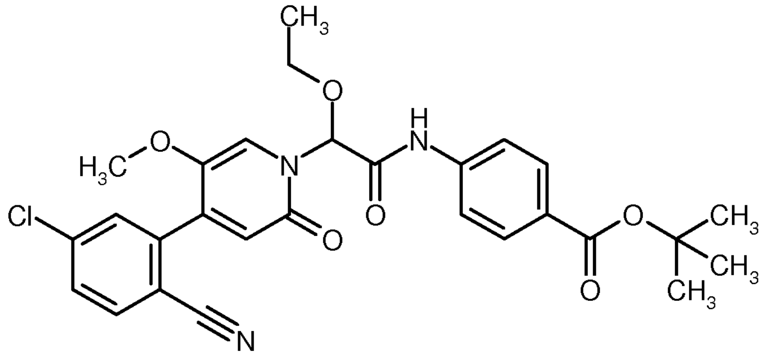

Nach der allgemeinen Methode 5A wurden 180 mg (0.50 mmol) [4-(5-Chlor-2-cyanophenyl)-5-methoxy-2-oxopyridin-1(2*H*)-yl](ethoxy)essigsäure (Racemat) mit 105 mg (0.55 mmol, 1.1 eq.) 4-Aminobenzoesäure-*tert*.-butylester umgesetzt. Ausbeute: 265 mg (quant.)
LC/MS [Methode 1]: $R_t$ = 1.22 min; MS (ESIpos): m/z = 538 $(M+H)^+$
$^1$H-NMR (400 MHz, DMSO-$d_6$): δ [ppm] = 10.74 (s, 1H), 8.01 (d, 1H), 7.90 (d, 2H), 7.80 (d, 2H), 7.79-7.71 (m, 2H), 7.35 (s, 1H), 6.57 (s, 1H), 6.40 (s, 1H), 3.82-3.72 (m, 1H), 3.72-3.60 (m, 1H), 3.67 (s, 3H), 1.54 (s, 9H), 1.27 (t, 3H).

**Beispiel 47.1A**

[4-(5-Chlor-2-cyanophenyl)-5-methoxy-2-oxopyridin-1(2*H*)-yl]essigsäure

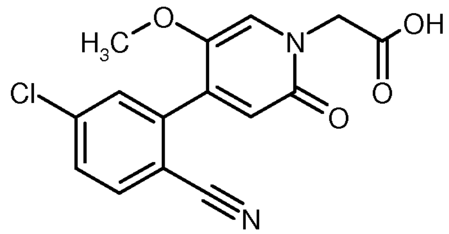

Gemäß allgemeiner Methode 6A wurden 187 mg (500 μmol) [4-(5-Chlor-2-cyanophenyl)-5-methoxy-2-oxopyridin-1(2*H*)-yl]essigsäure-*tert*.-butylester mit 770 μl (10.0 mmol) TFA umgesetzt. Ausbeute: 159 mg (93% d. Th.)
LC/MS [Methode 1]: $R_t$ = 0.72 min; MS (ESIneg): m/z = 317 $(M-H)^-$
$^1$H-NMR (400 MHz, DMSO-$d_6$): δ [ppm] = 13.1 (s, 1H), 8.00 (d, 1H), 7.74 (dd, 1H), 7.72 (s, 1H), 7.58 (s, 1H), 6.51 (s, 1H), 4.64 (s, 2H), 3.62 (s, 3H).

**Beispiel 47.1B**

4-({[4-(5-Chlor-2-cyanophenyl)-5-methoxy-2-oxopyridin-1(2*H*)-yl]acetyl}amino)benzoesäure-*tert*.-butylester

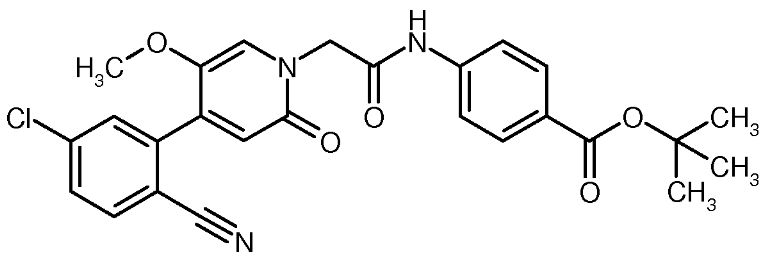

Gemäß allgemeiner Methode 5A wurden 159 mg (499 μmol) [4-(5-Chlor-2-cyanophenyl)-5-methoxy-2-oxopyridin-1(2/7)-yl]essigsäure, 116 mg (599 μmol) 4-Aminobenzoesäure-*tert*.-butylester, 228 mg (599 μmol) HATU und 261 μl (1.50 mmol) *N,N*-Diisopropylethylamin in 8 ml Dimethylformamid umgesetzt. Das Lösungsmittel wurde entfernt und der Rückstand mittels präparativer HPLC [Säule: Chromatorex C18, 10 μm, 125 mm x 30 mm, Eluent: Acetonitril/0.1% Ameisensäure-Gradient (0 bis 3 min 10% Acetonitril, bis 35 min 90% Acetonitril und weitere 3 min 90% Acetonitril)] aufgereinigt. Ausbeute: 54.5 mg (22% d. Th.)
LC/MS [Methode 1]: $R_t$ = 1.09 min; MS (ESIpos): m/z = 494 $(M+H)^+$ $^1$H-NMR (400 MHz, DMSO-$d_6$): δ [ppm] = 10.7 (s, 1H), 8.00 (d, 1H), 7.88 (d, 2H), 7.75-7.71 (m, 4H), 7.60 (s, 1H), 6.52 (s, 1H), 4.81 (s, 2H), 3.64 (s, 3H), 1.54 (s, 9H).

**Beispiel 48.1A**

4-{[*tert*.-Butyl(dimethyl)silyl]oxy}cyclohexancarbonsäuremethylester (*trans*/*cis*-Gemisch)

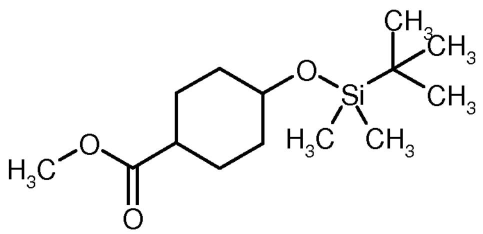

5.0 g (32 mmol) 4-Hydroxycyclohexancarbonsäuremethylester wurden in 100ml Dimethylformamid vorgelegt. Anschließend wurden 6.7g (44 mmol) *tert.*-Butyldimethylsilylchlorid und 4.1g (60 mmol) Imidazol hinzugegeben und 14h bei RT nachgerührt. Das Lösungsmittel wurde unter reduziertem Druck entfernt und der Rückstand mit 100 ml Methyl-tert.-butylether und 100 ml gesättigter, wässriger Natriumhydrogencarbonat-Lösung aufgenommen. Die Phasen wurden getrennt und die organische Phase über Magnesiumsulfat getrocknet, filtriert und das Lösungsmittel unter reduziertem Druck entfernt. Ausbeute: 8.1g (93% d. Th.)
GC/MS [Methode 9]: $R_t$ = 4.79 min; MS: m/z = 272 $(M)^+$.

**Beispiel 48.1B**

(4-{[tert.-Butyl(dimethyl)silyl]oxy}cyclohexyl)methanol (*trans*/*cis-Gemisch*)

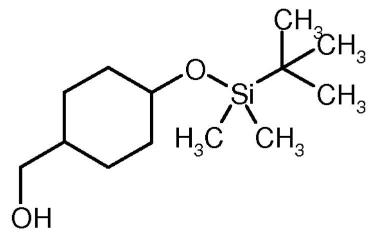

Zu einer Lösung von 50 ml (100 mmol) Lithiumaluminiumhydrid (2M in THF) wurden bei 0°C 8.1g (29.7 mmol) 4-{[*tert*.-Butyl(di-methyl)silyl]oxy}cyclohexancarbonsäuremethylester *(trans*/*cis-Gemisch)* als Lösung in 50 ml THF getropft. Es wurde 1 h bei 0°C und 2 h bei RT nachgerührt. Anschließend wurde die Reaktion nacheinander mit 3.8 ml Wasser, 3.8 ml wässriger Natriumhydroxid-Lösung (15%) und 11.4 ml Wasser versetzt und der Niederschlag abfiltriert. Die organische Phase wurde mit 50 ml gesättigter, wässriger Natriumchlorid-Lösung gewaschen, über Magnesiumsulfat getrocknet, filtriert und das Lösungsmittel unter reduziertem Druck entfernt. Ausbeute: 7.00 g (92% d. Th.)
GC/MS [Methode 9]: $R_t$ = 4.74 min; MS: m/z = 244 $(M)^+$.

**Beispiel 48.1C**

(4-{[*tert*.-Butyl(dimethyl)silyl]oxy}cyclohexyl)methyl-trifluormethansulfonat(*trans*/*cis*-Gemisch)

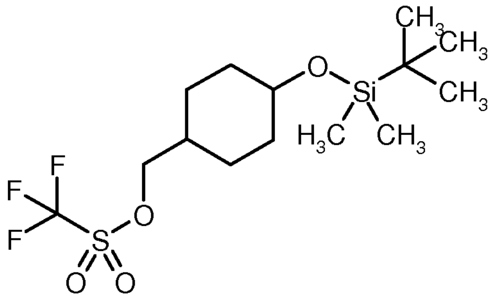

Gemäß allgemeiner Methode 8A wurden 1.00 g (4.09 mmol) (4-{[*tert.*-Butyl(dimethyl)silyl]oxy}cyclohexyl)methanol (*trans*/*cis*-Gemisch) in 25 ml Dichlormethan mit 715 μl (6.14 mmol) Lutidin und 1.04 ml (6.14 mmol) Trifluormethansulfonsäureanhydrid umgesetzt. Das Rohprodukt wurde ohne weitere Aufreinigung im nächsten Schritt eingesetzt. Ausbeute: 1.47 g (91% d. Th.)

**Beispiel 48.1D**

3-(4-{[*tert*.-Butyl(dimethyl)silyl]oxy}cyclohexyl)-2-[4-(5-chlor-2-cyanophenyl)-5-methoxy-2-oxopyridin-1(2*H*)-yl]propansäure-*tert*.-butylester(Gemisch zweier racemischer Diastereomere)

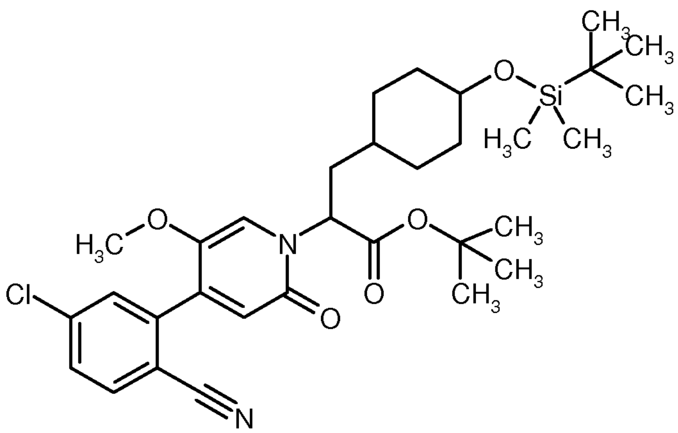

Gemäß allgemeiner Methode 7B wurden 500 mg (1.26 mmol) [4-(5-Chlor-2-cyanophenyl)-5-methoxy-2-oxopyridin-1(2*H*)-yl]essigsäure-*tert*.-butylester, 712 mg (1.89 mmol) (4-{[*tert.*-Butyl(dimethyl)silyl]oxy}cyclohexyl)methyltrifluormethansulfonat (*trans*/*cis-Gemisch*) und 1.39 ml (1.39 mmol) Bis-(trimethylsilyl)-lithiumamid (1M in THF) in 10 ml THF zur Reaktion gebracht. Nach säulenchromatographischer Reinigung (120 g Silica Kartusche, Fluss: 85 ml/min, Cyclohexan/Essigsäureethylester-Gradient) wurde die Titelverbindung erhalten. Ausbeute: 479 mg (63% d. Th.)
LC/MS [Methode 1]: $R_t$ = 1.56/1.59 min; MS (ESIpos): m/z = 601 $(M+H)^+$.

**Beispiel 48.1E**

3-(4-{[*tert*.-4-(5-chlor-2-cyanophenyl)-5-methoxy-2-oxopyridin-1(2*H*)-yl]propansäure (Gemisch zweier racemischer Diastereomere)

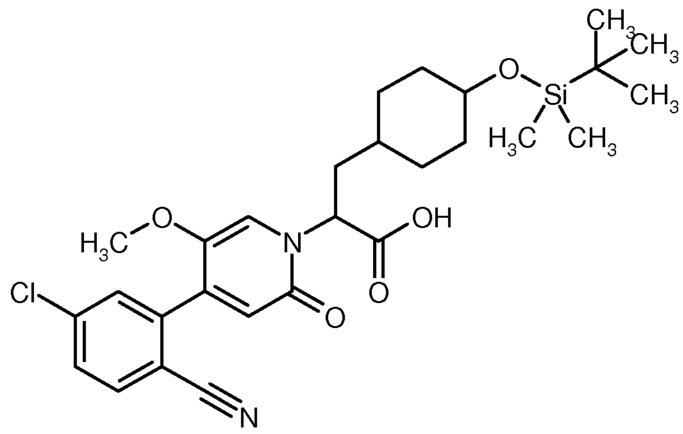

Gemäß allgemeiner Methode 6B wurden 479 mg (797 µmol) *3*-(*4*-{[*tert.*-Butyl(dimethyl)silyl]oxy}cyclohexyl)-2-[4-(5-chlor-2-cyanophenyl)-5-methoxy-2-oxopyridin-1(2*H*)-yl]propansäure-*tert.*-butylester (Gemisch zweier racemischer Diastereomere) mit 4ml wässriger Lithiumhydroxid-Lösung (1N) umgesetzt und die Titelverbindung erhalten. Ausbeute: 400 mg (80% d. Th.)
LC/MS [Methode 1]: $R_t$ = 1.36/1.39 min; MS (ESIpos): m/z = 545 $(M+H)^+$.

## Beispiel 48.1F

4-({3-(4-{[*tert.*-Butyl(dimethyl)silyl]oxylcyclohexyl)-2-[4-(5-chlor-2-cyanophenyl)-5-methoxy-2-oxopyridin-1(2*H*)-yl]propanoyl}amino)benzoesäure-*tert.*-butylester (Gemisch zweier racemischer Diastereomere)

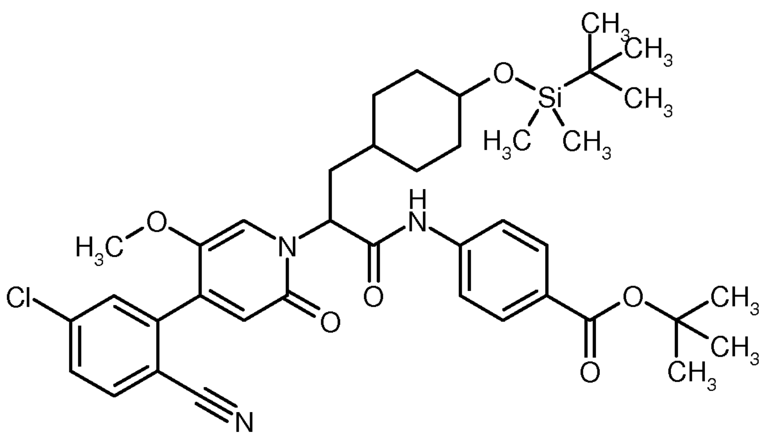

Gemäß allgemeiner Methode 5B wurden 400mg (734 µmol) *3*-(*4*-{[*tert.*-Butyl(dimethyl)silyl]oxy}cyclohexyl)-2-[4-(5-chlor-2-cyanophenyl)-5-methoxy-2-oxopyridin-1(2*H*)-yl]propansäure (Gemisch zweier racemischer Diastereomere), 142 mg (734 µmol) 4-Aminobenzoesäure-*tert.*-butylester, 104 mg (734 µmol) Oxima und 114 µl (734 µmol) DIC in 7.3 ml Dimethylformamid zur Reaktion gebracht. Das Rohprodukt wurde mittels Flash-Chromatographie (40 g Kartusche, 40 ml/min, Cyclohexan/Essigsäureethylester-Gradient) gereinigt. Ausbeute: 341 mg (64% d. Th.)
LC/MS [Methode 1]: $R_t$ = 1.61/1.64 min; MS (ESIpos): m/z = 720 $(M+H)^+$.

## Beispiel 49.1A

2-[4-(5-Chlor-2-cyanophenyl)-5-methoxy-2-oxopyridin-1(2*H*)-yl]-4-(trifluormethoxy)butansäure-*tert.*-butylester (Racemat)

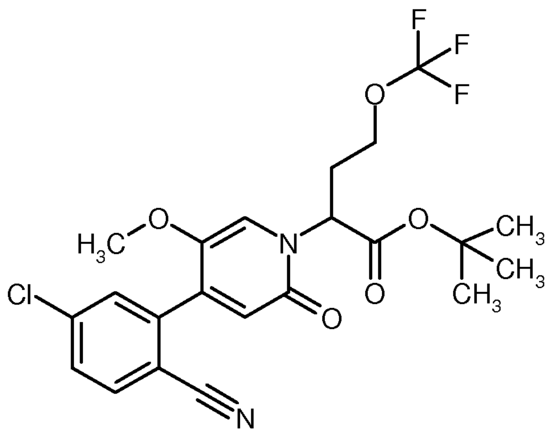

Gemäß allgemeiner Methode 7B wurden 500 mg (1.29 mmol) [4-(5-Chlor-2-cyanophenyl)-5-methoxy-2-oxopyridin-1(2*H*)-yl]essigsäure-*tert*.-butylester, 495 mg (1.89 mmol) 2-(Trifluormethoxy)ethyltrifluormethansulfonat und 1.39 ml (1.39 mmol) Bis-(trimethylsilyl)-lithiumamid (1M in THF) in 10 ml THF zur Reaktion gebracht. Nach säulenchromatographischer Reinigung (24 g Silica Kartusche, Fluss: 35 ml/min, Cyclohexan/Essigsäureethylester-Gradient) wurde die Titelverbindung erhalten. Ausbeute: 386 mg (62% d. Th.)
LC/MS [Methode 1]: $R_t$ = 1.18 min; MS (ESIpos): m/z = 487 $(M+H)^+$
$^1$H-NMR (400 MHz, DMSO-$d_6$): $\delta$ [ppm] = 7.99 (d, 1H), 7.75-7.70 (m, 2H), 7.45 (s, 1H), 6.52 (s, 1H), 5.14 (dd, 1H), 4.22-4.16 (m, 1H), 4.04-3.98 (m, 1H), 3.63 (s, 3H), 2.59-2.51 (m, 2H), 1.40 (s, 9H).

**Beispiel 49.1B**

2-[4-(5-Chlor-2-cyanophenyl)-5-methoxy-2-oxopyridin-1(2*H*)-yl]-4-(trifluormethoxy)butansäure (Racemat)

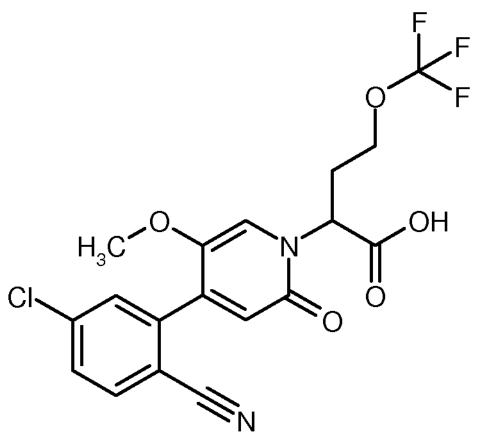

Gemäß allgemeiner Methode 6A wurde 384 mg (789 µmol) 2-[4-(5-Chlor-2-cyanophenyl)-5-methoxy-2-oxopyridin-1(2*H*)-yl]-4-(trifluormethoxy)butansäure-*tert*.-butylester (Racemat) in 7.9 ml Dichlormethan mit 2.28 ml (29.6 mmol) TFA umgesetzt. Ausbeute: 330 mg (96% d. Th.)
LC/MS [Methode 1]: $R_t$ = 0.94 min; MS (ESIpos): m/z = 431 $(M+H)^+$
$^1$H-NMR (400 MHz, DMSO-$d_6$): $\delta$ [ppm] = 13.2 (s, 1H), 7.99 (d, 1H), 7.74-7.71 (m, 2H), 7.50 (s, 1H), 6.51 (s, 1H), 5.18 (dd, 1H), 4.22-4.15 (m, 1H), 4.02-3.95 (m, 1H), 3.63 (s, 3H), 2.62-2.51 (m, 2H).

**Beispiel 49.1C**

4-({2-[4-(5-Chlor-2-cyanophenyl)-5-methoxy-2-oxopyridin-1(2*H*)-yl]-4-(trifluormethoxy)butanoyl}amino)benzoesäure-*tert*.-butylester (Racemat)

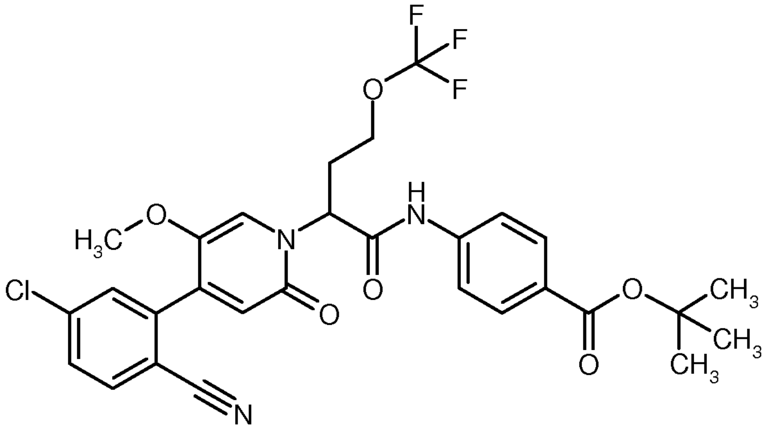

Gemäß allgemeiner Methode 5B wurden 330 mg (766 µmol) 2-[4-(5-Chlor-2-cyanophenyl)-5-methoxy-2-oxopyridin-1(2*H*)-yl]-4-(trifluormethoxy)butansäure (Racemat), 148 mg (766 µmol) 4-Aminobenzoesäure-*tert.*-butylester, 109 mg (766 µmol) Oxima und 120 µl (766 µmol) DIC in 7.7 ml Dimethylformamid zur Reaktion gebracht. Das Rohprodukt wurde mittels Flash-Chromatographie (40g Kartusche, 40 ml/min, Cyclohexan/Essigsäureethylester-Gradient) gereinigt. Ausbeute: 329 mg (64% d. Th.)
LC/MS [Methode 1]: $R_t$ = 1.27 min; MS (ESIpos): m/z = 606 $(M+H)^+$
$^1$H-NMR (400 MHz, DMSO-$d_6$): $\delta$ [ppm] = 10.8 (s, 1H), 8.00 (d, 1H), 7.88 (d, 2H), 7.77-7.71 (m, 4H), 7.51 (s, 1H), 6.56 (s, 1H), 5.81 (dd, 1H), 4.20-4.14 (m, 1H), 4.03-3.95 (m, 1H), 3.69 (s, 3H), 2.68-2.60 (m, 2H), 1.54 (s, 9H).

## Beispiel 50.1A

4-Brom-2,5-dimethoxypyridin

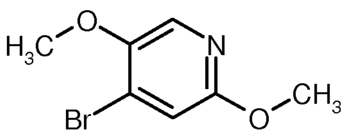

Eine Mischung aus 2.25 g (12.05 mmol) 2,5-Dimethoxypyridin-4-ylboronsäure und 4.04 g (18.08 mmol, 1.5 eq.) Kupfer(II)bromid in 48 ml Methanol/Wasser (1:1) wurde 60 min bei 100°C in der Mikrowelle bestrahlt. Nach Abkühlen wurde der Niederschlag filtriert, mit Wasser gewaschen, anschließend in 600 ml Methanol 1 h bei 65°C gerührt und filtriert. Der Rückstand wurde in Dichlormethan gelöst, diese Lösung mit verdünnter Ammoniak-Lösung gewaschen, getrocknet (Natriumsulfat), filtriert und im Vakuum eingeengt und getrocknet. Ausbeute: 1.71 g (65% d. Th.)
LC/MS [Methode 3]: $R_t$ = 2.12 min; MS (ESIpos): m/z = 218 $(M+H)^+$.

## Beispiel 50.1B

4-Brom-5-methoxypyridin-2(1*H*)-on

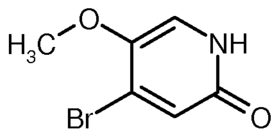

Eine Lösung von 1.94 g (8.81 mmol) 4-Brom-2,5-dimethoxypyridin in 80 ml Dimethylformamid wurde mit 2.82 g (176 mmol, 20 eq.) Pyridinium-Hydrobromid versetzt, 3 h bei 100°C gerührt und das Reaktionsgemisch im Vakuum eingeengt. Der Rückstand wurde mit 50 ml Wasser verrührt, filtriert, mit Wasser gewaschen und im Vakuum getrocknet.

Das Filtrat wurde zweimal mit Dichlormethan/Methanol (10:1) extrahiert. Die vereinigten, organischen Phasen wurden getrocknet (Natriumsulfat), filtriert und im Vakuum eingeengt und getrocknet. Ausbeute: 771 mg (43% d. Th.) und 465 mg (88% Reinheit, 23% d. Th.)
LC/MS [Methode 3]: $R_t$ = 1.38 min; MS (ESIpos): m/z = 204 $(M+H)^+$.

**Beispiel 50.1C**

2-(4-Brom-5-methoxy-2-oxopyridin-1(2*H*)-yl)propansäure (Racemat)

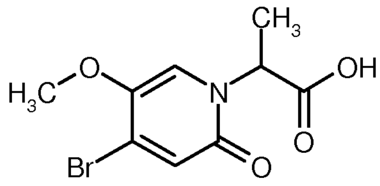

Eine Suspension von 1.76 g (10.3 mmol, 2.0 eq.) Magnesiumdi-*tert*.-butylat, 1.24g (5.15 mmol) 4-Brom-5-methoxypyridin-2(1*H*)-on und 607 mg (5.41 mmol, 1.05 eq.) Kalium-*tert*.-butylat in 30 ml Tetrahydrofuran wurde unter Argon 10 min bei RT gerührt. Das Reaktionsgemisch wurde im Eisbad gekühlt und mit 695 µl (7.72 mmol, 1.5 eq.) 2-Brompropionsäure (Racemat) versetzt. Anschließend wurde das Reaktionsgemisch zunächst 2.5 h bei RT und anschließend über Nacht bei 50°C nachgerührt, mit wässriger Salzsäure (6N) angesäuert und durch Zugabe von Essigsäureethylester/Wasser verdünnt. Der sich bildende Niederschlag wurde filtriert und im Vakuum getrocknet. Ausbeute: 205 mg (14% d. Th.)

Nach Phasentrennung des Filtrates wurde die wässrige Phase mit Essigsäureethylester extrahiert. Die vereinigten, organischen Phasen wurden getrocknet (Natriumsulfat), filtriert und im Vakuum eingeengt. Das Rohprodukt wurde anschließend erneut wie oben beschrieben mit 1.05 g (6.18 mmol) Magnesiumdi-*tert*.-butylat, 376 mg (3.35 mmol) Kalium-*tert*.-butylat und 371 µl (4.12 mmol) 2-Brompropionsäure (Racemat) in 30 ml Tetrahydrofuran umgesetzt und analog aufgearbeitet, wobei ein weiterer Niederschlag isoliert werden konnte. Ausbeute: 571 mg (39% d. Th.)
LC/MS [Methode 1]: $R_t$ = 0.57 min; MS (ESIpos): m/z = 276 $(M+H)^+$.

**Beispiel 50.1D**

4-{[2-(4-Brom-5-methoxy-2-oxopyridin-1(2*H*)-yl)propanoyl]amino}benzoesäure-*tert*.-butylester (Racemat)

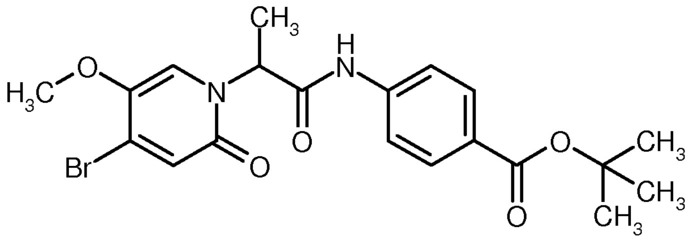

Nach der allgemeinen Methode 5A wurden 571 mg (2.01 mmol) 2-(4-Brom-5-methoxy-2-oxopyridin-1(2*H*)-yl)propansäure (Racemat) mit 426mg (2.21 mmol, 1.1 eq.) 4-Aminobenzoesäure-*tert*.-butylester umgesetzt. Ausbeute: 562 mg (61% d. Th.)
LC/MS [Methode 1]: $R_t$ = 1.10 min; MS (ESIpos): m/z = 451 $(M+H)^+$.

**Beispiel 50.1E**

4-[(2-{4-[5-Chlor-2-(trifluormethoxy)phenyl] -5-methoxy-2-oxopyridin-1(2*H*)-yl}propanoyl)-amino]benzoesäure-*tert*.-butylester (Racemat)

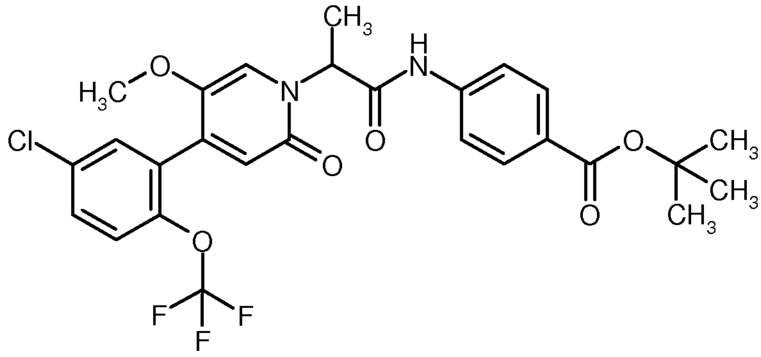

125 mg (0.28 mmol) 4-{[2-(4-Brom-5-methoxy-2-oxopyridin-1(2*H*)-yl)propanoyl]amino}-benzoesäure-*tert*.-butylester (Racemat), 80mg (0.33 mmol, 1.2 eq.) 5-Chlor-2-trifluormethoxyphenylboronsäure, 115 mg (0.83 mmol, 3.0 eq.) Kaliumcarbonat und 23 mg (0.03 mmol, 0.1 eq.) [1,1-Bis-(diphenylphosphino)-ferrocen]-palladium(II)chlorid-Dichlormethan-Monoaddukt wurden unter Argon (im ausgeheizten Kolben) in 5.0 ml Dioxan suspendiert und in einem bereits auf 110°C vorgeheiztem Ölbad über Nacht gerührt. Das Reaktionsgemisch wurde über Celite filtriert und der Rückstand mit Dioxan nachgewaschen. Die vereinigten Filtrate wurden im Vakuum eingeengt. Der Rückstand wurde mit Wasser verrührt, filtriert, mit Wasser nachgewaschen und im Vakuum getrocknet. Ausbeute: 155 mg (83% Reinheit, 82% d.Th.)
LC/MS [Methode 1]: $R_t$ = 1.34 min; MS (ESIpos): m/z = 567 $(M+H)^+$.

**Beispiel 50.2A**

4-({2-[4-(2-Brom-5-chlorphenyl)-5-methoxy-2-oxopyridin-1(2*H*)-yl]propanoyl}amino)-benzoesäure-*tert*.-butylester (Racemat)

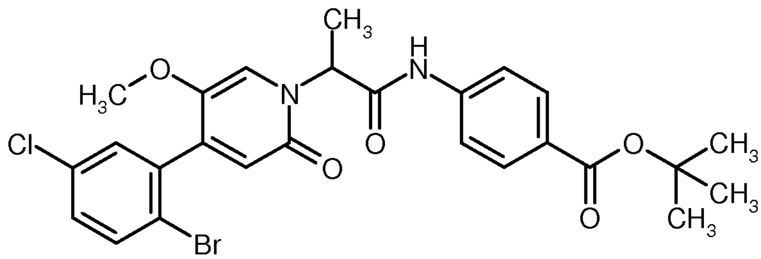

113 mg (0.25 mmol) 4-{[2-(4-Brom-5-methoxy-2-oxopyridin-1(2*H*)-yl)propanoyl]amino}-benzoesäure-*tert*.-butylester (Racemat), 70 mg (0.30 mmol, 1.2 eq.) 2-Brom-5-chlorphenylboronsäure, 103 mg (0.74 mmol, 3.0 eq.) Kaliumcarbonat und 20 mg (0.03 mmol, 0.1 eq.) [1,1 -Bis-(diphenylphosphino)-ferrocen]-palladium(II)chlorid-Dichlormethan-Monoaddukt wurden unter Argon (im ausgeheizten Kolben) in 5.0 ml Dioxan suspendiert und in einem bereits auf 110°C vorgeheiztem Ölbad über Nacht gerührt. Das Reaktionsgemisch wurde mit weiteren 10 mg (0.01 mmol, 0.05 eq.) [1,1-Bis-(diphenylphosphino)-ferrocen]-palladium(II)chlorid-Dichlormethan-Monoaddukt und 29 mg (0.12 mmol, 0.5 eq.) 2-Brom-5-chlorphenylboronsäure versetzt, eine weitere Nacht bei 110°C gerührt und anschließend über Celite filtriert. Der Rückstand wurde mit Dioxan nachgewaschen. Die vereinigten Filtrate wurden im Vakuum eingeengt. Der Rückstand wurde mit Wasser verrührt, filtriert, mit Wasser nachgewaschen, im Vakuum getrocknet und mittels Flash-Chromatographie (Kieselgel-50, Cyclohexan/ Essigsäureethylester-Gradient) aufgereinigt. Ausbeute: 72 mg (73% Reinheit, 38% d.Th.)
LC/MS [Methode 1]: $R_t$ = 1.27 min; MS (ESIpos): m/z = 561 $(M+H)^+$.

**Beispiel 50.3A**

4-({2-[4-(5-Chlor-2-methylphenyl)-5-methoxy-2-oxopyridin-1(2*H*)-yl]propanoyl}amino)-benzoesäure-*tert*.-butylester (Racemat)

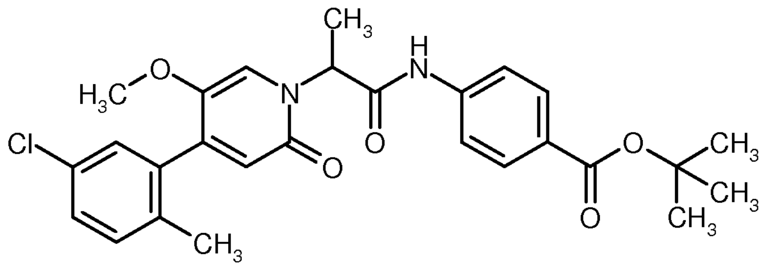

92 mg (0.20 mmol) 4-{[2-(4-Brom-5-methoxy-2-oxopyridin-1(2/7)-yl)propanoyl]amino}-benzoesäure-*tert*.-butylester (Racemat), 41 mg (0.24 mmol, 1.2 eq.) 5-Chlor-2-methylphenylboronsäure, 84 mg (0.61 mmol, 3.0 eq.) Kaliumcarbonat und 16 mg (0.02 mmol, 0.1 eq.) [1,1-Bis-(diphenylphosphino)-ferrocen]-palladium(II)chlorid-Dichlormethan-Monoaddukt wurden unter Argon (im ausgeheizten Kolben) in 5.0 ml Dioxan suspendiert und in einem bereits auf 110°C vorgeheiztem Ölbad über Nacht gerührt. Das Reaktionsgemisch wurde über Celite filtriert und der Rückstand mit Dioxan nachgewaschen. Die vereinigten Filtrate wurden im Vakuum eingeengt. Der Rückstand wurde mit Wasser verrührt, filtriert, mit Wasser nachgewaschen und im Vakuum getrocknet. Ausbeute: 105 mg (91 % Reinheit, 95% d.Th.)
LC/MS [Methode 1]: $R_t$ = 1.26 min; MS (ESIpos): m/z = 497 $(M+H)^+$.

**Beispiel 50.4A**

4-[(2-{4-[5-Chlor-2-(trifluormethyl)phenyl]-5-methoxy-2-oxopyridin-1(2*H*)-yl}propanoyl)amino]-benzoesäure-*tert*.-butylester (Racemat)

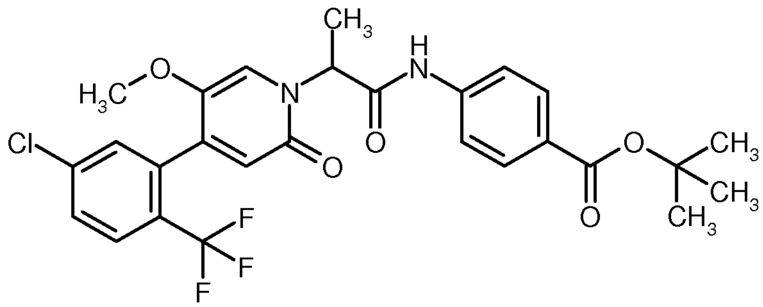

113 mg (0.25 mmol) 4-{[2-(4-Brom-5-methoxy-2-oxopyridin-1(2*H*)-yl)propanoyl]amino}-benzoesäure-*tert*.-butylester (Racemat), 67 mg (0.30 mmol, 1.2 eq.) 5-Chlor-2-trifluormethylphenylboronsäure, 103 mg (0.74 mmol, 3.0 eq.) Kaliumcarbonat und 20 mg (0.03 mmol, 0.1 eq.) [1,1-Bis-(diphenylphosphino)-ferrocen]-palladium(II)chlorid-Dichlormethan-Monoaddukt wurden unter Argon (im ausgeheizten Kolben) in 5.0 ml Dioxan suspendiert und in einem bereits auf 110°C vorgeheiztem Ölbad über Nacht gerührt. Das Reaktionsgemisch wurde mit weiteren 10 mg (0.01 mmol, 0.05 eq.) [1,1-Bis-(diphenylphosphino)-ferrocen]-palladium(II)chlorid-Dichlormethan-Monoaddukt und 22 mg (0.10 mmol, 0.4 eq.) 5-Chlor-2-trifluormethylphenylboronsäure versetzt, weitere 20 h bei 110°C gerührt und anschließend über Celite filtriert. Der Rückstand wurde mit Dioxan nachgewaschen. Die vereinigten Filtrate wurden im Vakuum eingeengt. Der Rückstand wurde mit Wasser verrührt, filtriert, mit Wasser nachgewaschen und im Vakuum getrocknet. Ausbeute: 145 mg (84% Reinheit, 89% d.Th.)
LC/MS [Methode 1]: $R_t$ = 1.26 min; MS (ESIpos): m/z = 551 $(M+H)^+$.

**Beispiel 50.5A**

4-({2-[4-(5-Chlor-2-cyclopropylphenyl)-5-methoxy-2-oxopyridin-1(2*H*)-yl]propanoyl}amino)-benzoesäure-tert.-butylester (Racemat)

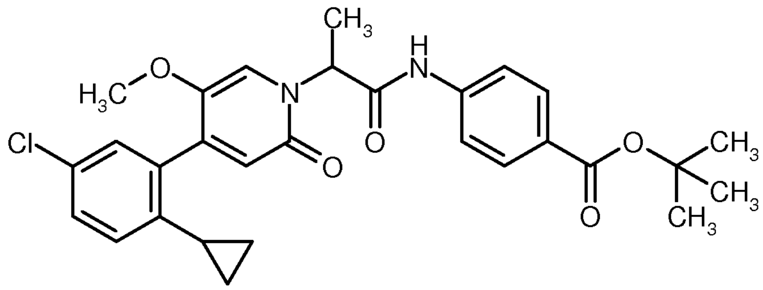

125 mg (0.27 mmol) 4-{[2-(4-Brom-5-methoxy-2-oxopyridin-1(2*H*)-yl)propanoyl]amino}-benzoesäure-*tert*.-butylester (Racemat), 92 mg (0.33 mmol, 1.2 eq.) 2-(5-Chlor-2-cyclopropylphenyl)-4,4,5,5-tetramethyl-1,3,2-dioxaborolan, 114 mg (0.82 mmol, 3.0 eq.) Kaliumcarbonat und 22 mg (0.03 mmol, 0.1 eq.) [1,1-Bis-(diphenylphosphino)-ferrocen]-palladium(II)chlorid-Dichlormethan-Monoaddukt wurden unter Argon (im ausgeheizten Kolben) in 5.0 ml Dioxan suspendiert und in einem bereits auf 110°C vorgeheiztem Ölbad über Nacht gerührt. Das Reaktionsgemisch wurde über Celite filtriert und der Rückstand mit Dioxan nachgewaschen. Die vereinigten Filtrate wurden im Vakuum eingeengt. Der Rückstand wurde mit Wasser verrührt, filtriert, mit Wasser nachgewaschen, im Vakuum getrocknet und mittels Flash-Chromatographie (Kieselgel-50, Cyclohexan/Essigsäureethylester-Gradient) aufgereinigt. Ausbeute: 114 mg (79% d.Th.)
LC/MS [Methode 1]: $R_t$ = 1.30 min; MS (ESIpos): m/z = 523 $(M+H)^+$.

**Beispiel 51.1A**

2-Brom-4-chlorphenyl-difluormethylether

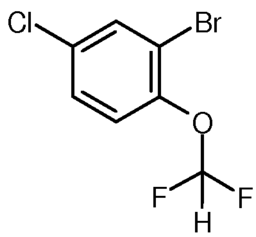

Eine Lösung von 3.5 g (16.9 mmol) 2-Brom-4-chlorphenol in 36 ml Acetonitril wurde mit 36 ml wässriger Kaliumhydroxid-Lösung (6M) versetzt, im Eisbad gekühlt und unter starkem Rühren tropfenweise mit 6.5 ml (26.9 mmol, 1.6 eq.) Difluormethyltrifluormethansulfonat [Angew. Chem. Int. Ed. 2013, 52, 1-5; Journal of Fluorine Chemistry 2009, 130, 667-670] versetzt. Das Reaktionsgemisch wurde 5 min gerührt und mit 200 ml Wasser verdünnt. Die wässrige Phase wurde zweimal mit je 150 ml Diethylether extrahiert. Die vereinigten, organischen Phasen wurden getrocknet (Natriumsulfat), filtriert, im Vakuum eingeengt und getrocknet. Die wässrige Phase wurde nochmals mit Diethylether extrahiert. Die organische Phase wurde getrocknet (Natriumsulfat), filtriert, im Vakuum eingeengt und getrocknet. Ausbeute beider vereinigter Rückstände: 3.4 g (80% d.Th.)
LC/MS [Methode 9]: $R_t$ = 3.51 min; MS (ESIpos): m/z = 256 $(M+H)^+$
$^1$H-NMR (400 MHz, DMSO-$d_6$): $\delta$ [ppm] = 7.91 (d, 1H), 7.55 (dd, 1H), 7.37 (d, 1H), 7.30 (t, 1H).

**Beispiel 51.1B**

4-[5-Chlor-2-(difluormethoxy)phenyl]-2,5-dimethoxypyridin

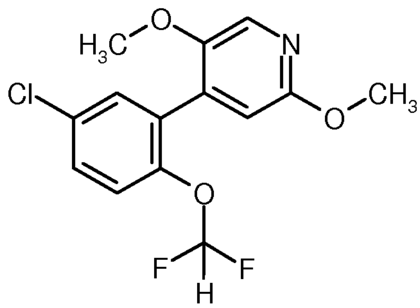

Nach der allgemeinen Methode 2A wurden 417 mg (2.19 mmol, 1.2 eq.) 2,5-Dimethoxypyridin-4-ylboronsäure mit 494 mg (1.82 mmol) 2-Brom-4-chlorphenyl-difluormethylether in Gegenwart von [1,1-Bis-(diphenylphosphino)-ferrocen]-palladium(II)chlorid-Dichlormethan-Monoaddukt umgesetzt. Das Rohprodukt wurde mittels Flash-Chromatographie (KP-SIL, Petrolether/ Essigsäureethylester 15-20%) aufgereinigt. Ausbeute: 170 mg (90% Reinheit, 27% d. Th.)
LC/MS [Methode 1]: $R_t$ = 1.16 min; MS (ESIpos): m/z = 316 $(M+H)^+$
$^1$H-NMR (400 MHz, DMSO-$d_6$): $\delta$ [ppm] = 7.96 (s, 1H), 7.57 (dd, 1H), 7.45 (d, 1H), 7.30 (d, 1H), 7.11 (t, 1H), 6.74 (s, 1H), 3.83 (s, 3H), 3.75 (s, 3H).

**Beispiel 51.1C**

4-[5-Chlor-2-(difluormethoxy)phenyl]-5-methoxypyridin-2(1*H*)-on

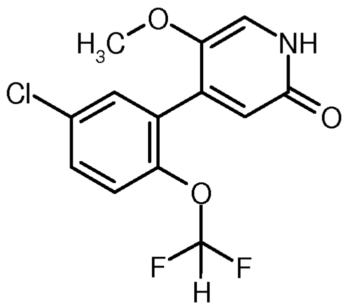

Nach der allgemeinen Methode 3A wurden 170 mg (90% Reinheit, 0.49 mmol) 4-[5-Chlor-2-(difluormethoxy)phenyl]-2,5-dimethoxypyridin mit Pyridinium-Hydrobromid umgesetzt. Ausbeute: 127 mg (87% d. Th.)
LC/MS [Methode 1]: $R_t$ = 0.84 min; MS (ESIpos): m/z = 302 $(M+H)^+$.

**Beispiel 51.1D**

2-{4-[5-Chlor-2-(difluormethoxy)phenyl]-5-methoxy-2-oxopyridin-1(2*H*)-yl}propansäure (Racemat)

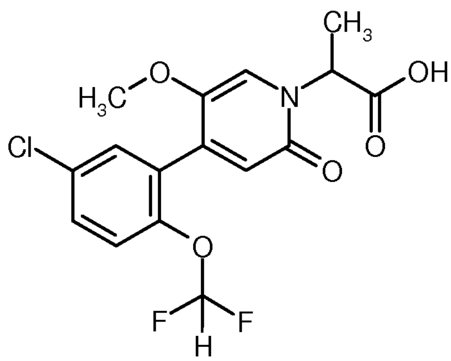

Nach der allgemeinen Methode 4A wurden 127 mg (0.42 mmol) 4-[5-Chlor-2-(difluormethoxy)phenyl]-5-methoxypyridin-2(1*H*)-on mit 1.5 eq. 2-Brompropansäure (Racemat) bei 90°C umgesetzt. Ausbeute: 220 mg Rohprodukt, das ohne weitere Reinigung in der nächsten Stufe umgesetzt wurde

## Beispiel 51.1E

4-[(2-{4-[5-Chlor-2-(difluormethoxy)phenyl]-5-methoxy-2-oxopyridin-1(2*H*)-yl}propanoyl)-amino]benzoesäure-*tert*.-butylester (Racemat)

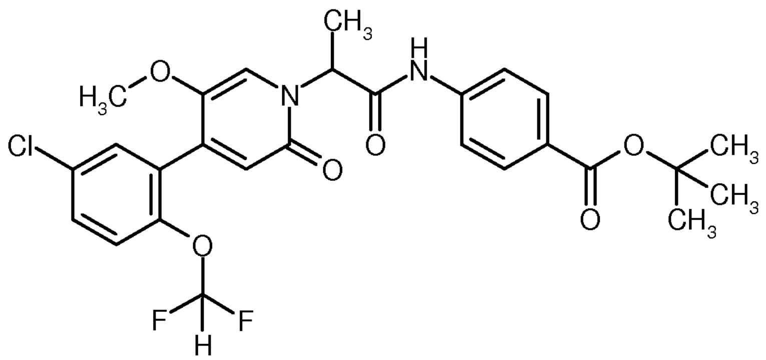

Nach der allgemeinen Methode 5A wurden 220 mg Rohprodukt von 2-{4-[5-Chlor-2-(difluormethoxy)phenyl]-5-methoxy-2-oxopyridin-1(2*H*)-yl}propansäure (Racemat) mit 89mg (0.46 mmol, 1.1 eq.) 4-Aminobenzoesäure-*tert*.-butylester umgesetzt. Ausbeute: 48 mg (21% d. Th.)
LC/MS [Methode 1]: $R_t$ = 1.26 min; MS (ESIpos): m/z = 549 $(M+H)^+$
$^1$H-NMR (400 MHz, DMSO-$d_6$): $\delta$ [ppm] = 10.70 (s, 1H), 7.87 (d, 2H), 7.73 (d, 2H), 7.58 (dd, 1H), 7.48 (d, 1H), 7.35 (s, 1H), 7.30 (d, 1H), 7.16 (t, 1H), 6.38 (s, 1H), 5.58 (q, 1H), 3.65 (s, 3H), 1.71 (d, 3H), 1.54 (s, 9H).

## Beispiel 51.2A

2-{4-[5-Chlor-2-(difluormethoxy)phenyl]-5-methoxy-2-oxopyridin-1(2*H*)-yl}butansäureethylester (Racemat)

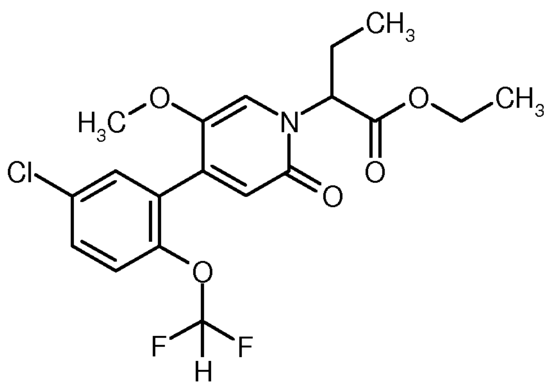

Eine Lösung von 618 mg (2.03 mmol) 4-[5-Chlor-2-(difluormethoxy)phenyl]-5-methoxypyridin-2(1*H*)-on in 25 ml Tetrahydrofuran wurde unter Argon bei RT mit 105 mg (2.64 mmol, 1.3 eq.) Natriumhydrid (60%ig in Mineralöl) versetzt, 60 min bei RT gerührt, anschließend tropfenweise mit 871 mg (2.64 mmol, 1.3 eq.) 2-{[(Trifluormethyl)sulfonyl]oxy}butansäureethylester (Racemat) [J. Castells et al. Tetrahedron, 1994, 50, 13765-13774] versetzt und 1 h bei RT gerührt. Das Reaktionsgemisch wurde mit weiteren 38 mg (0.96 mmol) Natriumhydrid (60%ig in Mineralöl) versetzt, 5 min bei RT gerührt, tropfenweise mit weiteren 871 mg (2.64 mmol, 1.3 eq.) 2-{[(Trifluormethyl)sulfonyl]oxy}butansäureethylester (Racemat) versetzt, 15 min bei RT gerührt und anschließend mit Wasser gequencht. Nach Phasentrennung

wurde die wässrige Phase zweimal mit Essigsäureethylester extrahiert. Die vereinigten, organischen Phasen wurden mit gesättigter, wässriger Natriumchchlorid-Lösung gewaschen, getrocknet (Natriumsulfat), filtriert und im Vakuum eingeengt. Der Rückstand wurde mittels Flash-Chromatographie (Kieselgel-50, Cyclohexan/Essigsäureethylester-Gradient) aufgereinigt. Ausbeute: 415 mg (48% d. Th.)
LC/MS [Methode 1]: $R_t$ = 1.08 min; MS (ESIpos): m/z = 416 $(M+H)^+$.

**Beispiel 51.2B**

2-{4-[5-Chlor-2-(difluormethoxy)phenyl]-5-methoxy-2-oxopyridin-1(2*H*)-yl}butansäure (Racemat)

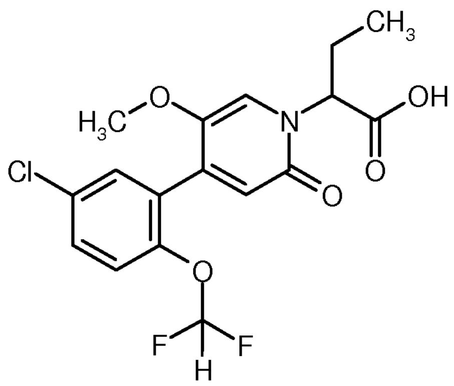

Nach der allgemeinen Methode 6B wurden 415 mg (0.97 mmol) 2-{4-[5-Chlor-2-(difluormethoxy)phenyl]-5-methoxy-2-oxopyridin-1(2*H*)-yl}butansäureethylester (Racemat) mit Lithiumhydroxid verseift. Ausbeute: 348 mg (93% d. Th.)
LC/MS [Methode 1]: $R_t$ = 0.91 min; MS (ESIpos): m/z = 388 $(M+H)^+$
$^1$H-NMR (400 MHz, DMSO-$d_6$): $\delta$ [ppm] = 12.96 (br. s, 1H), 7.57 (dd, 1H), 7.50 (d, 1H), 7.34-7.25 (m, 2H), 7.12 (t, 1H), 6.35 (s, 1H), 5.06 (dd, 1H), 3.58 (s, 3H), 2.20-2.06 (m, 2H), 0.82 (t, 3H).

**Beispiel 51.2C**

4-[(2-{4-[5-Chlor-2-(difluormethoxy)phenyl]-5-methoxy-2-oxopyridin-1(2*H*)-yl}butanoyl)-amino]benzoesäure-*tert*.-butylester (Racemat)

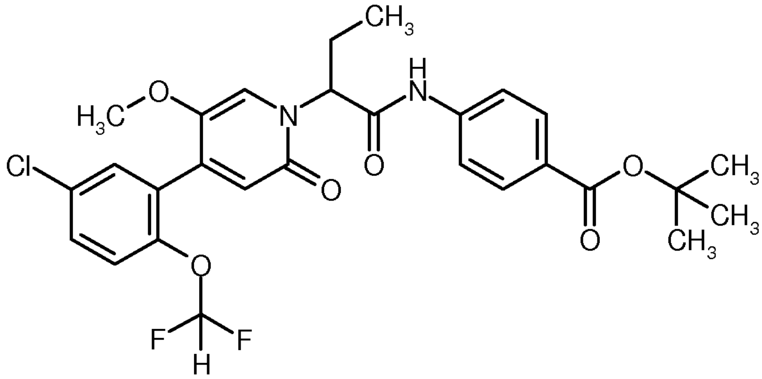

Nach der allgemeinen Methode 5A wurden 116 mg (0.30 mmol) 2-{4-[5-Chlor-2-(difluormethoxy)phenyl]-5-methoxy-2-oxopyridin-1(2*H*)-yl}butansäure (Racemat) mit 64 mg (0.33 mmol, 1.1 eq.) 4-Aminobenzoesäure-*tert*.-butylester umgesetzt. Ausbeute: 127 mg (75% d. Th.)
LC/MS [Methode 1]: $R_t$ = 1.32 min; MS (ESIpos): m/z = 563 $(M+H)^+$.

**Beispiel 52.1A**

5-Ethoxy-4-iod-2-methoxypyridin

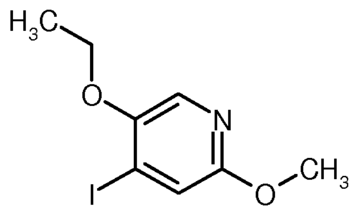

Eine Lösung von 405 mg (1.5 mmol) 4-Iod-6-methoxypyridin-3-ol in 10 ml Aceton wurde bei 0°C mit 304 mg (1.95 mmol, 1.3 eq.) Iodethan und 415 mg (3.0 mmol, 2.0 eq.) Kaliumcarbonat versetzt, über Nacht bei 80°C gerührt und im Vakuum eingeengt. Der Rückstand wurde mit Wasser verrührt, filtriert und im Vakuum getrocknet. Ausbeute: 322 mg (93% Reinheit, 72% d. Th.)
LC/MS [Methode 1]: $R_t$ = 1.07 min; MS (ESIpos): m/z = 280 $(M+H)^+$.

**Beispiel 52.1B**

4-Chlor-2-(5-ethoxy-2-methoxypyridin-4-yl)benzonitril

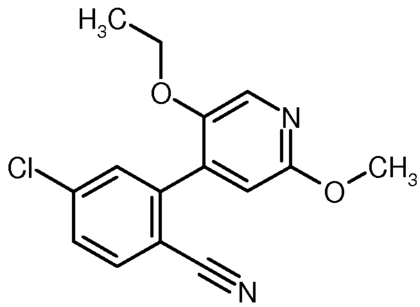

Nach der allgemeinen Methode 2A wurden 322 mg (93% Reinheit, 1.07 mmol) 5-Ethoxy-4-iod-2-methoxypyridin mit 234 mg (1.29 mmol, 1.2 eq.) 5-Chlor-2-cyanophenylboronsäure in Gegenwart von [1,1-Bis-(diphenylphosphino)-ferrocen]-palladium(II)chlorid-Dichlormethan-Monoaddukt umgesetzt. Das Rohprodukt wurde mittels Flash-Chromatographie (Kieselgel-50, Cyclohexan/Essigsäureethylester-Gradient) aufgereinigt. Ausbeute: 135 mg (41% d. Th.)
LC/MS [Methode 1]: $R_t$ = 1.15 min; MS (ESIpos): m/z = 289 $(M+H)^+$.

**Beispiel 52.1C**

4-Chlor-2-(5-ethoxy-2-oxo-1,2-dihydropyridin-4-yl)benzonitril

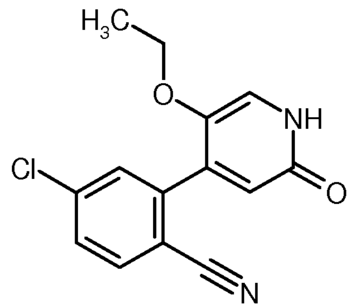

Nach der allgemeinen Methode 3A wurden 135 mg (0.44 mmol) 4-Chlor-2-(5-ethoxy-2-methoxypyridin-4-yl)benzonitril mit Pyridinium-Hydrobromid umgesetzt. Ausbeute: 134 mg (76% Reinheit, 83% d. Th.)
LC/MS [Methode 1]: $R_t$ = 0.81 min; MS (ESIpos): m/z = 275 $(M+H)^+$.

**Beispiel 52.1D**

2-[4-(5-Chlor-2-cyanophenyl)-5-ethoxy-2-oxopyridin-1(2*H*)-yl]propansäure (Racemat)

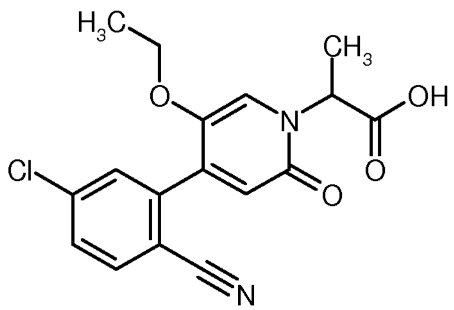

Nach der allgemeinen Methode 4A wurden 134 mg (0.37 mmol) 4-Chlor-2-(5-ethoxy-2-oxo-1,2-dihydropyridin-4-yl)benzonitril mit 1.5 eq. 2-Brompropansäure (Racemat) bei 50°C umgesetzt. Das Rohprodukt wurde mittels präparativer HPLC (Reprosil C18, Wasser-Acetonitril-Gradient) aufgereinigt. Ausbeute: 89 mg (86% Reinheit, 60% d. Th.)
LC/MS [Methode 1]: $R_t$ = 0.87 min; MS (ESIpos): m/z = 347 $(M+H)^+$.

**Beispiel 52.1E**

4-({2-[4-(5-chlor-2-cyanophenyl)-5-ethoxy-2-oxopyridin-1(2*H*)-yl]propanoyl}amino)benzoesäure-*tert*.-butylester (Racemat)

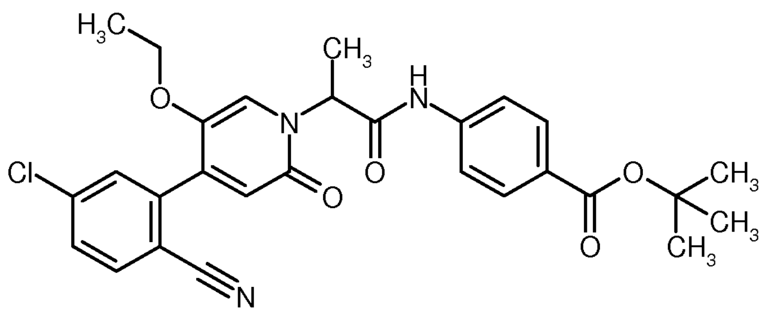

Nach der allgemeinen Methode 5A wurden 89 mg (86% Reinheit, 0.22 mmol) 2-[4-(5-Chlor-2-cyanophenyl)-5-ethoxy-2-oxopyridin-1(2*H*)-yl]propansäure (Racemat) mit 47 mg (0.24 mmol, 1.1 eq.) 4-Aminobenzoesäure-*tert*.-butylester umgesetzt. Ausbeute: 32 mg (89% Reinheit, 25% d. Th.)

LC/MS [Methode 3]: $R_t$ = 2.77 min; MS (ESIpos): m/z = 522 $(M+H)^+$.

**Beispiel 53.1A**

5-(Difluormethoxy)-4-iod-2-methoxypyridin

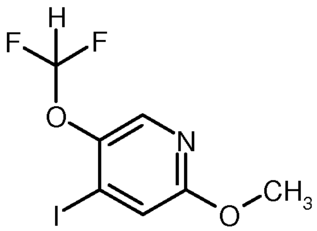

Eine Lösung von 600 mg (93% Reinheit, 2.22 mmol) 4-Iod-6-methoxypyridin-3-ol in 4.8 ml Acetonitril wurde mit 4.8 ml wässriger Kaliumhydroxid-Lösung (6M) versetzt, im Eisbad gekühlt und unter starkem Rühren mit 863 µl (75% Reinheit, 3.56 mmol, 1.6 eq.) Difluormethyltrifluormethansulfonat [Angew. Chem. Int. Ed. 2013, 52, 1-5; Journal of Fluorine Chemistry 2009, 130, 667-670] versetzt. Das Reaktionsgemisch wurde 2 min gerührt und mit 33 ml Wasser verdünnt. Die wässrige Phase wurde zweimal mit je 40 ml Diethylether extrahiert. Die vereinigten, organischen Phasen wurden getrocknet (Natriumsulfat), filtriert, im Vakuum eingeengt und getrocknet. Das Rohprodukt wurde mittels Flash-Chromatographie (IR-50SI, Petrolether/Essigsäureethylester 12-20%) aufgereinigt. Ausbeute: 407 mg (90% Reinheit, 55% d.Th.)
$^1$H-NMR (400 MHz, DMSO-$d_6$): δ [ppm] = 8.1 (s, 1H), 7.45 (s, 1H), 7.16 (t, 1H), 3.84 (s, 3H).

**Beispiel 53.1B**

4-Chlor-2-[5-(difluormethoxy)-2-methoxypyridin-4-yl]benzonitril

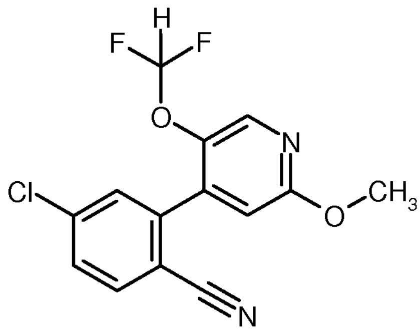

Nach der allgemeinen Methode 2A wurden 460 mg (90% Reinheit, 1.38 mmol) 5-(Difluormethoxy)-4-iod-2-methoxypyridin mit 299 mg (1.65 mmol, 1.2 eq.) 5-Chlor-2-cyanophenylboronsäure in Gegenwart von [1,1-Bis-(diphenylphosphino)-ferrocen]-palladium(II)chlorid-Dichlormethan-Monoaddukt umgesetzt. Das Rohprodukt wurde mittels Flash-Chromatographie (IR-50SI, Petrolether/Essigsäureethylester 10-15%) aufgereinigt. Ausbeute: 230 mg (80% Reinheit, 43% d. Th.)
LC/MS [Methode 1]: $R_t$ = 1.12 min; MS (ESIpos): m/z = 311 $(M+H)^+$.
$^1$H-NMR (400 MHz, DMSO-$d_6$): δ [ppm] = 8.26 (s, 1H), 8.06 (d, 1H), 7.82-7.74 (m, 2H), 7.09 (s, 1H), 7.06 (t, 1H), 3.91 (s, 3H).

**Beispiel 53.1C**

4-Chlor-2-[5-(difluormethoxy)-2-oxo-1,2-dihydropyridin-4-yl]benzonitril

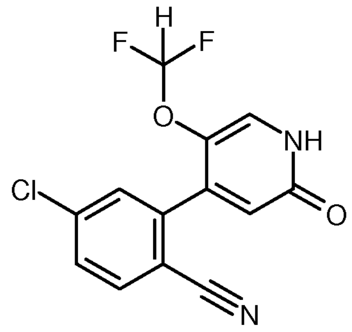

Nach der allgemeinen Methode 3A wurden 230 mg (80% Reinheit, 0.59 mmol) 4-Chlor-2-[5-(difluormethoxy)-2-methoxypyridin-4-yl]benzonitril mit Pyridinium-Hydrobromid umgesetzt. Das Rohprodukt wurde mittels Flash-Chromatographie (IR-50SI, Dichlormethan/Methanol 3-25%) aufgereinigt. Ausbeute: 167 mg (95% d. Th.)
LC/MS [Methode 1]: $R_t$ = 0.79 min; MS (ESIpos): m/z = 297 $(M+H)^+$
$^1$H-NMR (400 MHz, DMSO-$d_6$): $\delta$ [ppm] = 11.88 (br. s, 1H), 8.03 (d, 1H), 7.80-7.65 (m, 3H), 6.87 (t, 1H), 6.56 (s, 1H).

**Beispiel 53.1D**

2-[4-(5-Chlor-2-cyanophenyl)-5-(difluormethoxy)-2-oxopyridin-1(2N)-yl]propansäure (Racemat)

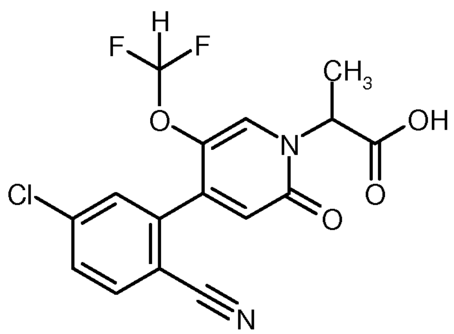

Nach der allgemeinen Methode 4A wurden 163 mg (0.55 mmol) 4-Chlor-2-[5-(difluormethoxy)-2-oxo-1,2-dihydropyridin-4-yl]benzonitril mit 2.0 eq. Magnesiumdi-*tert*.-butylat, 1.05 eq. Kalium-*tert*.-butylat und 1.5 eq. 2-Brompropansäure (Racemat) bei 45°C umgesetzt und aufgearbeitet. Aufgrund unvollständigem Umsatz wurde das Rohprodukt anschließend erneut wie oben beschrieben mit 1.2 eq. Magnesiumdi-*tert*.-butylat, 0.65 eq. Kalium-*tert*.-butylat und 0.8 eq. 2-Brompropionsäure (Racemat) in 3.5 ml Tetrahydrofuran umgesetzt und analog aufgearbeitet. Ausbeute: 270 mg (63% Reinheit, 84% d. Th.)
LC/MS [Methode 1]: $R_t$ = 0.86 min; MS (ESIpos): m/z = 369 $(M+H)^+$.

**Beispiel 53.1E**

4-({2-[4-(5-Chlor-2-cyanophenyl)-5-(difluormethoxy)-2-oxopyridin-1(2*H*)-yl]propanoyl}amino)-benzoesäure-*tert*.-butylester (Racemat)

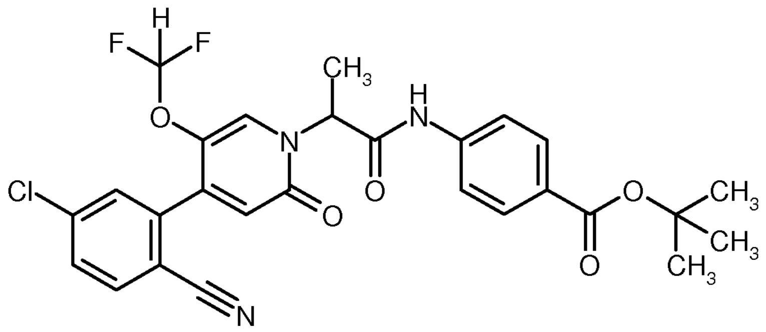

Nach der allgemeinen Methode 5A wurden 270 mg (63% Reinheit, 0.46 mmol) 2-[4-(5-Chlor-2-cyanophenyl)-5-(difluormethoxy)-2-oxopyridin-1(2*H*)-yl]propansäure (Racemat) mit 98 mg (0.51 mmol, 1.1 eq.) 4-Aminobenzoesäure-*tert.*-butylester umgesetzt. Ausbeute: 100 mg (40% d. Th.)
LC/MS [Methode 1]: $R_t$ = 1.23 min; MS (ESIpos): m/z = 544 $(M+H)^+$
$^1$H-NMR (400 MHz, DMSO-$d_6$): δ [ppm] = 10.78 (s, 1H), 8.05 (d, 1H), 7.99 (s, 1H), 7.88 (d, 2H), 7.82-7.69 (m, 4H), 6.89 (t, 1H), 6.65 (s, 1H), 5.56 (q, 1H), 1.72 (d, 3H), 1.54 (s, 9H).

**Beispiel 54.1A**

4-Iod-2-methoxy-5-(2,2,2-trifluorethoxy)pyridin

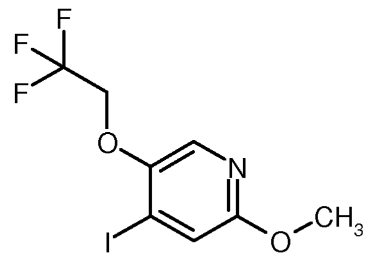

Eine Lösung von 455 mg (93% Reinheit, 1.7 mmol) 4-Iod-6-methoxypyridin-3-ol in 10 ml Dimethylformamid und 0.4 ml Acetonitril wurde mit 466 mg (3.4 mmol, 2.0 eq.) Kaliumcarbonat und 567 mg (2.5 mmol, 1.5 eq.) 2,2,2-Trifluorethyltrifluormethansulfonat versetzt und 30 min bei 150°C in der Mikrowelle bestrahlt. Das Reaktionsgemisch wurde mit weiteren 393 mg (1.7 mmol, 1.0 eq.) 2,2,2-Trifluorethyltrifluormethansulfonat versetzt und erneut 30 min bei 150°C in der Mikrowelle bestrahlt. Nach Zugabe von Wasser/Essigsäureethylester und Phasentrennung wurde die wässrige Phase zweimal mit Essigsäureethylester extrahiert. Die vereinigten, organischen Phasen wurden mit gesättigter, wässriger Natriumchlorid-Lösung gewaschen, getrocknet (Natriumsulfat), filtriert, im Vakuum eingeengt und getrocknet. Ausbeute: 500 mg (94% Reinheit, 94% d. Th.)
LC/MS [Methode 1]: $R_t$ = 1.11 min; MS (ESIpos): m/z = 334 $(M+H)^+$.

**Beispiel 54.1B**

4-Chlor-2-[2-methoxy-5-(2,2,2-trifluorethoxy)pyridin-4-yl]benzonitril

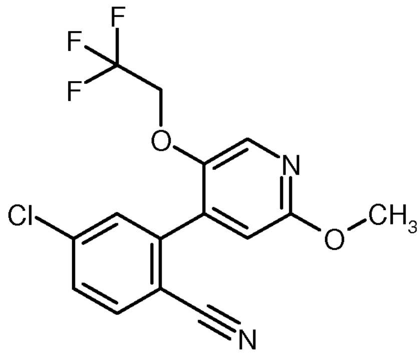

Nach der allgemeinen Methode 2A wurden 500 mg (94% Reinheit, 1.41 mmol) 4-Iod-2-methoxy-5-(2,2,2-trifluorethoxy)pyridin mit 282 mg (1.55 mmol, 1.1 eq.) 5-Chlor-2-cyanophenylboronsäure in Gegenwart von [1,1-Bis-(diphenylphosphino)-ferrocen]-palladium(II)chlorid-Dichlormethan-Monoaddukt umgesetzt. Das Rohprodukt wurde mittels Flash-Chromatographie (Kieselgel-50, Cyclohexan/Essigsäureethylester-Gradient) aufgereinigt. Ausbeute: 168 mg (33% d. Th.)
LC/MS [Methode 1]: $R_t$ = 1.16 min; MS (ESIpos): m/z = 343 $(M+H)^+$.

**Beispiel 54.1C**

4-Chlor-2-[2-oxo-5-(2,2,2-trifluorethoxy)-1,2-dihydropyridin-4-yl]benzonitril

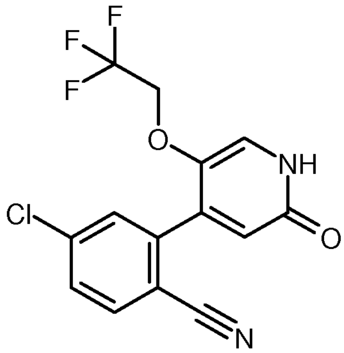

Nach der allgemeinen Methode 3A wurden 168 mg (0.47 mmol) 4-Chlor-2-[2-methoxy-5-(2,2,2-trifluorethoxy)pyridin-4-yl]benzonitril mit Pyridinium-Hydrobromid umgesetzt. Ausbeute: 112 mg (92% Reinheit, 67% d. Th.)
LC/MS [Methode 1]: $R_t$ = 0.87 min; MS (ESIpos): m/z = 329 $(M+H)^+$.

**Beispiel 54.1D**

2-[4-(5-Chlor-2-cyanophenyl)-2-oxo-5-(2,2,2-trifluorethoxy)pyridin-1(2*H*)-yl]propansäure (Racemat)

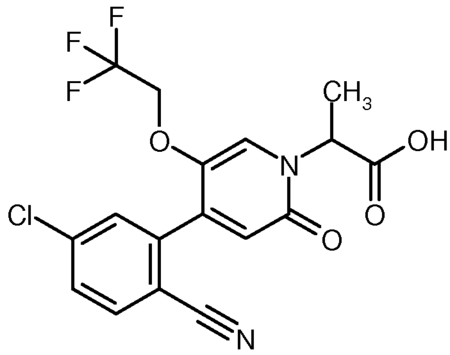

Nach der allgemeinen Methode 4A wurden 140 mg (87% Reinheit, 0.37 mmol) 4-Chlor-2-[2-oxo-5-(2,2,2-trifluorethoxy)-1,2-dihydropyridin-4-yl]benzonitril mit 2.0 eq. Magnesiumdi-*tert*.-butylat, 1.05 eq. Kalium-*tert*.-butylat und 1.5 eq. 2-Brompropansäure (Racemat) bei 50°C umgesetzt und nach wässriger Aufarbeitung ohne weitere Reinigung in die nächste Stufe umgesetzt. Ausbeute: 214 mg (73% Reinheit, quant.)
LC/MS [Methode 1]: $R_t$ = 0.91 min; MS (ESIpos): m/z = 401 $(M+H)^+$.

**Beispiel 54.1E**

4-({2-[4-(5-Chlor-2-cyanophenyl)-2-oxo-5-(2,2,2-trifluorethoxy)pyridin-1(2*H*)-yl]propanoyl}-amino)benzoesäure-*tert*.-butylester (Racemat)

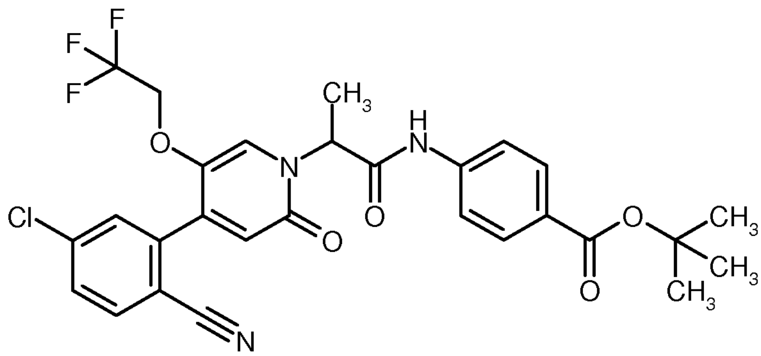

Nach der allgemeinen Methode 5A wurden 214 mg (73% Reinheit, 0.39 mmol) 2-[4-(5-Chlor-2-cyanophenyl)-2-oxo-5-(2,2,2-trifluorethoxy)pyridin-1(2*H*)-yl]propansäure (Racemat) mit 83 mg (0.43 mmol, 1.1 eq.) 4-Aminobenzoesäure-*tert*.-butylester umgesetzt. Ausbeute: 113 mg (70% Reinheit, 35% d. Th.)
LC/MS [Methode 1]: $R_t$ = 1.23 min; MS (ESIpos): m/z = 576 $(M+H)^+$.

**Beispiel 55.1A**

2-Methoxybutyl-trifluormethansulfonat (Racemat)

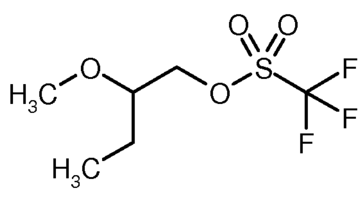

Nach der allgemeinen Methode 8A wurden 1.0 g (9.6 mmol) 2-Methoxybutanol mit 1.78 ml (10.6 mmol, 1.1

eq.) Trifluormethansulfonsäureanhydrid in Gegenwart von 1.47 ml (10.6 mmol, 1.1 eq.) Triethylamin umgesetzt. Das Rohprodukt wurde ohne weitere Reinigung in der nächsten Stufe umgesetzt.
$^1$H-NMR (400 MHz, $CDCl_3$): δ [ppm] = 4.51 (dd, 1H), 4.43 (dd, 1H), 3.44 (s, 3H), 3.44-3.39 (m, 1H), 1.65-1.54 (m, 2H), 0.98 (t, 3H).

**Beispiel 55.1B**

2-[4-(5-Chlor-2-cyanophenyl)-5-methoxy-2-oxopyridin-1(2*H*)-yl]-4-methoxyhexansäure-*tert.*-butylester (Gemisch racemischer Diastereomere)

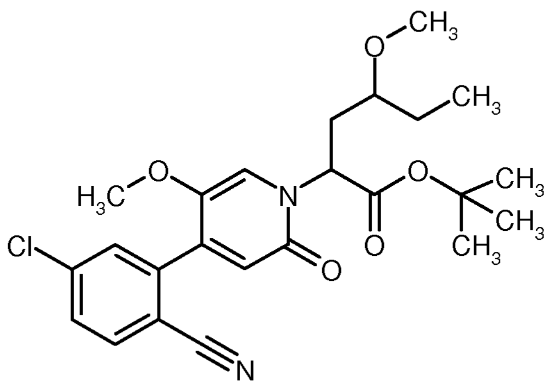

Nach der allgemeinen Methode 7B wurden 1.00 g (2.67 mmol) [4-(5-Chlor-2-cyanophenyl)-5-methoxy-2-oxopyridin-1(2*H*)-yl]essigsäure-*tert.*-butylester in Gegenwart von 2.94 ml (2.94 mmol, 1.1 eq.) Bis-(trimethylsilyl)-lithiumamid (1M in Tetrahydrofuran) mit 945 mg (4.00 mmol, 1.5 eq.) 2-Methoxybutyl-trifluormethansulfonat (Racemat) umgesetzt. Ausbeute: 669 mg (54% d. Th.)
LC/MS [Methode 1]: $R_t$ = 1.15 min; MS (ESIpos): m/z = 461 $(M+H)^+$.
$^1$H-NMR (400 MHz, DMSO-$d_6$): δ [ppm] = 7.98 (d, 1H), 7.75-7.70 (m, 2H), 7.44/7.40 (2x s, 1H), 6.49/6.48 (2x s, 1H), 5.24-5.17 (m, 1H), 3.64 (2x s, 3H), 3.19/3.16 (2x s, 3H), 2.81-2.74 (m, 1H), 2.45-2.28 (m, 1H), 2.16-2.03 (m, 1H), 1.57-1.38 (m, 2H), 1.41 (2x s, 9H), 0.84/0.80 (2x t, 3H).

**Beispiel 55.1C**

2-[4-(5-Chlor-2-cyanophenyl)-5-methoxy-2-oxopyridin-1(2*H*)-yl]-4-methoxyhexansäure (Gemisch racemischer Diastereomere)

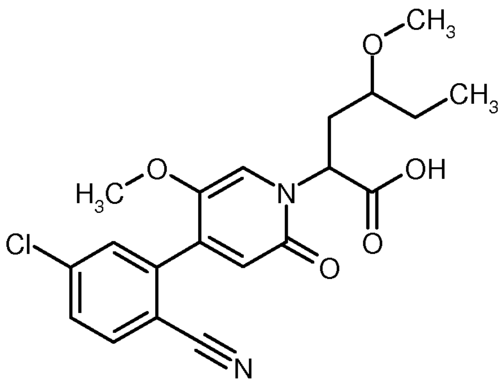

Nach der allgemeinen Methode 6A wurden 668 mg (1.45 mmol) 2-[4-(5-Chlor-2-cyanophenyl)-5-methoxy-2-oxopyridin-1(2*H*)-yl]-4-methoxyhexansäure-*tert.*-butylester (Gemisch racemischer Diastereomere) mit Trifluoressigsäure verseift. Ausbeute: 623 mg (94% Reinheit, quant.)
LC/MS [Methode 1]: $R_t$ = 0.89 min; MS (ESIpos): m/z = 405 $(M+H)^+$
$^1$H-NMR (400 MHz, DMSO-$d_6$): δ [ppm] = 13.0 (br. s, 1H), 7.98 (d, 1H), 7.77-7.70 (m, 2H), 7.50/7.44 (2x s, 1H), 6.48/6.47 (2x s, 1H), 5.28-5.20 (m, 1H), 3.64 (2x s, 3H), 3.16/3.15 (2x s, 3H), 2.78-2.71 (m, 1H), 2.48-2.28 (m, 1H), 2.24-2.06 (m, 1H), 1.55-1.41 (m, 2H), 0.83/0.79 (2x t, 3H).

**Beispiel 55.1D**

4-({2-[4-(5-Chlor-2-cyanophenyl)-5-methoxy-2-oxopyridin-1(2*H*)-yl]-4-methoxyhexanoyl}amino)-benzoesäureethylester (Gemisch racemischer Diastereomere)

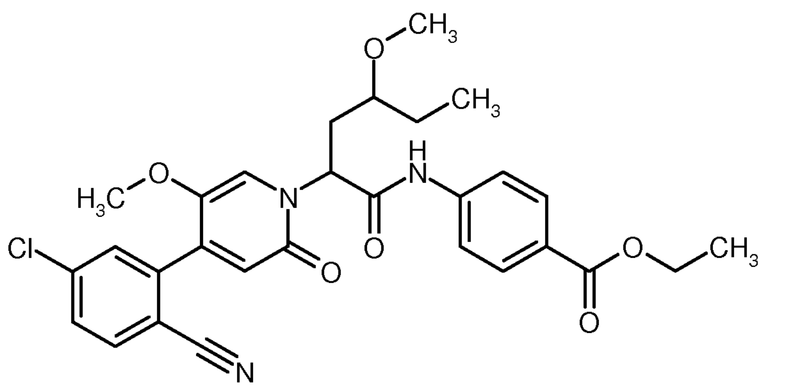

Gemäß allgemeiner Methode 5B wurden 620 mg (1.53 mmol) 2-[4-(5-Chlor-2-cyanophenyl)-5-methoxy-2-oxopyridin-1(2*H*)-yl]-4-methoxyhexansäure (Gemisch racemischer Diastereomere), 253 mg (1.53 mmol) 4-Aminobenzoesäureethylester, 218 mg (1.53 mmol) Oxima und 239 µl (1.53 mmol) DIC in 15.3 ml Dimethylformamid zur Reaktion gebracht. Das Rohprodukt wurde mittels Flash-Chromatographie (120 g Kartusche, 85 ml/min, Cyclohexan/Essigsäureethylester-Gradient) gereinigt. Ausbeute: 634 mg (70% d. Th.)
LC/MS [Methode 2]: Diastereomer 1: $R_t$ = 3.75 min; MS (ESIpos): m/z = 552 $(M+H)^+$; Diastereomer 2: $R_t$ = 3.81 min; MS (ESIpos): m/z = 552 $(M+H)^+$.
$^1$H-NMR (400 MHz, DMSO-$d_6$): δ [ppm] = 10.8 (2x s, 1H), 8.01-7.98 (m, 1H), 7.95-7.91 (m, 2H), 7.83-7.79 (m, 2H), 7.76-7.72 (m, 2H), 7.59/7.51 (2x s, 1H), 6.54 (s, 1H), 5.87-5.80 (m, 1H), 4.31/4.29 (2x q, 2H), 3.69 (s, 3H), 3.19/3.13 (2x s, 3H), 3.08-2.88 (2x m, 1H), 2.44-2.17 (m, 2H), 1.62-1.44 (m, 2H), 1.31/1.28 (2x t, 3H), 0.86/0.85 (2x t, 3H).

**Beispiel 56.1A**

[4-(5-Chlor-2-cyanophenyl)-5-methoxy-2-oxopyridin-1(2*H*)-yl]essigsäurebenzylester

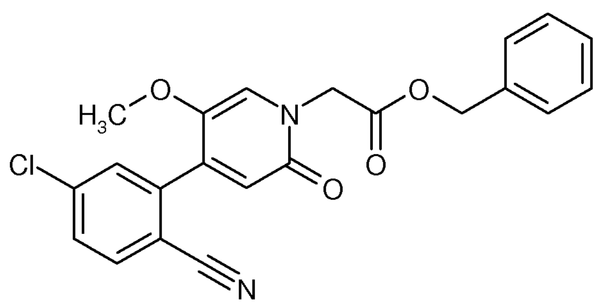

Eine Lösung von 4.00 g (15.3 mmol) 4-Chlor-2-(5-methoxy-2-oxo-1,2-dihydropyridin-4-yl)benzonitril und 2.92 ml (18.4 mmol) Bromessigsäurebenzylester in 53.3 ml Dimethylformamid wurde mit 3.18 g (23.0 mmol) Kaliumcarbonat versetzt und anschließend für 45 min bei 100°C gerührt. Die Reaktionsmischung wurde auf RT abgekühlt und die

Reaktion durch Zugabe von 530 ml Wasser und 10.0 g (236 mmol) Lithiumchlorid beendet. Es wurde dreimal mit 200 ml Essigsäureethylester extrahiert. Die vereinigten, organischen Phasen wurden über Magnesiumsulfat getrocknet, filtriert und das Lösungsmittel unter reduziertem Druck entfernt. Der Rückstand wurde in 50 ml Dichlormethan gelöst, auf Diatominerde aufgezogen und mittels Flash-Chromatographie (120 g Kartusche, 80 ml/min, Essigsäureethylester/Cyclohexan-Gradienten) gereinigt. Ausbeute: 3.90 g (61% d. Th.)
LC/MS [Methode 1]: $R_t$ = 1.02 min; MS (ESIpos): m/z = 409 $(M+H)^+$
$^1$H-NMR (400 MHz, DMSO-$d_6$): δ [ppm] = 8.00 (d, 1H), 7.75-7.72 (m, 2H), 7.61 (s, 1H), 7.42-7.33 (m, 5H), 6.54 (s, 1H), 5.22 (s, 2H), 4.81 (s, 2H), 3.62 (s, 3H).

**Beispiel 57.1A**

2-{[tert. -Butyl(diphenyl)silyl] oxy} ethanol

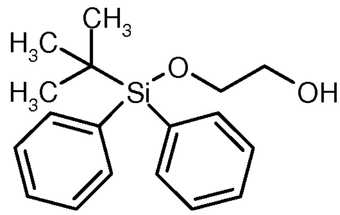

Zu einer Lösung von 10.1 ml (182 mmol) 1,2-Ethandiol und 2.97 g (43.7 mmol) Imidazol in 12 ml Tetrahydrofuran wurden 10.0 g (36.4 mmol) Chlor-*tert.*-butyl(diphenyl)silan gelöst in 88 ml Tetrahydrofuran über einen Zeitraum von 6 h getropft und anschließend über Nacht bei RT nachgerührt. Das Lösungsmittel wurde unter reduziertem Druck entfernt und der Rückstand mittels Flash-Chromatographie (340 g Silica-Kartusche, 100 ml/min, Cyclohexan/Essigsäureethylester-Gradient) gereinigt. Ausbeute: 8.34 g (76% d. Th.)
LC/MS [Methode 1]: $R_t$ = 1.57 min; MS (ESIpos): m/z = 323 $(M+Na)^+$
$^1$H-NMR (400 MHz, DMSO-$d_6$): δ [ppm] = 7.66-7.62 (m, 4H), 7.49-7.40 (m, 6H), 4.64 (t, 1H), 3.67-3.61 (m, 2H), 3.54-3.49 (m, 2H), 0.99 (s, 9H).

**Beispiel 57.1B**

2-{[*tert.*-Butyl(diphenyl)silyl]oxy}ethyl-trifluormethansulfonat

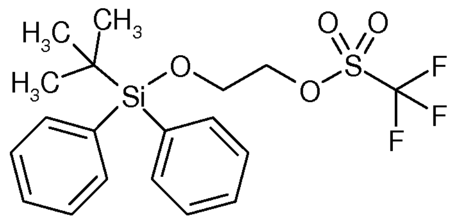

Zu 924 μl (5.49 mmol) Trifluormethansulfonsäureanhydrid in 5 ml Dichlormethan wurde bei -78°C eine Lösung aus 1.50 g (4.99 mmol) 2-{[*tert.*-Butyl(diphenyl)silyl]oxy}ethanol und 765 μl (5.49 mmol) Triethylamin in 5 ml Dichlormethan getropft, so dass die interne Temperatur -50°C nicht überstieg. Es wurde 15 min bei -78°C nachgerührt und spontan auf RT erwärmt. Die Reaktionsmischung wurde mit 50 ml Methyl-*tert.*-butylether verdünnt und dreimal mit 25 ml einer Mischung aus gesättigter, wässriger Natriumchlorid-Lösung und gesättigter, wässriger Ammoniumchlorid-Lösung (3:1) gewaschen, über Magnesiumsulfat getrocknet, filtriert und bei 25°C und ≥100 mbar Druck eingeengt. Ausbeute: 2.08 g (71% d. Th.)
$^1$H-NMR (400 MHz, DMSO-$d_6$): δ [ppm] = 7.66-7.62 (m, 4H), 7.52-7.44 (m, 6H), 4.45-4.41 (m, 2H), 3.89-3.85 (m, 2H), 1.03 (s, 9H).

**Beispiel 57.1C**

4-{[*tert.*-Butyl(diphenyl)silyl]oxy}-2-[4-(5-chlor-2-cyanophenyl)-5-methoxy-2-oxopyridin-1(2*H*)-yl]butansäurebenzylester (Racemat)

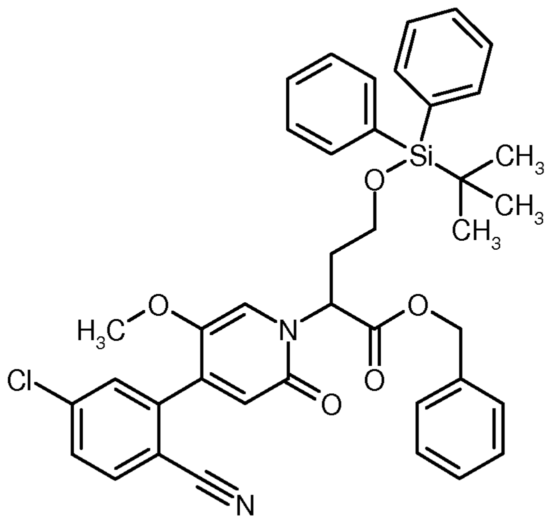

Nach der allgemeinen Methode 7B wurden 1.00 g (2.45 mmol) [4-(5-Chlor-2-cyanophenyl)-5-methoxy-2-oxopyridin-1(2*H*)-yl]essigsäurebenzylester in Gegenwart von 2.69 ml (2.69 mmol, 1.1 eq.) Bis-(trimethylsilyl)-lithiumamid (1 M in Tetrahydrofuran) mit 1.59 g (3.67 mmol, 1.5 eq.) 2-{[*tert.*-Butyl(diphenyl)silyl]oxy}ethyl-trifluormethansulfonat umgesetzt. Ausbeute: 708 mg (40% d. Th.)
LC/MS [Methode 1]: $R_t$ = 1.55 min; MS (ESIpos): m/z = 691 $(M+H)^+$
$^1$H-NMR (400 MHz, DMSO-$d_6$): δ [ppm] = 8.00 (d, 1H), 7.74 (dd, 1H), 7.62-7.56 (m, 5H), 7.47-7.29 (m, 12H), 6.56 (s, 1H), 5.51 (dd, 1H), 5.19 (s, 2H), 3.75-3.69 (m, 1H), 3.63-3.57 (m, 1H), 3.56 (s, 3H), 2.52-2.40 (m, 2H), 0.97 (s, 9H).

**Beispiel 57.1D**

4-{[*tert.*-Butyl(diphenyl)silyl]oxy}-2-[4-(5-chlor-2-cyanophenyl)-5-methoxy-2-oxopyridin-1(2*H*)-yl]butansäure (Racemat)

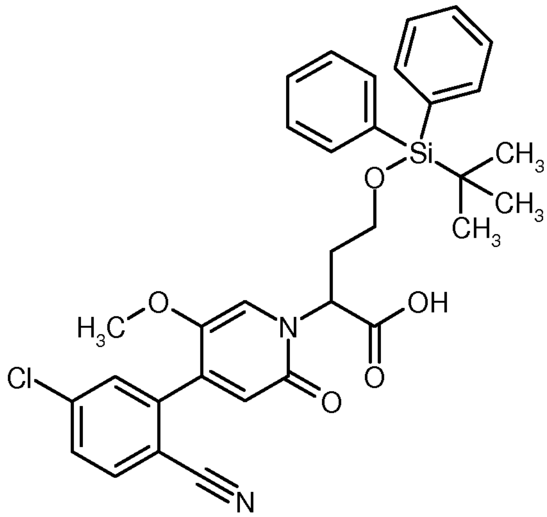

605 mg (875 µmol) 4-{[*tert.*-Butyl(diphenyl)silyl]oxy}-2-[4-(5-chlor-2-cyanophenyl)-5-methoxy-2-oxopyridin-1(2*H*)-yl]butansäurebenzylester (Racemat) wurden in 6 ml Tetrahydrofuran gelöst und mit 2.2 ml (2.2 mmol, 2.5 eq.) wässriger Natriumhydroxid-Lösung (1.0M) versetzt. Es wurde für 1 h bei RT nachgerührt und anschließend mit wässriger Salzsäure (1N) neutralisiert. Die Phasen wurden getrennt und die wässrige Phase zweimal mit 25 ml Essigsäureethylester extrahiert. Die vereinigten, organischen Phasen wurden über Magnesiumsulfat getrocknet, filtriert und das Lösungsmittel unter reduziertem Druck entfernt. Das Rohprodukt wurde ohne weitere Reinigung im nächsten Schritt eingesetzt. Ausbeute: 568 mg (93% d. Th.)
LC/MS [Methode 1]: $R_t$ = 1.39 min; MS (ESIpos): m/z = 601 $(M+H)^+$.

**Beispiel 57.1E**

4-[(4-{[*tert*.-Butyl(diphenyl)silyl]oxy}-2-[4-(5-chlor-2-cyanophenyl)-5-methoxy-2-oxopyridin-1(2*H*)-yl]butanoyl)amino]benzoesäureethylester (Racemat)

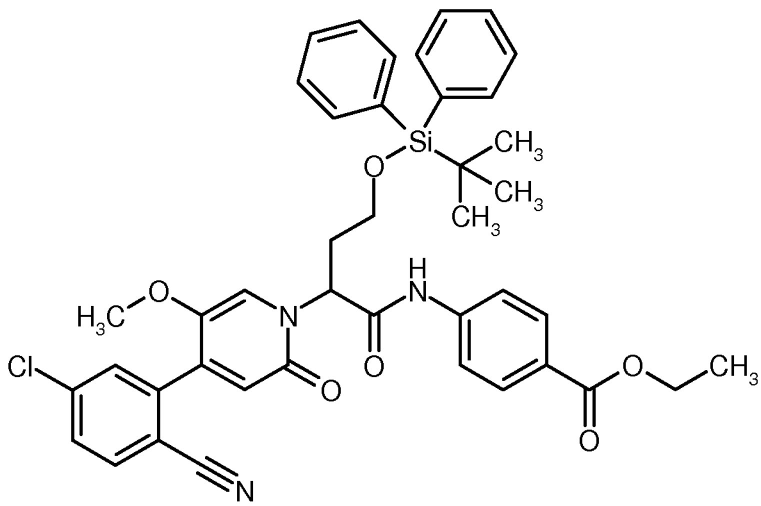

Gemäß allgemeiner Methode 5B wurden 565 mg (940 μmol) 4-{[*tert*.-Butyl(diphenyl)silyl]oxy}-2-[4-(5-chlor-2-cyanophenyl)-5-methoxy-2-oxopyridin-1(2*H*)-yl]butansäure (Racemat), 155 mg (940 μmol) 4-Aminobenzoesäureethylester, 134 mg (940 μmol) Oxima und 146 μl (940 μmol) DIC in 19 ml Dimethylformamid zur Reaktion gebracht. Das Rohprodukt wurde mittels Flash-Chromatographie (120 g Kartusche, 85 ml/min, Cyclohexan/Essigsäureethylester-Gradient) gereinigt. Ausbeute: 268 mg (38% d. Th.)
LC/MS [Methode 1]: $R_t$ = 1.54 min; MS (ESIpos): m/z = 748 $(M+H)^+$
$^1$H-NMR (400 MHz, DMSO-$d_6$): δ [ppm] = 10.8 (s, 1H), 8.00 (d, 1H), 7.94 (d, 2H), 7.79 (d, 2H), 7.74 (dd, 1H), 7.63-7.57 (m, 5H), 7.48 (s, 1H), 7.46-7.36 (m, 6H), 6.55 (s, 1H), 5.87 (dd, 1H), 4.29 (q, 2H), 3.72-3.67 (m, 2H), 3.64 (s, 3H), 2.52-2.48 (m, 2H, unter Lösungsmittelresonanz), 1.32 (t, 3H), 0.94 (s, 9H).

**Beispiel 58.1A**

2-[4-(5-Chlor-2-cyanophenyl)-5-methoxy-2-oxopyridin-1(2*H*)-yl]-3-(3-ethyloxetan-3-yl)prop-2-ensäure-tert. -butylester (Isomerengemisch)

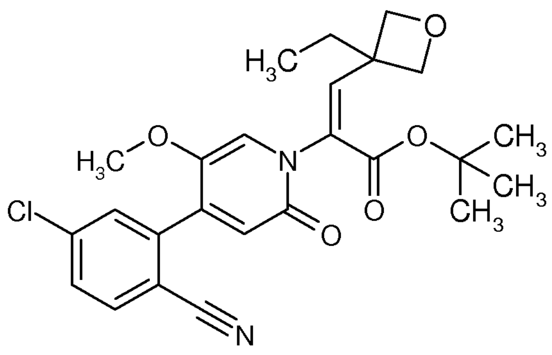

Eine Lösung von 300 mg (800 μmol) [4-(5-Chlor-2-cyanophenyl)-5-methoxy-2-oxopyridin-1(2*H*)-yl]essigsäure-*tert*.-butylester in 8 ml Dimethylformamid wurde nacheinander mit 38.4 mg (1.60 mmol, 2 eq., 60% in Mineralöl) Natriumhydrid und 457 mg (4.00 mmol) 3-Ethyloxetan-3-carbaldehyd in 1 ml Dimethylformamid versetzt. Nach 15 min bei RT wurde die Reaktion durch Zugabe von 10 ml gesättigter, wässriger Ammoniumchlorid-Lösung beendet und das Reaktionsgemisch anschließend dreimal mit 20 ml Essigsäureethylester extrahiert. Die vereinigten, organischen Phasen wurden über Magnesiumsulfat getrocknet, filtriert und das Lösungsmittel unter reduziertem Druck entfernt. Der Rückstand wurde mittels Flash-Chromatographie (24 g Kartusche, 35 ml/min, Cyclohexan/Essigsäureethylester-Gradient) gereinigt. Ausbeute: 320 mg (82% d. Th.)
LC/MS [Methode 1]: $R_t$ = 1.06 min; MS (ESIpos): m/z = 471 $(M+H)^+$
$^1$H-NMR (400 MHz, DMSO-$d_6$): δ [ppm] = 8.00 (d, 1H), 7.79-7.71 (m, 2H), 7.27 (s, 1H), 7.04 (s, 1H), 6.52 (s, 1H), 4.63 (d, 1H), 4.48 (d, 1H), 4.22 (d, 1H), 4.13 (d, 1H), 3.69/3.60 (2x s, 3H), 2.10-2.04/1.94-1.86 (2x q, 2H), 1.44-1.40 (2x s, 9H), 0.99/0.95 (2x t, 3H).

**Beispiel 58.1B**

2-[4-(5-Chlor-2-cyanophenyl)-5-methoxy-2-oxopyridin-1(2*H*)-yl]-3-(3-ethyloxetan-3-yl)propansäure-*tert*.-butylester (Racemat)

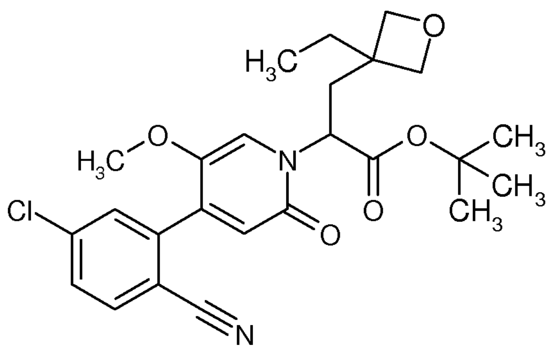

302 mg (641 μmol) 2-[4-(5-Chlor-2-cyanophenyl)-5-methoxy-2-oxopyridin-1(2*H*)-yl]-3-(3-ethyloxetan-3-yl)prop-2-ensäure-*tert*.-butylester (Isomerengemisch) wurden bei RT mit 20 ml einer "Hot Stryker's"-Reagenzlösung [B. A. Baker et al. Org. Lett. 2008, 10, 289-292] versetzt. Das Reaktionsgemisch wurde 1.5 h bei RT gerührt und anschließend mit 20 ml gesättigter, wässriger Ammoniumchlorid-Lösung versetzt. Nach Phasentrennung wurde die wässrige Phase dreimal mit 25 ml Essigsäureethylester extrahiert. Die vereinigten, organischen Phasen wurden über Magnesiumsulfat getrocknet, filtriert und das Lösungsmittel unter reduziertem Druck entfernt. Der Rückstand wurde mittels Flash-Chromatographie (120 g Silica Kartusche, 85 ml/min, Cyclohexan/Essigsäureethylester-Gradient) aufgereinigt. Ausbeute: 275 mg (91% d. Th.)
LC/MS [Methode 1]: $R_t$ = 1.11 min; MS (ESIpos): m/z = 473 $(M+H)^+$
$^1$H-NMR (400 MHz, DMSO-$d_6$): δ [ppm] = 7.98 (d, 1H), 7.75-7.71 (m, 2H), 7.51 (s, 1H), 6.49 (s, 1H), 5.22-5.13 (m, 1H), 4.31 (d, 1H), 4.23 (d, 1H), 4.04 (d, 1H), 3.93 (d, 1H), 3.65 (s, 3H), 2.65 (dd, 1H), 2.39 (dd, 1H), 1.83-1.72 (m, 2H), 1.41 (s, 9H), 0.83 (t, 3H).

**Beispiel 58.1C**

2-[4-(5-Chlor-2-cyanophenyl)-5-methoxy-2-oxopyridin-1(2*H*)-yl]-3-(3-ethyloxetan-3-yl)propansäure (Racemat)

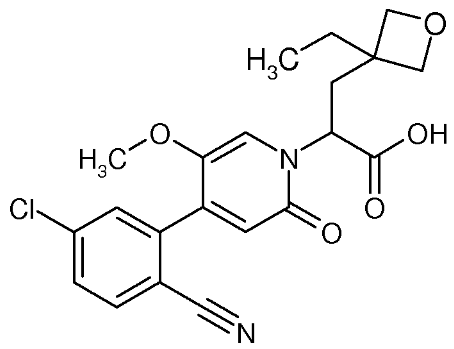

Gemäß allgemeiner Methode 6B wurden 275 mg (599 μmol) 2-[4-(5-Chlor-2-cyanophenyl)-5-methoxy-2-oxopyridin-1(2*H*)-yl]-3-(3-ethyloxetan-3-yl)propansäure-*tert*.-butylester (Racemat) mit 3 ml wässriger Lithiumhydroxid-Lösung (1N) umgesetzt und die Titelverbindung erhalten. Ausbeute: 193 mg (76% d. Th.)
LC/MS [Methode 1]: $R_t$ = 0.82 min; MS (ESIpos): m/z = 417 $(M+H)^+$
$^1$H-NMR (400 MHz, DMSO-$d_6$): δ [ppm] = 13.1 (br. s, 1H), 7.99 (d, 1H), 7.77-7.71 (m, 2H), 7.56 (s, 1H), 6.49 (s, 1H), 5.25-5.13 (m, 1H), 4.30 (d, 1H), 4.22 (d, 1H), 4.02 (d, 1H), 3.92 (d, 1H), 3.65 (s, 3H), 2.69 (dd, 1H), 2.42 (dd, 1H), 1.84-1.73 (m, 2H), 0.82 (t, 3H).

**Beispiel 59.1A**

1,1,2,2,2-Pentadeuteroethyl-trifluormethansulfonat

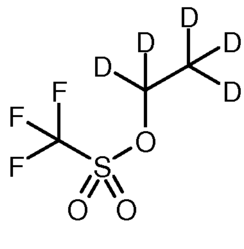

Nach der allgemeinen Methode 8A wurden 1.0 g (19.57 mmol) 1,1,2,2,2-Pentadeuteroethanol mit 3.48 ml (20.55 mmol, 1.05 eq.) Trifluormethansulfonsäureanhydrid in Gegenwart von 2.51 ml (21.53 mmol, 1.1 eq.) 2,6-Dimethylpyridin umgesetzt. Das Rohprodukt wurde ohne weitere Reinigung in der nächsten Stufe umgesetzt.

**Beispiel 59.1B**

2-[4-(5-Chlor-2-cyanophenyl)-5-methoxy-2-oxopyridin-1(2*H*)-yl](3,3,4,4,4-pentadeutero)-butansäure-*tert*.-butylester (Racemat)

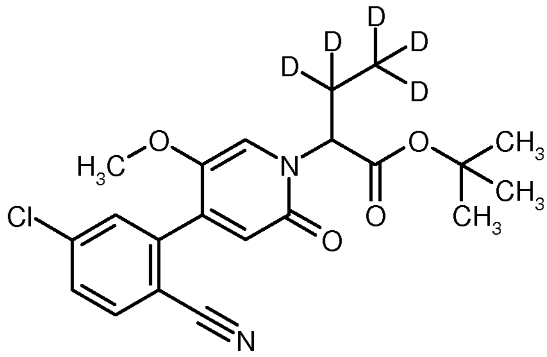

Nach der allgemeinen Methode 7B wurden 250 mg (0.67 mmol) [4-(5-Chlor-2-cyanophenyl)-5-methoxy-2-oxopyridin-1(2*H*)-yl]essigsäure-*tert*.-butylester in Gegenwart von 0.80 ml (0.80 mmol, 1.2 eq.) Bis-(trimethylsilyl)-lithiumamid (1M in THF) mit 183 mg (1.00 mmol, 1.5 eq.) 1,1,2,2,2-Pentadeuteroethyl-trifluormethansulfonat umgesetzt. Ausbeute: 206 mg (94% Reinheit, 71% d. Th.)
LC/MS [Methode 8]: $R_t$ = 1.37 min; MS (ESIpos): m/z = 352 (M+H-COO-*tert*.-butyl)$^+$,
$^1$H-NMR (400 MHz, DMSO-$d_6$): $\delta$ [ppm] = 7.99 (d, 1H), 7.76-7.69 (m, 2H), 7.36 (s, 1H), 6.50 (s, 1H), 5.01 (s, 1H), 3.63 (s, 3H), 1.40 (s, 9H).

**Beispiel 59.1C**

2-[4-(5-Chlor-2-cyanophenyl)-5-methoxy-2-oxopyridin-1(2*H*)-yl](3,3,4,4,4-pentadeutero)-butansäure (Racemat)

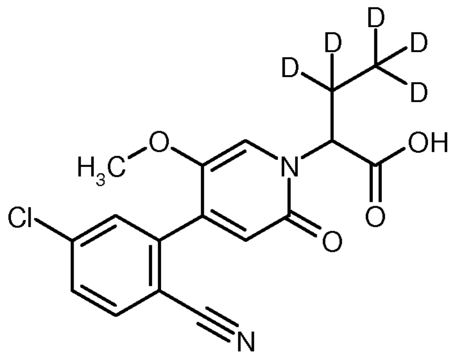

Nach der allgemeinen Methode 6A wurden 206 mg (94% Reinheit, 0.48 mmol) 2-[4-(5-Chlor-2-cyanophenyl)-5-methoxy-2-oxopyridin-1(2*H*)-yl](3,3,4,4,4-pentadeutero)butansäure-*tert*.-butylester (Racemat) mit TFA verseift. Ausbeute: 208 mg (71% Reinheit, 88% d. Th.)
LC/MS [Methode 1]: $R_t$ = 0.81 min; MS (ESIpos): m/z = 352 (M+H)$^+$.

**Beispiel 59.1D**

4-({2-[4-(5-Chlor-2-cyanophenyl)-5-methoxy-2-oxopyridin-1(2*H*)-yl](3,3,4,4,4-pentadeutero)-butanoyl}amino)benzoesäure-*tert*.-butylester (Racemat)

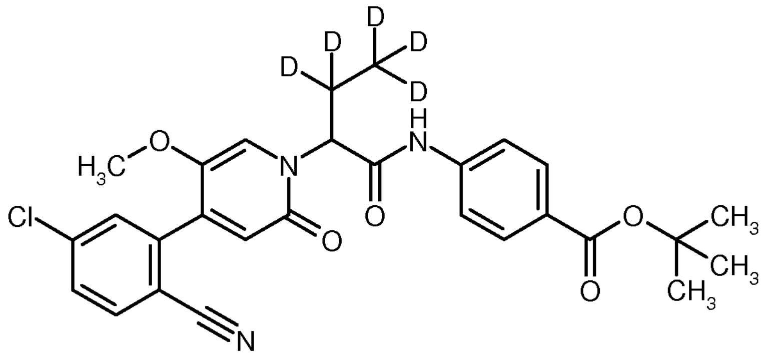

Nach der allgemeinen Methode 5A wurden 208 mg (71% Reinheit, 0.42 mmol) 2-[4-(5-Chlor-2-cyanophenyl)-5-methoxy-2-oxopyridin-1(2*H*)-yl](3,3,4,4,4-pentadeutero)butansäure (Racemat) mit 89 mg (0.46 mmol, 1.1 eq.) 4-Aminobenzoesäure-*tert*.-butylester umgesetzt. Ausbeute: 79 mg (91% Reinheit, 33% d. Th.)
LC/MS [Methode 1]: $R_t$ = 1.21 min; MS (ESIpos): m/z = 527 $(M+H)^+$.

**Beispiel 60.1A**

[1-(Trifluormethyl)cyclopropyl]methanol

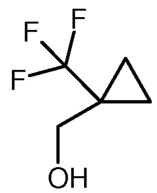

Bei 0°C wurden 26.7 ml (26.7 mmol) Di-*iso*-butylaluminium-hydrid (1M in Dichlormethan) zu einer Lösung von 1.89 g (10.7 mmol) 1-(Trifluormethyl)cyclopropan-carbonsäuremethylester in 10 ml Dichlormethan langsam zugetropft. Anschließend wurde für 2 h bei 0°C nachgerührt und dann die Reaktion durch die Zugabe von 10 ml Methanol beendet. Die Reaktionsmischung wurde mit 30 ml wässriger 20%iger Natrium/Kaliumtartrat-Lösung und 30 ml wässriger Puffer-Lösung (pH 7) verdünnt und über Nacht stark bei Raumtemperatur gerührt. Die Phasen wurden getrennt und die wässrige Phase dreimal mit Dichlormethan extrahiert. Die vereinigten organischen Phasen wurden über Magnesiumsulfat getrocknet, filtriert und das Lösungsmittel unter reduziertem Druck entfernt. Das Rohprodukt entsprach der Titelverbindung. Ausbeute: 0.96 g (64% d. Th.)
$^1$H-NMR (400 MHz, $CDCl_3$): δ [ppm] = 3.74 (d, 2H), 1.65 (t, 1H), 1.06-1.02 (m, 2H), 0.81-0.76 (m, 2H).

**Beispiel 60.1B**

[1-(Trifluormethyl)cyclopropyl]methyl-trifluormethansulfonat

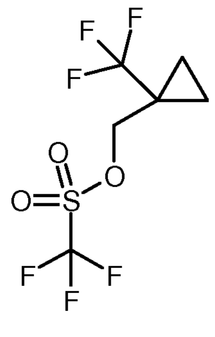

Zu 569 µl (3.36 mmol) Trifluormethansulfonsäureanhydrid in 1.5 ml Dichlormethan wurde bei -78°C eine Lösung aus 428 mg (3.06 mmol) [1-(Trifluormethyl)cyclopropyl]methanol und 468 µl (3.36 mmol) Triethylamin in 1.5 ml Dichlormethan getropft, so dass die interne Temperatur -50°C nicht übersteigt. Es wurde 30 min bei -78°C nachgerührt und spontan auf RT erwärmt. Die Reaktionsmischung wurde mit 25 ml Methyl-*tert*.-butylether verdünnt, dreimal mit 20 ml einer Mischung aus gesättigter, wässriger Natriumchlorid/1N Salzsäure (3:1) gewaschen, über Magnesiumsulfat getrocknet, filtriert und bei 25°C und ≥100 mbar Druck eingeengt. Das Rohprodukt wurde ohne weitere Aufreinigung im nächsten Schritt eingesetzt. Ausbeute: 701 mg (84% d. Th.)

**Beispiel** 60.1C

2-[4-(5-Chlor-2-cyanophenyl)-5-methoxy-2-oxopyridin-1(2*H*)-yl]-3-[1-(trifluormethyl)-cyclopropyl]-propansäure-*tert*.-butylester (Racemat)

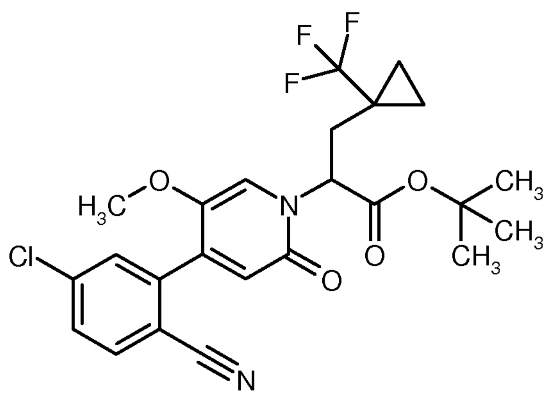

Gemäß allgemeiner Methode 7B wurden 500 mg (1.33 mmol) [4-(5-Chlor-2-cyanophenyl)-5-methoxy-2-oxopyridin-1(2*H*)-yllessigsäure-*tert*.-butylester, 701 mg (2.58 mmol) [1-(Trifluormethyl)-cyclopropyl]methyl-trifluormethansulfonat und 1.73 ml (1.73 mmol) Bis-(trimethylsilyl)-lithiumamid (1M in THF) in 13 ml THF zur Reaktion gebracht. Das Rohprodukt wurde säulenchromatographisch gereinigt (Essigsäureethylester-Cyclohexan-Gradient, 40 g Silica Kartusche, 40 ml/min Fluss) und die Titelverbindung erhalten. Ausbeute: 295 mg (45% d. Th.)
LC/MS [Methode 1]: $R_t$ = 1.20 min; MS (ESIpos): m/z = 497 $(M+H)^+$,
$^1$H-NMR (400 MHz, DMSO-$d_6$): δ [ppm] = 7.99 (d, 1H), 7.75-7.71 (m, 2H), 7.42 (s, 1H), 6.50 (s, 1H), 5.19-5.11 (m, 1H), 3.63 (s, 3H), 2.68 (dd, 1H), 2.33 (dd, 1H), 1.40 (s, 9H), 0.95-0.74 (m, 3H), 0.56-0.49 (m, 1H).

**Beispiel 60.1D**

2-[4-(5-Chlor-2-cyanophenyl)-5-methoxy-2-oxopyridin-1(2*H*)-yl]-3-[1-(trifluormethyl)-cyclopropyl]-propansäure (Racemat)

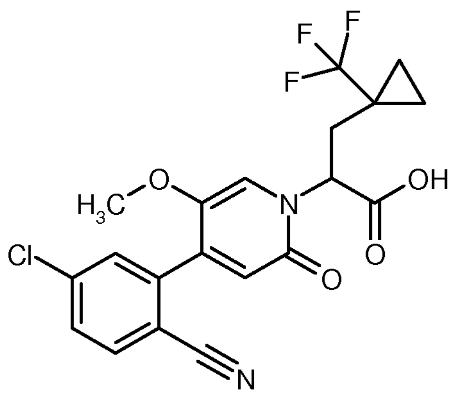

Gemäß allgemeiner Methode 6A wurden 295 mg (594 μmol) 2-[4-(5-Chlor-2-cyanophenyl)-5-methoxy-2-oxopyridin-1(2*H*)-yl]-3-[1-(trifluormethyl)cyclopropyl]-propansäure-*tert*.-butylester (Racemat) in 6 ml Dichlormethan mit 915 μl (11.9 mmol) TFA umgesetzt. Ausbeute: 258 mg (92% Reinheit, 91% d. Th.)
LC/MS [Methode 1]: $R_t$ = 0.96 min; MS (ESIneg): m/z = 439 $(M-H)^-$,
$^1$H-NMR (400 MHz, DMSO-$d_6$): δ [ppm] = 13.2 (br. s, 1H), 7.99 (d, 1H), 7.76-7.71 (m, 2H), 7.46 (s, 1H), 6.49 (s, 1H), 5.23-5.14 (m, 1H), 3.63 (s, 3H), 2.71 (dd, 1H), 2.37 (dd, 1H), 0.92-0.81 (m, 2H), 0.78-0.71 (m, 1H), 0.54-0.46 (m, 1H).

**Beispiel 60.1E**

4-({2-[4-(5-Chlor-2-cyanophenyl)-5-methoxy-2-oxopyridin-1(2*H*)-yl]-3-[l-(trifluormethyl)cyclopropyl]propanoyl}amino)benzoesäure-*tert*.-butylester (Racemat)

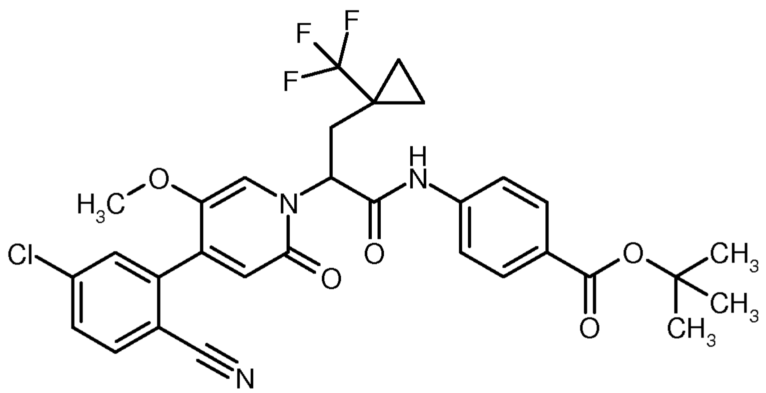

Gemäß allgemeiner Methode 5B wurden 188 mg (426 μmol) 2-[4-(5-Chlor-2-cyanophenyl)-5-methoxy-2-oxopyridin-1(2*H*)-yl]-3-[1-(trifluormethyl)cyclopropyl] -propansäure (Racemat), 82.4 mg (426 μmol) 4-Aminobenzoesäure-*tert*.-butylester, 6.1 mg (43 μmol) Oxima und 66 μl (0.43 mmol) DIC in 4 ml Dimethylformamid zur Reaktion gebracht. Nach präparativer HPLC [Säule: Chromatorex C18, 10 μm, 125 mm x 30 mm, Eluent: Acetonitril/0.1% Ameisensäure-Gradient (0 bis 3 min 10% Acetonitril, bis 35 min 90% Acetonitril und weitere 3 min 90% Acetonitril)] wurde die Titelverbindung erhalten. Ausbeute: 157 mg (59% d. Th.)
LC/MS [Methode 1]: $R_t$ = 1.35 min; MS (ESIpos): m/z = 616 $(M+H)^+$,
$^1$H-NMR (400 MHz, DMSO-$d_6$): δ [ppm] = 10.8 (s, 1H), 7.99 (d, 1H), 7.88 (d, 2H), 7.78-7.71 (m, 4H), 7.53 (s, 1H), 6.55 (s, 1H), 5.86-5.80 (m, 1H), 3.67 (s, 3H), 2.60-2.47 (m, 2H), 1.54 (s, 9H), 0.97-0.80 (m, 4H).

**Beispiel 61.1A**

[1-(Trifluormethyl)cyclobutyl]methyltrifluormethansulfonat

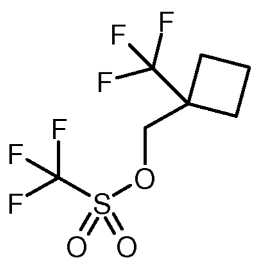

Nach der allgemeinen Methode 8A wurden 330 mg (2.03 mmol) [1-(Trifluormethyl)-cyclobutyl]methanol mit 0.38 ml (2.24 mmol, 1.1 eq.) Trifluormethansulfonsäureanhydrid in Gegenwart von 312 μl (2.24 mmol, 1.1 eq.) Triethylamin umgesetzt. Das Rohprodukt wurde ohne weitere Reinigung in der nächsten Stufe umgesetzt.
$^1$H-NMR (400 MHz, DMSO-$d_6$): δ [ppm] = 4.56 (s, 2H), 2.28-2.19 (m, 2H), 2.11-1.89 (m, 4H).

**Beispiel 61.1B**

2-[4-(5-Chlor-2-cyanophenyl)-5-methoxy-2-oxopyridin-1(2*H*)-yl]-3-[1-(trifluormethyl)cyclobutyl]-propansäure-*tert.*-butylester (Racemat)

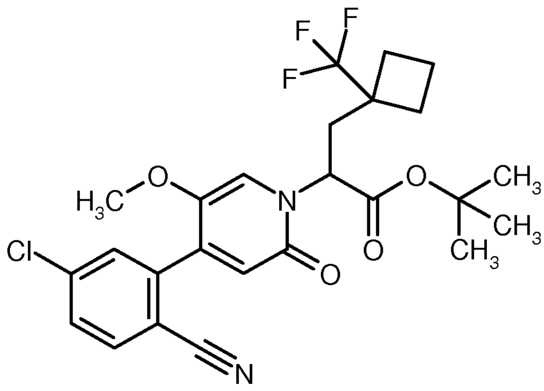

Nach der allgemeinen Methode 7B wurden 383 mg (1.02 mmol) [4-(5-Chlor-2-cyanophenyl)-5-methoxy-2-oxopyridin-1(2*H*)-yl]essigsäure-*tert.*-butylester in Gegenwart von 1.23 ml (1.23 mmol, 1.2 eq.) Bis-(trimethylsilyl)-lithiumamid (1M in THF) mit 780 mg (60% Reinheit, 1.64 mmol, 1.6 eq.) [1-(Trifluormethyl)cyclobutyl]methyltrifluormethansulfonat umgesetzt. Ausbeute: 119 mg (91% Reinheit, 21% d. Th.)
LC/MS [Methode 8]: $R_t$ = 1.54 min; MS (ESIpos): m/z = 455 (M+H-COO-*tert.*-butyl)$^+$.

**Beispiel 61.1C**

2-[4-(5-Chlor-2-cyanophenyl)-5-methoxy-2-oxopyridin-1(2*H*)-yl]-3-[1-(trifluormethyl)cyclobutyl]propansäure (Racemat)

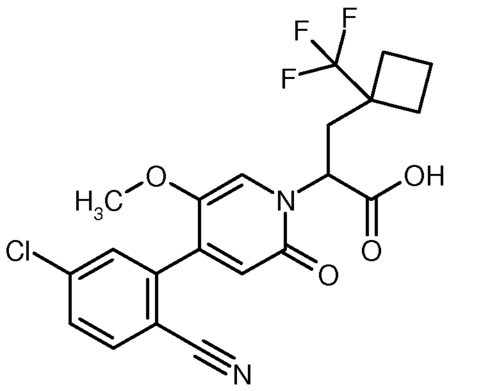

Nach der allgemeinen Methode 6A wurden 119 mg (91% Reinheit, 0.21 mmol) 2-[4-(5-Chlor-2-cyanophenyl)-5-methoxy-2-oxopyridin-1(2*H*)-yl]-3-[1-(trifluormethyl)cyclobutyl]propansäure-*tert.*-butylester (Racemat) mit TFA verseift. Ausbeute: 91 mg (69% Reinheit, 65% d. Th.)
LC/MS [Methode 1]: $R_t$ = 1.01 min; MS (ESIpos): m/z = 455 (M+H)$^+$.

**Beispiel 61.1D**

4-({2-[4-(5-Chlor-2-cyanophenyl)-5-methoxy-2-oxopyridin-1(2*H*)-yl]-3-[1-(trifluormethyl)-cyclobutyl]propanoyl}amino)benzoesäure-*tert.*-butylester (Racemat)

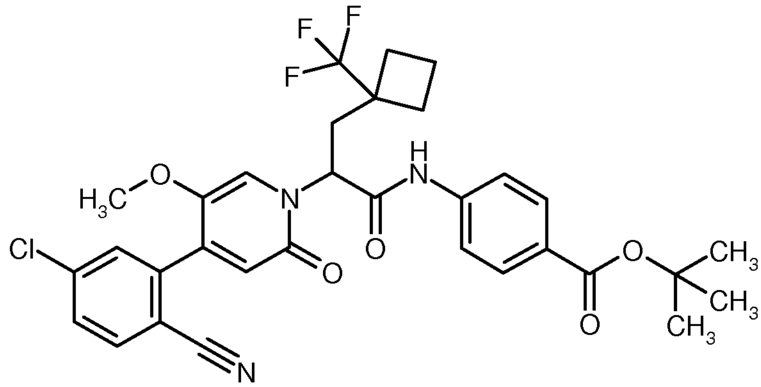

Nach der allgemeinen Methode 5A wurden 90 mg (69% Reinheit, 0.14 mmol) 2-[4-(5-Chlor-2-cyanophenyl)-5-methoxy-2-oxopyridin-1(2*H*)-yl]-3-[1-(trifluormethyl)cyclo-butyl]propansäure (Racemat) mit 29 mg (0.15 mmol, 1.1 eq.) 4-Aminobenzoesäure-*tert*.-butylester umgesetzt. Ausbeute: 58 mg (67% d. Th.)
LC/MS [Methode 8]: $R_t$ = 1.65 min; MS (ESIneg): m/z = 628 $(M-H)^-$.

**Beispiel 62.1A**

(3,3-Difluorcyclobutyl)methyltrifluormethansulfonat

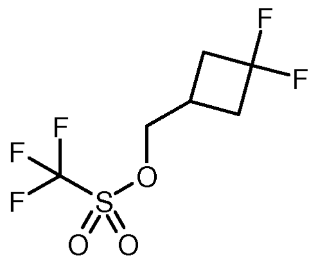

Nach der allgemeinen Methode 8A wurden 500 mg (4.09 mmol) (3,3-Difluorcyclobutyl)methanol mit 0.76 ml (4.50 mmol, 1.1 eq.) Trifluormethansulfonsäureanhydrid in Gegenwart von 628 µl (4.50 mmol, 1.1 eq.) Triethylamin umgesetzt. Das Rohprodukt wurde ohne weitere Reinigung in der nächsten Stufe umgesetzt.
$^1$H-NMR (400 MHz, DMSO-$d_6$): δ [ppm] = 4.33 (d, 2H), 2.76-2.61 (m, 2H), 2.60-2.50 (m, 1H), 2.50-2.37 (m, 2H).

**Beispiel 62.1B**

2-[4-(5-Chlor-2-cyanophenyl)-5-methoxy-2-oxopyridin-1(2*H*)-yl]-3-(3,3-difluorcyclobutyl)-propansäure-tert. -butylester (Racemat)

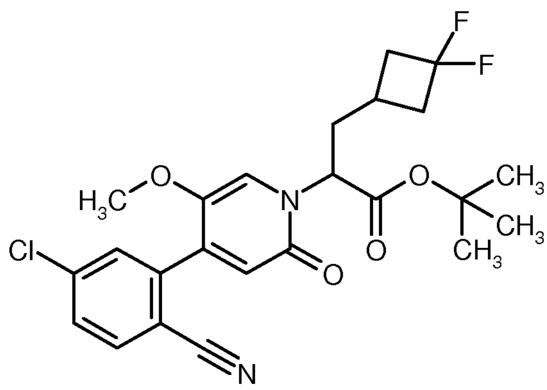

Nach der allgemeinen Methode 7B wurden 600 mg (1.60 mmol) [4-(5-Chlor-2-cyanophenyl)-5-methoxy-2-oxopyridin-1(2*H*)-yl]essigsäure-*tert*.-butylester in Gegenwart von 1.92 ml (1.92 mmol, 1.2 eq.) Bis-(trimethylsilyl)-lithiumamid (1M in THF) mit 1162 mg (70% Reinheit, 3.20 mmol, 2.0 eq.) (3,3-Difluorcyclobutyl)methyltrifluormethansulfonat umgesetzt. Ausbeute: 417 mg (52% d. Th.)
LC/MS [Methode 8]: $R_t$ = 1.44 min; MS (ESIpos): m/z = 423 (M+H-COO-*tert*.-butyl)$^+$.

**Beispiel 62.1C**

2-[4-(5-Chlor-2-cyanophenyl)-5-methoxy-2-oxopyridin-1(2*H*)-yl]-3-(3,3-difluorcyclobutyl)-propansäure (Racemat)

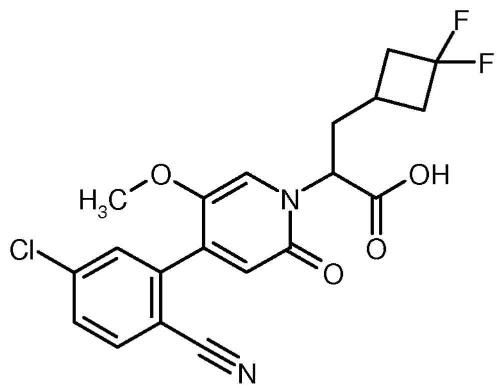

Nach der allgemeinen Methode 6A wurden 184 mg (0.37 mmol) 2-[4-(5-Chlor-2-cyanophenyl)-5-methoxy-2-oxopyridin-1(2*H*)-yl]-3-(3,3-difluorcyclobutyl)propansäure-*tert*.-butylester (Racemat) mit TFA verseift. Ausbeute: 200 mg (88% Reinheit, quant.)
LC/MS [Methode 1]: $R_t$ = 0.93 min; MS (ESIpos): m/z = 423 (M+H)$^+$.

**Beispiel 62.1D**

4-([2-[4-(5-Chlor-2-cyanophenyl)-5-methoxy-2-oxopyridin-1(2*H*)-yl]-3-(3,3-difluorcyclobutyl)-propanoyl}amino)benzoesäure-*tert*.-butylester (Racemat)

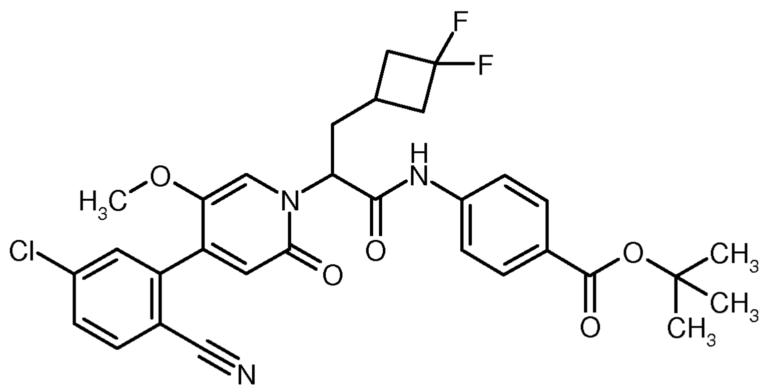

Nach der allgemeinen Methode 5A wurden 200 mg (88% Reinheit, 0.42 mmol) 2-{4-[5-Chlor-2-(difluormethoxy)phenyl]-5-methoxy-2-oxopyridin-1(2*H*)-yl}-4-methylpropansäure (Racemat) mit 89 mg (0.46 mmol, 1.1 eq.) 4-Aminobenzoesäure-*tert.*-butylester umgesetzt. Ausbeute: 175 mg (70% d. Th.)
LC/MS [Methode 1]: $R_t$ = 1.26 min; MS (ESIpos): m/z = 598 $(M+H)^+$.

**Beispiel 63.1A**

3-Methyloxetan-3-carbaldehyd

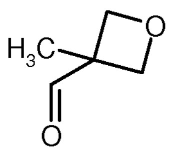

6.75 g (31.3 mmol) Pyridiniumchlorchromat wurde in 100 ml Dichlormethan vorlegt und bei Raumtemperatur mit einer Lösung von 2.00 g (19.6 mmol) (3-Methyloxetan-3-yl)methanol in 20 ml Dichlormethan versetzt. Anschließend wurden 7 g Celite® hinzugegeben und für 4 h bei Raumtemperatur nachgerührt. Es wurde über Kieselgel abgesaugt und das Lösungsmittel unter reduziertem Druck bei Raumtemperatur entfernt. Das Rohprodukt wurde ohne weitere Aufreinigung in der nächsten Stufe eingesetzt. Ausbeute: 1.55 g (79% d. Th.)
$^1$H-NMR (400 MHz, $CDCl_3$): δ [ppm] = 9.95 (s, 1H), 4.89 (d, 2H), 4.51 (d, 2H), 1.48 (s, 3H).

**Beispiel 63.1B**

2-[4-(5-Chlor-2-cyanophenyl)-5-methoxy-2-oxopyridin-1(2*H*)-yl]-3-(3-methyloxetan-3-yl)prop-2-ensäure-tert. -butylester (Isomerengemisch)

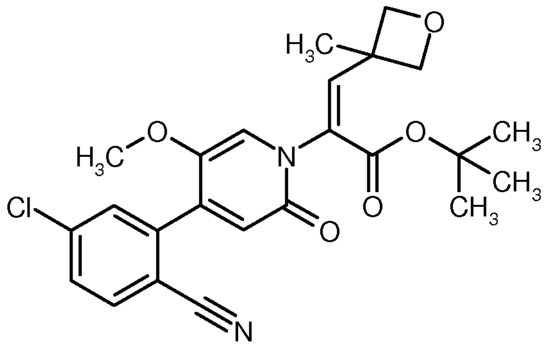

Eine Lösung von 300 mg (800 µmol))[4-(5-Chlor-2-cyanophenyl)-5-methoxy-2-oxopyridin-1(2*H*)-yl]essigsäure-*tert*.-butylester in 9 ml Dimethylformamid wurde nacheinander mit 64.0 mg (1.60 mmol, 60% in Mineralöl) Natriumhydrid und 200 mg (2.00 mmol) 3-Methyloxetan-3-carbaldehyd in 1 ml Dimethylformamid versetzt. Nach 15 min bei Raumtemperatur wurde die Reaktion durch Zugabe von gesättigter, wässriger Ammoniumchlorid-Lösung beendet und das Reaktionsgemisch anschließend dreimal mit 20 ml Essigsäureethylester extrahiert. Die vereinigten, organischen Phasen wurden über Magnesiumsulfat getrocknet, filtriert und das Lösungsmittel unter reduziertem Druck entfernt. Der Rückstand wurde mittels Flash-Chromatographie (24 g Silica Kartusche, 35 ml/min Fluss, Cyclohexan/Essigsäureethylester-Gradient) gereinigt. Ausbeute: 356 mg (96% d. Th.)
LC/MS [Methode 1]: $R_t$ = 1.03 min; MS (ESIpos): m/z = 457 $(M+H)^+$.

**Beispiel 63.1C**

2-[4-(5-Chlor-2-cyanophenyl)-5-methoxy-2-oxopyridin-1(2*H*)-yl]-3-(3-methyloxetan-3-yl)propansäure-*tert*.-butylester (Racemat)

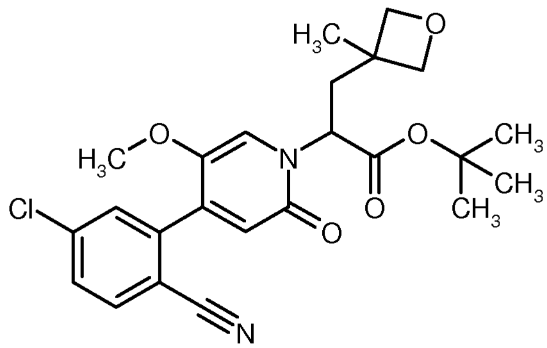

350 mg (766 µmol) 2-[4-(5-Chlor-2-cyanophenyl)-5-methoxy-2-oxopyridin-1(2*H*)-yl]-3-(3-methyloxetan-3-yl)prop-2-ensäure-*tert*.-butylester (Isomerengemisch) wurden bei Raumtemperatur mit 20 ml einer "Hot Stryker's"-Reagenzlösung [B. A. Baker et al. Org. Lett. 2008, 10, 289-292] versetzt. Das Reaktionsgemisch wurde für 1 h bei Raumtemperatur gerührt und anschließend mit gesättigter, wässriger Ammoniumchlorid-Lösung versetzt. Nach Phasentrennung wurde die wässrige Phase dreimal mit Essigsäureethylester extrahiert. Die vereinigten, organischen Phasen wurden über Magnesiumsulfat getrocknet, filtriert und das Lösungsmittel unter reduziertem Druck entfernt. Der Rückstand wurde mittels Flash-Chromatographie (40 g Silica Kartusche, 40 ml/min Fluss, Cyclohexan/Essigsäureethylester-Gradient) aufgereinigt. Ausbeute: 345 mg (97% d. Th.)
LC/MS [Methode 1]: $R_t$ = 1.03 min; MS (ESIpos): m/z = 459 $(M+H)^+$,
$^1$H-NMR (400 MHz, DMSO-$d_6$): δ [ppm] = 7.98 (d, 1H), 7.75-7.70 (m, 2H), 7.47 (s, 1H), 6.50 (s, 1H), 5.25-5.18 (m, 1H), 4.38 (d, 1H), 4.16 (d, 1H), 4.07 (d, 1H), 3.92 (d, 1H), 3.65 (s, 3H), 2.67 (dd, 1H), 2.31 (dd, 1H), 1.41 (s, 9H), 1.35 (s, 3H).

**Beispiel 63.1D**

2-[4-(5-Chlor-2-cyanophenyl)-5-methoxy-2-oxopyridin-1(2*H*)-yl]-3-(3-methyloxetan-3-yl)propansäure (Racemat)

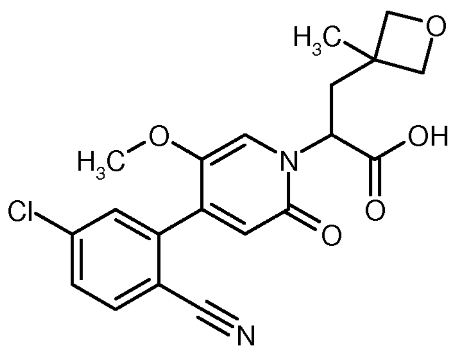

340 mg (741 µmol) 2-[4-(5-Chlor-2-cyanophenyl)-5-methoxy-2-oxopyridin-1(2*H*)-yl]-3-(3-methyloxetan-3-yl)propan-säure-*tert*.-butylester (Racemat) wurden in 5 ml Tetrahydrofuran, 2.5 ml Ethanol und 2.5 ml Wasser gelöst und mit 3.7 ml (3.7 mmol, 5 eq.) wässriger Lithiumhydroxid-Lösung (1M) versetzt. Es wurde für 7 h bei Raumtemperatur nachgerührt und anschließend 20 ml gesättigter wässriger Ammoniumchlorid-Lösung und 30 ml Essigsäureethylester verdünnt und mit wässriger Salzsäure (1N) auf pH 4-5 eingestellt. Die Phasen wurden getrennt und die wässrige Phase dreimal mit 10 ml Essigsäureethylester extrahiert. Die vereinigten, organischen Phasen wurden über Magnesiumsulfat getrocknet, filtriert und das Lösungsmittel unter reduziertem Druck entfernt. Das Rohprodukt wurde ohne weitere Reinigung im nächsten Schritt eingesetzt. Ausbeute: 275 mg (95% Reinheit, 88% d. Th.)
LC/MS [Methode 1]: $R_t$ = 0.78 min; MS (ESIneg): m/z = 401 $(M-H)^-$,
$^1$H-NMR (400 MHz, DMSO-$d_6$): δ [ppm] = 13.2 (br. s, 1H), 7.99 (d, 1H), 7.77-7.71 (m, 2H), 7.53 (s, 1H), 6.49 (s, 1H), 5.31-5.22 (m, 1H), 4.37 (d, 1H), 4.16 (d, 1H), 4.05 (d, 1H), 3.90 (d, 1H), 3.65 (s, 3H), 2.71 (dd, 1H), 2.33 (dd, 1H), 1.35 (s, 3H).

**Beispiel 63.1E**

4-({2-[4-(5-Chlor-2-cyanophenyl)-5-methoxy-2-oxopyridin-1(2*H*)-yl]-3-(3-methyloxetan-3-yl)propanoyl}amino)benzoesäureallylester (Racemat)

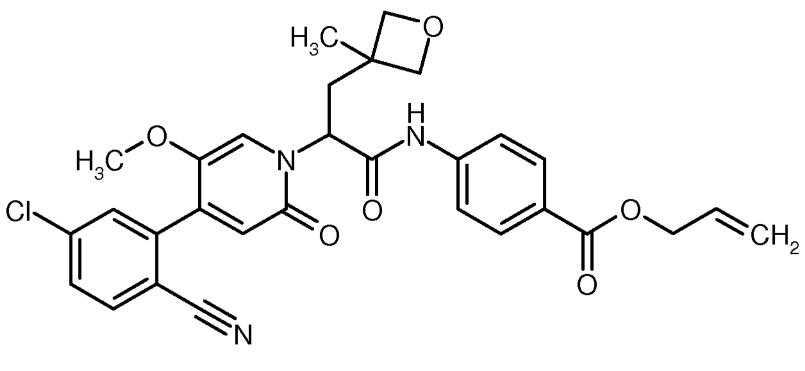

Gemäß allgemeiner Methode 5B wurden 115 mg (285 µmol) 2-[4-(5-Chlor-2-cyanophenyl)-5-methoxy-2-oxopyridin-1(2*H*)-yl]-3-(3-methyloxetan-3-yl)propansäure (Racemat), 51 mg (285 µmol) 4-Aminobenzoesäureallylester, 40.6 mg (285 µmol) Oxima und 45 µl (0.29 mmol) DIC in 2 ml Dimethylformamid zur Reaktion gebracht. Nach präparativer HPLC (Acetonitril-Wasser-Gradient) wurde die Titelverbindung erhalten. Ausbeute: 25 mg (16% d. Th.)
LC/MS [Methode 1]: $R_t$ = 1.06 min; MS (ESIpos): m/z = 562 $(M+H)^+$,
$^1$H-NMR (400 MHz, DMSO-$d_6$): δ [ppm] = 10.9 (s, 1H), 8.01-7.96 (m, 3H), 7.80 (d, 2H), 7.74-7.71 (m, 2H), 7.65 (s, 1H), 6.53 (s, 1H), 6.09-5.99 (m, 1H), 5.87 (dd, 1H), 5.43-5.37 (m, 1H), 5.30-5.25 (m, 1H), 4.80-4.77 (m, 2H), 4.46 (d, 1H), 4.29 (d, 1H), 4.11 (d, 1H), 3.95 (d, 1H), 3.72 (s, 3H), 2.74-2.65 (m, 1H), 2.40-2.33 (m, 1H), 1.38 (s, 3H).

**Beispiel 64.1A**

4-Methyltetrahydro-2*H*-pyran-4-carbonsäuremethylester

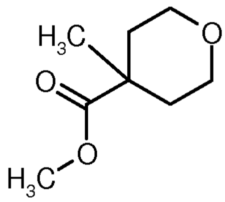

Bei -78°C wurden 22.1 ml *n*-Butyllithium (35.4 mmol, 1.6M in Hexan) zu einer Lösung von 4.91ml (35.0 mmol) Di-*iso*-propylamin in 45 ml Tetrahydrofuran langsam zugetropft und für 10 min bei -78°C und 25 min bei 0°C nachgerührt. Anschließend wurde bei -78°C eine Lösung von 5.00 g (34.7 mmol) Tetrahydro-2*H*-pyran-4-carbonsäuremethylester in 45 ml Tetrahydrofuran zugegeben und für 15 min bei -78°C und 30 min bei 0°C nachgerührt. Bei -78°C wurden 2.16 ml (34.7 mmol) Methyliodid tropfenweise hinzugefügt und die Reaktionsmischung langsam auf -25°C und dann über Nacht auf Raumtemperatur erwärmt. Die Reaktion wurde durch Zugabe von 80 ml 0.1N Salzsäure beendet und die Phasen getrennt. Die wässrige Phase wurde zweimal mit Essigsäureethylester extrahiert und die vereinigten organischen Phasen über Magnesiumsulfat getrocknet, filtriert und das Lösungsmittel unter reduziertem Druck entfernt. Die erhaltene Suspension wurde mit 20 ml Methyl-*tert*.-butylether verrührt, der Niederschlag abgesaugt, die Mutterlauge unter reduziertem Druck eingeengt und die Titelverbindung erhalten. Ausbeute: 5.88 g (95% Reinheit, quant.)
$^1$H-NMR (400 MHz, DMSO-$d_6$): δ [ppm] = 3.70-3.64 (m, 2H), 3.64 (s, 3H), 3.37-3.30 (m, 2H), 1.94-1.88 (m, 2H), 1.45-1.37 (m, 2H), 1.16 (s, 3H).

**Beispiel 64.1B**

(4-Methyltetrahydro-2*H*-pyran-4-yl)methanol

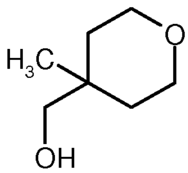

Bei -78°C wurde zu einer Lösung von 5.80 g (36.7 mmol) 4-Methyltetrahydro-2*H*-pyran-4-carbonsäuremethylester in 220 ml Toluol 73.3 ml (73.3 mmol, 1.0M in Toluol) Di-*iso*butylaluminiumhydrid so zugetropft, dass die interne Temperatur -70°C nicht überstieg. Anschließend wurde für 90 min bei -78°C und über Nacht bei Raumtemperatur nachgerührt. Die Reaktion wurde durch Zugabe von 40 ml Methanol und 300 ml 1N Salzsäure beendet. Die Phasen wurden getrennt und die wässrige Phase dreimal mit 150 ml Essigsäureethylester extrahiert. Die vereinigten organischen Phasen wurden mit gesättigter, wässriger Natriumchlorid-Lösung gewaschen, über Magnesiumsulfat getrocknet, filtriert und das Lösungsmittel unter reduziertem Druck entfernt. Das Rohprodukt entsprach der Titelverbindung. Ausbeute: 2.14 g (45% d. Th.)
$^1$H-NMR (400 MHz, $CDCl_3$): δ [ppm] = 3.78-3.73 (m, 2H), 3.66-3.60 (m, 2H), 3.41 (s, 2H), 1.62-1.54 (m, 2H), 1.43 (br. s, 1H), 1.32-1.26 (m, 2H), 1.04 (s, 3H).

**Beispiel 64.1C**

(4-Methyltetrahydro-2*H*-pyran-4-yl)methyl-trifluormethansulfonat

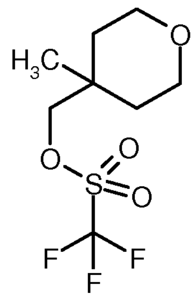

Zu 1.42 ml (8.45 mmol) Trifluormethansulfonsäureanhydrid in 5 ml Dichlormethan wurde bei -78°C eine Lösung aus 1.00 g (7.68 mmol) (4-Methyltetrahydro-2*H*-pyran-4-yl)methanol und 1.18 ml (8.45 mmol) Triethylamin in 5 ml Dichlormethan getropft, so dass die interne Temperatur -50°C nicht übersteigt. Es wurde 15 min bei -78°C nachgerührt und spontan auf RT erwärmt. Die Reaktionsmischung wurde mit 50 ml Methyl-*tert*.-butylether verdünnt, dreimal mit 25 ml einer Mischung aus gesättigter, wässriger Natriumchlorid/1N Salzsäure (3:1) gewaschen, über Magnesiumsulfat getrocknet, filtriert und bei 25°C und ≥100 mbar Druck eingeengt. Ausbeute: 2.0 g (99% d. Th.)
$^1$H-NMR (400 MHz, $CDCl_3$): δ [ppm] = 4.26 (s, 2H), 3.80-3.74 (m, 2H), 3.67-3.60 (m, 2H), 1.66-1.58 (m, 2H), 1.42-1.36 (m, 2H), 1.16 (s, 3H).

**Beispiel 64.1D**

2-[4-(5-Chlor-2-cyanophenyl)-5-methoxy-2-oxopyridin-1(2*H*)-yl]-3-(4-methyltetrahydro-2*H*-pyran-4-yl)propansäure-tert.-butylester (Racemat)

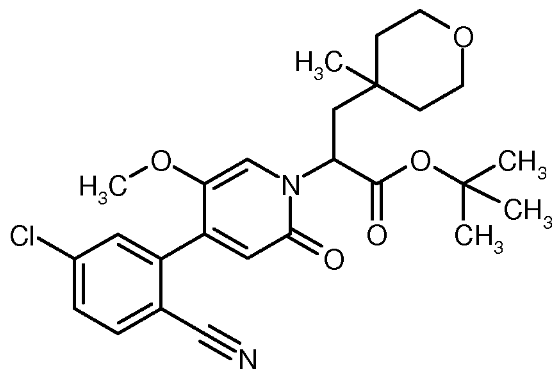

Gemäß allgemeiner Methode 7B wurden 1.91 g (5.08 mmol) [4-(5-Chlor-2-cyanophenyl)-5-methoxy-2-oxopyridin-1(2*H*)-yl]essigsäure-*tert*.-butylester, 2.00 g (7.63 mmol) (4-Methyltetrahydro-2*H*-pyran-4-yl)methyl-trifluormethansulfonat und 6.61 ml (6.61 mmol) Bis-(trimethylsilyl)-lithiumamid (1M in THF) in 10 ml THF zur Reaktion gebracht. Nach Reinigung mittels präparativer HPLC (Acetonitril-Wasser-Gradient) wurde die Titelverbindung erhalten. Ausbeute: 212 mg (8% d. Th.)
LC/MS [Methode 1]: $R_t$ = 1.10 min; MS (ESIpos): m/z = 487 $(M+H)^+$,
$^1$H-NMR (400 MHz, DMSO-$d_6$): δ [ppm] = 8.00-7.96 (m, 1H), 7.74-7.70 (m, 2H), 7.50 (s, 1H), 6.48 (s, 1H), 5.48-5.41 (m, 1H), 3.66 (s, 3H), 3.55-3.42 (m, 4H), 2.30-2.22 (m, 1H), 2.15-2.09 (m, 1H), 1.49-1.10 (m, 4H), 1.41 (s, 9H), 0.95 (s, 3H).

**Beispiel 64.1E**

2-[4-(5-Chlor-2-cyanophenyl)-5-methoxy-2-oxopyridin-1(2*H*)-yl]-3-(4-methyltetrahydro-2*H*-pyran-4-yl)propansäure (Racemat)

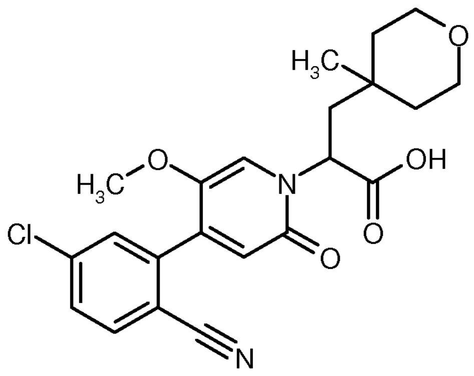

Gemäß allgemeiner Methode 6A wurden 210 mg (431 µmol) 2-[4-(5-Chlor-2-cyanophenyl)-5-methoxy-2-oxopyridin-1(2*H*)-yl]-3-(4-methyltetrahydro-2*H*-pyran-4-yl)propansäure-*tert.*-butylester (Racemat) in 4.3 ml Dichlormethan mit 598 µl (7.76 mmol) TFA umgesetzt. Ausbeute: 210 mg (83% Reinheit, 94% d. Th.)
LC/MS [Methode 1]: $R_t$ = 0.86 min; MS (ESIneg): m/z = 429 $(M-H)^-$,
$^1$H-NMR (400 MHz, DMSO-$d_6$): δ [ppm] = 13.2 (br. s, 1H), 7.98 (d, 1H), 7.75-7.70 (m, 2H), 7.55 (s, 1H), 6.48 (s, 1H), 5.58-5.43 (m, 1H), 3.65 (s, 3H), 3.55-3.38 (m, 4H), 2.37-2.31 (m, 1H), 2.18-2.12 (m, 1H), 1.48-1.41 (m, 1H), 1.35-1.28 (m, 1H), 1.27-1.19 (m, 1H), 1.13-1.06 (m, 1H), 0.96 (s, 3H).

**Beispiel 64.1F**

4-({2-[4-(5-Chlor-2-cyanophenyl)-5-methoxy-2-oxopyridin-1(2*H*)-yl]-3-(4-methyltetrahydro-2*H*-pyran-4-yl)propanoyl}amino)benzoesäureethylester (Racemat)

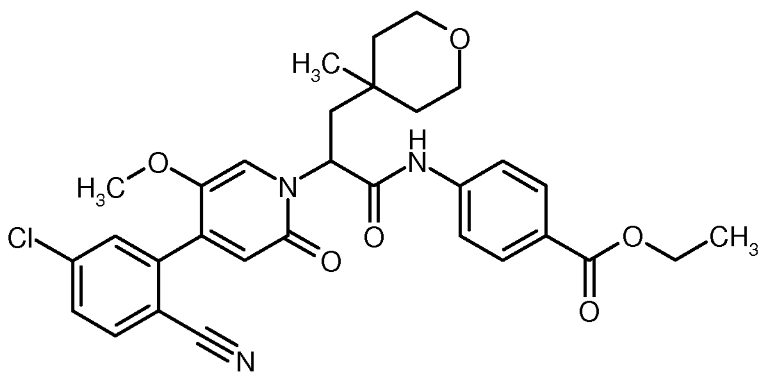

Gemäß allgemeiner Methode 5B wurden 206 mg (478 µmol) 2-[4-(5-Chlor-2-cyanophenyl)-5-methoxy-2-oxopyridin-1(2*H*)-yl]-3-(4-methyltetrahydro-2*H*-pyran-4-yl)propansäure (Racemat), 79 mg (0.48 mmol) 4-Aminobenzoesäureethylester, 6.8 mg (48 µmol) Oxima und 74 µl (0.48 mmol) DIC in 4.8 ml Dimethylformamid zur Reaktion gebracht. Nach präparativer HPLC (Acetonitril-Wasser-Gradient) wurde die Titelverbindung erhalten. Ausbeute: 70 mg (95% Reinheit, 24% d. Th.)
LC/MS [Methode 1]: $R_t$ = 1.14 min; MS (ESIpos): m/z = 578 $(M+H)^+$,
$^1$H-NMR (400 MHz, DMSO-$d_6$): δ [ppm] = 10.9 (s, 1H), 7.98 (d, 1H), 7.94 (d, 2H), 7.80 (d, 2H), 7.75-7.71 (m, 2H), 7.66 (s, 1H), 6.52 (s, 1H), 6.05 (dd, 1H), 4.29 (q, 2H), 3.71 (s, 3H), 3.65-3.39 (m, 4H), 2.34-2.27 (m, 1H), 2.07-2.00 (m, 1H), 1.57-1.48 (m, 1H), 1.40-1.25 (m, 2H), 1.31 (t, 3H), 1.16-1.09 (m, 1H), 1.03 (s, 3H).

**Beispiel 65.1A**

4-Methoxycyclohexancarbonsäuremethylester (*cis*/*trans*-Isomerengemisch)

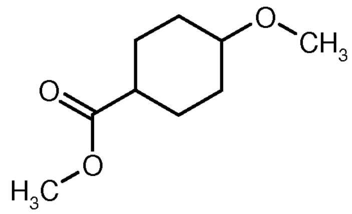

Zu einer Lösung von 4.00 g (25.3 mmol) 4-Methoxycyclohexancarbonsäure in 32 ml Methanol wurde 1.8 ml konzentrierte Schwefelsäure gegeben und die resultierende Reaktionslösung über Nacht unter Rückfluss erhitzt. Es wurde auf Raumtemperatur abgekühlt und der pH-Wert auf 7-8 mit Hilfe von gesättigter, wässriger Natriumhydrogencarbonat-Lösung eingestellt. Anschließend wurde dreimal mit 70 ml Essigsäureethylester extrahiert. Die vereinigten organischen Phasen wurden über Natriumsulfat getrocknet, filtriert und das Lösungsmittel unter reduziertem Druck entfernt. Das Rohprodukt entsprach der Titelverbindung. Ausbeute: 4.30 g (99% d. Th.)
GC/MS [Methode 9]: $R_t$ = 3.65 min; MS: m/z = 172 $(M)^+$
$^1$H-NMR (400 MHz, DMSO-$d_6$): δ [ppm] = 3.58 (s, 3H), 3.33-3.29/3.11-3.04 (2x m, 1H), 3.22/3.19 (2x s, 3H), 2.42-2.35/2.31-2.23 (2x m, 1H), 2.00-1.93/1.92-1.85 (2x m, 1H), 1.73-1.61 (m, 3H), 1.58-1.43 (m, 3H), 1.41-1.30/1.19-1.08 (2x m, 1H).

**Beispiel 65.1B**

(4-Methoxycyclohexyl)methanol (*cis*/*trans*-Isomerengemisch)

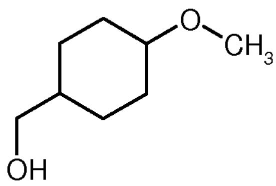

13.7 ml (27.5 mmol) Lithiumaluminiumhydrid-Lösung (2M in THF) wurde mit 82 ml Methyl-*tert.*-butylether verdünnt und anschließend tropfenweise mit einer Lösung von 4.30 g (25.0 mmol) 4-Methoxycyclohexancarbonsäuremethylester (*cis*/*trans*-Isomerengemisch) in 82 ml Methyl-*tert.*-butylether versetzt. Die Reaktionslösung wurde für 6 h bei 40°C nachgerührt und die Reaktion im Anschluss durch Zugabe von 10 ml Wasser und 10 ml wässriger 10%iger Kaliumhydroxid-Lösung beendet. Die organische Phase wurde dekantiert, über Natriumsulfat getrocknet, filtriert und das Lösungsmittel unter reduziertem Druck entfernt. Ausbeute: 3.24 g (90% d. Th.)
GC/MS [Methode 9]: $R_t$ = 3.36/3.47 min; MS: m/z = 144 $(M)^+$
$^1$H-NMR (400 MHz, $CDCl_3$): δ [ppm] = 3.48/3.46 (2x d, 2H), 3.45-3.42/3.13-3.06 (2x m, 1H), 3.35/3.30 (2x s, 3H), 2.13-1.82 (m, 2H), 1.65 (br. s, 1H), 1.59-1.29 (m, 6H), 1.24-1.15/1.03-0.95 (2x m, 1H).

**Beispiel 65.1C**

(4-Methoxycyclohexyl)methyl-trifluormethansulfonat (*cis*/*trans*-Isomerengemisch)

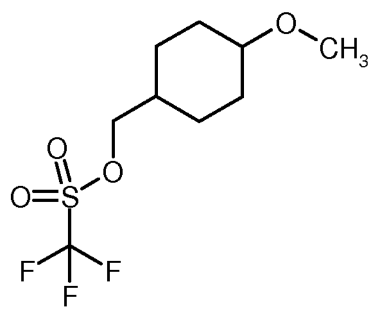

Gemäß allgemeiner Methode 8A wurden 4.30 g (29.8 mmol) (4-Methoxycyclohexyl)methanol (*cis*/*trans*-Isomerengemisch) in 158 ml Dichlormethan mit 5.21 ml (44.7 mmol) Lutidin und 7.57 ml (44.7 mmol) Trifluormethansulfonsäureanhydrid umgesetzt. Ausbeute: 6.00 g (73% d. Th.) Das Rohprodukt wurde ohne weitere Aufreinigung im nächsten Schritt eingesetzt.

**Beispiel 65.1D**

2-[4-(5-Chlor-2-cyanophenyl)-5-methoxy-2-oxopyridin-1(2*H*)-yl]-3-(4-methoxycyclohexyl)-propansäure-*tert*.-butylester (racemisches *cis*/*trans*-Isomerengemisch)

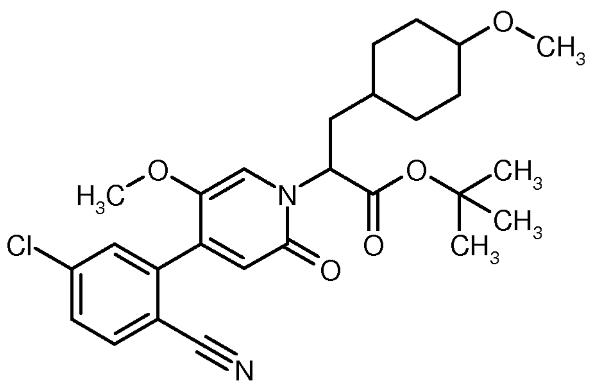

Gemäß allgemeiner Methode 7B wurden 5.40 g (14.4 mmol) [4-(5-Chlor-2-cyanophenyl)-5-methoxy-2-oxopyridin-1(2*H*)-yl]essigsäure-*tert*.-butylester, 5.97 g (21.6 mmol) (4-Methoxycyclohexyl)methyl-trifluormethansulfonat (*cis*/*trans*-Isomerengemisch) und 15.8 ml (15.8 mmol) Bis-(trimethylsilyl)-lithiumamid (1M in THF) in 114 ml THF zur Reaktion gebracht. Ausbeute: 10.8 g (51% Reinheit) Rohprodukt
LC/MS [Methode 1]: $R_t$ = 1.20/1.23 min; MS (ESIpos): m/z = 501 $(M+H)^+$.

**Beispiel 65.1E**

2-[4-(5-Chlor-2-cyanophenyl)-5-methoxy-2-oxopyridin-1(2*H*)-yl]-3-(*cis*-4-methoxycyclohexyl)-propansäure-*tert*.-butylester (Racemat)

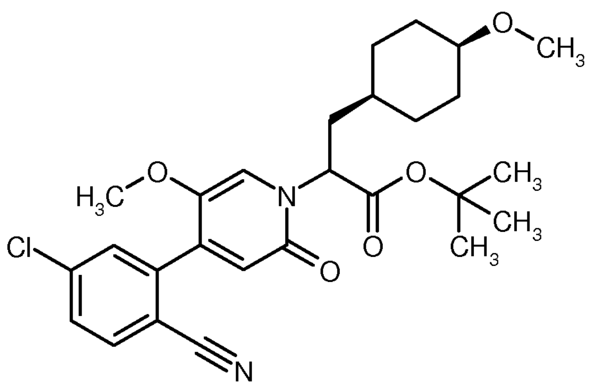

Das Rohprodukt aus Beispiel 65.1D wurde säulenchromatographisch gereinigt (Essigsäureethylester-Cyclohexan-Gradient, 340 g Silica Kartusche, 100 ml/min Fluss) und die Titelverbindung erhalten. Ausbeute: 2.1 g (29% d. Th.)
LC/MS [Methode 1]: $R_t$ = 1.18 min; MS (ESIpos): m/z = 501 $(M+H)^+$,
$^1$H-NMR (400 MHz, DMSO-$d_6$): $\delta$ [ppm] = 7.99 (d, 1H), 7.76-7.71 (m, 2H), 7.38 (s, 1H), 6.50 (s, 1H), 5.31-5.26 (m, 1H), 3.64 (s, 3H), 3.18 (s, 3H), 2.16-2.07 (m, 1H), 1.97-1.90 (m, 1H), 1.79-1.70 (m, 2H), 1.57-1.51 (m,1H), 1.44-1.13 (m, 6H),

1.40 (s, 9H).

**Beispiel 65.2E**

2-[4-(5-Chlor-2-cyanophenyl)-5-methoxy-2-oxopyridin-1(2*H*)-yl]-3-(*trans*-4-methoxycyclohexyl)-propansäure-*tert*.-butylester (Racemat)

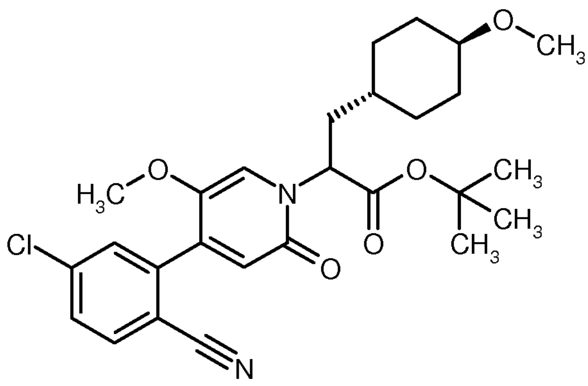

Das Rohprodukt aus Beispiel 65.1D wurde säulenchromatographisch gereinigt (Essigsäureethylester-Cyclohexan-Gradient, 340 g Silica Kartusche, 100 ml/min Fluss) und die Titelverbindung erhalten. Ausbeute: 1.7 g (87% Reinheit, 21% d. Th.)
LC/MS [Methode 1]: $R_t$ = 1.15 min; MS (ESIpos): m/z = 501 $(M+H)^+$,
$^1$H-NMR (400 MHz, DMSO-$d_6$): δ [ppm] = 7.99 (d, 1H), 7.77-7.71 (m, 2H), 7.39 (s, 1H), 6.50 (s, 1H), 5.28-5.22 (m, 1H), 3.64 (s, 3H), 3.20 (s, 3H), 3.07-2.98 (m, 1H), 2.17-2.08 (m, 1H), 1.98-1.83 (m, 4H), 1.68-1.61 (m, 1H), 1.40 (s, 9H), 1.07-0.89 (m, 5H).

**Beispiel 65.1F**

2-[4-(5-Chlor-2-cyanophenyl)-5-methoxy-2-oxopyridin-1(2*H*)-yl]-3-(*cis*-4-methoxycyclohexyl)-propansäure (Racemat)

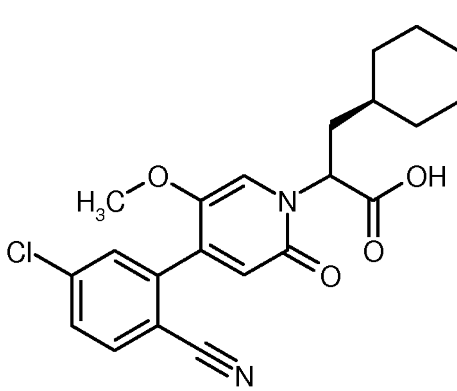

Gemäß allgemeiner Methode 6A wurden 2.10 g (4.19 mmol) 2-[4-(5-Chlor-2-cyanophenyl)-5-methoxy-2-oxopyridin-1(2*H*)-yl]-3-(*cis*-4-methoxycyclohexyl)propansäure-*tert*.-butylester (Racemat) in 6 ml Dichlormethan mit 6.23 ml (83.8 mmol) TFA umgesetzt. Ausbeute: 2.10 g (quant.)
LC/MS [Methode 1]: $R_t$ = 0.95 min; MS (ESIneg): m/z = 443 $(M-H)^-$.

**Beispiel 65.2F**

2-[4-(5-Chlor-2-cyanophenyl)-5-methoxy-2-oxopyridin-1(2*H*)-yl]-3-(*trans*-4-methoxycyclohexyl)-propansäure (Racemat)

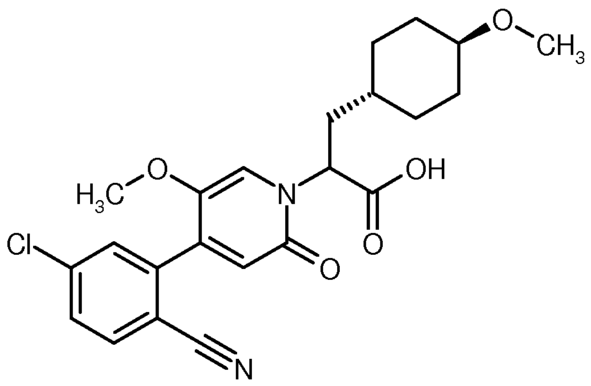

Gemäß allgemeiner Methode 6A wurden 1.70 g (87% Reinheit, 3.39 mmol) 2-[4-(5-Chlor-2-cyanophenyl)-5-methoxy-2-oxopyridin-1(2*H*)-yl]-3-(*trans*-4-methoxycyclohexyl)propansäure-*tert*.-butylester (Racemat) in 6 ml Dichlormethan mit 5.04 ml (67.9 mmol) TFA umgesetzt. Ausbeute: 1.70 g (quant.)
LC/MS [Methode 1]: $R_t$ = 0.90 min; MS (ESIneg): m/z = 443 $(M-H)^-$,
$^1$H-NMR (400 MHz, DMSO-$d_6$): $\delta$ [ppm] = 13.0 (br. s, 1H), 7.98 (d, 1H), 7.76-7.70 (m, 2H), 7.43 (s, 1H), 6.49 (s, 1H), 5.34-5.26 (m, 1H), 3.64 (s, 3H), 3.19 (s, 3H), 3.06-2.97 (m, 1H), 2.20-2.10 (m, 1H), 2.00-1.81 (m, 4H), 1.64-1.57 (m, 1H), 1.06-0.86 (m, 5H).

**Beispiel 65.1G**

4-({2-[4-(5-Chlor-2-cyanophenyl)-5-methoxy-2-oxopyridin-1(2*H*)-yl]-3-(*cis*-4-methoxy-cyclohexyl) -propanoyl}amino)benzoesäureethylester (Racemat)

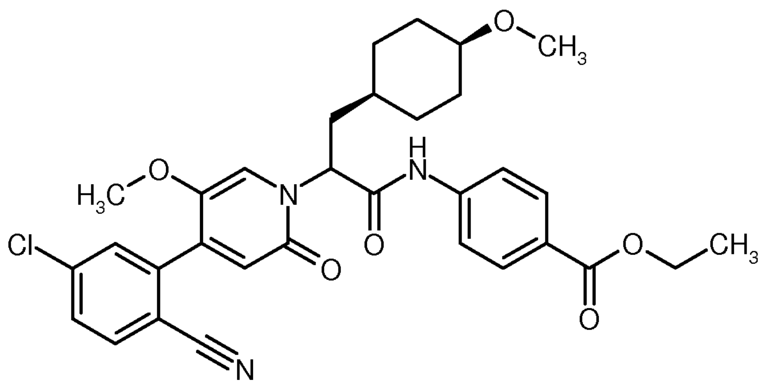

Gemäß allgemeiner Methode 5B wurden 2.10 g (74% Reinheit, 4.72 mmol) 2-[4-(5-Chlor-2-cyanophenyl)-5-methoxy-2-oxopyridin-1(2*H*)-yl]-3-(*cis*-4-methoxycyclohexyl)propansäure (Racemat), 780 mg (4.72 mmol) 4-Aminobenzoesäureethylester, 671 mg (4.72 mmol) Oxima und 735 µl (4.72 mmol) DIC in 47 ml Dimethylformamid zur Reaktion gebracht. Nach vollständigem Umsatz wurde die Reaktionslösung mit 263 ml 10%iger Lithiumchlorid-Lösung und 210 ml Essigsäureethylester verdünnt und die Phasen getrennt. Die wässrige Phase wurde zweimal mit 210 ml Essigsäureethylester extrahiert. Die vereinigten organischen Phasen wurden über Magnesiumsulfat getrocknet, filtriert und das Lösungsmittel unter reduziertem Druck entfernt. Das Rohprodukt wurde säulenchromatographisch gereinigt (Essigsäureethylester-Cyclohexan-Gradient, 100 g Silica Kartusche, 50 ml/min Fluss) und die Titelverbindung erhalten. Ausbeute:

2.1 g (75% d. Th.)
LC/MS [Methode 1]: $R_t$ = 1.26 min; MS (ESIpos): m/z = 592 $(M+H)^+$,
$^1$H-NMR (400 MHz, DMSO-$d_6$): δ [ppm] = 10.8 (s, 1H), 7.99 (d, 1H), 7.94 (d, 2H), 7.78 (d, 2H), 7.76-7.71 (m, 2H), 7.49 (s, 1H), 6.53 (s, 1H), 5.87-5.81 (m, 1H), 4.29 (q, 2H), 3.69 (s, 3H), 3.19 (s, 3H), 2.25-2.14 (m, 1H), 2.02-1.92 (m, 1H), 1.82-1.70 (m, 2H), 1.52-1.12 (m, 7H), 1.31 (t, 3H).

**Beispiel 65.2G**

4-({2-[4-(5-Chlor-2-cyanophenyl)-5-methoxy-2-oxopyridin-1(2*H*)-yl]-3-(*trans*-4-methoxy-cyclohexyl) -propanoyl}amino)benzoesäureethylester (Racemat)

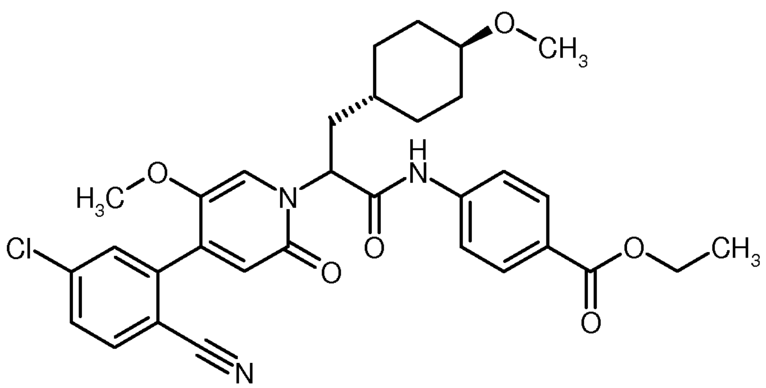

Gemäß allgemeiner Methode 5B wurden 1.70 g (82% Reinheit, 3.82 mmol) 2-[4-(5-Chlor-2-cyanophenyl)-5-methoxy-2-oxopyridin-1(2*H*)-yl]-3-(*trans*-4-methoxycyclohexyl)propansäure (Racemat), 631 mg (3.82 mmol) 4-Aminobenzoesäureethylester, 543 mg (3.82 mmol) Oxima und 595 μl (3.82 mmol) DIC in 38 ml Dimethylformamid zur Reaktion gebracht. Nach vollständigem Umsatz wurde die Reaktionslösung mit 213 ml 10%iger Lithiumchlorid-Lösung und 170 ml Essigsäureethylester verdünnt und die Phasen getrennt. Die wässrige Phase wurde zweimal mit 170 ml Essigsäureethylester extrahiert. Die vereinigten organischen Phasen wurden über Magnesiumsulfat getrocknet, filtriert und das Lösungsmittel unter reduziertem Druck entfernt. Das Rohprodukt wurde säulenchromatographisch gereinigt (Essigsäureethylester-Cyclohexan-Gradient, 100 g Silica Kartusche, 50 ml/min Fluss) und die Titelverbindung erhalten. Ausbeute: 1.1 g (78% Reinheit, 49% d. Th.)
LC/MS [Methode 1]: $R_t$ = 1.23 min; MS (ESIpos): m/z = 592 $(M+H)^+$,
$^1$H-NMR (400 MHz, DMSO-$d_6$): δ [ppm] = 10.8 (s, 1H), 7.99 (d, 1H), 7.94 (d, 2H), 7.80-7.71 (m, 4H), 7.49 (s, 1H), 6.54 (s, 1H), 5.87-5.81 (m, 1H), 4.29 (q, 2H), 3.69 (s, 3H), 3.20 (s, 3H), 3.08-2.98 (m, 1H), 2.24-2.15 (m, 1H), 2.00-1.88 (m, 3H), 1.83-1.72 (m, 2H), 1.31 (t, 3H), 1.12-0.89 (m, 5H).

**Beispiel 66.1A**

6-({2-[4-(5-Chlor-2-cyanophenyl)-5-methoxy-2-oxopyridin-1(2*H*)-yl]-3-cyclobutyl-propanoyl}amino)-2-methyl-3-oxo-2,3-dihydro-1*H*-indazol-1-carbonsäure-*tert.*-butylester (Racemat)

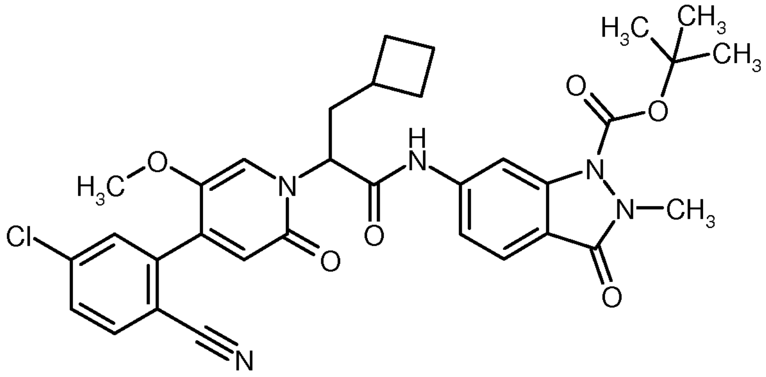

Nach der allgemeinen Methode 5A wurden 114 mg (94% Reinheit, 0.35 mmol) 2-[4-(5-Chlor-2-cyanophenyl)-5-methoxy-2-oxopyridin-1(2*H*)-yl]butansäure (Racemat) mit 103 mg (0.39 mmol, 1.1 eq.) 6-Amino-2-methyl-3-oxo-2,3-dihydro-1*H*-indazol-1-carbonsäure-*tert*.-butylester umgesetzt. Das Rohprodukt wurde mittels präparativer HPLC (Reprosil C18, Wasser-Acetonitril-Gradient) aufgereinigt. Ausbeute: 101 mg (45% d. Th.)
LC/MS [Methode 1]: $R_t$ = 1.27 min; MS (ESIpos): m/z = 632 $(M+H)^+$.

**Beispiel 67.1A**

2-[4-(5-Chlor-2-cyanophenyl)-5-methoxy-2-oxopyridin-1(2*H*)-yl]-3-methylbutansäureethylester (Racemat)

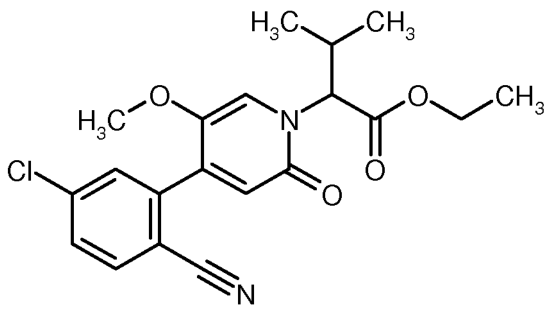

Nach der allgemeinen Methode 4E wurden 3.50 g (13.4 mmol) 4-Chlor-2-(5-methoxy-2-oxo-1,2-dihydropyridin-4-yl)benzonitril in Gegenwart von 1.5 eq. Natriumhydrid mit 5.60 g (20.1 mmol, 1.5 eq.) 3-Methyl-2-{[(trifluormethyl)sulfonyl]oxy}butansäureethylester (Racemat) bei RT umgesetzt. Das Rohprodukt wurde mittels Flash-Chromatographie (Kieselgel-50, Cyclohexan-Essigsäureethylester-Gemische) aufgereinigt. Ausbeute: 3.70 g (66% d. Th.)
LC/MS [Methode 1]: $R_t$ = 1.08 min; MS (ESIpos): m/z = 389 $(M+H)^+$,
$^1$H-NMR (400 MHz, DMSO-$d_6$): $\delta$ [ppm] = 7.99 (d, 1H), 7.76 (d, 1H), 7.73 (dd, 1H), 7.43 (s, 1H), 6.51 (s, 1H), 5.00-4.95 (m, 1H), 4.21-4.11 (m, 2H), 3.65 (s, 3H), 2.65-2.56 (m, 1H), 1.19 (t, 3H), 1.11 (d, 3H), 0.76 (d, 3H).

**Beispiel 67.1B**

2-[4-(5-Chlor-2-cyanophenyl)-5-methoxy-2-oxopyridin-1(2*H*)-yl]-3-methylbutansäure (Racemat)

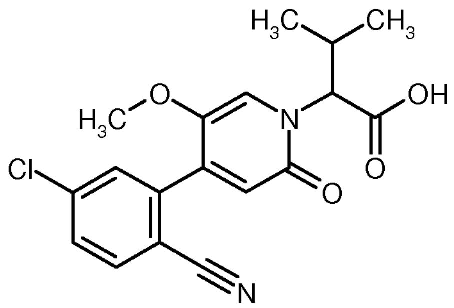

Nach der allgemeinen Methode 6B wurden 3.70 g (9.52 mmol) 2-[4-(5-Chlor-2-cyanophenyl)-5-methoxy-2-oxopyridin-1(2*H*)-yl]-3-methylbutansäureethylester (Racemat) mit Lithiumhydroxid verseift. Ausbeute: 2.80 g (82% d. Th.)
LC/MS [Methode 1]: $R_t$ = 0.89 min; MS (ESIpos): m/z = 361 $(M+H)^+$
$^1$H-NMR (400 MHz, DMSO-$d_6$): δ [ppm] = 13.1 (br. s, 1H), 7.99 (d, 1H), 7.76 (d, 1H), 7.73 (dd, 1H), 7.41 (s, 1H), 6.51 (s, 1H), 4.94 (d, 1H), 3.64 (s, 3H), 2.62-2.56 (m, 1H), 1.12 (d, 3H), 0.75 (d, 3H).

## Beispiel 67.1C

4-({2-[4-(5-Chlor-2-cyanophenyl)-5-methoxy-2-oxopyridin-1(2*H*)-yl]-3-methylbutanoyl}amino)-benzoesäure-*tert*.-butylester (Racemat)

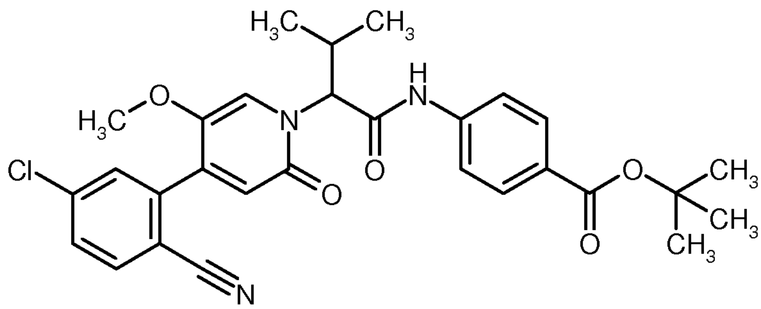

Nach der allgemeinen Methode 5A wurden 120 mg (333 μmol) 2-[4-(5-Chlor-2-cyanophenyl)-5-methoxy-2-oxopyridin-1(2*H*)-yl]-3-methylbutansäure (Racemat) mit 70.7 mg (366 μmol, 1.1 eq.) 4-Aminobenzoesäure-*tert*.-butylester umgesetzt. Das Rohprodukt wurde durch Flash-Chromatographie (Kieselgel-50, Cyclohexan-Essigsäureethylester-Gemische) aufgereinigt. Ausbeute: 134 mg (75% d. Th.)
LC/MS [Methode 1]: $R_t$ = 1.29 min; MS (ESIpos): m/z = 536 $(M+H)^+$,
$^1$H-NMR (400 MHz, DMSO-$d_6$): δ [ppm] = 11.0 (s, 1H), 8.00 (d, 1H), 7.88 (d, 2H), 7.78 (d, 2H), 7.76 (d, 1H), 7.73 (dd, 1H), 7.63 (s, 1H), 6.56 (s, 1H), 5.52 (d, 1H), 3.69 (s, 3H), 2.61-2.55 (m, 1H), 1.54 (s, 9H), 1.08 (d, 3H), 0.82 (d, 3H).

## Beispiel 67.2A

4-({2-[4-(5-Chlor-2-cyanophenyl)-5-methoxy-2-oxopyridin-1(2*H*)-yl]-3-methylbutanoyl}amino)-2-methylbenzoesäuremethylester (Racemat)

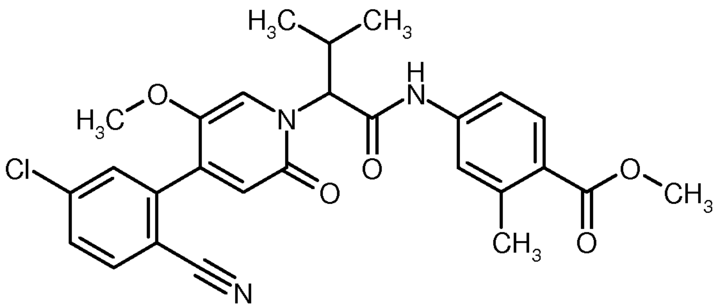

Nach der allgemeinen Methode 5A wurden 120 mg (333 μmol) 2-[4-(5-Chlor-2-cyanophenyl)-5-methoxy-2-oxopyridin-1(2*H*)-yl]-3-methylbutansäure (Racemat) mit 60.4 mg (366 μmol, 1.1 eq.) 4-Amino-2-methylbenzoesäuremethylester umgesetzt. Das Rohprodukt wurde durch Flash-Chromatographie (Kieselgel-50, Cyclohexan-Essigsäureethylester-Gemische) aufgereinigt. Ausbeute: 155 mg (88% d. Th.)
LC/MS [Methode 1]: $R_t$ = 1.17 min; MS (ESIpos): m/z = 508 $(M+H)^+$,
$^1$H-NMR (400 MHz, DMSO-$d_6$): δ [ppm] = 10.9 (s, 1H), 8.00 (d, 1H), 7.85 (d, 1H), 7.77-7.71 (m, 2H), 7.66-7.59 (m, 3H), 6.56 (s, 1H), 5.51 (d, 1H), 3.80 (s, 3H), 3.69 (s, 3H), 2.69 (s, 3H), 1.08 (d, 3H), 0.82 (d, 3H).

**Beispiel 67.3A**

6-({2-[4-(5-Chlor-2-cyanophenyl)-5-methoxy-2-oxopyridin-1(2*H*)-yl]-3-methylbutanoyl}amino)-1*H*-benzimidazol-2-carbonsäureethylester (Racemat)

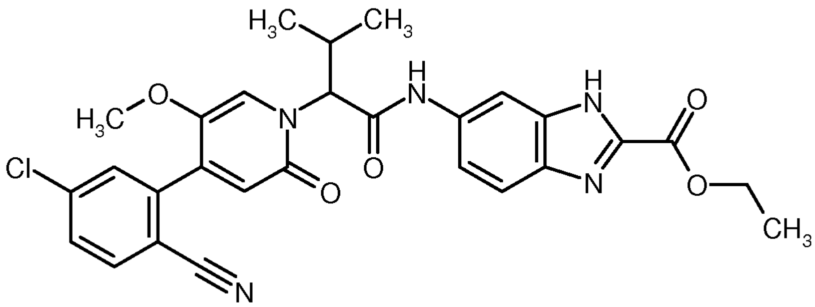

Nach der allgemeinen Methode 5B wurden 100 mg (277 μmol) 2-[4-(5-Chlor-2-cyanophenyl)-5-methoxy-2-oxopyridin-1(2*H*)-yl]-3-methylbutansäure (Racemat) mit 57 mg (277 μmol, 1.0 eq.) 6-Amino-1*H*-benzimidazol-2-carbonsäureethylester, 39.4 mg (277 μmol) Oxima und 42.9 μl (277 μmol) DIC in 5.4 ml Dimethylformamid umgesetzt. Das Rohprodukt wurde durch Flash-Chromatographie (Kieselgel-50, Cyclohexan-Essigsäureethylester-Gemische) aufgereinigt. Ausbeute: 139 mg (91% d. Th.)
LC/MS [Methode 1]: $R_t$ = 0.99 min; MS (ESIpos): m/z = 548 $(M+H)^+$.

**Beispiel 68.1A**

6-({2-[4-(5-Chlor-2-cyanophenyl)-5-methoxy-2-oxopyridin-1(2*H*)-yl]-4-methoxybutanoyl}amino)-2-methyl-3-oxo-2,3-dihydro-1*H*-indazol-1-carbonsäure-*tert*.-butylester (Racemat)

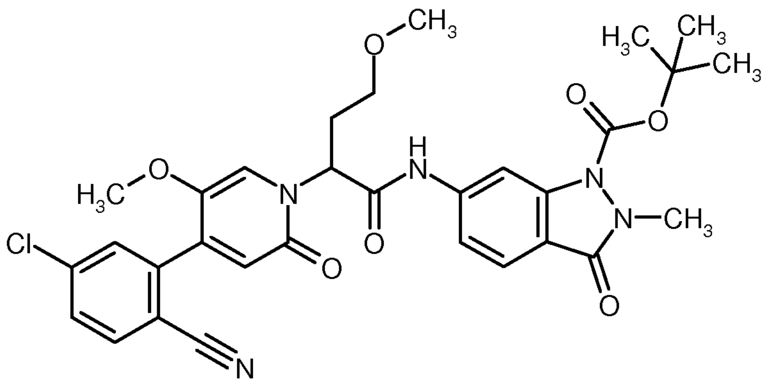

Nach der allgemeinen Methode 5A wurden 300 mg (796 μmol) 2-[4-(5-Chlor-2-cyanophenyl)-5-methoxy-2-oxopyridin-1(2*H*)-yl]-4-methoxybutansäure (Racemat) mit 231 mg (876 μmol, 1.1 eq.) 6-Amino-2-methyl-3-oxo-2,3-dihydro-1*H*-indazol-1-carbonsäure-*tert*.-butylester umgesetzt. Das Rohprodukt wurde durch Flash-Chromatographie (Kieselgel-50, Cyclohexan-Essigsäureethylester-Gemische) aufgereinigt. Ausbeute 229 mg (46% d. Th.)
LC/MS [Methode 1]: $R_t$ = 1.09 min; MS (ESIpos): m/z = 622 $(M+H)^+$, $^1$H-NMR (400 MHz, DMSO-$d_6$): δ [ppm] = 10.9 (s, 1H), 8.53 (d, 1H), 8.01-7.98 (m, 1H), 7.75-7.71 (m, 3H), 7.53 (s, 1H), 7.49 (dd, 1H), 6.53 (s, 1H), 5.79 (dd, 1H), 3.69 (s, 3H), 3.46 (s, 3H), 3.44-3.38 (m, 1H), 3.31 (s, 1H), 3.21 (s, 3H), 2.48-2.36 (m, 2H), 1.60 (s, 9H).

**Beispiel 68.2A**

6-({2-[4-(5-Chlor-2-cyanophenyl)-5-methoxy-2-oxopyridin-1(2*H*)-yl]-4-methoxybutanoyl}amino)-1*H*-indol-2-carbonsäureethylester (Racemat)

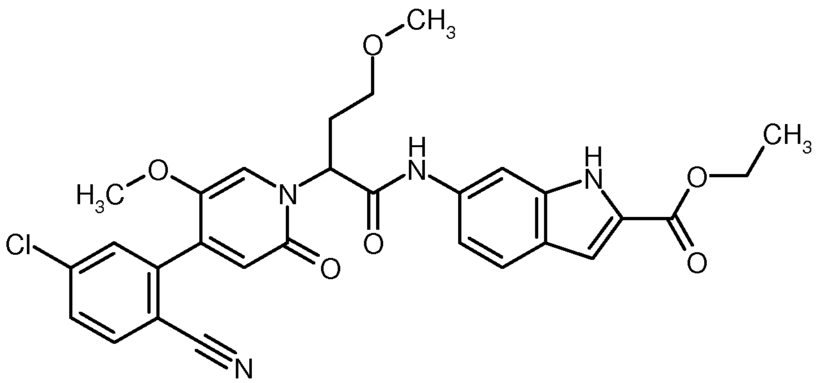

Nach der allgemeinen Methode 5B wurden 100 mg (265 μmol) 2-[4-(5-Chlor-2-cyanophenyl)-5-methoxy-2-oxopyridin-1(2*H*)-yl]-4-methoxybutansäure (Racemat) mit 54.2 mg (265 μmol, 1.0 eq.) 6-Amino-1*H*-indol-2-carbonsäureethylester, 37.7 mg (265 μmol) Oxima und 41.4 μl (265 μmol) DIC in 5.5 ml Dimethylformamid umgesetzt. Das Rohprodukt wurde mittels präparativer HPLC (Säule: Chromatorex 125 mm x 30 mm, 10 μm, Eluent: Wasser/0.1% Ameisensäure und Acetonitril/0.1% Ameisensäure, Gradient 10% Acetonitril bis 90% Acetonitril) gereinigt. Ausbeute: 85 mg (54% d. Th.)
LC/MS [Methode 1]: $R_t$ = 1.07 min; MS (ESIpos): m/z = 563 $(M+H)^+$,
$^1$H-NMR (400 MHz, DMSO-$d_6$): δ [ppm] = 11.8 (d, 1H), 10.5 (s, 1H), 8.04 (br. s, 1H), 8.00 (d, 1H), 7.76-7.72 (m, 2H), 7.58 (d, 1H), 7.56 (s, 1H), 7.23 (dd, 1H), 7.11-7.08 (m, 1H), 6.53 (s, 1H), 5.80 (dd, 1H), 4.32 (q, 2H), 3.70 (s, 3H), 3.43-3.26 (m, 2H), 3.22 (s, 3H), 2.47-2.36 (m, 2H), 1.33 (t, 3H).

**Beispiel 68.3.A**

6-({2-[4-(5-Chlor-2-cyanophenyl)-5-methoxy-2-oxopyridin-1(2*H*)-yl]-4-methoxybutanoyl}amino)-5-methoxy-1*H*-indol-2-carbonsäureethylester (Racemat)

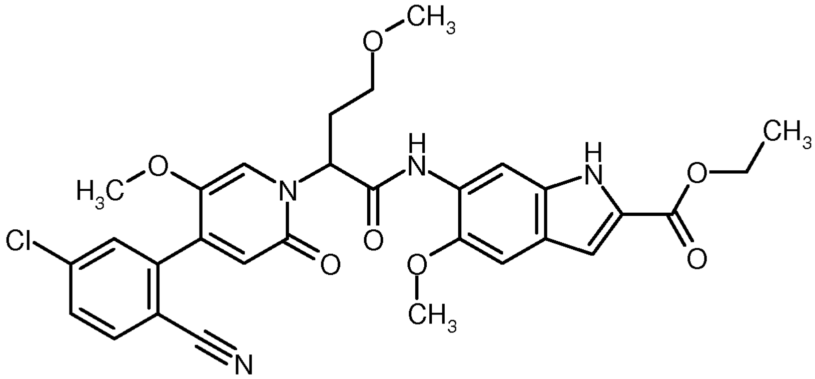

Nach der allgemeinen Methode 5A wurden 150 mg (398 μmol) 2-[4-(5-Chlor-2-cyanophenyl)-5-methoxy-2-oxopyridin-1(2*H*)-yl]-4-methoxybutansäure (Racemat) mit 103 mg (438 μmol, 1.1 eq.) 6-Amino-5-methoxy-1*H*-indol-2-carbonsäureethylester umgesetzt. Das Rohprodukt wurde mittels präparativer HPLC (Säule: Chromatorex 125 mm x 30 mm, 10 μm, Eluent: Wasser und Acetonitril, Gradient 10% Acetonitril bis 90% Acetonitril) gereinigt. Ausbeute: 87 mg (36% d. Th.)
LC/MS [Methode 1]: $R_t$ = 1.13 min; MS (ESIpos): m/z = 593 $(M+H)^+$,
$^1$H-NMR (500 MHz, DMSO-$d_6$): δ [ppm] = 11.7 (d, 1H), 9.43 (s, 1H), 8.26 (s, 1H), 8.00 (d, 1H), 7.76-7.72 (m, 2H), 7.51 (s, 1H), 7.20 (s, 1H), 7.04-7.03 (m, 1H), 6.58 (s, 1H), 5.79 (dd, 1H), 4.32 (q, 2H), 3.86 (s, 3H), 3.69 (s, 3H), 3.45-3.34 (m, 2H), 3.25 (s, 3H), 2.57-2.46 (m, 1H), 2.43-2.34 (m, 1H), 1.33 (t, 3H).

**Beispiel 69.1A**

2-[5-Chlor-4-(5-chlor-2-cyanophenyl)-2-oxopyridin-1(2*H*)-yl]-4-methylpentansäure-*tert*.-butylester (Racemat)

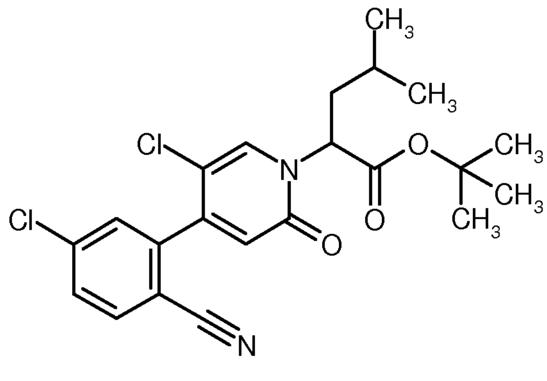

Nach der allgemeinen Methode 7B wurden 416 mg (1.00 mmol) [5-Chlor-4-(5-chlor-2-cyanophenyl)-2-oxopyridin-1(2*H*)-yl]essigsäure-*tert*.-butylester in Gegenwart von 0.95 ml (0.95 mmol, 1.2 eq.) Bis-(trimethylsilyl)-lithiumamid (1M in THF) mit 326 mg (1.58 mmol, 2.0 eq.) Isobutyltrifluormethansulfonat umgesetzt. Ausbeute: 139 mg (85% Reinheit, 34% d. Th.)
LC/MS [Methode 1]: $R_t$ = 1.27 min; MS (ESIpos): m/z = 435 $(M+H)^+$.

**Beispiel 69.1B**

2-[5-Chlor-4-(5-chlor-2-cyanophenyl)-2-oxopyridin-1(2*H*)-yl]-4-methylpentansäure (Racemat)

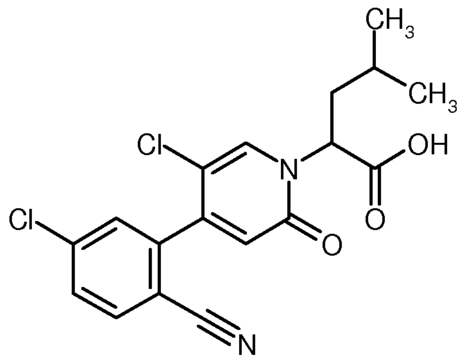

Nach der allgemeinen Methode 6A wurden 139 mg (85% Reinheit, 0.27 mmol) 2-[5-Chlor-4-(5-chlor-2-cyanophenyl)-2-oxopyridin-1(2*H*)-yl]-4-methylpentansäure-*tert*.-butylester (Racemat) mit TFA verseift. Ausbeute: 87 mg (84% d. Th.)
LC/MS [Methode 1]: $R_t$ = 1.01 min; MS (ESIpos): m/z = 379 $(M+H)^+$.

**Beispiel 69.1C**

4-({2-[5-Chlor-4-(5-chlor-2-cyanophenyl)-2-oxopyridin-1(2*H*)-yl]-4-methy|pentanoyl}amino)-benzoesäure-*tert*.-butylester (Racemat)

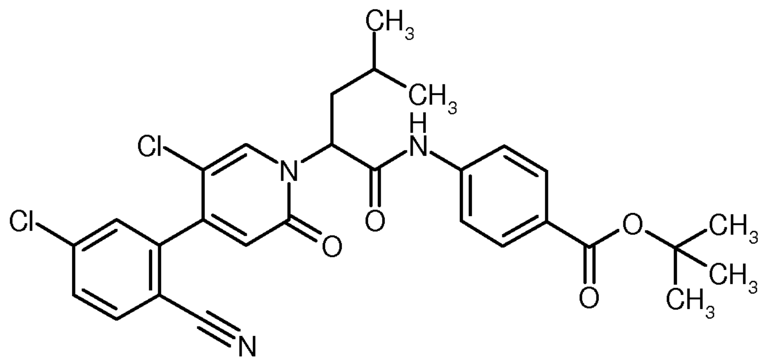

Nach der allgemeinen Methode 5A wurden 87 mg (0.23 mmol) 2-[5-Chlor-4-(5-chlor-2-cyanophenyl)-2-oxopyridin-1(2*H*)-yl]-4-methylpentansäure (Racemat) mit 49 mg (0.25 mmol, 1.1 eq.) 4-Aminobenzoesäure-*tert*.-butylester umgesetzt. Ausbeute: 45 mg (35% d. Th.)
LC/MS [Methode 1]: $R_t$ = 1.40 min; MS (ESIpos): m/z = 554 $(M+H)^+$.

**Beispiel 70.1A**

2-[5-Chlor-4-(5-chlor-2-cyanophenyl)-2-oxopyridin-1(2*H*)-yl]-4-(trifluormethoxy)butansäure-*tert*.-butylester (Racemat)

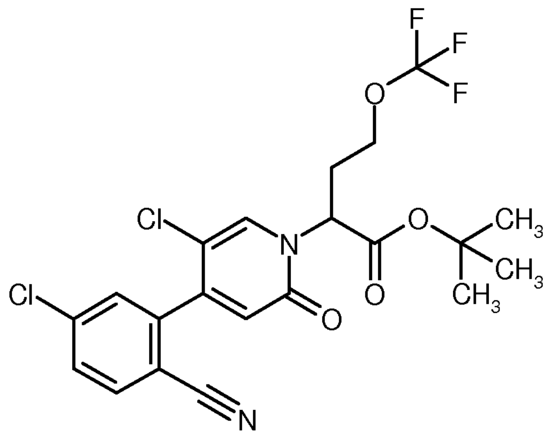

Nach der allgemeinen Methode 7B wurden 300 mg (0.79 mmol) [5-Chlor-4-(5-chlor-2-cyanophenyl)-2-oxopyridin-1(2*H*)-yl]essigsäure-*tert*.-butylester in Gegenwart von 0.95 ml (0.95 mmol, 1.2 eq.) Bis-(trimethylsilyl)-lithiumamid (1M in THF) mit 207 mg (0.79 mmol, 1.0 eq.) 2-(Trifluormethoxy)ethyltrifluormethansulfonat umgesetzt. Ausbeute: 244 mg (92% Reinheit, 58% d. Th.)
LC/MS [Methode 8]: $R_t$ = 1.53 min; MS (ESIpos): m/z = 435 (M+H-COO-*tert*.-butyl)$^+$.

**Beispiel 70.1B**

2-[5-Chlor-4-(5-chlor-2-cyanophenyl)-2-oxopyridin-1(2*H*)-yl]-4-(trifluormethoxy)butansäure (Racemat)

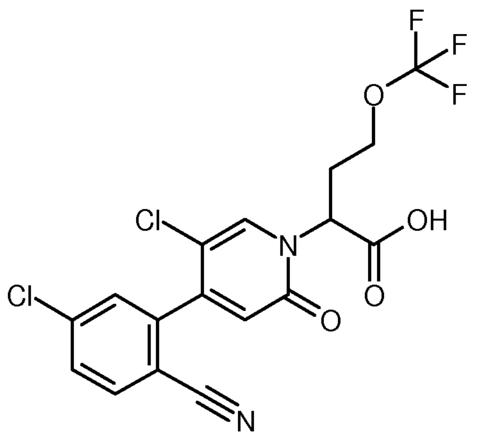

Nach der allgemeinen Methode 6A wurden 244 mg (92% Reinheit, 0.46 mmol) 2-[5-Chlor-4-(5-chlor-2-cyanophenyl)-2-oxopyridin-1(2*H*)-yl]-4-(trifluormethoxy)butansäure-*tert*. -butylester (Racemat) mit TFA verseift. Ausbeute: 237 mg (84% Reinheit, quant.)
LC/MS [Methode 1]: $R_t$ = 1.00 min; MS (ESIpos): m/z = 435 (M+H)$^+$.

**Beispiel 70.1C**

4-({2-[5-Chlor-4-(5-chlor-2-cyanophenyl)-2-oxopyridin-1(2*H*)-yl]-4-(trifluormethoxy)butanoyl}-amino)benzoesäure-*tert*.-butylester (Racemat)

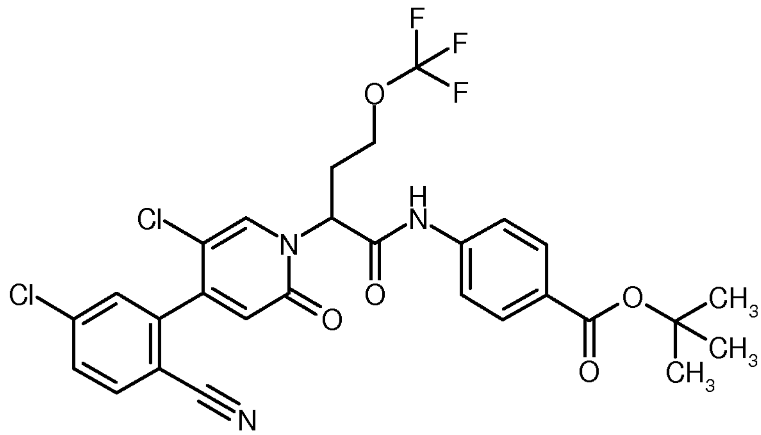

Nach der allgemeinen Methode 5A wurden 237 mg (84% Reinheit, 0.46 mmol) 2-[5-Chlor-4-(5-chlor-2-cyanophenyl)-2-oxopyridin-1(2*H*)-yl]-4-(trifluormethoxy)butansäure (Racemat) mit 97 mg (0.50 mmol, 1.1 eq.) 4-Aminobenzoesäure-*tert*.-butylester umgesetzt. Ausbeute: 142 mg (81% Reinheit, 41% d. Th.)
LC/MS [Methode 1]: $R_t$ = 1.35 min; MS (ESIneg): m/z = 608 $(M-H)^-$.

**Beispiel 71.1A**

{4-[5-Chlor-2-(difluormethoxy)phenyl]-5-methoxy-2-oxopyridin-1(2*H*)-yl}essigsäure-*tert*.-butylester

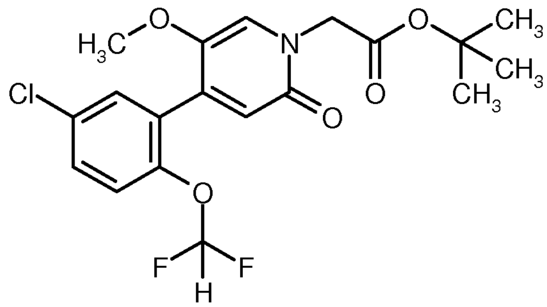

Nach der allgemeinen Methode 4B wurden 5.43 g (80% Reinheit, 14.4 mmol) 4-[5-Chlor-2-(difluormethoxy)phenyl]-5-methoxypyridin-2(1*H*)-on mit 1.2 eq. Bromessigsäure-*tert*.-butylester in Gegenwart von 1.5 eq. Kaliumcarbonat bei 100°C umgesetzt. Ausbeute: 3.64 g (61% d. Th.)
LC/MS [Methode 1]: $R_t$ = 1.05 min; MS (ESIpos): m/z = 416 $(M+H)^+$,
$^1$H-NMR (400 MHz, DMSO-$d_6$): $\delta$ [ppm] = 7.57 (dd, 1H), 7.45 (d, 1H), 7.43 (s, 1H), 7.30 (d, 1H), 7.13 (t, 1H), 6.35 (s, 1H), 4.58 (s, 2H), 3.56 (s, 3H), 1.44 (s, 9H).

**Beispiel 71.1B**

2-{4-[5-Chlor-2-(difluorrethoxy)penyl]-5-methoxy-2-oxopyridin-1(2*H*)-yl}-4-methylpentansäure-*tert*. -butylester (Racemat)

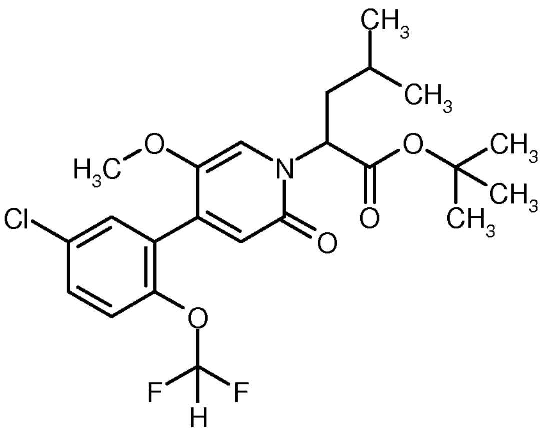

Nach der allgemeinen Methode 7B wurden 416 mg (1.00 mmol) {4-[5-Chlor-2-(difluormethoxy)phenyl]-5-methoxy-2-oxopyridin-1(2*H*)-yl}essigsäure-*tert*.-butylester in Gegenwart von 1.20 ml (1.20 mmol, 1.2 eq.) Bis-(trimethylsilyl)-lithiumamid (1M in THF) mit 309 mg (1.50 mmol, 1.5 eq.) Isobutyltrifluormethansulfonat umgesetzt. Ausbeute: 370 mg (75% d. Th.)
LC/MS [Methode 1]: $R_t$ = 1.23 min; MS (ESIpos): m/z = 472 $(M+H)^+$,
$^1$H-NMR (400 MHz, DMSO-$d_6$): δ [ppm] = 7.57 (dd, 1H), 7.49 (d, 1H), 7.29 (d, 1H), 7.25 (s, 1H), 7.10 (t, 1H), 6.36 (s, 1H), 5.29 (dd, 1H), 3.59 (s, 3H), 2.19-2.08 (m, 1H), 1.91-1.81 (m 1H), 1.43-1.33 (m, 1H), 1.40 (s, 9H), 0.9 (dd, 6H).

**Beispiel 71.1C**

2-{4-[5-Chlor-2-(difluormethoxy)phenyl]-5-methoxy-2-oxopyridm-1(2*H*)-yl}-4-methylpentansäure (Racemat)

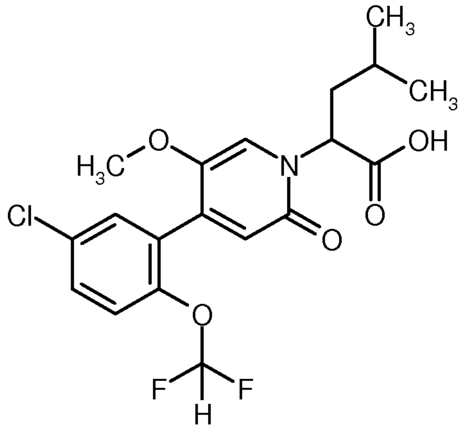

Nach allgemeinen Methode 6A wurden 370 mg (0.75 mmol) 2-{4-[5-Chlor-2-(difluormethoxy)phenyl]-5-methoxy-2-oxopyridin-1(2*H*)-yl}-4-methylpentansäure-*tert*.-butylester (Racemat) mit TFA verseift. Ausbeute: 319 mg (91% Reinheit, 93% d. Th.)
LC/MS [Methode 8]: $R_t$ = 1.27 min; MS (ESIpos): m/z = 416 $(M+H)^+$.

**Beispiel 71.1D**

4-[(2-{4-[5-Chlor-2-(difluormethoxy)phenyl]-5-methoxy-2-oxopyridin-1(2*H*)-yl}-4-methylpentanoyl)amino]benzoesäure-*tert*.-butylester (Racemat)

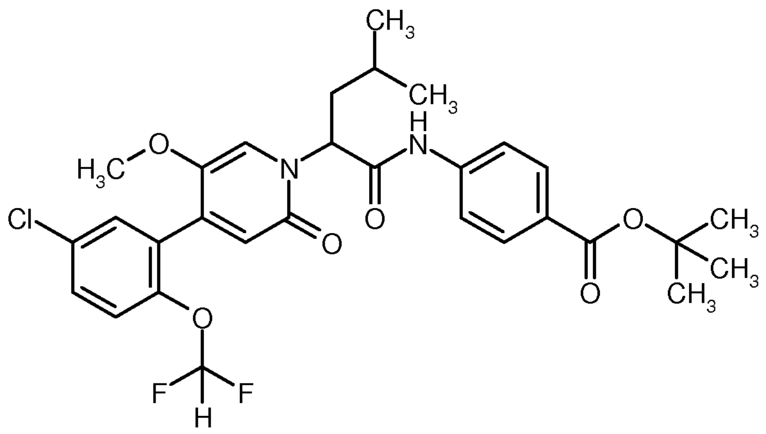

Nach der allgemeinen Methode 5A wurden 319 mg (91% Reinheit, 0.70 mmol) 2-{4-[5-Chlor-2-(difluormethoxy)phenyl]-5-methoxy-2-oxopyridin-1(2*H*)-yl}-4-methylpentansäure (Racemat) mit 148 mg (0.77 mmol, 1.1 eq.) 4-Aminobenzoesäure-*tert*.-butylester umgesetzt. Ausbeute: 263 mg (88% Reinheit, 56% d. Th.)
LC/MS [Methode 1]: $R_t$ = 1.37 min; MS (ESIpos): m/z = 591 $(M+H)^+$.

**Beispiel 72.1A**

2-{4-[5-Chlor-2-(difluormethoxy)penyl]-5-methoxy-2-oxopyridin-1(2*H*)-yl}-4-methoxybutansäure-*tert*.-butylester (Racemat)

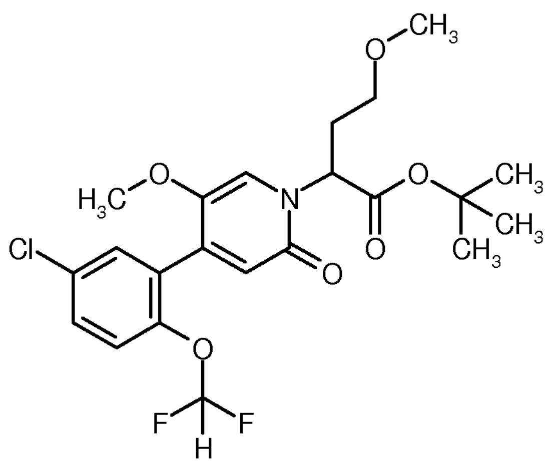

Nach der allgemeinen Methode 7B wurden 312 mg (0.75 mmol) {4-[5-Chlor-2-(difluormethoxy)phenyl]-5-methoxy-2-oxopyridin-1(2*H*)-yl}essigsäure-*tert*.-butylester in Gegenwart von 0.90 ml (0.90 mmol, 1.2 eq.) Bis-(trimethylsilyl)-lithiumamid (1M in THF) mit 312 mg (1.50 mmol, 1.5 eq.) 2-Methoxyethyltrifluormethansulfonat umgesetzt. Ausbeute: 201 mg (91% Reinheit, 51% d. Th.)
LC/MS [Methode 8]: $R_t$ = 1.40 min; MS (ESIpos): m/z = 418 $(M+H\text{-}COO\text{-}tert.\text{-}butyl)^+$.

**Beispiel 72.1B**

2-{4-[5-Chlor-2-(difluormethoxy)phenyl]-5-methoxy-2-oxopyridin-1(2*H*)-yl}-4-methoxybutansäure (Racemat)

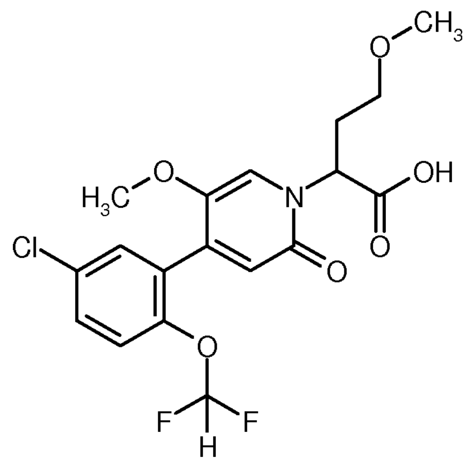

Nach der allgemeinen Methode 6A wurden 201 mg (91% Reinheit, 0.39 mmol) 2-{4-[5-Chlor-2-(difluormethoxy)phenyl] -5-methoxy-2-oxopyridin-1(2*H*)-yl}-4-methoxybutansäure-*tert*.-butylester (Racemat) mit TFA verseift. Ausbeute: 212 mg (82% Reinheit, quant.)
LC/MS [Methode 8]: $R_t$ = 1.11 min; MS (ESIpos): m/z = 418 $(M+H)^+$.

**Beispiel 72.1C**

4-[(2-{4-[5-Chlor-2-(difluormethoxy)phenyl]-5-methoxy-2-oxopyridin-1(2*H*)-yl}-4-methoxybutanoyl)amino]benzoesäure-*tert*.-butylester (Racemat)

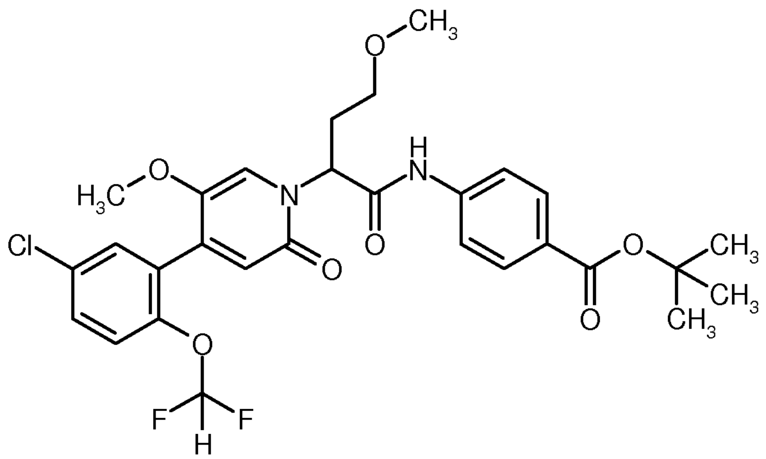

Nach der allgemeinen Methode 5A wurden 90 mg (77% Reinheit, 0.17 mmol) 2-{4-[5-Chlor-2-(difluormethoxy)phenyl]-5-methoxy-2-oxopyridin-1(2*H*)-yl}-4-methoxybutansäure (Racemat) mit 35 mg (0.18 mmol, 1.1 eq.) 4-Aminobenzoesäure-*tert*.-butylester umgesetzt. Ausbeute: 66 mg (67% d. Th.)
LC/MS [Methode 8]: $R_t$ = 1.52 min; MS (ESIneg): m/z = 591 $(M-H)^-$.

**Beispiel 72.2A**

6-[(2-{4-[5-Chlor-2-(difluormethoxy)phenyl]-5-methoxy-2-oxopyridin-1(2*H*)-yl}-4-methoxybutanoyl)amino]-2-methyl-3-oxo-2,3-dihydro-1*H*-indazol-1-carbonsäure-*tert*.-butylester (Racemat)

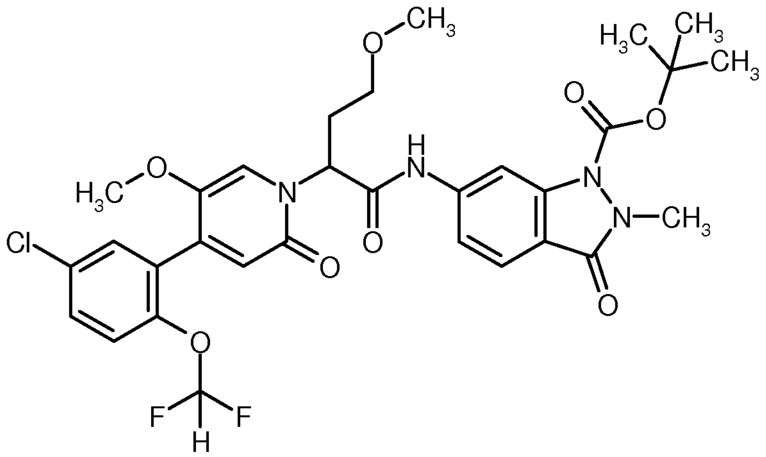

Nach der allgemeinen Methode 5A wurden 113 mg (82% Reinheit, 0.26 mmol) 2-{4-[5-Chlor-2-(difluormethoxy)phenyl]-5-methoxy-2-oxopyridin-1(2*H*)-yl}-4-methoxybutansäure (Racemat) mit 79 mg (0.29 mmol, 1.1 eq.) 6-Amino-2-methyl-3-oxo-2,3-dihydro-1*H*-indazol-1-carbonsäure-*tert*.-butylester umgesetzt. Ausbeute: 132 mg (73% d. Th.)
LC/MS [Methode 1]: $R_t$ = 1.15 min; MS (ESIpos): m/z = 663 $(M+H)^+$.

**Beispiel 73.1A**

2-{4-[5-Chlor-2-(difluormethoxy)phenyl]-5-methoxy-2-oxopyridin-1(2*H*)-yl}-4-(trifluormethoxy)-butansäure-*tert*. -butylester (Racemat)

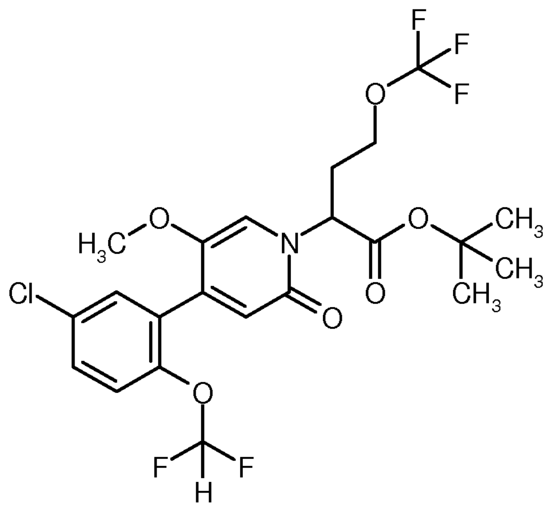

Nach der allgemeinen Methode 7B wurden 416 mg (1.00 mmol) {4-[5-Chlor-2-(difluormethoxy)phenyl]-5-methoxy-2-oxopyridin-1(2*H*)-yl}essigsäure-*tert*.-butylester in Gegenwart von 1.20 ml (1.20 mmol, 1.2 eq.) Bis-(trimethylsilyl)-lithiumamid (1M in THF) mit 393 mg (1.50 mmol, 1.5 eq.) 2-(Trifluormethoxy)ethyltrifluormethansulfonat umgesetzt. Ausbeute: 327 mg (62% d. Th.)
LC/MS [Methode 1]: $R_t$ = 1.23 min; MS (ESIpos): m/z = 528 $(M+H)^+$,
$^1H$-NMR (400 MHz, DMSO-$d_6$): $\delta$ [ppm] = 7.57 (dd, 1H), 7.45 (d, 1H), 7.32 (s, 1H), 7.30 (d, 1H), 7.09 (t, 1H), 6.37 (s, 1H), 5.11 (dd, 1H), 4.21-4.13 (m, 1H), 4.05-3.95 (m, 1H), 3.57 (s, 3H), 1.40 (s, 9H).

**Beispiel 73.1B**

2-{4-[5-Chlor-2-(difluormethoxy)penyl]-5-methoxy-2-oxopyridin-1(2*H*)-yl}-4-(trifluormethoxy)butansäure (Racemat)

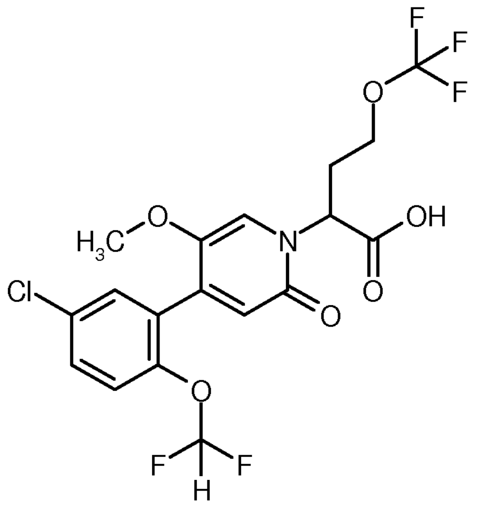

Nach der allgemeinen Methode 6A wurden 327 mg (0.62 mmol) 2-{4-[5-Chlor-2-(difluormethoxy)phenyl]-5-methoxy-2-oxopyridin-1(2*H*)-yl}-4-(trifluormethoxy)butansäure-*tert*.-butylester (Racemat) mit TFA verseift. Ausbeute: 290 mg (93% Reinheit, 92% d. Th.)
LC/MS [Methode 1]: $R_t$ = 0.99 min; MS (ESIpos): m/z = 472 $(M+H)^+$.

**Beispiel 73.1C**

4-{[2-{4-[5-Chlor-2-(difluormethoxy)phenyl] -5-methoxy-2-oxopyridin-1(2*H*)-yl}-4-(trifluormethoxy)butanoyl]amino}benzoesäure-*tert*.-butylester (Racemat)

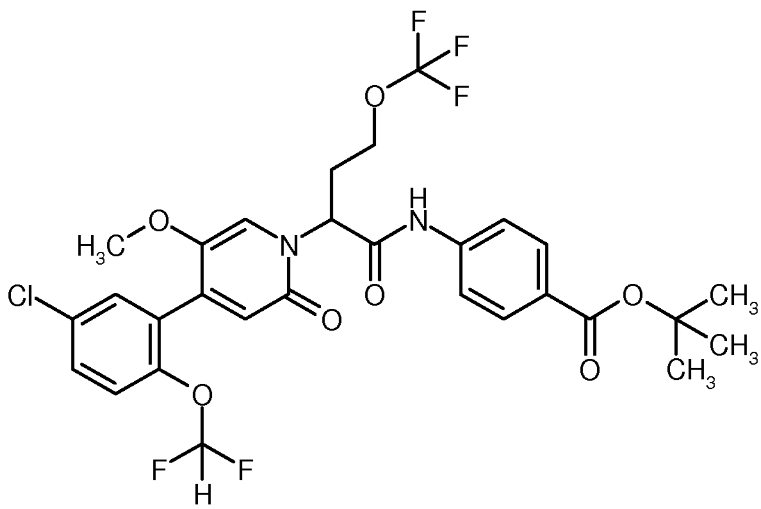

Nach der allgemeinen Methode 5A wurden 290 mg (93% Reinheit, 0.57 mmol) 2-{4-[5-Chlor-2-(difluormethoxy)phenyl]-5-methoxy-2-oxopyridin-1(2*H*)-yl}-4-(trifluormethoxy)butansäure (Racemat) mit 122 mg (0.63 mmol, 1.1 eq.) 4-Aminobenzoesäure-*tert.*-butylester umgesetzt. Ausbeute: 195 mg (50% d. Th.)
LC/MS [Methode 1]: $R_t$ = 1.33 min; MS (ESIpos): m/z = 647 $(M+H)^+$.

**Beispiel 74.1A**

(2*R*)-Tetrahydro-2*H*-pyran-2-ylmethyltrifluormethansulfonat

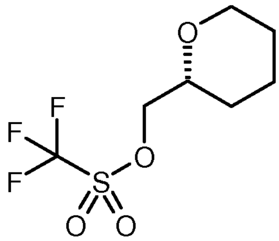

Nach der allgemeinen Methode 8A wurden 568 mg (69% Reinheit, 3.48 mmol) (2*R*)-Tetrahydro-2*H*-pyran-2-ylmethanol mit 0.71 ml (4.18 mmol, 1.2 eq.) Trifluormethansulfonsäureanhydrid in Gegenwart von 0.58 ml (4.18 mmol, 1.2 eq.) Triethylamin umgesetzt. Das Rohprodukt wurde ohne weitere Reinigung in der nächsten Stufe umgesetzt.

**Beispiel 74.1B**

2-{4-[5-Chlor-2-(difluormethoxy)phenyl]-5-methoxy-2-oxopyridin-1(2*H*)-yl}-3-[(2*R*)-tetrahydro-2*H*-pyran-2-yl]propansäure-*tert*.-butylester (Gemisch enantiomerenreiner Diastereomere 1 und 2)

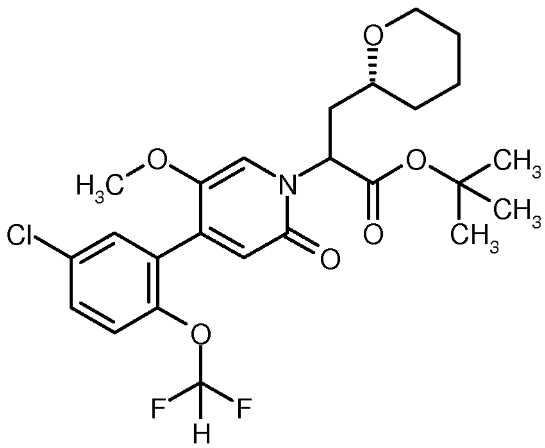

Nach der allgemeinen Methode 7B wurden 333 mg (0.80 mmol) {4-[5-Chlor-2-(difluormethoxy)phenyl]-5-methoxy-2-oxopyridin-1(2*H*)-yl}essigsäure-*tert*.-butylester in Gegenwart von 0.96 ml (0.96 mmol, 1.2 eq.) Bis-(trimethylsilyl)-lithiumamid (1M in THF) mit 426 mg (70% Reinheit, 1.20 mmol, 1.5 eq.) (2*R*)-Tetrahydro-2*H*-pyran-2-ylmethyltrifluormethansulfonat umgesetzt. Ausbeute: 85 mg (94% Reinheit, 19% d. Th.)
LC/MS [Methode 1]: $R_t$ = 1.25 min; MS (ESIpos): m/z = 514 $(M+H)^+$.

**Beispiel 74.1C**

2-14-[5-Chlor-2-(difluormethoxy)phenyl]-5-methoxy-2-oxopyridin-1(2*H*)-yl}-3-[(2*R*)-tetrahydro-2*H*-pyran-2-yl]propansäure (Gemisch enantiomerenreiner Diastereomere 1 und 2)

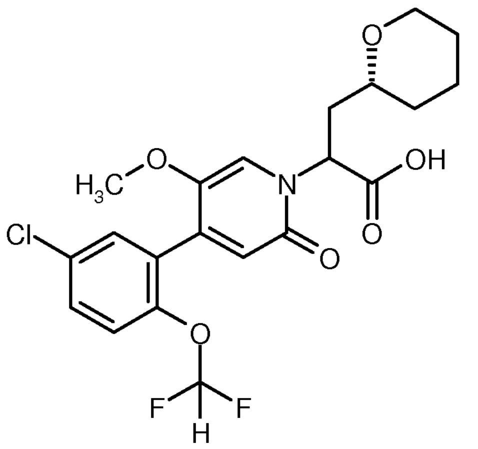

Nach der allgemeinen Methode 6A wurden 85 mg (94% Reinheit, 0.16 mmol) 2-{4-[5-Chlor-2-(difluormethoxy)phenyl] -5-methoxy-2-oxopyridin-1(2*H*)-yl} -3-[(2*R*)-tetrahydro-2*H*-pyran-2-yl]propansäure-*tert*.-butylester (Gemisch enantiomerenreiner Diastereomere 1 und 2) mit TFA verseift. Ausbeute: 83 mg (85% Reinheit, 99% d. Th.)
LC/MS [Methode 1]: $R_t$ = 0.98 min; MS (ESIpos): m/z = 458 $(M+H)^+$.

**Beispiel 74.1D**

4-[(2-14-[5-Chlor-2-(difluormethoxy)phenyl]-5-methoxy-2-oxopyridin-1(2*H*)-yl}-3-[(2*R*)-tetrahydro-2*H*-pyran-2-yl]propanoyl)amino]benzoesäure-*tert*.-butylester (Gemisch enantiomerenreiner Diastereomere 1 und 2)

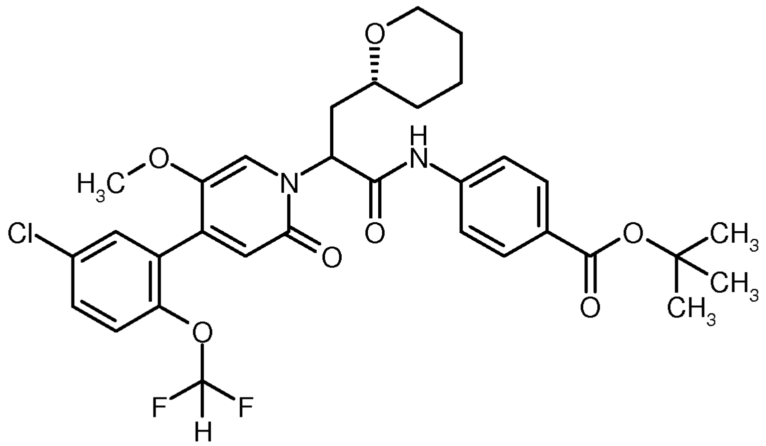

Nach der allgemeinen Methode 5A wurden 82 mg (85% Reinheit, 0.15 mmol) 2-{4-[5-Chlor-2-(difluormethoxy)phenyl]-5-methoxy-2-oxopyridin-1(2*H*)-yl}-3-[(2*R*)-tetrahydro-2*H*-pyran-2-yl]propansäure (Gemisch enantiomerenreiner Diastereomere 1 und 2) mit 32 mg (0.17 mmol, 1.1 eq.) 4-Aminobenzoesäure-*tert.*-butylester umgesetzt. Ausbeute: 67 mg (70% d. Th.)
LC/MS [Methode 1]: Diastereomer 1: $R_t$ = 1.35 min; MS (ESIpos): m/z = 633 $(M+H)^+$; Diastereomer 2: $R_t$ = 1.36 min; MS (ESIpos): m/z = 633 $(M+H)^+$.

**Beispiel 75.1A**

4-({2-[4-(5-Chlor-2-cyanophenyl)-5-methoxy-2-oxopyridin-1(2*H*)-yl]butanoyl}amino)-2-fluorbenzoesäure-*tert*. -butylester (Racemat)

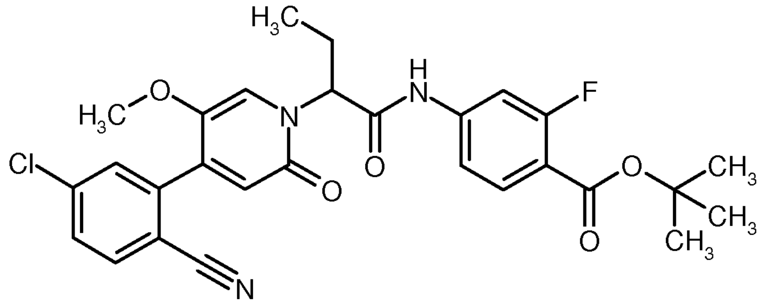

Nach der allgemeinen Methode 5C wurden 150 mg (0.43 mmol, 1.0 eq.) 2-[4-(5-Chlor-2-cyanophenyl)-5-methoxy-2-oxopyridin-1(2*H*)-yl]butansäure (Racemat) und 152 mg (0.65 mmol, 1.5 eq.) 2-Fluor-4-aminophenylcarbonsäure-*tert*.-butylester umgesetzt. Das Rohprodukt wurde mittels Normalphasen-Chromatographie (Eluent: Cyclohexan-Essigsäureethylester 20-50% Gemische) aufgereinigt. Ausbeute: 250 mg (93% Reinheit, 99% d. Th.)
LC/MS [Methode 8]: $R_t$ = 1.51 min; MS (ESIneg): m/z = 538 $(M-H)^-$
$^1$H-NMR (400 MHz, DMSO-$d_6$): δ [ppm] = 10.95 (s, 1H), 8.02-7.97 (m, 1H), 7.81 (t, 1H), 7.75-7.66 (m, 3H), 7.47 (s, 1H), 7.42 (dd, 1H), 6.54 (s, 1H), 5.59 (dd, 1H), 3.69 (s, 3H), 2.25-2.13 (m, 2H), 1.53 (s, 9H), 0.90 (t, 3H).

**Beispiel 75.2A**

4-({2-[4-(5-Chlor-2-cyanophenyl)-5-methoxy-2-oxopyridin-1(2*H*)-yl]butanoyl}amino)-3-fluorbenzoesäure-*tert*.-butylester (Racemat)

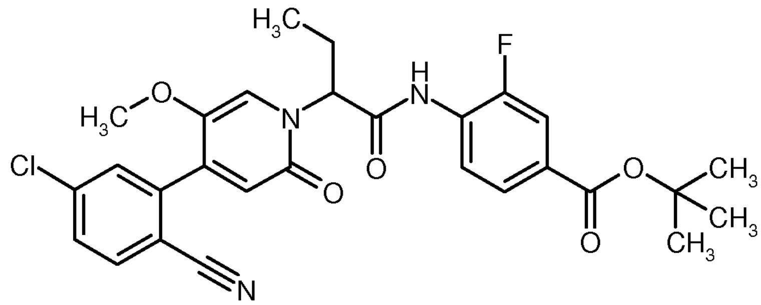

Nach der allgemeinen Methode 5C wurden 100 mg (0.29 mmol, 1.0 eq.) 2-[4-(5-Chlor-2-cyanophenyl)-5-methoxy-2-oxopyridin-1(2*H*)-yl]butansäure (Racemat) und 91 mg (0.43 mmol, 1.5 eq.) 4-Amino-3-fluorbenzoesäure-*tert.*-butylester umgesetzt. Das Rohprodukt wurde mittels Normalphasen-Chromatographie (Eluent: Cyclohexan-Essigsäureethylester 35-50% Gemische) aufgereinigt. Ausbeute: 126 mg (81% d. Th.)
LC/MS [Methode 1]: $R_t$ = 1.25 min; MS (ESIpos): m/z = 540 $(M+H)^+$,
$^1$H-NMR (400 MHz, DMSO-$d_6$): δ [ppm] = 10.56 (s, 1H), 8.15-8.08 (m, 1H), 8.02-7.97 (m, 1H), 7.76-7.68 (m, 4H), 7.46 (s, 1H), 6.54 (s, 1H), 5.79 (dd, 1H), 3.68 (s, 3H), 2.25-2.15 (m, 2H), 1.54 (s, 9H), 0.91 (t, 3H).

**Beispiel 75.3A**

4-({2-[4-(5-Chlor-2-cyanophenyl)-5-methoxy-2-oxopyridin-1(2*H*)-yl]butanoyl}amino)-2,5-difluorbenzoesäure-*tert*.-butylester (Racemat)

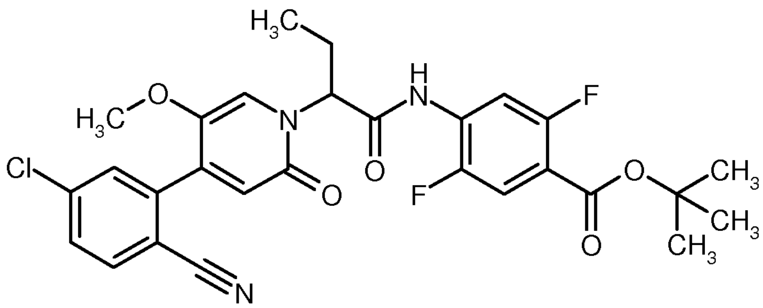

Nach der allgemeinen Methode 5B wurden 150 mg (0.43 mmol) 2-[4-(5-Chlor-2-cyanophenyl)-5-methoxy-2-oxopyridin-1(2*H*)-yl]butansäure (Racemat) und 154 mg (0.61 mmol, 1.4 eq.) 4-Amino-2,5-difluorbenzoesäure-*tert*.-butylester umgesetzt. Das Rohprodukt wurde mittels Normalphasen-Chromatographie (Eluent: Cyclohexan-Essigsäureethylester 20-40% Gemische) aufgereinigt. Ausbeute: 180 mg (71% d. Th.)
LC/MS [Methode 1]: $R_t$ = 1.31 min; MS (ESIpos): m/z = 558 $(M+H)^+$,
$^1$H-NMR (400 MHz, DMSO-$d_6$): $\delta$ [ppm] = 10.75 (s, 1H), 8.05 (dd, 1H), 8.01-7.97 (m, 1H), 7.76-7.71 (m, 2H), 7.69 (dd, 1H), 7.45 (s, 1H), 6.55 (s, 1H), 5.84-5.78 (m, 1H), 3.69 (s, 3H), 2.24-2.15 (m, 2H), 1.53 (s, 9H), 0.90 (t, 3H).

**Beispiel 75.4A**

4-({2-[4-(5-Chlor-2-cyanophenyl)-5-methoxy-2-oxopyridin-1(2*H*)-yl]butanoyl}amino)-2,3-difluorbenzoesäuremethylester (Racemat)

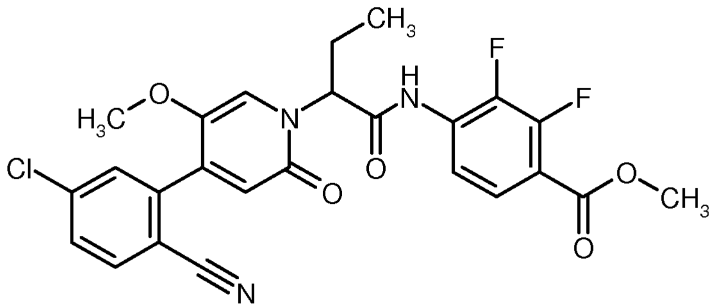

Nach der allgemeinen Methode 5C wurden 100 mg (0.29 mmol, 1.0 eq.) 2-[4-(5-Chlor-2-cyanophenyl)-5-methoxy-2-oxopyridin-1(2*H*)-yl]butansäure (Racemat) und 85 mg (0.43 mmol, 1.5 eq.) 4-Amino-2,3-difluorbenzoesäuremethylester umgesetzt. Das Rohprodukt wurde mittels Normalphasen-Chromatographie (Eluent: Cyclohexan-Essigsäureethylester 35-50% Gemische) aufgereinigt. Ausbeute: 113 mg (76% d. Th.)
LC/MS [1]: $R_t$ = 1.08 min; MS (ESIpos): m/z = 516 $(M+H)^+$, $^1$H-NMR (400 MHz, DMSO-$d_6$): $\delta$ [ppm] = 10.77 (s, 1H), 8.02-7.97 (m, 1H), 7.96-7.89 (m, 1H), 7.76-7.66 (m, 3H), 7.45 (s, 1H), 6.55 (s, 1H), 5.79 (dd, 1H), 3.86 (s, 3H), 3.68 (s, 3H), 2.26-2.12 (m, 2H), 0.91 (t, 3H).

**Beispiel 75.5A**

4-({2-[4-(5-Chlor-2-cyanophenyl)-5-methoxy-2-oxopyridin-1(2*H*)-yl]butanoyl}amino)-2,6-difluorbenzoesäuremethylester (Racemat)

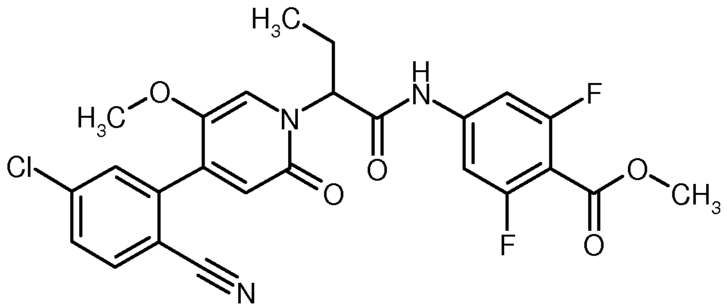

Eine Lösung von 46 mg (0.13 mmol) 2-[4-(5-Chlor-2-cyanophenyl)-5-methoxy-2-oxopyridin-1(2*H*)-yl]butansäure (Racemat) und 51 mg (0.27 mmol, 2 eq.) 4-Amino-2,6-difluorbenzoesäuremethylester in 1 ml Pyridin wurde bei 60-70°C tropfenweise mit 110 µl (0.46 mmol, 4 eq.) T3P (50%ig in Essigsäureethylester) versetzt. Das Reaktionsgemisch wurde auf 90°C erhitzt, 30 min bei 90°C gerührt, auf RT gekühlt und mit Wasser und Essigsäureethylester versetzt. Nach Phasentrennung wurde die wässrige Phase mit Essigsäureethylester extrahiert. Die vereinigten, organischen Phasen wurden mit gesättigter, wässriger Natriumchlorid-Lösung gewaschen, getrocknet (Natriumsulfat), filtriert und im Vakuum eingeengt. Das Rohprodukt wurde mittels Normalphasen-Chromatographie (Eluent: Cyclohexan-Essigsäureethylester 10-50% Gemische) aufgereinigt. Ausbeute: 54 mg (79% d. Th.)
LC/MS [Methode 1]: $R_t$ = 1.10 min; MS (ESIpos): m/z = 516 $(M+H)^+$,
$^1$H-NMR (400 MHz, DMSO-$d_6$): δ [ppm] = 11.06 (s, 1H), 8.02-7.98 (m, 1H), 7.75-7.72 (m, 2H), 7.50-7.44 (m, 3H), 6.55 (s, 1H), 5.55 (dd, 1H), 3.85 (s, 3H), 3.69 (s, 3H), 2.24-2.14 (m, 2H), 0.90 (t, 3H).

## Beispiel 76.1A

2-[4-(5-Chlor-2-cyanophenyl)-5-methoxy-2-oxopyridin-1(2*H*)-yl]butansäure-*tert.*-butylester (Racemat)

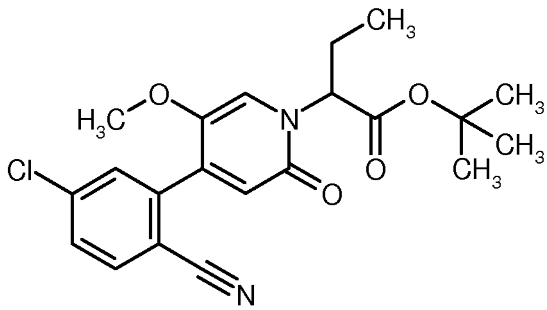

Eine Lösung von 5.0 g (13.3 mmol) [4-(5-Chlor-2-cyanophenyl)-5-methoxy-2-oxopyridin-1(2*H*)-yl]essigsäure-*tert.*-butylester in 100 ml Tetrahydrofuran wurde unter Argon bei -78°C tropfenweise mit 14.0 ml (1.0M in THF, 14.0 mmol, 1.05 eq.) Bis-(trimethylsilyl)-lithiumamid versetzt und 15 min bei -78°C gerührt. Anschließend wurden tropfenweise 2.6 g (14.7 mmol, 1.1 eq.) Ethyltrifluormethansulfonat als Reinsubstanz hinzugegeben. Das Kältebad wurde entfernt und die Reaktionsmischung 1 h bei RT nachgerührt. Das Reaktionsgemisch wurde auf 0°C gekühlt und mit gesättigter, wässriger Ammoniumchlorid-Lösung versetzt. Nach Phasentrennung wurde die wässrige Phase zweimal mit Methyl-*tert.*-butylether extrahiert. Die vereinigten, organischen Phasen wurden getrocknet (Natriumsulfat), filtriert und im Vakuum eingeengt. Das Rohprodukt wurde anschließend mittels Flash-Chromatographie (340 g Kieselgel, Eluent: Cyclohexan-Essigsäureethylester-Gemische 8:1, 4:1) aufgereinigt. Die produkthaltigen Fraktionen wurden vereinigt und im Vakuum eingeengt. Der Rückstand wurde in der Wärme in Methyl-*tert.*-butylether gelöst, die Lösung offen stehen gelassen, wobei nach 10 min das Gemisch fast vollständig durchkristallisierte. Der Kristallisat wurde filtriert und zweimal mit Methyl-*tert.*-butylether gewaschen. Die vereinigten Filtrate wurden im Vakuum eingeengt und der Rückstand wie beschrieben nochmals auskristallisiert. Beide Kristallisate wurden vereinigt und im Vakuum getrocknet. Ausbeute: 4.2 g (78% d. Th.)
LC/MS [Methode 1]: $R_t$ = 1.05 min; MS (ESIpos): m/z = 403 $(M+H)^+$,
$^1$H-NMR (400 MHz, DMSO-$d_6$): δ [ppm] = 7.99 (d, 1H), 7.77-7.70 (m, 2H), 7.36 (s, 1H), 6.50 (s, 1H), 5.03 (dd, 1H), 3.64 (s, 3H), 2.19-2.06 (m, 2H), 1.40 (s, 9H), 0.85 (t, 3H).

**Beispiel 76.1B**

4-({2-[4-(5-Chlor-2-cyanophenyl)-5-methoxy-2-oxopyridin-1(2*H*)-yl]butanoyl}amino)-benzoesäureethylester (Racemat)

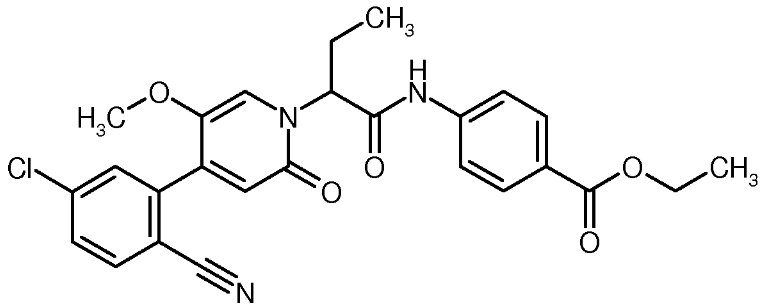

Eine Lösung von 1.0 g (2.9 mmol) 2-[4-(5-Chlor-2-cyanophenyl)-5-methoxy-2-oxopyridin-1(2*H*)-yl]butansäure (Racemat) in 10 ml Dimethylformamid wurde unter Argon bei RT mit 476 mg (2.9 mmol, 1.0 eq.) 4-Amino-benzoesäureethylester, mit 41 mg (0.29 mmol, 0.1 eq.) Oxima und anschließend tropfenweise mit 452 µl (2.9 mmol, 1.0 eq.) *N,N'*-Diisopropylcarbodiimid (DIC) versetzt. Das Reaktionsgemisch wurde 3 h bei 40-45°C gerührt, auf RT abgekühlt, mit Methyl-*tert*.-butylether und Wasser versetzt und 10 min kräftig gerührt, wobei ein Niederschlag ausfiel. Der Niederschlag wurde filtriert, zweimal mit Wasser und Methyl-*tert*.-butylether gewaschen und im Vakuum getrocknet (Niederschlag 1). Nach Phasentrennung der vereinigten Filtrate wurde die wässrige Phase einmal mit Methyl-*tert*.-butylether extrahiert. Die vereinigten, organischen Phasen wurden einmal mit gesättigter, wässriger Natriumchlorid-Lösung gewaschen, getrocknet (Natriumsulfat), filtriert und im Vakuum eingeengt. Der Rückstand wurde in Methyl-*tert*.-butylether verrührt, filtriert, zweimal mit Methyl-*tert*.-butylether gewaschen und im Vakuum getrocknet. Dieser Niederschlag wurde 10 min in wässiger Salzsäure (1N) verrührt, filtriert, zweimal mit Wasser und einmal mit Acetonitril gewaschen und im Vakuum getrocknet (Niederschlag 2). Ausbeute: Niederschlag 1: 1.12 g (79% d. Th.), Niederschlag 2: 127 mg (enthält laut $^1$H NMR noch 1,3-Diisopropylharnstoff)
LC/MS [Methode 1]: $R_t$ = 1.11 min; MS (ESIneg): m/z = 492 $(M-H)^-$,
$^1$H-NMR (400 MHz, DMSO-$d_6$): δ [ppm] = 10.82 (s, 1H), 8.00 (d, 1H), 7.93 (d, 2H), 7.81-7.70 (m, 4H), 7.49 (s, 1H), 6.54 (s, 1H), 5.63 (dd, 1H), 4.29 (q, 2H), 3.69 (s, 3H), 2.26-2.11 (m, 2H), 1.31 (t, 3H), 0.91 (t, 3H).

**Beispiel 77.1A**

(1,1-Dioxidotetrahydro-2*H*-thiopyran-4-yl)methanol

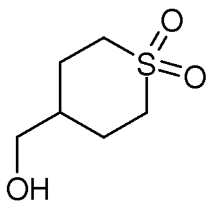

Zu einer Lösung aus 1.86 g (8.70 mmol) Natriumperiodat in 18 ml Wasser wurde bei Raumtemperatur unter Kühlung eine Lösung aus 500 mg (3.78 mmol) Tetrahydrothiopyran-4-ylmethanol in 10 ml Methanol gegeben. Es wurde 1 h bei 60°C nachgerührt und anschließend Methanol im Vakuum abdestilliert und der entstandene Niederschlag absaugt. Die verbliebene wässrige Phase wurde zweimal mit 10 ml Diethylether, zweimal mit 10 ml Dichlormethan, zweimal mit 10 ml Dichlormethan/Methanol (1:1, v/v) und nach Zugabe von 5 ml Wasser zweimal mit 10 ml Essigsäureethylester extrahiert. Die vereinigten organischen Phase wurden über Natriumsulfat getrocknet, filtriert und das Lösungsmittel unter reduziertem Druck entfernt. Die wässrige Phase wurde nochmals viermal mit 20 ml 2-Methyltetrahydrofuran extrahiert. Die vereinigten organischen Phasen wurden über Natriumsulfat getrocknet, filtriert und das Lösungsmittel unter reduziertem Druck entfernt. Kombinierte Ausbeute: 406 mg (65% d. Th.)
$^1$H-NMR (400 MHz, $CDCl_3$): δ [ppm] = 3.60-3.56 (m, 2H), 3.13-3.06 (m, 2H), 3.02-2.93 (m, 2H), 2.24-2.16 (m, 2H),

1.95-1.83 (m, 2H), 1.80-1.63 (m, 1H), 1.50 (t, 1H).

**Beispiel 77.1B**

(1,1-Dioxidotetrahydro-2*H*-thiopyran-4-yl)methyl-trifluormethansulfonat

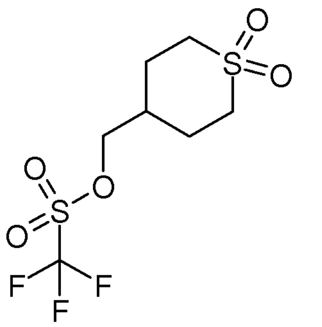

Zu 428 µl (2.55 mmol) Trifluormethansulfonsäureanhydrid in 3 ml Dichlormethan wurde bei -78°C eine Lösung aus 380 mg (2.31 mmol) (1,1-Dioxidotetrahydro-2*H*-thiopyran-4-yl)methanol und 355 µl (2.55 mmol) Triethylamin in 3 ml Dichlormethan getropft, so dass die interne Temperatur -50°C nicht überstieg. Es wurde 30 min bei -78°C nachgerührt und spontan auf RT erwärmt. Die Reaktionsmischung wurde mit 30 ml Methyl-*tert*.-butylether verdünnt mit gesättigter wässriger Ammoniumchlorid-Lösung, gesättigter wässriger Natriumchlorid-Lösung und gesättigter wässriger Natriumhydrogencarbonat-Lösung gewaschen, über Magnesiumsulfat getrocknet, filtriert und bei 25°C und ≥ 100 mbar Druck eingeengt. Ausbeute: 503 mg (73% d. Th.)
$^1$H-NMR (400 MHz, $CDCl_3$): δ [ppm] = 4.40 (d, 2H), 3.17-3.09 (m, 2H), 3.07-2.98 (m, 2H), 2.25-2.17 (m, 2H), 2.16-1.95 (m, 3H).

**Beispiel 77.1C**

2-[4-(5-Chlor-2-cyanophenyl)-5-methoxy-2-oxopyridin-1(2*H*)-yl]-3-(1,1-dioxidotetrahydro-2*H*-thiopyran-4-yl)propan-säure-*tert*.-butylester (Racemat)

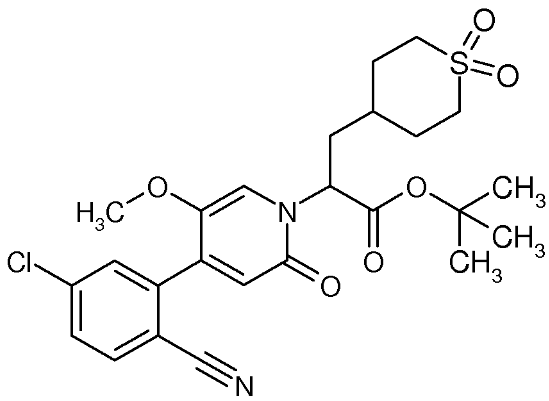

Gemäß allgemeiner Methode 7B wurden 421 mg (1.13 mmol) [4-(5-Chlor-2-cyanophenyl)-5-methoxy-2-oxopyridin-1(2*H*)-yl]essigsäure-*tert*.-butylester, 500 mg (1.69 mmol) (1,1-Dioxidotetrahydro-2*H*-thiopyran-4-yl)methyl-trifluormethansulfonat und 1.46 ml (1.46 mmol) Bis-(trimethylsilyl)-lithiumamid (1M in THF) in 15 ml THF zur Reaktion gebracht. Nach wässriger Aufarbeitung wurde die Titelverbindung erhalten. Ausbeute: 862 mg (91% Reinheit, quant.)
LC/MS [Methode 1]: $R_t$ = min; MS (ESIpos): m/z = 521 $(M+H)^+$

**Beispiel 77.1D**

2-[4-(5-Chlor-2-cyanophenyl)-5-methoxy-2-oxopyridin-1(2*H*)-yl]-3-(1,1-dioxidotetrahydro-2*H*-thiopyran-4-yl)propansäure (Racemat)

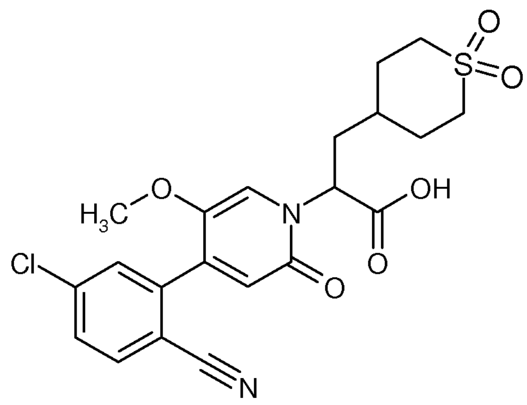

Gemäß allgemeiner Methode 6A wurden 860 mg (1.65 mmol) 2-[4-(5-Chlor-2-cyanophenyl)-5-methoxy-2-oxopyridin-1(2*H*)-yl]-3-(1,1-dioxidotetrahydro-2*H*-thiopyran-4-yl)propansäure-*tert*.-butylester (Racemat) in 16.6 ml Dichlormethan mit 4.77 ml (61.9 mmol) TFA umgesetzt. Ausbeute: 1.18 g (72% Reinheit, quant.)
LC/MS [Methode 1]: $R_t$ = 0.76 min; MS (ESIneg): m/z = 463 $(M-H)^-$

**Beispiel 77.1E**

4-({2-[4-(5-Chlor-2-cyanophenyl)-5-methoxy-2-oxopyridin-1(2*H*)-yl]-3-(1,1-dioxidotetrahydro-2*H*-thiopyran-4-yl)propanoyl}amino)benzoesäureethylester (Racemat)

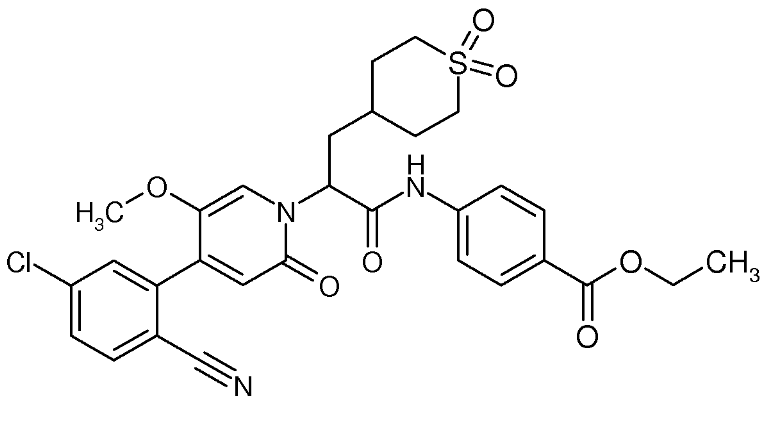

Gemäß allgemeiner Methode 5B wurden 768 mg (1.19 mmol) 2-[4-(5-Chlor-2-cyanophenyl)-5-methoxy-2-oxopyridin-1(2*H*)-yl]-3-(1,1-dioxidotetrahydro-2*H*-thiopyran-4-yl)propansäure (Racemat, 72% Reinheit), 786 mg (4.76 mmol) 4-Aminobenzoesäureethylester, 67.6 mg (476 μmol) Oxima und 741 μl (4.76 mmol) DIC in 11.5 ml Dimethylformamid zur Reaktion gebracht. Nach präparativer HPLC (Acetonitril-Wasser-Gradient) wurde die Titelverbindung erhalten. Ausbeute: 331 mg (45% d. Th.)
LC/MS [Methode 1]: $R_t$ = 1.07 min; MS (ESIpos): m/z = 612 $(M+H)^+$ $^1H$-NMR (400 MHz, DMSO-$d_6$): δ [ppm] = 10.8 (s, 1H), 8.00 (d, 1H), 7.95 (d, 2H), 7.80-7.71 (m, 4H), 7.50 (s, 1H), 6.55 (s, 1H), 5.85 (dd, 1H), 4.29 (q, 2H), 3.70 (s, 3H), 3.14-2.95 (m, 4H), 2.37-2.28 (m, 1H), 2.15-2.02 (m, 3H), 1.81-1.66 (m, 2H), 1.56-1.43 (m, 1H), 1.31 (t, 3H).

**Beispiel 78.1A**

2-[(Benzyloxy)methyl]tetrahydro-2*H*-pyran (Racemat)

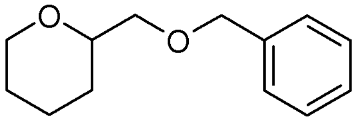

Zu einer Suspension von 9.47 g (237 mmol, 60% in Mineralöl) Natriumhydrid in 500 ml THF wurde bei 0°C eine Lösung von 25.0 g (215 mmol) Tetrahydro-2*H*-pyran-2-ylmethanol (Racemat) in 500 ml THF langsam zugetropft und nach vollständiger Zugabe 30 min bei 0°C nachgerührt. Anschließend wurden 25.7 ml (215 mmol) Benzylbromid zugegeben und für 30 min bei 0°C und 1 h bei Raumtemperatur nachgerührt. Die Reaktion wurde durch Zugabe von 200 ml gesättigter, wässriger Ammoniumchlorid-Lösung beendet und die Phasen getrennt. Die wässrige Phase wurde zweimal mit 200 ml Methyl-*tert*.-butylether extrahiert. Die vereinigten organischen Phasen wurden über Magnesiumsulfat getrocknet, filtriert und das Lösungsmittel unter reduziertem Druck entfernt. Das Rohprodukt wurde säulenchromatographisch gereinigt (Essigsäureethylester-Cyclohexan-Gradient, 340 g Silica Kartusche, 100 ml/min Fluss) und die Titelverbindung erhalten. Ausbeute: 41.9g (94% d. Th.)
LC/MS [Methode 3]: $R_t$ = 2.18 min; MS (ESIpos): m/z = 207 $(M+H)^+$
$^1$H-NMR (400 MHz, DMSO-$d_6$): $\delta$ [ppm] = 7.37-7.25 (m, 5H), 4.47 (s, 2H), 3.87-3.81 (m, 1H), 3.47-3.28 (m, 4H), 1.80-1.72 (m, 1H), 1.58-1.37 (m, 4H), 1.25-1.13 (m, 1H).

**Beispiel 78.1B**

(*R*)-2-[(Benzyloxy)methyl]tetrahydro-2*H*-pyran

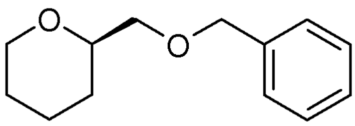

Enantiomerentrennung von 41.9g des Racemates aus Beispiel 78.1A ergab 16.7g der Titelverbindung Beispiel 78.1B (Enantiomer 1): Chirale HPLC: $R_t$ = 5.28 min; 99% ee, 93% Reinheit.
Drehwert: $[\alpha]_{589}^{20.0}$= + 14.9° (c 0.43 g/100cm$^3$, $CHCl_3$)
Trennmethode: Säule: OD-H 5 $\mu$m 250 mm x 20 mm; Eluent: 95% iso-Hexan, 5% 2-Propanol; Temperatur: 25°C; Fluss: 25 ml/min; UV-Detektion: 210 nm.
Analytik: Säule: OD-H 5 $\mu$m 250 mm x 4.6 mm; Eluent: 95% iso-Hexan, 5% 2-Propanol; Fluss: 1 ml/min; UV-Detektion: 220 nm.

**Beispiel 78.2B**

(*S*)-2-[(Benzyloxy)methyl]tetrahydro-2*H*-pyran

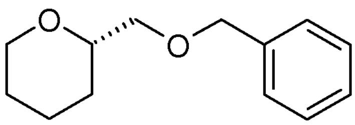

Enantiomerentrennung von 41.9g des Racemates aus Beispiel 78.1A ergab 17.0g der Titelverbindung Beispiel 78.2B (Enantiomer 2): Chirale HPLC: $R_t$ = 7.36 min; 96% ee, 96% Reinheit.
Drehwert: $[\alpha]_{589}^{20.0}$ = - 13.9° (c 0.61 g/100cm$^3$, $CHCl_3$)
Trennmethode: Säule: OD-H 5 $\mu$m 250 mm x 20 mm; Eluent: 95% iso-Hexan, 5% 2-Propanol; Temperatur: 25°C; Fluss:

25 ml/min; UV-Detektion: 210 nm.
Analytik: Säule: OD-H 5 μm 250 mm x 4.6 mm; Eluent: 95% iso-Hexan, 5% 2-Propanol; Fluss: 1 ml/min; UV-Detektion: 220 nm.

**Beispiel 78.1C**

(2*R*)-Tetrahydro-2*H*-pyran-2-ylmethanol

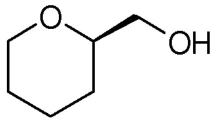

Zu einer Lösung von 16.7 g (75.3 mmol) (R)-2-[(Benzyloxy)methyl]tetrahydro-2H-pyran (93% Reinheit) in 150 ml Ethanol wurde 2.0 g Palladium auf Kohle (10%) gegeben und über Nacht bei Normalbedingungen hydriert. Anschließend wurde die Reaktionsmischung über Celite filtriert und erneut mit 1.5g Palladium auf Kohle (10%) versetzt. Es wurde für weitere 72 h hydriert. Die Reaktionsmischung wurde über Celite filtriert, der Filterkuchen mit Ethanol nachgewaschen und das Filtrat unter reduziertem Druck eingeengt. Der Rückstand wurde chromatographisch (Silica, Essigsäureethylester/Cyclohexan-Gradient) gereinigt und die Produktfraktionen bei < 35°C und > 80 mbar vom Lösungsmittel befreit.
Ausbeute: 5.47 g (63% d. Th.)
Drehwert: $[\alpha]_{589}^{20.0}$ = - 9.4° (c 0.4 g/100cm$^3$, $CHCl_3$)
GC/MS [Methode 9]: $R_t$ = 2.16 min; MS: m/z = 116 $(M)^+$
$^1$H-NMR (400 MHz, DMSO-$d_6$): δ [ppm] = 4.51 (t, 1H), 3.87-3.81 (m, 1H), 3.37-3.18 (m, 4H), 1.80-1.71 (m, 1H), 1.59-1.50 (m, 1H), 1.49-1.36 (m, 3H), 1.19-1.05 (m, 1H).

**Beispiel 78.2C**

(2*S*)-Tetrahydro-2*H*-pyran-2-ylmethanol

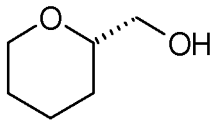

Zu einer Lösung 17.0 g (82.4 mmol) (*S*)-2-[(Benzyloxy)methyl]tetrahydro-2*H*-pyran (96% ee, 96% Reinheit) in 120 ml Ethanol wurde 3.51 g (3.30 mmol) Palladium auf Kohle (10%) gegeben und über Nacht bei Raumtemperatur und Normaldruck hydriert. Anschließend wurde noch einmal 1.75 g (1.65 mmol) Palladium auf Kohle (10%) hinzugegeben und für weitere 72 h bei Raumtemperatur hydriert. Anschließend wurde die Reaktionsmischung über Celite filtriert und das Filtrat eingeengt. Der Rückstand wurde chromatographisch (Silica, Dichlormethan/Methanol-Gradient) gereinigt und die Produktfraktionen bei < 25°C und > 50 mbar vom Lösungsmittel befreit. Ausbeute: 8.23 g (86% d. Th.)
Drehwert: $[\alpha]_{589}^{20.0}$ = + 9.1° (c 0.36 g/100cm$^3$, $CHCl_3$), vgl. A. Aponick, B. Biannic, Org. Lett.n 2011, 13, 1330-1333.
GC/MS [Methode 7]: $R_t$ = 1.82 min; MS: m/z = 116 $(M)^+$
$^1$H-NMR (400 MHz, DMSO-$d_6$): δ [ppm] = 4.51 (t, 1H), 3.87-3.81 (m, 1H), 3.37-3.18 (m, 4H), 1.80-1.71 (m, 1H), 1.59-1.50 (m, 1H), 1.49-1.36 (m, 3H), 1.19-1.05 (m, 1H).

**Beispiel 79.1A**

(2*S*)-Tetrahydro-2*H*-pyran-2-ylmethyltrifluormethansulfonat

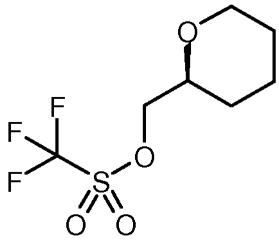

Nach der allgemeinen Methode 8A wurden 330 mg (2.84 mmol) (2*S*)-Tetrahydro-2*H*-pyran-2-ylmethanol mit 0.57 ml (3.41 mmol, 1.2 eq.) Trifluormethansulfonsäureanhydrid in Gegenwart von 0.48 ml (3.41 mmol, 1.2 eq.) Triethylamin umgesetzt. Das Rohprodukt wurde ohne weitere Reinigung in der nächsten Stufe umgesetzt.
$^1$H-NMR (400 MHz, DMSO-$d_6$): $\delta$ [ppm] = 4.32 (dd, 1H), 4.18 (dd, 1H), 4.00-3.92 (m, 1H), 3.60-3.52 (m, 1H), 3.48-3.39 (m, 1H), 1.85-1.74 (m, 1H), 1.56-1.41 (m, 4H), 1.28-1.14 (m, 1H).

**Beispiel 79.1B**

2-{4-[5-Chlor-2-(difluormethoxy)phenyl]-5-methoxy-2-oxopyridin-1(2*H*)-yl}-3-[(2*S*)-tetrahydro-2*H*-pyran-2-yl]propansäure-*tert*.-butylester (Gemisch enantiomerenreiner Diastereomere 1 und 2)

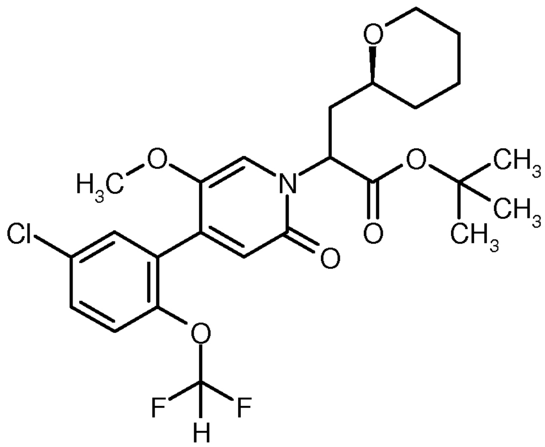

Nach der allgemeinen Methode 7B wurden 237 mg (0.55 mmol) {4-[5-Chlor-2-(difluormethoxy)phenyl]-5-methoxy-2-oxopyridin-1(2*H*)-yl}essigsäure-*tert*.-butylester in Gegenwart von 0.72 ml (0.72 mmol, 1.3 eq.) Bis-(trimethylsilyl)-lithiumamid (1M in THF) mit 274 mg (1.11 mmol, 2.0 eq.) (2*S*)-Tetrahydro-2*H*-pyran-2-ylmethyltrifluormethansulfonat umgesetzt. Ausbeute: 130 mg (45% d. Th.)
LC/MS [Methode 1]: $R_t$ = 1.25 min; MS (ESIpos): m/z = 514 $(M+H)^+$.

**Beispiel 79.1C**

2-{4-[5-Chlor-2-(difluormethoxy)phenyl]-5-methoxy-2-oxopyridin-1(2*H*)-yl}-3-[(2*S*)-tetrahydro-2*H*-pyran-2-yl]propansäure (Gemisch enantiomerenreiner Diastereomere 1 und 2)

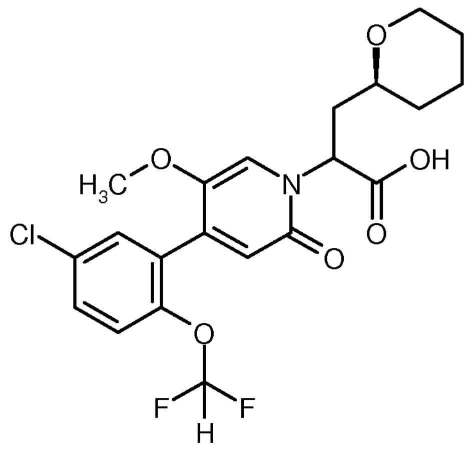

Nach der allgemeinen Methode 6A wurden 311 mg (90% Reinheit, 0.55 mmol) 2-{4-[5-Chlor-2-(difluormethoxy)phenyl]-5-methoxy-2-oxopyridin-1(2*H*)-y1}-3-[(2*S*)-tetrahydro-2*H*-pyran-2-yl]propansäure-*tert*.-butylester (Gemisch enantiomerenreiner Diastereomere 1 und 2) mit TFA verseift. Ausbeute: 312 mg (87% Reinheit, quant.)
LC/MS [Methode 2]: Diastereomer 1: $R_t$ = 2.86 min; MS (ESIpos): m/z = 458 $(M+H)^+$; Diastereomer 2: $R_t$ = 2.92 min; MS (ESIpos): m/z = 458 $(M+H)^+$.

**Beispiel 79.1D**

4-[(2-{4-[5-Chlor-2-(difluormethoxy)phenyl]-5-methoxy-2-oxopyridin-1(2*H*)-yl}-3-[(2*S*)-tetrahydro-2*H*-pyran-2-yl]propanoyl)amino]benzoesäureethylester (Gemisch enantiomerenreiner Diastereomere 1 und 2)

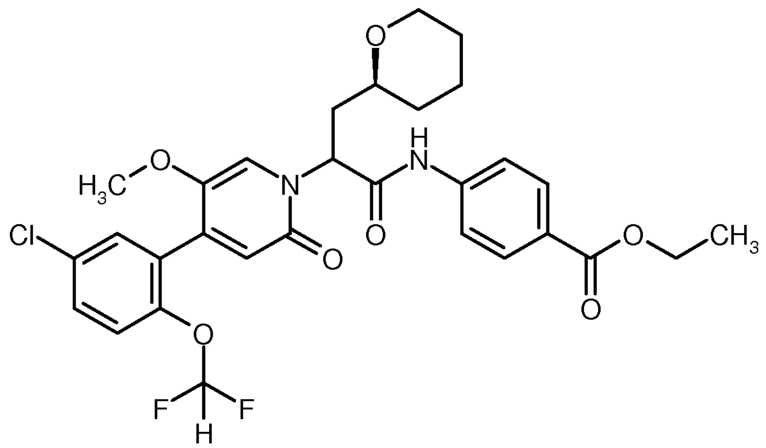

Nach der allgemeinen Methode 5B wurden zwei Fraktionen, 312 mg (87% Reinheit, 0.59 mmol) und 86 mg (0.19 mmol) 2-{4-[5-Chlor-2-(difluormethoxy)phenyl]-5-methoxy-2-oxopyridin-1(2*H*)-yl}-3-[(2*S*)-tetrahydro-2*H*-pyran-2-yl]propansäure (Gemisch enantiomerenreiner Diastereomere 1 und 2), mit insgesamt 202 mg (1.23 mmol, 1.6 eq.) 4-Aminobenzoesäureethylester umgesetzt. Ausbeute: 315 mg (83% d. Th.)
LC/MS [Methode 1]: Diastereomer 1: $R_t$ = 1.20 min; MS (ESIpos): m/z = 605 $(M+H)^+$; Diastereomer 2: $R_t$ = 1.22 min; MS (ESIpos): m/z = 605 $(M+H)^+$.

**Beispiel 80.1A**

2-[5-Chlor-4-(5-chlor-2-cyanophenyl)-2-oxopyridin-1(2*H*)-yl]-3-[(2*S*)-tetrahydro-2*N*-pyran-2-yl]propansäure-*tert*.-butylester (Gemisch enantiomerenreiner Diastereomere 1 und 2)

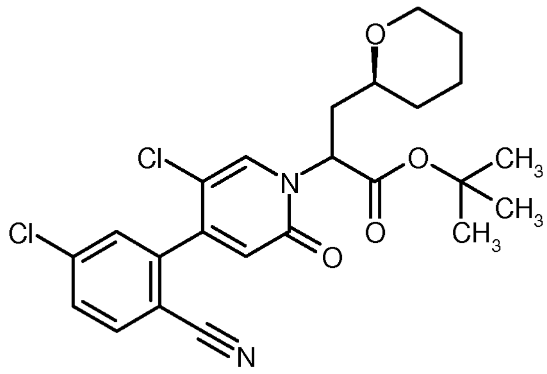

Nach der allgemeinen Methode 7B wurden 570 mg (1.46 mmol) [5-Chlor-4-(5-chlor-2-cyanophenyl)-2-oxopyridin-1(2*H*)-yl]essigsäure-*tert*.-butylester in Gegenwart von 1.75 ml (1.75 mmol, 1.2 eq.) Bis-(trimethylsilyl)-lithiumamid (1M in THF) mit 707 mg (87% Reinheit, 2.48 mmol, 1.7 eq.) (2*S*)-Tetrahydro-2*H*-pyran-2-ylmethyltrifluormethansulfonat umgesetzt. Ausbeute: 396 mg (57% d. Th.)
LC/MS [Methode 1]: $R_t$ = 1.25 min; MS (ESIpos): m/z = 421 (M+H-COO-*tert*.-butyl)$^+$.

**Beispiel 80.1B**

2-[5-Chlor-4-(5-chlor-2-cyanophenyl)-2-oxopyridin-1(2*H*)-yl]-3-[(2*S*)-tetrahydro-2*H*-pyran-2-yl]propansäure (Gemisch enantiomerenreiner Diastereomere 1 und 2)

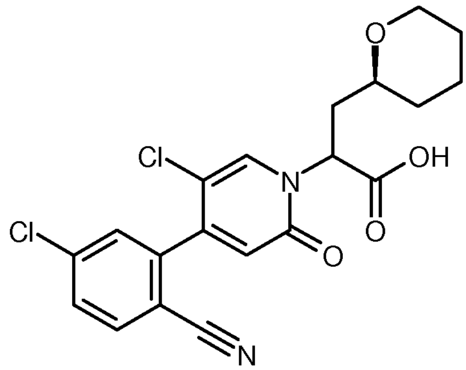

Nach der allgemeinen Methode 6A wurden 396 mg (0.83 mmol) 2-[5-Chlor-4-(5-chlor-2-cyanophenyl)-2-oxopyridin-1(2*H*)-yl]-3- [(2*S*)-tetrahydro-2*H*-pyran-2-yl]propansäure-*tert*. - butylester (Gemisch enantiomerenreiner Diastereomere 1 und 2) mit TFA verseift. Ausbeute: 396 mg (quant.)
LC/MS [Methode 1]: $R_t$ = 0.96 min; MS (ESIpos): m/z = 421 (M+H)$^+$.

**Beispiel 80.1C**

4-({2-[5-Chlor-4-(5-chlor-2-cyanophenyl)-2-oxopyridin-1(2*H*)-yl]-3-[(2*S*)-tetrahydro-2*H*-pyran-2-yl]propanoyl}amino)benzoesäure-*tert*.-butylester (Gemisch enantiomerenreiner Diastereomere 1 und 2)

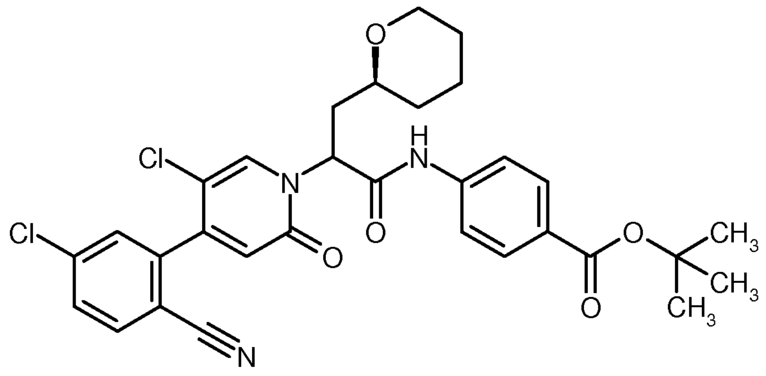

Nach der allgemeinen Methode 5A wurden 396 mg (0.90 mmol) 2-[5-Chlor-4-(5-chlor-2-cyanophenyl)-2-oxopyridin-1(2*H*)-yl]-3-[(2*S*)-tetrahydro-2*H*-pyran-2-yl]propansäure (Gemisch enantiomerenreiner Diastereomere 1 und 2) mit 192 mg (0.99 mmol, 1.1 eq.) 4-Aminobenzoesäure-*tert*.-butylester umgesetzt. Ausbeute: 364 mg (68% d. Th.)
LC/MS [Methode 1]: Diastereomer 1: $R_t$ = 1.32 min; MS (ESIpos): m/z = 596 $(M+H)^+$; Diastereomer 2: $R_t$ = 1.34 min; MS (ESIpos): m/z = 596 $(M+H)^+$.

**Beispiel 81.1A**

2-[5-Chlor-4-(5-chlor-2-cyanophenyl)-2-oxopyridin-1(2*H*)-yl]-4-methoxybutansäure-*tert*.-butylester (Racemat)

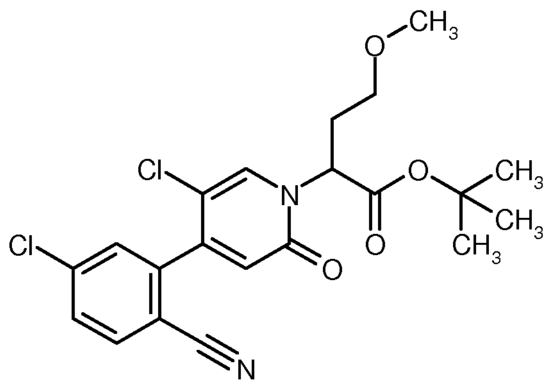

Nach der allgemeinen Methode 7B wurden 500 mg (1.32 mmol) [5-Chlor-4-(5-chlor-2-cyanophenyl)-2-oxopyridin-1(2*H*)-yl]essigsäure-*tert*.-butylester in Gegenwart von 1.58 ml (1.58 mmol, 1.2 eq.) Bis-(trimethylsilyl)-lithiumamid (1M in THF) mit 556 mg (74% Reinheit, 1.98 mmol, 1.5 eq.) 2-Methoxyethyltrifluormethansulfonat umgesetzt. Ausbeute: 455 mg (80% Reinheit, 63% d. Th.)
LC/MS [Methode 1]: $R_t$ = 1.16 min; MS (ESIpos): m/z = 381 (M+H-COO-*tert*.-butyl)$^+$.

**Beispiel 81.1B**

2-[5-Chlor-4-(5-chlor-2-cyanophenyl)-2-oxopyridin-1(2*H*)-yl]-4-methoxybutansäure (Racemat)

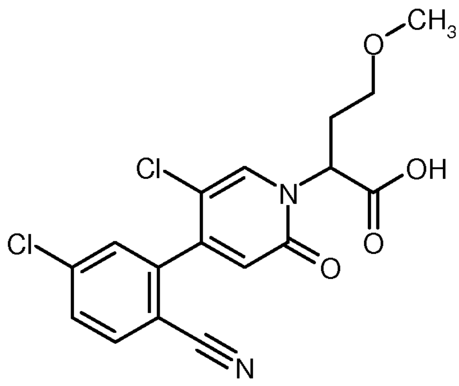

Nach der allgemeinen Methode 6A wurden 455 mg (80% Reinheit, 0.83 mmol) 2-[5-Chlor-4-(5-chlor-2-cyanophenyl)-2-oxopyridin-1(2*H*)-yl]-4-methoxybutansäure-*tert*.-butylester (Racemat) mit TFA verseift. Ausbeute: 417 mg (76% Reinheit, quant.)
LC/MS [Methode 8]: $R_t$ = 0.86 min; MS (ESIpos): m/z = 381 $(M+H)^+$.

**Beispiel 81.1C**

4-({2-[5-Chlor-4-(5-chlor-2-cyanophenyl)-2-oxopyridin-1(2*H*)-yl]-4-methoxybutanoyl}amino)-benzoesäure-*tert*.-butylester (Racemat)

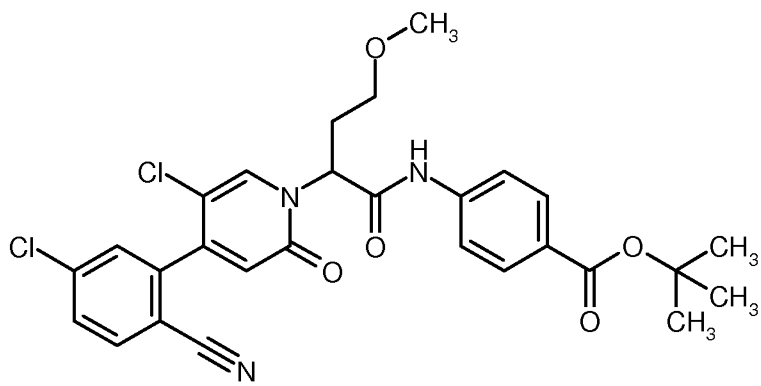

Nach der allgemeinen Methode 5A wurden 417 mg (76% Reinheit, 0.83 mmol) 2-[5-Chlor-4-(5-chlor-2-cyanophenyl)-2-oxopyridin-1(2*H*)-yl]-4-methoxybutansäure (Racemat) mit 177 mg (0.91 mmol, 1.1 eq.) 4-Aminobenzoesäure-*tert.*-butylester umgesetzt. Ausbeute: 438 mg (92% d. Th.)
LC/MS [Methode 1]: $R_t$ = 1.24 min; MS (ESIpos): m/z = 556 $(M+H)^+$.

**Ausführungsbeispiele**

**Allgemeine Methode 1: Amidkupplung mit HATU/DIEA**

Eine Lösung der entsprechenden Carbonsäure (1.0 eq.) in Dimethylformamid (ca. 12 ml/mmol) wurde unter Argon bei RT mit dem entsprechenden Amin (1.1 eq.), *N,N*-Diisopropylethylamin (DIEA) (2.2 eq.) und einer Lösung aus HATU (1.2 eq.) in wenig DMF versetzt. Das Reaktionsgemisch wurde bei RT gerührt. Nach Zugabe von Wasser/Ethylacetat und Phasentrennung wurde die organische Phase mit Wasser und mit gesättigter, wässriger Natriumchlorid-Lösung gewaschen, getrocknet (Natriumsulfat), filtriert und im Vakuum eingeengt. Die Aufreinigung des Rohproduktes erfolgte anschließend mittels präparativer HPLC (Reprosil C18, Wasser-Acetonitril-Gradient oder Wasser-Methanol-Gradient).

**Allgemeine Methode 2: Verseifung eines *tert.*-Butylesters oder eines Boc-geschützten Amins mittels TFA**

Eine Lösung des entsprechenden *tert.*-Butylestersderivates oder eines Boc-geschützten Amins (1.0 eq.) in Dichlormethan (ca. 25 ml/mmol) wurde bei RT mit TFA (20 eq.) versetzt und bei RT für 1-8 h gerührt. Anschließend wurde das Reaktionsgemisch im Vakuum eingeengt. Der Rückstand wurde mehrmals mit Dichlormethan und/oder Toluol coevaporiert. Die Aufreinigung des Rohproduktes erfolgte anschließend mittels präparative RP-HPLC (Eluent: Acetonitril-Wasser-Gradient oder Wasser-Methanol-Gradient).

**Allgemeine Methode 3: Verseifung eines Methyl- oder Ethylesters mit Lithiumhydroxid**

Eine Lösung des entsprechenden Esters (1.0 eq.) in einem Gemisch aus Tetrahydrofuran/Wasser (3:1, ca. 15 ml/mmol) wurde bei RT mit Lithiumhydroxid (2-4 eq.) versetzt und bei RT gerührt. Anschließend wurde das Reaktionsgemisch mit wässriger Salzsäure-Lösung (1N) auf pH 1 gestellt. Nach Zugabe von Wasser/Ethylacetat wurde die wässrige Phase dreimal mit Ethylacetat extrahiert. Die vereinigten, organischen Phasen wurden getrocknet (Natriumsulfat), filtriert und im Vakuum eingeengt. Das Rohprodukt wurde anschließend entweder mittels Flash-Chromatographie (Kieselgel-60, Eluent: Cyclohexan-Ethylacetat-Gemische oder Dichlormethan-Methanol-Gemische) oder präparativer HPLC (Reprosil C18, Wasser-Acetonitril-Gradient oder Wasser-Methanol-Gradient) aufgereinigt.

**Allgemeine Methode 4: Verseifung eines Methyl- oder Ethylesters mit Cäsiumcarbonat**

Zu einer Lösung des entsprechenden Methyl- oder Ethylesters (1 eq.) in einer Mischung aus Methanol/Wasser (4/1, 0.05-0.2M) wurde Cäsiumcarbonat (2 eq.) gegeben und die resultierende Suspension 3-8 h bei RT bis 60°C gerührt. Das Reaktionsgemisch wurde gegebenenfalls auf RT abgekühlt und mit wässriger Salzsäure (1N) auf pH 3 eingestellt. Methanol wurde bei 30°C im Vakuum entfernt. Die wässrige Phase wurde mit Essigsäureethylester extrahiert. Die vereinigten, organischen Phasen wurden getrocknet (Natriumsulfat oder Magnesiumsulfat), filtriert und unter reduziertem Druck eingeengt. Der Rückstand wurde mittels präparativer HPLC aufgereinigt.

**Allgemeine Methode 5: Amid-Kupplung mit OXIMA/DIC**

Zu einer entgasten Lösung der entsprechenden Carbonsäure (1 eq.), Anilin (1 eq.) und Hydroxyiminocyanoessigsäureethylester (Oxima) (0.1-1 eq.) in Dimethylformamid (0.1M) wurde tropfenweise *N*,*N*'-Diisopropylcarbodiimid (DIC) (1 eq.) gegeben und die resultierende Reaktionslösung für 8-24 h bei RT-40°C gerührt. Das Lösungsmittel wurde unter reduziertem Druck entfernt. Der Rückstand wurde entweder mit Wasser versetzt und das gewünschte Produkt filtriert oder mittels Normalphasen-Chromatographie (Cyclohexan/Essigsäureethylester-Gradient) oder präparativer RP-HPLC (Wasser-Acetonitril-Gradient oder Wasser-Methanol-Gradient) aufgereinigt.

**Allgemeine Methode 6: Amid-Kupplung mit T3P/DIEA**

Eine Lösung der Carbonsäure und des entsprechenden Amins (1.1-1.5 eq.) in Dimethylformamid (0.15-0.05 mmol) wurde unter Argon bei 0°C tropfenweise mit *N,N*-Diisopropylethylamin (3 eq.) und Propylphosphonsäureanhydrid (T3P, 50%ig in Dimethylformamid, 3 eq.) versetzt. Das Reaktionsgemisch wurde bei RT gerührt und anschließend im Vakuum eingeengt. Nach Zugabe von Wasser/Essigsäureethylester und Phasentrennung wurde die wässrige Phase zweimal mit Essigsäureethylester extrahiert. Die vereinigten, organischen Phasen wurden getrocknet (Natriumsulfat oder Magnesiumsulfat), filtriert und im Vakuum eingeengt. Das Rohprodukt wurde anschließend entweder mittels Flash-Chromatographie (Kieselgel-60, Eluent: Cyclohexan-Essigsäureethylester-Gemische oder Dichlormethan-Methanol-Gemische) oder präparativer HPLC (Reprosil C18, Wasser-Acetonitril-Gradient oder Wasser-Methanol-Gradient) aufgereinigt.

**Allgemeine Methode 7: Amid-Kupplung mit T3P/Pyridin**

Eine Lösung der Carbonsäure (1 eq.) und des entsprechenden Amins (1.5 eq.) in Pyridin (0.15-0.05M) wurde unter Argon bei 0°C tropfenweise mit Propylphosphonsäureanhydrid (T3P, 50%ig in Essigsäureethylester, 4 eq.) versetzt. Dieses Gemisch wurde auf 90°C erhitzt und 1-20 h bei 90°C gerührt. Das Reaktionsgemisch wurde auf RT gekühlt und mit Wasser und Essigsäureethylester versetzt. Nach Phasentrennung wurde die wässrige Phase mit Essigsäureethylester extrahiert. Die vereinigten, organischen Phasen wurden mit wässriger PufferLösung (pH 5), mit gesättigter, wässriger Natriumhydrogencarbonat-Lösung und mit gesättigter, wässriger Natriumchlorid-Lösung gewaschen, getrocknet (Natriumsulfat oder Magnesiumsulfat), filtriert und im Vakuum eingeengt. Das Rohprodukt wurde gegebenenfalls anschließend entweder mittels Normalphasen-Chromatographie (Eluent: Cyclohexan-Essigsäureethylester-Gemische

oder Dichlormethan-Methanol-Gemische) oder präparativer RP-HPLC (Wasser-Acetonitril-Gradient oder Wasser-Methanol-Gradient) aufgereinigt.

Die Beispiele 1-33, 53, 54, 62 und 131 sind nicht Teil der Erfindung.

## Beispiel 1

2-[4-(5-Chlor-2-cyanophenyl)-2-oxopyridin-1(2*H*)-yl]-*N*-[4-(1*H*-tetrazol-5-yl)phenyl]-propansäureamid (Racemat)

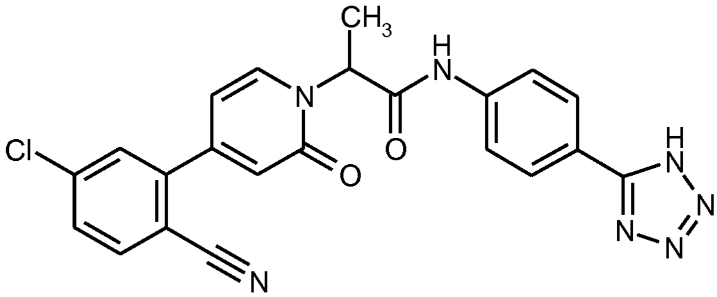

Nach der allgemeinen Methode 1 wurden 65 mg (83% Reinheit, 0.18 mmol) 2-[4-(5-Chlor-2-cyanophenyl)-2-oxopyridin-1(2*H*)-yl]propansäure (Racemat) (Beispiel 2.2B) mit 1.2 eq. 4-(1*H*-Tetrazol-5-yl)anilin umgesetzt. Ausbeute: 17 mg (22% d. Th.)

LC/MS [Methode 1]: $R_t$ = 0.82 min; MS (ESIpos): m/z = 446 $(M+H)^+$

$^1$H-NMR (400 MHz, DMSO-$d_6$): $\delta$ [ppm] = 10.78 (s, 1H), 8.08-7.94 (m, 4H), 7.87-7.80 (m, 3H), 7.78 (dd, 1H), 6.67 (d, 1H), 6.58 (d, 1H), 5.59 (q, 1H), 1.71 (d, 3H).

## Beispiel 2

2-[4-(5-Chlor-2-cyanophenyl)-2-oxopyridin-1(2*H*)-yl]-*N*-{4-[3-(trifluormethyl)-1*H*-1,2,4-triazol-5-yl]phenyl}propansäureamid (Racemat)

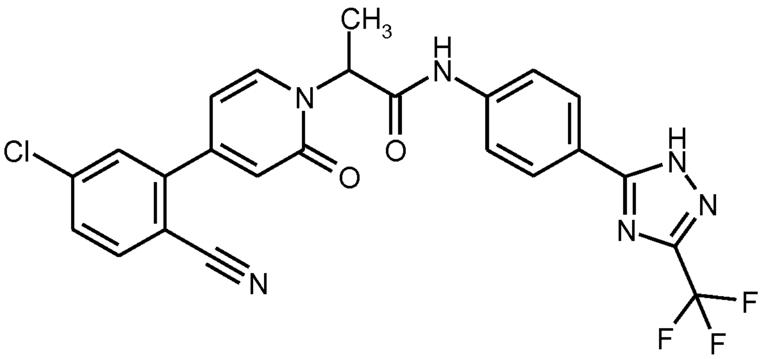

Nach der allgemeinen Methode 1 wurden 95 mg (83% Reinheit, 0.26 mmol) 2-[4-(5-Chlor-2-cyanophenyl)-2-oxopyridin-1(2*H*)-yl]propansäure (Racemat) (Beispiel 2.2B) und 75 mg (0.31 mmol) 4-[3-(Trifluormethyl)-1*H*-1,2,4-triazol-5-yl]anilin (Beispiel 1.1C) umgesetzt. Ausbeute: 30 mg (92% Reinheit, 21% d. Th.)

LC/MS [Methode 1]: $R_t$ = 1.06 min; MS (ESIpos): m/z = 513 $(M+H)^+$

$^1$H-NMR (400 MHz, DMSO-$d_6$): $\delta$ [ppm] = 15.17 (s, 1H), 10.75 (s, 1H), 8.04 (d, 1H), 8.00 (d, 2H), 7.96 (d, 1H), 7.81 (m, 3H), 7.77 (dd, 1H), 6.67 (d, 1H), 6.57 (dd, 1H), 5.59 (q, 1H), 1.71 (d, 3H).

**Beispiel 3**

2-[4-(5-Chlor-2-cyanophenyl)-2-oxopyridin-1(2*H*)-yl]-*N*-{4-[3-(methoxymethyl)-1*H*-1,2,4-triazol-5-yl]phenyl}propansäureamid (Racemat)

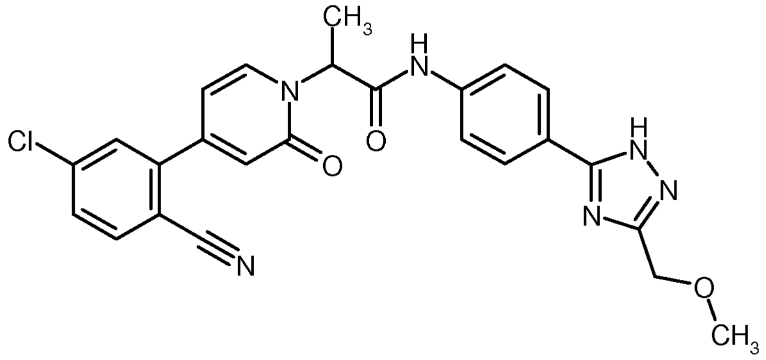

Nach der allgemeinen Methode 1 wurden 90 mg (93% Reinheit, 0.28 mmol) 2-[4-(5-Chlor-2-cyanophenyl)-2-oxopyridin-1(2*H*)-yl]propansäure (Racemat) (Beispiel 2.2B) und 80 mg (0.33 mmol) 4-[3-(Methoxymethyl)-1*H*-1,2,4-triazol-5-yl]anilin-Monohydrochlorid umgesetzt. Ausbeute: 45 mg (33% d. Th.)
LC/MS [Methode 1]: $R_t$ = 0.87 min; MS (ESIpos): m/z = 489 $(M+H)^+$
$^1$H-NMR (400 MHz, DMSO-$d_6$): δ [ppm] = 10.62 (br. s, 1H), 8.04 (d, 1H), 7.99-7.92 (m, 3H), 7.82 (d, 1H), 7.77 (dd, 1H), 7.73 (m, 2H), 6.67 (d, 1H), 6.56 (dd, 1H), 5.60 (q, 1H), 1.70 (d, 3H).

**Beispiel 4**

2-[4-(5-Chlor-2-cyanophenyl)-2-oxopyridin-1(2*H*)-yl]-*N*-[4-(1*H*-imidazol-2-yl)phenyl]-propansäureamid (Racemat)

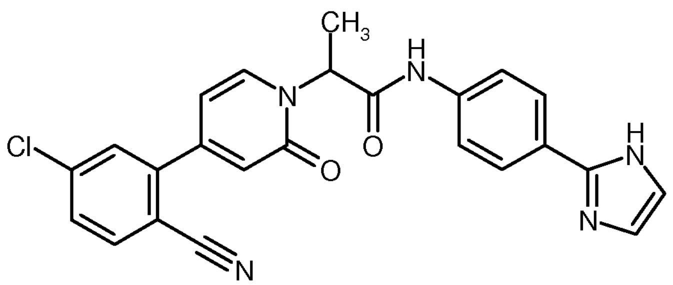

Nach der allgemeinen Methode 1 wurden 71 mg (0.23 mmol) 2-[4-(5-Chlor-2-cyanophenyl)-2-oxopyridin-1(2*H*)-yl]propansäure (Racemat) (Beispiel 2.2B) und 40 mg (0.25 mmol) 4-(1*H*-Imidazol-2-yl)anilin (Beispiel 1.2A) umgesetzt. Ausbeute: 4 mg (4% d. Th.)
LC/MS [Methode 1]: $R_t$ = 0.66 min; MS (ESIpos): m/z = 444 $(M+H)^+$
$^1$H-NMR (400 MHz, DMSO-$d_6$): δ [ppm] = 12.39 (br. s, 1H), 10.56 (br. s, 1H), 8.05 (d, 1H), 7.96 (d, 1H), 7.88 (d, 2H), 7.82 (d, 1H), 7.77 (dd, 1H), 7.67 (d, 2H), 7.21 (br. s, 1H), 6.99 (br. s, 1H), 6.67 (d, 1H), 6.56 (dd, 1H), 5.60 (q, 1H), 1.70 (d, 3H).

**Beispiel 5**

2-[4-(5-Chlor-2-cyanophenyl)-2-oxopyridin-1(2*H*)-yl]-N-[4-(1*H*-imidazol-4-yl)phenyl]-propansäureamid (Racemat)

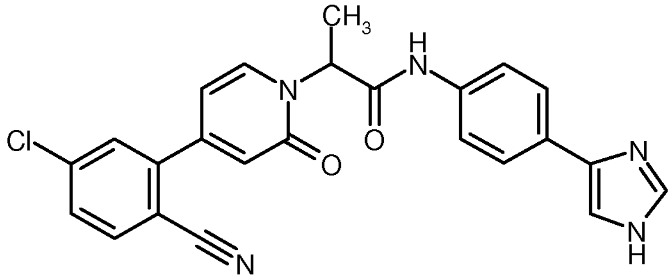

Nach der allgemeinen Methode 1 wurden 78 mg (0.25 mmol) 2-[4-(5-Chlor-2-cyanophenyl)-2-oxopyridin-1(2*H*)-yl]propansäure (Racemat) (Beispiel 2.2B) und 44 mg (0.28 mmol) 4-(1*H*-Imidazol-4-yl)anilin umgesetzt. Ausbeute: 40 mg (36% d. Th.)

LC/MS [Methode 1]: $R_t$ = 0.74 min; MS (ESIpos): m/z = 444 $(M+H)^+$

$^1$H-NMR (400 MHz, DMSO-$d_6$): $\delta$ [ppm] = 12.13 (br. s, 1H), 10.45 (s, 1H), 8.05 (d, 1H), 7.96 (d, 1H), 7.83 (d, 1H), 7.77 (dd, 1H), 7.74-7.66 (m, 3H), 7.61 (d, 2H), 7.52 (br. s, 1H), 6.67 (d, 1H), 6.56 (dd, 1H), 5.61 (q, 1H), 1.69 (d, 3H).

## Beispiel 6

2-[4-(5-Chlor-2-cyanophenyl)-2-oxopyridin-1(2/7)-yl]-*N*-{4-[2-(trifluormethyl)-1*H*-imidazol-5-yl]phenyl}propansäureamid (Racemat)

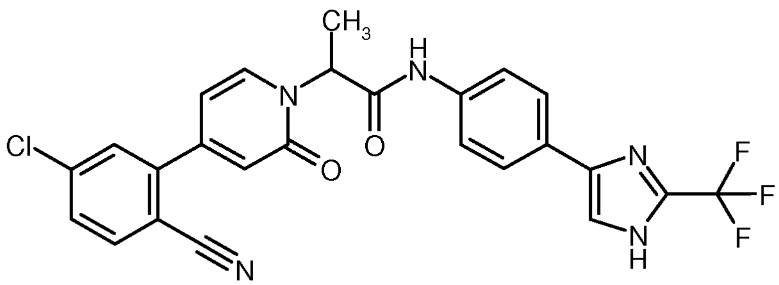

Nach der allgemeinen Methode 1 wurden 67 mg (0.22 mmol) 2-[4-(5-Chlor-2-cyanophenyl)-2-oxopyridin-1(2*H*)-yl]propansäure (Racemat) (Beispiel 2.2B) und 49 mg (0.22 mmol) 4-[2-(Trifluormethyl)-1*H*-imidazol-5-yl]anilin (Beispiel 1.3B) umgesetzt. Ausbeute: 26 mg (22% d. Th.)

LC/MS [Methode 1]: $R_t$ = 1.00 min; MS (ESIpos): m/z = 512 $(M+H)^+$

$^1$H-NMR (400 MHz, DMSO-$d_6$): $\delta$ [ppm] = 13.66 (br. s, 1H), 10.51 (s, 1H), 8.05 (d, 1H), 7.96 (d, 1H), 7.89 (s, 1H), 7.83 (d, 1H), 7.81-7.74 (m, 3H), 7.65 (d, 2H), 6.67 (d, 1H), 6.56 (dd, 1H), 5.61 (q, 1H), 1.70 (d, 3H).

## Beispiel 7

2-[4-(5-Chlor-2-cyanophenyl)-2-oxopyridin-1(2*H*)-yl]-*N*-[4-(5-oxo-2,5-dihydro-1*H*-pyrazol-3-yl)phenyl]propansäureamid (Racemat)

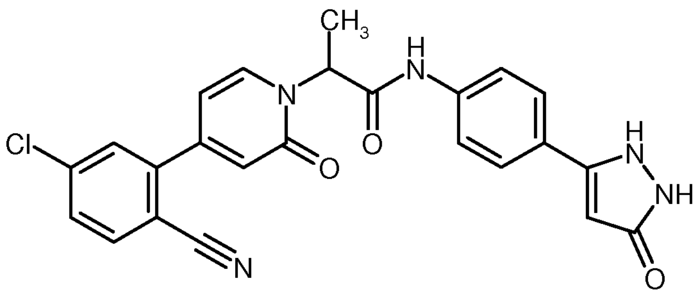

Nach der allgemeinen Methode 2 wurden 110 mg (0.20 mmol) 5-[4-({2-[4-(5-Chlor-2-cyanophenyl)-2-oxopyridin-1(2*H*)-yl]propanoyl}amino)phenyl]-3-oxo-2,3-dihydro-1*H*-pyrazol-1-carbonsäure-*tert*.-butylester (Racemat) (Beispiel 2.3A) mit TFA verseift. Ausbeute: 24 mg (27% d. Th.)
LC/MS [Methode 1]: $R_t$ = 0.87 min; MS (ESIpos): m/z = 460 $(M+H)^+$
$^1$H-NMR (400 MHz, DMSO-$d_6$): $\delta$ [ppm] = 11.79 (br. s, 1H), 10.55 (s, 1H), 9.57 (br. s, 1H), 8.04 (d, 1H), 7.96 (d, 1H), 7.83 (d, 1H), 7.77 (dd, 1H), 7.68-7.59 (m, 4H), 6.67 (d, 1H), 6.56 (dd, 1H), 5.84 (br. s, 1H), 5.60 (q, 1H), 1.70 (d, 3H).

**Beispiel 8**

2-[4-(5-Chlor-2-cyanophenyl)-2-oxopyridin-1(2*H*)-yl]-*N*-[4-(5-oxo-4,5-dihydro-1,2,4-oxadiazol-3-yl)phenyl]propansäureamid (Racemat)

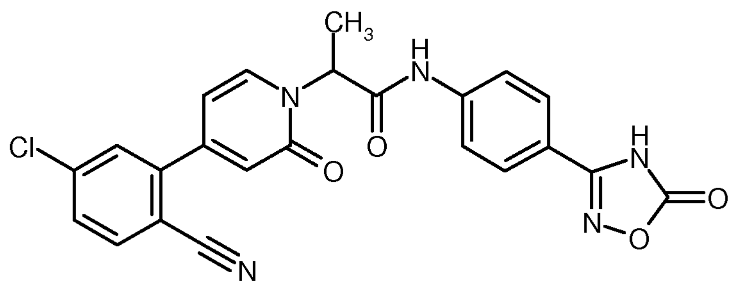

Nach der allgemeinen Methode 1 wurden 120 mg (93% Reinheit, 0.37 mmol) 2-[4-(5-Chlor-2-cyanophenyl)-2-oxopyridin-1(2*H*)-yl]propansäure (Racemat) (Beispiel 2.2B) mit 1.2 eq. 3-(4-Aminophenyl)-1,2,4-oxadiazol-5(4*H*)-on (Beispiel 1.5A) umgesetzt. Ausbeute: 23 mg (13% d. Th.)
LC/MS [Methode 1]: $R_t$ = 0.91 min; MS (ESIpos): m/z = 462 $(M+H)^+$
$^1$H-NMR (400 MHz, DMSO-$d_6$): $\delta$ [ppm] = 12.88 (br. s, 1H), 10.81 (s, 1H), 8.05 (d, 1H), 7.96 (d, 1H), 7.82 (d, 1H), 7.81-7.78 (m, 4H), 7.77 (dd, 1H), 6.67 (s, 1H), 6.57 (d, 1H), 5.57 (q, 1H), 1.71 (d, 3H).

**Beispiel 9**

4-({2-[4-(5-Chlor-2-cyanophenyl)-2-oxopyridin-1(2*H*)-yl]propanoyl}amino)benzoesäure (Racemat)

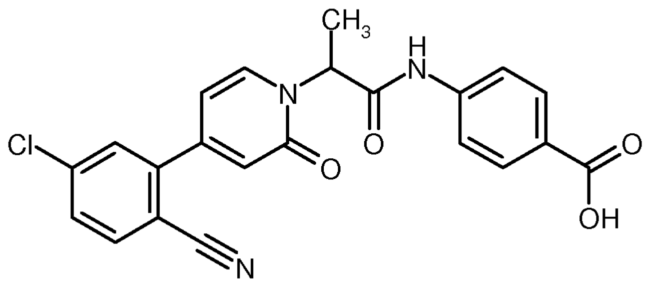

Nach der Allgemeinen Methode 2 wurden 43 mg (0.09 mmol) 4-({2-[4-(5-Chlor-2-cyanophenyl)-2-oxopyridin-1(2*H*)-yl]propanoyl}amino)benzoesäure-*tert*.-butylester (Racemat) (Beispiel 2.2C) mit TFA verseift. Ausbeute: 20 mg (89% Reinheit, 48% d. Th.)
LC/MS [Methode 1]: $R_t$ = 0.88 min; MS (ESIpos): m/z = 422 $(M+H)^+$
$^1$H-NMR (400 MHz, DMSO-$d_6$): $\delta$ [ppm] = 12.73 (br. s, 1H), 10.74 (s, 1H), 8.04 (d, 1H), 7.95 (d, 1H), 7.91 (d, 2H), 7.82 (d, 1H), 7.76 (dd, 1H), 7.72 (d, 2H), 6.66 (d, 1H), 6.56 (dd, 1H), 5.58 (q, 1H), 1.70 (s, 3H).

**Beispiel 10**

4-({2-[4-(5-Chlor-2-ethinylphenyl)-2-oxopyridin-1(2*H*)-yl]propanoyl}amino)benzoesäure (Racemat)

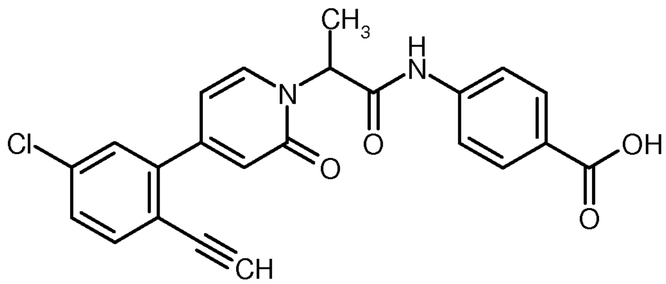

Nach der allgemeinen Methode 3 wurden 15 mg (0.03 mmol) 4-({2-[4-(5-Chlor-2-ethinylphenyl)-2-oxopyridin-1(2*H*)-yl]propanoyl}amino)benzoesäuremethylester (Racemat) (Beispiel 2.8F) mit Lithiumhydroxid verseift. Ausbeute: 4 mg (92% Reinheit, 28% d. Th.)
LC/MS [Methode 1]: $R_t$ = 0.98 min; MS (ESIpos): m/z = 421 $(M+H)^+$
$^1$H-NMR (400 MHz, DMSO-$d_6$): δ [ppm] = 12.72 (br. s, 1H), 10.75 (s, 1H), 7.91 (d, 2H), 7.86 (d, 1H), 7.73 (d, 2H), 7.65 (d, 1H), 7.58 (d, 1H), 7.54 (dd, 1H), 6.60 (d, 1H), 6.53 (dd, 1H), 5.57 (q, 1H), 3.57 (s, 1H), 1.68 (d, 3H).

**Beispiel 11**

4-({2-[4-(2,5-Dichlorphenyl)-2-oxopyridin-1(2*H*)-yl]propanoyl}amino)benzoesäure (Racemat)

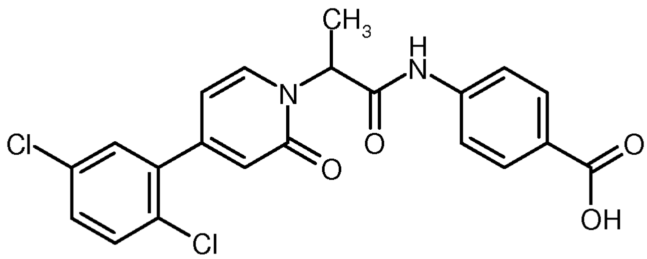

Nach der allgemeinen Methode 2 wurden 83 mg (0.17 mmol) 4-({2-[4-(2,5-Dichlorphenyl)-2-oxopyridin-1(2*H*)-yl]propanoyl}amino)benzoesäure-*tert*.-butylester (Racemat) (Beispiel 2.9C) mit TFA verseift. Ausbeute: 21 mg (88% Reinheit, 23% d. Th.)
LC/MS [Methode 1]: $R_t$ = 0.99 min; MS (ESIpos): m/z = 431 $(M+H)^+$
$^1$H-NMR (400 MHz, DMSO-$d_6$): δ [ppm] = 10.70 (s, 1H), 7.92-7.84 (m, 3H), 7.68 (d, 2H), 7.63 (d, 1H), 7.59-7.52 (m, 2H), 6.48 (d, 1H), 6.42 (dd, 1H), 5.58 (q, 1H), 1.68 (d, 3H).

**Beispiel 12**

4-({2-[4-(2-Brom-5-chlorphenyl)-2-oxopyridin-1(2*H*)-yl]propanoyl}amino)benzoesäure (Racemat)

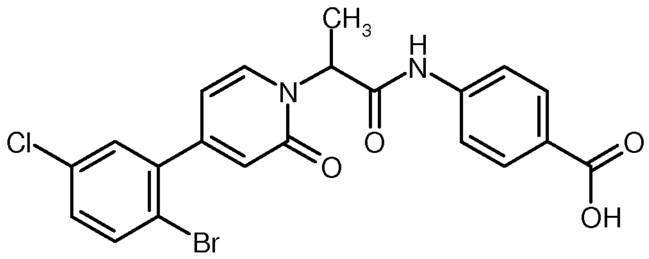

Nach der allgemeinen Methode 2 wurden 96 mg (0.18 mmol) 4-({2-[4-(2-Brom-5-chlorphenyl)-2-oxopyridin-1(2*H*)-yl]propanoyl}amino)benzoesäure-*tert*.-butylester (Racemat) (Beispiel 2.10C) mit TFA verseift. Ausbeute: 69 mg (82% d. Th.)

LC/MS [Methode 1]: $R_t$ = 1.03 min; MS (ESIpos): m/z = 475 $(M+H)^+$

$^1$H-NMR (400 MHz, DMSO-$d_6$): $\delta$ [ppm] = 12.77 (br. s, 1H), 10.76 (s, 1H), 7.91 (d, 2H), 7.87 (d, 1H), 7.79 (d, 1H), 7.73 (d, 2H), 7.53 (d, 1H), 7.46 (dd, 1H), 6.43 (s, 1H), 6.38 (dd, 1H), 5.57 (q, 1H), 1.68 (d, 3H).

**Beispiel 13**

4-{[2-{4-[5-Chlor-2-(trifluormethyl)phenyl]-2-oxopyridin-1(2*H*)-yl}propanoyl]amino}benzoesäure (Racemat)

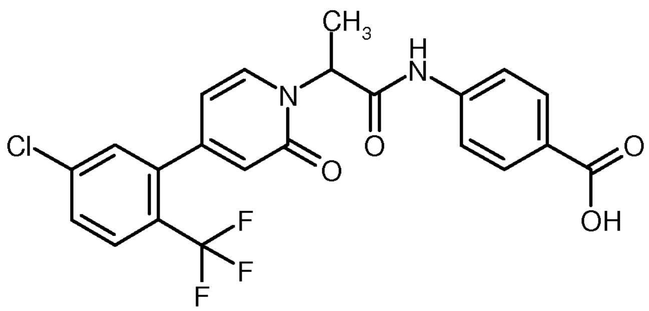

Nach der allgemeinen Methode 2 wurden 104 mg (0.20 mmol) 4-[(2-{4-[5-Chlor-2-(trifluormethyl)phenyl] -2-oxopyridin-1(2*H*)-yl}propanoyl)amino]benzosäure-*tert*.-butylester (Racemat) (Beispiel 2.11C) mit TFA verseift. Ausbeute: 67 mg (73% d. Th.)

LC/MS [Methode 1]: $R_t$ = min; MS (ESIpos): m/z = 465 $(M+H)^+$

$^1$H-NMR (400 MHz, DMSO-$d_6$): $\delta$ [ppm] = 12.76 (br. s, 1H), 10.74 (s, 1H), 7.93-7.84 (m, 4H), 7.78-7.70 (m, 3H), 7.60 (d, 1H), 6.38 (d, 1H), 6.33 (dd, 1H), 5.57 (q, 1H), 1.68 (d, 3H).

**Beispiel 14**

4-({2-[4-(5-Chlor-2-cyanophenyl)-2-oxopyridin-1(2*H*)-yl]propanoyl}amino)-2-fluorbenzoesäure (Racemat)

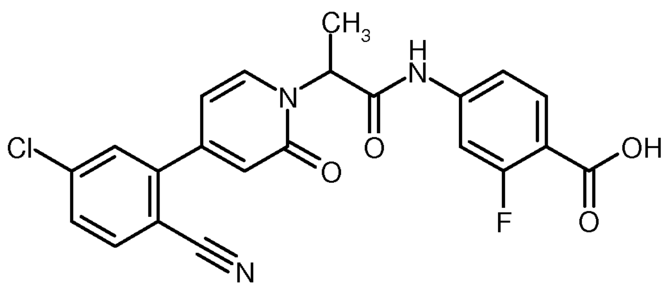

Nach der allgemeinen Methode 3 wurden 82 mg (0.17 mmol) 4-({2-[4-(5-Chlor-2-cyanophenyl)-2-oxopyridin-1(2*H*)-yl]propanoyl}amino)-2-fluorbenzoesäuremethylester (Racemat) (Beispiel 2.4A) mit Lithiumhydroxid verseift. Ausbeute: 8 mg (93% Reinheit, 10% d. Th.)
LC/MS [Methode 1]: $R_t$ = 0.92 min; MS (ESIpos): m/z = 440 $(M+H)^+$
$^1$H-NMR (400 MHz, DMSO-$d_6$): δ [ppm] = 13.05 (br. s, 1H), 10.93 (s, 1H), 8.05 (d, 1H), 7.95 (d, 1H), 7.86 (t, 1H), 7.83 (d, 1H), 7.77 (dd, 1H), 7.66 (d, 1H), 7.40 (d, 1H), 6.66 (d, 1H), 6.56 (dd, 1H), 5.53 (q, 1H), 1.70 (d, 3H).

**Beispiel 15**

2-Chlor-4-({2-[4-(5-chlor-2-cyanophenyl)-2-oxopyridin-1(2*H*)-yl]propanoyl}amino)benzoesäure (Racemat)

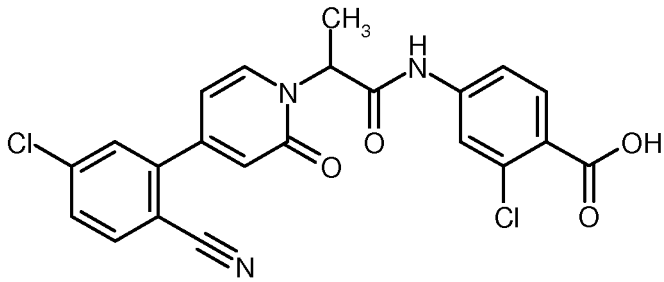

Nach der allgemeinen Methode 3 wurden 100 mg (94% Reinheit, 0.20 mmol) 2-Chlor-4-({2-[4-(5-chlor-2-cyanophenyl)-2-oxopyridin-1(2*H*)-yl]propanoyl}amino)benzoesäuremethylester (Racemat) (Beispiel 2.5A) mit Lithiumhydroxid verseift. Ausbeute: 8 mg (93% Reinheit, 10% d. Th.)
LC/MS [Methode 1]: $R_t$ = 0.93 min; MS (ESIpos): m/z = 456 $(M+H)^+$
$^1$H-NMR (400 MHz, DMSO-$d_6$): δ [ppm] = 13.14 (br. s, 1H), 10.83 (s, 1H), 8.04 (d, 1H), 7.94 (d, 1H), 7.90 (d, 1H), 7.86 (d, 1H), 7.81 (d, 1H), 7.76 (dd, 1H), 7.57 (dd, 1H), 6.67 (d, 1H), 6.56 (dd, 1H), 5.52 (q, 1H), 1.70 (d, 3H).

**Beispiel 16**

4-(12-[4-(5-Chlor-2-cyanophenyl)-2-oxopyridin-1(2*H*)-yl]propanoyl}amino)-2-methylbenzoesäure (Racemat)

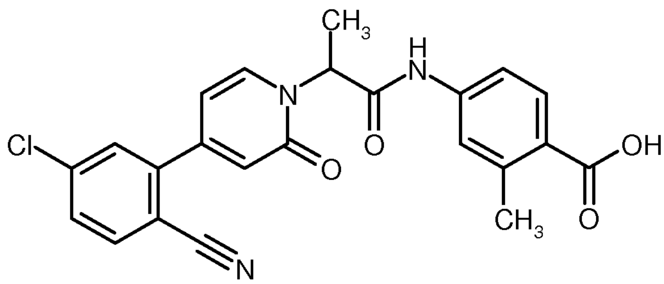

Nach der allgemeinen Methode 3 wurden 120 mg (0.27 mmol) 4-({2-[4-(5-Chlor-2-cyanophenyl)-2-oxopyridin-1(2*H*)-yl]propanoyl}amino)-2-methylbenzoesäuremethylester (Racemat) (Beispiel 2.6A) mit Lithiumhydroxid verseift. Ausbeute: 39 mg (93% Reinheit, 33% d. Th.)
LC/MS [Methode 1]: $R_t$ = 0.91 min; MS (ESIpos): m/z = 436 $(M+H)^+$
$^1$H-NMR (400 MHz, DMSO-$d_6$): δ [ppm] = 12.61 (br. s, 1H), 10.64 (s, 1H), 8.05 (d, 1H), 7.94 (d, 1H), 7.86-7.81 (m, 2H), 7.76 (dd, 1H), 7.56-7.49 (m, 2H), 6.66 (d, 1H), 6.56 (dd, 1H), 5.56 (q, 1H), 1.69 (d, 3H).

**Beispiel 17**

2-[4-(5-Chlor-2-cyanophenyl)-2-oxopyridin-1(2*H*)-yl]-*N*-(2-methyl-3-oxo-2,3-dihydro-1*H*-indazol-6-yl)propansäureamid (Racemat)

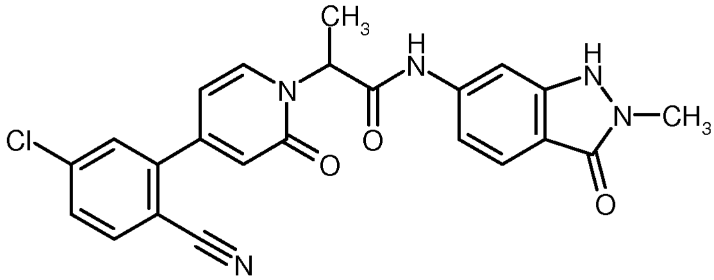

Nach der allgemeinen Methode 2 wurden 75 mg (0.14 mmol) 6-({2-[4-(5-Chlor-2-cyanophenyl)-2-oxopyridin-1(2*H*)-yl]propanoyl}amino)-2-methyl-3-oxo-2,3-dihydro-1*H*-indazol-1-carbonsäure-*tert*.-butylester (Racemat) (Beispiel 2.7A) mit TFA verseift. Ausbeute: 37 mg (60% d. Th.)
LC/MS [Methode 1]: $R_t$ = 0.79 min; MS (ESIpos): m/z = 448 $(M+H)^+$
$^1$H-NMR (400 MHz, DMSO-$d_6$): $\delta$ [ppm] = 10.65 (s, 1H), 10.20 (s, 1H), 8.05 (d, 1H), 7.96 (d, 1H), 7.82 (d, 1H), 7.77 (dd, 1H), 7.74 (s, 1H), 7.56 (d, 1H), 7.13 (dd, 1H), 6.66 (d, 1H), 6.56 (dd,1H), 5.58 (q, 1H), 1.70 (d, 3H).

**Beispiel 18**

N-(1*H*-Benzimidazol-6-yl)-2-[4-(5-chlor-2-cyanophenyl)-2-oxopyridin-1(2*H*)-yl]propansäureamid (Racemat)

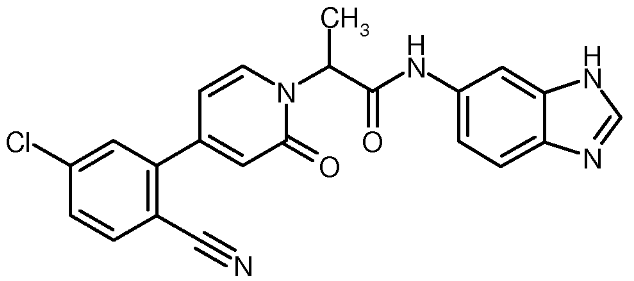

Nach der allgemeinen Methode 1 wurden 65 mg (83% Reinheit, 0.18 mmol) 2-[4-(5-Chlor-2-cyanophenyl)-2-oxopyridin-1(2*H*)-yl]propansäure (Racemat) (Beispiel 2.2B) und 26 mg (0.20 mmol) 1*H*-Benzimidazol-6-amin umgesetzt. Ausbeute: 11 mg (15% d. Th.)
LC/MS [Methode 1]: $R_t$ = 0.64 min; MS (ESIpos): m/z = 418 $(M+H)^+$
$^1$H-NMR (400 MHz, DMSO-$d_6$): $\delta$ [ppm] = 12.39 (s, 1H), 10.46 (s, 1H), 8.15 (s, 1H), 8.08-7.95 (m, 3H), 7.83 (d, 1H), 7.77 (dd, 1H), 7.61-7.21 (m, 2H), 6.68 (d, 1H), 6.57 (dd, 1H), 5.63 (m, 1H), 1.69 (d, 3H).

**Beispiel 19**

2-[4-(5-Chlor-2-cyanophenyl)-2-oxopyridin-1(2*H*)-yl]-*N*-[4-(1*H*-tetrazol-5-yl)phenyl]butan-säureamid (Racemat)

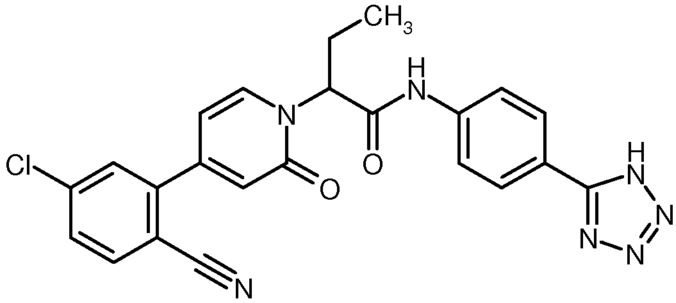

Nach der allgemeinen Methode 1 wurden 212 mg (60% Reinheit, 0.4 mmol) 2-[4-(5-Chlor-2-cyanophenyl)-2-oxopyridin-1(2*H*)-yl]butansäure (Racemat) (Beispiel 3.1C) und 31 mg (0.19 mmol) 4-(1*H*-Tetrazol-5-yl)anilin umgesetzt.
Ausbeute: 143 mg (77% d. Th.)
LC/MS [Methode 1]: $R_t$ = 0.88 min; MS (ESIpos): m/z = 460 $(M+H)^+$
$^1H$-NMR (400 MHz, DMSO-$d_6$): δ [ppm] = 16.77 (br. s, 1H), 10.87 (s, 1H), 8.05 (d, 1H), 8.01 (d, 2H), 7.98 (d, 1H), 7.87-7.82 (m, 3H), 7.77 (dd, 1H), 6.69 (d, 1H), 6.57 (dd, 1H), 5.62 (q, 1H), 2.25-2.15 (m, 1H), 2,15-2.04 (m, 1H), 0.92 (t, 3H).

**Beispiel 20**

2-[4-(5-Chlor-2-cyanophenyl)-2-oxopyridin-1(2*H*)-yl]-*N*-[4-(1*H*-tetrazol-5-yl)phenyl]butan-säureamid (Enantiomer 1)

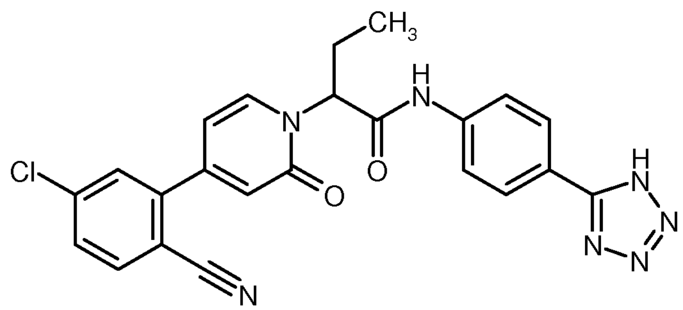

Enantiomerentrennung von 140 mg des Racemates aus Beispiel 19 ergab 51 mg der Titelverbindung Beispiel 20 (Enantiomer 1): Chirale HPLC: $R_t$ = 4.7 min; 99% ee.

Trennmethode: Säule: Daicel Chiralpak AZ-H 5 μm 250x20 mm; Eluent A: iso-Hexan, Eluent B: Ethanol + 0.2% Essigsäure; Gradient: 0.0 min 70% A → 3 min 70% A → 20 min 0% A → 30 min 0% A; Ofen: 45°C; Fluss: 15 ml/min; UV-Detektion: 220 nm.

Analytik: Säule: Daicel Chiralpak AZ-H 5 μm 250x4.6 mm; Eluent: 90% iso-Hexan, 10% Ethanol; Ofen: 30°C; Fluss: 1 ml/min; UV-Detektion: 220 nm.

**Beispiel 21**

2-[4-(5-Chlor-2-cyanophenyl)-2-oxopyridin-1(2*H*)-yl]-*N*-[4-(1*H*-tetrazol-5-yl)phenyl]butansäureamid (Enantiomer 2)

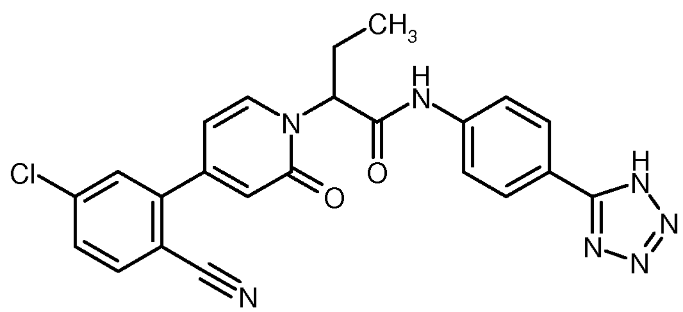

Enantiomerentrennung von 140 mg des Racemates aus Beispiel 19 ergab 59 mg der Titelverbindung Beispiel 21 (Enantiomer 2): Chirale HPLC: $R_t$ = 10.7 min; 99% ee.

Trennmethode: Säule: Daicel Chiralpak AZ-H 5 μm 250x20 mm; Eluent A: iso-Hexan, Eluent B: Ethanol + 0.2% Essigsäure; Gradient: 0.0 min 70% A → 3 min 70% A → 20 min 0% A → 30 min 0% A; Ofen: 45°C; Fluss: 15 ml/min; UV-Detektion: 220 nm; Probenvorbereitung: 140 mg Racemat in 0.5 ml Triethanolamin/5.5 ml Ethanol gelöst.

Analytik: Säule: Daicel Chiralpak AZ-H 5 μm 250x4.6 mm; Eluent: 90% iso-Hexan, 10% Ethanol;

Ofen: 30°C; Fluss: 1 ml/min; UV-Detektion: 220 nm.

**Beispiel 22**

2-[4-(5-Chlor-2-cyanophenyl)-2-oxopyridin-1(2*H*)-yl]-3-methyl-*N*-[4-(1*H*-tetrazol-5-yl)phenyl]-butansäureamid (Racemat)

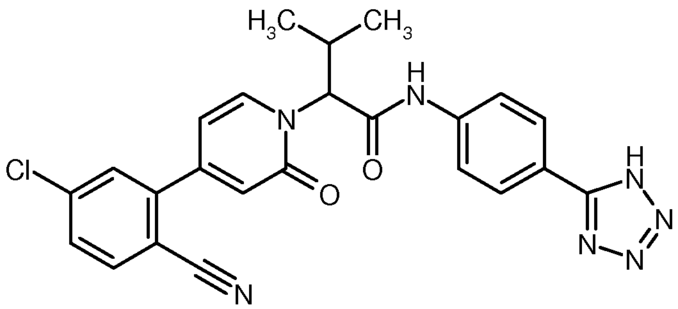

Nach der allgemeinen Methode 1 wurden 79 mg (86% Reinheit, 0.21 mmol) 2-[4-(5-Chlor-2-cyanophenyl)-2-oxopyridin-1(2*H*)-yl]-3-methylbutansäure (Racemat) (Beispiel 4.1C) und 40 mg (0.25 mmol) 4-(1*H*-Tetrazol-5-yl)anilin umgesetzt. Ausbeute: 45 mg (46% d. Th.)
LC/MS [Methode 1]: $R_t$ = 1.01 min; MS (ESIpos): m/z = 474 $(M+H)^+$
$^1$H-NMR (400 MHz, DMSO-$d_6$): $\delta$ [ppm] = 11.03 (s, 1H), 8.10 (d, 1H), 8.04 (d, 3H), 8.01 (d, 2H), 7.87 (d, 2H), 7.84 (d, 1H), 7.76 (dd, 1H), 6.70 (d, 1H), 6.57 (dd, 1H), 5.52 (d, 1H), 1.10 (d, 3H), 0.82 (d, 3H).

**Beispiel 23**

2-[4-(5-Chlor-2-cyanophenyl)-2-oxopyridin-1(2*H*)-yl]-*N*-[4-(1*H*-tetrazol-5-yl)phenyl]hexansäureamid (Racemat)

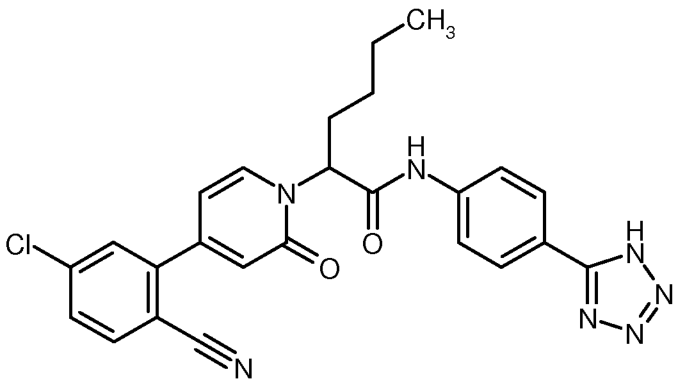

Nach der allgemeinen Methode 1 wurden 95 mg (78% Reinheit, 0.22 mmol) 2-[4-(5-Chlor-2-cyanophenyl)-2-oxopyridin-1(2*H*)-yl]hexansäure (Racemat) (Beispiel 5.1C) und 42 mg (0.26 mmol) 4-(1*H*-Tetrazol-5-yl)anilin umgesetzt. Ausbeute: 32 mg (30% d. Th.)
LC/MS [Methode 1]: $R_t$ = 1.07 min; MS (ESIpos): m/z = 488 $(M+H)^+$
$^1$H-NMR (400 MHz, DMSO-$d_6$): $\delta$ [ppm] = 16.77 (br. s, 1H), 10.88 (s, 1H), 8.05 (d, 1H), 8.03-7.97 (m, 2H), 7.87-7.82 (m, 3H), 7.77 (dd, 1H), 6.68 (d, 1H), 6.56 (dd, 1H), 5.71 (dd, 1H), 2.21-2.04 (m, 2H), 1.41-1.31 (m, 2H), 1.29-1.20 (m, 2H), 0.88 (t, 3H).

**Beispiel 24**

4-[(2-{4-[5-Chlor-2-(trifluormethyl)phenyl]-2-oxopyridin-1(2*H*)-yl}-3-cyclopropylpropanoyl)-amino]benzoesäure (Racemat)

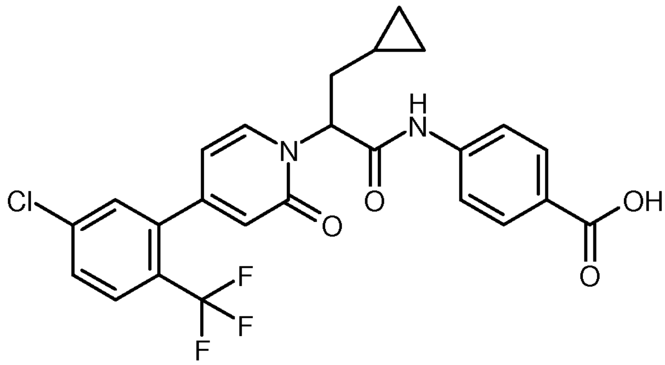

Nach der allgemeinen Methode 3 wurden 200 mg (48% Reinheit, 0.19 mmol) 4-[(2-{4-[5-Chlor-2-(trifluormethyl)phenyl]-2-oxopyridin-1(2*H*)-yl}-3-cyclopropylpropanoyl)amino]benzoesäuremethylester (Racemat) (Beispiel 6.1G) mit Lithiumhydroxid verseift. Ausbeute: 46 mg (49% d. Th.)
LC/MS [Methode 1]: $R_t$ = 1.14 min; MS (ESIpos): m/z = 505 $(M+H)^+$
$^1$H-NMR (400 MHz, DMSO-$d_6$): δ [ppm] = 12.78 (br. s, 1H), 10.84 (s, 1H), 7.90 (t, 4H), 7.78-7.71 (m, 3H), 7.64 (d, 1H), 6.39 (d, 1H), 6.33 (dd, 1H), 5.88-5.77 (m, 1H), 5.74-5.67 (m, 1H), 5.05-4.96 (m, 2H), 2.27-2.15 (m, 2H), 2.11-1.93 (m, 2H).

**Beispiel 25**

4-[(2-{4-[5-Chlor-2-(trifluormethoxy)phenyl]-2-oxopyridin-1(2*H*)-yl}-3-cyclopropylpropanoyl)-amino]benzoesäure (Racemat)

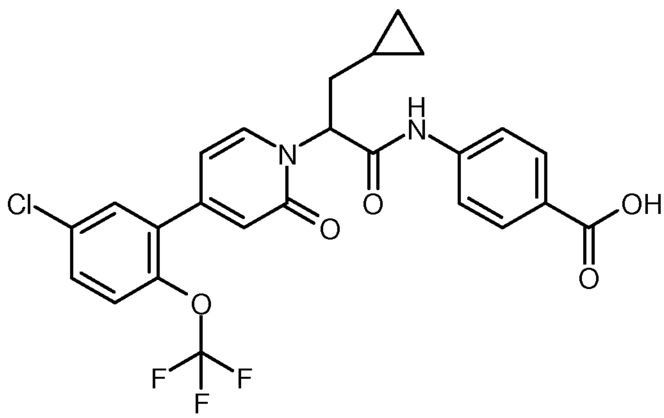

Nach der allgemeinen Methode 2 wurden 77 mg (92% Reinheit, 0.12 mmol) 4-[(2-{4-[5-Chlor-2-(trifluormethoxy)phenyl] -2-oxopyridin-1(2*H*)-yl}-3-cyclopropylpropanoyl)amino]benzoesäure-*tert*.-butylester (Racemat) (Beispiel 6.2C) mit TFA verseift. Ausbeute: 41 mg (64% d. Th.)
LC/MS [Methode 3]: $R_t$ = 2.60 min; MS (ESIpos): m/z = 521 $(M+H)^+$
$^1$H-NMR (400 MHz, DMSO-$d_6$): δ [ppm] = 12.78 (br. s, 1H), 10.84 (s, 1H), 7.94-7.88 (m, 3H), 7.76-7.71 (m, 3H), 7.67 (dd, 1H), 6.58 (dd, 1H), 6.55 (d, 1H), 6.45 (dd, 1H), 5.87-5.76 (m, 1H), 5.72-5.65 (m, 1H), 5.03-4.95 (m, 2H), 2.27-2.17 (m, 2H), 2.09-1.92 (m, 2H).

**Beispiel 26**

2-[4-(5-Chlor-2-cyanophenyl)-2-oxopyridin-1(2*H*)-yl]-3-phenyl-*N*-[4-(1*H*-tetrazol-5-yl)phenyl]-propansäureamid-Diethylaminaddukt (Racemat)

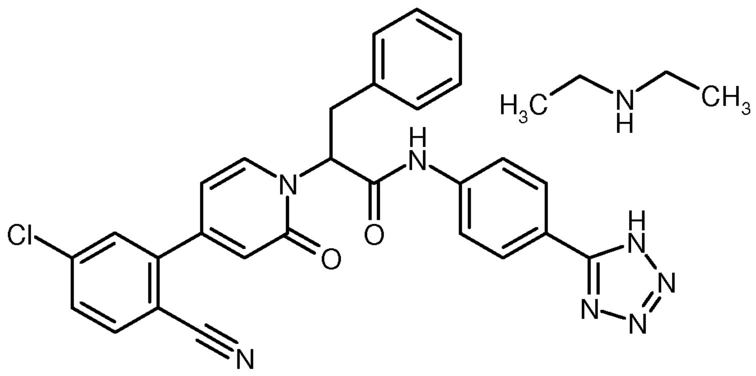

Nach der allgemeinen Methode 1 wurden 77 mg (85% Reinheit, 0.17 mmol) 2-[4-(5-Chlor-2-cyanophenyl)-2-oxopyridin-1(2*H*)-yl]-3-phenylpropansäure (Racemat) (Beispiel 7.1C) und 31 mg (0.19 mmol) 4-(1*H*-Tetrazol-5-yl)anilin umgesetzt. Ausbeute: 17 mg (94% Reinheit, 16% d. Th.)
LC/MS [Methode 1]: $R_t$ = 1.02 min; MS (ESIpos): m/z = 522 $(M+H)^+$ $^1$H-NMR (400 MHz, DMSO-$d_6$): $\delta$ [ppm] = 10.66 (s, 1H), 8.16 (d, 1H), 8.01 (d, 1H), 7.92 (d, 2H), 7.77 (d, 1H), 7.74 (dd, 1H), 7.65 (d, 2H), 7.33-7.24 (m, 4H), 7.21-7.16 (m, 1H), 6.56 (d, 1H), 6.50 (dd, 1H), 6.10 (dd, 1H), 2.93 (q, 4H), 1.15 (t, 6H).

**Beispiel 27**

4-{[2-{4-[5-Chlor-2-(trifluormethyl)phenyl]-2-oxopyridin-1(2*H*)-yl}-3-(pyridin-2-yl)propanoyl]-amino}benzoesäure (Racemat)

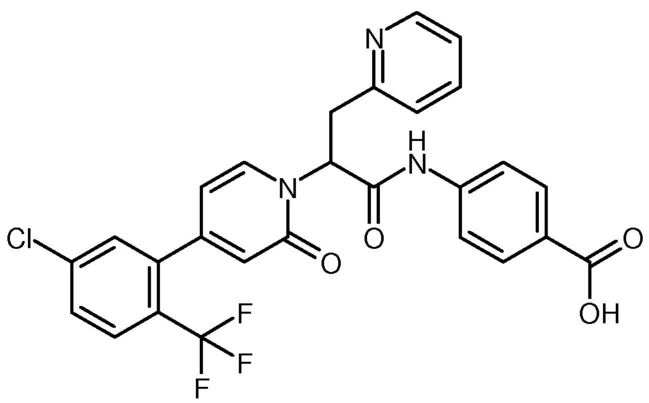

Nach der allgemeinen Methode 2 wurden 163 mg (93% Reinheit, 0.25 mmol) 4-[(2-{4-[5-Chlor-2-(trifluormethoxy)phenyl]-2-oxopyridin-1(2*H*)-yl}-3-cyclopropylpropanoyl)amino]benzoesäure-*tert*.-butylester (Racemat) (Beispiel 8.1E) mit TFA verseift. Ausbeute: 98 mg (71% d. Th.)
LC/MS [Methode 1]: $R_t$ = 1.01 min; MS (ESIpos): m/z = 542 $(M+H)^+$
$^1$H-NMR (400 MHz, DMSO-$d_6$): $\delta$ [ppm] = 12.77 (br. s, 1H), 10.92 (s, 1H), 8.48 (d, 1H), 7.95-7.89 (m, 3H), 7.86 (d, 1H), 7.78-7.69 (m, 4H), 7.54 (d, 1H), 7.35 (d, 1H), 7.25 (dd, 1H), 6.28 (d, 1H), 6.23-6.16 (m, 2H), 3.71-3.56 (m, 2H).

**Beispiel 28**

4-{[2-{4-[5-Chlor-2-(trifluormethyl)phenyl]-2-oxopyridin-1(2*H*)-yl}-3-(pyridin-3-yl)propanoyl]-amino}benzoesäure (Racemat)

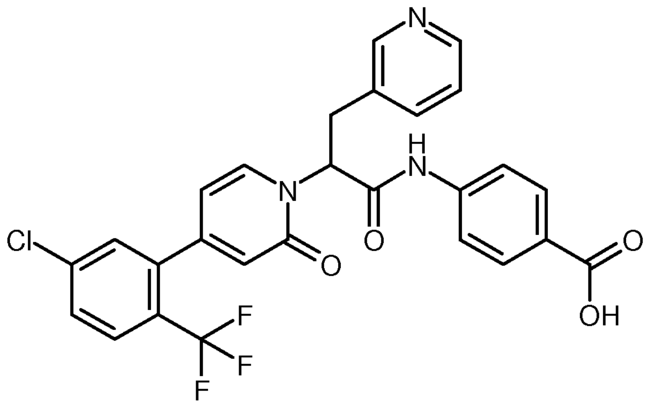

Nach der allgemeinen Methode 2 wurden 33 mg (94% Reinheit, 0.05 mmol) 4-{[2-{4-[5-Chlor-2-(trifluormethyl)phenyl]-2-oxopyridin-1(2*H*)-yl}-3-(pyridin-3-yl)propanoyl]amino}benzoesäure-*tert.*-butylester (Racemat) (Beispiel 9.1C) mit TFA versetzt. Ausbeute: 11 mg (40% d. Th.)
LC/MS [Methode 1]: $R_t$ = 0.91 min; MS (ESIpos): m/z = 542 $(M+H)^+$
$^1$H-NMR (400 MHz, DMSO-$d_6$): δ [ppm] = 12.79 (br. s, 1H), 10.87 (s, 1H), 8.48 (s, 1H), 8.41 (d, 1H), 8.05 (d, 1H), 7.93 (d, 2H), 7.86 (d, 1H), 7.76-7.71 (m, 3H), 7.61 (d, 1H), 7.54 (s, 1H), 7.28 (dd, 1H), 6.30.6.26 (m, 2H), 6.05 (dd, 1H), 3.56-3.43 (m, 2H).

**Beispiel 29**

4-({2-[4-(2-Brom-5-chlorphenyl)-2-oxopyridin-1(2*H*)-yl]-3-(pyridin-3-yl)propanoyl}amino)-benzoesäure (Racemat)

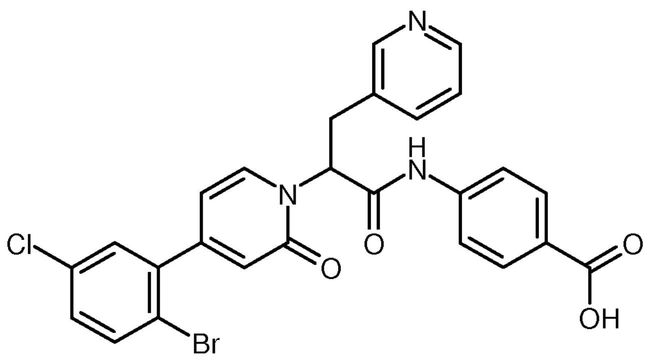

Nach der allgemeinen Methode 3 wurden 65 mg (85% Reinheit, 0.10 mmol) 4-({2-[4-(2-Brom-5-chlorphenyl)-2-oxopyridin-1(2*H*)-yl]-3-(pyridin-3-yl)propanoyl}amino)benzoesäuremethylester (Racemat) (Beispiel 9.2E) mit Lithiumhydroxid verseift. Ausbeute: 14 mg (26% d. Th.)
LC/MS [Methode 1]: $R_t$ = 0.90 min; MS (ESIpos): m/z = 552 $(M+H)^+$ $^1$H-NMR (400 MHz, DMSO-$d_6$): δ [ppm] = 12.79 (br. s, 1H), 10.86 (s, 1H), 8.48 (s, 1H), 8.40 (d, 1H), 8.06 (d, 1H), 7.93 (d, 2H), 7.78-7.70 (m, 3H), 7.65 (dd, 1H), 7.45 (d, 1H), 7.44 (dd, 1H), 7.31 (dd, 1H), 6.35-6.31 (m, 2H), 6.05 (dd, 1H), 3.56-3.43 (m, 2H).

**Beispiel 30**

4-{[2-{4-[5-Chlor-2-(trifluormethyl)phenyl]-2-oxopyridin-1(2*H*)-yl}-3-(pyridin-4-yl)propanoyl]-amino}benzoesäure (Racemat)

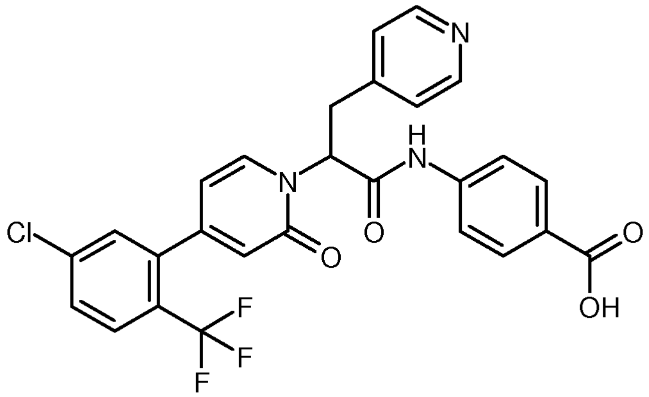

Nach der allgemeinen Methode 2 wurden 383 mg (0.62 mmol) 4-{[2-{4-[5-Chlor-2-(trifluormethyl)phenyl]-2-oxopyridin-1(2*H*)-yl}-3-(pyridin-4-yl)propanoyl]amino}benzoesäure-*tert*.-butylester (Racemat) (Beispiel 10.1C) mit TFA verseift. Ausbeute: 180 mg (53% d. Th.)
LC/MS [Methode 1]: $R_t$ = 0.86 min; MS (ESIpos): m/z = 542 $(M+H)^+$
$^1$H-NMR (400 MHz, DMSO-$d_6$): $\delta$ [ppm] = 12.85 (br. s, 1H), 10.88 (s, 1H), 8.64 (d, 2H), 8.01 (d, 1H), 7.94 (d, 2H), 7.87 (d, 1H), 7.77-7.70 (m, 3H), 7.58-7.52 (m, 3H), 6.31-6.26 (m, 2H), 6.13 (dd, 1H), 3.72-3.59 (m, 2H).

**Beispiel 31**

4-{[2-{4-[5-Chlor-2-(trifluormethyl)phenyl]-2-oxopyridin-1(2*H*)-yl}-3-(pyridin-4-yl)propanoyl]-amino}benzoesäure (Enantiomer 1)

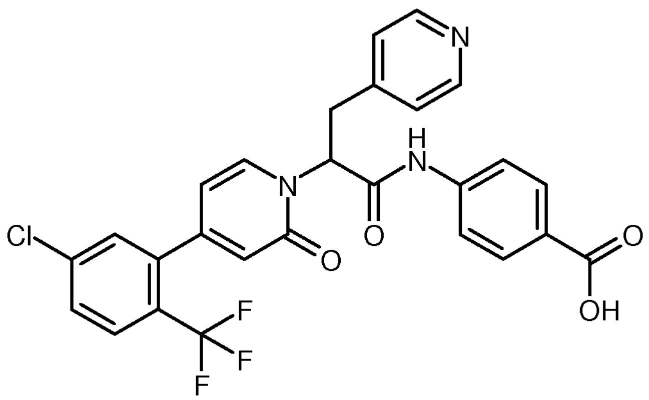

Enantiomerentrennung von 155 mg des Racemates aus Beispiel 30 ergab 56 mg (89% Reinheit) der Titelverbindung Beispiel 31 (Enantiomer 1): Chirale HPLC: $R_t$ = 6.8 min; 100% ee.

Trennmethode: Säule: Daicel Chiralpak AZ-H 5 µm 250x20 mm; Eluent: 50% iso-Hexan, 46% Ethanol, 4% 2%ige Trifluoressigsäure in Ethanol; Ofen: 25°C; Fluss: 20 ml/min; UV-Detektion: 230 nm.

Analytik: Säule: Daicel Chiralpak AZ-H 5 µm 250x4.6 mm; Eluent: 50% iso-Hexan, 50% Ethanol + 0.2% TFA in 1% Wasser; Ofen: 30°C; Fluss: 1 ml/min; UV-Detektion: 220 nm.

**Beispiel 32**

4-{[2-{4-[5-Chlor-2-(trifluormethyl)phenyl]-2-oxopyridin-1(2*H*)-yl}-3-(pyridin-4-yl)propanoyl]-amino}benzoesäure (Enantiomer 2)

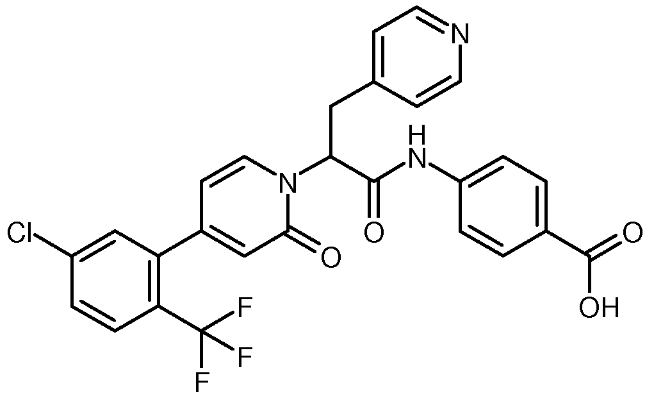

Enantiomerentrennung von 155 mg des Racemates aus Beispiel 30 ergab 21 mg der Titelverbindung Beispiel 32 (Enantiomer 2): Chirale HPLC: $R_t$ = 10.6 min; 95% ee.

Trennmethode: Säule: Daicel Chiralpak AZ-H 5 μm 250x20 mm; Eluent: 50% iso-Hexan, 46% Ethanol, 4% 2%ige Trifluoressigsäure in Ethanol; Ofen: 25°C; Fluss: 20 ml/min; UV-Detektion: 230 nm.

Analytik: Säule: Daicel Chiralpak AZ-H 5 μm 250x4.6 mm; Eluent: 50% iso-Hexan, 50% Ethanol + 0.2% TFA in 1% Wasser; Ofen: 30°C; Fluss: 1 ml/min; UV-Detektion: 220 nm.

**Beispiel 33**

4-({2-[4-(2-Brom-5-chlorphenyl)-2-oxopyridin-1(2*H*)-yl]-3-(pyridin-4-yl)propanoyl}amino)-benzoesäure (Racemat)

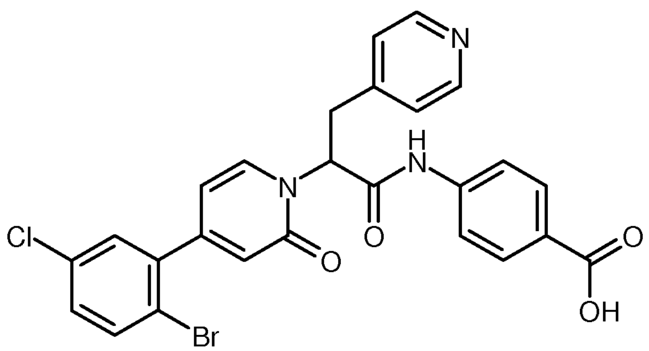

Nach der allgemeinen Methode 2 wurden 734 mg (92% Reinheit, 1.11 mmol) 4-({2-[4-(2-Brom-5-chlorphenyl)-2-oxopyridin-1(2*H*)-yl]-3-(pyridin-4-yl)propanoyl}amino)benzoesäure-*tert.*-butylester (Racemat) (Beispiel 10.2C) mit TFA verseift. Ausbeute: 126 mg (21% d. Th.)

LC/MS [Methode 1]: $R_t$ = 0.83 min; MS (ESIpos): m/z = 552 $(M+H)^+$

$^1$H-NMR (400 MHz, DMSO-$d_6$): δ [ppm] = 12.85 (br. s, 1H), 10.88 (s, 1H), 8.48 (d, 2H), 8.02 (d, 1H), 7.93 (d, 2H), 7.78-7.71 (m 3H), 7.48 (d, 1H), 7.44 (dd, 1H), 7.31 (d, 1H), 6.34 (d, 1H), 6.31 (dd, 1H), 6.09 (dd, 1H), 3.57-3.49 (m, 2H).

**Beispiel 34**

4-({2-[4-(5-Chlor-2-cyanophenyl)-5-fluor-2-oxopyridin-1(2*H*)-yl]propanoyl}amino)benzoesäure (Racemat)

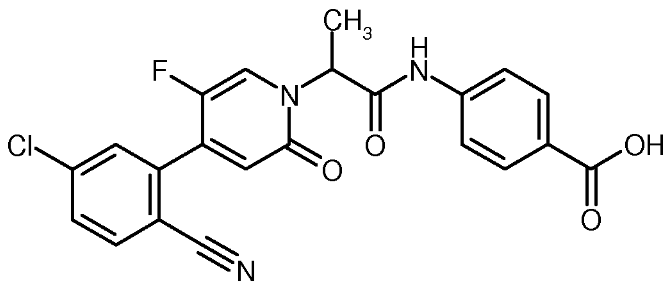

Nach der allgemeinen Methode 2 wurden 53 mg (0.11 mmol) 4-({2-[4-(5-Chlor-2-cyanophenyl)-5-fluor-2-oxopyridin-1(2*H*)-yl]propanoyl}amino)benzoesäure-*tert*.-butylester (Racemat) (Beispiel 11.1E) mit TFA verseift. Ausbeute: 36 mg (77% d. Th.).
LC/MS [Methode 1]: $R_t$ = 0.90 min; MS (ESIpos): m/z = 440 $(M+H)^+$
$^1$H-NMR (400 MHz, DMSO-$d_6$): $\delta$ [ppm] = 12.78 (br. s, 1H), 10.77 (s, 1H), 8.24 (d, 1H), 8.09 (d, 1H), 7.91 (d, 2H), 7.88 (d, 1H), 7.82 (dd, 1H), 7.72 (d, 2H), 6.67 (d, 1H), 5.55 (q, 1H), 1.72 (d, 3H).

**Beispiel 35**

4-({2-[5-Chlor-4-(5-chlor-2-cyanophenyl)-2-oxopyridin-1(2*H*)-yl]propanoyl}amino)benzoesäure (Racemat)

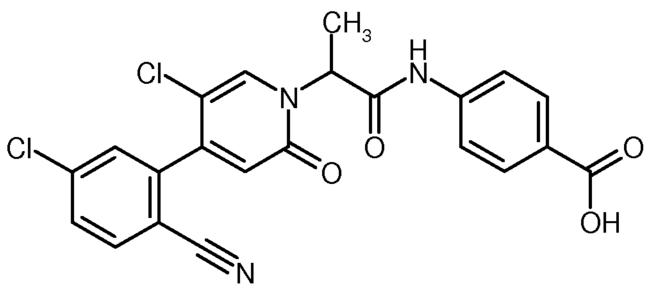

Nach der allgemeinen Methode 2 wurden 281 mg (58% Reinheit, 0.32 mmol) 4-({2-[5-Chlor-4-(5-chlor-2-cyanophenyl)-2-oxopyridin-1(2*H*)-yl]propanoyl}amino)benzoesäure-*tert*.-butylester (Racemat) (Beispiel 12.1E) mit TFA verseift. Ausbeute: 97 mg (67% d. Th.)
LC/MS [Methode 1]: $R_t$ = 0.96 min; MS (ESIpos): m/z = 456 $(M+H)^+$
$^1$H-NMR (400 MHz, DMSO-$d_6$): $\delta$ [ppm] = 12.78 (s, 1H), 10.77 (s, 1H), 8.19 (s, 1H), 8.08 (d, 1H), 7.92 (d, 2H), 7.80 (d, 2H), 7.73 (d, 2H), 6.68 (s, 1H), 5.56 (q, 1H), 1.74 (d, 3H).

**Beispiel 36**

4-({2-[5-Chlor-4-(5-chlor-2-cyanophenyl)-2-oxopyridin-1(2*H*)-yl]propanoyl}amino)benzoesäure (Enantiomer 1)

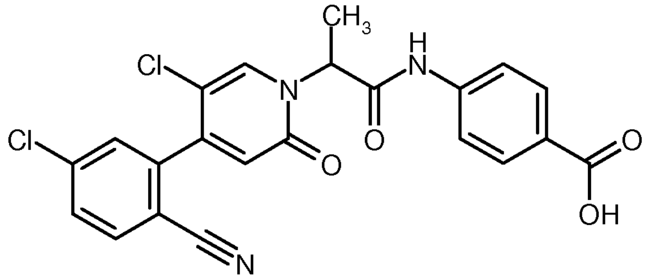

Enantiomerentrennung von 59 mg des Racemates aus Beispiel 35 ergab 24 mg der Titelverbindung Beispiel 36 (Enantiomer 1): Chirale HPLC: $R_t$ = 8.6 min; 100% ee.

Trennmethode: Säule: Daicel Chiralpak AZ-H 5 μm 250x30 mm; Eluent: 50% iso-Hexan, 50% Ethanol; Ofen: 25°C; Fluss: 40 ml/min; UV-Detektion: 220 nm.

Analytik: Säule: Daicel Chiralpak AZ-H 5 μm 250x4.6 mm; Eluent: 50% iso-Hexan, 50% Ethanol + 0.2% TFA in 1% Wasser; Ofen: 30°C; Fluss: 1 ml/min; UV-Detektion: 220 nm.

**Beispiel 37**

4-({2-[5-Chlor-4-(5-chlor-2-cyanophenyl)-2-oxopyridin-1(2*H*)-yl]propanoyl}amino)benzoesäure (Enantiomer 2)

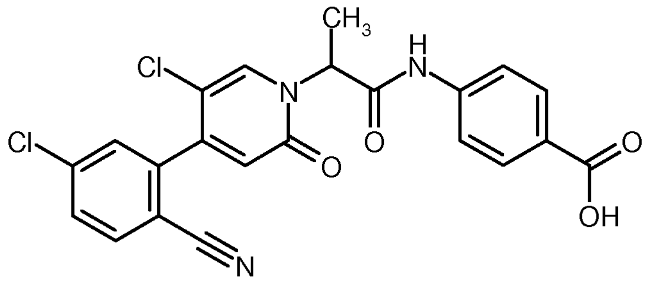

Enantiomerentrennung von 59 mg des Racemates aus Beispiel 35 nach ergab 15 mg der Titelverbindung Beispiel 37 (Enantiomer 2): Chirale HPLC: $R_t$ = 15.5 min; 100% ee.

Trennmethode: Säule: Daicel Chiralpak AZ-H 5 μm 250x30 mm; Eluent: 50% iso-Hexan, 50% Ethanol; Ofen: 25°C; Fluss: 40 ml/min; UV-Detektion: 220 nm.

Analytik: Säule: Daicel Chiralpak AZ-H 5 μm 250x4.6 mm; Eluent: 50% iso-Hexan, 50% Ethanol + 0.2% TFA in 1% Wasser; Ofen: 30°C; Fluss: 1 ml/min; UV-Detektion: 220 nm.

**Beispiel 38**

4-[(2-{5-Chlor-4-[5-chlor-2-(difluormethyl)phenyl]-2-oxopyridin-1(2*H*)-yl}propanoyl)amino]-benzoesäure (Racemat)

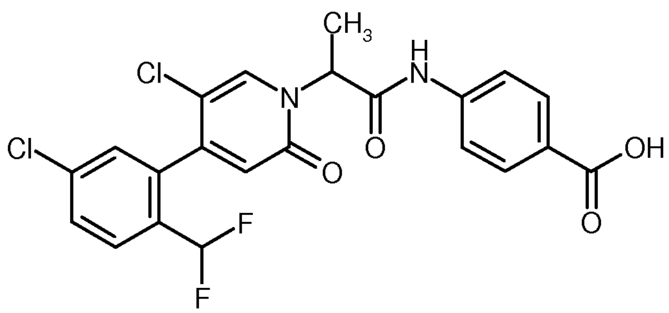

Nach der allgemeinen Methode 2 wurden 79 mg (0.15 mmol) 4-[(2-{5-Chlor-4-[5-chlor-2-(difluormethyl)phenyl]-2-oxopyridin-1(2*H*)-yl}propanoyl)amino]benzoesäure-*tert*.-butylester (Racemat) (Beispiel 14.1E) mit TFA verseift. Ausbeute: 54 mg (76% d. Th.)
LC/MS [Methode 1]: $R_t$ = 1.03 min; MS (ESIpos): m/z = 481 $(M+H)^+$
$^1$H-NMR (400 MHz, DMSO-$d_6$): δ [ppm] = 12.79 (br. s, 1H), 10.75/10.72 (2s, 1H), 8.14/8.12 (2s, 1H), 7.92 (d, 2H), 7.80-7.70 (m, 4H), 7.54 (br. d, 1H), 7.02-6.75 (br. t, 1H), 6.50/6.49 (2s, 1H), 5.57 (q, 1H), 1.73/1.71 (2d, 3H).

## Beispiel 39

4-[(2-{5-Chlor-4-[5-chlor-2-(trifluormethyl)phenyl]-2-oxopyridin-1(2*H*)-yl}propanoyl)amino]-benzoesäure (Racemat)

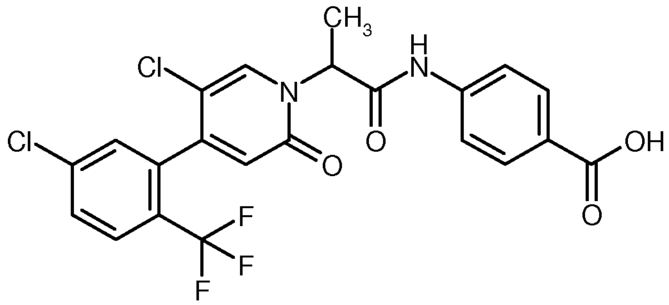

Nach der allgemeinen Methode 2 wurden 75 mg (0.14 mmol) 4-[(2-{5-Chlor-4-[5-chlor-2-(trifluormethyl)phenyl]-2-oxopyridin-1(2*H*)-yl}propanoyl)amino]benzoesäure-*tert*.-butylester (Racemat) (Beispiel 15.1E) mit TFA verseift. Ausbeute: 56 mg (83% d. Th.)
LC/MS [Methode 1]: $R_t$ = 1.07 min; MS (ESIpos): m/z = 499 $(M+H)^+$
$^1$H-NMR (400 MHz, DMSO-$d_6$): δ [ppm] = 12.79 (s, 1H), 10.78/10.75 (2s, 1H), 8.12/8.11 (2s, 1H), 7.95-7.89 (m, 3H), 7.80 (s, 1H), 7.72 (d, 2H), 7.69 (br. t, 1H), 6.52/6.50 (2s, 1H), 5.57 (q, 1H), 1.73/1.71 (2d, 3H).

## Beispiel 40

4-({2-[5-Chlor-4-(5-chlor-2-cyanophenyl)-2-oxopyridin-1(2*H*)-yl]propanoyl}amino)-2-fluorbenzoesäure (Racemat)

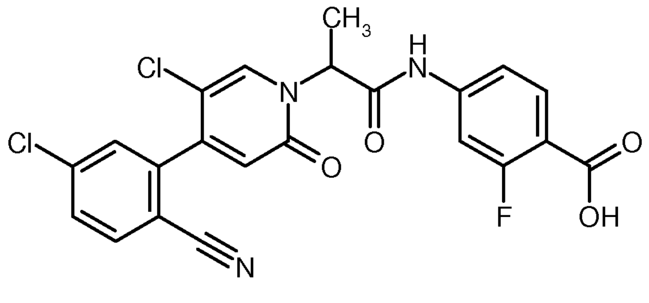

Eine Lösung von 100 mg (0.30 mmol) 2-[5-Chlor-4-(5-chlor-2-cyanophenyl)-2-oxopyridin-1(2*H*)-yl]propansäure (Racemat) (Beispiel 12.1D) in 2 ml Dichlorethan wurde unter Argon bei RT mit 37 mg (0.30 mmol, 1.0 eq.) *N,N'*-Diisopropylcarbodiimid versetzt, 10 min gerührt, mit 46 mg (0.30 mmol) 4-Amino-2-fluorbenzoesäure versetzt und über Nacht unter Rückfluß gerührt. Anschließend wurde das Reaktionsgemisch im Vakuum eingeengt und das Rohprodukt mittels präparativer HPLC (Reprosil C18, Wasser-Methanol-Gradient) aufgereinigt. Ausbeute: 41 mg (29% d. Th.)
LC/MS [Methode 1]: $R_t$ = 0.98 min; MS (ESIpos): m/z = 474 $(M+H)^+$
$^1$H-NMR (400 MHz, DMSO-$d_6$): δ [ppm] = 13.04 (br. s, 1H), 10.93 (s, 1H), 8.19 (s, 1H), 8.07 (d, 1H), 7.87 (t, 1H), 7.83-7.76 (m, 1H), 7.80 (dd, 1H), 7.65 (dd, 1H), 7.41 (dd, 1H), 6.69 (s, 1H), 5.52 (q, 1H), 1.75 (d, 3H).

**Beispiel 41**

2-[5-Chlor-4-(5-chlor-2-cyanophenyl)-2-oxopyridin-1(2*H*)-yl]-*N*-{4-[3-(trifluormethyl)-1*H*-1,2,4-triazol-5-yl]phenyl}propansäureamid (Racemat)

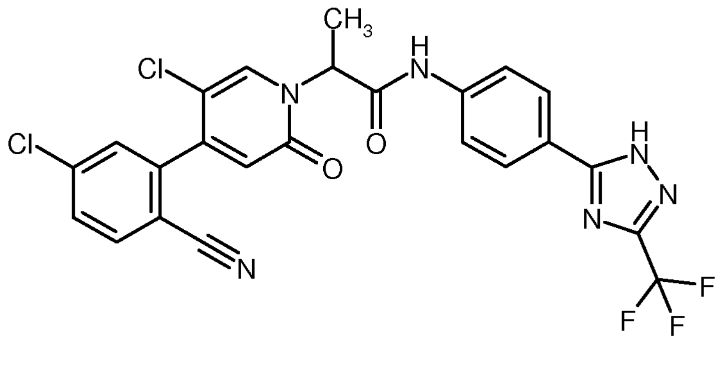

Nach der allgemeinen Methode 1 wurden 126 mg (66% Reinheit, 0.25 mmol) 2-[5-Chlor-4-(5-chlor-2-cyanophenyl)-2-oxopyridin-1(2H)-yl]propansäure (Racemat) (Beispiel 12.1D) und 64 mg (0.27 mmol) 4-[3-(Trifluormethyl)-1*H*-1,2,4-triazol-5-yl]anilin (Beispiel 1.1C) umgesetzt. Ausbeute: 76 mg (57% d. Th.)
LC/MS [Methode 1]: $R_t$ = 1.10 min; MS (ESIpos): m/z = 547 $(M+H)^+$
$^1$H-NMR (400 MHz, DMSO-$d_6$): $\delta$ [ppm] = 15.20 (s, 1H), 10.77 (s, 1H), 8.20 (s, 1H), 8.08 (d, 1H), 8.01 (d, 2H), 7.84-7.77 (m, 4H), 6.69 (s, 1H), 5.58 (q, 1H), 1.76 (d, 3H).

**Beispiel 42**

2-[5-Chlor-4-(5-chlor-2-cyanophenyl)-2-oxopyridin-1(2*H*)-yl]-*N*-[4-(5-oxo-2,5-dihydro-1*H*-pyrazol-3-yl)phenyl]propansäureamid (Racemat)

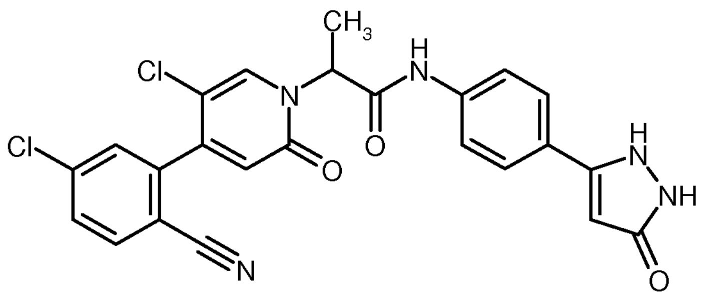

Nach der allgemeinen Methode 2 wurden 17.8 mg (78% Reinheit, 0.02 mmol) 5-[4-({2-[5-Chlor-4-(5-chlor-2-cyanophenyl)-2-oxopyridin-1(2*H*)-yl]propanoyl}amino)phenyl]-3-oxo-2,3-dihydro-1*H*-pyrazol-1-carbonsäure-*tert.*-butylester (Racemat) (Beispiel 12.2A) mit TFA umgesetzt. Ausbeute: 6 mg (55% d. Th.)
LC/MS [Methode 1]: $R_t$ = 0.92 min; MS (ESIpos): m/z = 494 $(M+H)^+$
$^1$H-NMR (400 MHz, DMSO-$d_6$): $\delta$ [ppm] = 11.96 (br. s, 1H), 10.54 (s, 1H), 9.56 (br. s, 1H), 8.18 (s, 1H), 8.07 (d, 1H), 7.80 (m, 2H), 7.63 (m, 4H), 6.67 (s, 1H), 5.85 (br. s, 1H), 5.59 (q, 1H), 1.73 (d, 3H).

**Beispiel 43**

2-[5-Chlor-4-(5-chlor-2-cyanophenyl)-2-oxopyridin-1(2*H*)-yl]-*N*-[4-(1*H*-imidazol-4-yl)phenyl]-propansäureamid (Racemat)

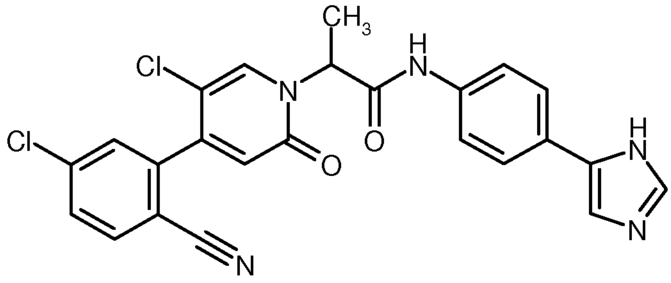

Nach der allgemeinen Methode 1 wurden 102 mg (82% Reinheit, 0.25 mmol) 2-[5-Chlor-4-(5-chlor-2-cyanophenyl)-2-oxopyridin-1(2*H*)-yl]propansäure (Racemat) (Beispiel 12.1D) und 43 mg (0.27 mmol) 4-(1*H*-Imidazol-4-yl)anilin umgesetzt. Ausbeute: 5 mg (94% Reinheit, 4% d. Th.)
LC/MS [Methode 1]: $R_t$ = 0.75 min; MS (ESIpos): m/z = 478 $(M+H)^+$
$^1$H-NMR (400 MHz, DMSO-$d_6$): δ [ppm] = 12.22 (br. s, 1H), 10.46 (s, 1H), 8.20 (s, 1H), 8.08 (d, 1H), 7.82 (m, 2H), 7.73 (m, 3H), 7.61 (d, 2H), 7.53 (br. s, 1H), 6.69 (s, 1H), 5.62 (q, 1H), 1.75 (d, 3H).

**Beispiel 44**

2-[5-Chlor-4-(5-chlor-2-cyanophenyl)-2-oxopyridin-1(2*H*)-yl]-*N*-[2-(trifluormethyl)-1*H*-benzimidazol-6-yl]propansäureamid (Racemat)

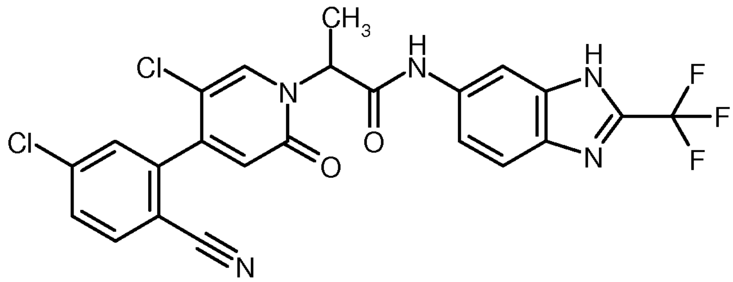

Nach der allgemeinen Methode 1 wurden 68 mg (94% Reinheit, 0.20 mmol) 2-[5-Chlor-4-(5-chlor-2-cyanophenyl)-2-oxopyridin-1(2*H*)-yl]propansäure (Racemat) (Beispiel 12.1D) und 44 mg (0.22 mmol) 2-(Trifluormethyl)-1*H*-benzimidazol-6-amin umgesetzt. Ausbeute: 63 mg (60% d. Th.)
LC/MS [Methode 1]: $R_t$ = 1.01 min; MS (ESIpos): m/z = 520 $(M+H)^+$
$^1$H-NMR (400 MHz, DMSO-$d_6$): δ [ppm] = 13.85 (s, 1H), 10.64 (s, 1H), 8.22 (s, 1H), 8.18 (s, 1H), 8.08 (d, 1H), 7.80 (m, 2H), 7.74 (br. s, 1H), 7.41 (br. s, 1H), 6.69 (s, 1H), 1.06 (q, 1H), 1.76 (d, 3H).

**Beispiel 45**

4-({2-[5-Chlor-4-(5-chlor-2-cyanophenyl)-2-oxopyridin-1(2*H*)-yl]-3-cyclopropylpropanoyl}-amino)benzoesäure (Racemat)

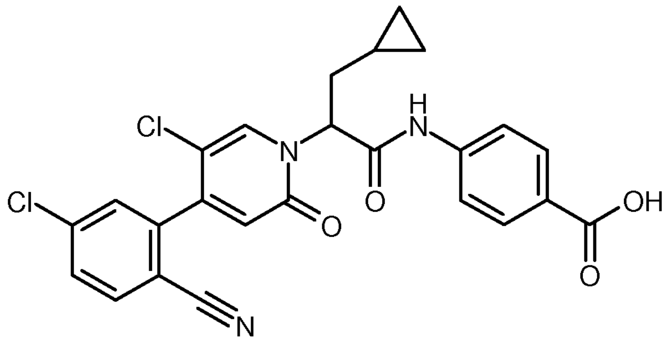

Nach der allgemeinen Methode 2 wurden 192 mg (0.34 mmol) 4-({2-[5-Chlor-4-(5-chlor-2-cyanophenyl)-2-oxopyridin-1(2*H*)-yl]-3-cyclopropylpropanoyl}amino)benzoesäure-tert. -butylester (Racemat) (Beispiel 13.1D) mit TFA verseift. Ausbeute: 141 mg (82% d. Th.)
LC/MS [Methode 1]: $R_t$ = 1.07 min; MS (ESIpos): m/z = 496 $(M+H)^+$
$^1$H-NMR (400 MHz, DMSO-$d_6$): δ [ppm] = 12.80 (s, 1H), 10.83 (s, 1H), 8.25 (s, 1H), 8.07 (d, 1H), 7.92 (d, 2H), 7.84-7.78 (m, 2H), 7.73 (d, 2H), 6.69 (s, 1H), 5.76 (m, 1H), 2.25 (m, 1H), 1.91 (m, 1H), 0.70-0.60 (m, 1H), 0.48-0.32 (m, 2H), 0.22-0.09 (m, 2H).

**Beispiel 46**

4-({2-[5-Chlor-4-(5-chlor-2-cyanophenyl)-2-oxopyridin-1(2*H*)-yl]-3-cyclopropylpropanoyl}-amino)benzoesäure (Enantiomer 1)

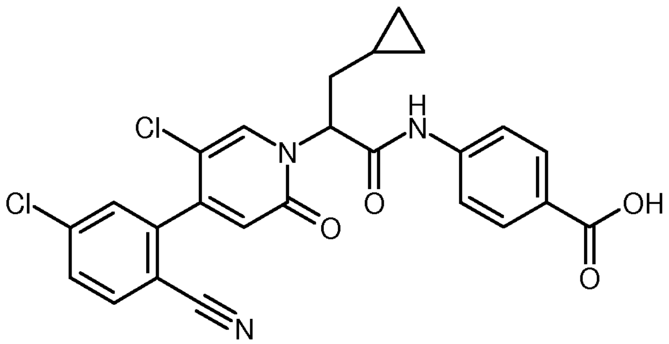

Enantiomerentrennung von 138 mg des Racemates aus Beispiel 45 ergab 42 mg der Titelverbindung Beispiel 46 (Enantiomer 1): Chirale HPLC: $R_t$ = 5.7 min; 100% ee.

Trennmethode: Säule: Daicel Chiralpak AZ-H 5 μm 250x30 mm; Eluent: 50% iso-Hexan, 50% 2-Propanol; Ofen: 20°C; Fluss: 50 ml/min; UV-Detektion: 270 nm.

Analytik: Säule: Daicel Chiralpak AZ-H 5 μm 250x4.6 mm; Eluent: 50% iso-Hexan, 50% 2-Propanol + 0.2% TFA in 1% Wasser; Ofen: 20°C; Fluss: 1 ml/min; UV-Detektion: 220 nm.

**Beispiel 47**

4-({2-[5-Chlor-4-(5-chlor-2-cyanophenyl)-2-oxopyridin-1(2*H*)-yl]-3-cyclopropylpropanoyl}-amino)benzoesäure (Enantiomer 2)

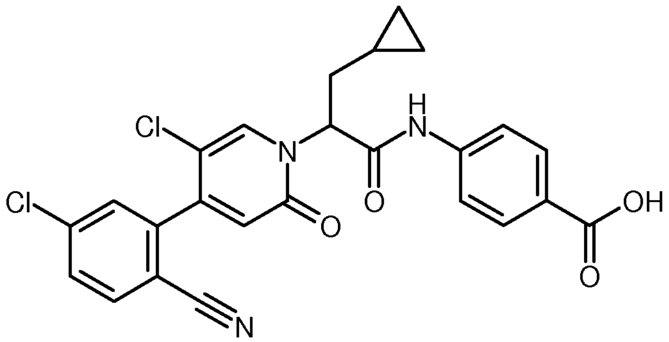

Enantiomerentrennung von 138 mg des Racemates aus Beispiel 45 ergab 41 mg der Titelverbindung Beispiel 47 (Enantiomer 2): Chirale HPLC: $R_t$ = 11.9 min; 97% ee.

Trennmethode: Säule: Daicel Chiralpak AZ-H 5 μm 250x30 mm; Eluent: 50% iso-Hexan, 50% 2-Propanol; Ofen: 20°C; Fluss: 50 ml/min; UV-Detektion: 270 nm.

Analytik: Säule: Daicel Chiralpak AZ-H 5 μm 250x4.6 mm; Eluent: 50% iso-Hexan, 50% 2-Propanol + 0.2% TFA in 1% Wasser; Ofen: 20°C; Fluss: 1 ml/min; UV-Detektion: 220 nm.

**Beispiel 48**

4-({2-[4-(5-Chlor-2-cyanophenyl)-5-cyano-2-oxopyridin-1(2*H*)-yl]propanoyl}amino)benzoesäure (Racemat)

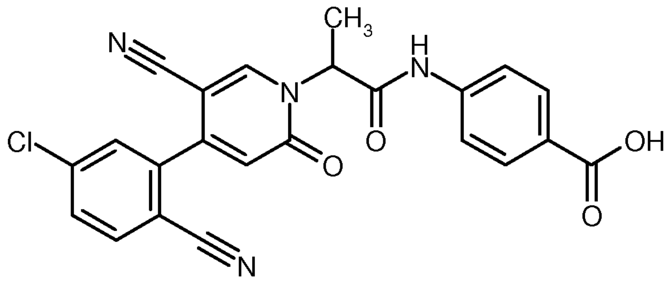

Nach der allgemeinen Methode 2 wurden 93 mg (0.19 mmol) 4-({2-[4-(5-Chlor-2-cyanophenyl)-5-cyano-2-oxopyridin-1(2*H*)-yl]propanoyl}amino)benzoesäure-tert. -butylester (Racemat) (Beispiel 16.1E) mit TFA verseift. Ausbeute: 91 mg (quant.)

LC/MS [Methode 1]: $R_t$ = 0.90 min; MS (ESIpos): m/z = 447 $(M+H)^+$

$^1$H-NMR (400 MHz, DMSO-$d_6$): δ [ppm] = 12.80 (br. s, 1H), 10.83 (s, 1H), 8.87 (s, 1H), 8.13 (d, 1H), 7.95-7.89 (m, 3H), 7.86 (dd, 1H), 7.72 (d, 2H), 6.78 (s, 1H), 5.57 (q, 1H), 1.78 (d, 3H).

**Beispiel 49**

4-[(2-{4-[5-Chlor-2-(difluormethyl)phenyl]-5-cyano-2-oxopyridin-1(2*H*)-yl}propanoyl)amino]-benzoesäure (Racemat)

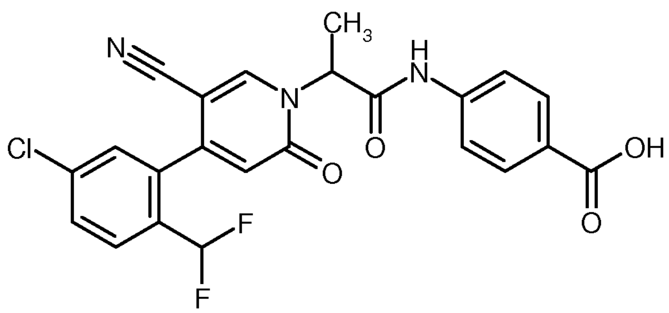

Nach der allgemeinen Methode 2 wurden 48 mg (0.09 mmol) 4-[(2-{4-[5-Chlor-2-(difluormethyl)phenyl]-5-cyano-2-oxopyridin-1(2*H*)-yl}propanoyl)amino]benzoesäure-*tert*.-butylester (Racemat) (Beispiel 17.1D) mit TFA verseift. Ausbeute: 30 mg (70% d. Th.)
LC/MS [Methode 1]: $R_t$ = 0.96 min; MS (ESIpos): m/z = 472 $(M+H)^+$
$^1$H-NMR (400 MHz, DMSO-$d_6$): δ [ppm] = 12.80 (s, 1H), 10.80 (s, 1H), 8.79 (s, 1H), 7.92 (d, 2H), 7.84-7.58 (m, 5H), 7.12-6.85 (br. t, 1H), 6.54 (s, 1H), 5.58 (q, 1H), 1.75 (d, 3H).

**Beispiel 50**

4-[(2-{4-[5-Chlor-2-(trifluormethyl)phenyl]-5-cyano-2-oxopyridin-1(2*H*)-yl}propanoyl)amino]-benzoesäure (Racemat)

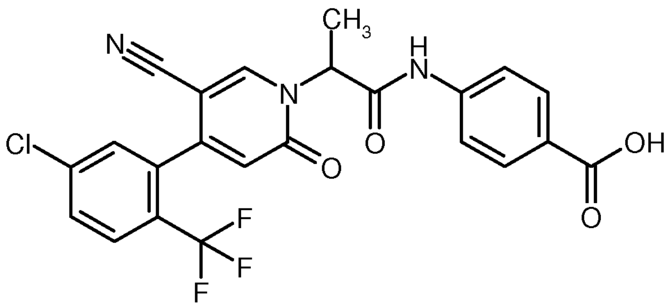

Nach der allgemeinen Methode 2 wurden 124 mg (0.22 mmol) 4-[(2-{4-[5-Chlor-2-(trifluormethyl)phenyl] -5-cyano-2-oxopyridin-1(2*H*)-yl}propanoyl)amino]benzoesäure-*tert*.-butylester (Racemat) (Beispiel 18.1E) mit TFA verseift. Ausbeute: 85 mg (79% d. Th.)
LC/MS [Methode 1]: $R_t$ = 0.99 min; MS (ESIpos): m/z = 490 $(M+H)^+$
$^1$H-NMR (400 MHz, DMSO-$d_6$): δ [ppm] = 12.79 (s, 1H), 10.82 (d, 1H), 8.79 (s, 1H), 7.96 (dd, 1H), 7.92 (d, 2H), 7.87-7.69 (m, 4H), 6.85 (s, 1H), 5.57 (q, 1H), 1.76 (d, 3H).

**Beispiel 51**

4-[(2-{4-[5-Chlor-2-(trifluormethoxy)phenyl]-5-cyano-2-oxopyridin-1(2*H*)-yl}propanoyl)amino]-benzoesäure (Racemat)

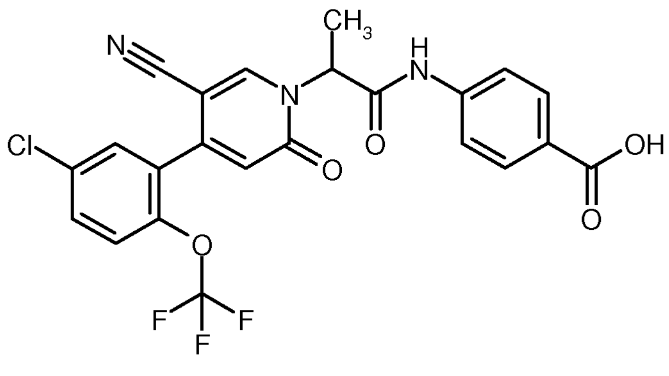

Nach der allgemeinen Methode 2 wurden 107 mg (94% Reinheit, 0.18 mmol) 4-[(2-{4-[5-Chlor-2-(trifluormethoxy)phenyl]-5-cyano-2-oxopyridin-1(2*H*)-yl}propanoyl)amino]benzoesäure-*tert*.-butylester (Racemat) (Beispiel 18.2A) mit TFA verseift. Ausbeute: 59 mg (65% d. Th.)
LC/MS [Methode 1]: $R_t$ = 1.00 min; MS (ESIpos): m/z = 506 $(M+H)^+$
$^1$H-NMR (400 MHz, DMSO-$d_6$): δ [ppm] = 12.79 (s, 1H), 10.80 (s, 1H), 8.79 (s, 1H), 7.92 (d, 2H), 7.78-7.61 (m, 5H), 6.65 (s, 1H), 5.55 (q, 1H), 1.76 (d, 3H).

**Beispiel 52**

4-({2-[4-(5-Chlor-2-cyclopropylphenyl)-5-cyano-2-oxopyridin-1(2*H*)-yl]propanoyl}amino)-benzoesäure (Racemat)

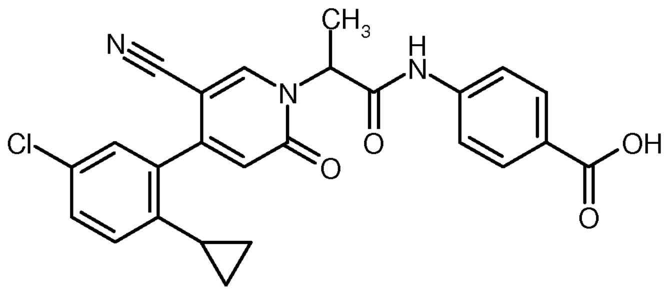

Nach der allgemeinen Methode 2 wurden 66 mg (0.13 mmol) 4-({2-[4-(5-Chlor-2-cyclopropylphenyl)-5-cyano-2-oxopyridin-1(2*H*)-yl]propanoyl}amino)benzoesäure-*tert*.-butylester (Racemat) (Beispiel 18.3A) mit TFA verseift. Ausbeute: 39 mg (68% d. Th.)
LC/MS [Methode 1]: $R_t$ = 1.02 min; MS (ESIpos): m/z = 462 $(M+H)^+$
$^1$H-NMR (400 MHz, DMSO-$d_6$): δ [ppm] = 12.79 (s, 1H), 10.79 (s, 1H), 8.76 (s, 1H), 7.92 (d, 2H), 7.71 (d, 2H), 7.45 (dd, 1H), 7.34 (s, 1H), 7.06 (d, 1H), 6.54 (s, 1H), 5.57 (q, 1H), 1.76 (d, 3H), 1.70 (m, 1H), 0.93 (d, 2H), 0.74 (d, 2H).

**Beispiel 53**

4-({2-[4-(5-Chlor-2-cyanophenyl)-5-(difluormethyl)-2-oxopyridin-1(2*H*)-yl]propanoyl}amino)-benzoesäure (Racemat)

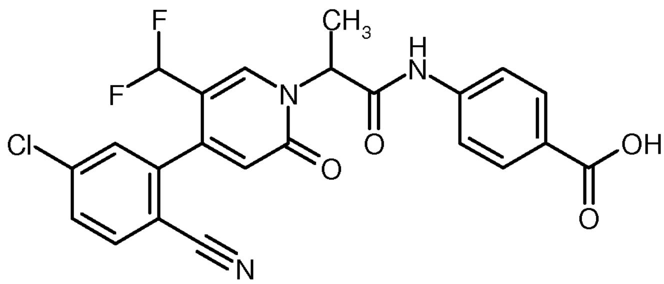

Nach der allgemeinen Methode 2 wurden 64 mg (0.12 mmol) 4-({2-[4-(5-Chlor-2-cyanophenyl)-5-(difluormethyl)-2-oxopyridin-1(2*H*)-yl]propanoyl}amino)benzoesäure-*tert*.-butylester (Racemat) (Beispiel 19.1F) mit TFA verseift. Ausbeute: 46 mg (80% d. Th.)
LC/MS [Methode 1]: $R_t$ = 0.95 min; MS (ESIpos): m/z = 472 $(M+H)^+$
$^1$H-NMR (400 MHz, DMSO-$d_6$): δ [ppm] = 12.79 (s, 1H), 10.83 (s, 1H), 8.30 (s, 1H), 8.02 (d, 1H), 7.91 (d, 2H), 7.79-7.69 (m, 4H), 6.85 (br. t, 1H), 6.57 (s, 1H), 5.58 (q, 1H), 1.74 (d, 3H).

**Beispiel 54**

4-({2-[4-(5-Chlor-2-cyanophenyl)-2-oxo-5-(trifluormethyl)pyridin-1(2*H*)-yl]propanoyl}amino)-benzoesäure (Racemat)

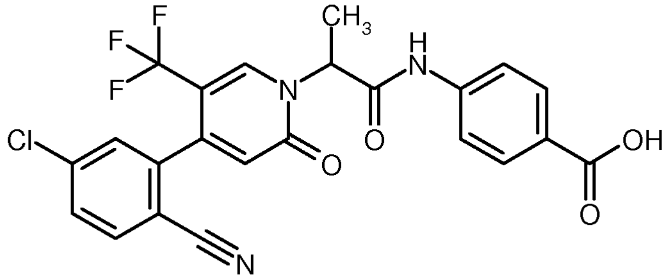

Nach der allgemeinen Methode 2 wurden 251 mg (79% Reinheit, 0.36 mmol) 4-({2-[4-(5-Chlor-2-cyanophenyl)-2-oxo-5-(trifluormethyl)pyridin-1(2*H*)-yl]propanoyl}amino)benzoesäure-*tert*.-butylester (Racemat) (Beispiel 20.1E) mit TFA verseift. Ausbeute: 35 mg (20% d. Th.)
LC/MS [Methode 1]: $R_t$ = 1.02 min; MS (ESIpos): m/z = 490 $(M+H)^+$
$^1$H-NMR (400 MHz, DMSO-$d_6$): δ [ppm] = 12.80 (s, 1H), 10.80 (d, 1H), 8.33 (d, 1H), 8.06 (dd, 1H), 7.92 (d, 2H), 7.85-7.68 (m, 4H), 6.68 (d, 1H), 5.63 (q, 1H), 1.79 (t, 3H).

**Beispiel 55**

4-({2-[4-(5-Chlor-2-cyanophenyl)-5-methoxy-2-oxopyridin-1(2*H*)-yl]propanoyl}amino)-benzoesäure (Racemat)

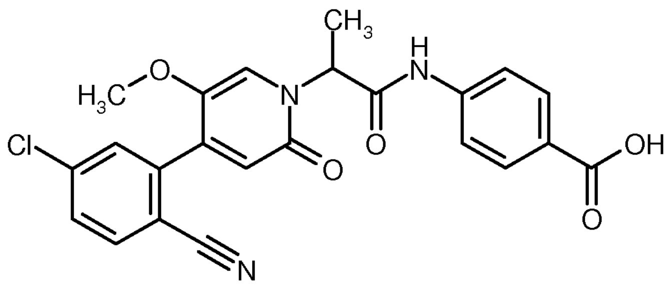

Nach der allgemeinen Methode 2 wurden 42 mg (0.08 mmol) 4-({2-[4-(5-Chlor-2-cyanophenyl)-5-methoxy-2-oxopyridin-1(2*H*)-yl]propanoyl}amino)benzoesäure-*tert*.-butylester (Racemat) (Beispiel 21.1E) mit TFA verseift. Ausbeute: 24 mg (64% d. Th.)
LC/MS [Methode 1]: $R_t$ = 0.92 min; MS (ESIpos): m/z = 452 $(M+H)^+$
$^1$H-NMR (400 MHz, DMSO-$d_6$): δ [ppm] = 12.77 (s, 1H), 10.71 (s, 1H), 8.01 (d, 1H), 7.91 (d, 2H), 7.77-7.71 (m, 4H), 7.47 (s, 1H), 6.53 (s, 1H), 5.60 (q, 1H), 3.70 (s, 3H), 1.74 (d, 3H).

**Beispiel 56**

4-({2-[4-(5-Chlor-2-cyanophenyl)-5-methoxy-2-oxopyridin-1(2*H*)-yl]propanoyl}amino)-benzoesäure (Enantiomer 1)

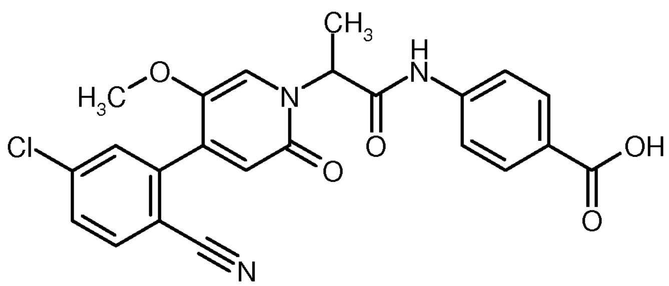

Enantiomerentrennung von 430 mg des Racemates aus Beispiel 55 ergab 214 mg der Titelverbindung Beispiel 56 (Enantiomer 1): Chirale HPLC: $R_t$ = 4.3 min; 99% ee.

Trennmethode: Säule: Daicel Chiralcel OZ 5 μm 250x20 mm; Eluent: 50% iso-Hexan, 50% Ethanol; Ofen: 40°C; Fluss: 15 ml/min; UV-Detektion: 220 nm.

Analytik: Säule: Daicel Chiralcel OZ 5 μm 250x4.6 mm; Eluent: 30% iso-Hexan, 70% Ethanol + 0.2% TFA in 1% Wasser; Ofen: 45°C; Fluss: 1 ml/min; UV-Detektion: 220 nm.

**Beispiel 57**

4-({2-[4-(5-Chlor-2-cyanophenyl)-5-methoxy-2-oxopyridin-1(2*H*)-yl]propanoyl}amino)-benzoesäure (Enantiomer 2)

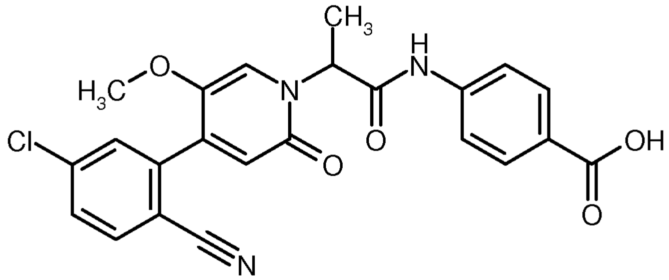

Enantiomerentrennung von 430 mg des Racemates aus Beispiel 55 ergab 223 mg der Titelverbindung Beispiel 57 (Enantiomer 2): Chirale HPLC: $R_t$ = 5.9 min; 99% ee.

Trennmethode: Säule: Daicel Chiralcel OZ 5 μm 250x20 mm; Eluent: 50% iso-Hexan, 50% Ethanol; Ofen: 40°C; Fluss: 15 ml/min; UV-Detektion: 220 nm.

Analytik: Säule: Daicel Chiralcel OZ 5 μm 250x4.6 mm; Eluent: 30% iso-Hexan, 70% Ethanol + 0.2% TFA in 1% Wasser; Ofen: 45°C; Fluss: 1 ml/min; UV-Detektion: 220 nm.

**Beispiel 58**

4-({2-[4-(5-Chlor-2-nitrophenyl)-5-methoxy-2-oxopyridin-1(2*H*)-yl]propanoyl}amino)benzoesäure (Racemat)

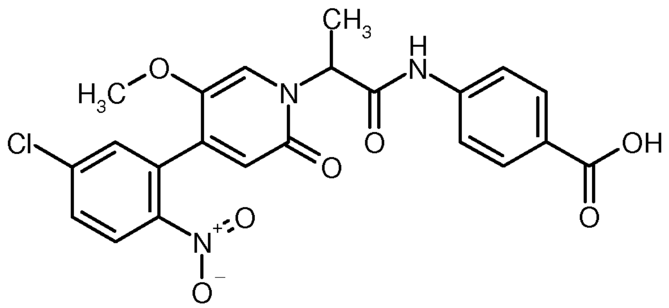

Nach der allgemeinen Methode 2 wurden 32 mg (0.06 mmol) 4-({2-[4-(5-Chlor-2-nitrophenyl)-5-methoxy-2-oxopyridin-1(2*H*)-yl]propanoyl}amino)benzoesäure-*tert*.-butylester (Racemat) (Beispiel 21.2D) mit TFA verseift. Ausbeute: 10 mg (36% d. Th.)
LC/MS [Methode 1]: $R_t$ = 0.93 min; MS (ESIpos): m/z = 472 $(M+H)^+$
$^1$H-NMR (400 MHz, DMSO-$d_6$): δ [ppm] = 12.77 (br. s, 1H), 10.69 (s, 1H), 8.14 (d, 1H), 7.91 (d, 2H), 7.80 (dd, 1H), 7.77-7.70 (m, 3H), 7.34 (s, 1H), 6.59 (s, 1H), 5.59 (q, 1H), 3.55 (s, 3H), 1.71 (d, 3H).

**Beispiel 59**

4-({2-[4-(5-Chlor-2-cyanophenyl)-5-methoxy-2-oxopyridin-1(2*H*)-yl]-3-cyclopropylpropanoyl}-amino)benzoesäure (Enantiomer 1)

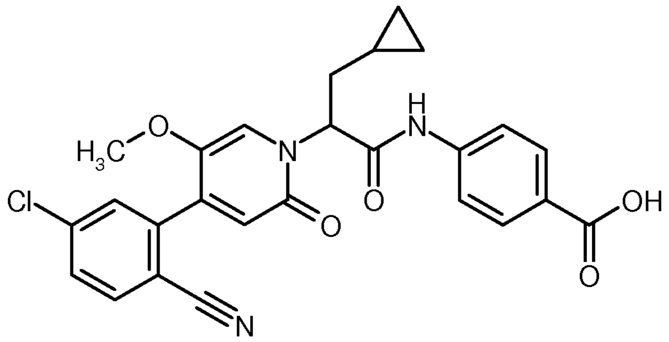

Nach der allgemeinen Methode 2 wurden 161 mg (0.29 mmol) 4-({2-[4-(5-Chlor-2-cyanophenyl)-5-methoxy-2-oxopyridin-1(2*H*)-yl]-3-cyclopropylpropanoyl}amino)benzosäure-*tert.*-butylester (Racemat) (Beispiel 22.1D) mit TFA verseift.

Anschließende Enantiomerentrennung von 88 mg des Racemates ergab 56 mg der Titelverbindung Beispiel 59 (Enantiomer 1): Chirale HPLC: $R_t$ = 4.1 min; 99% ee.

Trennmethode: Säule: Daicel Chiralpak IF 5 μm 250x20 mm; Eluent: 25% iso-Hexan, 75% Ethanol; Ofen: 45°C; Fluss: 15 ml/min; UV-Detektion: 220 nm.

Analytik: Säule: Daicel Chiralpak IF 5 μm 250x4.6 mm; Eluent: 25% iso-Hexan, 75% Ethanol + 0.2% TFA in 1% Wasser; Ofen: 30°C; Fluss: 1 ml/min; UV-Detektion: 220 nm.
LC/MS [Methode 1]: $R_t$ = 1.01 min; MS (ESIpos): m/z = 492 $(M+H)^+$
$^1$H-NMR (400 MHz, DMSO-$d_6$): δ [ppm] = 12.77 (br. s, 1H), 10.79 (s, 1H), 8.00 (d, 1H), 7.91 (d, 2H), 7.78-7.71 (m, 4H), 7.54 (s, 1H), 6.53 (s, 1H), 5.80 (dd, 1H), 3.70 (s, 3H), 2.28-2.18 (m, 1H), 1.96-1.87 (m, 1H), 0.71-0.61 (m, 1H), 0.47-0.33 (m, 2H), 0.23-0.10 (m, 2H).

**<u>Beispiel 60</u>**

4-({2-[4-(5-Chlor-2-cyanophenyl)-5-methoxy-2-oxopyridin-1(2*H*)-yl]-3-cyclopropylpropanoyl}-amino)benzoesäure (Enantiomer 2)

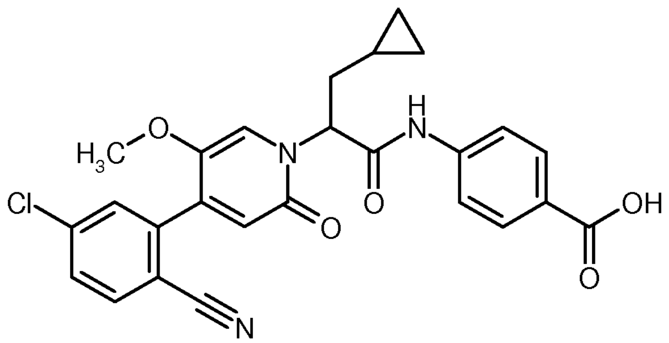

Nach der allgemeinen Methode 2 wurden 150 mg (0.27 mmol) 4-({2-[4-(5-Chlor-2-cyanophenyl)-5-methoxy-2-oxopyridin-1(2*H*)-yl]-3-cyclopropylpropanoyl}amino)benzoesäure-*tert.*-butylester (Beispiel 22.1D) mit TFA verseift.

Anschließende Enantiomerentrennung von 81 mg des Racemates ergab 52 mg der Titelverbindung Beispiel 60 (Enantiomer 2): Chirale HPLC: $R_t$ = 5.4 min; 98% ee.

Trennmethode: Säule: Daicel Chiralpak IF 5 μm 250x20 mm; Eluent: 25% iso-Hexan, 75% Ethanol; Ofen: 45°C; Fluss: 15 ml/min; UV-Detektion: 220 nm.

Analytik: Säule: Daicel Chiralpak IF 5 μm 250x4.6 mm; Eluent: 25% iso-Hexan, 75% Ethanol + 0.2% TFA in 1% Wasser; Ofen: 30°C; Fluss: 1 ml/min; UV-Detektion: 220 nm.
LC/MS [Methode 1]: $R_t$ = 1.02 min; MS (ESIpos): m/z = 492 $(M+H)^+$
$^1$H-NMR (400 MHz, DMSO-$d_6$): δ [ppm] = 12.77 (br. s, 1H), 10.79 (s, 1H), 8.00 (d, 1H), 7.91 (d, 2H), 7.78-7.71 (m, 4H), 7.54 (s, 1H), 6.53 (s, 1H), 5.80 (dd, 1H), 3.70 (s, 3H), 2.28-2.18 (m, 1H), 1.96-1.87 (m, 1H), 0.71-0.61 (m, 1H), 0.47-0.33 (m, 2H), 0.23-0.10 (m, 2H).

**Beispiel 61**

4-({2-[4-(5-Chlor-2-cyanophenyl)-5-methoxy-2-oxopyridin-1(2*H*)-yl]pent-4-inoyl}amino)-benzoesäure (Racemat)

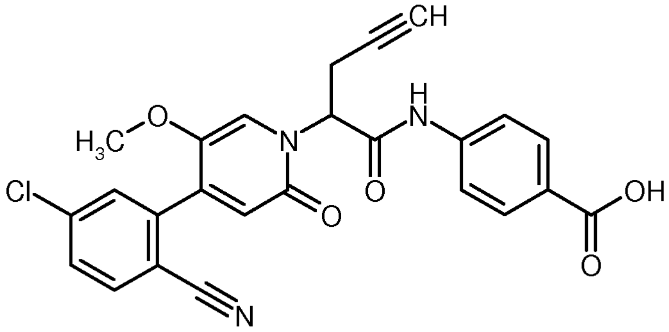

Nach der allgemeinen Methode 2 wurden 91 mg (0.17 mmol) 4-({2-[4-(5-Chlor-2-cyanophenyl)-5-methoxy-2-oxopyridin-1(2*H*)-yl]pent-4-inoyl}amino)benzoesäure-*tert*.-butylester (Racemat) (Beispiel 23.1C) mit TFA verseift. Ausbeute: 55 mg (67% d. Th.)
LC/MS [Methode 1]: $R_t$ = 0.94 min; MS (ESIpos): m/z = 476 $(M+H)^+$
$^1$H-NMR (400 MHz, DMSO-$d_6$): $\delta$ [ppm] = 12.78 (s, 1H), 10.78 (s, 1H), 8.01 (d, 1H), 7.92 (d, 2H), 7.78-7.71 (m, 4H), 7.60 (s, 1H), 6.55 (s, 1H), 5.78 (dd, 1H), 3.69 (s, 3H), 3.30-3.22 (m, 1H), 3.18-3.10 (m, 1H), 3.01-2.97 (m, 1H).

**Beispiel 62**

4-({2-[4-(5-Chlor-2-cyanophenyl)-2-oxo-5-(propan-2-yloxy)pyridin-1(2*H*)-yl]propanoyl}amino)-benzoesäure (Racemat)

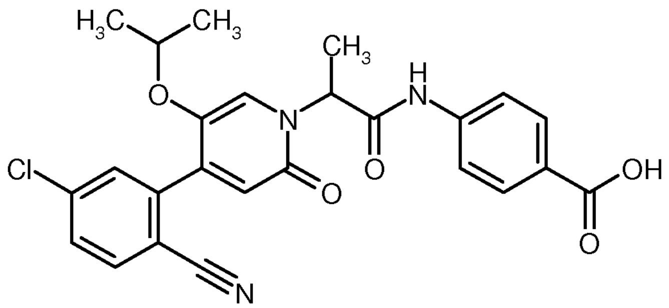

Nach der allgemeinen Methode 2 wurden 31 mg (0.06 mmol) 4-({2-[4-(5-Chlor-2-cyanophenyl)-2-oxo-5-(propan-2-yloxy)pyridin-1(2*H*)-yl]propanoyl}amino)benzoesäure-*tert*.-butylester (Racemat) (Beispiel 24.1I) mit TFA verseift. Ausbeute: 5 mg (18% d. Th.)
LC/MS [Methode 1]: $R_t$ = 1.00 min; MS (ESIpos): m/z = 480 $(M+H)^+$
$^1$H-NMR (400 MHz, DMSO-$d_6$): $\delta$ [ppm] = 12.78 (s, 1H), 10.73 (s, 1H), 8.01 (d, 1H), 7.92 (d, 2H), 7.77-7.70 (m, 4H), 7.55 (s, 1H), 6.53 (s, 1H), 5.65 (q, 1H), 4.10-4.02 (m, 1H), 1.71 (d, 3H), 1.07 (dd, 6H).

**Beispiel 63**

4-({2-[4-(5-Chlor-2-cyanophenyl)-5-methoxy-2-oxopyridin-1(2*H*)-yl]-4-methylpentanoyl}-amino)benzoesäure (Racemat)

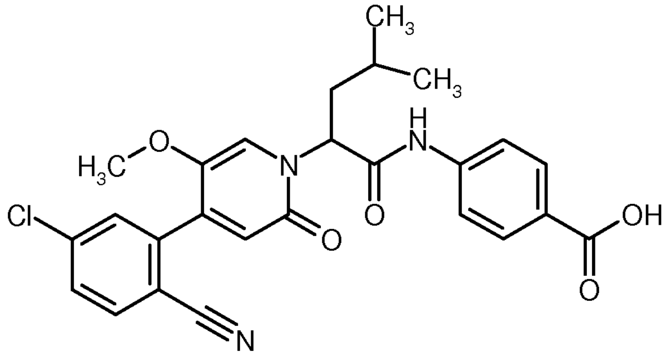

Nach der allgemeinen Methode 2 wurden 127 mg (0.23 mmol) 4-({2-[4-(5-Chlor-2-cyanophenyl)-5-methoxy-2-oxopyridin-1(2*H*)-yl]-4-methylpentanoyl}amino)benzoesäure-*tert.*-butylester (Racemat) (Beispiel 25.1C) mit TFA verseift. Ausbeute: 79 mg (71% d. Th.)
LC/MS [Methode 1]: $R_t$ = 1.07 min; MS (ESIpos): m/z = 494 $(M+H)^+$
$^1$H-NMR (400 MHz, DMSO-$d_6$): δ [ppm] = 12.78 (s, 1H), 10.85 (s, 1H), 8.00 (d, 1H), 7.92 (d, 2H), 7.79-7.71 (m, 4H), 7.50 (s, 1H), 6.54 (s, 1H), 5.86 (dd, 1H), 3.69 (s, 3H), 2.27-2.17 (br. m, 1H), 1.93-1.84 (br. m, 1H), 1.49-1.39 (br. m, 1H), 0.95 (t, 6H).

**Beispiel 64**

4-({2-[4-(5-Chlor-2-cyanophenyl)-5-methoxy-2-oxopyridin-1(2*H*)-yl]-4-methylpentanoyl}amino)-benzoesäure (Enantiomer 1)

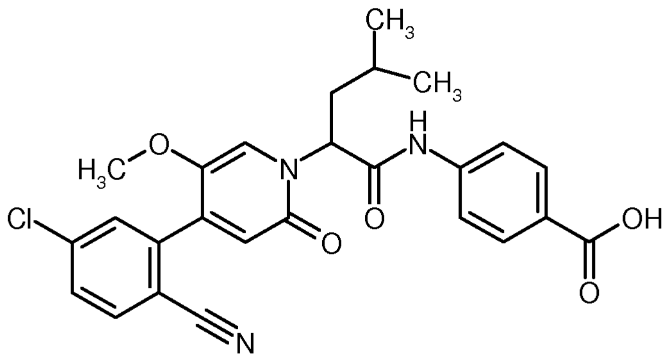

Enantiomerentrennung von 73 mg des Racemates aus Beispiel 63 ergab 37 mg der Titelverbindung Beispiel 64 (Enantiomer 1): Chirale HPLC: $R_t$ = 4.6 min; 99% ee.

Trennmethode: Säule: Daicel Chiralpak IF 5 µm 250x20 mm; Eluent: 50% iso-Hexan, 50% Ethanol; Ofen: 40°C; Fluss: 15 ml/min; UV-Detektion: 220 nm.

Analytik: Säule: Daicel Chiralpak AZ-H 5 µm 250x4.6 mm; Eluent: 50% iso-Hexan, 50% Ethanol + 0.2% TFA in 1% Wasser; Ofen: 40°C; Fluss: 1 ml/min; UV-Detektion: 220 nm.

**Beispiel 65**

4-({2-[4-(5-Chlor-2-cyanophenyl)-5-methoxy-2-oxopyridin-1(2*H*)-yl]-4-methylpentanoyl}amino)-benzoesäure (Enantiomer 2)

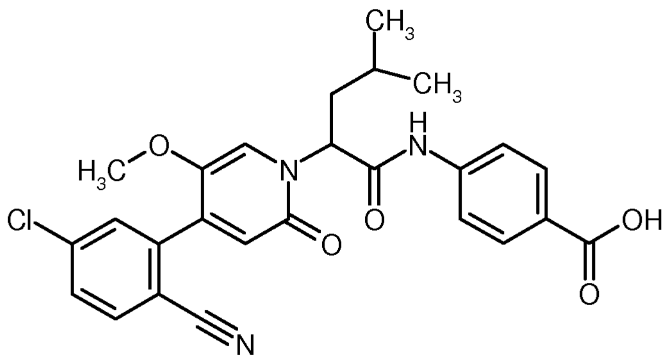

Enantiomerentrennung von 73 mg des Racemates aus Beispiel 63 ergab 33 mg der Titelverbindung Beispiel 65 (Enantiomer 2): Chirale HPLC: $R_t$ = 7.0 min; 99% ee.

Trennmethode: Säule: Daicel Chiralpak IF 5 μm 250x20 mm; Eluent: 50% iso-Hexan, 50% Ethanol; Ofen: 40°C; Fluss: 15 ml/min; UV-Detektion: 220 nm.

Analytik: Säule: Daicel Chiralpak AZ-H 5 μm 250x4.6 mm; Eluent: 50% iso-Hexan, 50% Ethanol + 0.2% TFA in 1% Wasser; Ofen: 40°C; Fluss: 1 ml/min; UV-Detektion: 220 nm.

**Beispiel 66**

4-({2-[4-(5-Chlor-2-cyanophenyl)-5-methoxy-2-oxopyridin-1(2*H*)-yl]butanoyl}amino)benzoesäure (Racemat)

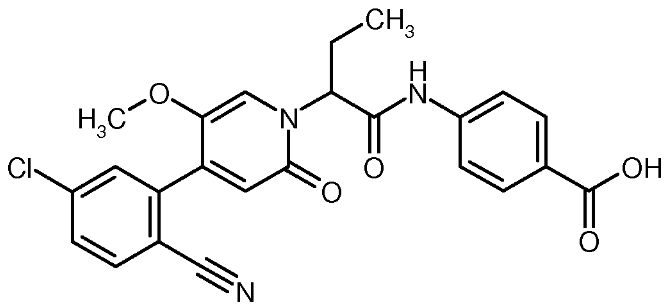

Nach der allgemeinen Methode 2 wurden 68 mg (0.13 mmol) 4-({2-[4-(5-Chlor-2-cyanophenyl)-5-methoxy-2-oxopyridin-1(2*H*)-yl]butanoyl}amino)benzoesäure-*tert*.-butylester (Racemat) (Beispiel 26.1B) mit TFA verseift. Ausbeute: 51 mg (84% d. Th.)
LC/MS [Methode 1]: $R_t$ = 0.94 min; MS (ESIpos): m/z = 466 $(M+H)^+$
$^1$H-NMR (400 MHz, DMSO-$d_6$): δ [ppm] = 12.78 (s, 1H), 10.81 (s, 1H), 8.00 (d, 1H), 7.92 (d, 2H), 7.79-7.71 (m, 4H), 7.50 (s, 1H), 6.54 (s, 1H), 5.64 (dd, 1H), 3.69 (s, 3H), 2.25-2.11 (m, 2H), 0.91 (t, 3H).

Alternativsynthese:

Eine Suspension von 200 mg (0.41 mmol) 4-({2-[4-(5-Chlor-2-cyanophenyl)-5-methoxy-2-oxopyridin-1(2*H*)-yl]butanoyl}amino)benzoesäureethylester (Racemat) in 2.0 ml Ethanol wurde bei RT mit 0.2 ml Wasser und 97 mg (1.22 mmol, 3.0 eq.) Natriumhydroxid versetzt. Das Reaktionsgemisch wurde 1 h bei RT und 6 h bei 60°C Ölbadtemperatur gerührt, über Nacht bei RT stehen gelassen und anschließend auf wässiger Salzsäure (1N) gegeben. Nach Verdünnen mit Wasser fiel ein Niederschlag aus, der filtriert, zweimal mit Wasser gewaschen und im Vakuum getrocknet wurde. Der Niederschlag wurde anschließend mittels Flash-Chromatographie (25 g Kieselgel, Eluent: Dichormethan → Dichlormethan/Methanol 50:1) aufgereinigt. Ausbeute: 144 mg (38% d. Th. bezogen auf 0.82 mmol, da zwei Rohprodukte der angegebenen Größe zusammen aufgereinigt wurden)
LC/MS [Methode 1]: $R_t$ = 0.92 min; MS (ESIneg): m/z = 464 $(M-H)^-$,
$^1$H-NMR (400 MHz, DMSO-$d_6$): δ [ppm] = 12.74 (s, 1H), 10.80 (s, 1H), 8.00 (d, 1H), 7.92 (d, 2H), 7.79-7.70 (m, 4H), 7.50 (s, 1H), 6.54 (s, 1H), 5.65 (dd, 1H), 3.69 (s, 3H), 2.26-2.11 (m, 2H), 0.91 (t, 3H).

**Beispiel 67**

4-({2-[4-(5-Chlor-2-cyanophenyl)-5-methoxy-2-oxopyridin-1(2*H*)-yl]butanoyl}amino)benzoesäure (Enantiomer 1)

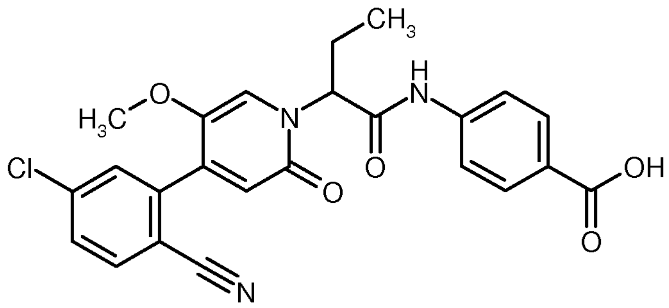

Enantiomerentrennung von 433 mg des Racemates aus Beispiel 66 ergab 196 mg der Titelverbindung Beispiel 67 (Enantiomer 1): Chirale HPLC: $R_t$ = 5.22 min; 99% ee. Trennmethode: Säule: Daicel Chiralpak IF 5 μm 250 mm x 20 mm; Eluent: 50% iso-Hexan, 50% Ethanol + 0.2% Essigsäure; Temperatur: 40°C; Fluss: 15 ml/min; UV-Detektion: 220 nm.

Analytik: Säule: Daicel Chiralpak AZ-H 5 μm 250 mm x 4.6 mm; Eluent: 50% iso-Hexan, 50% Ethanol + 0.2% TFA + 1% Wasser; Temperatur: 40°C; Fluss: 1 ml/min; UV-Detektion: 220 nm.
$^1$H-NMR (400 MHz, DMSO-$d_6$): δ [ppm] = 12.77 (s, 1H), 10.81 (s, 1H), 8.00 (d, 1H), 7.91 (d, 2H), 7.79-7.71 (m, 4H), 7.50 (s, 1H), 6.54 (s, 1H), 5.64 (dd, 1H), 3.69 (s, 3H), 2.26-2.11 (m, 2H), 0.91 (t, 3H).

**Beispiel 68**

4-({2-[4-(5-Chlor-2-cyanophenyl)-5-methoxy-2-oxopyridin-1(2*H*)-yl]butanoyl}amino)benzoesäure (Enantiomer 2)

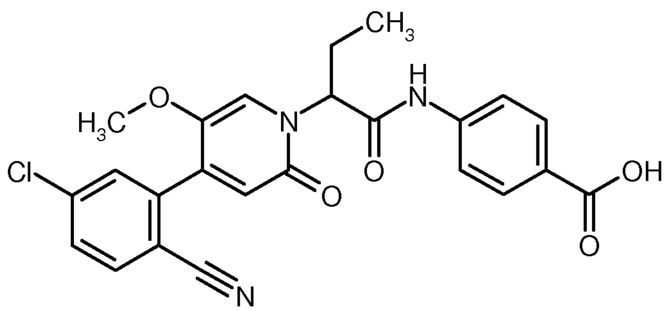

Enantiomerentrennung von 433 mg des Racemates aus Beispiel 66 ergab 201 mg der Titelverbindung Beispiel 68 (Enantiomer 2): Chirale HPLC: $R_t$ = 8.19 min; 99% ee.

Trennmethode: Säule: Daicel Chiralpak IF 5 μm 250 mm x 20 mm; Eluent: 50% iso-Hexan, 50% Ethanol + 0.2% Essigsäure; Temperatur: 40°C; Fluss: 15 ml/min; UV-Detektion: 220 nm.

Analytik: Säule: Daicel Chiralpak AZ-H 5 μm 250 mm x 4.6 mm; Eluent: 50% iso-Hexan, 50% Ethanol + 0.2% TFA + 1% Wasser; Temperatur: 40°C; Fluss: 1 ml/min; UV-Detektion: 220 nm.
$^1$H-NMR (400 MHz, DMSO-$d_6$): δ [ppm] = 12.77 (s, 1H), 10.81 (s, 1H), 8.00 (d, 1H), 7.91 (d, 2H), 7.79-7.71 (m, 4H), 7.50 (s, 1H), 6.54 (s, 1H), 5.64 (dd, 1H), 3.69 (s, 3H), 2.26-2.11 (m, 2H), 0.91 (t, 3H).

**Beispiel 69**

2-[4-(5-Chlor-2-cyanophenyl)-5-methoxy-2-oxopyridin-1(2*H*)-yl]-*N*-[4-(5-oxo-2,5-dihydro-1*H*-pyrazol-3-yl)phenyl]butanamid (Racemat)

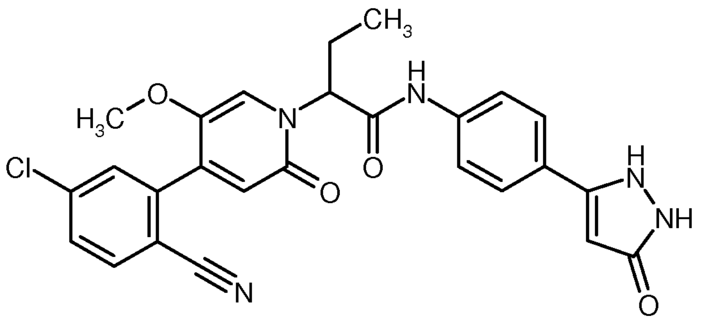

Nach der allgemeinen Methode 2 wurden 17 mg (70% Reinheit, 0.02 mmol) 5-[4-({2-[4-(5-Chlor-2-cyanophenyl)-5-methoxy-2-oxopyridin-1(2*H*)-yl]butanoyl}amino)phenyl]-3-oxo-2,3-dihydro-1*H*-pyrazol-1-carbonsäure-*tert*.-butylester (Racemat) mit TFA verseift. Ausbeute: 8 mg (78% d. Th.)
LC/MS [Methode 1]: $R_t$ = 0.89 min; MS (ESIpos): m/z = 504 $(M+H)^+$
$^1$H-NMR (400 MHz, DMSO-$d_6$): δ [ppm] = 11.96 (br. s, 1H), 10.57 (s, 1H), 9.54 (br. s, 1H), 8.00 (d, 1H), 7.74 (s, 1H), 7.73 (d, 1H), 7.66 (d, 2H), 7.61 (d, 2H), 7.51 (s, 1H), 6.54 (s, 1H), 5.84 (br. s, 1H), 5.64 (dd, 1H), 3.69 (s, 3H), 2.24-2.10 (m, 2H), 0.91 (t, 3H).

**Beispiel 70**

2-[4-(5-Chlor-2-cyanophenyl)-5-methoxy-2-oxopyridin-1(2*H*)-yl]-*N*-(2-methyl-3-oxo-2,3-dihydro-1*H*-indazol-6-yl)butanamid (Racemat)

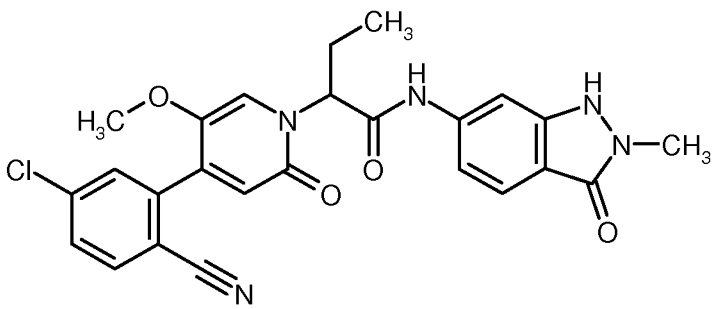

Nach der allgemeinen Methode 2 wurden 112 mg (0.19 mmol) 6-({2-[4-(5-Chlor-2-cyanophenyl)-5-methoxy-2-oxopyridin-1(2*H*)-yl]butanoyl}amino)-2-methyl-3-oxo-2,3-dihydro-1*H*-indazol-1-carbonsäure-*tert*.-butylester (Racemat) mit TFA verseift. Ausbeute: 34 mg (35% d. Th.)
LC/MS [Methode 1]: $R_t$ = 0.85 min; MS (ESIpos): m/z = 492 $(M+H)^+$
$^1$H-NMR (400 MHz, DMSO-$d_6$): δ [ppm] = 10.73 (s, 1H), 10.21 (s, 1H), 8.00 (d, 1H), 7.81-7.71 (m, 3H), 7.57 (d, 1H), 7.51 (s, 1H), 7.16 (dd, 1H), 6.55 (s, 1H), 5.65 (dd, 1H), 3.69 (s, 3H), 2.27-2.10 (m, 2H), 0.91 (t, 3H).

**Beispiel 71**

2-[4-(5-Chlor-2-cyanophenyl)-5-methoxy-2-oxopyridin-1(2*H*)-yl]-*N*-(2-oxo-2,3-dihydro-1*H*-benzimidazol-5-yl)butanamid (Racemat)

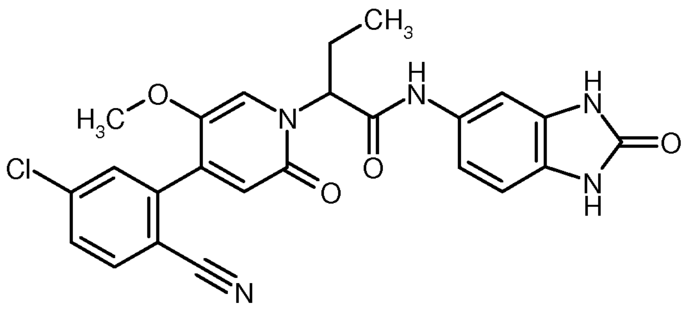

Nach der allgemeinen Methode 1 wurden 71 mg (0.20 mmol) 2-[4-(5-Chlor-2-cyanophenyl)-5-methoxy-2-oxopyridin-1(2*H*)-yl]butansäure (Racemat) mit 33 mg (0.22 mmol, 1.1 eq.) 5-Amino-1,3-dihydro-2*H*-benzimidazol-2-on umgesetzt. Ausbeute: 74 mg (76% d. Th.)
LC/MS [Methode 1]: $R_t$ = 0.86 min; MS (ESIpos): m/z = 478 $(M+H)^+$
$^1$H-NMR (400 MHz, DMSO-$d_6$): δ [ppm] = 10.58 (s, 1H), 10.53 (s, 1H), 10.37 (s, 1H), 8.00 (d, 1H), 7.77-7.70 (m, 2H), 7.53 (s, 1H), 7.45 (d, 1H), 7.08 (dd, 1H), 6.85 (d, 1H), 6.53 (s, 1H), 5.63 (dd, 1H), 3.69 (s, 3H), 2.22-2.03 (m, 2H), 0.90 (t, 3H).

## Beispiel 72

2-[4-(5-Chlor-2-cyanophenyl)-5-methoxy-2-oxopyridin-1(2*H*)-yl]-*N*-[4-(2-oxo-2,3-dihydro-1,3-oxazol-5-yl)phenyl]butanamid (Racemat)

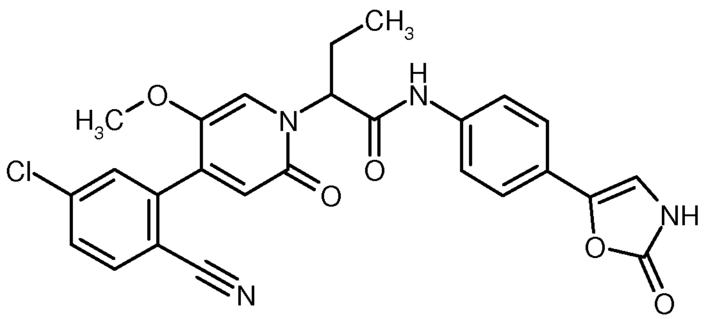

Nach der allgemeinen Methode 1 wurden 87 mg (0.25 mmol) 2-[4-(5-Chlor-2-cyanophenyl)-5-methoxy-2-oxopyridin-1(2*H*)-yl]butansäure (Racemat) mit 60 mg (0.30 mmol, 1.2 eq.) 5-(4-Aminophenyl)-1,3-oxazol-2(3*H*)-on umgesetzt. Ausbeute: 23 mg (93% Reinheit, 17% d. Th.)
LC/MS [Methode 8]: $R_t$ = 1.16 min; MS (ESIneg): m/z = 503 $(M-H)^-$
$^1$H-NMR (400 MHz, DMSO-$d_6$): δ [ppm] = 10.77 (s, 1H), 10.60 (s, 1H), 8.00 (d, 1H), 7.76-7.70 (m, 2H), 7.67 (d, 2H), 7.50 (s, 1H), 7.46 (d, 2H), 7.39 (d, 1H), 6.53 (s, 1H), 5.63 (dd, 1H), 3.69 (s, 3H), 2.25-2.09 (m, 2H), 0.90 (t, 3H).

## Beispiel 73

6-({2-[4-(5-Chlor-2-cyanophenyl)-5-methoxy-2-oxopyridin-1(2*H*)-yl]butanoyl}amino)-1*H*-benzimidazol-2-carbonsäure (Racemat)

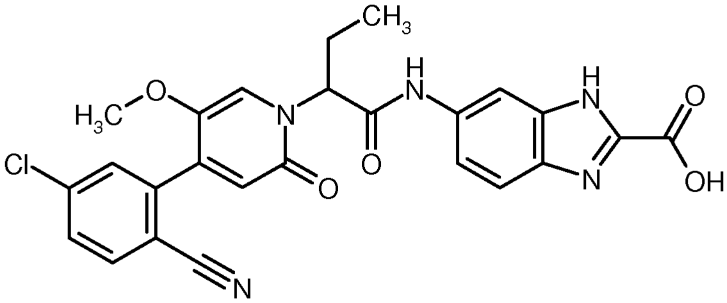

Nach der allgemeinen Methode 3 wurden 86 mg (0.16 mmol) 6-({2-[4-(5-Chlor-2-cyanophenyl)-5-methoxy-2-oxopyridin-1(2*H*)-yl]butanoyl}amino)-1*H*-benzimidazol-2-carbonsäureethylester (Racemat) mit Lithiumhydroxid verseift. Ausbeute: 67 mg (82% d. Th.)

LC/MS [Methode 1]: $R_t$ = 0.76 min; MS (ESIpos): m/z = 506 $(M+H)^+$

$^1$H-NMR (400 MHz, DMSO-$d_6$): $\delta$ [ppm] = 10.68 (s, 1H), 8.17 (s, 1H), 8.01 (d, 1H), 7.78-7.71 (m, 2H), 7.64 (d, 1H), 7.54 (s, 1H), 7.46 (dd, 1H), 6.55 (s, 1H), 5.66 (dd, 1H), 3.70 (s, 3H), 2.28-2.10 (m, 2H), 0.91 (t, 3H).

## Beispiel 74

6-({2-[4-(5-Chlor-2-cyanophenyl)-5-methoxy-2-oxopyridin-1(2*H*)-yl]butanoyl}amino)-1*H*-indol-2-carbonsäure (Racemat)

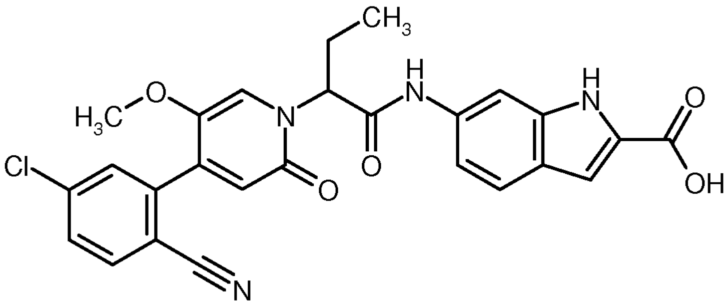

Nach der allgemeinen Methode 3 wurden 75 mg (0.14 mmol) 6-({2-[4-(5-Chlor-2-cyanophenyl)-5-methoxy-2-oxopyridin-1(2*H*)-yl]butanoyl}amino)-1*H*-indol-2-carbonsäureethylester (Racemat) mit Lithiumhydroxid verseift. Ausbeute: 38 mg (48% d. Th.)

LC/MS [Methode 1]: $R_t$ = 1.18 min; MS (ESIneg): m/z = 503 $(M-H)^-$

$^1$H-NMR (400 MHz, DMSO-$d_6$): $\delta$ [ppm] = 12.84 (br. s, 1H), 11.70 (s, 1H), 10.55 (s, 1H), 8.06-7.98 (m, 2H), 7.79-7.70 (m, 2H), 7.57 (d, 1H), 7.54 (s, 1H), 7.19 (dd, 1H), 7.03 (s, 1H), 6.54 (s, 1H), 5.68 (dd, 1H), 3.70 (s, 3H), 2.27-2.08 (m, 2H), 0.92 (t, 3H).

## Beispiel 75

5-({2-[4-(5-Chlor-2-cyanophenyl)-5-methoxy-2-oxopyridin-1(2*H*)-yl]butanoyl}amino)-1*H*-indol-2-carbonsäure (Racemat)

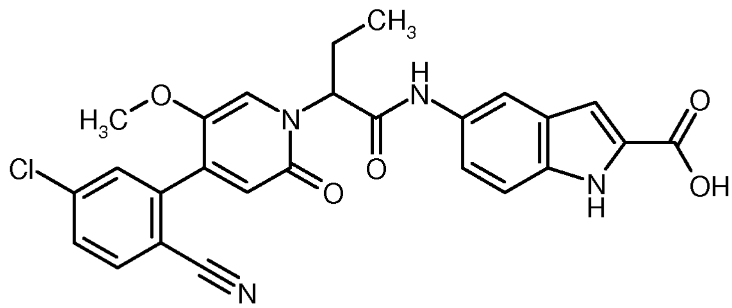

Nach der allgemeinen Methode 3 wurden 94 mg (0.18 mmol) 5-({2-[4-(5-Chlor-2-cyanophenyl)-5-methoxy-2-oxopyridin-1(2*H*)-yl]butanoyl}amino)-1*H*-indol-2-carbonsäureethylester (Racemat) mit Lithiumhydroxid verseift. Ausbeute: 39 mg (43% d. Th.)
LC/MS [Methode 1]: $R_t$ = 1.15 min; MS (ESIneg): m/z = 503 $(M-H)^-$
$^1$H-NMR (400 MHz, DMSO-$d_6$): $\delta$ [ppm] = 10.77 (s, 1H), 10.26 (s, 1H), 8.00 (d, 1H), 7.82 (s, 1H), 7.75 (s, 1H), 7.73 (dd, 1H), 7.56 (s, 1H), 7.22 (d, 1H), 7.15 (d, 1H), 6.54 (s, 1H), 6.43 (s, 1H), 5.68 (dd, 1H), 3.70 (s, 3H), 2.25-2.02 (m, 2H), 0.90 (t, 3H).

**Beispiel 76**

4-({2-[4-(5-Chlor-2-cyanophenyl)-5-methoxy-2-oxopyridin-1(2*H*)-yl]-4-methoxybutanoyl}-amino)benzoesäure (Racemat)

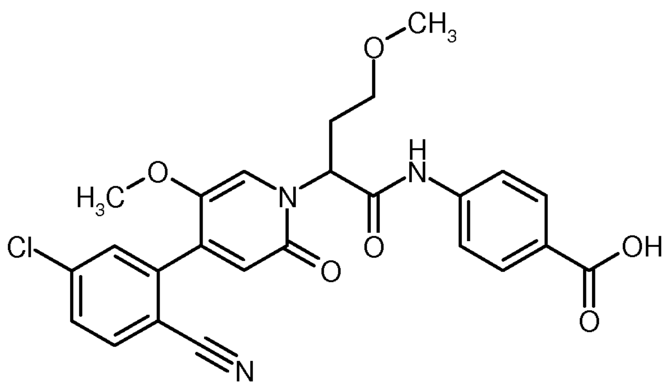

Gemäß allgemeiner Methode 4 wurden 1.80 g (3.40 mmol) 4-({2-[4-(5-Chlor-2-cyanophenyl)-5-methoxy-2-oxopyridin-1(2*H*)-yl]-4-methoxybutanoyl}amino)benzoesäureethylester (Racemat) in 45 ml Methanol/Wasser (4/1) mit 2.24 g (6.87 mmol) Cäsiumcarbonat zur Reaktion gebracht und die Titelverbindung erhalten. Ausbeute: 1.66 g (88% d. Th.)
LC/MS [Methode 1]: $R_t$ = 0.89 min; MS (ESIneg): m/z = 494 $(M-H)^-$
$^1$H-NMR (400 MHz, DMSO-$d_6$): $\delta$ [ppm] = 12.8 (s, 1H), 10.8 (s, 1H), 8.00 (d, 1H), 7.91 (d, 2H), 7.77 (d, 2H), 7.75 (s, 1H), 7.74 (dd, 1H), 7.51 (s, 1H), 6.53 (s, 1H), 5.76 (t, 1H), 3.69 (s, 3H), 3.43-3.25 (m, 2H), 3.20 (s, 3H), 2.44-2.39 (m, 2H).

**Beispiel 77**

(+)-4-({2-[4-(5-Chlor-2-cyanophenyl)-5-methoxy-2-oxopyridin-1(2*H*)-yl]-4-methoxybutanoyl}-amino)benzoesäure (Enantiomer 1)

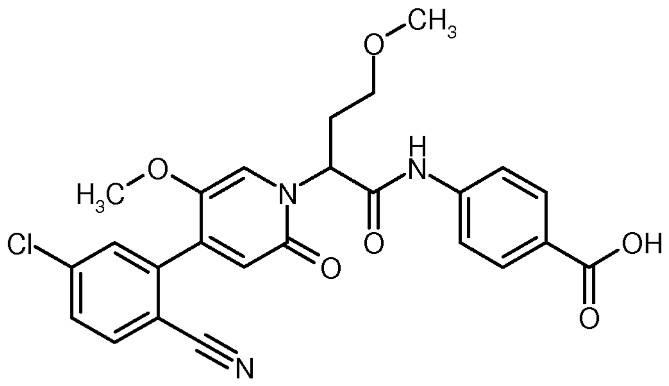

Enantiomerentrennung von 1.66 g des Racemates aus Beispiel 76 ergab 707 mg der Titelverbindung Beispiel 77 (Enantiomer 1): Chirale HPLC: $R_t$ = 4.59 min; 99% ee.
Drehwert: $[\alpha]_{589}^{20.1}$ = + 95.82° (c 0.255 g/100 ml, Methanol)

Trennmethode (SFC): Säule: AZ-H 5 μm 250 mm x 20 mm; Eluent: 70% Kohlendioxid, 30% Ethanol; Temperatur: 40°C; Fluss: 80 ml/min; Druck: 100 bar; UV-Detektion: 210 nm.

Analytik (SFC): Säule: AZ-H 5 μm 250 mm x 4.6 mm; Eluent: 70% Kohlendioxid, 30% Ethanol; Temperatur: 40°C; Fluss: 3 ml/min; Druck: 100 bar; UV-Detektion: 210 nm.

## Beispiel 78

(-)-4-({2-[4-(5-Chlor-2-cyanophenyl)-5-methoxy-2-oxopyridin-1(2*H*)-yl]-4-methoxybutanoyl}-amino)benzoesäure (Enantiomer 2)

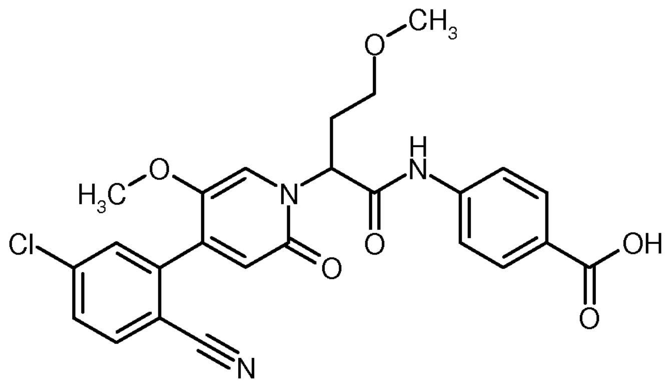

Enantiomerentrennung von 1.66 g des Racemates aus Beispiel 76 ergab 631 mg der Titelverbindung Beispiel 78 (Enantiomer 2): Chirale HPLC: $R_t$ = 8.11 min; 98% ee.
Drehwert: $[\alpha]_{589}^{19.9}$ = - 95.05° (c 0.33 g/100 ml, Methanol)

Trennmethode (SFC): Säule: AZ-H 5 μm 250x20 mm; Eluent: 70% Kohlendioxid, 30% Ethanol; Temperatur: 40°C; Fluss: 80 ml/min; Druck: 100 bar; UV-Detektion: 210 nm.

Analytik (SFC): Säule: AZ-H 5 μm 250x4.6 mm; Eluent: 70% Kohlendioxid, 30% Ethanol; Temperatur: 40°C; Fluss: 3 ml/min; Druck: 100 bar; UV-Detektion: 210 nm.

## Beispiel 79

4-({(4*S*)-2-[4-(5-Chlor-2-cyanophenyl)-5-methoxy-2-oxopyridin-1(2*H*)-yl]-4-methoxypentanoyl}-amino)benzoesäure (Gemisch enantiomerenreiner Diastereomere 1 und 2)

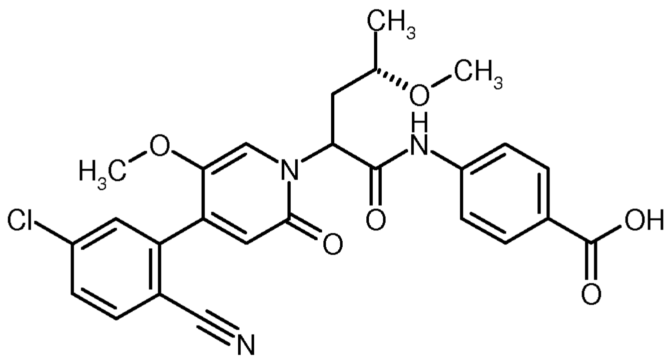

Nach der allgemeinen Methode 2 wurden 387 mg (0.66 mmol) 4-({(4*S*)-2-[4-(5-Chlor-2-cyanophenyl)-5-methoxy-2-oxopyridin-1(2*H*)-yl]-4-methoxypentanoyl}amino)benzoesäure-*tert*.-butylester (Gemisch enantiomerenreiner Diastereomere 1 und 2) mit TFA verseift. Ausbeute: 245 mg (70% d. Th.)
LC/MS [Methode 1]: $R_t$ = 0.96 min; MS (ESIpos): m/z = 510 $(M+H)^+$
$^1$H-NMR (400 MHz, DMSO-$d_6$): δ [ppm] = 12.77 (br. s, 1H), 10.80/10.75 (2x s, 1H), 8.00 (d, 1H), 7.94-7.87 (m, 2H), 7.81-7.71 (m, 4H), 7.57/7.51 (2x s, 1H), 6.53 (2x s, 1H), 5.89-5.80 (m, 1H), 3.69 (s, 3H), 3.25-3.19/3.17-3.09 (2x m, 1H), 3.19/3.12 (2x s, 3H), 2.43-2.28 (m, 1H), 2.28-2.17 (m, 1H), 1.16/1.14 (2x d, 3H).

## Beispiel 80

4-({(4*S*)-2-[4-(5-Chlor-2-cyanophenyl)-5-methoxy-2-oxopyridin-1(2*H*)-yl]-4-methoxypentanoyl}-amino)benzoesäure (enantiomerenreines Diastereomer 1)

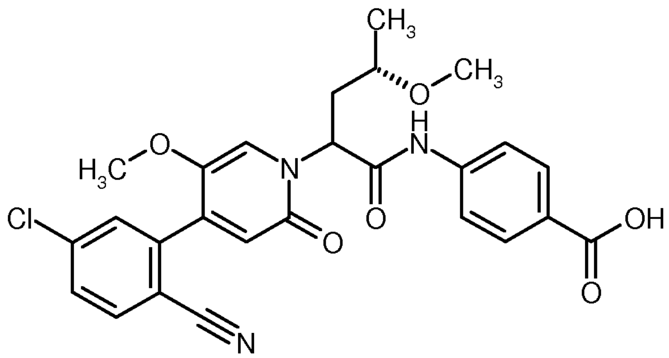

Diastereomerentrennung von 240 mg des Gemisches aus Beispiel 79 ergab 57 mg der Titelverbindung Beispiel 80 (enantiomerenreines Diastereomer 1): Chirale HPLC: $R_t$ = 8.1 min; Diastereomerenreinheit: >99%.
Trennmethode: Säule: Daicel Chiralpak IF 5 μm 250 mm x 20 mm; Eluent: 50% iso-Hexan, 50% Ethanol + 0.2% TFA + 1% Wasser; Temperatur: 23°C; Fluss: 20 ml/min; UV-Detektion: 220 nm.
Analytik: Säule: Daicel Chiralpak AZ-H 5 μm 250 mm x 4.6 mm; Eluent: 50% iso-Hexan, 50% Ethanol + 0.2% TFA + 1% Wasser; Temperatur: 40°C; Fluss: 1 ml/min; UV-Detektion: 220 nm.
$^1$H-NMR (400 MHz, DMSO-$d_6$): δ [ppm] = 12.8 (br. s, 1H), 10.75 (s, 1H), 8.00 (d, 1H), 7.90 (d, 2H), 7.81-7.70 (m, 4H), 7.51 (s, 1H), 6.53 (s, 1H), 5.84 (dd, 1H), 3.69 (s, 3H), 3.17-3.08 (m, 1H), 3.12 (s, 3H), 2.44-2.34 (m, 1H), 2.29-2.18 (m, 1H), 1.14 (d, 3H).

## Beispiel 81

4-({(4*S*)-2-[4-(5-Chlor-2-cyanophenyl)-5-methoxy-2-oxopyridin-1(2*H*)-yl]-4-methoxypentanoyl}-amino)benzoesäure (enantiomerenreines Diastereomer 2)

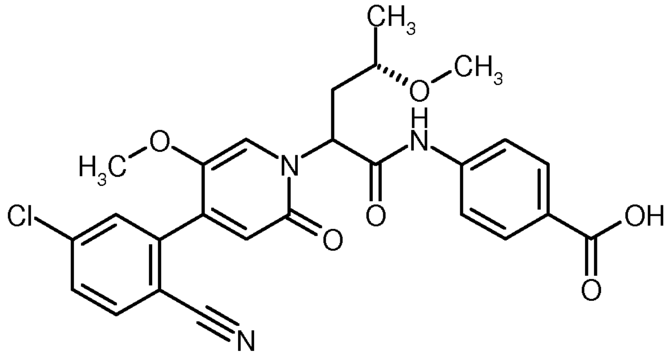

Diastereomerentrennung von 240 mg des Gemisches aus Beispiel 79 ergab 10 mg der Titelverbindung Beispiel 81 (enantiomerenreines Diastereomer 2): Chirale HPLC: $R_t$ = 10.9 min; Diastereomerenreinheit: 98%.

Trennmethode: Säule: Daicel Chiralpak IF 5 μm 250 mm x 20 mm; Eluent: 50% iso-Hexan, 50% Ethanol + 0.2% TFA + 1% Wasser; Temperatur: 23°C; Fluss: 20 ml/min; UV-Detektion: 220 nm.

Analytik: Säule: Daicel Chiralpak AZ-H 5 μm 250 mm x 4.6 mm; Eluent: 50% iso-Hexan, 50% Ethanol + 0.2% TFA + 1% Wasser; Temperatur: 40°C; Fluss: 1 ml/min; UV-Detektion: 220 nm.
$^1$H-NMR (400 MHz, DMSO-$d_6$): δ [ppm] = 12.75 (br. s, 1H), 10.77 (s, 1H), 7.99 (d, 1H), 7.91 (d, 2H), 7.81-7.70 (m, 4H), 7.57 (s, 1H), 6.53 (s, 1H), 5.85 (dd, 1H), 3.69 (s, 3H), 3.26-3.20 (m, 1H), 3.19 (s, 3H), 2.36-2.27 (m, 1H), 2.26-2.18 (m, 1H), 1.16 (d, 3H).

## Beispiel 82

4-({(4*R*)-2-[4-(5-Chlor-2-cyanophenyl)-5-methoxy-2-oxopyridin-1(2*H*)-yl]-4-methoxypentanoyl}-amino)benzoesäure (Gemisch enantiomerenreiner Diastereomere 1 und 2)

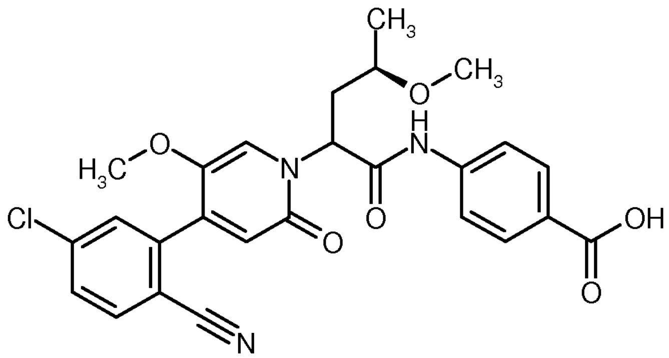

Nach der allgemeinen Methode 2 wurden 51 mg (0.09 mmol) 4-({(4*R*)-2-[4-(5-Chlor-2-cyanophenyl)-5-methoxy-2-oxopyridin-1(2*H*)-yl]-4-methoxypentanoyl}amino)benzoesäure-*tert*.-butylester (Gemisch enantiomerenreiner Diastereomere 1 und 2) mit TFA verseift. Ausbeute: 27 mg (58% d. Th.)
LC/MS [Methode 8]: $R_t$ = 1.20 min; MS (ESIneg): m/z = 508 $(M-H)^-$
$^1$H-NMR (400 MHz, DMSO-$d_6$): δ [ppm] = 12.76 (br. s, 1H), 10.80/10.74 (2x s, 1H), 8.00 (d, 1H), 7.94-7.86 (m, 2H), 7.81-7.70 (m, 4H), 7.57/7.51 (2x s, 1H), 6.53 (2x s, 1H), 5.89-5.79 (m, 1H), 3.69 (s, 3H), 3.25-3.19/3.17-3.09 (2x m, 1H), 3.19/3.12 (2x s, 3H), 2.43-2.28 (m, 1H), 2.28-2.17 (m, 1H), 1.16/1.14 (2x d, 3H).

## Beispiel 83

4-({(4*R*)-2-[4-(5-Chlor-2-cyanophenyl)-5-methoxy-2-oxopyridin-1(2*H*)-yl]-4-methoxypentanoyl}-amino)benzoesäure (enantiomerenreines Diastereomer 1)

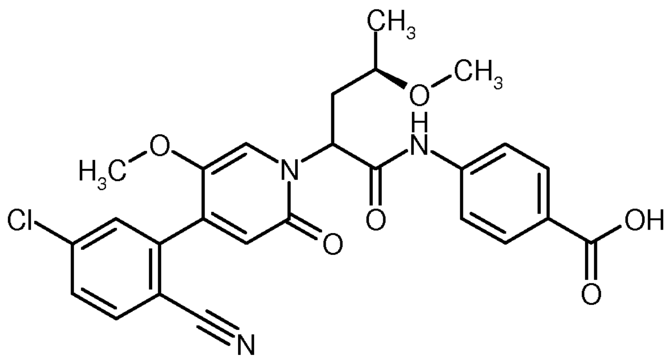

Nach der allgemeinen Methode 2 wurden 26 mg (0.05 mmol) 4-({(4*R*)-2-[4-(5-Chlor-2-cyanophenyl)-5-methoxy-2-oxopyridin-1(2*H*)-yl]-4-methoxypentanoyl}amino)benzoesäure-*tert*.-butylester (enantiomerenreines Diastereomer 1) mit TFA verseift. Ausbeute: 11 mg (45% d. Th.)
LC/MS [Methode 8]: $R_t$ = 1.20 min; MS (ESIneg): m/z = 508 $(M-H)^-$ $^1$H-NMR (400 MHz, DMSO-$d_6$): δ [ppm] = 12.76 (br. s, 1H), 10.74 (s, 1H), 8.00 (d, 1H), 7.94-7.86 (m, 2H), 7.81-7.70 (m, 4H), 7.51 (s, 1H), 6.53 (s, 1H), 5.84 (dd, 1H), 3.69 (s, 3H), 3.25-3.09 (m, 1H), 3.12 (s, 3H), 2.43-2.28 (m, 1H), 2.28-2.17 (m, 1H), 1.14 (d, 3H).

**Beispiel 84**

4-({2-[4-(5-Chlor-2-cyanophenyl)-5-methoxy-2-oxopyridin-1(2*H*)-yl]-3-[(2*R*)-tetrahydrofuran-2-yl]propanoyl}amino)benzoesäure (Gemisch enantiomerenreiner Diastereomere 1 und 2)

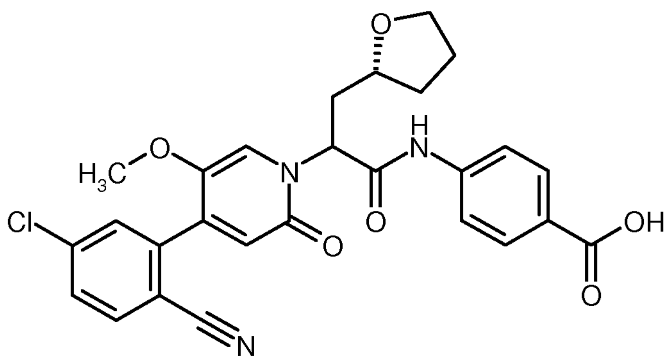

Nach der allgemeinen Methode 2 wurden 327 mg (0.57 mmol) 4-({2-[4-(5-Chlor-2-cyanophenyl)-5-methoxy-2-oxopyridin-1(2*H*)-yl]-3-[(2*R*)-tetrahydrofuran-2-yl]propanoyl}amino)benzoesäure-*tert*.-butylester (Gemisch enantiomerenreiner Diastereomere 1 und 2) mit TFA verseift. Ausbeute: 227 mg (94% Reinheit, 72% d. Th.)
LC/MS [Methode 1]: $R_t$ = 0.94 min; MS (ESIpos): m/z = 522 $(M+H)^+$.

**Beispiel 85**

4-({2-[4-(5-Chlor-2-cyanophenyl)-5-methoxy-2-oxopyridin-1(2*H*)-yl]-3-[(2*R*)-tetrahydrofuran-2-yl]propanoyl}amino)benzoesäure (enantiomerenreines Diastereomer 1)

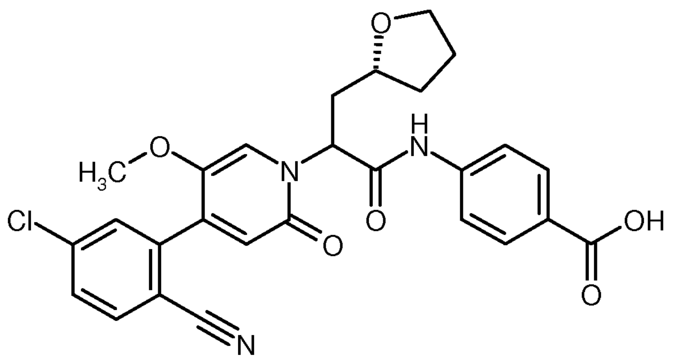

Diastereomerentrennung von 227 mg des Gemisches aus Beispiel 84 ergab 61 mg der Titelverbindung Beispiel 85 (enantiomerenreines Diastereomer 1): Chirale HPLC: $R_t$ = 4.04 min; Diastereomerenreinheit: >99%.

Trennmethode (SFC): Säule: Daicel Chiralpak IF 5 μm 250 mm x 20 mm; Eluent: 80% Kohlendioxid, 20% Ethanol; Temperatur: 40°C; Fluss: 100 ml/min; UV-Detektion: 210 nm.

Analytik (SFC): Säule: Daicel Chiralpak AZ-H 5 μm 250 mm x 4.6 mm; Eluent: 60% Kohlendioxid, 40% Ethanol; Fluss: 3 ml/min; UV-Detektion: 220 nm.

$^1$H-NMR (400 MHz, DMSO-$d_6$): δ [ppm] = 10.80 (s, 1H), 8.00 (d, 1H), 7.91 (d, 2H), 7.81-7.70 (m, 4H), 7.55 (s, 1H), 6.52 (s, 1H), 5.83 (t, 1H), 3.80-3.70 (m, 2H), 3.68 (s, 3H), 3.59 (q, 1H), 2.39-2.24 (m, 2H), 2.01-1.72 (3x m, 3H), 1.69-1.57 (m, 1H).

## Beispiel 86

4-({2-[4-(5-Chlor-2-cyanophenyl)-5-methoxy-2-oxopyridin-1(2*H*)-yl]-3-[(2*R*)-tetrahydrofuran-2-yl]propanoyl}amino)benzoesäure (enantiomerenreines Diastereomer 2)

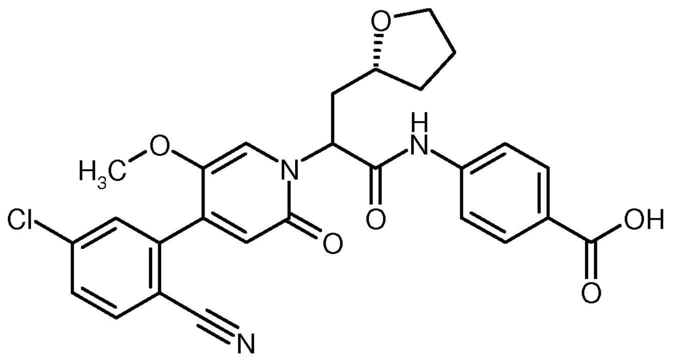

Diastereomerentrennung von 227 mg des Gemisches aus Beispiel 84 ergab 70 mg der Titelverbindung Beispiel 86 (enantiomerenreines Diastereomer 2): Chirale HPLC: $R_t$ = 6.62 min; Diastereomerenreinheit: 95%.

Trennmethode (SFC): Säule: Daicel Chiralpak IF 5 μm 250 mm x 20 mm; Eluent: 80% Kohlendioxid, 20% Ethanol; Temperatur: 40°C; Fluss: 100 ml/min; UV-Detektion: 210 nm.

Analytik (SFC): Säule: Daicel Chiralpak AZ-H 5 μm 250 mm x 4.6 mm; Eluent: 60% Kohlendioxid, 40% Ethanol; Fluss: 3 ml/min; UV-Detektion: 220 nm.

$^1$H-NMR (400 MHz, DMSO-$d_6$): δ [ppm] = 10.79 (s, 1H), 8.00 (d, 1H), 7.91 (d, 2H), 7.81-7.70 (m, 4H), 7.51 (s, 1H), 6.52 (s, 1H), 5.81 (dd, 1H), 3.81-3.73 (m, 1H), 3.73-3.65 (m, 1H), 3.69 (s, 3H), 3.63-3.54 (q, 1H), 2.50-2.41 (m, 1H), 2.31-2.21 (m, 1H), 2.01-1.72 (3x m, 3H), 1.55-1.42 (m, 1H).

**Beispiel 87**

4-({2-[4-(5-Chlor-2-cyanophenyl)-5-methoxy-2-oxopyridin-1(2*H*)-yl]-3-[(2*S*)-tetrahydrofuran-2-yl]propanoyl}amino)benzoesäure (Gemisch enantiomerenreiner Diastereomere 1 und 2)

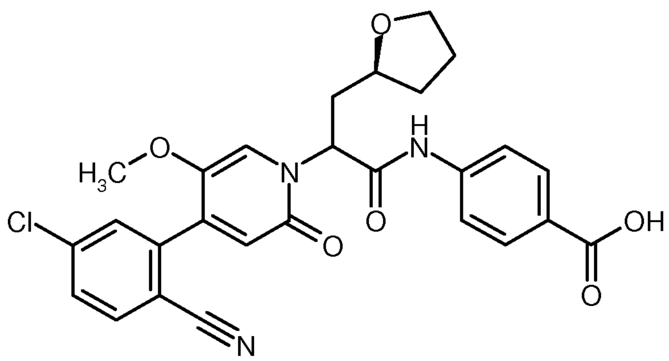

Nach der allgemeinen Methode 2 wurden 1612 mg (2.78 mmol) 4-({2-[4-(5-Chlor-2-cyanophenyl)-5-methoxy-2-oxopyridin-1(2*H*)-yl]-3-[(2*S*)-tetrahydrofuran-2-yl]propanoyl}amino)benzoesäure-*tert.*-butylester (Gemisch enantiomerenreiner Diastereomere 1 und 2) mit TFA verseift. Ausbeute: 1270 mg (90% Reinheit, 79% d. Th.)
LC/MS [Methode 8]: $R_t$ = 1.19 min; MS (ESIpos): m/z = 522 $(M+H)^+$.

**Beispiel 88**

4-({2-[4-(5-Chlor-2-cyanophenyl)-5-methoxy-2-oxopyridin-1(2*H*)-yl]-3-[(2*S*)-tetrahydrofuran-2-yl]propanoyl}amino)benzoesäure (enantiomerenreines Diastereomer 1)

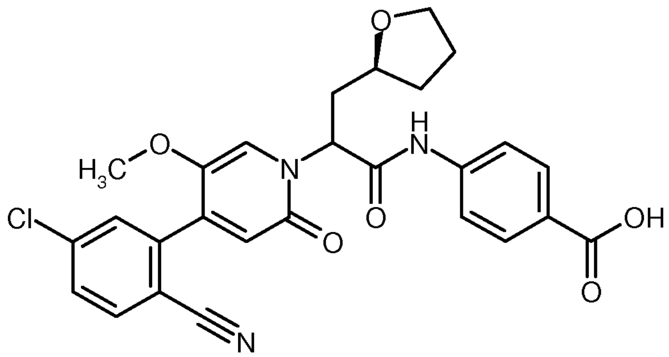

Diastereomerentrennung von 1270 mg des Gemisches aus Beispiel 87 ergab 350 mg der Titelverbindung Beispiel 88 (enantiomerenreines Diastereomer 1): Chirale HPLC: $R_t$ = 4.31 min; Diastereomerenreinheit: >99%.

Trennmethode (SFC): Säule: Daicel Chiralpak IC 5 μm 250 mm x 20 mm; Eluent: 25%

Kohlendioxid, 75% Ethanol; Temperatur: 40°C; Fluss: 100 ml/min; UV-Detektion: 210 nm.

Analytik (SFC): Säule: Daicel IC 5 μm 250 mm x 4.6 mm; Eluent: 70% Kohlendioxid, 30% Ethanol; Fluss: 3 ml/min; UV-Detektion: 210 nm.
$^1$H-NMR (400 MHz, DMSO-$d_6$): δ [ppm] = 12.75 (br. s, 1H), 10.77 (s, 1H), 7.99 (d, 1H), 7.91 (d, 2H), 7.77 (d, 2H), 7.75-7.70 (m, 2H), 7.55 (s, 1H), 6.52 (s, 1H), 5.83 (t, 1H), 3.80-3.70 (m, 2H), 3.68 (s, 3H), 3.59 (q, 1H), 2.36-2.24 (m, 2H), 2.01-1.72 (3x m, 3H), 1.69-1.57 (m, 1H).

**Beispiel 89**

4-({2-[4-(5-Chlor-2-cyanophenyl)-5-methoxy-2-oxopyridin-1(2H*h*)-yl]-3-[(2*S*)-tetrahydrofuran-2-yl]propanoyl}amino)benzoesäure (enantiomerenreines Diastereomer 2)

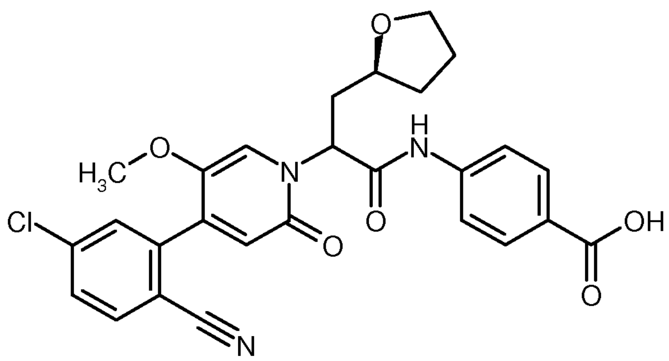

Diastereomerentrennung von 1270 mg des Gemisches aus Beispiel 87 ergab 452 mg der Titelverbindung Beispiel 89 (enantiomerenreines Diastereomer 2): Chirale HPLC: $R_t$ = 6.69 min; Diastereomerenreinheit: >99%.

Trennmethode (SFC): Säule: Daicel Chiralpak IC 5 μm 250 mm x 20 mm; Eluent: 25% Kohlendioxid, 75% Ethanol; Temperatur: 40°C; Fluss: 100 ml/min; UV-Detektion: 210 nm.

Analytik (SFC): Säule: Daicel IC 5 μm 250 mm x 4.6 mm; Eluent: 70% Kohlendioxid, 30% Ethanol; Fluss: 3 ml/min; UV-Detektion: 210 nm.

$^1$H-NMR (400 MHz, DMSO-$d_6$): δ [ppm] = 12.74 (s, 1H), 10.77 (s, 1H), 7.99 (d, 1H), 7.90 (d, 2H), 7.80-7.70 (m, 4H), 7.51 (s, 1H), 6.52 (s, 1H), 5.81 (dd, 1H), 3.81-3.73 (m, 1H), 3.73-3.65 (m, 1H), 3.69 (s, 3H), 3.59 (q, 1H), 2.50-2.41 (m, 1H), 2.31-2.22 (m, 1H), 2.01-1.72 (3x m, 3H), 1.54-1.43 (m, 1H).

**Beispiel 90**

4-({2-[4-(5-Chlor-2-cyanophenyl)-5-methoxy-2-oxopyridin-1(2*H*)-yl]-3-(tetrahydrofuran-3-yl)propanoyl}amino)benzoesäure (Gemisch racemischer Diastereomere)

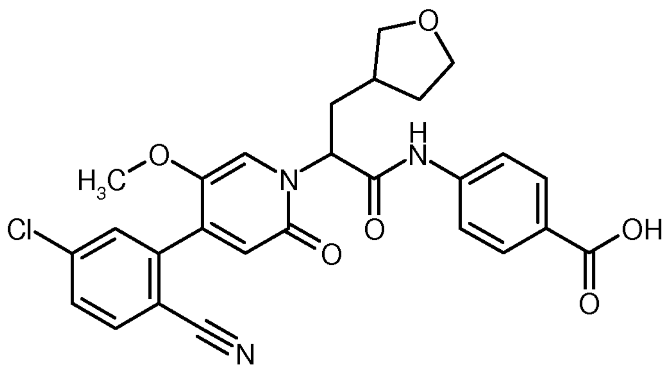

Nach der allgemeinen Methode 2 wurden 795 mg (1.3 mmol) 4-({2-[4-(5-Chlor-2-cyanophenyl)-5-methoxy-2-oxopyridin-1(2*H*)-yl]-3-(tetrahydrofuran-3-yl)propanoyl}amino)benzoesäure-*tert*.-butylester (Gemisch racemischer Diastereomere) mit TFA verseift. Ausbeute: 405 mg (59% d. Th.)

LC/MS [Methode 1]: $R_t$ = 0.92 min; MS (ESIpos): m/z = 522 $(M+H)^+$

$^1$H-NMR (400 MHz, DMSO-$d_6$): δ [ppm] = 12.78 (s, 1H), 10.86/10.85 (2x s, 1H), 8.00 (d, 1H), 7.92 (d, 2H), 7.80-7.70 (m, 4H), 7.56/7.53 (2x s, 1H), 6.55 (s, 1H), 5.81/5.76 (2x dd, 1H), 3.82-3.68 (m, 2H), 3.70 (s, 3H), 3.60 (q, 1H), 3.44/3.26 (2x dd, 1H), 2.39-2.26 (m, 1H), 2.23-2.10 (m, 1H), 2.05-1.88 (m, 2H), 1.74-1.62/1.58-1.45 (2x m, 1H).

**Beispiel 91**

4-({2-[4-(5-Chlor-2-cyanophenyl)-5-methoxy-2-oxopyridin-1(2*H*)-yl]-3-(tetrahydrofuran-3-yl)propanoyl}amino)benzoesäure (Enantiomer 1 des 2. Diastereomers)

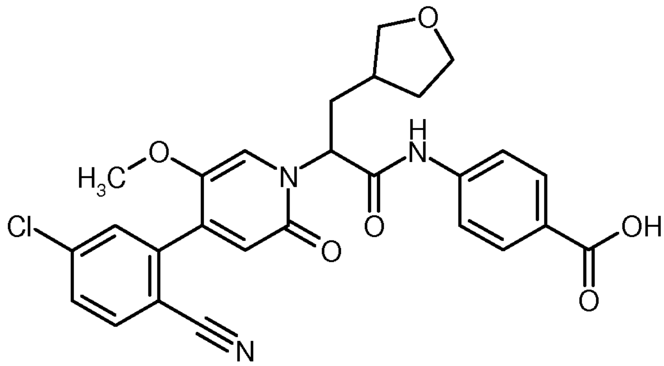

Diastereomeren- und Enantiomerentrennung von 400 mg des Gemisches aus Beispiel 90 ergab 8 mg der Titelverbindung Beispiel 91 (Enantiomer 1 des 2. Diastereomers): Chirale HPLC: $R_t$ = 5.73 min; >99% ee.

Trennmethode (SFC): Säule: Daicel Chiralpak AD-H 5 μm 250 mm x 20 mm; Eluent: 80% Kohlendioxid, 20% Ethanol; Temperatur: 40°C; Fluss: 80 ml/min; UV-Detektion: 210 nm.

Analytik (SFC): Säule: Daicel Chiralpak AZ-H 5 μm 250 mm x 4.6 mm; Eluent: 70% Kohlendioxid, 30% Ethanol; Fluss: 3 ml/min; UV-Detektion: 220 nm.
$^1$H-NMR (400 MHz, DMSO-$d_6$): δ [ppm] = 12.74 (s, 1H), 10.84 (s, 1H), 8.00 (d, 1H), 7.92 (d, 2H), 7.82-7.68 (m, 4H), 7.53 (s, 1H), 6.55 (s, 1H), 5.81 (br. s, 1H), 3.82-3.65 (m, 2H), 3.70 (s, 3H), 3.65-3.53 (m, 1H), 3.30-3.23 (m, 1H), 2.39-2.26 (m, 1H), 2.23-2.10 (m, 1H), 2.07-1.88 (m, 2H), 1.74-1.62 (m, 1H).

**Beispiel 92**

4-({2-[4-(5-Chlor-2-cyanophenyl)-5-methoxy-2-oxopyridin-1(2*H*)-yl]-3-(tetrahydrofuran-3-yl)propanoyl}amino)benzoesäure (Enantiomer 2 des 2. Diastereomers)

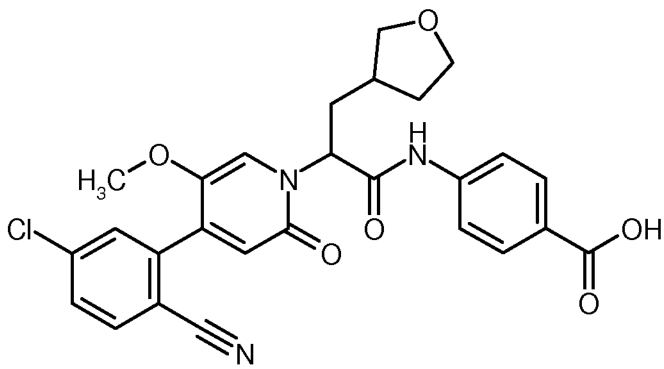

Diastereomeren- und Enantiomerentrennung von 400 mg des Gemisches aus Beispiel 90 ergab 32 mg der Titelverbindung Beispiel 92 (Enantiomer 2 des 2. Diastereomers): Chirale HPLC: $R_t$ = 8.96 min; >99% ee.

Trennmethode (SFC): Säule: Daicel Chiralpak OJ-H 5 μm 250 mm x 20 mm; Eluent: 75% Kohlendioxid, 25% Ethanol; Temperatur: 40°C; Fluss: 80 ml/min; UV-Detektion: 210 nm.

Analytik (SFC): Säule: Daicel Chiralpak AZ-H 5 μm 250 mm x 4.6 mm; Eluent: 70% Kohlendioxid, 30% Ethanol; Fluss: 3 ml/min; UV-Detektion: 220 nm.
$^1$H-NMR (400 MHz, DMSO-$d_6$): δ [ppm] = 12.74 (s, 1H), 10.83 (s, 1H), 8.00 (d, 1H), 7.92 (d, 2H), 7.79-7.70 (m, 4H), 7.53 (s, 1H), 6.55 (s, 1H), 5.81 (dd, 1H), 3.80-3.72 (m, 2H), 3.70 (s, 3H), 3.60 (q, 1H), 3.28 (dd, 1H), 2.38-2.28 (m, 1H),

2.21-2.11 (m, 1H), 2.06-1.89 (m, 2H), 1.73-1.63 (m, 1H).

**Beispiel 93**

4-({2-[4-(5-Chlor-2-cyanophenyl)-5-methoxy-2-oxopyridin-1(2*H*)-yl]-3-(tetrahydrofuran-3-yl)propanoyl}amino)benzoesäure (Enantiomer 2 des 1. Diastereomers)

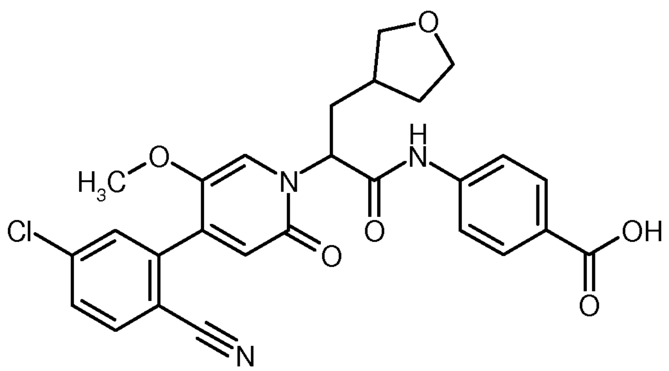

Diastereomeren- und Enantiomerentrennung von 400 mg des Gemisches aus Beispiel 90 ergab 43 mg der Titelverbindung Beispiel 93 (Enantiomer 2 des 1. Diastereomers): Chirale HPLC: $R_t$ = 10.27 min; >99% ee.

Trennmethode (SFC): Säule: Daicel Chiralpak OJ-H 5 μm 250 mm x 20 mm; Eluent: 75% Kohlendioxid, 25% Ethanol; Temperatur: 40°C; Fluss: 80 ml/min; UV-Detektion: 210 nm.

Analytik (SFC): Säule: Daicel Chiralpak AZ-H 5 μm 250 mm x 4.6 mm; Eluent: 70% Kohlendioxid, 30% Ethanol; Fluss: 3 ml/min; UV-Detektion: 220 nm.

$^1$H-NMR (400 MHz, DMSO-$d_6$): δ [ppm] = 12.78 (s, 1H), 10.85 (s, 1H), 8.00 (d, 1H), 7.92 (d, 2H), 7.80-7.70 (m, 4H), 7.56 (s, 1H), 6.55 (s, 1H), 5.77 (dd, 1H), 3.79-3.67 (m, 2H), 3.70 (s, 3H), 3.60 (q, 1H), 3.44 (dd, 1H), 2.37-2.27 (m, 1H), 2.23-2.13 (m, 1H), 2.05-1.93 (m, 2H), 1.58-1.45 (m, 1H).

**Beispiel 94**

4-({2-[4-(5-Chlor-2-cyanophenyl)-5-methoxy-2-oxopyridin-1(2*H*)-yl]-3-(tetrahydro-2*H*-pyran-4-yl)propanoyl}amino)benzoesäure (Racemat)

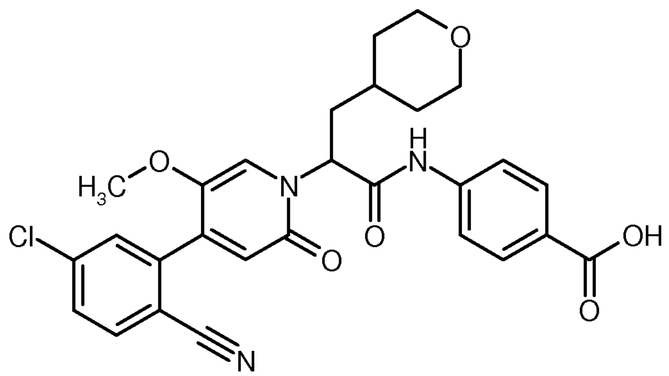

Gemäß allgemeiner Methode 4 wurden 1.07 g (1.89 mmol) ({2-[4-(5-Chlor-2-cyanophenyl)-5-methoxy-2-oxopyridin-1(2*H*)-yl]-3-(tetrahydro-2*H*-pyran-4-yl)propanoyl}amino)benzoesäureethylester (Racemat) in 24 ml Methanol/Wasser (4/1) mit 1.24 g (3.79 mmol) Cäsiumcarbonat zur Reaktion gebracht. Ausbeute: 1.24 g (73% Reinheit, 88% d. Th.)

LC/MS [Methode 1]: $R_t$ = 0.92 min; MS (ESIneg): m/z = 534 $(M-H)^-$

$^1$H-NMR (400 MHz, DMSO-$d_6$): δ [ppm] = 12.8 (s, 1H), 10.8 (s, 1H), 8.00 (d, 1H), 7.92 (d, 2H), 7.78-7.72 (m, 4H), 7.52

(s, 1H), 6.54 (s, 1H), 5.92-5.85 (m, 1H), 3.86-3.76 (m, 2H), 3.69 (s, 3H), 3.24-3.12 (m, 2H), 2.30-2.20 (m, 1H), 2.03-1.94 (m, 1H), 1.65-1.56 (m, 2H), 1.42-1.18 (m, 3H).

**Beispiel 95**

4-({2-[4-(5-Chlor-2-cyanophenyl)-5-methoxy-2-oxopyridin-1(2*H*)-yl]-3-(tetrahydro-2*H*-pyran-4-yl)propanoyl}amino)benzoesäure (Enantiomer 1)

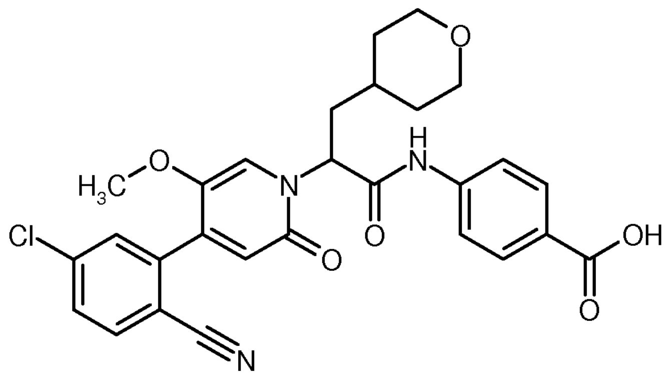

Enantiomerentrennung von 1.24 g (73% Reinheit) des Racemates aus Beispiel 94 ergab 57.6 mg der Titelverbindung Beispiel 95 (Enantiomer 1): Chirale HPLC: $R_t$ = 3.77 min; 99% ee.

Trennmethode (SFC): Säule: AD-H 5 µm 250 mm x 20 mm; Eluent: 80% Kohlendioxid, 20% 2-Propanol; Temperatur: 40°C; Fluss: 80 ml/min; Druck: 100 bar; UV-Detektion: 210 nm.

Analytik (SFC): Säule: AD-3 5 µm 250 mm x 4.6 mm; Eluent: 95-50% Kohlendioxid, 5-50% 2-Propanol; Temperatur: 40°C; Fluss: 3 ml/min; UV-Detektion: 210 nm.

**Beispiel 96**

4-({2-[4-(5-Chlor-2-cyanophenyl)-5-methoxy-2-oxopyridin-1(2*H*)-yl]-3-(tetrahydro-2*N*-pyran-4-yl)propanoyl}amino)benzoesäure (Enantiomer 2)

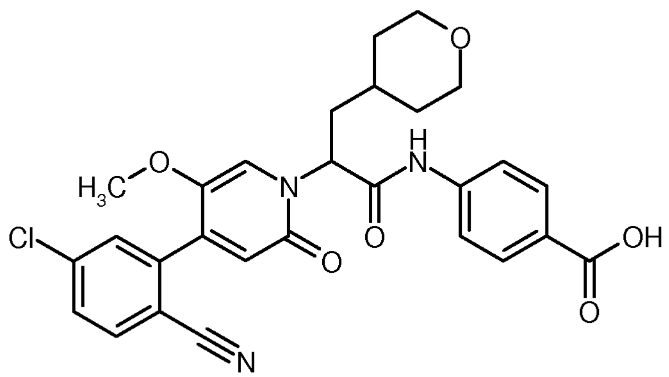

Enantiomerentrennung von 1.24 g (73% Reinheit) des Racemates aus Beispiel 94 ergab 132 mg der Titelverbindung Beispiel 96 (Enantiomer 2): Chirale HPLC: Rt = 4.17 min; 98% ee.

Trennmethode (SFC): Säule: AD-H 5 µm 250 mm x 20 mm; Eluent: 80% Kohlendioxid, 20% 2-Propanol; Temperatur: 40°C; Fluss: 80 ml/min; Druck: 100 bar; UV-Detektion: 210 nm.

Analytik (SFC): Säule: AD-3 5 µm 250 mm x 4.6 mm; Eluent: 95-50% Kohlendioxid, 5-50% 2-Propanol; Temperatur: 40°C; Fluss: 3 ml/min; UV-Detektion: 210 nm.

**Beispiel 97**

4-({2-[4-(5-Chlor-2-cyanophenyl)-5-methoxy-2-oxopyridin-1(2*H*)-yl]-3-(tetrahydro-2*H*-pyran-3-yl)propanoyl}amino)benzoesäure (Gemisch racemischer Diastereomere)

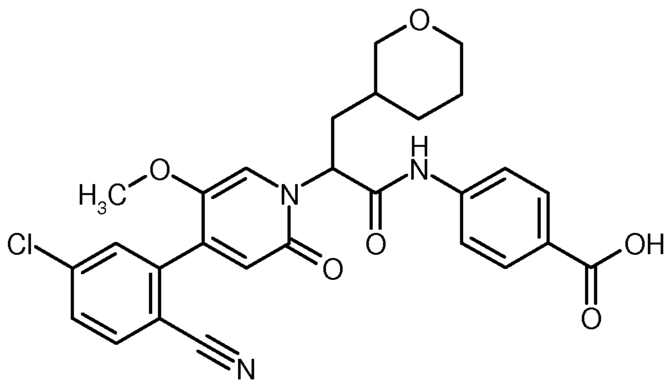

Nach der allgemeinen Methode 2 wurden 712 mg (85% Reinheit, 1.02 mmol) 4-({2-[4-(5-Chlor-2-cyanophenyl)-5-methoxy-2-oxopyridin-1(2*H*)-yl]-3-(tetrahydro-2*H*-pyran-3-yl)propanoyl}amino)-benzoesäure-*tert*.-butylester (Gemisch racemischer Diastereomere) mit TFA verseift. Ausbeute: 209 mg (38% d. Th.)
LC/MS [Methode 2]: $R_t$ = 2.81 min; MS (ESIpos): m/z = 536 $(M+H)^+$; $R_t$ = 2.88 min; MS (ESIpos): m/z = 536 $(M+H)^+$
$^1$H-NMR (400 MHz, DMSO-$d_6$): $\delta$ [ppm] = 10.86/10.83 (2x s, 1H), 8.00 (d, 1H), 7.92 (d, 2H), 7.81-7.70 (m, 4H), 7.53/7.50 (2x s, 1H), 6.56/6.55 (2x s, 1H), 5.86/5.82 (2x dd, 1H), 3.87-3.74 (m, 1H), 3.75-3.66 (m, 1H), 3.70 (s, 3H), 3.17-3.02 (m, 1H), 2.23-2.08 (m, 1H), 2.02-1.77 (m, 2H), 1.64-1.49 (m, 1H), 1.48-1.30 (m, 2H), 1.30-1.13 (m, 1H).

**Beispiel 98**

4-({2-[4-(5-Chlor-2-cyanophenyl)-5-methoxy-2-oxopyridin-1(2*H*)-yl]-3-(tetrahydro-2*H*-pyran-3-yl)propanoyl}amino)benzoesäure (Enantiomer 1 des 1. Diastereomers)

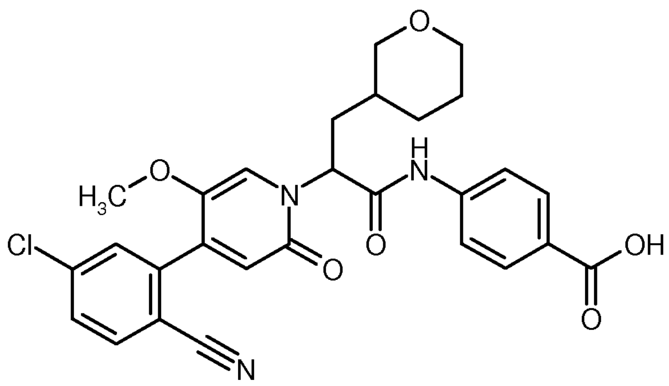

Diastereomeren- und Enantiomerentrennung von 205 mg des Gemisches aus Beispiel 97 ergab 27 mg der Titelverbindung Beispiel 98 (Enantiomer 1 des 1. Diastereomers): Chirale HPLC: $R_t$ = 9.48 min; >99% ee.

Trennmethode: Säule: Daicel Chiralpak AZ-H 5 $\mu$m 250 mm x 20 mm; Eluent: 50% iso-Hexan, 50% Ethanol + 0.2% TFA; Temperatur: 30°C; Fluss: 15 ml/min; UV-Detektion: 220 nm.

Analytik: Säule: Daicel Chiralpak AZ-H 5 $\mu$m 250 mm x 4.6 mm; Eluent: 50% iso-Hexan, 50% Ethanol + 0.2% TFA + 1% Wasser; Fluss: 3 ml/min; UV-Detektion: 220 nm.
$^1$H-NMR (400 MHz, DMSO-$d_6$): $\delta$ [ppm] = 12.78 (br. s, 1H), 10.83 (s, 1H), 8.00 (d, 1H), 7.92 (d, 2H), 7.81-7.70 (m, 4H), 7.53 (s, 1H), 6.56 (s, 1H), 5.82 (dd, 1H), 3.87-3.78 ((m, 1H), 3.76-3.66 (m, 1H), 3.70 (s, 3H), 3.30-3.21 (m, 1H), 3.12

(dd, 1H), 2.18-2.07 (m, 1H), 2.02-1.91 (m, 1H), 1.86-1.75 (m, 1H), 1.59-1.49 (m, 1H), 1.49-1.30 (m, 2H), 1.29-1.13 (m, 1H).

**Beispiel 99**

4-({2-[4-(5-Chlor-2-cyanophenyl)-5-methoxy-2-oxopyridin-1(2*H*)-yl]-3-(tetrahydro-2*H*-pyran-3-yl)propanoyl}amino)benzoesäure (Enantiomer 1 des 2. Diastereomers)

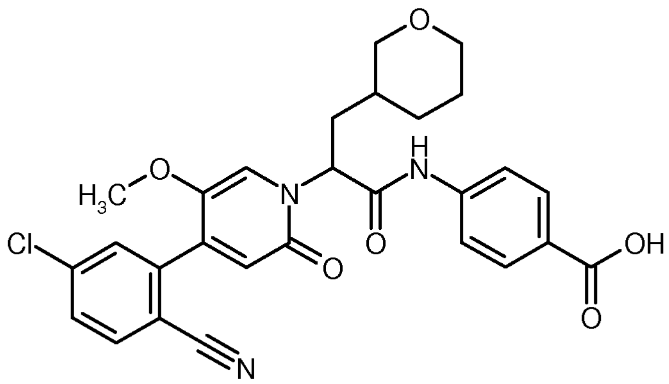

Diastereomeren- und Enantiomerentrennung von 205 mg des Gemisches aus Beispiel 97 ergab 27 mg (88% Reinheit) der Titelverbindung Beispiel 99 (Enantiomer 1 des 2. Diastereomers): Chirale HPLC: $R_t$ = 10.86 min; >99% ee.

Trennmethode: Säule: Daicel Chiralpak AZ-H 5 µm 250 mm x 20 mm; Eluent: 50% iso-Hexan, 50% Ethanol + 0.2% TFA; Temperatur: 30°C; Fluss: 15 ml/min; UV-Detektion: 220 nm.

Analytik: Säule: Daicel Chiralpak AZ-H 5 µm 250 mm x 4.6 mm; Eluent: 50% iso-Hexan, 50% Ethanol + 0.2% TFA + 1% Wasser; Fluss: 3 ml/min; UV-Detektion: 220 nm.

$^1$H-NMR (400 MHz, DMSO-$d_6$): δ [ppm] = 12.78 (br. s, 1H), 10.86 (s, 1H), 8.00 (d, 1H), 7.92 (d, 2H), 7.81-7.70 (m, 4H), 7.50 (s, 1H), 6.55 (s, 1H), 5.86 (dd, 1H), 3.81-3.74 (m, 1H), 3.74-3.65 (m, 1H), 3.70 (s, 3H), 3.33-3.22 (m, 1H), 3.07 (dd, 1H), 2.23-2.11 (m, 1H), 1.94-1.78 (m, 2H), 1.64-1.54 (m, 1H), 1.44-1.20 (m, 3H).

**Beispiel 100**

4-({2-[4-(5-Chlor-2-cyanophenyl)-5-methoxy-2-oxopyridin-1(2*H*)-yl]-3-(tetrahydro-2*H*-pyran-3-yl)propanoyl}amino)benzoesäure (Gemisch aus Enantiomer 2 des 1. Diastereomers und Enantiomer 2 des 2. Diastereomers)

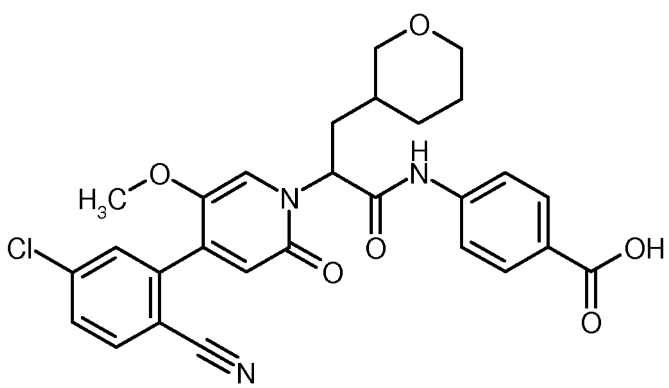

Diastereomeren- und Enantiomerentrennung von 205 mg des Gemisches aus Beispiel 97 ergab 74 mg (95% Reinheit) der Titelverbindung Beispiel 100 (Gemisch aus Enantiomer 2 des 1. Diastereomers und Enantiomer 2 des 2. Diastereomers): Chirale HPLC: $R_t$ = 14.38 min.

Trennmethode: Säule: Daicel Chiralpak AZ-H 5 µm 250 mm x 20 mm; Eluent: 50% iso-Hexan, 50% Ethanol

+ 0.2% TFA; Temperatur: 30°C; Fluss: 15 ml/min; UV-Detektion: 220 nm.

Analytik: Säule: Daicel Chiralpak AZ-H 5 µm 250 mm x 4.6 mm; Eluent: 50% iso-Hexan, 50% Ethanol + 0.2% TFA + 1% Wasser; Fluss: 3 ml/min; UV-Detektion: 220 nm.

$^1$H-NMR (400 MHz, DMSO-$d_6$): δ [ppm] = 12.78 (br. s, 1H), 10.86/10.83 (2x s, 1H), 8.00 (d, 1H), 7.92 (d, 2H), 7.81-7.70 (m, 4H), 7.53/7.50 (2x s, 1H), 6.56/6.55 (2x s, 1H), 5.86/5.82 (2x dd, 1H), 3.87-3.74 (m, 1H), 3.74-3.65 (m, 1H), 3.70 (s, 3H), 3.34-3.22 (m, 1H), 3.17-3.02 (m, 1H), 2.23-2.08 (m, 1H), 2.02-1.77 (m, 2H), 1.64-1.49 (m, 1H), 1.48-1.30 (m, 2H), 1.30-1.13 (m, 1H).

## Beispiel 101

({2-[4-(5-Chlor-2-cyanophenyl)-5-methoxy-2-oxopyridin-1(2*H*)-yl]-3-[tetrahydro-2*H*-pyran-2-yl]propanoyl}amino)benzoesäure (Gemisch racemischer Diastereomere)

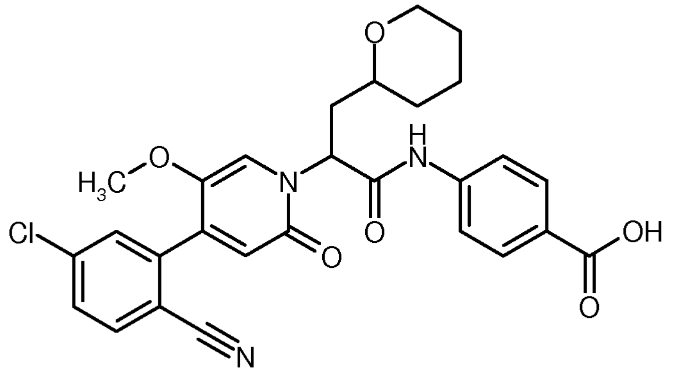

Gemäß allgemeiner Methode 4 wurden 1.24 g (2.25 mmol) ({2-[4-(5-Chlor-2-cyanophenyl)-5-methoxy-2-oxopyridin-1(2*H*)-yl]-3-[tetrahydro-2*H*-pyran-2-yl]propanoyl}amino)benzoesäure-methylester (Gemisch racemischer Diastereomere) in 30 ml Methanol/Wasser (4/1) mit 1.47 g (4.51 mmol) Cäsiumcarbonat zur Reaktion gebracht. Ausbeute: 1.17 g (85% d. Th.)

LC/MS [Methode 1]: $R_t$ = 1.00 min; MS (ESIneg): m/z = 534 $(M-H)^-$

$^1$H-NMR (400 MHz, DMSO-$d_6$): δ [ppm] = 12.8 (s, 1H), 10.8/10.7 (2x s, 1H), 8.02-7.98 (m, 1H), 7.93-7.87 (m, 2H), 7.80-7.71 (m, 4H), 7.53/7.49 (2x s, 1H), 6.52/6.51 (2x s, 1H), 5.85-5.71 (m, 1H), 3.90-3.78 (m, 1H), 3.69-3.68 (2x s, 3H), 3.29-3.15 (m, 1H), 3.13-3.05 (m, 1H), 2.42-2.11 (m, 2H), 1.78-1.70 (m, 1H), 1.67-1.56 (m, 1H), 1.47-1.35 (m, 3H), 1.30-1.19 (m, 1H).

## Beispiel 102

({2-[4-(5-Chlor-2-cyanophenyl)-5-methoxy-2-oxopyridin-1(2*H*)-yl]-3-[tetrahydro-2*H*-pyran-2-yl]propanoyl}amino)benzoesäure (Enantiomer 1 des 1. Diastereomers)

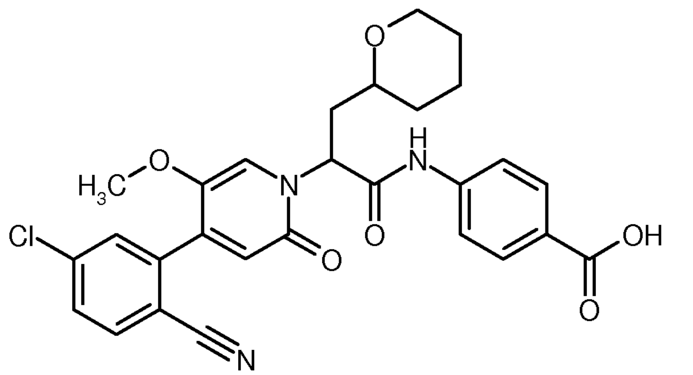

Diastereomeren- und Enantiomerentrennung von 1.17 g des Gemisches aus Beispiel 101 ergab 231 mg der Titelverbindung Beispiel 102 (Enantiomer 1 des 1. Diastereomers): Chirale HPLC: $R_t$ = 9.96 min; 87% ee.
Trennmethode (SFC): Säule: AD-H 5 μm 250 mm x 20 mm; Eluent: 80% Kohlendioxid, 20% 2-Propanol; Temperatur: 40°C; Fluss: 80 ml/min; Druck: 100 bar; UV-Detektion: 210 nm.
Analytik: Säule: AZ-H 5 μm 250 mm x 4.6 mm; Eluent: 50% iso-Hexan, 50% 2-Propanol + 0.2% TFA + 1% Wasser; Temperatur: 40°C; Fluss: 3 ml/min; UV-Detektion: 210 nm.
$^1$H-NMR (400 MHz, DMSO-$d_6$): δ [ppm] = 12.7 (s, 1H), 10.7 (s, 1H), 8.00 (d, 1H), 7.90 (d, 2H), 7.77-7.72 (m, 4H), 7.49 (s, 1H), 6.51 (s, 1H), 5.78-5.71 (m, 1H), 3.84-3.79 (m, 1H), 3.69 (s, 3H), 3.23-3.15 (m, 1H), 3.13-3.05 (m, 1H), 2.42-2.32 (m, 1H), 2.26-2.18 (m, 1H), 1.78-1.71 (m, 1H), 1.62-1.56 (m, 1H), 1.46-1.37 (m, 3H), 1.30-1.20 (m, 1H).

## Beispiel 103

({2-[4-(5-Chlor-2-cyanophenyl)-5-methoxy-2-oxopyridin-1(2*H*)-yl]-3-[tetrahydro-2*H*-pyran-2-yl]propanoyl}amino)benzoesäure (Enantiomer 1 des 2. Diastereomers)

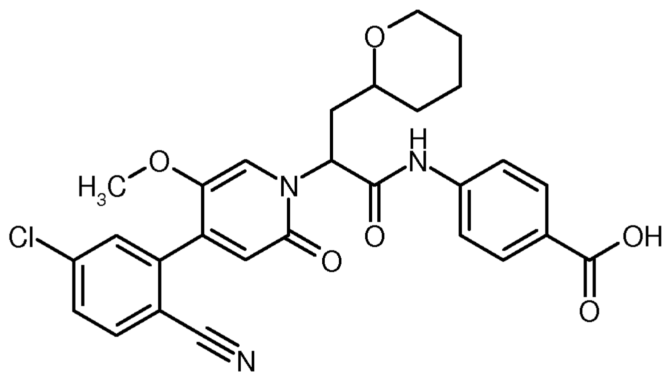

Diastereomeren- und Enantiomerentrennung von 1.17 g des Gemisches aus Beispiel 101 ergab 54 mg der Titelverbindung Beispiel 103 (Enantiomer 1 des 2. Diastereomers): Chirale HPLC: $R_t$ = 15.34 min; 99% ee.
Trennmethode (SFC): Säule: AD-H 5 μm 250 mm x 20 mm; Eluent: 80% Kohlendioxid, 20% 2-Propanol; Temperatur: 40°C; Fluss: 80 ml/min; Druck: 100 bar; UV-Detektion: 210 nm.
Analytik: Säule: AZ-H 5 μm 250 mm x 4.6 mm; Eluent: 50% iso-Hexan, 50% 2-Propanol + 0.2% TFA + 1% Wasser; Temperatur: 40°C; Fluss: 3 ml/min; UV-Detektion: 210 nm.
$^1$H-NMR (400 MHz, DMSO-$d_6$): δ [ppm] = 12.8 (s, 1H), 10.7 (s, 1H), 7.99 (d, 1H), 7.90 (d, 2H), 7.77 (d, 2H), 7.74-7.71 (m, 2H), 7.52 (s, 1H), 6.52 (s, 1H), 5.82 (t, 1H), 3.90-3.84 (m, 1H), 3.68 (s, 3H), 3.28-3.22 (m, 2H), 2.34-2.27 (m, 1H), 2.19-2.10 (m, 1H), 1.78-1.73 (m, 1H), 1.67-1.60 (m, 1H), 1.47-1.36 (m, 3H), 1.33-1.23 (m, 1H).

## Beispiel 104

({2-[4-(5-Chlor-2-cyanophenyl)-5-methoxy-2-oxopyridin-1(2*H*)-yl]-3-[tetrahydro-2*H*-pyran-2-yl]propanoyl}amino)benzoesäure (Enantiomer 2 des 2. Diastereomers)

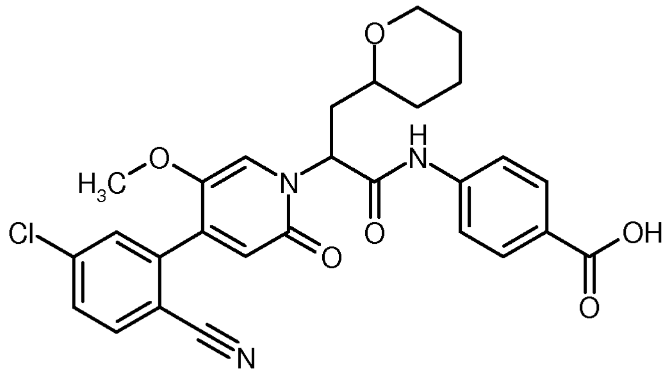

Diastereomeren- und Enantiomerentrennung von 1.17 g des Gemisches aus Beispiel 101 ergab 91 mg der Titelverbindung Beispiel 104 (Enantiomer 2 des 2. Diastereomers): Chirale HPLC: $R_t$ = 20.83 min; 99% ee.

Trennmethode (SFC): Säule: AD-H 5 μm 250 mm x 20 mm; Eluent: 80% Kohlendioxid, 20% 2-Propanol; Temperatur: 40°C; Fluss: 80 ml/min; Druck: 100 bar; UV-Detektion: 210 nm.

Analytik: Säule: AZ-H 5 μm 250 mm x 4.6 mm; Eluent: 50% iso-Hexan, 50% 2-Propanol + 0.2% TFA + 1% Wasser; Temperatur: 40°C; Fluss: 3 ml/min; UV-Detektion: 210 nm.

$^1$H-NMR (400 MHz, DMSO-$d_6$): δ [ppm] = 12.8 (s, 1H), 10.7 (s, 1H), 7.99 (d, 1H), 7.90 (d, 2H), 7.77 (d, 2H), 7.74-7.71 (m, 2H), 7.52 (s, 1H), 6.52 (s, 1H), 5.82 (t, 1H), 3.90-3.84 (m, 1H), 3.68 (s, 3H), 3.28-3.22 (m, 2H), 2.34-2.27 (m, 1H), 2.19-2.10 (m, 1H), 1.78-1.73 (m, 1H), 1.67-1.60 (m, 1H), 1.47-1.36 (m, 3H), 1.33-1.23 (m, 1H).

## Beispiel 105

({2-[4-(5-Chlor-2-cyanophenyl)-5-methoxy-2-oxopyridin-1(2*H*)-yl]-3-[tetrahydro-2*H*-pyran-2-yl]propanoyl}amino)benzoesäure (Enantiomer 2 des 1. Diastereomers)

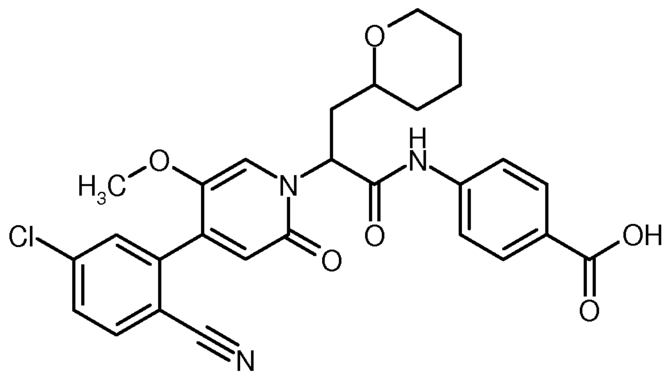

Diastereomeren- und Enantiomerentrennung von 1.17 g des Gemisches aus Beispiel 101 ergab 183 mg der Titelverbindung Beispiel 105 (Enantiomer 2 des 1. Diastereomers): Chirale HPLC: $R_t$ = 27.14 min; 99% ee.

Trennmethode (SFC): Säule: AD-H 5 μm 250 mm x 20 mm; Eluent: 80% Kohlendioxid, 20% 2-Propanol; Temperatur: 40°C; Fluss: 80 ml/min; Druck: 100 bar; UV-Detektion: 210 nm.

Analytik: Säule: AZ-H 5 μm 250 mm x 4.6 mm; Eluent: 50% iso-Hexan, 50% 2-Propanol + 0.2% TFA + 1% Wasser; Temperatur: 40°C; Fluss: 3 ml/min; UV-Detektion: 210 nm.

$^1$H-NMR (400 MHz, DMSO-$d_6$): δ [ppm] = 12.7 (s, 1H), 10.7 (s, 1H), 8.00 (d, 1H), 7.90 (d, 2H), 7.77-7.72 (m, 4H), 7.49 (s, 1H), 6.51 (s, 1H), 5.78-5.71 (m, 1H), 3.84-3.79 (m, 1H), 3.69 (s, 3H), 3.23-3.15 (m, 1H), 3.13-3.05 (m, 1H), 2.42-2.32 (m, 1H), 2.26-2.18 (m, 1H), 1.78-1.71 (m, 1H), 1.62-1.56 (m, 1H), 1.46-1.37 (m, 3H), 1.30-1.20 (m, 1H).

**Beispiel 106**

4-({2-[4-(5-Chlor-2-cyanophenyl)-5-methoxy-2-oxopyridin-1(2*H*)-yl]-3-(1,4-dioxan-2-yl)propanoyl}amino)benzoesäure (Gemisch racemischer Diastereomere)

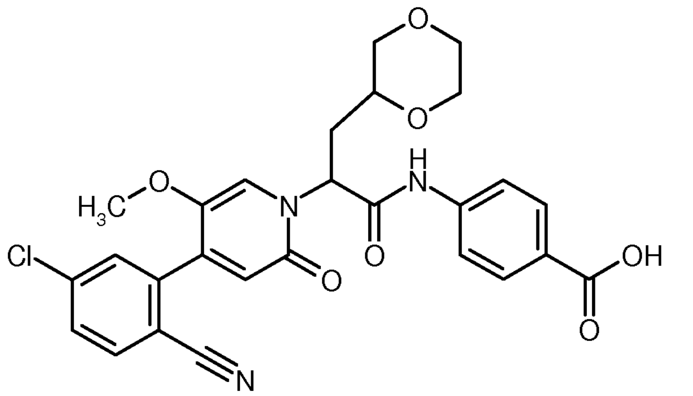

Nach der allgemeinen Methode 2 wurden 103 mg (0.17 mmol) 4-({2-[4-(5-Chlor-2-cyanophenyl)-5-methoxy-2-oxopyridin-1(2*H*)-yl]-3-(1,4-dioxan-2-yl)propanoyl}amino)benzoesäure-*tert*.-butylester (Gemisch racemischer Diastereomere) mit TFA verseift. Ausbeute: 60 mg (64% d. Th.)
LC/MS [Methode 8]: $R_t$ = 1.12 min; MS (ESIneg): m/z = 536 $(M-H)^-$; $R_t$ = 1.13 min; MS (ESIneg): m/z = 536 $(M-H)^-$
$^1$H-NMR (400 MHz, DMSO-$d_6$): $\delta$ [ppm] = 12.77 (br. s, 1H), 10.75/10.73 (2x s, 1H), 8.03-7.97 (m, 1H), 7.94-7.86 (m, 2H), 7.80-7.70 (m, 4H), 7.53/7.48 (2x s, 1H), 6.54/6.53 (2x s, 1H), 5.83-5.73 (m, 1H), 3.79-3.65 (m, 2H), 3.70/3.68 (2x s, 3H), 3.65-3.56 (m, 1H), 3.55-3.39 (m, 2H), 3.30-3.20 (m, 2H), 2.41-2.09 (m. 2H).

**Beispiel 107**

4-({2-[4-(5-Chlor-2-cyanophenyl)-5-methoxy-2-oxopyridin-1(2*H*)-yl]-4-fluorbutanoyl}amino)-benzoesäure (Racemat)

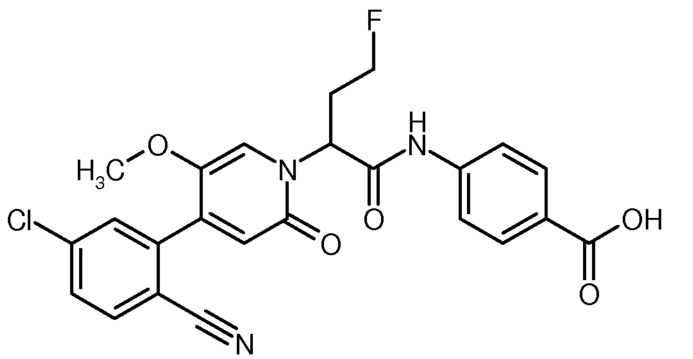

Gemäß allgemeiner Methode 2 wurden 114 mg (211 µmol) 4-({2-[4-(5-Chlor-2-cyanophenyl)-5-methoxy-2-oxopyridin-1(2*H*)-yl]-4-fluorbutanoyllamino)-benzoesäure-*tert.*-butylester (Racemat) mit 325 µl (4.22 mmol) TFA umgesetzt. Das Rohprodukt wurde mittels präparativer HPLC [Säule: Chromatorex C18, 10 µm, 125 mm x 30 mm, Eluent: Acetonitril/Wasser-Gradient (0 bis 3 min 15% Acetonitril, bis 35 min 90% Acetonitril und weitere 3 min 90% Acetonitril)] gereinigt. Ausbeute: 45 mg (44% d. Th.)
LC/MS [Methode 1]: $R_t$ = 0.91 min; MS (ESIneg): m/z = 482 $(M-H)^-$
$^1$H-NMR (400 MHz, DMSO-$d_6$): $\delta$ [ppm] = 12.8 (br. s, 1H), 10.8 (s, 1H), 8.00 (d, 1H), 7.92 (d, 2H), 7.79-7.72 (m, 4H), 7.52 (s, 1H), 6.55 (s, 1H), 5.85 (t, 1H), 4.67-4.49 (m, 1H), 4.48-4.29 (m, 1H), 3.69 (s, 3H), 2.69-2.55 (m, 2H).

**Beispiel 108**

4-({2-[4-(5-Chlor-2-cyanophenyl)-5-methoxy-2-oxopyridin-1(2*H*)-yl]-4,4-difluorbutanoyl}amino)-benzoesäure (Racemat)

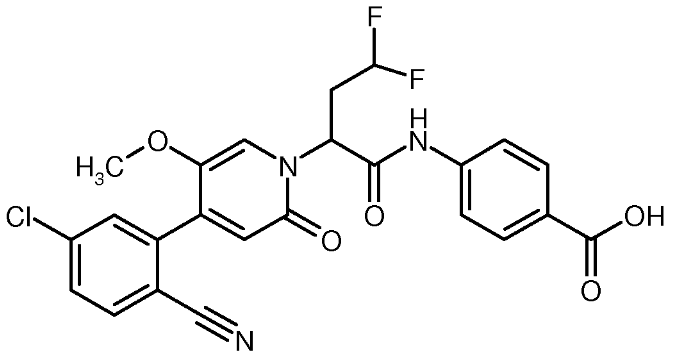

Gemäß allgemeiner Methode 4 wurden 80 mg (151 μmol) 4-({2-[4-(5-Chlor-2-cyanophenyl)-5-methoxy-2-oxopyridin-1(2*H*)-yl]-4,4-difluorbutanoyl} amino)benzoesäureethylester (Racemat) in 2 ml Methanol/Wasser (4/1) mit 326 mg (302 μmol) Cäsiumcarbonat zur Reaktion gebracht. Das Rohprodukt wurde mittels präparativer HPLC [Säule: Chromatorex C18, 10 μm, 125 mm x 30 mm, Eluent: Acetonitril/0.05% Ameisensäure-Gradient (0 bis 3 min 10% Acetonitril, bis 35 min 90% Acetonitril und weitere 3 min 90% Acetonitril)] gereinigt. Ausbeute: 18 mg (23% d. Th.)
LC/MS [Methode 1]: $R_t$ = 0.92 min; MS (ESIneg): m/z = 500 $(M-H)^-$
$^1$H-NMR (400 MHz, DMSO-$d_6$): δ [ppm] = 12.8 (br. s, 1H), 10.7 (s, 1H), 8.00 (d, 1H), 7.91 (d, 2H), 7.76-7.70 (m, 4H), 7.56 (s, 1H), 6.55 (s, 1H), 6.15 (tt, 1H), 5.91 (dd, 1H), 3.69 (s, 3H), 2.99-2.75 (m, 2H).

**Beispiel 109**

4-({2-[4-(5-Chlor-2-cyanophenyl)-5-methoxy-2-oxopyridin-1(2*H*)-yl]-4,4,4-trifluorbutanoyl}-amino)benzoesäure (Racemat)

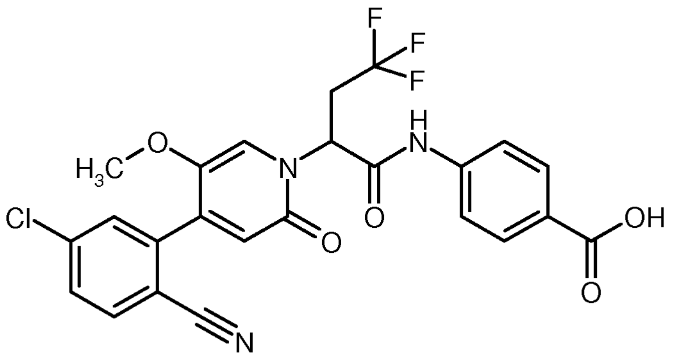

Gemäß allgemeiner Methode 4 wurden 21 mg (38 μmol) 4-({2-[4-(5-Chlor-2-cyanophenyl)-5-methoxy-2-oxopyridin-1(2*H*)-yl]-4,4,4-trifluorbutanoyl} amino)benzoesäureethylester (Racemat) in 0.75 ml Methanol/Wasser (4/1) mit 25 mg (77 μmol) Cäsiumcarbonat zur Reaktion gebracht. Das Rohprodukt wurde mittels präparativer HPLC [Säule: Chromatorex C18, 10 μm, 125 mm x 30 mm, Eluent: Acetonitril/0.05% Ameisensäure-Gradient (0 bis 3 min 10% Acetonitril, bis 35 min 90% Acetonitril und weitere 3 min 90% Acetonitril)] gereinigt. Ausbeute: 9 mg (46% d. Th.)
LC/MS [Methode 1]: $R_t$ = 0.98 min; MS (ESIneg): m/z = 518 $(M-H)^-$
$^1$H-NMR (400 MHz, DMSO-$d_6$): δ [ppm] = 12.8 (s, 1H), 10.8 (s, 1H), 8.00 (d, 1H), 7.92 (d, 2H), 7.75-7.72 (m, 4H), 7.63 (s, 1H), 6.56 (s, 1H), 6.11-6.05 (m, 1H), 3.69 (s, 3H), 3.56-3.25 (m, 2H).

**Beispiel 110**

4-({2-[4-(5-Chlor-2-cyanophenyl)-5-methoxy-2-oxopyridin-1(2*H*)-yl]-4-fluorpentanoyl}amino)-benzoesäure (Gemisch racemischer Diastereomere)

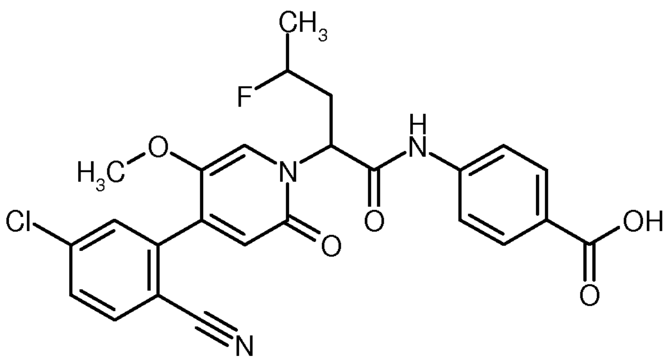

Nach der allgemeinen Methode 3 wurden 170 mg (91% Reinheit, 0.29 mmol) 4-({2-[4-(5-Chlor-2-cyanophenyl)-5-methoxy-2-oxopyridin-1(2*H*)-yl]-4-fluorpentanoyl}amino)benzoesäureethylester (Gemisch racemischer Diastereomere) mit Lithiumhydroxid verseift. Ausbeute: 67 mg (45% d. Th.)
LC/MS [Methode 8]: $R_t$ = 1.19 min; MS (ESIpos): m/z = 498 $(M+H)^+$
$^1$H-NMR (400 MHz, DMSO-$d_6$): δ [ppm] = 10.83 (s, 1H), 8.00 (d, 1H), 7.95-7.87 (m, 2H), 7.81-7.70 (m, 4H), 7.55/7.51 (2x s, 1H), 6.54 (s, 1H), 5.94-5.83 (m, 1H), 4.84-4.45 (2x dm, 2H), 3.69 (s, 3H), 2.46-2.41 (m, 1H), 1.39/1.33 (2x t, 3H).

**Beispiel 111**

4-({2-[4-(5-Chlor-2-cyanophenyl)-5-methoxy-2-oxopyridin-1(2*H*)-yl]-5,5,5-trifluor-4-methylpentanoyl}amino)benzoesäure (Gemisch racemischer Diastereomere)

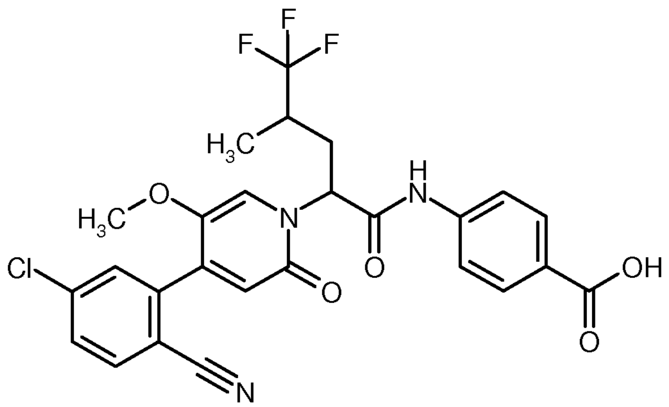

Nach der allgemeinen Methode 2 wurden 48 mg (0.08 mmol) 4-({2-[4-(5-Chlor-2-cyanophenyl)-5-methoxy-2-oxopyridin-1(2*H*)-yl]-5,5,5-trifluor-4-methylpentanoyl}amino)benzoesäure-*tert.*-butylester (Gemisch racemischer Diastereomere) mit TFA verseift. Ausbeute: 24 mg (56% d. Th.)
LC/MS [Methode 1]: $R_t$ = 1.07 min; MS (ESIpos): m/z = 548 $(M+H)^+$
$^1$H-NMR (400 MHz, DMSO-$d_6$): δ [ppm] = 10.89/10.78 (2x s, 1H), 8.00 (d, 1H), 7.96-7.88 (m, 2H), 7.81-7.70 (m, 4H), 7.53 (s, 1H), 6.59/6.58 (2x s, 1H), 5.91/5.78 (2x dd, 1H), 3.69/3.67 (2x s, 3H), 2.72-2.62 (m, 1H), 2.44-2.31 (m, 1H), 2.22-1.98 (m, 1H), 1.19/1.14 (2x d, 3H).

**Beispiel 112**

4-({2-[4-(5-Chlor-2-cyanophenyl)-5-methoxy-2-oxopyridin-1(2*H*)-yl]-4,4-dimethylpentanoyl}-amino)benzoesäure (Racemat)

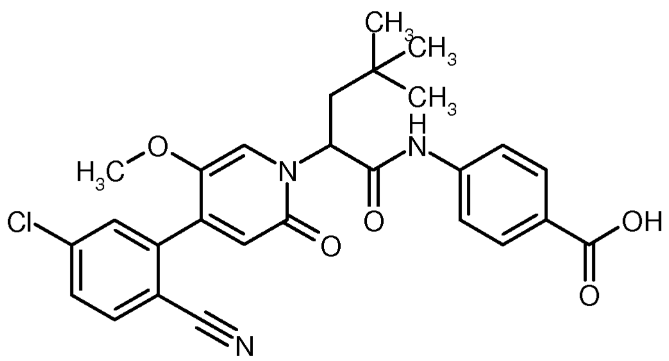

Gemäß allgemeiner Methode 2 wurden 99 mg (176 μmol) 4-({2-[4-(5-Chlor-2-cyanophenyl)-5-methoxy-2-oxopyridin-1(2*H*)-yl]-4,4-dimethylpentanoyl}amino)benzoesäure-*tert*.-butylester (Racemat) mit 270 μl (3.51 μmol) TFA umgesetzt. Das Rohprodukt wurde mittels präparativer HPLC [Säule: Chromatorex C18, 10 μm, 125 mm x 30 mm, Eluent: Acetonitril/Wasser-Gradient (0 bis 3 min 15% Acetonitril, bis 35 min 90% Acetonitril und weitere 3 min 90% Acetonitril)] gereinigt. Ausbeute: 57 mg (64% d. Th.)
LC/MS [Methode 1]: $R_t$ = 1.07 min; MS (ESIneg): m/z = 506 $(M-H)^-$
$^1$H-NMR (400 MHz, DMSO-$d_6$): δ [ppm] = 12.8 (s, 1H), 10.9 (s, 1H), 7.99 (d, 1H), 7.91 (d, 2H), 7.77 (d, 2H), 7.74-7.71 (m, 2H), 7.60 (s, 1H), 6.54 (s, 1H), 5.99 (dd, 1H), 3.70 (s, 3H), 2.12 (dd, 1H), 2.03 (dd, 1H), 0.92 (s, 9H).

**Beispiel 113**

4-({2-[4-(5-Chlor-2-cyanophenyl)-5-methoxy-2-oxopyridin-1(2*H*)-yl]-3-[2,2-difluorcyclopropyl]-propanoyl}amino)benzoesäure (Gemisch racemischer Diastereomere)

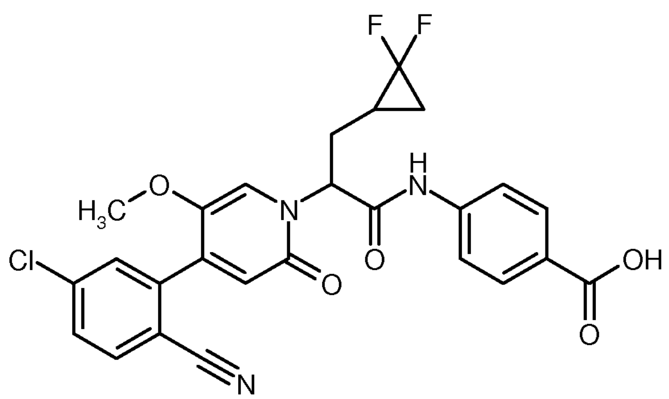

Gemäß allgemeiner Methode 2 wurden 74.0 mg (127 μmol) 4-({2-[4-(5-Chlor-2-cyanophenyl)-5-methoxy-2-oxopyridin-1(2*H*)-yl]-3-[2,2-difluorcyclopropyl]propanoyl}amino)benzoesäure-*tert*.-butylester (Gemisch racemischer Diastereomere) mit 195 μl (2.53 mmol) TFA umgesetzt. Das Rohprodukt wurde mittels präparativer HPLC [Säule: Chromatorex C18, 10 μm, 125 mm x 30 mm, Eluent: Acetonitril/Wasser-Gradient (0 bis 3 min 15% Acetonitril, bis 35 min 90% Acetonitril und weitere 3 min 90% Acetonitril)] gereinigt. Ausbeute: 45 mg (52% d. Th.)
LC/MS [Methode 1]: $R_t$ = 0.99 min; MS (ESIneg): m/z = 526 $(M-H)^-$
$^1$H-NMR (400 MHz, DMSO-$d_6$): δ [ppm] = 12.8 (s, 1H), 10.8/10.7 (2x s, 1H), 8.00 (d, 1H), 7.93-7.90 (m, 2H), 7.77-7.71 (m, 4H), 7.53 (s, 1H), 6.56 (s, 1H), 5.80-5.70 (m, 1H), 3.70/3.69 (2x s, 3H), 2.60-2.37 (m, 1H), 2.30-2.09 (2x m, 1H),

1.70-1.49 (m, 2H), 1.32-1.05 (2x m, 1H).

**Beispiel 114**

4-({2-[4-(5-Chlor-2-cyanophenyl)-5-methoxy-2-oxopyridin-1(2*H*)-yl]-3-[2,2-difluorcyclopropyl]-propanoyl}amino)benzoesäure (Enantiomer 1 des 1. Diastereomers und Enantiomer 1 des 2. Diastereomers)

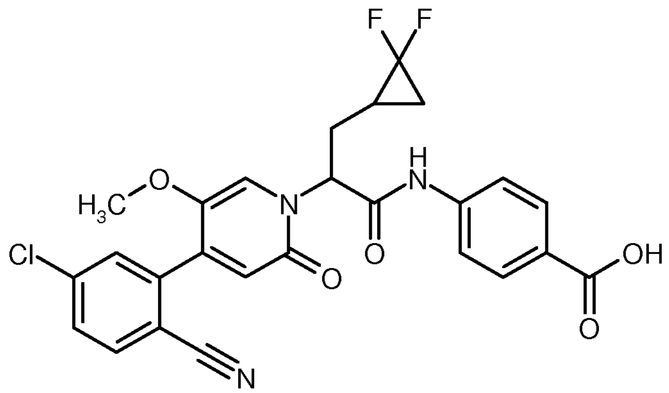

Enantiomerentrennung von 44 mg des Gemisches racemischer Diastereomere aus Beispiel 113 ergab 15 mg der Titelverbindung Beispiel 114: Chirale HPLC: $R_t$ = 5.42/5.81 min; 99% ee, Diastereomerenverhältnis 1:1.

Trennmethode: Säule: AZ-H 5 µm 250 mm x 20 mm; Eluent: 50% iso-Hexan, 50% 2-Propanol + 0.2% TFA +1% Wasser; Temperatur: 30°C; Fluss: 20 ml/min; UV-Detektion: 220 nm.

Analytik: Säule: AZ-H 5 µm 250 mm x 4.6 mm; Eluent: 50% iso-Hexan, 50% 2-Propanol + 0.2% TFA + 1% Wasser; Temperatur: 30°C; Fluss: 1 ml/min; UV-Detektion: 220 nm.

$^1$H-NMR (400 MHz, DMSO-$d_6$): δ [ppm] = 12.8 (s, 1H), 10.8/10.7 (2x s, 1H), 8.00 (d, 1H), 7.93-7.90 (m, 2H), 7.77-7.71 (m, 4H), 7.53 (s, 1H), 6.56 (s, 1H), 5.80-5.70 (m, 1H), 3.70/3.69 (2x s, 3H), 2.60-2.37 (m, 1H), 2.30-2.09 (2x m, 1H), 1.70-1.49 (m, 2H), 1.32-1.05 (2x m, 1H).

**Beispiel 115**

4-({2-[4-(5-Chlor-2-cyanophenyl)-5-methoxy-2-oxopyridin-1(2*H*)-yl]-3- [2,2-difluorcyclopropyl]-propanoyl}amino)benzoesäure (Enantiomer 2 des 1. Diastereomers und Enantiomer 2 des 2. Diastereomers)

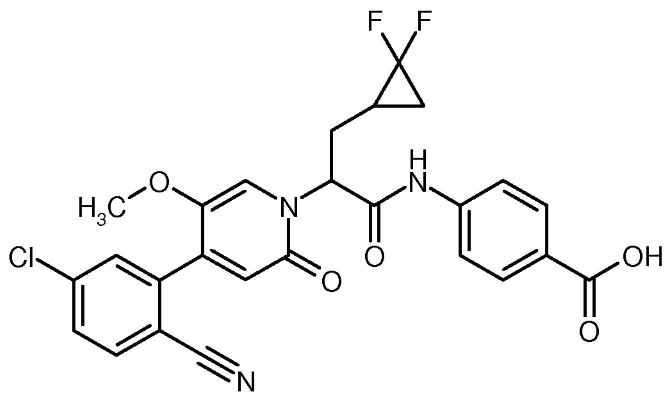

Enantiomerentrennung von 44 mg des Gemisches racemischer Diastereomereaus Beispiel 113 ergab 15 mg der Titelverbindung Beispiel 115: Chirale HPLC: $R_t$ = 8.75/9.79 min; 99% ee, Diastereomerenverhältnis 1:1.

Trennmethode: Säule: AZ-H 5 µm 250 mm x 20 mm; Eluent: 50% iso-Hexan, 50% 2-Propanol + 0.2% TFA +1% Wasser; Temperatur: 30°C; Fluss: 20 ml/min; UV-Detektion: 220 nm.

Analytik: Säule: AZ-H 5 μm 250 mm x 4.6 mm; Eluent: 50% iso-Hexan, 50% 2-Propanol + 0.2% TFA + 1% Wasser; Temperatur: 30°C; Fluss: 1 ml/min; UV-Detektion: 220 nm.
$^1$H-NMR (400 MHz, DMSO-$d_6$): δ [ppm] = 12.8 (s, 1H), 10.8/10.7 (2x s, 1H), 8.00 (d, 1H), 7.93-7.90 (m, 2H), 7.77-7.71 (m, 4H), 7.53 (s, 1H), 6.56 (s, 1H), 5.80-5.70 (m, 1H), 3.70/3.69 (2x s, 3H), 2.60-2.37 (m, 1H), 2.30-2.09 (2x m, 1H), 1.70-1.49 (m, 2H), 1.32-1.05 (2x m, 1H).

**Beispiel 116**

4-({2-[4-(5-Chlor-2-cyanophenyl)-5-methoxy-2-oxopyridin-1(2*H*)-yl]-3-(1-methylcyclopropyl)propanoyl}amino)benzoesäure (Racemat)

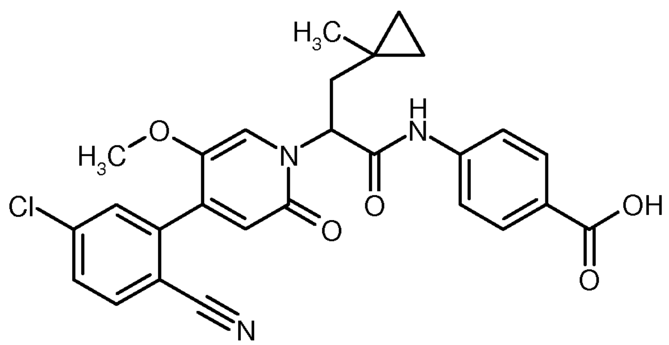

Gemäß allgemeiner Methode 2 wurden 100 mg (178 μmol) 4-({2-[4-(5-Chlor-2-cyanophenyl)-5-methoxy-2-oxopyridin-1(2*H*)-yl]-3-(1-methylcyclopropyl)propanoyl}amino)benzocsäure-*tert*.-butylester (Racemat) mit 274 μl (3.56 mmol) TFA umgesetzt. Das Reaktionsgemisch wurde mittels präparativer HPLC [Säule: Chromatorex C18, 10 μm, 125 mm x 30 mm, Eluent: Acetonitril/Wasser-Gradient (0 bis 3 min 15% Acetonitril, bis 35 min 90% Acetonitril und weitere 3 min 90% Acetonitril)] gereinigt. Ausbeute: 107 mg (27% d. Th.)
LC/MS [Methode 1]: $R_t$ = 1.05 min; MS (ESIneg): m/z = 504 $(M-H)^-$
$^1$H-NMR (400 MHz, DMSO-$d_6$): δ [ppm] = 12.8 (s, 1H), 10.8 (s, 1H), 8.00 (d, 1H), 7.91 (d, 2H), 7.79-7.72 (m, 4H), 7.55 (s, 1H), 6.53 (s, 1H), 5.97 (dd, 1H), 3.68 (s, 3H), 2.20 (dd, 1H), 2.04 (dd, 1H), 1.08 (s, 3H), 0.35-0.25 (m, 2H), 0.21-0.12 (m, 2H).

**Beispiel 117**

4-({2-[4-(5-Chlor-2-cyanophenyl)-5-methoxy-2-oxopyridin-1(2*H*)-yl]-3-cyclobutylpropanoyl}-amino)benzoesäure (Racemat)

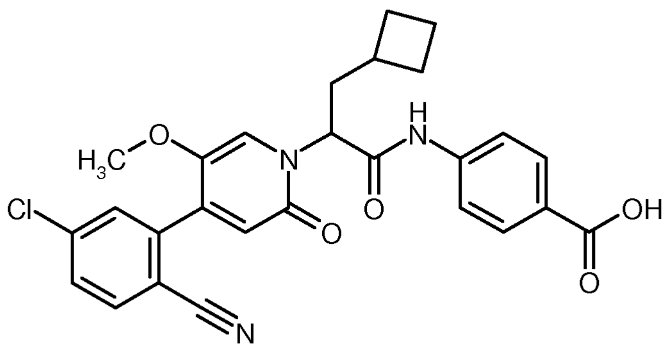

Nach der allgemeinen Methode 2 wurden 64 mg (86% Reinheit, 0.10 mmol) 4-({2-[4-(5-Chlor-2-cyanophenyl)-5-methoxy-2-oxopyridin-1(2*H*)-yl]-3-cyclobutylpropanoyl}amino)benzoesäure-*tert*.-butylester (Racemat) mit TFA verseift. Ausbeute: 33 mg (67% d. Th.)
LC/MS [Methode 1]: $R_t$ = 1.07 min; MS (ESIpos): m/z = 506 $(M+H)^+$

$^1$H-NMR (400 MHz, DMSO-$d_6$): δ [ppm] = 12.77 (s, 1H), 10.80 (s, 1H), 8.00 (s, 1H), 7.91 (d, 2H), 7.80-7.70 (m, 4H), 7.51 (s, 1H), 6.51 (s, 1H), 5.75-5.65 (m, 1H), 3.69 (s, 3H), 2.35-2.16 (m, 3H), 2.01-1.88 (m, 2H), 1.85-1.60 (m, 4H).

**Beispiel 118**

4-({[4-(5-Chlor-2-cyanophenyl)-5-methoxy-2-oxopyridin-1(2*H*)-yl](ethoxy)acetyl}amino)-benzoesäure (Racemat)

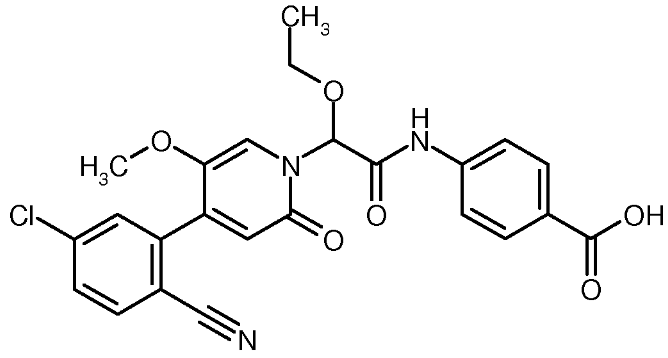

Nach der allgemeinen Methode 2 wurden 260 mg (0.48 mmol) 4-({[4-(5-Chlor-2-cyanophenyl)-5-methoxy-2-oxopyridin-1(2*H*)-yl](ethoxy)acetyl}amino)benzoesäure-*tert*.-butylester (Racemat) mit TFA verseift. Ausbeute: 184 mg (79% d. Th.)
LC/MS [Methode 1]: $R_t$ = 0.96 min; MS (ESIpos): m/z = 482 $(M+H)^+$
$^1$H-NMR (400 MHz, DMSO-$d_6$): δ [ppm] = 12.81 (br. s, 1H), 10.72 (s, 1H), 8.01 (d, 1H), 7.94 (d, 2H), 7.84-7.71 (m, 4H), 7.35 (s, 1H), 6.57 (s, 1H), 6.41 (s, 1H), 3.82-3.73 (m, 1H), 3.73-3.62 (m, 1H), 3.67 (s, 3H), 1.27 (t, 3H).

**Beispiel 119**

4-({[4-(5-Chlor-2-cyanophenyl)-5-methoxy-2-oxopyridin-1(2*H*)-yl]acetyl}amino)benzoesäure

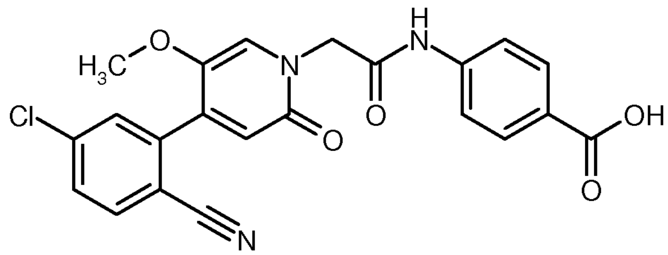

Gemäß allgemeiner Methode 2 wurden 49.0 mg (99.0 μmol) 4-({[4-(5-Chlor-2-cyanophenyl)-5-methoxy-2-oxopyridin-1(2*H*)-yl]acetyllanüno)benzoesäure-*tert*.-butylester mit 153 μl (1.98 mmol) TFA umgesetzt. Ausbeute: 20 mg (44% d. Th.)
LC/MS [Methode 1]: $R_t$ = 0.81 min; MS (ESIneg): m/z = 436 $(M-H)^-$
$^1$H-NMR (400 MHz, DMSO-$d_6$): δ [ppm] = 12.7 (s, 1H), 10.7 (s, 1H), 8.01 (d, 1H), 7.92 (d, 2H), 7.75-7.71 (m, 4H), 7.60 (s, 1H), 6.52 (s, 1H), 4.82 (s, 2H), 3.64 (s, 3H).

**Beispiel 120**

4-[(2-{4-[5-Chlor-2-(trifluormethoxy)phenyl]-5-methoxy-2-oxopyridin-1(2*H*)-yl}propanoyl)-amino]benzoesäure (Racemat)

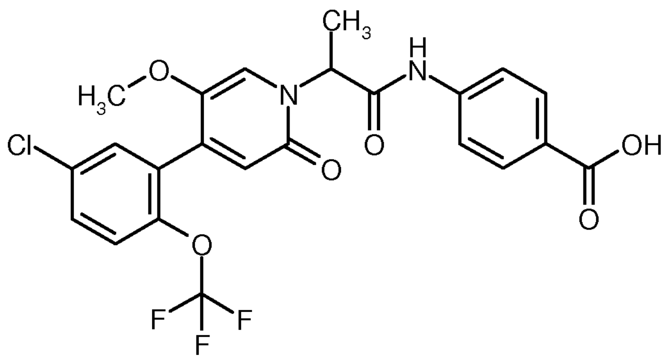

Nach der allgemeinen Methode 2 wurden 155 mg (0.23 mmol) 4-[(2-{4-[5-Chlor-2-(trifluormethoxy)phenyl] -5-methoxy-2-oxopyridin-1(2*H*)-yl}propanoyl)amino]benzoesäure-*tert*.-butylester mit TFA verseift. Ausbeute: 67 mg (94% Reinheit, 54% d. Th.)
LC/MS [Methode 1]: $R_t$ = 1.03 min; MS (ESIpos): m/z = 511 $(M+H)^+$
$^1$H-NMR (400 MHz, DMSO-$d_6$): δ [ppm] = 12.76 (br. s, 1H), 10.68 (s, 1H), 7.91 (d, 2H), 7.73 (d, 2H), 7.65 (dd, 1H), 7.59 (d, 1H), 7.51 (dd, 1H), 7.40 (s, 1H), 6.41 (s, 1H), 5.59 (q, 1H), 3.65 (s, 3H), 1.72 (d, 3H).

**Beispiel 121**

4-({2-[4-(2-Brom-5-chlorphenyl)-5-methoxy-2-oxopyridin-1(2*H*)-yl]propanoyl}amino)-benzoesäure (Racemat)

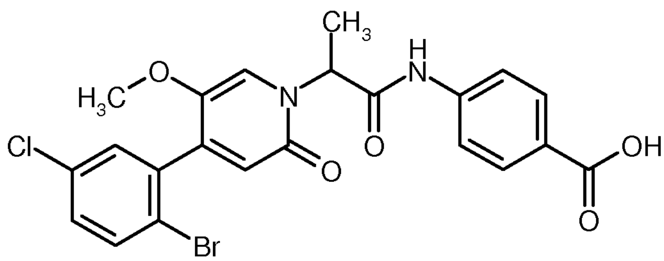

Nach der allgemeinen Methode 2 wurden 72 mg (73% Reinheit, 0.09 mmol) 4-({2-[4-(2-Brom-5-chlorphenyl)-5-methoxy-2-oxopyridin-1(2*H*)-yl]propanoyl}amino)benzoesäure-*tert*.-butylester (Racemat) mit TFA verseift. Ausbeute: 20 mg (42% d. Th.)
LC/MS [Methode 1]: $R_t$ = 0.98 min; MS (ESIpos): m/z = 505 $(M+H)^+$
$^1$H-NMR (400 MHz, DMSO-$d_6$): δ [ppm] = 12.76 (s, 1H), 10.70 (s, 1H), 7.91 (d, 2H), 7.78-7.69 (m, 3H), 7.51-7.41 (m, 2H), 7.40 (s, 1H), 6.32 (s, 1H), 5.61 (q, 1H), 3.66 (s, 3H), 1.72 (d, 3H).

**Beispiel 122**

4-([2-[4-(5-Chlor-2-methylphenyl)-5-methoxy-2-oxopyridin-1(2*H*)-yl]propanoyl}amino)-benzoesäure (Racemat)

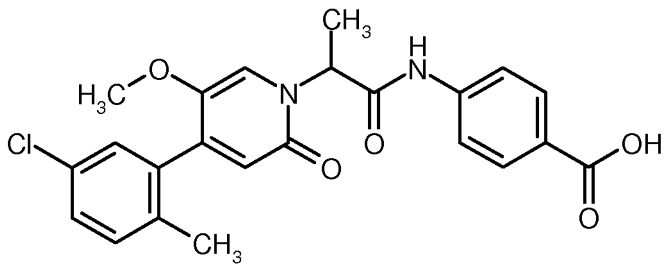

Nach der allgemeinen Methode 2 wurden 105 mg (91% Reinheit, 0.19 mmol) 4-({2-[4-(5-Chlor-2-methylphenyl)-5-methoxy-2-oxopyridin-1(2*H*)-yl]propanoyl}amino)benzoesäure-*tert*.-butylester (Racemat) mit TFA verseift. Ausbeute: 47 mg (60% d. Th.)
LC/MS [Methode 1]: $R_t$ = 0.99 min; MS (ESIpos): m/z = 441 $(M+H)^+$
$^1$H-NMR (400 MHz, DMSO-$d_6$): δ [ppm] = 12.76 (s, 1H), 10.67 (s, 1H), 7.91 (d, 2H), 7.74 (d, 2H), 7.42-7.34 (m, 2H), 7.31 (d, 1H), 7.22 (d, 1H), 6.26 (s, 1H), 5.60 (q, 1H), 3.63 (s, 3H), 2.11 (s, 3H), 1.72 (d, 3H).

**Beispiel 123**

4-[(2-{4-[5-Chlor-2-(trifluormethyl)phenyl]-5-methoxy-2-oxopyridin-1(2*H*)-yl}propanoyl)-amino]benzoesäure (Racemat)

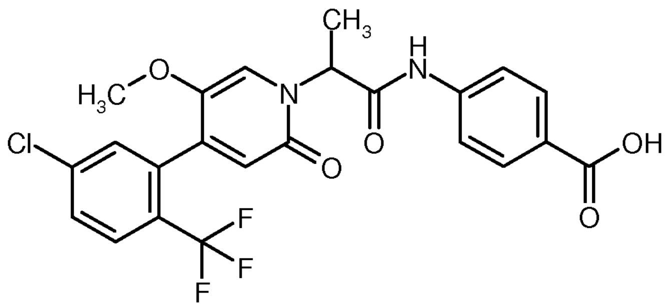

Nach der allgemeinen Methode 2 wurden 145 mg (84% Reinheit, 0.22 mmol) 4-[(2-{4-[5-Chlor-2-(trifluormethyl)phenyl]-5-methoxy-2-oxopyridin-1(2*H*)-yl}propanoyl)amino]benzoesäure-*tert*.-butylester (Racemat) mit TFA verseift. Ausbeute: 41 mg (37% d. Th.)
LC/MS [Methode 1]: $R_t$ = 1.00 min; MS (ESIpos): m/z = 495 $(M+H)^+$
$^1$H-NMR (400 MHz, DMSO-$d_6$): δ [ppm] = 12.76 (s, 1H), 10.70/10.68 (2x s, 1H), 7.91 (d, 2H), 7.87 (d, 1H), 7.79-7.69 (m, 3H), 7.58/7.54 (2x s, 1H), 7.37 (s, 1H), 6.36/6.34 (2x s, 1H), 5.61 (q, 1H), 3.63 (s, 3H), 1.72 (2x d, 3H).

**Beispiel 124**

4-({2-[4-(5-Chlor-2-cyclopropylphenyl)-5-methoxy-2-oxopyridin-1(2*H*)-yl]propanoyl}amino)-benzoesäure (Racemat)

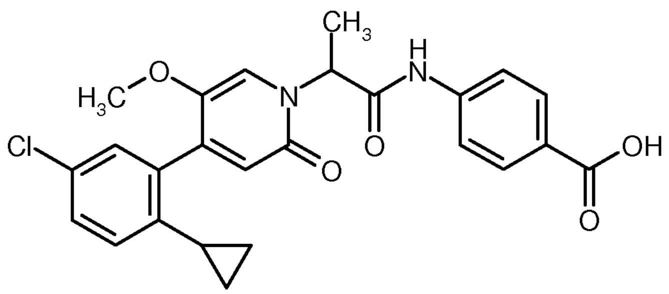

Nach der allgemeinen Methode 2 wurden 114 mg (0.22 mmol) 4-({2-[4-(5-Chlor-2-cyclopropylphenyl)-5-methoxy-2-oxopyridin-1(2*H*)-yl]propanoyl}amino)benzoesäure-*tert*.-butylester (Racemat) mit TFA verseift. Ausbeute: 73 mg (78% d. Th.)
LC/MS [Methode 1]: $R_t$ = 1.03 min; MS (ESIpos): m/z = 467 $(M+H)^+$
$^1$H-NMR (400 MHz, DMSO-$d_6$): δ [ppm] = 12.77 (s, 1H), 10.68 (s, 1H), 7.91 (d, 2H), 7.74 (d, 2H), 7.38 (s, 1H), 7.35 (dd, 1H), 7.19 (d, 1H), 6.95 (d, 1H), 6.31 (s, 1H), 5.61 (q, 1H), 3.65 (s, 3H), 1.72 (d, 3H), 1.70-1.59 (m, 1H), 0.85 (d, 2H), 0.65 (br. s, 2H).

**Beispiel 125**

4-({2-[4-(5-Chlor-2-cyanophenyl)-5-methoxy-2-oxopyridin-1(2*H*)-yl]-4-methoxyhexanoyl}amino)-benzoesäure (Gemisch racemischer Diastereomere)

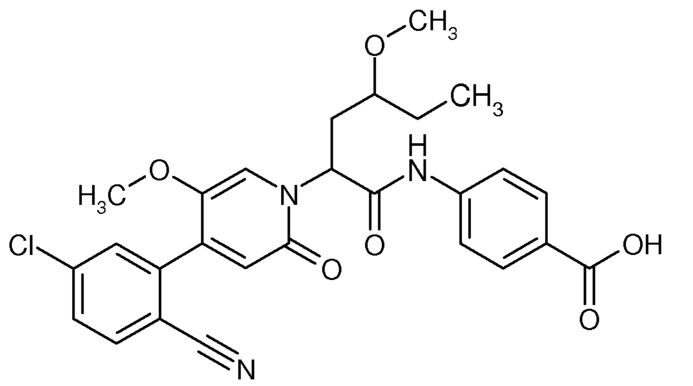

Gemäß allgemeiner Methode 4 wurden 631 mg (1.14 mmol) 4-({2-[4-(5-Chlor-2-cyanophenyl)-5-methoxy-2-oxopyridin-1(2*H*)-yl]-4-methoxyhexanoyl} amino)-benzoesäureethylester (Gemisch racemischer Diastereomere) in 22.5 ml Methanol/Wasser (4/1) mit 745 mg (2.29 mmol) Cäsiumcarbonat zur Reaktion gebracht. Ausbeute: 580 mg (87% Reinheit, 84% d. Th.)
LC/MS [Methode 1]: $R_t$ = 1.00 min; MS (ESIpos): m/z = 524 $(M+H)^+$
$^1$H-NMR (400 MHz, DMSO-$d_6$): δ [ppm] = 12.7 (br. s, 1H), 10.8 (2x s, 1H), 8.01-7.89 (m, 1H), 7.93-7.88 (m, 2H), 7.80-7.72 (m, 4H), 7.59/7.52 (2x s, 1H), 6.54 (s, 1H), 5.88-5.81 (m, 1H), 3.69 (s, 3H), 3.19/3.13 (2x s, 3H), 3.08-3.02/2.95-2.89 (2x m, 1H), 2.44-2.31 (m, 1H), 2.27-2.16 (m, 1H), 1.62-1.45 (m, 2H), 0.86/0.85 (2x t, 3H).

**Beispiel 126**

4-({2-[4-(5-Chlor-2-cyanophenyl)-5-methoxy-2-oxopyridin-1(2*H*)-yl]-4-hydroxybutanoyl}amino)-benzoesäure (Racemat)

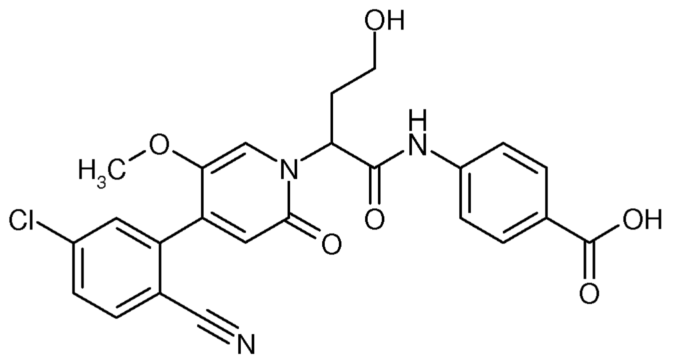

Gemäß allgemeiner Methode 4 wurden 266 mg (355 μmol) 4-[(4-{[*tert.*-Butyl(diphenyl)silyl]oxy}-2-[4-(5-chlor-2-cyanophenyl)-5-methoxy-2-oxopyridin-1(2*H*)-yl]butanoyl)amino]benzoesäureethylester (Racemat) in 7 ml Methanol/Wasser (4/1) mit 232 mg (711 μmol) Cäsiumcarbonat zur Reaktion gebracht. Das Rohprodukt wurde mittels präparativer HPLC [Säule: Chromatorex C18, 10 μm, 125 mm x 30 mm, Eluent: Acetonitril/0.05% Ameisensäure-Gradient (0 bis 3 min 10% Acetonitril, bis 35 min 90% Acetonitril und weitere 3 min 90% Acetonitril)] gereinigt. Ausbeute: 16.5 mg (9% d. Th.)
LC/MS [Methode 1]: $R_t$ = 0.80 min; MS (ESIpos): m/z = 482 $(M+H)^+$
$^1$H-NMR (400 MHz, DMSO-$d_6$): δ [ppm] = 12.8 (br. s, 1H), 10.7 (s, 1H), 8.00 (d, 1H), 7.90 (d, 2H), 7.78-7.71 (m, 4H),

7.49 (s, 1H), 6.53 (s, 1H), 5.76 (dd, 1H), 4.76 (t, 1H), 3.69 (s, 3H), 3.51-3.38 (m, 2H), 2.38-2.26 (m, 2H).

**Beispiel 127**

4-[(2-{4-[5-Chlor-2-(difluormethoxy)phenyl]-5-methoxy-2-oxopyridin-1(2*H*)-yl}propanoyl)-amino]benzoesäure (Racemat)

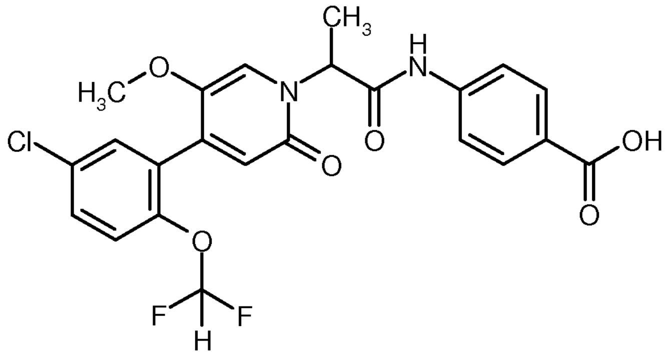

Nach der allgemeinen Methode 2 wurden 47 mg (0.09 mmol) 4-[(2-{4-[5-Chlor-2-(difluormethoxy)phenyl]-5-methoxy-2-oxopyridin-1(2*H*)-yl}propanoyl)amino]benzoesäure-*tert.*-butylester (Racemat) mit TFA verseift. Ausbeute: 33 mg (78% d. Th.)
LC/MS [Methode 1]: $R_t$ = 0.93 min; MS (ESIpos): m/z = 493 $(M+H)^+$
$^1$H-NMR (400 MHz, DMSO-$d_6$): $\delta$ [ppm] = 12.76 (br. s, 1H), 10.68 (s, 1H), 7.91 (d, 2H), 7.73 (d, 2H), 7.58 (dd, 1H), 7.47 (d, 1H), 7.36 (s, 1H), 7.30 (d, 1H), 7.16 (t, 1H), 6.38 (s, 1H), 5.59 (q, 1H), 3.65 (s, 3H), 1.72 (d, 3H).

**Beispiel 128**

4-[(2-{4-[5-Chlor-2-(difluormethoxy)phenyl]-5-methoxy-2-oxopyridin-1(2*H*)-yl}butanoyl)-amino]benzoesäure (Racemat)

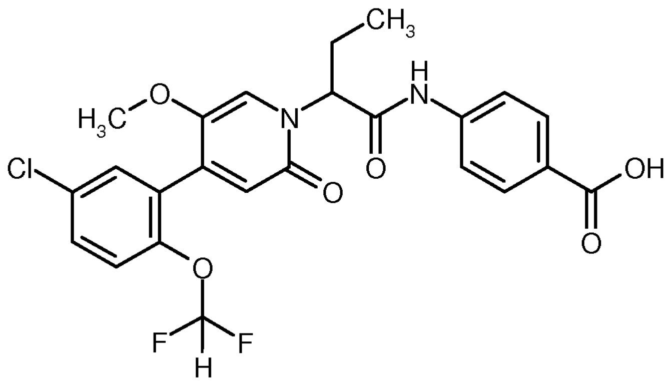

Nach der allgemeinen Methode 2 wurden 127 mg (0.23 mmol) 4-[(2-{4-[5-Chlor-2-(difluormethoxy)phenyl]-5-methoxy-2-oxopyridin-1(2*H*)-yl}butanoyl)amino]benzoesäure-*tert.*-butylester (Racemat) mit TFA verseift. Ausbeute: 72 mg (63% d. Th.)
LC/MS [Methode 1]: $R_t$ = 0.99 min; MS (ESIpos): m/z = 507 $(M+H)^+$
$^1$H-NMR (400 MHz, DMSO-$d_6$): $\delta$ [ppm] = 12.75 (br. s, 1H), 10.78 (s, 1H), 7.91 (d, 2H), 7.75 (d, 2H), 7.58 (dd, 1H), 7.49 (dd, 1H), 7.39 (s, 1H), 7.30 (d, 1H), 7.13 (t, 1H), 6.40 (s, 1H), 5.63 (dd, 1H), 3.64 (s, 3H), 2.24-2.06 (m, 2H), 0.90 (t, 3H).

**Beispiel 129**

4-({2-[4-(5-Chlor-2-cyanophenyl)-5-ethoxy-2-oxopyridin-1(2*H*)-yl]propanoyl}amino)benzoesäure (Racemat)

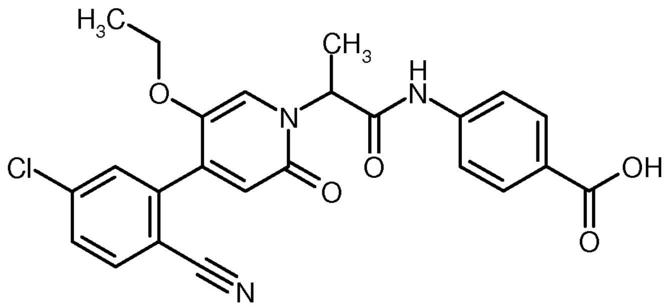

Nach der allgemeinen Methode 2 wurden 32 mg (89% Reinheit, 0.06 mmol) 4-({2-[4-(5-Chlor-2-cyanophenyl)-5-ethoxy-2-oxopyridin-1(2*H*)-yl]propanoyl}amino)benzoesäure-*tert*.-butylester (Racemat) mit TFA verseift. Ausbeute: 11 mg (44% d. Th.)
LC/MS [Methode 3]: $R_t$ = 2.25 min; MS (ESIpos): m/z = 466 $(M+H)^+$
$^1$H-NMR (400 MHz, DMSO-$d_6$): δ [ppm] = 12.76 (s, 1H), 10.70 (s, 1H), 8.01 (d, 1H), 7.91 (d, 2H), 7.78-7.68 (m, 4H), 7.49 (s, 1H), 6.53 (s, 1H), 5.60 (q, 1H), 3.92 (q, 2H), 1.72 (d, 3H), 1.18 (t, 3H).

**Beispiel 130**

4-({2-[4-(5-Chlor-2-cyanophenyl)-5-(difluormethoxy)-2-oxopyridin-1(2*H*)-yl]propanoyl}-amino)benzoesäure (Racemat)

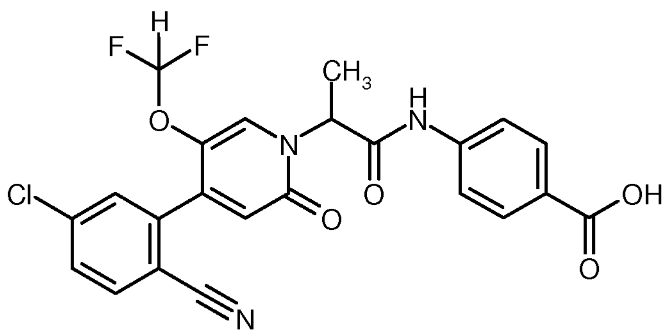

Nach der allgemeinen Methode 2 wurden 99 mg (0.18 mmol) 4-({2-[4-(5-Chlor-2-cyanophenyl)-5-(difluormethoxy)-2-oxopyridin-1(2*H*)-yl]propanoyl}amino)benzoesäure-*tert*.-butylester (Racemat) mit TFA verseift. Ausbeute: 75 mg (84% d. Th.)
LC/MS [Methode 1]: $R_t$ = 0.94 min; MS (ESIpos): m/z = 488 $(M+H)^+$
$^1$H-NMR (400 MHz, DMSO-$d_6$): δ [ppm] = 12.77 (s, 1H), 10.78 (s, 1H), 8.05 (d, 1H), 7.99 (s, 1H), 7.91 (d, 2H), 7.82-7.68 (m, 4H), 6.89 (t, 1H), 6.65 (s, 1H), 5.57 (q, 1H), 1.72 (d, 3H).

**Beispiel 131**

4-({2-[4-(5-Chlor-2-cyanophenyl)-2-oxo-5-(2,2,2-trifluorethoxy)pyridin-1(2*H*)-yl]propanoyl}-amino)benzoesäure (Racemat)

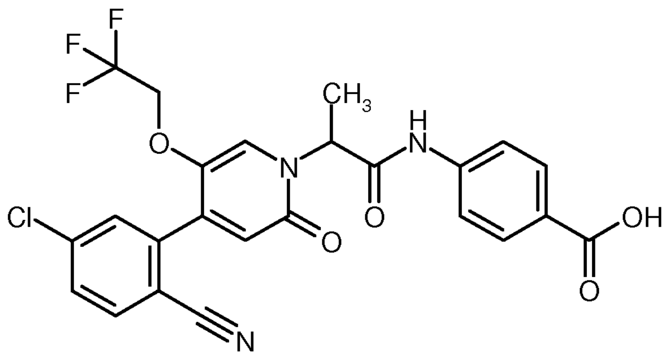

Nach der allgemeinen Methode 2 wurden 113 mg (0.14 mmol) 4-({2-[4-(5-Chlor-2-cyanophenyl)-2-oxo-5-(2,2,2-trifluorethoxy)pyridin-1(2*H*)-yl]propanoyl}amino)benzoesäure-*tert*.-butylester (Racemat) mit TFA verseift. Ausbeute: 39 mg (90% Reinheit, 49% d. Th.)
LC/MS [Methode 1]: $R_t$ = 0.98 min; MS (ESIpos): m/z = 520 $(M+H)^+$
$^1$H-NMR (400 MHz, DMSO-$d_6$): δ [ppm] = 12.77 (br. s, 1H), 10.73 (s, 1H), 8.03 (d, 1H), 7.92 (d, 2H), 7.82-7.70 (m, 5H), 6.59 (s, 1H), 5.58 (q, 1H), 4.66 (dq, 2H), 1.74 (d, 3H).

## Beispiel 132

4-({2-[4-(5-Chlor-2-cyanophenyl)-5-methoxy-2-oxopyridin-1(2*H*)-yl]-3-(4-hydroxycyclohexyl)-propanoyl}amino)benzoesäure (Gemisch racemischer Diastereomere)

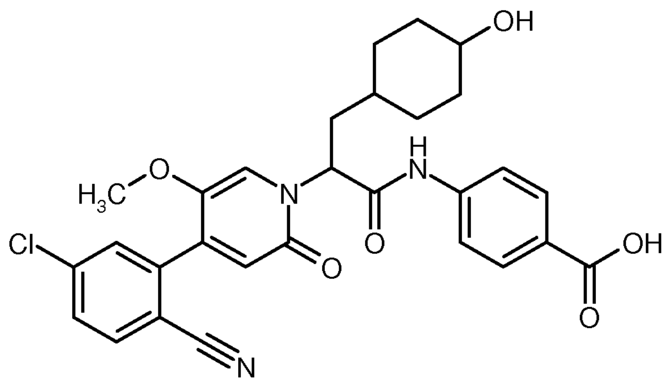

Gemäß allgemeiner Methode 2 wurden 341 mg (473 μmol) 4-({3-(4-{[*tert.*-Butyl(dimethyl)silyl]oxy}cyclohexyl)-2-[4-(5-chlor-2-cyanophenyl)-5-methoxy-2-oxopyridin-1(2*H*)-yl]propanoyl}amino)benzoesäure-*tert*.-butylester (Gemisch racemischer Diastereomere) mit 912 μl (11.8 mmol) TFA umgesetzt. Das Rohprodukt wurde mittels präparativer HPLC [Säule: Chromatorex C18, 10 μm, 125 mm x 30 mm, Eluent: Acetonitril/Wasser-Gradient (0 bis 3 min 15% Acetonitril, bis 35 min. 90% Acetonitril und weitere 3 min 90% Acetonitril)] gereinigt. Ausbeute: 107 mg (27% d. Th.)
LC/MS [Methode 1]: $R_t$ = 0.87-0.89 min; MS (ESIneg): m/z = 548 $(M-H)^-$
$^1$H-NMR (400 MHz, DMSO-$d_6$): δ [ppm] = 12.8 (s, 1H), 10.8 (2x s, 1H), 8.00 (d, 1H), 7.92 (d, 2H), 7.79-7.71 (m, 4H), 7.50/7.49 (2x s, 1H), 6.54/6.53 (2x s, 1H), 5.88-5.81 (m, 1H), 4.45/4.28 (2x d, 1H), 3.69 (s, 3H), 2.23-2.13 (m, 1H), 2.04-1.84 (m, 1H), 1.82-1.69 (m, 2H), 1.63-1.28 (m, 4H), 1.24-1.13 (m, 1H), 1.10-0.95 (m, 2H).

## Beispiel 133

4-({2-[4-(5-Chlor-2-cyanophenyl)-5-methoxy-2-oxopyridin-1(2*H*)-yl]-3-(4-hydroxycyclohexyl)-propanoyl}amino)benzoesäure (Enantiomer 1 der 1. Diastereomers und Enantiomer 1 des 2. Diastereomers)

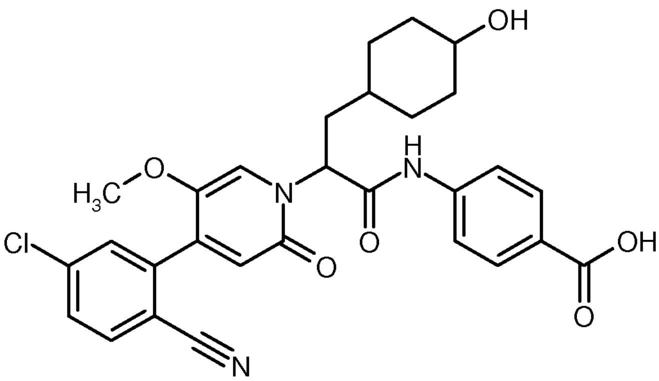

Enantiomerentrennung von 107 mg des Gemisches racemischer Diastereomereaus Beispiel 132 ergab 23 mg der Titelverbindung Beispiel 133: Chirale HPLC: $R_t$ = 5.77/5.84 min; 99% ee, Diastereomerenverhältnis: 1:1.

Trennmethode: Säule: AZ-H 5 μm 250 mm x 20 mm; Eluent: 50% iso-Hexan, 50% Ethanol + 0.2% Essigsäure; Temperatur: 30°C; Fluss: 15 ml/min; UV-Detektion: 230 nm.

Analytik: Säule: AZ-H 5 μm 250 mm x 4.6 mm; Eluent: 50% iso-Hexan, 50% Ethanol + 0.2% TFA + 1% Wasser; Temperatur: 30°C; Fluss: 1 ml/min; UV-Detektion: 220 nm.
$^1$H-NMR (400 MHz, DMSO-$d_6$): δ [ppm] = 12.8 (s, 1H), 10.8 (2x s, 1H), 8.00 (d, 1H), 7.92 (d, 2H), 7.79-7.71 (m, 4H), 7.50/7.49 (2x s, 1H), 6.54/6.53 (2x s, 1H), 5.88-5.81 (m, 1H), 4.45/4.28 (2x d, 1H), 3.69 (s, 3H), 2.23-2.13 (m, 1H), 2.04-1.84 (m, 1H), 1.82-1.69 (m, 2H), 1.63-1.28 (m, 4H), 1.24-1.13 (m, 1H), 1.10-0.95 (m, 2H).

## Beispiel 134

4-({2-[4-(5-Chlor-2-cyanophenyl)-5-methoxy-2-oxopyridin-1(2*H*)-yl]-3-(4-hydroxycyclohexyl)-propanoyl}amino)benzoesäure (Enantiomer 2 der 1. Diastereomers)

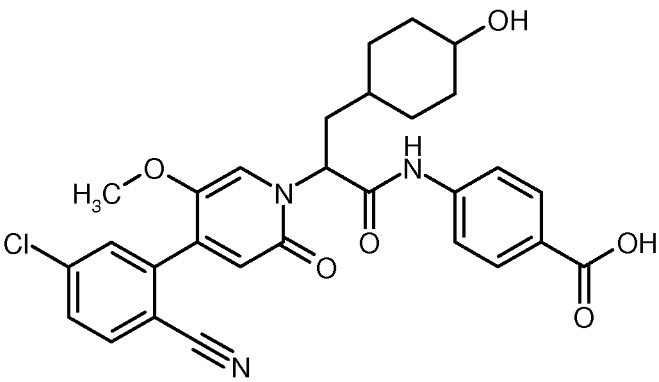

Enantiomerentrennung von 107 mg des Gemisches racemischer Diastereomereaus Beispiel 132 ergab 11 mg der Titelverbindung Beispiel 134: Chirale HPLC: $R_t$ = 9.44 min; 99% ee.

Trennmethode: Säule: AZ-H 5 μm 250 mm x 20 mm; Eluent: 50% iso-Hexan, 50% Ethanol + 0.2% Essigsäure; Temperatur: 30°C; Fluss: 15 ml/min; UV-Detektion: 230 nm.

Analytik: Säule: AZ-H 5 μm 250 mm x 4.6 mm; Eluent: 50% iso-Hexan, 50% Ethanol + 0.2% TFA + 1% Wasser; Temperatur: 30°C; Fluss: 1 ml/min; UV-Detektion: 220 nm.
$^1$H-NMR (400 MHz, DMSO-$d_6$): δ [ppm] = 12.8 (s, 1H), 10.8 (s, 1H), 8.00 (d, 1H), 7.91 (d, 2H), 7.78-7.71 (m, 4H), 7.49 (s, 1H), 6.54 (s, 1H), 5.87-5.80 (m, 1H), 4.45 (d, 1H), 3.69 (s, 3H), 2.23-2.13 (m,1H), 1.95-1.85 (m, 1H), 1.84-1.69 (m, 4H), 1.10-0.93 (m, 5H).

**Beispiel 135**

4-({2-[4-(5-Chlor-2-cyanophenyl)-5-methoxy-2-oxopyridin-1(2*H*)-yl]-3-(4-hydroxycyclohexyl)-propanoyl}amino)benzoesäure (Enantiomer 2 des 2. Diastereomers)

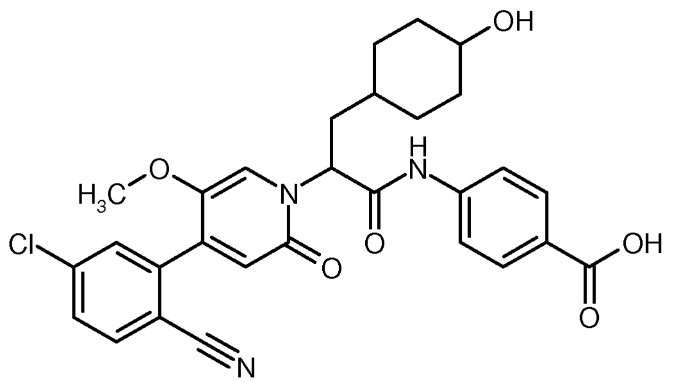

Enantiomerentrennung von 107 mg des Gemisches racemischer Diastereomereaus Beispiel 132 ergab 14 mg der Titelverbindung Beispiel 135: Chirale HPLC: $R_t$ = 11.77 min; 89% ee.

Trennmethode: Säule: AZ-H 5 μm 250 mm x 20 mm; Eluent: 50% iso-Hexan, 50% Ethanol + 0.2% Essigsäure; Temperatur: 30°C; Fluss: 15 ml/min; UV-Detektion: 230 nm.

Analytik: Säule: AZ-H 5 μm 250 mm x 4.6 mm; Eluent: 50% iso-Hexan, 50% Ethanol + 0.2% TFA + 1% Wasser; Temperatur: 30°C; Fluss: 1 ml/min; UV-Detektion: 220 nm.

$^1$H-NMR (400 MHz, DMSO-$d_6$): δ [ppm] = 12.8 (s, 1H), 10.8 (s, 1H), 8.00 (d, 1H), 7.91 (d, 2H), 7.79-7.71 (m ,4H), 7.50 (s, 1H), 6.53 (s, 1H), 5.89-5.81 (m, 1H), 4.29 (d, 1H), 3.69 (s, 3H), 2.22-2.12 (m, 1H), 2.05-1.95 (m, 1H), 1.64-1.14 (m, 9H).

**Beispiel 136**

4-({2-[4-(5-Chlor-2-cyanophenyl)-5-methoxy-2-oxopyridin-1(2*H*)-yl]-4-(trifluormethoxy)-butanoyl }amino)benzoesäure (Racemat)

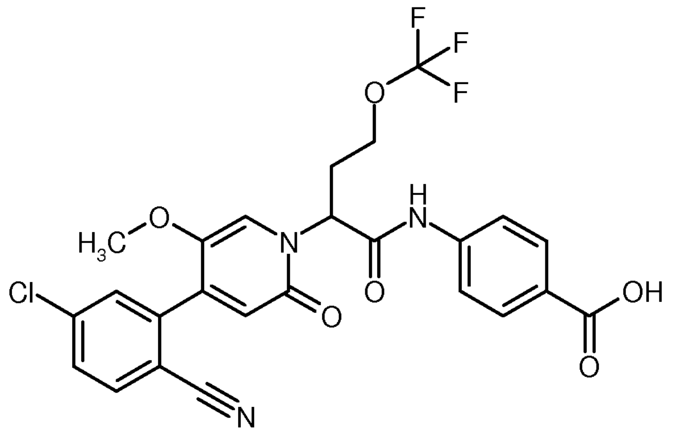

Gemäß allgemeiner Methode 2 wurden 151 mg (248 μmol) 4-({2-[4-(5-Chlor-2-cyanophenyl)-5-methoxy-2-oxopyridin-1(2*H*)-yl]-4-(trifluormethoxy)butanoyl}amino)benzoesäure-*tert*.-butylester (Racemat) mit 574 μl (7.45 mmol) TFA umgesetzt. Das Rohprodukt wurde mittels präparativer HPLC [Säule: Chromatorex C18, 10 μm, 125 mm x 30 mm, Eluent: Acetonitril/Wasser-Gradient (0 bis 3 min 15% Acetonitril, bis 35 min 90% Acetonitril und weitere 3 min 90% Acetonitril)] gereinigt. Ausbeute: 65 mg (47% d. Th.)

LC/MS [Methode 1]: $R_t$ = 1.02 min; MS (ESIneg): m/z = 548 $(M-H)^-$

$^1$H-NMR (400 MHz, DMSO-$d_6$): δ [ppm] = 12.8 (s, 1H), 10.8 (s, 1H), 8.00 (d, 1H), 7.91 (d, 2H), 7.76-7.70 (m, 4H), 7.51 (s, 1H), 6.56 (s, 1H), 5.81 (t, 1H), 4.20-4.15 (m, 1H), 4.03-3.99 (m, 1H), 3.69 (s, 3H), 2.66-2.60 (m, 2H).

**Beispiel 137**

4-({2-[4-(5-Chlor-2-cyanophenyl)-5-methoxy-2-oxopyridin-1(2*H*)-yl]-4-(trifluormethoxy)-butanoyl}amino)benzoesäure (Enantiomer 1)

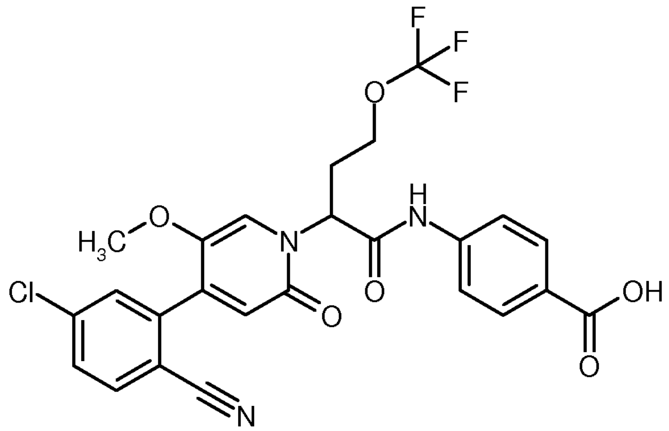

Enantiomerentrennung von 165 mg des Gemisches racemischer Diastereomere aus Beispiel 136 ergab 65 mg der Titelverbindung Beispiel 137: Chirale HPLC: $R_t$ = 1.00 min; 99% ee.

Trennmethode (SFC): Säule: AZ-H 5 μm 250 mm x 30 mm; Eluent: 70% Kohlendioxid, 30% Ethanol; Temperatur: 40°C; Fluss: 100 ml/min; UV-Detektion: 210 nm.

Analytik (SFC): Säule: AZ-3 5 μm 250 mm x 4.6 mm; Eluent: 85% Kohlendioxid, 15% Ethanol; Temperatur: 30°C; Fluss: 3 ml/min; UV-Detektion: 220 nm.

**Beispiel 138**

4-({2-[4-(5-Chlor-2-cyanophenyl)-5-methoxy-2-oxopyridin-1(2*H*)-yl]-4-(trifluormethoxy)-butanoyl}amino)benzoesäure (Enantiomer 2)

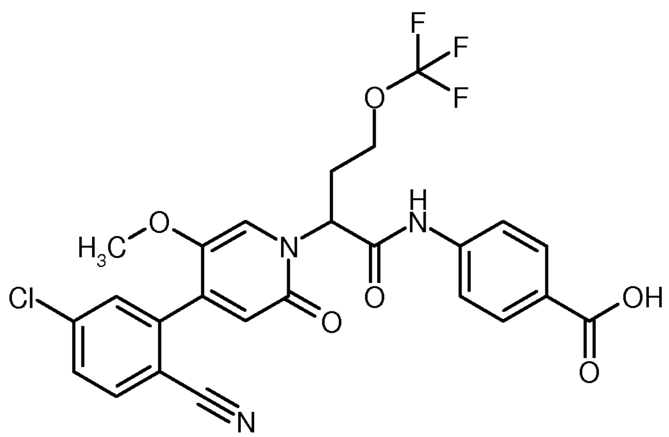

Enantiomerentrennung von 165 mg des Gemisches racemischer Diastereomere aus Beispiel 136 ergab 69 mg der Titelverbindung Beispiel 138: Chirale HPLC: $R_t$ = 2.01 min; 94% ee.

Trennmethode (SFC): Säule: AZ-H 5 μm 250 mm x 30 mm; Eluent: 70% Kohlendioxid, 30% Ethanol; Temperatur: 40°C; Fluss: 100 ml/min; UV-Detektion: 210 nm.

Analytik (SFC): Säule: AZ-3 5 μm 250 mm x 4.6 mm; Eluent: 85% Kohlendioxid, 15% Ethanol; Temperatur: 30°C; Fluss: 3 ml/min; UV-Detektion: 220 nm.

**Beispiel 139**

2-[4-(5-Chlor-2-cyanophenyl)-5-methoxy-2-oxopyridin-1(2*H*)-yl]-3-(3-ethyloxetan-3-yl)-*N*-[4-(5-oxo-4,5-dihydro-1,2,4-oxadiazol-3-yl)phenyl]propanamid (Racemat)

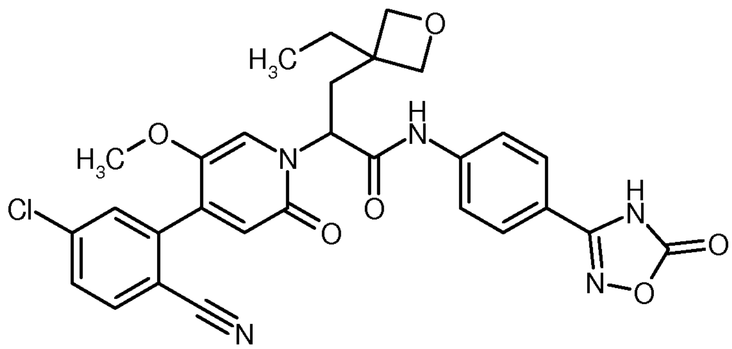

Gemäß allgemeiner Methode 5 wurden 37.4 mg (89.6 μmol) 2-[4-(5-Chlor-2-cyanophenyl)-5-methoxy-2-oxopyridin-1(2*H*)-yl]-3-(3-ethyloxetan-3-yl)propansäure (Racemat), 15.9 mg (89.6 μmol) 3-(4-Aminphenyl)-1,2,4-oxadiazol-5(4*H*)-on, 12.7 mg (89.6 μmol) Oxima und 14.0 μl (89.6 μmol) DIC in 950 μl Dimethylformamid zur Reaktion gebracht. Das Rohprodukt wurde mittels präparativer HPLC [Säule: Chromatorex C18, 10 μm, 125 mm x 30 mm, Eluent: Acetonitril/0.05% Ameisensäure-Gradient (0 bis 3 min 10% Acetonitril, bis 35 min 90% Acetonitril und weitere 3 min 90% Acetonitril)] und abschließend unter Verwendung von Methode 10 aufgereinigt. Ausbeute: 2 mg (4% d. Th.)
LC/MS [Methode 1]: $R_t$ = 0.96 min; MS (ESIpos): m/z = 576 $(M+H)^+$
$^1$H-NMR (400 MHz, DMSO-$d_6$): δ [ppm] = 12.9 (br. s, 1H), 10.9 (s, 1H), 7.99 (d, 1H), 7.83 (d, 2H), 7.79 (m, 2H), 7.74-7.70 (m, 3H), 6.54 (s, 1H), 5.82-5.76 (m, 1H), 4.40 (d, 1H), 4.26 (d, 1H), 4.15 (d, 1H), 4.05 (d, 1H), 3.72 (s, 3H), 2.56-2.44 (m, 2H), 1.83-1.75 (m, 2H), 0.90 (t, 3H).

**Beispiel 140**

4-({2-[4-(5-Chlor-2-cyanophenyl)-5-methoxy-2-oxopyridin-1(2*H*)-yl](3,3,4,4,4-pentadeutero)butanoyl}amino)benzoesäure (Racemat)

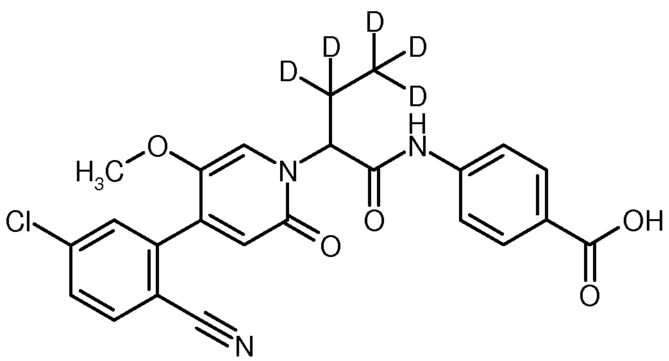

Nach der allgemeinen Methode 2 wurden 79 mg (91% Reinheit, 0.14 mmol) 4-({2-[4-(5-Chlor-2-cyanophenyl)-5-methoxy-2-oxopyridin-1(2*H*)-yl](3,3,4,4,4-pentadeutero)butanoyl}amino)-benzoesäure-*tert*.-butylester (Racemat) mit TFA verseift. Das Rohprodukt wurde mittels präparativer HPLC (Reprosil C18, Wasser-Acetonitril-Gradient) aufgereinigt. Ausbeute: 30 mg (46% d. Th.)

LC/MS [Methode 1]: $R_t$ = 0.93 min; MS (ESIpos): m/z = 471 $(M+H)^+$,
$^1$H-NMR (400 MHz, DMSO-$d_6$): δ [ppm] = 12.74 (br. s, 1H), 10.78 (s, 1H), 8.00 (d, 1H), 7.91 (d, 2H), 7.79-7.70 (m, 4H), 7.49 (s, 1H), 6.54 (s, 1H), 5.63 (s, 1H), 3.69 (s, 3H).

**Beispiel 141**

4-({2-[4-(5-Chlor-2-cyanophenyl)-5-methoxy-2-oxopyridin-1(2*H*)-yl]-4-methoxyhexanoyl}amino)-benzoesäure (Enantiomer 1 des 1. Diastereomers)

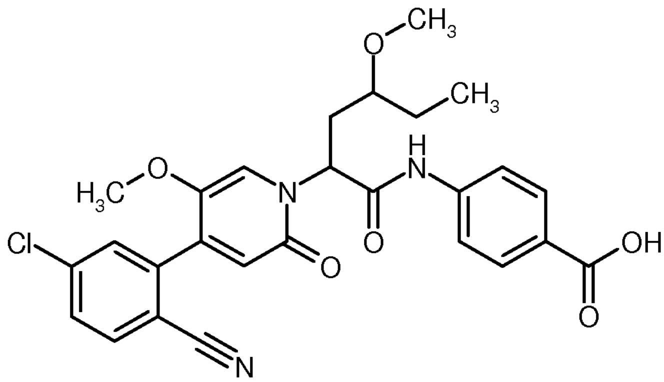

Diastereomeren- und Enantiomerentrennung von 528 mg des Gemisches aus Beispiel 125 ergab nach weiterer präparativer HPLC 32.5 mg der Titelverbindung Beispiel 141 (Enantiomer 1 des 1. Diastereomers): Chirale HPLC: $R_t$ = 3.44 min; 99% ee.

Trennmethode (SFC): Säule: AD-H 5 μm 250 mm x 20 mm; Eluent: 85% Kohlendioxid, 15% 2-Propanol; Temperatur: 40°C; Fluss: 80 ml/min; Druck: 100 bar; UV-Detektion: 210 nm.

Analytik: Säule: AD-3 5 μm 250 mm x 4.6 mm; Eluent: 95-50% Kohlendioxid, 5-50% 2-Propanol; Temperatur: 40°C; Fluss: 3 ml/min; UV-Detektion: 220 nm.
$^1$H-NMR (400 MHz, DMSO-$d_6$): δ [ppm] = 12.7 (br. s, 1H), 10.8 (s, 1H), 7.99 (d, 1H), 7.90 (d, 2H), 7.79-7.71 (m, 4H), 7.52 (s, 1H), 6.54 (s, 1H), 5.87-5.81 (m, 1H), 3.68 (s, 3H), 3.13 (s, 3H), 2.96-2.89 (m, 1H), 2.43-2.31 (m, 1H), 2.26-2.17 (m, 1H), 1.58-1.46 (m, 2H), 0.85 (t, 3H).

**Beispiel 142**

4-({2-[4-(5-Chlor-2-cyanophenyl)-5-methoxy-2-oxopyridin-1(2*H*)-yl]-4-methoxyhexanoyl}amino)-benzoesäure (Enantiomer 2 des 1. Diastereomers)

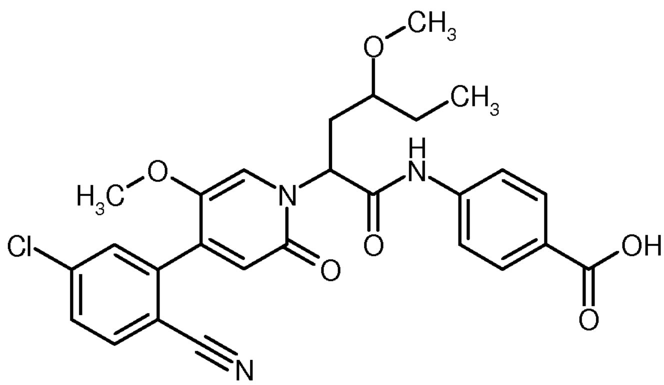

Diastereomeren- und Enantiomerentrennung von 528 mg des Gemisches aus Beispiel 125 ergab nach weiterer

präparativer HPLC 32.4 mg der Titelverbindung Beispiel 142 (Enantiomer 2 des 1. Diastereomers): Chirale HPLC: $R_t$ = 3.53 min; 99% ee.

Trennmethode (SFC): Säule: AD-H 5 μm 250 mm x 20 mm; Eluent: 85% Kohlendioxid, 15% 2-Propanol; Temperatur: 40°C; Fluss: 80 ml/min; Druck: 100 bar; UV-Detektion: 210 nm.

Analytik: Säule: AD-3 5 μm 250 mm x 4.6 mm; Eluent: 95-50% Kohlendioxid, 5-50% 2-Propanol; Temperatur: 40°C; Fluss: 3 ml/min; UV-Detektion: 220 nm.

$^1$H-NMR (400 MHz, DMSO-$d_6$): δ [ppm] = 12.7 (br. s, 1H), 10.8 (s, 1H), 7.99 (d, 1H), 7.90 (d, 2H), 7.79-7.71 (m, 4H), 7.52 (s, 1H), 6.54 (s, 1H), 5.87-5.81 (m, 1H), 3.68 (s, 3H), 3.13 (s, 3H), 2.96-2.89 (m, 1H), 2.43-2.31 (m, 1H), 2.26-2.17 (m, 1H), 1.58-1.46 (m, 2H), 0.85 (t, 3H).

## Beispiel 143

4-({2-[4-(5-Chlor-2-cyanophenyl)-5-methoxy-2-oxopyridin-1(2*H*)-yl]-4-methoxyhexanoyl}amino)-benzoesäure (Enantiomer 1 des 2. Diastereomers)

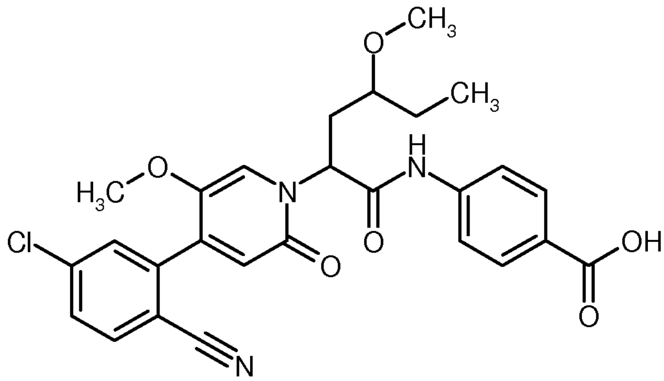

Diastereomeren- und Enantiomerentrennung von 528 mg des Gemisches aus Beispiel 125 ergab nach weiterer präparativer HPLC 25.9 mg der Titelverbindung Beispiel 143 (Enantiomer 1 des 2. Diastereomers): Chirale HPLC: $R_t$ = 3.71 min; 99% ee.

Trennmethode (SFC): Säule: AD-H 5 μm 250 mm x 20 mm; Eluent: 85% Kohlendioxid, 15% 2-Propanol; Temperatur: 40°C; Fluss: 80 ml/min; Druck: 100 bar; UV-Detektion: 210 nm.

Analytik: Säule: AD-3 5 μm 250 mm x 4.6 mm; Eluent: 95-50% Kohlendioxid, 5-50% 2-Propanol; Temperatur: 40°C; Fluss: 3 ml/min; UV-Detektion: 220 nm.

$^1$H-NMR (400 MHz, DMSO-$d_6$): δ [ppm] = 12.8 (br. s, 1H), 10.8 (s, 1H), 7.99 (d, 1H), 7.91 (d, 2H), 7.77 (d, 2H), 7.75-7.71 (m, 2H), 7.59 (s, 1H), 6.54 (s, 1H), 5.86-5.81 (m, 1H), 3.68 (s, 3H), 3.19 (s, 3H), 3.09-3.02 (m, 1H), 2.28-2.21 (m, 2H), 1.63-1.45 (m, 2H), 0.86 (t, 3H).

## Beispiel 144

4-([2-[4-(5-Chlor-2-cyanophenyl)-5-methoxy-2-oxopyridin-1(2*H*)-yl]-4-methoxyhexanoyl}amino)-benzoesäure (Enantiomer 2 des 2. Diastereomers)

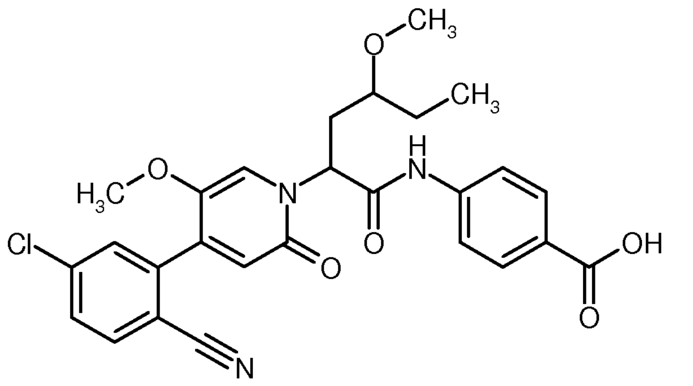

Diastereomeren- und Enantiomerentrennung von 528 mg des Gemisches aus Beispiel 125 ergab nach weiterer präparativer HPLC 21.9 mg der Titelverbindung Beispiel 144 (Enantiomer 2 des 2. Diastereomers): Chirale HPLC: $R_t$ = 4.27 min; 99% ee.

Trennmethode (SFC): Säule: AD-H 5 µm 250 mm x 20 mm; Eluent: 85% Kohlendioxid, 15% 2-Propanol; Temperatur: 40°C; Fluss: 80 ml/min; Druck: 100 bar; UV-Detektion: 210 nm.

Analytik: Säule: AD-3 5 µm 250 mm x 4.6 mm; Eluent: 95-50% Kohlendioxid, 5-50% 2-Propanol; Temperatur: 40°C; Fluss: 3 ml/min; UV-Detektion: 220 nm.

$^1$H-NMR (400 MHz, DMSO-$d_6$): δ [ppm] = 12.8 (br. s, 1H), 10.8 (s, 1H), 7.99 (d, 1H), 7.91 (d, 2H), 7.77 (d, 2H), 7.75-7.71 (m, 2H), 7.59 (s, 1H), 6.54 (s, 1H), 5.86-5.81 (m, 1H), 3.68 (s, 3H), 3.19 (s, 3H), 3.09-3.02 (m, 1H), 2.28-2.21 (m, 2H), 1.63-1.45 (m, 2H), 0.86 (t, 3H).

## Beispiel 145

4-({2-[4-(5-Chlor-2-cyanophenyl)-5-methoxy-2-oxopyridin-1(2*H*)-yl]-3-[1-(trifluormethyl)cyclopropyl]propanoyl}amino)benzoesäure (Racemat)

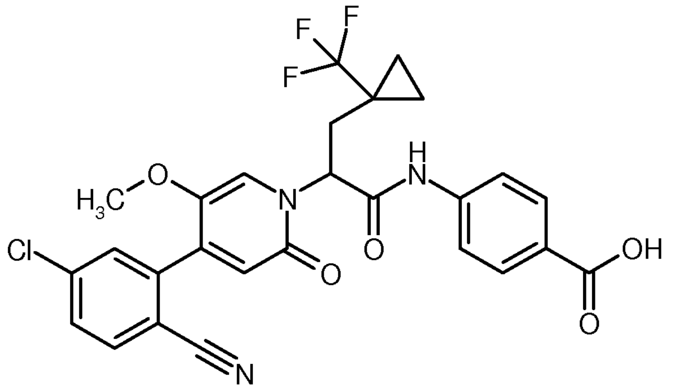

Nach der allgemeinen Methode 2 wurden 157 mg (255 µmol) 4-({2-[4-(5-Chlor-2-cyanophenyl)-5-methoxy-2-oxopyridin-1(2*H*)-yl]-3-[1-(trifluormethyl)cyclopropyl]propanoyl}amino)benzoesäure-*tert*.-butylester (Racemat) mit 393 µl (5.10 mmol) Trifluoressigsäure verseift und nach präparativer HPLC [Säule: Chromatorex C18, 10 µm, 125 mm x 30mm, Eluent: Acetonitril/0.1% Ameisensäure-Gradient (0 bis 3 min 10% Acetonitril, bis 35 min 90% Acetonitril und weitere 3 min 90% Acetonitril)] die Titelverbindung erhalten. Ausbeute: 94 mg (65% d. Th.)

LC/MS [Methode 1]: $R_t$ = 1.03 min; MS (ESIneg): m/z = 558 $(M-H)^-$,

$^1$H-NMR (400 MHz, DMSO-$d_6$): δ [ppm] = 12.8 (br. s, 1H), 10.8 (s, 1H), 7.99 (d, 1H), 7.92 (d, 2H), 7.78-7.71 (m, 4H), 7.52 (s, 1H), 6.55 (s, 1H), 5.86-5.79 (m, 1H), 3.67 (s, 3H), 2.60-2.44 (m, 2H), 0.97-0.79 (m, 4H).

**Beispiel 146**

4-({2-[4-(5-Chlor-2-cyanophenyl)-5-methoxy-2-oxopyridin-1(2*H*)-yl]-3-[1-(trifluormethyl)cyclopropyl]propanoyl}amino)benzoesäure (Enantiomer 1)

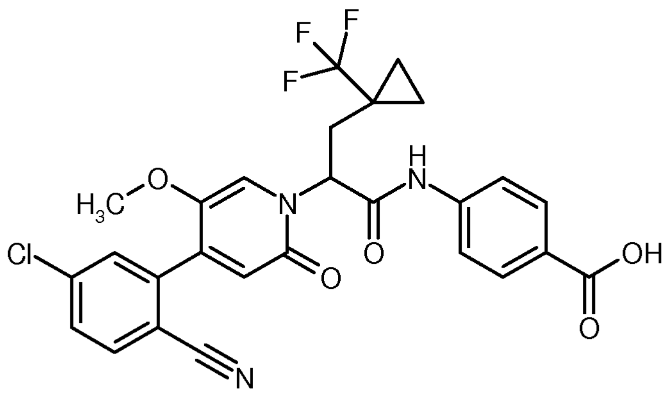

Enantiomerentrennung von 94 mg des Racemates aus Beispiel 145 ergab 29.9 mg der Titelverbindung Beispiel 146 (Enantiomer 1): Chirale HPLC: $R_t$ = 3.71 min; 99% ee.
Trennmethode (SFC): Säule: AZ-H 5 μm 250 mm x 30 mm; Eluent: 75% Kohlendioxid, 25% Ethanol; Temperatur: 40°C; Fluss: 100 ml/min; Druck: 100 bar; UV-Detektion: 210 nm.
Analytik (SFC): Säule: AZ-H 5 μm 250 mm x 4.6 mm; Eluent: 80% Kohlendioxid, 20% Ethanol; Temperatur: 40°C; Fluss: 3 ml/min; UV-Detektion: 210 nm.

**Beispiel 147**

4-({2-[4-(5-Chlor-2-cyanophenyl)-5-methoxy-2-oxopyridin-1(2*H*)-yl]-3-[1-(trifluormethyl)cyclopropyl]propanoyl}amino)benzoesäure (Enantiomer 2)

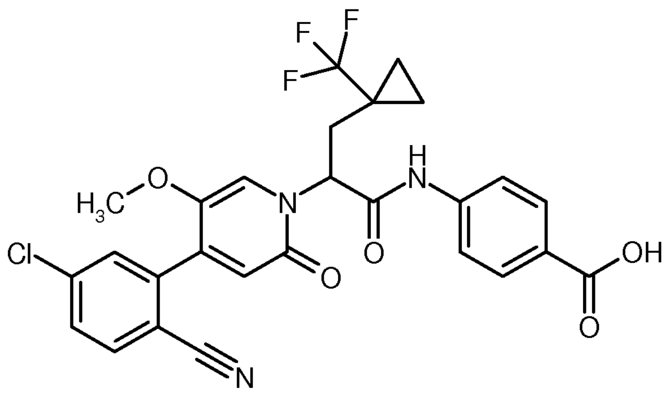

Enantiomerentrennung von 94 mg des Racemates aus Beispiel 145 ergab 27.8 mg der Titelverbindung Beispiel 147 (Enantiomer 2): Chirale HPLC: $R_t$ = 5.32 min; 99% ee.
Trennmethode (SFC): Säule: AZ-H 5 μm 250 mm x 30 mm; Eluent: 75% Kohlendioxid, 25% Ethanol; Temperatur: 40°C; Fluss: 100 ml/min; Druck: 100 bar; UV-Detektion: 210 nm.
Analytik (SFC): Säule: AZ-H 5 μm 250 mm x 4.6 mm; Eluent: 80% Kohlendioxid, 20% Ethanol; Temperatur: 40°C; Fluss: 3 ml/min; UV-Detektion: 210 nm.

**Beispiel 148**

4-({2-[4-(5-Chlor-2-cyanophenyl)-5-methoxy-2-oxopyridin-1(2*H*)-yl]-3-[1-(trifluormethyl)-cyclobutyl]propanoyl}amino)benzoesäure (Racemat)

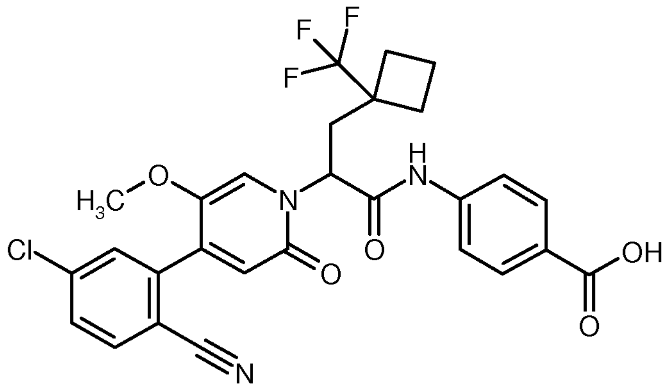

Nach der allgemeinen Methode 2 wurden 58 mg (0.09 mmol) 4-({2-[4-(5-Chlor-2-cyanophenyl)-5-methoxy-2-oxopyridin-1(2*H*)-yl]-3-[1-(trifluormethyl)cyclobutyl]propanoyl}amino)benzoesäure-*tert*.-butylester (Racemat) mit TFA verseift. Das Rohprodukt wurde mittels präparativer HPLC (Reprosil C18, Wasser-Acetonitril-Gradient) aufgereinigt. Ausbeute: 35 mg (65% d. Th.)
LC/MS [Methode 1]: $R_t$ = 1.09 min; MS (ESIpos): m/z = 574 $(M+H)^+$,
$^1$H-NMR (400 MHz, DMSO-$d_6$): δ [ppm] = 10.88 (s, 1H), 7.99 (d, 1H), 7.92 (d, 2H), 7.81-7.70 (m, 4H), 7.58 (s, 1H), 6.58 (s, 1H), 5.95 (t, 1H), 3.68 (s, 3H), 2.61 (dd, 1H), 2.48 (dd, 1H), 2.21 (t, 2H), 2.18-2.12 (m, 1H), 2.12-2.02 (m, 1H), 1.99-1.83 (m, 2H).

**Beispiel 149**

4-({2-[4-(5-Chlor-2-cyanophenyl)-5-methoxy-2-oxopyridin-1(2*H*)-yl]-3-(3,3-difluorcyclobutyl)-propanoyl }amino)benzoesäure (Racemat)

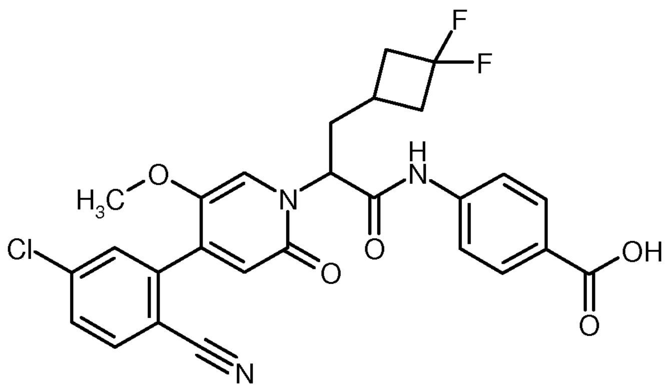

Nach der allgemeinen Methode 2 wurden 175 mg (0.29 mmol) 4-({2-[4-(5-Chlor-2-cyanophenyl)-5-methoxy-2-oxopyridin-1(2*H*)-yl]-3-(3,3-difluorcyclobutyl)propanoyl}amino)benzoesäure-*tert*.-butylester (Racemat) mit TFA verseift. Das Rohprodukt wurde mittels präparativer HPLC (Reprosil C18, Wasser-Acetonitril-Gradient) aufgereinigt. Ausbeute: 122 mg (77% d. Th.)
LC/MS [Methode 1]: $R_t$ = 1.01 min; MS (ESIpos): m/z = 542 $(M+H)^+$,
$^1$H-NMR (400 MHz, DMSO-$d_6$): δ [ppm] = 12.77 (s, 1H), 10.79 (s, 1H), 8.00 (d, 1H), 7.92 (d, 2H), 7.79-7.69 (m, 4H), 7.53 (s, 1H), 6.54 (s, 1H), 5.73 (dd, 1H), 3.70 (s, 3H), 2.68-2.50 (m, 2H), 2.50-2.2.39 (m, 2H), 2.39-2.21 (m, 2H), 2.08-1.95

(m, 1H).

**Beispiel 150**

4-({2-[4-(5-Chlor-2-cyanophenyl)-5-methoxy-2-oxopyridin-1(2*H*)-yl]-3-(3-methyloxetan-3-yl)propanoyl}amino)benzoesäure (Racemat)

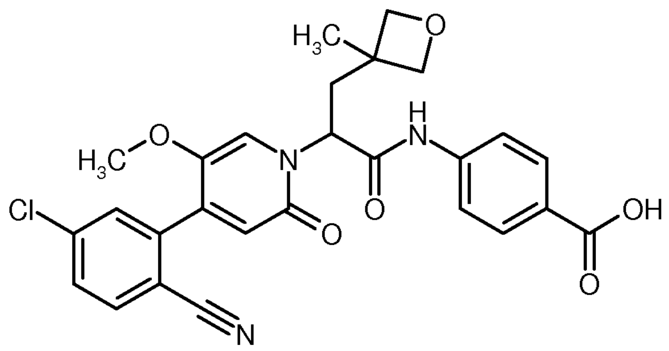

25 mg (44 µmol) 4-({2-[4-(5-Chlor-2-cyanophenyl)-5-methoxy-2-oxopyridin-1(2*H*)-yl]-3-(3-methyloxetan-3-yl)propanoyl}amino)benzoesäureallylester (Racemat) und 48.2 µl (445 µmol) *N*-Methylanilin wurden in 1 ml Tetrahydrofuran vorgelegt und die resultierende Lösung entgast. Anschließend wurde 5 mg (4 µmol) Tetrakis(triphenylphosphin)palladium(0) hinzugegeben und für 30 min bei Raumtemperatur nachgerührt. Die Reaktionslösung wurde direkt mittels präparativer HPLC (neutral) gereinigt und die Titelverbindung nach abschließender präparativer Dünnschichtchromatographie (Essigsäurethylester/Cyclohexan = 1:1) erhalten. Ausbeute: 10.1 mg (92% Reinheit, 42% d. Th.)
LC/MS [Methode 1]: $R_t$ = 0.89 min; MS (ESIpos): m/z = 522 $(M+H)^+$,
$^1$H-NMR (400 MHz, DMSO-$d_6$): δ [ppm] = 12.8 (br. s, 1H), 10.8 (s, 1H), 7.99 (d, 1H), 7.92 (d, 2H), 7.77-7.71 (m, 4H), 7.66 (s, 1H), 6.52 (s, 1H), 5.87 (dd, 1H), 4.46 (d, 1H), 4.29 (d, 1H), 4.11 (d, 1H), 3.95 (d, 1H), 3.72 (s, 3H), 2.68 (dd, 1H), 2.37 (dd, 1H), 1.38 (s, 3H).

**Beispiel 151**

4-({2-[4-(5-Chlor-2-cyanophenyl)-5-methoxy-2-oxopyridin-1(2*H*)-yl]-3-(4-methyltetrahydro-2*H*-pyran-4-yl)propanoyl}amino)benzoesäure (Racemat)

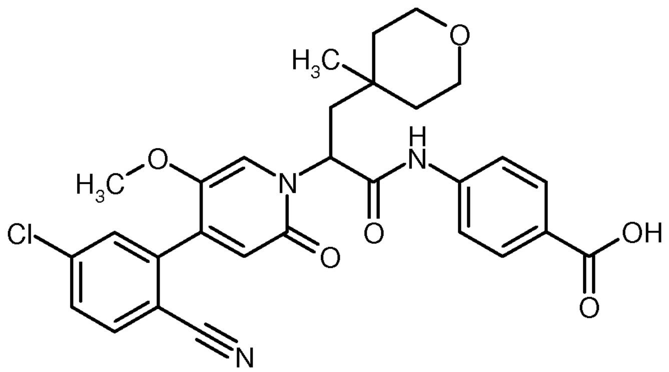

Gemäß allgemeiner Methode 4 wurden 69.0 mg (119 µmol) 4-({2-[4-(5-Chlor-2-cyanophenyl)-5-methoxy-2-oxopyridin-1(2*H*)-yl]-3-(4-methyltetrahydro-2*H*-pyran-4-yl)propanoyl}amino)benzoesäureethylester (Racemat) in 4.3 ml Ethanol/Wasser (3/1) mit 198 mg (609 µmol) Cäsiumcarbonat zur Reaktion gebracht und die Titelverbindung nach mittels präparativer HPLC [Säule: Chromatorex C18, 10 µm, 125 mm x 30 mm, Eluent: Acetonitril/0.1% Ameisensäure-Gradient (0 bis 3 min 10% Acetonitril, bis 35 min 90% Acetonitril und weitere 3 min 90% Acetonitril)] erhalten. Ausbeute:

38 mg (57% d. Th.)
LC/MS [Methode 1]: $R_t$ = 0.96 min; MS (ESIpos): m/z = 550 $(M+H)^+$,
$^1$H-NMR (400 MHz, DMSO-$d_6$): δ [ppm] = 12.8 (br. s, 1H), 10.9 (s, 1H), 7.98 (d, 1H), 7.92 (d, 2H), 7.77 (d, 2H), 7.74-7.71 (m, 2H), 7.66 (s, 1H), 6.52 (s, 1H), 6.05 (dd, 1H), 3.71 (s, 3H), 3.65-3.40 (m, 4H), 2.29 (dd, 1H), 2.04 (dd, 1H), 1.57-1.49 (m, 1H), 1.41-1.25 (m, 2H), 1.16-1.09 (m, 1H), 1.03 (s, 3H).

**Beispiel 152**

4-({2-[4-(5-Chlor-2-cyanophenyl)-5-methoxy-2-oxopyridin-1(2*H*)-yl]-3-(*cis*-4-methoxycyclohexyl)propanoyl}amino)benzoesäure (Racemat)

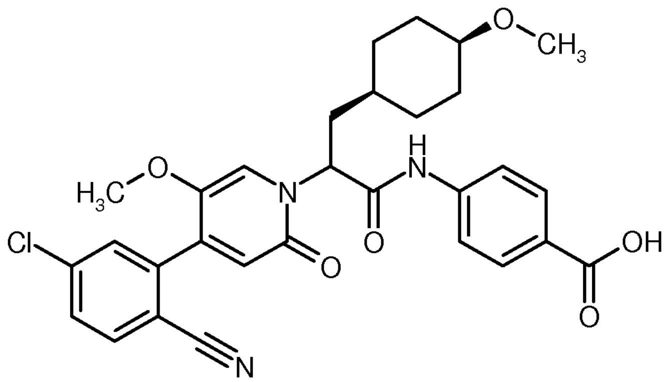

Gemäß allgemeiner Methode 4 wurden 2.10 g (3.55 mmol) 4-({2-[4-(5-Chlor-2-cyanophenyl)-5-methoxy-2-oxopyridin-1(2*H*)-yl]-3-(*cis*-4-methoxycyclohexyl)propanoyl}amino)benzoesäureethylester (Racemat) in 129 ml Ethanol/Wasser (2.6/1) mit 5.89 g (18.1 mmol) Cäsiumcarbonat zur Reaktion gebracht. Nach vollständigem Umsatz wurde mit Salzsäure (1N) auf pH 5-6 eingestellt und für 30 min nachgerührt. Der Niederschlag wurde abgesaugt, mit wenig Wasser gewaschen, im Hochvakuum getrocknet und entsprach der Titelverbindung. Ausbeute: 1.24 g (60% d. Th.)
LC/MS [Methode 1]: $R_t$ = 1.03 min; MS (ESIpos): m/z = 564 $(M+H)^+$,
$^1$H-NMR (400 MHz, DMSO-$d_6$): δ [ppm] = 12.8 (s, 1H), 10.8 (s, 1H), 7.99 (d, 1H), 7.92 (d, 2H), 7.79-7.71 (m, 4H), 7.50 (s, 1H), 6.53 (s, 1H), 5.88-5.82 (m, 1H), 3.69 (s, 3H), 3.19 (s, 3H), 2.23-2.13 (m, 1H), 2.02-1.92 (m, 1H), 1.82-1.70 (m, 2H), 1.52-1.42 (m, 2H), 1.40-1.16 (m, 5H).

**Beispiel 153**

4-({2-[4-(5-Chlor-2-cyanophenyl)-5-methoxy-2-oxopyridin-1(2*H*)-yl]-3-(*cis*-4-methoxycyclohexyl)propanoyl}amino)benzoesäure (Enantiomer 1)

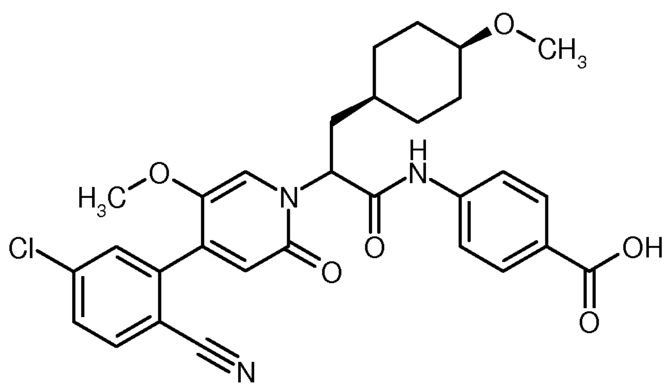

Enantiomerentrennung von 1.24 g des Racemates aus Beispiel 152 ergab 562 mg der Titelverbindung Beispiel 153 (Enantiomer 1): Chirale HPLC: $R_t$ = 3.18 min; 99% ee.

Trennmethode (SFC): Säule: AD-H 5 µm 250 mm x 20 mm; Eluent: 80% Kohlendioxid, 20% 2-Propanol; Temperatur: 40°C; Fluss: 80 ml/min; Druck: 100 bar; UV-Detektion: 210 nm.

Analytik (SFC): Säule: AD-3 5 µm 250 mm x 4.6 mm; Eluent: 95-50% Kohlendioxid, 5-50% 2-Propanol; Temperatur: 40°C; Fluss: 3 ml/min; UV-Detektion: 220 nm.

## Beispiel 154

4-({2-[4-(5-Chlor-2-cyanophenyl)-5-methoxy-2-oxopyridin-1(2*H*)-yl]-3-(*cis*-4-methoxycyclohexyl)propanoyl}amino)benzoesäure (Enantiomer 2)

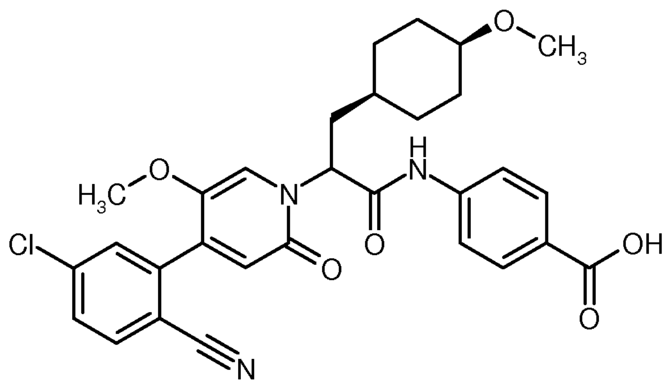

Enantiomerentrennung von 1.24 g des Racemates aus Beispiel 152 ergab 566 mg der Titelverbindung Beispiel 154 (Enantiomer 2): Chirale HPLC: $R_t$ = 3.82 min; 98% ee.

Trennmethode (SFC): Säule: AD-H 5 µm 250 mm x 20 mm; Eluent: 80% Kohlendioxid, 20% 2-Propanol; Temperatur: 40°C; Fluss: 80 ml/min; Druck: 100 bar; UV-Detektion: 210 nm.

Analytik (SFC): Säule: AD-3 5 µm 250 mm x 4.6 mm; Eluent: 95-50% Kohlendioxid, 5-50% 2-Propanol; Temperatur: 40°C; Fluss: 3 ml/min; UV-Detektion: 220 nm.

## Beispiel 155

4-({2-[4-(5-Chlor-2-cyanophenyl)-5-methoxy-2-oxopyridin-1(2*H*)-yl]-3-(*trans*-4-methoxycyclohexyl)propanoyl} amino)benzoesäure (Racemat)

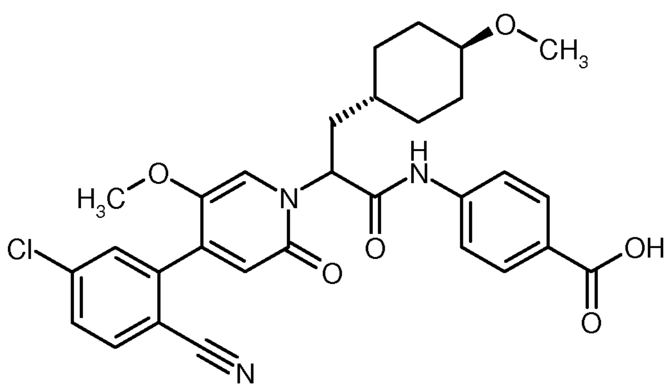

Gemäß allgemeiner Methode 4 wurden 1.10 g (1.86 mmol) 4-({2-[4-(5-Chlor-2-cyanophenyl)-5-methoxy-2-oxopyridin-1(2*H*)-yl]-3-(*trans*-4-methoxycyclohexyl)propanoyl}amino)benzoesäureethylester (Racemat) in 67 ml Ethanol/Wasser (2.6/1) mit 3.09 g (9.48 mmol) Cäsiumcarbonat zur Reaktion gebracht. Nach vollständigem Umsatz wurde mit Salzsäure (1N) auf pH 5-6 eingestellt und für 30 min nachgerührt. Der Niederschlag wurde abgesaugt, mit wenig Wasser gewaschen, im Hochvakuum getrocknet. Ausbeute: 690 mg (89% Reinheit, 59% d. Th.)

100 mg des Rohproduktes wurden mittels präparativer HPLC [Säule: Chromatorex C18, 10 μm, 125 mm x 30 mm, Eluent: Acetonitril/0.1% Ameisensäure-Gradient (0 bis 3 min 10% Acetonitril, bis 35 min 90% Acetonitril und weitere 3 min 90% Acetonitril)] aufgereinigt und lieferten die Titelverbindung. Ausbeute: 30 mg (3% d. Th.)
LC/MS [Methode 1]: $R_t$ = 1.00 min; MS (ESIpos): m/z = 564 $(M+H)^+$,
$^1$H-NMR (400 MHz, DMSO-$d_6$): δ [ppm] = 12.8 (s, 1H), 10.8 (s, 1H), 7.99 (d, 1H), 7.91 (d, 2H), 7.77-7.71 (m, 4H), 7.49 (s, 1H), 6.54 (s, 1H), 5.87-5.81 (m, 1H), 3.69 (s, 3H), 3.20 (s, 3H), 3.07-2.99 (m, 1H), 2.24-2.15 (m, 1H), 2.00-1.88 (m, 3H), 1.85-1.73 (m, 2H), 1.15-0.88 (m, 5H).

## Beispiel 156

4-({2-[4-(5-Chlor-2-cyanophenyl)-5-methoxy-2-oxopyridin-1(2*H*)-yl]-3-(*trans*-4-methoxycyclohexyl)propanoyl}amino)benzoesäure (Enantiomer 1)

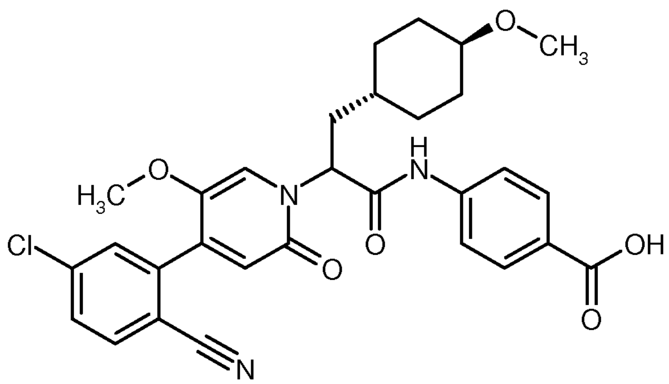

Enantiomerentrennung von 586 mg des Racemates aus Beispiel 155 ergab nach weiterer präparativer HPLC 145 mg der Titelverbindung Beispiel 156 (Enantiomer 1): Chirale HPLC: $R_t$ = 5.62 min; 99% ee.

Trennmethode (SFC): Säule: AZ-H 5 μm 250 mm x 20 mm; Eluent: 75% Kohlendioxid, 25% Ethanol; Temperatur: 40°C; Fluss: 100 ml/min; Druck: 100 bar; UV-Detektion: 210 nm.

Analytik (SFC): Säule: AZ-H 5 μm 250 mm x 4.6 mm; Eluent: 70% Kohlendioxid, 30% Ethanol; Temperatur: 40°C; Fluss: 3 ml/min; UV-Detektion: 220 nm.

## Beispiel 157

4-({2-[4-(5-Chlor-2-cyanophenyl)-5-methoxy-2-oxopyridin-1(2*H*)-yl]-3-(*trans*-4-methoxycyclohexyl)propanoyl}amino)benzoesäure (Enantiomer 2)

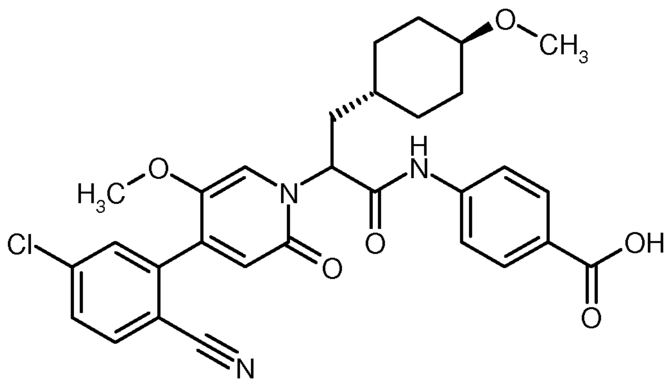

Enantiomerentrennung von 586 mg des Racemates aus Beispiel 155 ergab nach weiterer präparativer HPLC 110 mg der Titelverbindung Beispiel 157 (Enantiomer 2): Chirale HPLC: $R_t$ = 8.17 min; 99% ee.

Trennmethode (SFC): Säule: AZ-H 5 µm 250 mm x 20 mm; Eluent: 75% Kohlendioxid, 25% Ethanol; Temperatur: 40°C; Fluss: 100 ml/min; Druck: 100 bar; UV-Detektion: 210 nm.

Analytik (SFC): Säule: AZ-H 5 µm 250 mm x 4.6 mm; Eluent: 70% Kohlendioxid, 30% Ethanol; Temperatur: 40°C; Fluss: 3 ml/min; UV-Detektion: 220 nm.

## Beispiel 158

2-[4-(5-Chlor-2-cyanophenyl)-5-methoxy-2-oxopyridin-1(2*H*)-yl]-3-cyclobutyl-*N*-[4-(2-oxo-2,3-dihydro-1,3-oxazol-5-yl)phenyl]propanamid (Racemat)

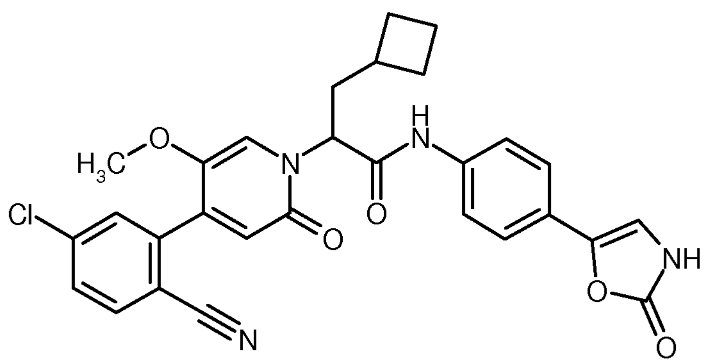

Nach der allgemeinen Methode 1 wurden 103 mg (94% Reinheit, 0.25 mmol) 2-[4-(5-Chlor-2-cyanophenyl)-5-methoxy-2-oxopyridin-1(2*H*)-yl]-3-cyclobutylpropansäure (Racemat) mit 55 mg (0.28 mmol, 1.1 eq.) 5-(4-Aminophenyl)-1,3-oxazol-2(3*H*)-on umgesetzt. Das Rohprodukt wurde mittels präparativer HPLC (Reprosil C18, Wasser-Acetonitril-Gradient) aufgereinigt. Ausbeute: 36 mg (27% d. Th.)
LC/MS [Methode 8]: $R_t$ = 1.30 min; MS (ESIneg): m/z = 543 $(M-H)^-$,
$^1$H-NMR (400 MHz, DMSO-$d_6$): $\delta$ [ppm] = 10.78 (s, 1H), 10.59 (s, 1H), 8.00 (d, 1H), 7.76-7.70 (m, 2H), 7.68 (d, 2H), 7.52 (s, 1H), 7.46 (d, 2H), 7.40 (s, 1H), 6.51 (s, 1H), 5.68 (t, 1H), 3.69 (s, 3H), 2.30-2.17 (m, 3H), 2.01-1.88 (m, 2H), 1.84-1.61 (m, 4H).

## Beispiel 159

2-[4-(5-Chlor-2-cyanophenyl)-5-methoxy-2-oxopyridin-1(2*H*)-yl]-3-cyclobutyl-*N*-(2-methyl-3-oxo-2,3-dihydro-1*H*-indazol-6-yl)propanamid (Racemat)

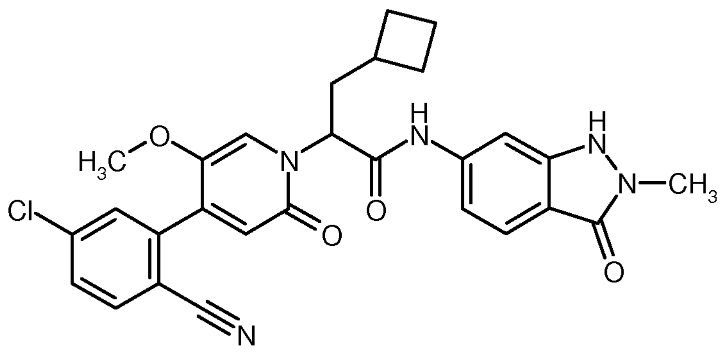

Nach der allgemeinen Methode 2 wurden 101 mg (0.16 mmol) 6-({2-[4-(5-Chlor-2-cyanophenyl)-5-methoxy-2-oxopyridin-1(2*H*)-yl]-3-cyclobutylpropanoyl}amino)-2-methyl-3-oxo-2,3-dihydro-1*H*-indazol-1-carbonsäure-*tert*.-butylester (Racemat) mit TFA verseift. Das Rohprodukt wurde mittels präparativer HPLC (Reprosil C18, Wasser-Acetonitril-Gradient) aufgereinigt. Ausbeute: 52 mg (62% d. Th.)
LC/MS [Methode 1]: $R_t$ = 0.95 min; MS (ESIpos): m/z = 532 $(M+H)^+$,
$^1$H-NMR (400 MHz, DMSO-$d_6$): δ [ppm] = 10.73 (s, 1H), 10.21 (s, 1H), 8.00 (d, 1H), 7.80-7.70 (m, 3H), 7.57 (d, 1H), 7.53 (s, 1H), 7.18 (dd, 1H), 6.52 (s, 1H), 5.70 (t, 1H), 3.70 (s, 3H), 2.30-2.17 (m, 3H), 2.01-1.88 (m, 2H), 1.84-1.61 (m, 4H).

**Beispiel 160**

4-({2-[4-(5-Chlor-2-cyanophenyl)-5-methoxy-2-oxopyridin-1(2*H*)-yl]-3-methylbutanoyl}-amino)benzoesäure (Racemat)

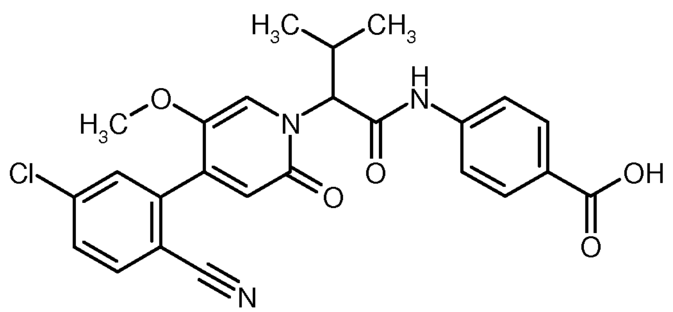

Nach der allgemeinen Methode 2 wurden 95 mg (177 μmol) 4-({2-[4-(5-Chlor-2-cyanophenyl)-5-methoxy-2-oxopyridin-1(2*H*)-yl]-3-methylbutanoyl}amino)benzoesäure-*tert*.-butylester (Racemat) mit TFA verseift. Das Rohprodukt wurde durch präparative HPLC (Säule: Chromatorex 125 mm x 30 mm, 10 μm, Eluent: 0.1% wässrige Ammoniumformiat-Lösung und Acetonitril, Gradient 30% Acetonitril bis 70% Acetonitril) aufgereinigt. Ausbeute: 28 mg (33% d. Th.)
LC/MS [Methode 1]: $R_t$ = 1.00 min; MS (ESIpos): m/z = 480 $(M+H)^+$,
$^1$H-NMR (400 MHz, DMSO-$d_6$): δ [ppm] = 11.0 (s, 1H), 8.00 (d, 1H), 7.89 (d, 2H), 7.78-7.71 (m, 4H), 7.65 (s, 1H), 6.56 (s, 1H), 5.52 (d, 1H), 3.69 (s, 3H), 1.08 (d, 3H), 0.82 (d, 3H).

**Beispiel 161**

4-({2-[4-(5-Chlor-2-cyanophenyl)-5-methoxy-2-oxopyridin-1(2*H*)-yl]-3-methylbutanoyl}amino)-2-methylbenzoesäure (Racemat)

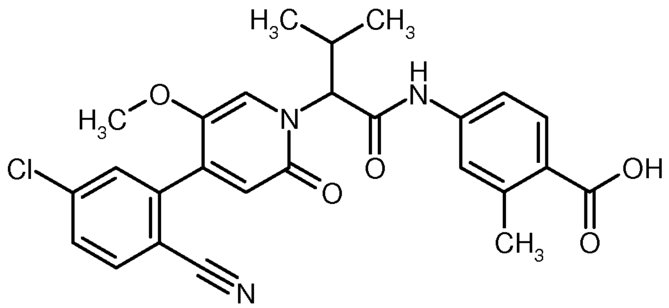

105 mg (207 µmol) 4-({2-[4-(5-Chlor-2-cyanophenyl)-5-methoxy-2-oxopyridin-1(2*H*)-yl]-3-methylbutanoyl}amino)-2-methylbenzoesäuremethylester (Racemat) wurden in 4.0 ml Methanol gelöst. Man setzte 0.83 ml einer 1N Natriumhydroxid-Lösung hinzu und erhitzte 1 h auf Rückfluss. Das Reaktionsgemisch wurde dann im Vakuum eingeengt, der Rückstand in Wasser aufgenommen und mit 1N wässriger Salzsäure angesäuert. Man extrahierte mit Essigsäureethylester, trocknete die organische Phase über Natriumsulfat und entfernte das Lösungsmittel im Vakuum. Der Rückstand wurde durch präparative HPLC (Säule: Chromatorex 125 mm x 30 mm, 10 µm, Eluent: 0.1% wässrige Ammoniumformiat-Lösung und Acetonitril, Gradient 30% Acetonitril bis 70% Acetonitril) aufgereinigt. Das isolierte Produkt wurde zuletzt mit Wasser verrührt und abgesaugt. Ausbeute: 15 mg (15% d. Th.)
LC/MS [Methode 1]: $R_t$ = 1.03 min; MS (ESIpos): m/z = 494 $(M+H)^+$,
$^1$H-NMR (400 MHz, DMSO-$d_6$): δ [ppm] = 12.6 (br. s, 1H), 10.9 (s, 1H), 8.00 (d, 1H), 7.84 (d, 1H), 7.76-7.71 (m, 2H), 7.64-7.60 (m, 2H), 7.57 (dd, 1H), 6.55 (s, 1H), 5.51 (d, 1H), 3.69 (s, 3H), 2.51 (s, 3H), 1.08 (d, 3H), 0.82 (d, 3H).

## Beispiel 162

6-({2-[4-(5-Chlor-2-cyanophenyl)-5-methoxy-2-oxopyridin-1(2*H*)-yl]-3-methylbutanoyl}amino)-1*H*-benzimidazol-2-carbonsäure (Racemat)

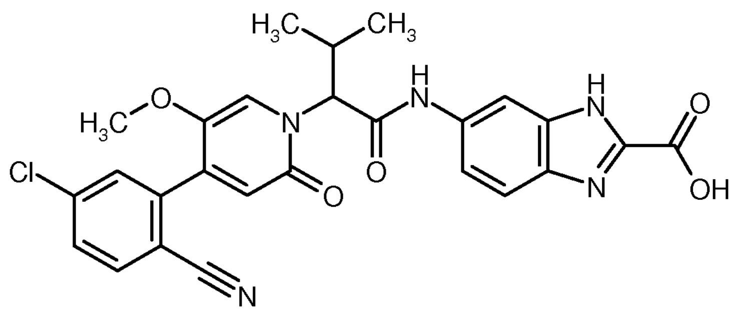

Nach der allgemeinen Methode 3 wurden 130 mg (237 µmol) 6-({2-[4-(5-Chlor-2-cyanophenyl)-5-methoxy-2-oxopyridin-1(2*H*)-yl]-3-methylbutanoyl}amino)-1*H*-benzimidazol-2-carbonsäure-ethylester (Racemat) mit Lithiumhydroxid verseift. Ausbeute: 56 mg (44% d. Th.)
LC/MS [Methode 1]: $R_t$ = 0.83 min; MS (ESIpos): m/z = 520 $(M+H)^+$,
$^1$H-NMR (400 MHz, DMSO-$d_6$): δ [ppm] = 10.9 (s, 1H), 8.20 (s, 1H), 8.14 (s, 1H), 8.00 (d, 1H), 7.76 (d, 1H), 7.75-7.72 (m, 1H), 7.68 (s, 1H), 7.63 (d, 1H), 7.49 (dd, 1H), 5.53 (d, 1H), 3.70 (s, 3H), 2.61-2.55 (m, 1H), 1.10 (d, 3H), 0.83 (d, 3H).

## Beispiel 163

2-[4-(5-Chlor-2-cyanophenyl)-5-methoxy-2-oxopyridin-1(2*H*)-yl]-4-methoxy-*N*-(2-oxo-2,3-dihydro-1*H*-benzimidazol-5-yl)butanamid (Racemat)

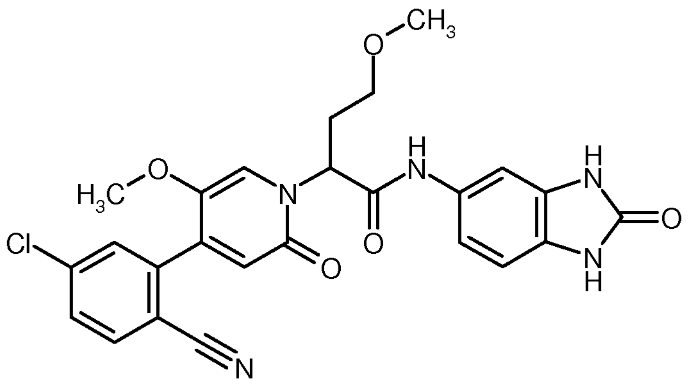

Nach der allgemeinen Methode 5 wurden 70 mg (186 µmol) 2-[4-(5-Chlor-2-cyanophenyl)-5-methoxy-2-oxopyridin-1(2*H*)-yl]-4-methoxybutansäure (Racemat) mit 27.7 mg (186 µmol, 1.0 eq.) 5-Aminobenzimidazolon, 26.4 mg (186 µmol) Oxima und 28.9 µl (186 µmol) DIC in 3.9 ml Dimethylformamid umgesetzt. Das Rohprodukt wurde mittels präparativer HPLC (Säule: Chromatorex 125 mm x 30 mm, 10 µm, Eluent: Wasser/0.1% Ameisensäure und Acetonitril/0.1% Ameisensäure, Gradient 10% Acetonitril bis 90% Acetonitril) gereinigt und im Anschluss basisch extrahiert (wässrige Natriumhydroxid-Lösung/Essigsäureethylester). Ausbeute: 44 mg (47% d. Th.)
LC/MS [Methode 2]: $R_t$ = 2.22 min; MS (ESIpos): m/z = 508 $(M+H)^+$,
$^1$H-NMR (400 MHz, DMSO-$d_6$): δ [ppm] = 10.6 (s, 1H), 10.5 (s, 1H), 10.4 (s, 1H), 8.02-7.98 (m, 1H), 7.76-7.71 (m, 2H), 7.54 (s, 1H), 7.45 (d, 1H), 7.09 (dd, 1H), 6.84 (d, 1H), 6.52 (s, 1H), 5.75 (dd, 1H), 3.68 (s, 3H), 3.43-3.23 (m, 2H), 3.21 (s, 3H), 2.44-2.29 (m, 2H).

**Beispiel 164**

2-[4-(5-Chlor-2-cyanophenyl)-5-methoxy-2-oxopyridin-1(2*H*)-yl]-4-methoxy-*N*-(3-oxo-2,3-dihydro-1*H*-indazol-6-yl)butanamid (Racemat)

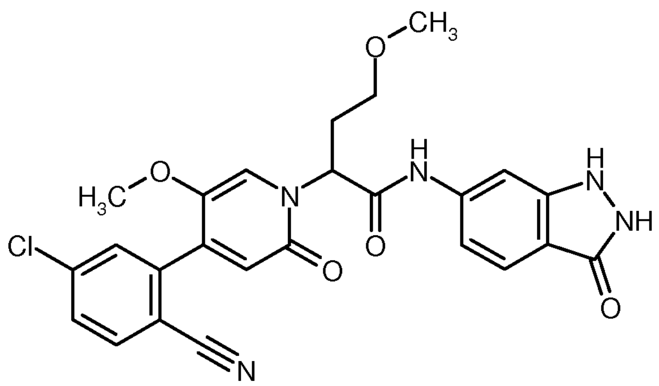

Nach der allgemeinen Methode 1 wurden 150 mg (377 µmol) 2-[4-(5-Chlor-2-cyanophenyl)-5-methoxy-2-oxopyridin-1(2*H*)-yl]-4-methoxybutansäure (Racemat) mit 65 mg (438 µmol, 1.1 eq.) 6-Amino-1*H*-indazol-3(2*H*)-on umgesetzt. Das Rohprodukt wurde mittels Flash-Chromatographie (Kieselgel-50, Dichlormethan-Methanol-Gemische) aufgereinigt. Ausbeute: 105 mg (52% d. Th.)
LC/MS [Methode 1]: $R_t$ = 0.79 min; MS (ESIpos): m/z = 508 $(M+H)^+$,
$^1$H-NMR (400 MHz, DMSO-$d_6$): δ [ppm] = 11.1 (br. s, 1H), 10.6-10.6 (m, 2H), 8.01-7.98 (m, 1H), 7.86 (d, 1H), 7.75-7.72 (m, 2H), 7.55-7.51 (m, 2H), 7.08 (dd, 1H), 6.53 (s, 1H), 5.79 (dd, 1H), 3.69 (s, 3H), 3.43-3.36 (m, 1H), 3.31-3.26 (m, 1H), 3.21 (s, 3H), 2.46-2.34 (m, 2H).

**Beispiel 165**

2-[4-(5-Chlor-2-cyanophenyl)-5-methoxy-2-oxopyridin-1(2*H*)-yl]-4-methoxy-*N*-(2-methyl-3-oxo-2,3-dihydro-1*H*-indazol-6-yl)butanamid (Racemat)

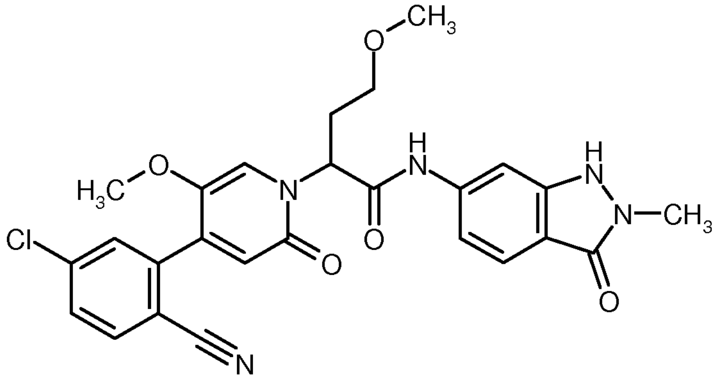

Nach der allgemeinen Methode 2 wurden 225 mg (362 μmol) 6-({2-[4-(5-Chlor-2-cyanophenyl)-5-methoxy-2-oxopyridin-1(2*H*)-yl]-4-methoxybutanoyl}amino)-2-methyl-3-oxo-2,3-dihydro-1*H*-indazol-1-carbonsäure-*tert*-butylester (Racemat) mit TFA verseift. Ausbeute: 205 mg (92% Reinheit, 100% d. Th.).
LC/MS [Methode 1]: $R_t$ = 0.82 min; MS (ESIpos): m/z = 522 $(M+H)^+$,
$^1$H-NMR (400 MHz, DMSO-$d_6$): δ [ppm] = 10.7 (s, 1H), 10.2 (s, 1H), 8.02-7.97 (m, 1H), 7.77 (d, 1H), 7.75-7.72 (m, 2H), 7.56 (d, 1H), 7.52 (s, 1H), 7.20 (dd, 1H), 6.53 (s, 1H), 5.77 (dd, 1H), 3.69 (s, 3H), 3.44-3.37 (m, 1H), 3.32 (s, 3H), 3.31-3.26 (m, 1H), 3.21 (s, 3H), 2.47-2.36 (m, 2H).

**Beispiel 166**

2-[4-(5-Chlor-2-cyanophenyl)-5-methoxy-2-oxopyridin-1(2*H*)-yl]-*N*-(1*H*-indazol-5-yl)-4-methoxybutanamid (Racemat)

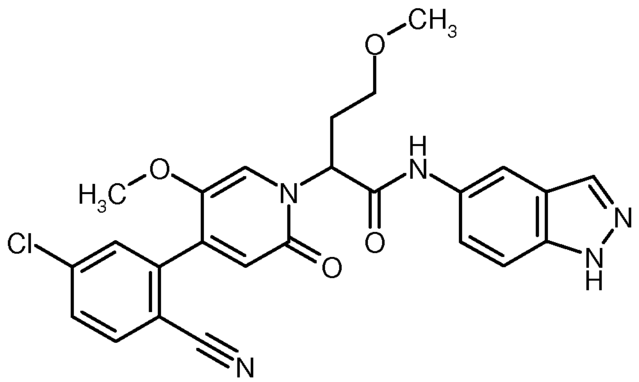

Nach der allgemeinen Methode 1 wurden 150 mg (398 μmol) 2-[4-(5-Chlor-2-cyanophenyl)-5-methoxy-2-oxopyridin-1(2*H*)-yl]-4-methoxybutansäure (Racemat) mit 58.3 mg (438 μmol, 1.1 eq.) 5-Aminoindazol umgesetzt. Das Rohprodukt wurde mittels präparativer HPLC (Säule: Chromatorex 125 mm x 30 mm, 10 μm, Eluent: Wasser und Acetonitril, Gradient 10% Acetonitril bis 90% Acetonitril) gereinigt. Ausbeute: 88 mg (42% d. Th.)
LC/MS [Methode 1]: $R_t$ = 0.87 min; MS (ESIpos): m/z = 492 $(M+H)^+$,
$^1$H-NMR (400 MHz, DMSO-$d_6$): δ [ppm] = 13.0 (s, 1H), 10.5 (s, 1H), 8.14 (s, 1H), 8.02 (s, 1H), 8.01-7.98 (m, 1H), 7.75-7.70 (m, 2H), 7.56 (s, 1H), 7.49 (s, 2H), 6.53 (s, 1H), 5.80 (dd, 1H), 3.69 (s, 3H), 3.44-3.37 (m, 1H), 3.34-3.26 (m, 1H), 3.22 (s, 3H), 2.48-2.31 (m, 2H).

## Beispiel 167

2-[4-(5-Chlor-2-cyanophenyl)-5-methoxy-2-oxopyridin-1(2*H*)-yl]-*N*-(3-chlor-1*H*-indazol-5-yl)-4-methoxybutanamid (Racemat)

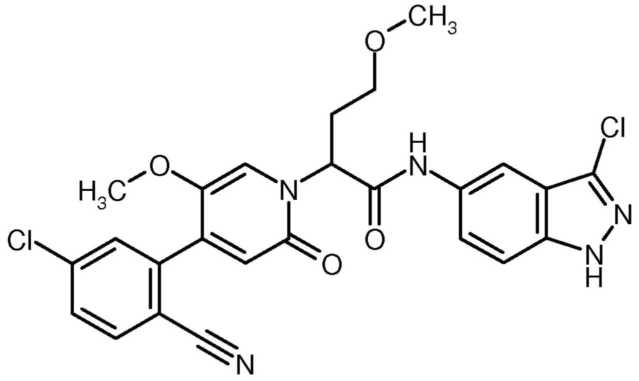

Nach der allgemeinen Methode 1 wurden 170 mg (451 µmol) 2-[4-(5-Chlor-2-cyanophenyl)-5-methoxy-2-oxopyridin-1(2*H*)-yl]-4-methoxybutansäure (Racemat) mit 92.4 mg (90% Reinheit, 496 µmol, 1.1 eq.) 5-Amino-3-chlor-1*H*-indazol umgesetzt. Das Rohprodukt wurde mittels Flash-Chromatographie (Kieselgel-50, Cyclohexan-Essigsäureethylester-Gemische) aufgereinigt. Ausbeute: 113 mg (48% d. Th.)
LC/MS [Methode 1]: $R_t$ = 0.97 min; MS (ESIpos): m/z = 526 $(M+H)^+$,
$^1$H-NMR (400 MHz, DMSO-$d_6$): $\delta$ [ppm] = 13.3 (s, 1H), 10.6 (s, 1H), 8.16 (s, 1H), 8.02-7.98 (m, 1H), 7.76-7.70 (m, 2H), 7.55 (s, 3H), 6.53 (s, 1H), 5.79 (dd, 1H), 3.70 (s, 3H), 3.45-3.37 (m, 1H), 3.31-3.26 (m, 1H), 3.22 (s, 3H), 2.48-2.31 (m, 2H).

## Beispiel 168

6-({2-[4-(5-Chlor-2-cyanophenyl)-5-methoxy-2-oxopyridin-1(2*H*)-yl]-4-methoxybutanoyl}amino)-1*H*-indol-2-carbonsäure (Racemat)

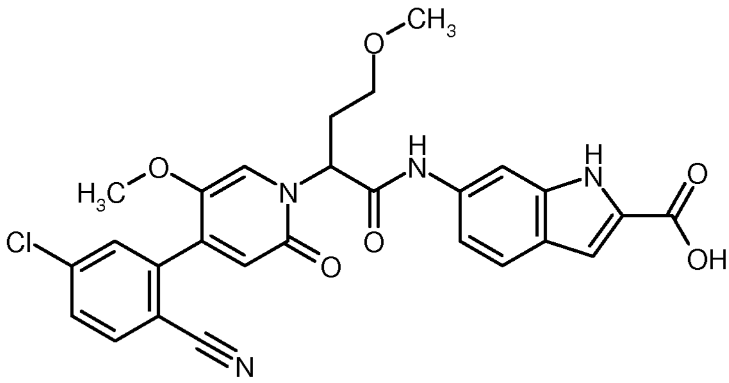

Nach der allgemeinen Methode 3 wurden 80 mg (142 µmol) 6-({2-[4-(5-Chlor-2-cyanophenyl)-5-methoxy-2-oxopyridin-1(2*H*)-yl]-4-methoxybutanoyl}amino)-1*H*-indol-2-carbonsäureethylester (Racemat) mit Lithiumhydroxid bei RT verseift. Ausbeute: 50 mg (66% d. Th.)
LC/MS [Methode 1]: $R_t$ = 0.92 min; MS (ESIpos): m/z = 535 $(M+H)^+$,
$^1$H-NMR (400 MHz, DMSO-$d_6$): $\delta$ [ppm] = 12.8 (br. s, 1H), 11.7-11.6 (m, 1H), 10.5 (s, 1H), 8.03-7.98 (m, 2H), 7.75-7.71 (m, 2H), 7.58-7.54 (m, 2H), 7.21 (dd, 1H), 7.04-7.02 (m, 1H), 6.52 (s, 1H), 5.80 (dd, 1H), 3.70 (s, 3H), 3.43-3.35 (m, 1H), (1H unter $H_2O$-Signal), 3.22 (s, 3H), 2.46-2.36 (m, 2H).

**Beispiel 169**

6-({2-[4-(5-Chlor-2-cyanophenyl)-5-methoxy-2-oxopyridin-1(2*H*)-yl]-4-methoxybutanoyl}amino)-5-methoxy-1*H*-indol-2-carbonsäure (Racemat)

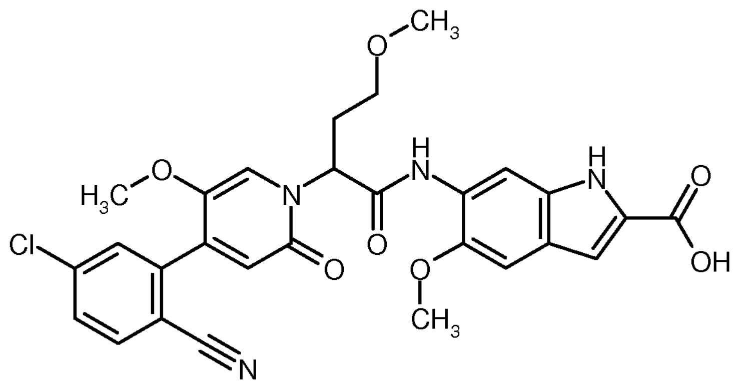

Nach der allgemeinen Methode 3 wurden 80 mg (135 μmol) 6-({2-[4-(5-Chlor-2-cyanophenyl)-5-methoxy-2-oxopyridin-1(2*H*)-yl]-4-methoxybutanoyl}amino)-5-methoxy-1*H*-indol-2-carbonsäureethylester (Racemat) mit Lithiumhydroxid bei RT verseift. Ausbeute: 63 mg (90% Reinheit, 74% d. Th.).
LC/MS [Methode 1]: $R_t$ = 0.93 min; MS (ESIpos): m/z = 565 $(M+H)^+$,
$^1$H-NMR (400 MHz, DMSO-$d_6$): δ [ppm] = 12.7 (br. s, 1H), 11.6 (s, 1H), 9.41 (s, 1H), 8.23 (s, 1H), 8.00 (d, 1H), 7.78-7.70 (m, 2H), 7.51 (s, 1H), 7.19 (s, 1H), 6.99-6.97 (m, 1H), 6.57 (s, 1H), 5.79 (dd, 1H), 3.86 (s, 3H), 3.69 (s, 3H), 3.45-3.35 (m, 2H), 3.24 (s, 3H), 2.44-2.31 (m, 2H).

**Beispiel 170**

4-({2-[5-Chlor-4-(5-chlor-2-cyanophenyl)-2-oxopyridin-1(2*H*)-yl]-4-methylpentanoyl}-amino)benzoesäure (Racemat)

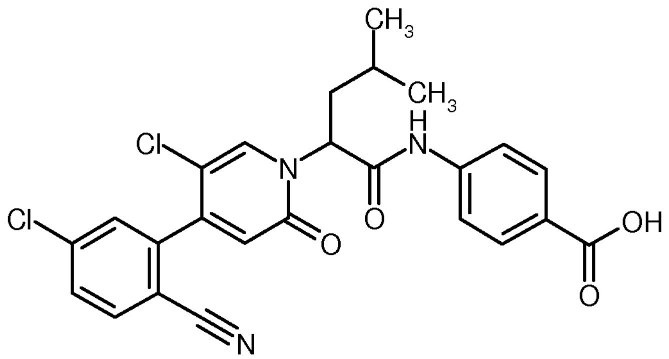

Nach der allgemeinen Methode 2 wurden 45 mg (0.08 mmol) 4-({2-[5-Chlor-4-(5-chlor-2-cyanophenyl)-2-oxopyridin-1(2*H*)-yl]-4-methylpentanoyl}amino)benzoesäure-*tert*.-butylester (Racemat) mit TFA verseift. Das Rohprodukt wurde mittels präparativer HPLC (Reprosil C18, Wasser-Acetonitril-Gradient) aufgereinigt. Ausbeute: 28 mg (90% Reinheit, 62% d. Th.)
LC/MS [Methode 1]: $R_t$ = 1.08 min; MS (ESIpos): m/z = 498 $(M+H)^+$,
$^1$H-NMR (400 MHz, DMSO-$d_6$): δ [ppm] = 12.79 (s, 1H), 10.89 (s, 1H), 8.22 (s, 1H), 8.07 (d, 1H), 7.92 (d, 2H), 7.84 (d, 1H), 7.79 (dd, 1H), 7.74 (d, 2H), 6.70 (s, 1H), 5.84 (dd, 1H), 2.32-2.22 (m, 1H), 1.93-1.82 (m, 1H), 1.48-1.35 (m, 1H), 0.94 (dd, 6H).

**Beispiel 171**

4-({2-[5-Chlor-4-(5-chlor-2-cyanophenyl)-2-oxopyridin-1(2*H*)-yl]-4-(trifluormethoxy)butanoyl}-amino)benzoesäure (Racemat)

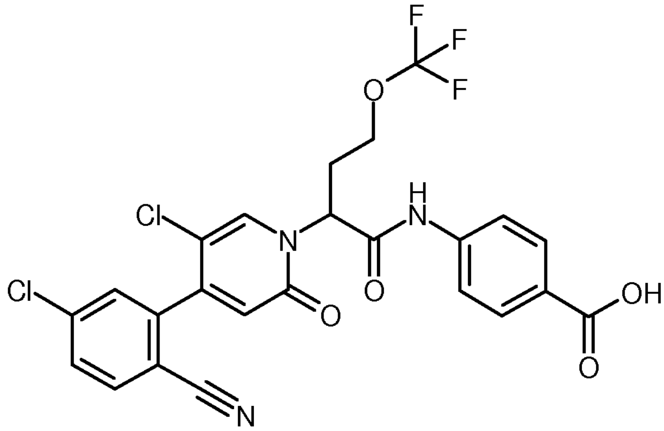

Nach der allgemeinen Methode 2 wurden 142 mg (81% Reinheit, 0.19 mmol) 4-({2-[5-Chlor-4-(5-chlor-2-cyanophenyl)-2-oxopyridin-1(2*H*)-yl]-4-(trifluormethoxy)butanoyl}amino)benzoesäure-*tert*.-butylester (Racemat) mit TFA verseift. Das Rohprodukt wurde mittels präparativer HPLC (Reprosil C18, Wasser-Acetonitril-Gradient) aufgereinigt. Ausbeute: 54 mg (52% d. Th.)
LC/MS [Methode 2]: $R_t$ = 3.32 min; MS (ESIpos): m/z = 554 $(M+H)^+$,
$^1$H-NMR (400 MHz, DMSO-$d_6$): $\delta$ [ppm] = 12.79 (s, 1H), 10.87 (s, 1H), 8.26 (s, 1H), 8.07 (d, 1H), 7.92 (d, 2H), 7.80 (dd, 1H), 7.77 (d, 1H), 7.74 (d, 2H), 6.71 (s, 1H), 5.81 (dd, 1H), 4.24-4.15 (m, 1H), 4.03-3.84 (m, 1H), 2.71-2.59 (m, 2H).

**Beispiel 172**

4-[(2-{4-[5-Chlor-2-(difluormethoxy)phenyl]-5-methoxy-2-oxopyridin-1(2*H*)-yl}-4-methylpentanoyl)amino]benzoesäure (Racemat)

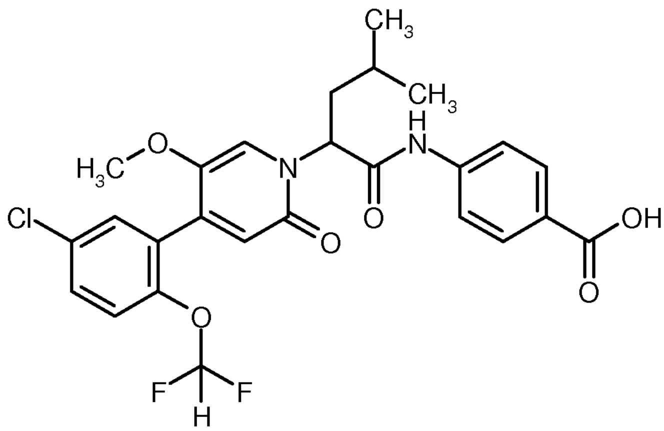

Nach der allgemeinen Methode 2 wurden 263 mg (88% Reinheit, 0.39 mmol) 4-[(2-{4-[5-Chlor-2-(difluormethoxy)phenyl]-5-methoxy-2-oxopyridin-1(2*H*)-yl}-4-methylpentanoyl)amino]benzoesäure-*tert*.-butylester (Racemat) mit TFA verseift. Das Rohprodukt wurde mittels präparativer HPLC (Reprosil C18, Wasser-Acetonitril-Gradient) aufgereinigt. Ausbeute: 140 mg (67% d. Th.)
LC/MS [Methode 1]: $R_t$ = 1.10 min; MS (ESIpos): m/z = 535 $(M+H)^+$,
$^1$H-NMR (400 MHz, DMSO-$d_6$): $\delta$ [ppm] = 12.78 (s, 1H), 10.84 (s, 1H), 7.91 (d, 2H), 7.76 (d, 2H), 7.58 (dd, 1H), 7.50 (d,

1H), 7.40 (s, 1H), 7.29 (d, 1H), 7.13 (t, 1H), 6.40 (s, 1H), 5.84 (dd, 1H), 3.63 (s, 3H), 2.22-2.12 (m, 1H), 1.92-1.82 (m, 1H), 1.49-1.36 (m, 1H), 0.94 (t, 6H).

**Beispiel 173**

4-[(2-{4-[5-Chlor-2-(difluormethoxy)phenyl]-5-methoxy-2-oxopyridin-1(2*H*)-yl}-4-methoxybutanoyl)amino]benzoesäure (Racemat)

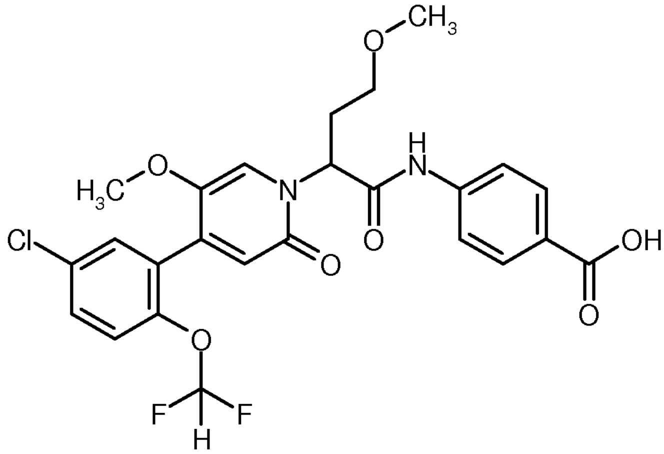

Nach der allgemeinen Methode 2 wurden 66 mg (0.11 mmol) 4-[(2-{4-[5-Chlor-2-(difluormethoxy)phenyl]-5-methoxy-2-oxopyridin-1(2*H*)-yl}-4-methoxybutanoyl)amino]benzoesäure-*tert*.-butylester (Racemat) mit TFA verseift. Das Rohprodukt wurde mittels präparativer HPLC (Reprosil C18, Wasser-Acetonitril-Gradient) aufgereinigt. Ausbeute: 31 mg (52% d. Th.)
LC/MS [Methode 1]: $R_t$ = 0.96 min; MS (ESIpos): m/z = 537 $(M+H)^+$,
$^1$H-NMR (400 MHz, DMSO-$d_6$): $\delta$ [ppm] = 12.73 (s, 1H), 10.73 (s, 1H), 7.90 (d, 2H), 7.76 (d, 2H), 7.57 (dd, 1H), 7.49 (d, 1H), 7.40 (s, 1H), 7.30 (d, 1H), 7.13 (t, 1H), 6.39 (s, 1H), 5.74 (dd, 1H), 3.63 (s, 3H), 3.42-3.34 (m, 1H), 3.30-3.24 (m, 1H), 3.20 (s, 3H), 2.45-2.34 (m, 2H).

**Beispiel 174**

2-{4-[5-Chlor-2-(difluormethoxy)phenyl]-5-methoxy-2-oxopyridin-1(2*H*)-yl}-4-methoxy-*N*-(2-methyl-3-oxo-2,3-dihydro-1*H*-indazol-6-yl)butanamid (Racemat)

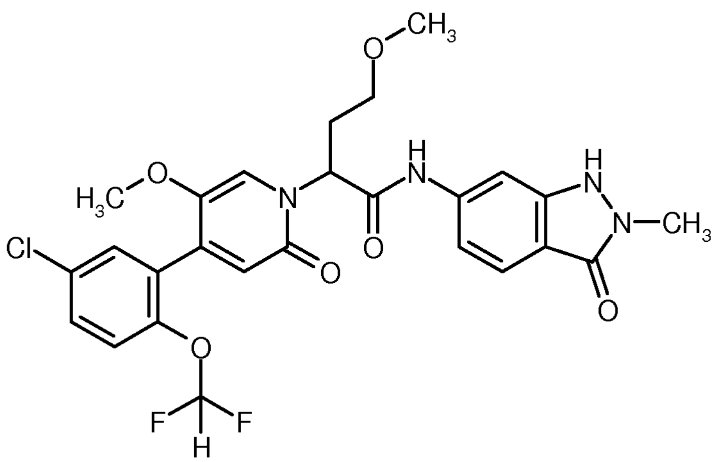

Nach der allgemeinen Methode 2 wurden 132mg (0.19 mmol) 6-[(2-{4-[5-Chlor-2-(difluormethoxy)phenyl]-5-methoxy-2-oxopyridin-1(2*H*)-yl}-4-methoxybutanoyl)amino]-2-methyl-3-oxo-2,3-dihydro-1*H*-indazol-1-carbonsäure-*tert*.-butylester (Racemat) mit TFA verseift. Das Rohprodukt wurde mittels präparativer HPLC (Reprosil C18, Wasser-Acetonitril-Gradient) aufgereinigt. Ausbeute: 48 mg (45% d. Th.)
LC/MS [Methode 1]: $R_t$ = 0.89 min; MS (ESIpos): m/z = 563 $(M+H)^+$,
$^1$H-NMR (400 MHz, DMSO-$d_6$): δ [ppm] = 10.65 (s, 1H), 10.19 (s, 1H), 7.77 (d, 1H), 7.61-7.51 (m, 2H), 7.49 (d, 1H), 7.42 (s, 1H), 7.33-7.25 (m, 2H), 7.19 (dd, 1H), 7.13 (t, 1H), 6.39 (s, 1H), 5.75 (dd, 1H), 3.63 (s, 3H), 3.32 (s, 3H), 3.20 (s, 3H), 2.43-2.30 (m, 2H).

**Beispiel 175**

2-{4-[5-Chlor-2-(difluormethoxy)phenyl]-5-methoxy-2-oxopyridin-1(2*H*)-yl}-4-methoxy-*N*-(2-oxo-2,3-dihydro-1*H*-benzimidazol-5-yl)butanamid (Racemat)

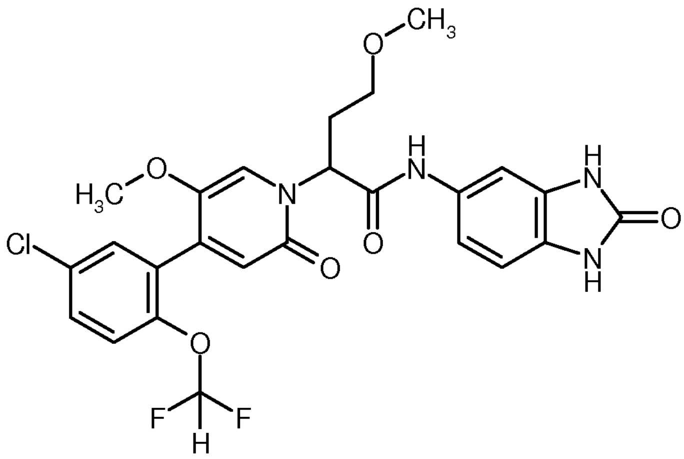

Nach der allgemeinen Methode 1 wurden 76 mg (82% Reinheit, 0.15 mmol) 2-{4-[5-Chlor-2-(difluormethoxy)phenyl]-5-methoxy-2-oxopyridin-1(2*H*)-yl}-4-methoxybutansäure (Racemat) mit 25 mg (0.17 μmol, 1.1 eq.) 5-Aminobenzimidazolon umgesetzt. Das Rohprodukt wurde mittels präparativer HPLC (Reprosil C18, Wasser-Acetonitril-Gradient) aufgereinigt. Ausbeute: 37 mg (45% d. Th.)
LC/MS [Methode 1]: $R_t$ = 0.86 min; MS (ESIpos): m/z = 549 $(M+H)^+$,
$^1$H-NMR (400 MHz, DMSO-$d_6$): δ [ppm] = 10.56 (s, 1H), 10.50 (s, 1H), 10.30 (s, 1H), 7.57 (dd, 1H), 7.48 (d, 1H), 7.46-7.40 (m, 2H), 7.30 (d, 1H), 7.12 (t, 1H), 7.09 (dd, 1H), 6.84 (d, 1H), 6.37 (s, 1H), 5.73 (dd, 1H), 3.62 (s, 3H), 3.34-3.23 (m, 2H), 3.20 (s, 3H), 2.40-2.25 (m, 2H).

**Beispiel 176**

4-{[2-{4-[5-Chlor-2-(difluormethoxy)phenyl]-5-methoxy-2-oxopyridin-1(2*H*)-yl}-4-(trifluormethoxy)butanoyl]amino}benzoesäure (Racemat)

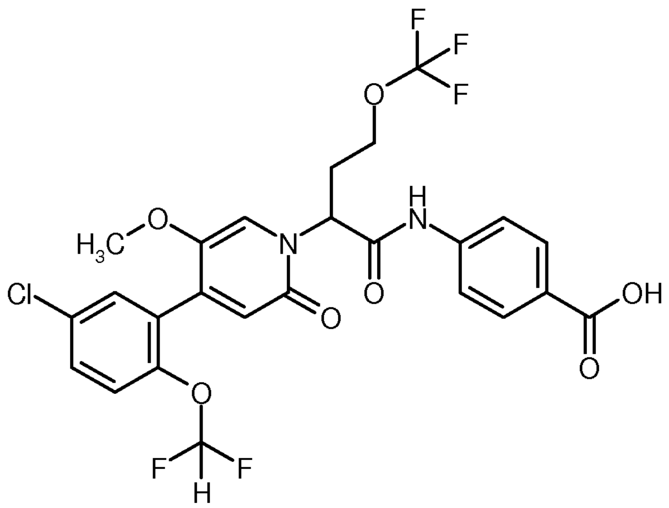

Nach der allgemeinen Methode 2 wurden 195 mg (0.29 mmol) 4-{[2-{4-[5-Chlor-2-(difluormethoxy)phenyl]-5-methoxy-2-oxopyridin-1(2*H*)-yl}-4-(trifluormethoxy)butanoyl]amino}-benzoesäure-*tert.*-butylester (Racemat) mit TFA verseift. Das Rohprodukt wurde mittels präparativer HPLC (Reprosil C18, Wasser-Acetonitril-Gradient) aufgereinigt. Ausbeute: 84 mg (50% d. Th.)
LC/MS [Methode 1]: $R_t$ = 1.07 min; MS (ESIpos): m/z = 591 $(M+H)^+$,
$^1$H-NMR (400 MHz, DMSO-$d_6$): $\delta$ [ppm] = 12.76 (s, 1H), 10.75 (s, 1H), 7.91 (d, 2H), 7.75 (d, 2H), 7.58 (dd, 1H), 7.46 (d, 1H), 7.40 (s, 1H), 7.30 (d, 1H), 7.11 (t, 1H), 6.41 (s, 1H), 5.79 (t, 1H), 4.19-4.11 (m, 1H), 4.04-3.95 (m, 1H), 3.63 (s, 3H), 2.65-2.57 (m, 2H).

**Beispiel 177**

4-[(2-{4-[5-Chlor-2-(difluormethoxy)phenyl]-5-methoxy-2-oxopyridin-1(2*H*)-yl}-3-[(2*R*)-tetrahydro-2*H*-pyran-2-yl]propanoyl)amino]benzoesäure (Gemisch enantiomerenreiner Diastereomere 1 und 2)

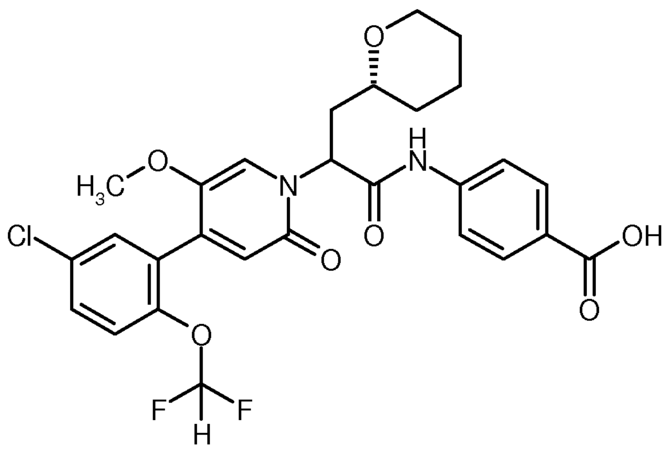

Nach der allgemeinen Methode 2 wurden 67 mg (0.11 mmol) 4-[(2-{4-[5-Chlor-2-(difluormethoxy)phenyl]-5-methoxy-2-oxopyridin-1(2*H*)-yl}-3-[(2*R*)-tetrahydro-2*H*-pyran-2-yl]propanoyl)amino]benzoesäure-*tert.*-butylester (Gemisch enantiomerenreiner Diastereomere 1 und 2) mit TFA verseift. Das Rohprodukt wurde mittels präparativer HPLC (Reprosil C18, Wasser-Acetonitril-Gradient) aufgereinigt. Ausbeute: 25 mg (41% d. Th.)
LC/MS [Methode 1]: $R_t$ = 1.05 min; MS (ESIpos): m/z = 577 $(M+H)^+$,
$^1$H-NMR (400 MHz, DMSO-$d_6$): $\delta$ [ppm] = 12.75 (s, 1H), 10.74/10.65 (2x s, 1H), 7.94-7.86 (m, 2H), 7.81-7.72 (m, 2H), 7.59/7.56 (2x t, 1H), 7.50/7.48 (2x d, 1H), 7.42/7.38 (2x s, 1H), 7.33-7.27 (m, 1H), 7.13/7.12 (2x t, 1H), 6.38/6.37 (2x s, 1H), 5.80/5.72 (t/dd, 1H), 3.90-3.78 (m, 1H), 3.63/3.62 (2x s, 3H), 3.29-3.03 (m, 2H), 2.39-2.09 (m, 2H), 1.79-1.70 (m,

1H), 1.67-1.54 (m, 1H), 1.48-1.18 (m, 4H).

**Beispiel 178**

4-({2-[4-(5-Chlor-2-cyanophenyl)-5-methoxy-2-oxopyridin-1(2*H*)-yl]butanoyl}amino)-2-fluorbenzoesäure (Racemat)

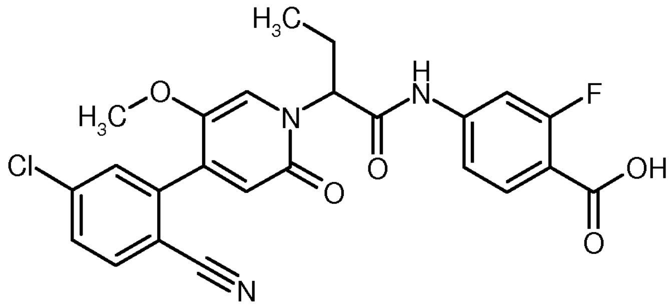

Nach der allgemeinen Methode 2 wurden 249 mg (0.43 mmol) 4-({2-[4-(5-Chlor-2-cyanophenyl)-5-methoxy-2-oxopyridin-1(2*H*)-yl]butanoyl}amino)-2-fluorbenzoesäure-*tert*.-butylester (Racemat) umgesetzt. Das Rohprodukt wurde mittels präparativer HPLC (Wasser-Acetonitril-0.1% Ameisensäure-Gradient) aufgereinigt. Ausbeute: 145 mg (65% d. Th.)
LC/MS [Methode 8]: $R_t$ = 1.19 min; MS (ESIpos): m/z = 484 $(M+H)^+$,
$^1$H-NMR (400 MHz, DMSO-$d_6$): δ [ppm] = 12.99 (br. s, 1H), 10.94 (s, 1H), 8.02-7.97 (m, 1H), 7.86 (t, 1H), 7.77-7.67 (m, 3H), 6.54 (s, 1H), 5.60 (dd, 1H), 3.69 (s, 3H), 3.26-2.11 (m, 2H), 0.91 (t, 3H),
$^{19}$F-NMR (376.54 MHz, DMSO-$d_6$): δ [ppm] = -107.7.

**Beispiel 179**

4-({(2*S*)-2-[4-(5-Chlor-2-cyanophenyl)-5-methoxy-2-oxopyridin-1(2*H*)-yl]butanoyl}amino)-2-fluorbenzoesäure (Enantiomer 2)

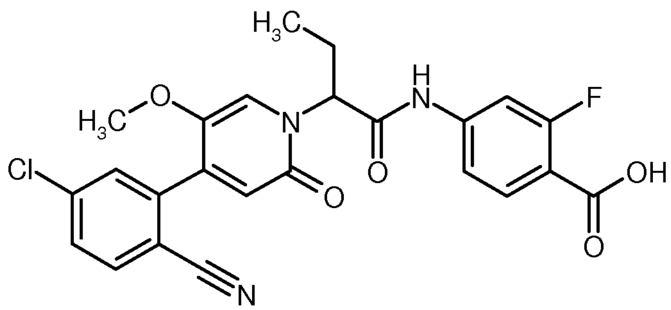

Enantiomerentrennung von 135 mg des Racemates aus Beispiel 178 ergab 36 mg der Titelverbindung Beispiel 179 (Enantiomer 2): Chirale SFC: $R_t$ = 2.67 min; 99% ee.
Trennmethode (SFC): Säule: Chiralpak AZ-H 5μm 250 mm x 30mm; Eluent: 60% Kohlendioxid, 40% Ethanol; Temperatur: 40°C; Fluss: 100 ml/min; Druck: 80 bar; UV-Detektion: 220 nm.
Analytik: Säule: Chiralpak AZ-H 5μ 250 mm x 4.6mm; Eluent: 60% Kohlendioxid, 40% Ethanol; Fluss: 3 ml/min; UV-Detektion: 220 nm.

**Beispiel 180**

4-({2-[4-(5-Chlor-2-cyanophenyl)-5-methoxy-2-oxopyridin-1(2*H*)-yl]butanoyl}amino)-3-fluorbenzoesäure (Racemat)

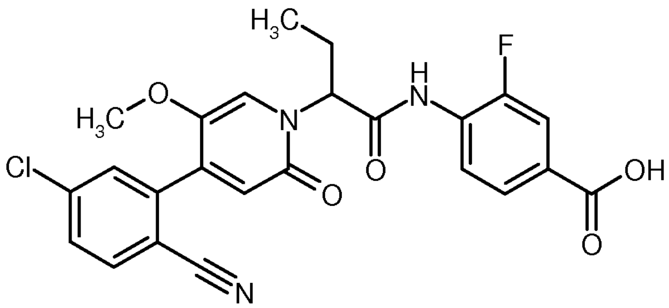

Nach der allgemeinen Methode 2 wurden 125 mg (0.23 mmol) 4-({2-[4-(5-Chlor-2-cyanophenyl)-5-methoxy-2-oxopyridin-1(2*H*)-yl]butanoyl}amino)-3-fluorbenzoesäure-*tert*.-butylester (Racemat) umgesetzt. Das Rohprodukt wurde mittels präparativer HPLC (Wasser-Acetonitril-0.1% Ameisensäure-Gradient) aufgereinigt. Ausbeute: 57 mg (51% d. Th.)
LC/MS [Methode 1]: $R_t$ = 0.94 min; MS (ESIpos): m/z = 484 $(M+H)^+$,
$^1$H-NMR (400 MHz, DMSO-$d_6$): δ [ppm] = 13.13 (br. s, 1H), 10.55 (s, 1H), 8.11 (t, 1H), 8.02-7.97 (m, 1H), 7.79-7.70 (m, 4H), 7.46 (s, 1H), 6.55 (s, 1H), 5.80 (dd, 1H), 3.68 (s, 3H), 2.27-2.10 (m, 2H), 0.91 (t, 3H),
$^{19}$F-NMR (376.54 MHz, DMSO-$d_6$): δ [ppm] = -123.9.

## Beispiel 181

4-({(2*S*)-2-[4-(5-Chlor-2-cyanophenyl)-5-methoxy-2-oxopyridin-1(2*H*)-yl]butanoyl}amino)-3-fluorbenzoesäure (Enantiomer 2)

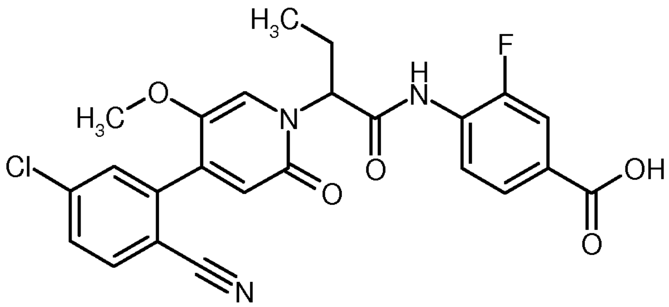

Enantiomerentrennung von 55 mg des Racemates aus Beispiel 180 ergab 27 mg der Titelverbindung Beispiel 181 (Enantiomer 2): Chirale SFC: $R_t$ = 7.07 min; >99% ee.
Trennmethode (SFC): Säule: AD-H 5 μm 250 mm x 20 mm; Eluent: 85% Kohlendioxid, 15% Ethanol; Temperatur: 40°C; Fluss: 100 ml/min; Druck: 100 bar; UV-Detektion: 210 nm.
Analytik: Säule: AD-H 5 μm 250 mm x 4.6 mm; Eluent: 80% Kohlendioxid, 20% Ethanol; Fluss: 3 ml/min; UV-Detektion: 220 nm.

## Beispiel 182

4-({2-[4-(5-Chlor-2-cyanophenyl)-5-methoxy-2-oxopyridin-1(2*H*)-yl]butanoyl}amino)-2,5-difluorbenzoesäure (Racemat)

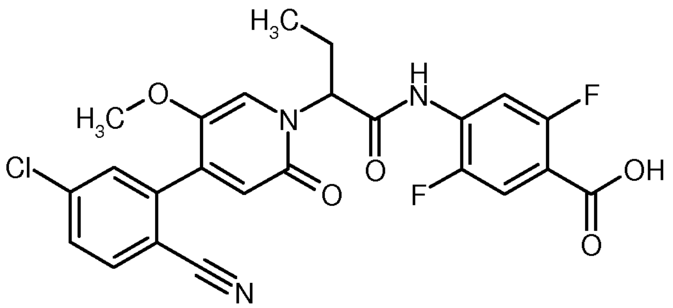

Nach der allgemeinen Methode 6A wurden 179 mg (0.32 mmol) 4-({2-[4-(5-Chlor-2-cyanophenyl)-5-methoxy-2-oxopyridin-1(2*H*)-yl]butanoyl}amino)-2,5-difluorbenzoesäure-*tert*.-butylester (Racemat) umgesetzt. Das Rohprodukt wurde mittels präparativer HPLC (Wasser-Acetonitril-0.1% Ameisensäure-Gradient) aufgereinigt. Ausbeute: 105 mg (65% d. Th.)

LC/MS [Methode 8]: $R_t$ = 1.21 min; MS (ESIpos): m/z = 502 $(M+H)^+$,

$^1$H-NMR (400 MHz, DMSO-$d_6$): δ [ppm] = 13.36 (br. s, 1H), 10.74 (s, 1H), 8.08-7.97 (m, 2H), 7.77-7.68 (m, 3H), 7.45 (s, 1H), 6.55 (s, 1H), 5.85-5.78 (m, 1H), 3.69 (s, 3H), 2.25-2.14 (m, 2H), 0.90 (t, 3H),

$^{19}$F-NMR (376.54 MHz, DMSO-$d_6$): δ [ppm] = -112.6, -129.8.

## Beispiel 183

4-({(2*S*)-2-[4-(5-Chlor-2-cyanophenyl)-5-methoxy-2-oxopyridin-1(2*H*)-yl]butanoyl}amino)-2,5-difluorbenzoesäure (Enantiomer 2)

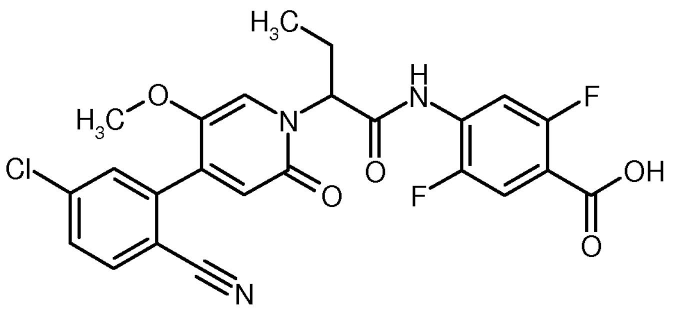

Enantiomerentrennung von 100 mg des Racemates aus Beispiel 182 ergab 33 mg der Titelverbindung Beispiel 183 (Enantiomer 2): Chirale SFC: $R_t$ = 3.05 min; >99% ee.

Trennmethode (SFC): Säule: Chiralpak AZ-H 5μm 250 mm x 30mm; Eluent: 75% Kohlendioxid, 25% Ethanol; Temperatur: 40°C; Fluss: 100 ml/min; Druck: 100 bar; UV-Detektion: 210 nm.

Analytik: Säule: Chiralpak AZ-H 5μ 250 mm x 4.6mm; Eluent: 70% Kohlendioxid, 30% Ethanol; Fluss: 3 ml/min; UV-Detektion: 220 nm.

## Beispiel 184

4-({2-[4-(5-Chlor-2-cyanophenyl)-5-methoxy-2-oxopyridin-1(2*H*)-yl]butanoyl}amino)-2,3-difluorbenzoesäure (Racemat)

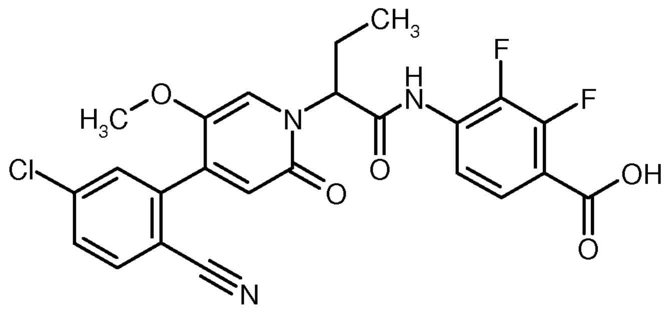

Nach der allgemeinen Methode 6B wurden 80 mg (0.15 mmol) 4-({2-[4-(5-Chlor-2-cyanophenyl)-5-methoxy-2-oxopyridin-1(2*H*)-yl]butanoyl}amino)-2,3-difluorbenzoesäuremethylester (Racemat) umgesetzt. Das Rohprodukt wurde mittels präparativer HPLC (Wasser-Acetonitril-0.1% Ameisensäure-Gradient) aufgereinigt. Ausbeute: 58 mg (74% d. Th.)
LC/MS [1]: $R_t$ = 0.96 min; MS (ESIpos): m/z = 502 $(M+H)^+$,
$^1$H-NMR (400 MHz, DMSO-$d_6$): δ [ppm] = 13.40 (br. s, 1H), 10.73 (s, 1H), 8.02-7.97 (m, 1H), 7.90-7.84 (m, 1H), 7.76-7.71 (m, 2H), 7.71-7.64 (m, 1H), 7.45 (s, 1H), 6.55 (s, 1H), 5.78 (dd, 1H), 3.68 (s, 3H), 2.27-2.13 (m, 2H), 0.91 (t, 3H),
$^{19}$F-NMR (376.54 MHz, DMSO-$d_6$): δ [ppm] = -135.72, -147.97.

**Beispiel 185**

4-({2-[4-(5-Chlor-2-cyanophenyl)-5-methoxy-2-oxopyridin-1(2*H*)-yl]butanoyl}amino)-2,6-difluorbenzoesäure (Racemat)

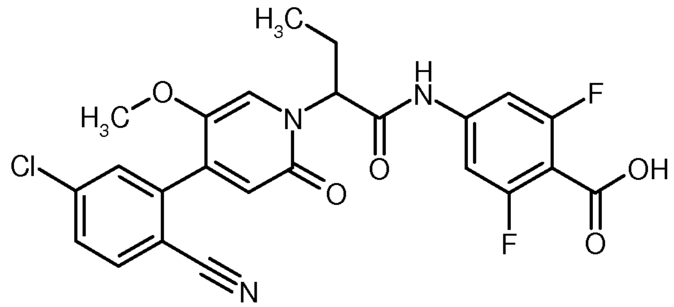

Eine Lösung von 53 mg (0.10 mmol) 4-({2-[4-(5-Chlor-2-cyanophenyl)-5-methoxy-2-oxopyridin-1(2*H*)-yl]butanoyl}amino)-2,6-difluorbenzoesäuremethylester (Racemat) in 1.25 ml einer Mischung aus Methanol/Wasser (4/1) wurde mit 67 mg (0.20 mmol, 2 eq.) Cäsiumcarbonat versetzt und die resultierende Suspension 1 h bei 60°C gerührt. Methanol wurde bei 30°C im Vakuum entfernt. Das Reaktionsgemisch wurde auf RT abgekühlt und mit wässriger Salzsäure (1N) auf pH 3 eingestellt. Die wässrige Phase wurde mit Essigsäureethylester extrahiert. Die vereinigten, organischen Phasen wurden getrocknet (Natriumsulfat), filtriert und unter reduziertem Druck eingeengt. Das Rohprodukt wurde mittels präparativer HPLC (Wasser-Acetonitril-0.1% Ameisensäure-Gradient) aufgereinigt. Ausbeute: 11 mg (21% d. Th.)
LC/MS [Methode 1]: $R_t$ = 0.96 min; MS (ESIpos): m/z = 502 $(M+H)^+$,
$^1$H-NMR (400 MHz, DMSO-$d_6$): δ [ppm] = 13.63 (br. s, 1H), 10.88 (br. s, 1H), 8.00 (d, 1H), 7.76-7.71 (m, 2H), 7.47 (s, 1H), 7.40-7.28 (m, 2H), 6.54 (s, 1H), 5.56 (dd, 1H), 3.69 (s, 3H), 2.24-2.11 (m, 2H), 0.89 (t, 3H).

**Beispiel 186**

2-[4-(5-Chlor-2-cyanophenyl)-5-methoxy-2-oxopyridin-1(2*H*)-yl]-*N*-[4-(5-oxo-4,5-dihydro-1,2,4-thiadiazol-3-yl)phenyl]butanamid (Racemat)

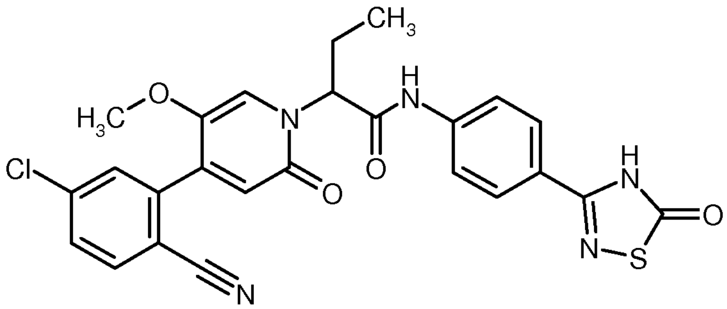

Nach der allgemeinen Methode 7 wurden 65 mg (0.19 mmol) 2-[4-(5-Chlor-2-cyanophenyl)-5-methoxy-2-oxopyridin-1(2*H*)-yl]butansäure (Racemat) und 54 mg (0.28 mmol, 1.5 eq.) 3-(4-Aminophenyl)-1,2,4-thiadiazol-5(4*H*)-on umgesetzt. Das Rohprodukt wurde mittels präparativer HPLC (Wasser-Acetonitril-0.1% Ameisensäure-Gradient) aufgereinigt. Ausbeute: 45 mg (46% d. Th.)
LC/MS [Methode 8]: Rt = 1.25 min; MS (ESIpos): m/z = 521 $(M+H)^+$,
$^1$H-NMR (400 MHz, DMSO-$d_6$): $\delta$ [ppm] = 13.31(br. s, 1H), 10.78 (s, 1H), 8.02-7.98 (m, 1H), 7.94-7.89 (m, 2H), 7.80-7.71 (m, 4H), 7.50 (s, 1H), 6.54 (s, 1H), 5.64 (dd, 1H), 3.69 (s, 3H), 2.26-2.10 (m, 2H), 0.91 (t, 3H).

## Beispiel 187

(2*S*)-2-[4-(5-Chlor-2-cyanophenyl)-5-methoxy-2-oxopyridin-1(2*H*)-yl]-*N*-[4-(5-oxo-4,5-dihydro-1,2,4-thiadiazol-3-yl)phenyl]butanamid (Enantiomer 2)

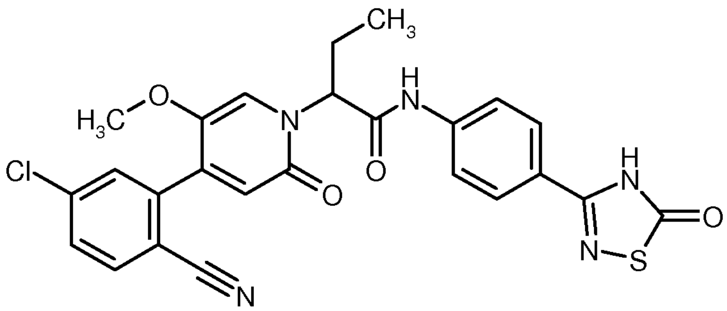

Enantiomerentrennung von 40 mg des Racemates aus Beispiel 186 ergab 10 mg der Titelverbindung Beispiel 187 (Enantiomer 2): Chirale HPLC: $R_t$ = 6.83 min; >99% ee.
Trennmethode (HPLC): Säule: Chiralpak IB 5 µm 250 mm x 20 mm; Eluent: 50% Hexan, 50% 2-Propanol; Temperatur: 35°C; Fluss: 20 ml/min; UV-Detektion: 220 nm.
Analytik: Säule: Chiralcel OD-H 5 µm 250 mm x 4.6 mm; Eluent: 50% Hexan, 50% 2-Propanol; Fluss: 1 ml/min; Temperatur: 30°C; UV-Detektion: 220 nm.

## Beispiel 188

2-[4-(5-Chlor-2-cyanophenyl)-5-methoxy-2-oxopyridin-1(2*H*)-yl]-*N*-[4-(5-thioxo-4,5-dihydro-1,2,4-oxadiazol-3-yl)phenyl]butanamid (Racemat)

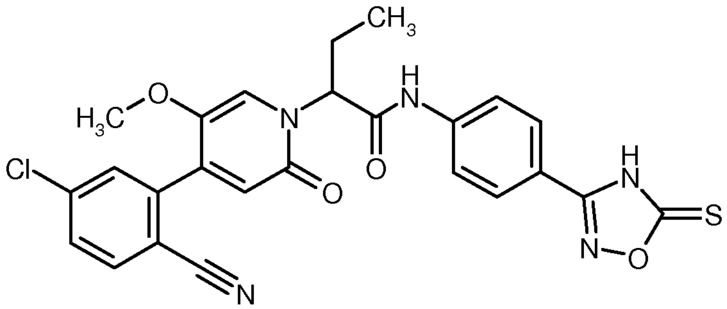

Nach der allgemeinen Methode 7 wurden 36 mg (0.10 mmol) 2-[4-(5-Chlor-2-cyanophenyl)-5-methoxy-2-oxopyridin-1(2*H*)-yl]butansäure (Racemat) und 30mg (0.16 mmol, 1.5 eq.) 3-(4-Aminophenyl)-1,2,4-oxadiazol-5(4*H*)-thion umgesetzt. Das Rohprodukt wurde mittels präparativer HPLC (Wasser-Acetonitril-0.1% Ameisensäure-Gradient) aufgereinigt. Ausbeute: 21 mg (38% d. Th.)
LC/MS [Methode 8]: Rt = 1.26 min; MS (ESIneg): m/z = 520 $(M-H)^-$,
$^1$H-NMR (400 MHz, DMSO-$d_6$): δ [ppm] = 10.85 (s, 1H), 8.02-7.97 (m, 1H), 7.89-7.80 (m, 4H), 7.76-7.71 (m, 2H), 7.50 (s, 1H), 6.54 (s, 1H), 5.64 (dd, 1H), 3.69 (s, 3H), 2.26-2.11 (m, 2H), 0.91 (t, 3H).

## Beispiel 189

2-[4-(5-Chlor-2-cyanophenyl)-5-methoxy-2-oxopyridin-1(2*H*)-yl]-*N*-[4-(1,3-oxazol-2-yl)phenyl]butanamid (Racemat)

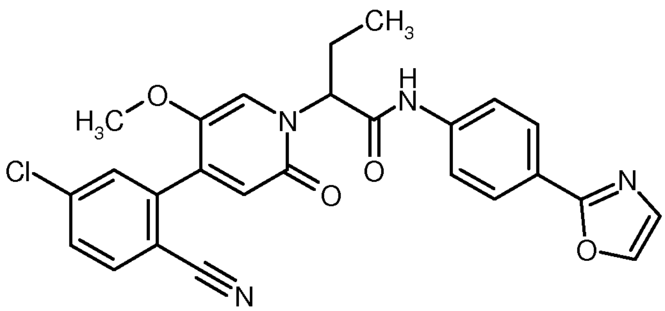

Nach der allgemeinen Methode 7 wurden 100 mg (0.29 mmol) 2-[4-(5-Chlor-2-cyanophenyl)-5-methoxy-2-oxopyridin-1(2*H*)-yl]butansäure (Racemat) und 73 mg (0.43 mmol, 1.5 eq.) 4-(1,3-Oxazol-2-yl)anilin umgesetzt. Das Rohprodukt wurde mittels Normalphasen-Chromatographie (Eluent: Dichlormethan-Methanol 1-10% Gemische) aufgereinigt. Ausbeute: 89 mg (63% d. Th.)
LC/MS [Methode 1]: Rt = 1.04 min; MS (ESIpos): m/z = 489 $(M+H)^+$,
$^1$H-NMR (400 MHz, DMSO-$d_6$): δ [ppm] = 10.76 (s, 1H), 8.18 (d, 1H), 8.00 (d, 1H), 7.98-7.93 (m, 2H), 7.83-7.78 (m, 2H), 7.76-7.71 (m, 2H), 7.51 (s, 1H), 7.35 (d, 1H), 6.54 (s, 1H), 5.65 (dd, 1H), 3.69 (s, 3H), 2.26-2.10 (m, 2H), 0.91 (t, 3H).

## Beispiel 190

2-[4-(5-Chlor-2-cyanophenyl)-5-methoxy-2-oxopyridin-1(2*H*)-yl]-*N*-[4-(1,2,4-oxadiazol-3-yl)phenyl]butanamid (Racemat)

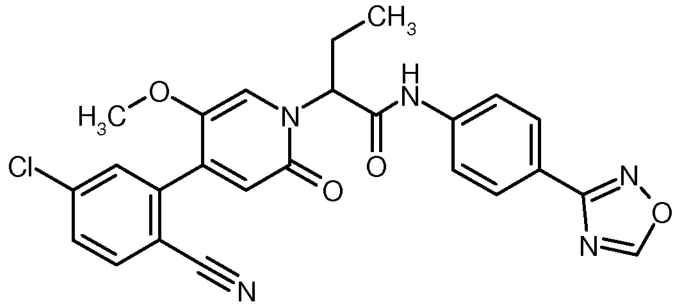

Nach der allgemeinen Methode 7 wurden 100 mg (0.29 mmol) 2-[4-(5-Chlor-2-cyanophenyl)-5-methoxy-2-oxopyridin-1(2*H*)-yl]butansäure (Racemat) und 73 mg (0.43 mmol, 1.5 eq.) 4-(1,2,4-Oxadiazol-3-yl)anilin umgesetzt. Das Rohprodukt wurde mittels Normalphasen-Chromatographie (Eluent: Dichlormethan-Methanol 2-5% Gemische) und anschließend mittels präparativer HPLC (Wasser-Acetonitril-0.1% Ameisensäure-Gradient) aufgereinigt. Ausbeute: 81 mg (55% d. Th.)
LC/MS [Methode 1]: Rt = 0.95 min; MS (ESIpos): m/z = 490 $(M+H)^+$,
$^1$H-NMR (400 MHz, DMSO-$d_6$): $\delta$ [ppm] = 10.85 (s, 1H), 9.30 (s, 1H), 8.03-7.97 (m, 3H), 7.89-7.84 (m, 2H), 7.76-7.71 (m, 2H), 7.51 (s, 1H), 6.55 (s, 1H), 5.65 (dd, 1H), 3.70 (s, 3H), 2.27-2.12 (m, 2H), 0.92 (t, 3H).

**Beispiel 191**

2-[4-(5-Chlor-2-cyanophenyl)-5-methoxy-2-oxopyridin-1(2*H*)-yl]-*N*-[4-(5-methyl-1,3,4-oxadiazol-2-yl)phenyl]butanamid (Racemat)

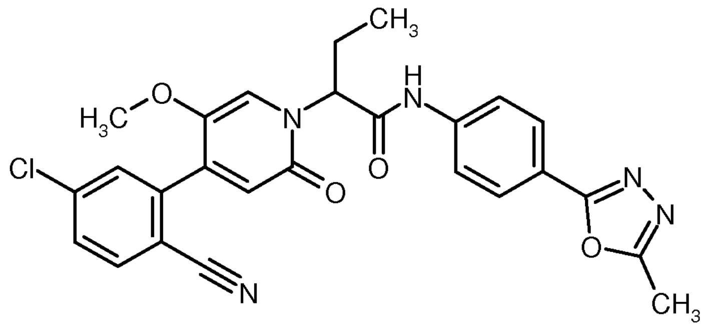

Nach der allgemeinen Methode 7 wurden 100 mg (0.29 mmol) 2-[4-(5-Chlor-2-cyanophenyl)-5-methoxy-2-oxopyridin-1(2*H*)-yl]butansäure (Racemat) und 76 mg (0.43 mmol, 1.5 eq.) 4-(5-Methyl-1,3,4-oxadiazol-2-yl)anilin umgesetzt. Das Rohprodukt wurde mittels Normalphasen-Chromatographie (Eluent: Dichlormethan-Methanol 1-10% Gemische) aufgereinigt. Ausbeute: 144 mg (99% d. Th.)
LC/MS [Methode 1]: Rt = 0.98 min; MS (ESIpos): m/z = 504 $(M+H)^+$,
$^1$H-NMR (400 MHz, DMSO-$d_6$): $\delta$ [ppm] = 10.83 (s, 1H), 8.02-7.97 (m, 1H), 7.97-7.91 (m, 2H), 7.88-7.82 (m, 2H), 7.76-7.71 (m, 2H), 7.50 (s, 1H), 6.54 (s, 1H), 5.65 (dd, 1H), 3.69 (s, 3H), 2.56 (s, 3H), 2.27-2.11 (m, 2H), 0.92 (t, 3H).

**Beispiel 192**

3-{5-[4-({2-[4-(5-Chlor-2-cyanophenyl)-5-methoxy-2-oxopyridin-1(2*H*)-yl]butanoyl}amino)-phenyl]-4*H*-1,2,4-triazol-3-yl}-2,2,3,3-tetrafluorpropansäuremethylester (Racemat)

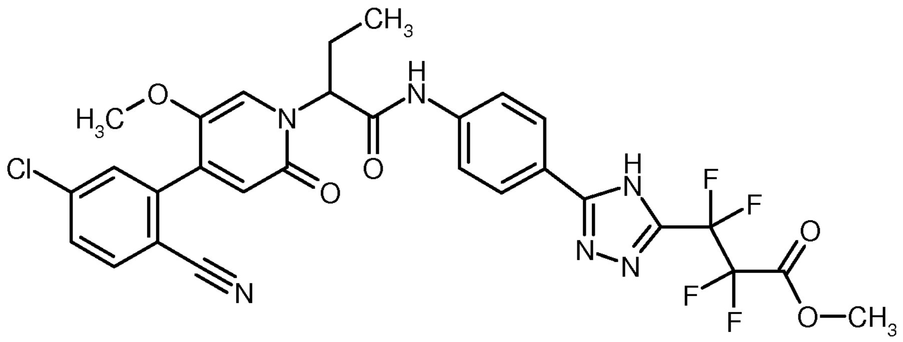

Nach der allgemeinen Methode 1 wurden 87 mg (0.25 mmol) 2-[4-(5-Chlor-2-cyanophenyl)-5-methoxy-2-oxopyridin-1(2*H*)-yl]butansäure (Racemat) mit 88 mg (0.28 mmol, 1.1 eq.) 3-[5-(4-Aminophenyl)-4*H*-1,2,4-triazol-3-yl]-2,2,3,3-tetrafluorpropansäuremethylester umgesetzt. Das Rohprodukt wurde mittels präparativer HPLC (Reprosil C18, Wasser-Acetonitril-Gradient) aufgereinigt. Ausbeute: 96 mg (94% Reinheit, 56% d. Th.)
LC/MS [Methode 1]: $R_t$ = 1.08 min; MS (ESIpos): m/z = 647 $(M+H)^+$.

**Beispiel 193**

3-{5-[4-({2-[4-(5-Chlor-2-cyanophenyl)-5-methoxy-2-oxopyridin-1(2*H*)-yl]butanoyl}amino)-phenyl]-4*H*-1,2,4-triazol-3-yl}-2,2,3,3-tetrafluorpropansäure (Racemat)

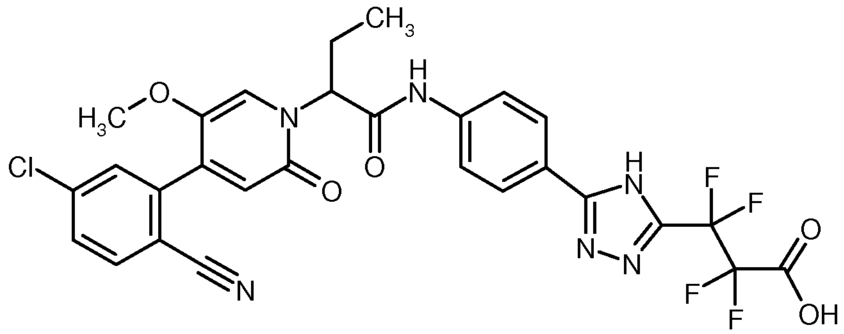

Nach der allgemeinen Methode 3 wurden 96 mg (94% Reinheit, 0.14 mmol) 3-{5-[4-({2-[4-(5-Chlor-2-cyanophenyl)-5-methoxy-2-oxopyridin-1(2*H*)-yl]butanoyl}amino)phenyl]-4*H*-1,2,4-triazol-3-yl}-2,2,3,3-tetrafluorpropansäuremethylester (Racemat) mit Lithiumhydroxid verseift. Das Rohprodukt wurde mittels präparativer HPLC (Reprosil C18, Wasser-Acetonitril-Gradient) aufgereinigt. Ausbeute: 28 mg (32% d. Th.)
LC/MS [Methode 1]: $R_t$ = 0.86 min; MS (ESIpos): m/z = 633 $(M+H)^+$,
$^1$H-NMR (400 MHz, DMSO-$d_6$): $\delta$ [ppm] = 15.01 (br. s, 1H), 10.77 (s, 1H), 8.03-7.94 (m, 3H), 7.82 (d, 2H), 7.77-7.71 (m, 2H), 7.51 (s, 1H), 6.55 (s, 1H), 5.65 (dd, 1H), 3.70 (s, 3H), 2.31-2.10 (m, 2H), 0.92 (t, 3H).

**Beispiel 194**

2-[4-(5-Chlor-2-cyanophenyl)-5-methoxy-2-oxopyridin-1(2*H*)-yl]-*N*-(4-fluor-3-oxo-2,3-dihydro-1*H*-indazol-6-yl)butanamid (Racemat)

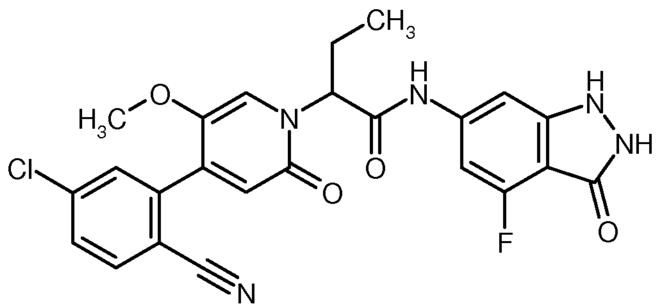

Nach der allgemeinen Methode 6 wurden 100 mg (0.28 mmol) 2-[4-(5-Chlor-2-cyanophenyl)-5-methoxy-2-oxopyridin-1(2*H*)-yl]butansäure (Racemat) mit 51 mg (0.31 mmol, 1.1 eq.) 6-Amino-4-fluor-1,2-dihydro-3*H*-indazol-3-on umgesetzt. Das Rohprodukt wurde mittels präparativer HPLC (Reprosil C18, Wasser-Acetonitril-Gradient) aufgereinigt. Ausbeute: 6 mg (4% d. Th.)
LC/MS [Methode 1]: $R_t$ = 0.86 min; MS (ESIpos): m/z = 496 $(M+H)^+$,
$^1$H-NMR (400 MHz, DMSO-$d_6$): δ [ppm] = 11.63 (br. s, 1H), 10.77 (br. s, 1H), 10.66 (s, 1H), 8.00 (d, 1H), 7.78-7.69 (m, 2H), 7.60 (s, 1H), 7.51 (s, 1H), 6.87 (d, 1H), 6.56 (s, 1H), 5.62 (dd, 1H), 3.70 (s, 3H), 2.25-2.08 (m, 2H), 0.91 (t, 3H).

**Beispiel 195**

2-[4-(5-Chlor-2-cyanophenyl)-5-methoxy-2-oxopyridin-1(2*H*)-yl]-*N*-[3-(trifluormethyl)-1*H*-indazol-6-yl]butanamid (Racemat)

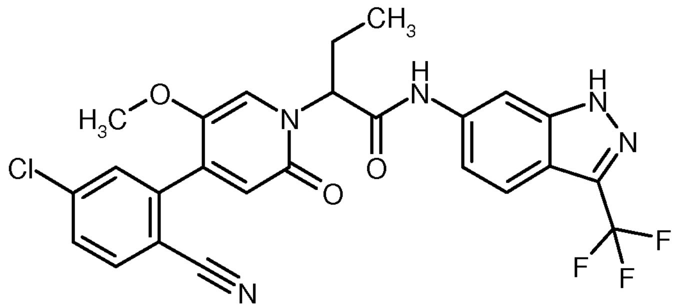

Nach der allgemeinen Methode 6 wurden 82 mg (0.23 mmol) 2-[4-(5-Chlor-2-cyanophenyl)-5-methoxy-2-oxopyridin-1(2*H*)-yl]butansäure (Racemat) mit 51 mg (0.25 mmol, 1.1 eq.) 3-(Trifluormethyl)-1*H*-indazol-6-amin umgesetzt. Das Rohprodukt wurde mittels präparativer HPLC (Reprosil C18, Wasser-Acetonitril-Gradient) aufgereinigt. Ausbeute: 30 mg (25% d. Th.)
LC/MS [Methode 1]: $R_t$ = 1.08 min; MS (ESIpos): m/z = 530 $(M+H)^+$,
$^1$H-NMR (400 MHz, DMSO-$d_6$): δ [ppm] = 13.83 (s, 1H), 10.81 (s, 1H), 8.28 (s, 1H), 8.00 (d, 1H), 7.79-7.71 (m, 3H), 7.53 (s, 1H), 7.39 (dd, 1H), 6.56 (s, 1H), 5.67 (dd, 1H), 3.70 (s, 3H), 2.27-2.11 (m, 2H), 0.92 (t, 3H).

**Beispiel 196**

2-[4-(5-Chlor-2-cyanophenyl)-5-methoxy-2-oxopyridin-1(2*H*)-yl]-*N*-(3-chlor-1*H*-indazol-5-yl)butanamid (Racemat)

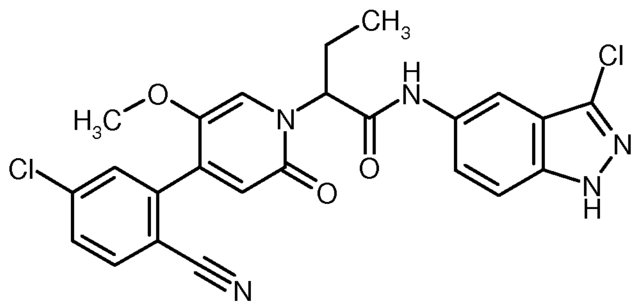

Nach der allgemeinen Methode 1 wurden in zwei Ansätzen insgesamt 242 mg (0.68 mmol) 2-[4-(5-Chlor-2-cyanophenyl)-5-methoxy-2-oxopyridin-1(2*H*)-yl]butansäure (Racemat) mit 404 mg (31% Reinheit, 0.75 mmol, 1.1 eq.) 3-Chlor-1*H*-indazol-5-amin umgesetzt. Die vereinigten Rohprodukte wurden mittels mehrfacher präparativer HPLC (Reprosil C18, Wasser-Acetonitril-Gradient) aufgereinigt. Ausbeute: 18 mg (94% Reinheit, 13% d. Th.) der Titelverbindung Beispiel 196 und 65 mg (55% d. Th.) der Titelverbindung Beispiel 197, welche als Nebenprodukt isoliert wurde aufgrund von Verunreinigen des verwendenten Eduktes 3-Chlor-1*H*-indazol-5-amins mit 1*H*-Indazol-5-amin.
LC/MS [Methode 1]: $R_t$ = 1.01 min; MS (ESIpos): m/z = 496 $(M+H)^+$,
$^1$H-NMR (400 MHz, DMSO-$d_6$): $\delta$ [ppm] = 13.25 (s, 1H), 10.64 (s, 1H), 8.16 (s, 1H), 8.00 (d, 1H), 7.77-7.70 (m, 2H), 7.59-7.49 (m, 3H), 6.55 (s, 1H), 5.66 (dd, 1H), 3.70 (s, 3H), 2.28-2.09 (m, 2H), 0.92 (t, 3H).

**Beispiel 197**

2-[4-(5-Chlor-2-cyanophenyl)-5-methoxy-2-oxopyridin-1(2*H*)-yl]-*N*-(1*H*-indazol-5-yl)butanamid (Racemat)

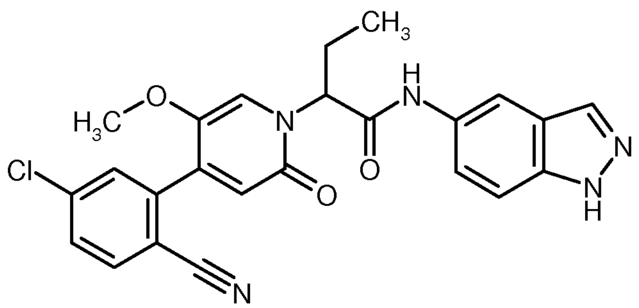

Die Titelverbindung Beispiel 197 wurde als Nebenprodukt bei der Darstellung der Titelverbindung Beispiel 196 isoliert. Ausbeute: 65 mg (55% d. Th.)
LC/MS [Methode 1]: $R_t$ = 0.90 min; MS (ESIpos): m/z = 462 $(M+H)^+$,
$^1$H-NMR (400 MHz, DMSO-$d_6$): $\delta$ [ppm] = 13.00 (s, 1H), 10.50 (s, 1H), 8.15 (s, 1H), 8.05-7.96 (m, 2H), 7.77-7.70 (m, 2H), 7.55 (s, 1H), 7.73-7.45 (m, 2H), 6.54 (s, 1H), 5.68 (dd, 1H), 3.69 (s, 3H), 2.26-2.08 (m, 2H), 0.92 (t, 3H).

**Beispiel 198**

4-({2-[4-(5-Chlor-2-cyanophenyl)-5-methoxy-2-oxopyridin-1(2*H*)-yl]-3-(1,1-dioxidotetrahydro-2*H*-thiopyran-4-yl)propanoyl}amino)benzoesäure (Racemat)

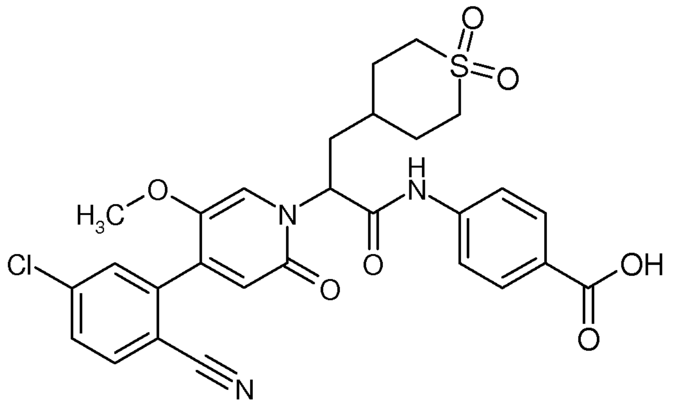

Gemäß allgemeiner Methode 6C wurden 329 mg (537 µmol) 4-({2-[4-(5-Chlor-2-cyanophenyl)-5-methoxy-2-oxopyridin-1(2*H*)-yl]-3-(1,1-dioxidotetrahydro-2*H*-thiopyran-4-yl)propanoyl} amino)-benzoesäureethylester (Racemat) in 19.4 ml Ethanol/Wasser (2.6/1) mit 893 mg (2.74 mmol) Cäsiumcarbonat zur Reaktion gebracht und die Titelverbindung nach präparativer HPLC [Säule: Chromatorex C18, 10 µm, 125 mm x 30 mm, Eluent: Acetonitril/0.05 % Ameisensäure -Gradient; (0 bis 3 min 10% Acetonitril bis 35 min 90% Acetonitril und weitere 3 min 90% Acetonitril)] erhalten. Ausbeute: 136 mg (43% d. Th.)

LC/MS [Methode 1]: $R_t$ = 0.89 min; MS (ESIpos): m/z = 584 $(M+H)^+$

$^1$H-NMR (400 MHz, DMSO-$d_6$): δ [ppm] = 12.8 (br. s, 1H), 10.8 (s, 1H), 8.01 (d, 1H), 7.92 (d, 2H), 7.77-7.71 (m, 4H), 7.51 (s, 1H), 6.56 (s, 1H), 5.85 (dd, 1H), 3.71 (s, 3H), 3.14-2.96 (m, 4H), 2.37-2.27 (m, 1H), 2.15-2.03 (m, 3H), 1.81-1.65 (m, 2H), 1.55-1.43 (m, 1H).

**Beispiel 199**

4-[(2-{4-[5-Chlor-2-(difluormethoxy)phenyl]-5-methoxy-2-oxopyridin-1(2*H*)-yl}-3-[(2*S*)-tetrahydro-2*H*-pyran-2-yl]propanoyl)amino]benzoesäure (Gemisch enantiomerenreiner Diastereomere 1 und 2)

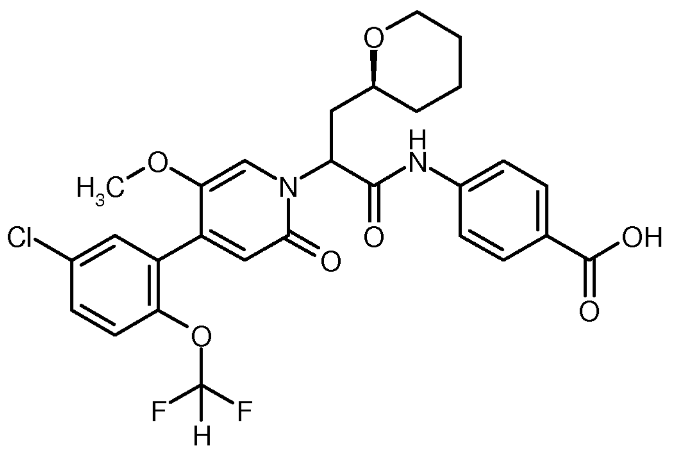

Nach der allgemeinen Methode 4 wurden 315 mg (0.49 mmol) 4-[(2-{4-[5-Chlor-2-(difluormethoxy)phenyl]-5-methoxy-2-oxopyridin-1(2*H*)-yl}-3-[(2*S*)-tetrahydro-2*H*-pyran-2-yl]propanoyl)amino]benzoesäureethylester (Gemisch enantiomerenreiner Diastereomere 1 und 2) in 6.5 ml Ethanol/Wasser (4/1) mit 322 mg (0.99 mmol, 2.0 eq.) Cäsiumcarbonat verseift. Das Rohprodukt wurde mittels präparativer HPLC (Reprosil C18, Wasser-Acetonitril-Gradient) aufgereinigt. Ausbeute: 111 mg (39% d. Th.)

LC/MS [Methode 1]: $R_t$ = 1.05 min; MS (ESIpos): m/z = 577 $(M+H)^+$,

$^1$H-NMR (400 MHz, DMSO-$d_6$): δ [ppm] = 12.76 (s, 1H), 10.74/10.65 (2x s, 1H), 7.94-7.86 (m, 2H), 7.81-7.72 (m, 2H), 7.59/7.57 (2x t, 1H), 7.50/7.48 (2x d, 1H), 7.42/7.38 (2x s, 1H), 7.33-7.27 (m, 1H), 7.13/7.12 (2x t, 1H), 6.38/6.37 (2x s, 1H), 5.80/5.72 (t/dd, 1H), 3.90-3.78 (m, 1H), 3.63/3.62 (2x s, 3H), 3.29-3.03 (m, 2H), 2.39-2.09 (m, 2H), 1.79-1.70 (m, 1H), 1.67-1.54 (m, 1H), 1.48-1.18 (m, 4H).

**Beispiel 200**

4-[(2-{4-[5-Chlor-2-(difluormethoxy)phenyl]-5-methoxy-2-oxopyridin-1(2*H*)-yl}-3-[(2*S*)-tetrahydro-2*H*-pyran-2-yl]propanoyl)amino]benzoesäure (enantiomerenreines Diastereomer 1)

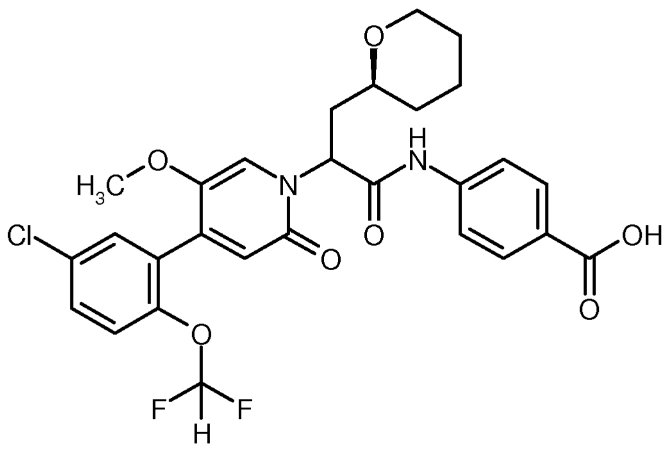

Diastereomerentrennung der Verbindung aus Beispiel 199 zu der Titelverbindung kann mittels SFC an chiralen Phasen (zum Beispiel Daicel Chiralpak AD-H 5 µm, Daicel Chiralpak AZ-H 5 µm, Daicel Chiralpak IF 5 µm, Daicel Chiralpak IC 5 µm oder Daicel Chiralpak OJ-H 5 µm) mit Kohlendioxid/Ethanol- oder mit Kohlendioxid/2-Propanol-Gemischen als Eluenten erfolgen.

**Beispiel 201**

4-[(2-{4-[5-Chlor-2-(difluormethoxy)phenyl]-5-methoxy-2-oxopyridin-1(2*H*)-yl}-3-[(2*S*)-tetrahydro-2*H*-pyran-2-yl]propanoyl)amino]benzoesäure (enantiomerenreines Diastereomer 2)

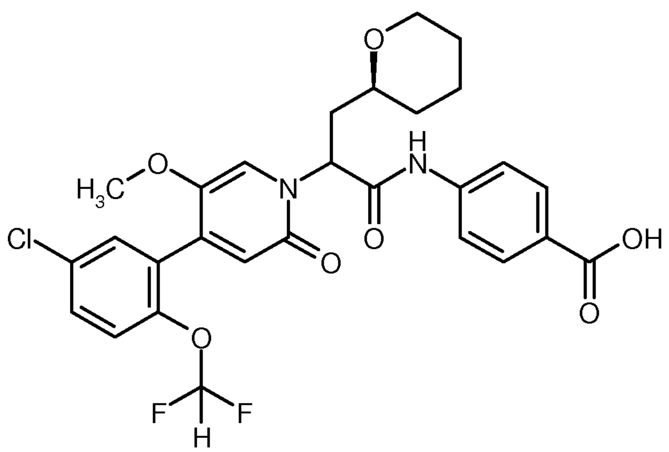

Diastereomerentrennung der Verbindung aus Beispiel 199 zu der Titelverbindung kann mittels SFC an chiralen Phasen (zum Beispiel Daicel Chiralpak AD-H 5 µm, Daicel Chiralpak AZ-H 5 µm, Daicel Chiralpak IF 5 µm, Daicel Chiralpak IC 5 µm oder Daicel Chiralpak OJ-H 5 µm) mit Kohlendioxid/Ethanol- oder mit Kohlendioxid/2-Propanol-Gemischen als Eluenten erfolgen.

**Beispiel 202**

4-({2-[5-Chlor-4-(5-chlor-2-cyanophenyl)-2-oxopyridin-1(2*H*)-yl]-3-[(2*S*)-tetrahydro-2*H*-pyran-2-yl]propanoyl}amino)benzoesäure (Gemisch enantiomerenreiner Diastereomere 1 und 2)

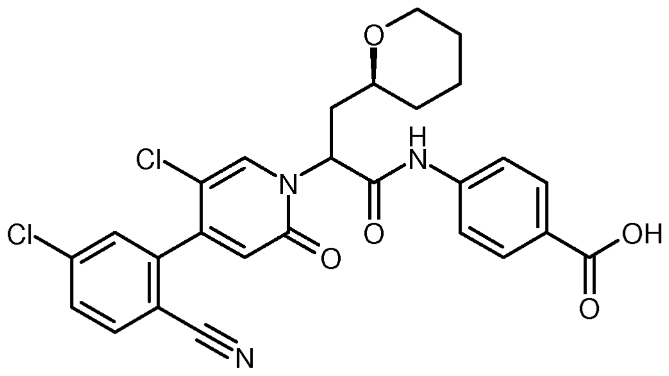

Nach der allgemeinen Methode 2 wurden 364 mg (0.61 mmol) 4-({2-[5-Chlor-4-(5-chlor-2-cyanophenyl)-2-oxopyridin-1(2*H*)-yl]-3-[(2*S*)-tetrahydro-2*H*-pyran-2-yl]propanoyl}amino)benzoesäure-*tert*.-butylester (Gemisch enantiomerenreiner Diastereomere 1 und 2) mit TFA verseift. Das Rohprodukt wurde mittels präparativer Flash-Chromatographie (Kieselgel-50, Eluent: Dichlormethan/Methanol-Gradient) aufgereinigt. Ausbeute: 264 mg (76% d. Th.)
LC/MS [Methode 1]: $R_t$ = 1.05 min; MS (ESIpos): m/z = 540 $(M+H)^+$,
$^1$H-NMR (400 MHz, DMSO-$d_6$): $\delta$ [ppm] = 12.75 (s, 1H), 10.78 (s, 1H), 8.20 (d, 1H), 8.06 (dd, 1H), 7.94-7.87 (m, 2H), 7.84-7.71 (m, 4H), 6.65 (s, 1H), 5.81 (br. s, 1H), 3.89-3.77 (m, 1H), 3.26-3.01 (m, 2H), 2.47-2.15 (m, 2H), 1.80-1.70 (m, 1H), 1.65-1.55 (m, 1H), 1.47-1.21 (m, 4H).

**Beispiel 203**

4-({2-[5-Chlor-4-(5-chlor-2-cyanophenyl)-2-oxopyridin-1(2*H*)-yl]-3-[(2*S*)-tetrahydro-2*H*-pyran-2-yl]propanoyl}amino)benzoesäure (enantiomerenreines Diastereomer 1)

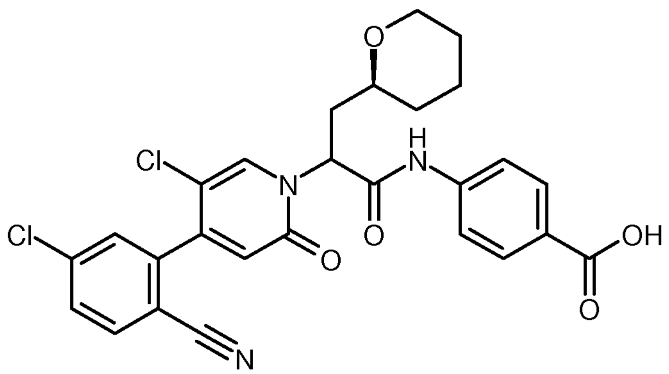

Diastereomerentrennung der Verbindung aus Beispiel 202 zu der Titelverbindung kann mittels SFC an chiralen Phasen (zum Beispiel Daicel Chiralpak AD-H 5 µm, Daicel Chiralpak AZ-H 5 µm, Daicel Chiralpak IF 5 µm, Daicel Chiralpak IC 5 µm oder Daicel Chiralpak OJ-H 5 µm) mit Kohlendioxid/Ethanol- oder mit Kohlendioxid/2-Propanol-Gemischen als Eluenten erfolgen.

**Beispiel 204**

4-({2-[5-Chlor-4-(5-chlor-2-cyanophenyl)-2-oxopyridin-1(2*H*)-yl]-3-[(2*S*)-tetrahydro-2*H*-pyran-2-yl]propanoyl}amino)benzoesäure (enantiomerenreines Diastereomer 2)

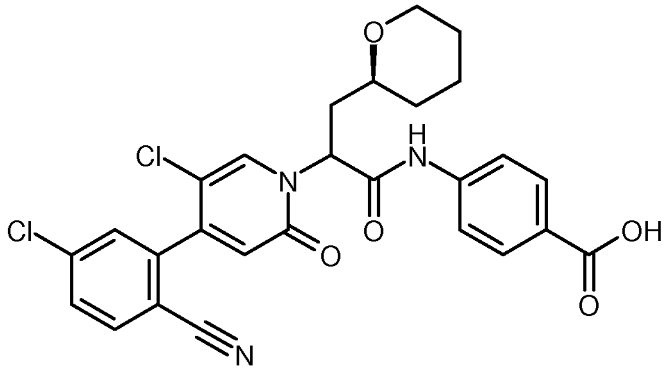

Diastereomerentrennung der Verbindung aus Beispiel 202 zu der Titelverbindung kann mittels SFC an chiralen Phasen (zum Beispiel Daicel Chiralpak AD-H 5 μm, Daicel Chiralpak AZ-H 5 μm, Daicel Chiralpak IF 5 μm, Daicel Chiralpak IC 5 μm oder Daicel Chiralpak OJ-H 5 μm) mit Kohlendioxid/Ethanol- oder mit Kohlendioxid/2-Propanol-Gemischen als Eluenten erfolgen.

**Beispiel 205**

4-({2-[5-Chlor-4-(5-chlor-2-cyanophenyl)-2-oxopyridin-1(2*H*)-yl]-4-methoxybutanoyl}amino)-benzoesäure (Racemat)

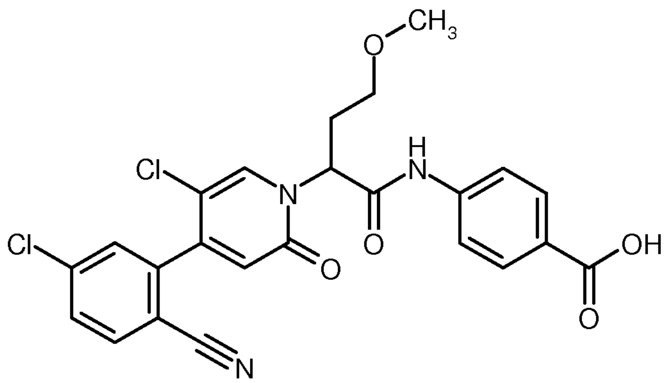

Nach der allgemeinen Methode 2 wurden 438 mg (0.76 mmol) 4-({2-[5-Chlor-4-(5-chlor-2-cyanophenyl)-2-oxopyridin-1(2*H*)-yl]-4-methoxybutanoyl}amino)benzoesäure-*tert.*-butylester (Racemat) mit TFA verseift. Das Rohprodukt wurde mittels präparativer HPLC (Reprosil C18, Wasser-Acetonitril-Gradient) aufgereinigt. Ausbeute: 123 mg (32% d. Th.)

LC/MS [Methode 1]: $R_t$ = 0.96 min; MS (ESIpos): m/z = 500 $(M+H)^+$,

$^1$H-NMR (400 MHz, DMSO-$d_6$): δ [ppm] = 12.77 (br. s, 1H), 10.82 (s, 1H), 8.22 (s, 1H), 8.07 (d, 1H), 7.91 (d, 2H), 7.84-7.77 (m, 2H), 7.75 (d, 2H), 6.67 (s, 1H), 5.77 (br. s, 1H), 3.45-3.38 (m, 1H), 3.30-3.22 (m, 1H), 3.19 (s, 3H), 2.46-2.39 (m, 2H).

**Beispiel 206**

4-({2-[5-Chlor-4-(5-chlor-2-cyanophenyl)-2-oxopyridin-1(2*H*)-yl]-4-methoxybutanoyl}amino)-benzoesäure (Enantiomer 1)

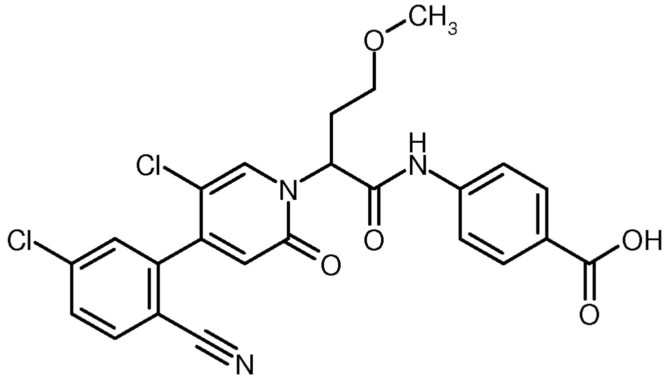

Enantiomerentrennung der Verbindung aus Beispiel 205 zu der Titelverbindung kann mittels SFC an chiralen Phasen (zum Beispiel Daicel Chiralpak AD-H 5 μm, Daicel Chiralpak AZ-H 5 μm, Daicel Chiralpak IF 5 μm, Daicel Chiralpak IC 5 μm oder Daicel Chiralpak OJ-H 5 μm) mit Kohlendioxid/Ethanol- oder mit Kohlendioxid/2-Propanol-Gemischen als Eluenten erfolgen.

**Beispiel 207**

4-({2-[5-Chlor-4-(5-chlor-2-cyanophenyl)-2-oxopyridin-1(2*H*)-yl]-4-methoxybutanoyl}amino)-benzoesäure (Enantiomer 2)

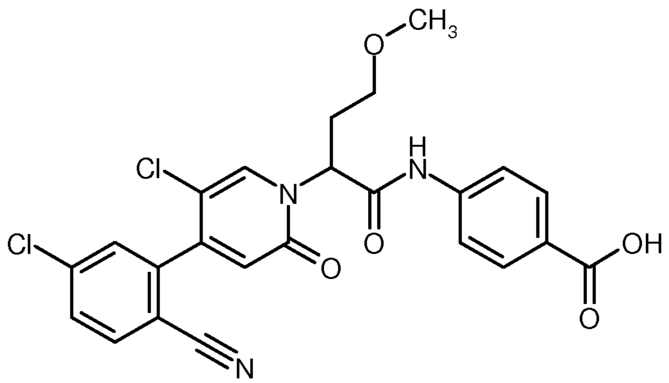

Enantiomerentrennung der Verbindung aus Beispiel 205 zu der Titelverbindung kann mittels SFC an chiralen Phasen (zum Beispiel Daicel Chiralpak AD-H 5 μm, Daicel Chiralpak AZ-H 5 μm, Daicel Chiralpak IF 5 μm, Daicel Chiralpak IC 5 μm oder Daicel Chiralpak OJ-H 5 μm) mit Kohlendioxid/Ethanol- oder mit Kohlendioxid/2-Propanol-Gemischen als Eluenten erfolgen.

**Beispiel 208**

4-({2-[5-Chlor-4-(5-chlor-2-cyanophenyl)-2-oxopyridin-1(2*H*)-yl]-4-(trifluormethoxy)butanoyl}-amino)benzoesäure (Enantiomer 1)

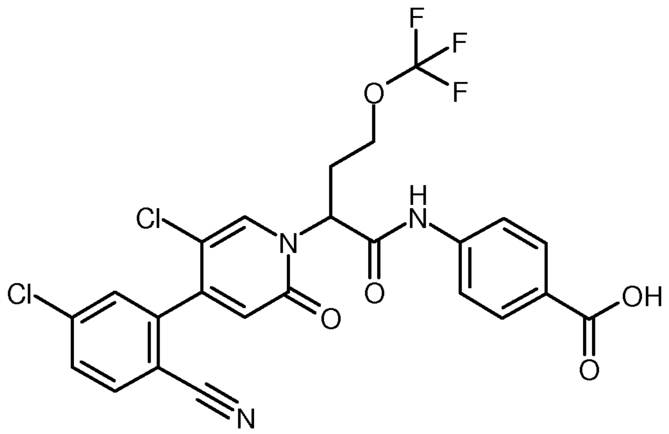

Enantiomerentrennung der Verbindung aus Beispiel 171 zu der Titelverbindung kann mittels SFC an chiralen Phasen (zum Beispiel Daicel Chiralpak AD-H 5 μm, Daicel Chiralpak AZ-H 5 μm, Daicel Chiralpak IF 5 μm, Daicel Chiralpak IC 5 μm oder Daicel Chiralpak OJ-H 5 μm) mit Kohlendioxid/Ethanol- oder mit Kohlendioxid/2-Propanol-Gemischen als Eluenten erfolgen.

## Beispiel 209

4-({2-[5-Chlor-4-(5-chlor-2-cyanophenyl)-2-oxopyridin-1(2*H*)-yl]-4-(trifluormethoxy)butanoyl}-amino)benzoesäure (Enantiomer 2)

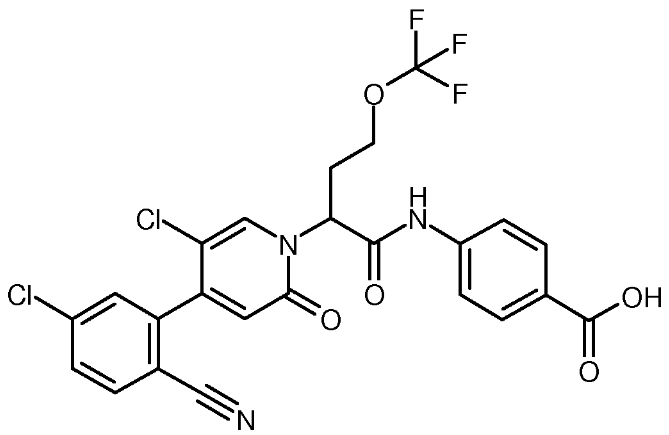

Enantiomerentrennung der Verbindung aus Beispiel 171 zu der Titelverbindung kann mittels SFC an chiralen Phasen (zum Beispiel Daicel Chiralpak AD-H 5 μm, Daicel Chiralpak AZ-H 5 μm, Daicel Chiralpak IF 5 μm, Daicel Chiralpak IC 5 μm oder Daicel Chiralpak OJ-H 5 μm) mit Kohlendioxid/Ethanol- oder mit Kohlendioxid/2-Propanol-Gemischen als Eluenten erfolgen.

## Beispiel 210

4-[(2-{4-[5-Chlor-2-(difluormethoxy)phenyl]-5-methoxy-2-oxopyridin-1(2*H*)-yl}-4-methoxybutanoyl)amino]benzoesäure (Enantiomer 1)

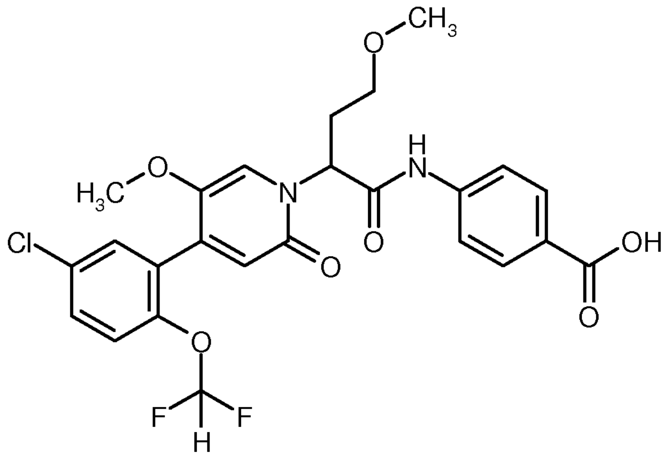

Enantiomerentrennung der Verbindung aus Beispiel 173 zu der Titelverbindung kann mittels SFC an chiralen Phasen (zum Beispiel Daicel Chiralpak AD-H 5 μm, Daicel Chiralpak AZ-H 5 μm, Daicel Chiralpak IF 5 μm, Daicel Chiralpak IC 5 μm oder Daicel Chiralpak OJ-H 5 μm) mit Kohlendioxid/Ethanol- oder mit Kohlendioxid/2-Propanol-Gemischen als Eluenten erfolgen.

**Beispiel 211**

4-[(2-{4-[5-Chlor-2-(difluormethoxy)phenyl]-5-methoxy-2-oxopyridin-1(2*H*)-yl}-4-methoxybutanoyl)amino]benzoesäure (Enantiomer 2)

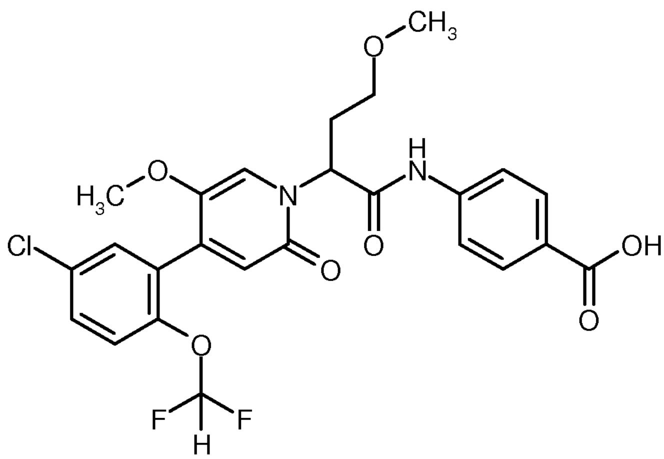

Enantiomerentrennung der Verbindung aus Beispiel 173 zu der Titelverbindung kann mittels SFC an chiralen Phasen (zum Beispiel Daicel Chiralpak AD-H 5 μm, Daicel Chiralpak AZ-H 5 μm, Daicel Chiralpak IF 5 μm, Daicel Chiralpak IC 5 μm oder Daicel Chiralpak OJ-H 5 μm) mit Kohlendioxid/Ethanol- oder mit Kohlendioxid/2-Propanol-Gemischen als Eluenten erfolgen.

**Beispiel 212**

4-{[2-{4-[5-Chlor-2-(difluormethoxy)phenyl]-5-methoxy-2-oxopyridin-1(2*H*)-yl}-4-(trifluormethoxy)butanoyl]amino}benzoesäure (Enantiomer 1)

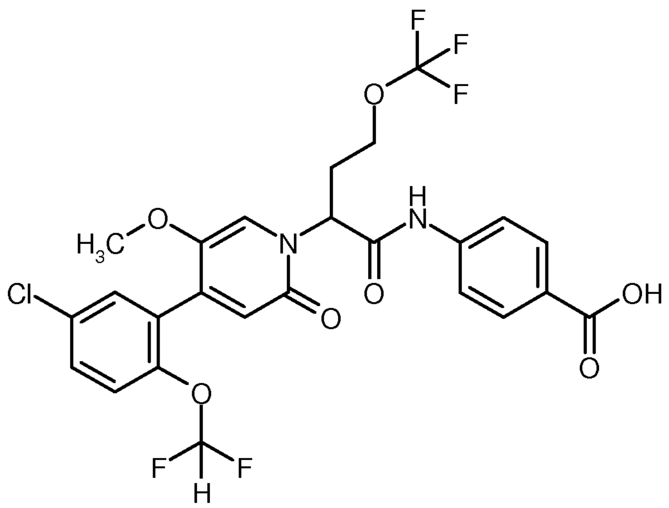

Enantiomerentrennung der Verbindung aus Beispiel 176 zu der Titelverbindung kann mittels SFC an chiralen Phasen (zum Beispiel Daicel Chiralpak AD-H 5 μm, Daicel Chiralpak AZ-H 5 μm, Daicel Chiralpak IF 5 μm, Daicel Chiralpak IC 5 μm oder Daicel Chiralpak OJ-H 5 μm) mit Kohlendioxid/Ethanol- oder mit Kohlendioxid/2-Propanol-Gemischen als Eluenten erfolgen.

## Beispiel 213

4-{[2-{4-[5-Chlor-2-(difluormethoxy)phenyl]-5-methoxy-2-oxopyridin-1(2*H*)-yl}-4-(trifluormethoxy)butanoyl]amino}benzoesäure (Enantiomer 2)

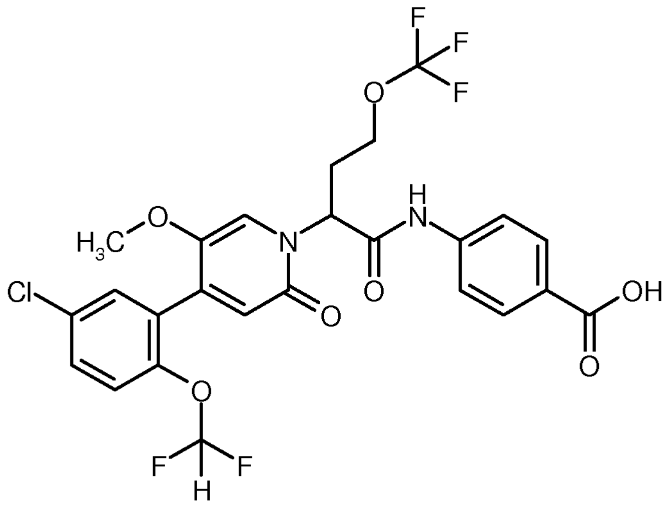

Enantiomerentrennung der Verbindung aus Beispiel 176 zu der Titelverbindung kann mittels SFC an chiralen Phasen (zum Beispiel Daicel Chiralpak AD-H 5 μm, Daicel Chiralpak AZ-H 5 μm, Daicel Chiralpak IF 5 μm, Daicel Chiralpak IC 5 μm oder Daicel Chiralpak OJ-H 5 μm) mit Kohlendioxid/Ethanol- oder mit Kohlendioxid/2-Propanol-Gemischen als Eluenten erfolgen.

**Beispiel 214**

4-[(2-{4-[5-Chlor-2-(difluormethoxy)phenyl]-5-methoxy-2-oxopyridin-1(2*H*)-yl}-3-[(2*R*)-tetrahydro-2*H*-pyran-2-yl]propanoyl)amino]benzoesäure (enantiomerenreinesDiastereomer 1)

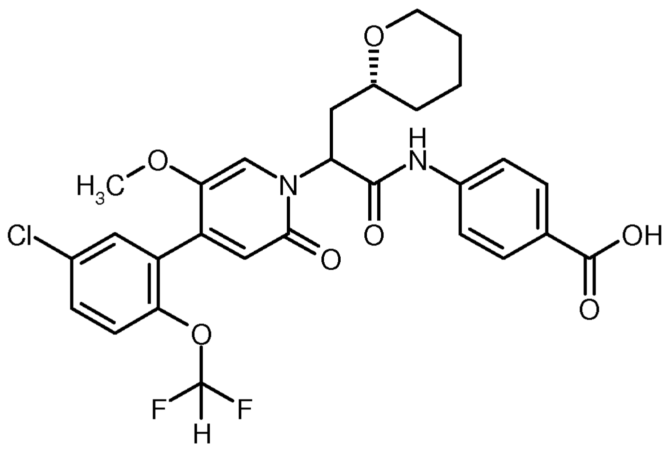

Enantiomerentrennung der Verbindung aus Beispiel 177 zu der Titelverbindung kann mittels SFC an chiralen Phasen (zum Beispiel Daicel Chiralpak AD-H 5 μm, Daicel Chiralpak AZ-H 5 μm, Daicel Chiralpak IF 5 μm, Daicel Chiralpak IC 5 μm oder Daicel Chiralpak OJ-H 5 μm) mit Kohlendioxid/Ethanol- oder mit Kohlendioxid/2-Propanol-Gemischen als Eluenten erfolgen.

**Beispiel 215**

4-[(2-{4-[5-Chlor-2-(difluormethoxy)phenyl]-5-methoxy-2-oxopyridin-1(2*H*)-yl}-3-[(2*R*)-tetrahydro-2*H*-pyran-2-yl]propanoyl)amino]benzoesäure (enantiomerenreinesDiastereomer 2)

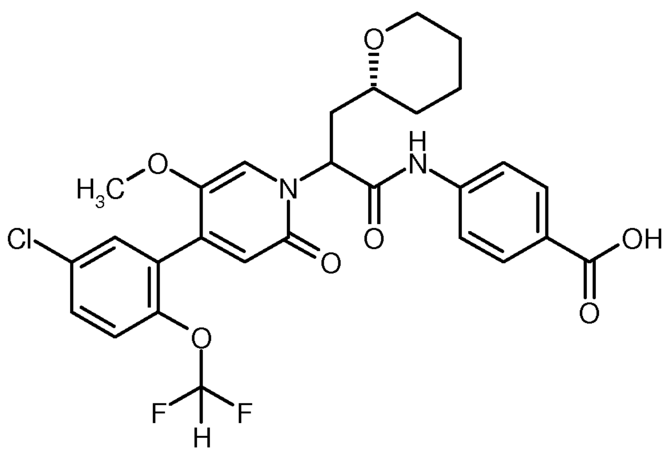

Enantiomerentrennung der Verbindung aus Beispiel 177 zu der Titelverbindung kann mittels SFC an chiralen Phasen (zum Beispiel Daicel Chiralpak AD-H 5 μm, Daicel Chiralpak AZ-H 5 μm, Daicel Chiralpak IF 5 μm, Daicel Chiralpak IC 5 μm oder Daicel Chiralpak OJ-H 5 μm) mit Kohlendioxid/Ethanol- oder mit Kohlendioxid/2-Propanol-Gemischen als Eluenten erfolgen.

## B) Bewertung der physiologischen Wirksamkeit

Die Eignung der erfindungsgemäßen Verbindungen zur Behandlung von thromboembolischen Erkrankungen kann in folgenden Assaysystemen gezeigt werden:

### a) Testbeschreibungen (*in vitro*)

a.1) Messung der FXIa-Hemmung

Zur Bestimmung der Faktor XIa-Hemmung der erfindungsgemäßen Substanzen wird ein biochemisches Testsystem verwendet, in dem die Umsetzung eines peptidischen Faktor XIa-Substrates zur Ermittlung der enzymatischen Aktivität von humanem Faktor XIa benutzt wird. Dabei spaltet Faktor XIa von dem peptischen Faktor XIa-Substrat das C-terminale Aminomethylcoumarin (AMC) ab, dessen Fluoreszenz gemessen wird. Die Bestimmungen werden in Mikrotiterplatten durchgeführt.

Prüfsubstanzen werden in Dimethylsulfoxid aufgelöst und seriell in Dimethylsulfoxid verdünnt (3000 μM bis 0.0078 μM; resultierende Endkonzentrationen im Test: 50 μM bis 0.00013 μM). Jeweils 1 μl der verdünnten Substanzlösungen werden in die Vertiefungen von weißen Mikrotiterplatten der Firma Greiner (384 Vertiefungen) vorgelegt. Anschließend werden nacheinander 20 μl Assaypuffer (50 mM Tris/HCl pH 7.4; 100 mM Natriumchlorid-Lösung; 5 mM Calciumchlorid-Lösung; 0.1% bovines Serumalbumin) und 20 μl Faktor XIa der Firma Kordia (0.45 nM in Assaypuffer) hinzugefügt. Nach 15 min Inkubation wird die Enzymreaktion durch Zugabe von 20 μl des in Assaypuffer gelösten Faktor XIa Substrates Boc-Glu(OBzl)-Ala-Arg-AMC (10 μM in Assaypuffer) der Firma Bachem gestartet, für 30 min bei Raumtemperatur (22°C) inkubiert und anschließend eine Fluoreszensmessung durchgeführt (Anregung: 360 nM, Emission: 460 nM). Die gemessenen Emissionen der Testansätze mit Prüfsubstanz werden mit denen von Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen $IC_{50}$-Werte berechnet. Wirkdaten aus diesem Test sind in der folgenden Tabelle A aufgeführt:

**Tabelle A**

| Beispiel-Nr. | $IC_{50}$ [nM] | | Beispiel-Nr. | $IC_{50}$ [nM] |
|---|---|---|---|---|
| 1 | 53 | | 2 | 260 |
| 3 | 430 | | 4 | 240 |
| 5 | 150 | | 6 | 440 |
| 7 | 82 | | 8 | 110 |
| 9 | 170 | | 10 | 730 |
| 11 | 750 | | 12 | 500 |
| 13 | 600 | | 14 | 330 |
| 15 | 430 | | 16 | 910 |
| 17 | 960 | | 18 | 840 |
| 19 | 59 | | 20 | 27000 |
| 21 | 26 | | 22 | 1700 |
| 23 | 150 | | 24 | 240 |
| 25 | 220 | | 26 | 7.6 |
| 27 | 42 | | 28 | 49 |
| 29 | 36 | | 30 | 50 |
| 31 | 24000 | | 32 | 65 |
| 33 | 33 | | 34 | 74 |
| 35 | 27 | | 36 | 17000 |
| 37 | 14 | | 38 | 230 |

(fortgesetzt)

| Beispiel-Nr. | $IC_{50}$ [nM] | | Beispiel-Nr. | $IC_{50}$ [nM] |
|---|---|---|---|---|
| 39 | 1100 | | 40 | 35 |
| 41 | 54 | | 42 | 14 |
| 43 | 40 | | 44 | 140 |
| 45 | 13 | | 46 | 3300 |
| 47 | 9.8 | | 48 | 42 |
| 49 | 43 | | 50 | 470 |
| 51 | 500 | | 52 | 700 |
| 53 | 630 | | 54 | 3500 |
| 55 | 5.9 | | 56 | 3400 |
| 57 | 2.0 | | 58 | 3.9 |
| 59 | 950 | | 60 | 1.6 |
| 61 | 4.8 | | 62 | 290 |
| 63 | 9.1 | | 64 | 4000 |
| 65 | 4.7 | | 66 | 4.7 |
| 67 | 1700 | | 68 | 1.8 |
| 69 | 3.5 | | 70 | 24 |
| 71 | 32 | | 72 | 34 |
| 73 | 6.2 | | 74 | 17 |
| 75 | 4.3 | | 76 | 1.6 |
| 77 | 2000 | | 78 | 1.3 |
| 79 | 3.2 | | 80 | 4000 |
| 81 | 0.7 | | 82 | 18 |
| 83 | 5.8 | | 84 | 4.4 |
| 85 | 1900 | | 86 | 2.4 |
| 87 | 2.8 | | 88 | 1.3 |
| 89 | 75 | | 90 | 8.5 |
| 91 | 470 | | 92 | 3.2 |
| 93 | 4.0 | | 94 | 5.5 |
| 95 | 520 | | 96 | 2.4 |
| 97 | 4.2 | | 98 | 890 |
| 99 | 920 | | 100 | 1.5 |
| 101 | 1.3 | | 102 | 660 |
| 103 | 0.5 | | 104 | 7.3 |
| 105 | 1.4 | | 106 | 2.3 |
| 107 | 5.3 | | 108 | 6.4 |
| 109 | 31 | | 110 | 9.2 |
| 111 | 34 | | 112 | 32 |
| 113 | 4.3 | | 114 | 600 |

(fortgesetzt)

| Beispiel-Nr. | $IC_{50}$ [nM] | | Beispiel-Nr. | $IC_{50}$ [nM] |
|---|---|---|---|---|
| 115 | 3.0 | | 116 | 2.3 |
| 117 | 1.9 | | 118 | 53 |
| 119 | 28 | | 120 | 46 |
| 121 | 17 | | 122 | 52 |
| 123 | 63 | | 124 | 170 |
| 125 | 3.6 | | 126 | 7.1 |
| 127 | 10 | | 128 | 11 |
| 129 | 24 | | 130 | 50 |
| 131 | 81 | | 132 | 2.0 |
| 133 | 130 | | 134 | 0.3 |
| 135 | 2.7 | | 136 | 1.6 |
| 137 | 1200 | | 138 | 1.0 |
| 139 | 2.5 | | 140 | 3.8 |
| 141 | 190 | | 142 | 3.9 |
| 143 | 0.6 | | 144 | 98 |
| 145 | 17 | | 146 | 130 |
| 147 | 14 | | 148 | 59 |
| 149 | 5.0 | | 150 | 4.7 |
| 151 | 14 | | 152 | 5.4 |
| 153 | 1100 | | 154 | 2.4 |
| 155 | 2.0 | | 156 | 600 |
| 157 | 0.8 | | 158 | 22 |
| 159 | 18 | | 160 | 120 |
| 161 | 2000 | | 162 | 1100 |
| 163 | 6.0 | | 164 | 2.2 |
| 165 | 11 | | 166 | 6.8 |
| 167 | 28 | | 168 | 3.9 |
| 169 | 160 | | 170 | 49 |
| 171 | 5.2 | | 172 | 21 |
| 173 | 2.6 | | 174 | 19 |
| 175 | 15 | | 176 | 4.5 |
| 177 | 3.4 | | 178 | 3.0 |
| 179 | 2.3 | | 180 | 13 |
| 181 | 11 | | 182 | 31 |
| 183 | 14 | | 184 | 10 |
| 185 | 5.5 | | 186 | 5.3 |
| 187 | 2.8 | | 188 | 4.5 |
| 189 | 66 | | 190 | 80 |

(fortgesetzt)

| Beispiel-Nr. | $IC_{50}$ [nM] | | Beispiel-Nr. | $IC_{50}$ [nM] |
|---|---|---|---|---|
| 191 | 49 | | 192 | 3.0 |
| 193 | 2.6 | | 194 | 6.8 |
| 195 | 150 | | 196 | 110 |
| 197 | 17 | | 198 | 12 |
| 199 | 1.3 | | 202 | 2.9 |
| 205 | 4.0 | | | |

a.2) Bestimmung der Selektivität

Zum Nachweis der Selektivität der Substanzen bezüglich FXIa-Hemmung werden die Prüfsubstanzen auf ihre Hemmung anderer humaner Serinproteasen wie Faktor Xa, Trypsin und Plasmin hin untersucht. Zur Bestimmung der enzymatischen Aktivität von Faktor Xa (1.3 nmol/l von Kordia), Trypsin (83 mU/ml von Sigma) und Plasmin (0.1 μg/ml von Kordia) werden diese Enzyme gelöst (50 mmol/l Tris-Puffer [C,C,C-Tris(hydroxymethyl)-aminomethan], 100 mmol/l Natriumchlorid, 0.1% BSA [bovines Serumalbumin], 5 mmol/l Calciumchlorid, pH 7.4) und für 15 min mit Prüfsubstanz in verschiedenen Konzentrationen in Dimethylsulfoxid sowie mit Dimethylsulfoxid ohne Prüfsubstanz inkubiert. Anschließend wird die enzymatische Reaktion durch Zugabe der entsprechenden Substrate gestartet (5 μmol/l Boc-Ile-Glu-Gly-Arg-AMC von Bachem für Faktor Xa und Trypsin, 5 50 μmol/l MeOSuc-Ala-Phe-Lys-AMC von Bachem für Plasmin). Nach einer Inkubationszeit von 30 min bei 22°C wird die Fluoreszenz gemessen (Anregung: 360 nm, Emission: 460 nm). Die gemessenen Emissionen der Testansätze mit Prüfsubstanz werden mit den Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen $IC_{50}$-Werte berechnet.

a.3) Thrombin Generation Assay (Thrombogram)

Die Wirkung der Prüfsubstanzen auf das Thrombogram (Thrombin Generation Assay nach Hemker) wird in vitro in Humanplasma (Octaplas® von der Firma Octapharma) bestimmt.

Im Thrombin Generation Assay nach Hemker wird die Aktivität von Thrombin in gerinnendem Plasma durch die Messung der fluoreszenten Spaltprodukte des Substrats I-1140 (Z-Gly-Gly-Arg-AMC, Bachem) bestimmt. Die Reaktionen werden in Gegenwart variierender Konzentrationen an Prüfsubstanz oder dem entsprechenden Lösungsmittel durchgeführt. Um die Reaktion zu starten werden Reagenzien der Firma Thrombinoscope verwendet (30 pM or 0.1 pM recombinant tissue factor, 24 μM phospholipids in HEPES). Außerdem wird ein Thrombin Calibrator der Firma Thrombinoscope verwendet, dessen amidolytische Aktivität zur Berechnung der Thrombinaktivität in einer Probe mit unbekannter Menge an Thrombin benötigt wird. Die Testdurchführung erfolgt nach Herstellerangaben (Thrombionsocpe BV): 4 μl der Prüfsubstanz oder des Lösungsmittels, 76 μl Plasma und 20 μl PPP-Reagenz oder Thrombin Calibrator werden 5 min bei 37°C inkubiert. Nach Zugabe von 20 μl 2.5 mM Thrombinsubstrat in 20 mM Hepes, 60 mg/ml BSA, 102 mM Calciumchlorid wird die Thrombin Generierung 120 min alle 20 s gemessen. Die Messung wird mit einem Fluorometer (Fluoroskan Ascent) der Firma Thermo Electron durchgeführt, der mit einem 390/460 nM Filterpaar und einem Dispenser ausgestattet ist.

Durch die Verwendung der "thrombinoscope software" wird das Thrombogramm berechnet und graphisch dargestellt. Die folgenden Parameter werden berechnet: lag time, time to Peak, Peak, ETP (endogenous thrombin potential) und start tail.

a.4) Bestimmung der antikoagulatorischen Wirkung

Die antikoagulatorische Wirkung der Prüfsubstanzen wird in vitro in Human- und Rattenplasma bestimmt. Dazu wird Blut unter Verwendung einer 0.11 molaren Natriumcitrat-Lösung als Vorlage in einem Mischungsverhältnis Natriumcitrat/Blut 1/9 abgenommen. Das Blut wird unmittelbar nach der Abnahme gut gemischt und 15 Minuten bei ca. 4000 g zentrifugiert. Der Überstand wird abpipettiert.

Die Prothrombinzeit (PT, Synonyme: Thromboplastinzeit, Quick-Test) wird in Gegenwart variierender Konzentrationen an Prüfsubstanz oder dem entsprechenden Lösungsmittel mit einem handelsüblichen Testkit (Neoplastin® von der Firma Boehringer Mannheim oder Hemoliance® RecombiPlastin von der Firma Instrumentation Laboratory) bestimmt. Die Testverbindungen werden 3 Minuten bei 37°C mit dem Plasma inkubiert. Anschließend wird durch Zugabe

von Thromboplastin die Gerinnung ausgelöst und der Zeitpunkt des Gerinnungseintritts bestimmt. Es wird die Konzentration an Prüfsubstanz ermittelt, die eine Verdoppelung der Prothrombinzeit bewirkt.

Die aktivierte partielle Thromboplastinzeit (APTT) wird in Gegenwart variierender Konzentrationen an Prüfsubstanz oder dem entsprechenden Lösungsmittel mit einem handelsüblichen Testkit (PTT Reagent von der Firma Roche) bestimmt. Die Testverbindungen werden 3 Minuten bei 37°C mit dem Plasma und dem PTT Reagenz (Cephalin, Kaolin) inkubiert. Anschließend wird durch Zugabe von 25 mM Calciumchlorid die Gerinnung ausgelöst und der Zeitpunkt des Gerinnungseintritts bestimmt. Es wird die Konzentration an Prüfsubstanz ermittelt, die eine 50%ige Verlängerung beziehungsweise ein Verdoppelung der APTT bewirkt.

a.5) Bestimmung der Plasma-Kallikrein Aktivität

Zur Bestimmung der Plasma Kallikrein-Hemmung der erfindungsgemäßen Substanzen wird ein biochemisches Testsystem verwendet, in dem die Umsetzung eines peptidischen Plasma Kallikrein-Substrates zur Ermittlung der enzymatischen Aktivität von humanem Plasma Kallikrein benutzt wird. Dabei spaltet Plasma Kallikrein von dem peptischen Plasma Kallikrein-Substrat das C-terminale Aminomethylcoumarin (AMC) ab, dessen Fluoreszenz gemessen wird. Die Bestimmungen werden in Mikrotiterplatten durchgeführt.

Prüfsubstanzen werden in Dimethylsulfoxid aufgelöst und seriell in Dimethylsulfoxid verdünnt (3000 μM bis 0.0078 μM; resultierende Endkonzentrationen im Test: 50 μM bis 0.00013 μM). Jeweils 1 μl der verdünnten Substanzlösungen werden in die Vertiefungen von weißen Mikrotiterplatten der Firma Greiner (384 Vertiefungen) vorgelegt. Anschließend werden nacheinander 20 μl Assaypuffer (50 mM Tris/HCl pH 7,4; 100 mM Natriumchlorid-Lösung; 5 mM Calciumchlorid-Lösung; 0.1% bovines Serumalbumin) und 20 μl Plasma-Kallikrein der Firma Kordia (0.6 nM in Assaypuffer) hinzugefügt. Nach 15 min Inkubation wird die Enzymreaktion durch Zugabe von 20 μl des in Assaypuffer gelösten Substrates H-Pro-Phe-Arg-AMC (10 μM in Assaypuffer) der Firma Bachem gestartet, für 30 min bei Raumtemperatur (22°C) inkubiert und anschließend eine Fluoreszensmessung durchgeführt (Anregung: 360 nM, Emission: 460 nM). Die gemessenen Emissionen der Testansätze mit Prüfsubstanz werden mit denen von Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen $IC_{50}$-Werte berechnet.

**Tabelle B**

| Beispiel-Nr. | $IC_{50}$ [nM] | | Beispiel-Nr. | $IC_{50}$ [nM] |
|---|---|---|---|---|
| 1 | 600 | | 3 | 650 |
| 4 | 590 | | 5 | 330 |
| 6 | 640 | | 7 | 220 |
| 8 | 490 | | 9 | 840 |
| 17 | 630 | | 18 | 490 |
| 21 | 120 | | 22 | 595 |
| 23 | 520 | | 26 | 67 |
| 27 | 780 | | 28 | 570 |
| 29 | 350 | | 30 | 620 |
| 32 | 800 | | 33 | 320 |
| 34 | 310 | | 35 | 510 |
| 37 | 275 | | 40 | 550 |
| 41 | 1600 | | 42 | 110 |
| 43 | 230 | | 44 | 140 |
| 45 | 200 | | 47 | 150 |
| 55 | 31 | | 57 | 17 |
| 58 | 46 | | 60 | 15.5 |
| 61 | 29 | | 63 | 67 |
| 65 | 25 | | 66 | 31 |

(fortgesetzt)

| Beispiel-Nr. | $IC_{50}$ [nM] | | Beispiel-Nr. | $IC_{50}$ [nM] |
|---|---|---|---|---|
| 68 | 14.5 | | 69 | 11 |
| 70 | 16 | | 71 | 17 |
| 72 | 29 | | 73 | 16 |
| 74 | 10 | | 75 | 28 |
| 76 | 12 | | 78 | 4.1 |
| 79 | 15 | | 81 | 7.6 |
| 82 | 45 | | 83 | 15 |
| 84 | 40 | | 86 | 16.5 |
| 87 | 14 | | 88 | 7.9 |
| 89 | 850 | | 90 | 54 |
| 92 | 35 | | 93 | 18 |
| 94 | 48 | | 96 | 19 |
| 97 | 26 | | 100 | 15 |
| 101 | 19 | | 103 | 5.9 |
| 104 | 67 | | 105 | 13 |
| 106 | 21 | | 107 | 56 |
| 108 | 59 | | 109 | 220 |
| 110 | 78 | | 111 | 220 |
| 112 | 200 | | 113 | 39 |
| 115 | 16 | | 116 | 11 |
| 117 | 12 | | 118 | 210 |
| 119 | 200 | | 121 | 220 |
| 122 | 700 | | 123 | 1100 |
| 125 | 23 | | 126 | 64 |
| 127 | 160 | | 128 | 180 |
| 129 | 390 | | 130 | 1300 |
| 132 | 18 | | 134 | 5.8 |
| 135 | 19 | | 136 | 9.4 |
| 138 | 6.5 | | 139 | 9.4 |
| 140 | 32 | | 141 | 1600 |
| 142 | 18 | | 143 | 4.6 |
| 144 | 1000 | | 145 | 98 |
| 146 | 1500 | | 147 | 70 |
| 148 | 430 | | 149 | 27 |
| 150 | 18 | | 151 | 110 |
| 152 | 75 | | 154 | 45 |
| 155 | 10 | | 157 | 5.2 |
| 158 | 29 | | 159 | 16 |

(fortgesetzt)

| Beispiel-Nr. | $IC_{50}$ [nM] | | Beispiel-Nr. | $IC_{50}$ [nM] |
|---|---|---|---|---|
| 160 | 240 | | 161 | 1500 |
| 162 | 250 | | 163 | 3.6 |
| 164 | 1.9 | | 165 | 8.2 |
| 166 | 6.3 | | 167 | 8.4 |
| 168 | 3.1 | | 169 | 54 |
| 170 | 670 | | 171 | 89 |
| 172 | 360 | | 173 | 54 |
| 174 | 28 | | 175 | 20 |
| 176 | 58 | | 177 | 120 |
| 178 | 33 | | 179 | 24 |
| 180 | 78 | | 181 | 45 |
| 182 | 130 | | 183 | 62 |
| 184 | 110 | | 185 | 40 |
| 186 | 31 | | 187 | 14 |
| 188 | 31 | | 189 | 63 |
| 190 | 35 | | 191 | 35 |
| 192 | 61 | | 193 | 46 |
| 194 | 6.3 | | 195 | 59 |
| 196 | 21 | | 197 | 10 |
| 198 | 39 | | 199 | 34 |
| 202 | 82 | | 205 | 68 |

a.6) Bestimmung der Endothel Integrität

Die Aktivität der erfindungsgemäßen Verbindungen werden mittels eines in-vitro Permeabilitäts-Assays auf "human umbilical venous cells" (HUVEC) charakterisiert. Mittels des EOS Apparatus (EC IS: Electric Cell-substrate Impedance Sensing; Applied Biophysics Inc; Troy, NY) können Unterschiede des transendothelialen elektrischen Widerstandes (TEER) über einer endothelialen Zell-Monoschicht, die über Gold-Elektroden plattiert wurde, kontinuierlich gemessen werden. HUVECs werden auf einer 96-well sensor Elektrodenplatte (96W1 E, Ibidi GmbH, Martinsried) ausgesäht. Eine Hyperpermeabilität der enstandenen konfluenten Zell-Monoschicht wird mittels Stimulation mit Kininogen, Prekallikrein und Faktor XII (je 100 nM) induziert. Die erfindungsgemäßen Verbindungen werden vor der Zugabe der oben angegebenen Substanzen zugegeben. Die üblichen Konzentationen der Verbindungen sind 1 x $10^{-10}$ bis 1 x $10^{-6}$ M.

a.7) Bestimmung der in-vitro Permeabilität von Endothelzellen

In einem weiteren Hyperpermeabilitäts-Modell wird die Aktivität der Substanzen auf die Modulation der makromolekularen Permeabilität bestimmt. HUVECs werden auf einer Fibronektin-überzogenen Transwell Filter Membran (24-well plates, 6.5 mm-Einsatz mit 0.4 $\mu$M Polykarbonat Membran; Costar #3413) ausgesäht. Die Filtermembran trennt den oberen von dem unteren Zellkulturraum mit der konfluenten Endothelzellschicht auf dem Boden des oberen Zellkulturraumes. Dem Medium des oberen Raumes wird 250 g/ml 40 kDa FITC-Dextan (Invitrogen, D1844) zugesetzt. Die Hyperpermeabilität der Monolayer-Schicht wird mittels Stimulation mit Kininogen, Prekallikrein und Faktor XII (je 100 nM) induziert. Medium Proben werden alle 30 min aus der unteren Kammer entnommen und die relative Fluoreszenz, als Parameter für die Veränderungen der makromolekularen Permeabilität in Abhängigkeit von der Zeit, mit einem Fluorimeter bestimmt. Die erfindungsgemäßen Verbindungen werden vor der Zugabe der oben angegebenen Substanzen zugegeben. Die üblichen Konzentationen der Verbindungen sind 1 x $10^{-10}$ bis 1 x $10^{-6}$ M.

**b) Bestimmung der antithrombotischen Wirkung *(in vivo)***

b.1) Arterielles Thrombose-Modell (Eisen(II)chlorid-induzierte Thrombose) in Kombination mit Ohrblutungszeit im Kaninchen

Die antithrombotische Aktivität der FXIa-Inhibitoren wird in einem arteriellen Thrombose-Modell getestet. Dabei wird die Thrombusbildung durch chemische Beschädigung eines Bereichs der Arteria carotis im Kaninchen ausgelöst. Simultan wird die Ohrblutungszeit bestimmt.

Männliche Kaninchen (Crl:KBL (NZW)BR, Charles River) unter normaler Diät mit einem Gewicht von 2.2 - 2.5 kg Körpergewicht werden durch intramuskuläre Applikation von Xylazin und Ketamin (Rompun, Bayer, 5 mg/kg und Ketavet, Pharmacia & Upjohn GmbH, 40 mg/kg Körpergewicht) anästhesiert. Die Anästhesie wird weiterhin durch intravenöse Gabe derselben Präparate (bolus: Dauerinfusion) über die rechte Ohrvene unterstützt.

Nach Freipräparation der rechten Arteria carotis wird der Gefäßschaden dadurch erzeugt, dass ein Stück Filterpapier (10 mm x 10 mm) auf einem Streifen Parafilm® (25 mm x 12 mm) um die A. carotis gewickelt wird, ohne den Blutfluß dadurch zu beeinträchtigen. Das Filterpapier enthält 100 µL einer 13%igen Lösung von Eisen(II)chlorid (Sigma) in Wasser. Nach 5 min wird das Filterpapier entfernt und das Gefäß zweimal mit wässriger 0.9%iger Natriumchlorid-Lösung gespült. 30 min nach der Verletzung wird die Arteria carotis im Bereich der Schädigung herauspräpariert und eventuell vorhandenes thrombotisches Material entnommen und gewogen.

Die Prüfsubstanzen werden entweder intravenös über die Vena femoralis den anästhesierten oder oral mittels Schlundsonde den wachen Tieren jeweils 5 min beziehungsweise 2 h vor Schädigung verabreicht.

Die Ohrblutungszeit wird 2 min nach der Schädigung der Arteria carotis bestimmt. Hierzu wird das linke Ohr rasiert und ein definierter Schnitt von 3 mm Länge (Klinge Art.Nummer 10-150-10, Martin, Tuttlingen, Germany) parallel zur Ohrlängsachse gesetzt. Dabei wird darauf geachtet, kein sichtbares Gefäß zu verletzen. Eventuell austretendes Blut wird in 15 Sekunden-Intervallen mit genau gewogenen Filterpapierstücken aufgenommen, ohne die Wunde direkt zu berühren. Die Blutungszeit wird berechnet als die Zeitspanne vom Setzen des Schnitts bis zu dem Zeitpunkt, an dem am Filterpapier kein Blut mehr nachweisbar ist. Das ausgetretene Blutvolumen wird nach Wiegen der Filterpapierstücke berechnet.

**c) Bestimmung der Wirkung auf die Extravasation/Ödembildung und/oder Neovaskularisierung im Auge (*in vivo*)**

c.1) Testung der Wirksamkeit von Substanzen im Laser-induzierten choroidalen Neovaskularisierungs-Modell

Diese Studie dient dem Zweck, die Wirksamkeit einer Testsubstanz auf die Reduktion der Extravasation/Ödembildung und/oder der choroidalen Neovaskularisierung im Rattenmodell der Laser-induzierten choroidalen Neovaskularisierung zu untersuchen.

Dafür werden pigmentierte Ratten vom Stamm Brown-Norway, die keine Anzeichen ophthalmologischer Erkrankungen aufweisen, ausgewählt und nach dem Zufallsprinzip Behandlungsgruppen zugeordnet. Am Tag 0 werden die Tiere mittels intraperitonealer Injektion narkotisiert (15 mg/kg Xylazin und 80 mg/kg Ketamin). Nach Instillation eines Tropfens einer 0.5%igen Tropicamid-Lösung zur Weitstellung der Pupillen wird die choroidale Neovaskularisierung mittels eines 532 nm Argon Laser Photokoagulators an sechs definierten Stellen um den Nervus opticus herum ausgelöst (50-75 µm Durchmesser, 150 mW Intensität, 100 ms Dauer). Die Testsubstanz und das entsprechende Vehikel (z.B. PBS, isotone Kochsalzlösung) werden entweder systemisch oral beziehungsweise intraperitonal appliziert oder lokal am Auge durch mehrfache Gabe als Augentropfen beziehungsweise intravitreale Injektion verabreicht. Das Körpergewicht aller Tiere wird vor Beginn der Studie, und anschließend täglich während der Studie bestimmt.

An Tag 21 wird eine Angiographie mittels einer Fluoreszenz-Funduskammera (z.B. Kowe, HRA) durchgeführt. In Narkose und nach erneuter Pupillenerweiterung wird ein 10%iger Natrium-Fluorescein-Farbstoff subkutan (s.c.) injiziert. 2-10 min später werden Bilder des Augenhintergrundes aufgenommen. Die Stärke der Extravasation/des Ödems, dargestellt durch die Leckage von Fluorescein, wird von zwei bis drei verblindeten Beobachtern beurteilt und in Schweregrade von 0 (keine Extravasation) bis 3 (starke Einfärbung über die eigentliche Läsion hinaus) eingeteilt.

Nach Abtöten der Tiere an Tag 23 werden die Augen entnommen und in 4%iger Paraformaldehyd-Lösung für eine Stunde bei Raumtemperatur fixiert. Nach einem Waschdurchgang wird die Retina vorsichtig herausgeschält, und der Sklera-Choroidea-Komplex wird mit einem FITC-Isolektin B4 Antikörper angefärbt und anschließend flach auf einen Objetträger aufgebracht. Die so erhaltenen Präparate werden mittels eines Fluoreszenz-Mikroskops (Apotom, Zeiss) bei einer Anregungs-Wellenlänge von 488 nm ausgewertet. Die Fläche beziehungsweise das Volumen der choroidalen Neovaskularisierung (in $\mu m^2$ bzw. $\mu m^3$) wird durch morphometrische Analyse mittels Axiovision 4.6 Software berechnet.

c.2) Testung der Wirksamkeit von Substanzen im Sauerstoff-induzierten Retinopathie Modell

Es wurde gezeigt, dass eine durch Sauerstoff induzierte Retinopathie ein wertvolles Tiermodell für die Untersuchung der pathologischen retinalen Angiogenese darstellt. Dieses Modell basiert auf der Beobachtung, dass eine Hyperoxie während der frühen postnatalen Entwicklung in der Retina zum Anhalten oder zur Verlangsamung des Wachstums von normalen retinalen Blutgefäßen führt. Sobald die Tiere nach einer 7-tägigen Hyperoxiephase zur normoxischen Raumluft zurückkehren, ist dieses gleichbedeutend einer relativen Hypoxie, da in der Retina die normalen Gefäße fehlen, welche erforderlich sind, um eine ausreichende Versorgung des neuralen Gewebes unter normoxischen Bedingungen zu gewährleisten. Die dadurch erzeugte ischämische Situation führt zur abnormen Neovaskularisation, die einige Ähnlichkeiten mit der pathophysiologischen Neovaskularisation in Augenerkrankungen wie der feuchten AMD aufzeigt. Darüber hinaus ist die hervorgerufene Neovaskularisierung sehr reproduzierbar, quantifizierbar und ein wichtiger Parameter für die Untersuchung der Krankheitsmechanismen und möglichen Behandlungen für verschiedenste Formen von Netzhauterkrankungen.
Das Ziel dieser Studie ist es, die Wirksamkeit von täglich systemisch verabreichten Dosen der Testverbindung auf das Wachstum der retinalen Gefäße im Sauerstoff-induzierten Retinopathie-Modell zu untersuchen. Neugeborene von C57B1 / 6 Mäusen und ihre Mütter werden am postnatalen Tag 7 (PD7) einer Hyperoxie (70% Sauerstoff) für 5 Tage ausgesetzt. Ab PD12, werden die Mäuse unter normoxischen Bedingungen (Raumluft, 21% Sauerstoff) bis zum PD17 gehalten. Von Tag 12 bis Tag 17 werden die Mäuse täglich mit der Testsubstanz oder dem entsprechenden Vehikel behandelt. Am Tag 17 werden alle Mäuse mit Isofluran narkotisiert und anschließend durch Genickbruch abgetötet. Die Augen werden entnommen und in 4% Formalin fixiert. Nach dem Waschen in Phosphat-gepufferter Kochsalzlösung wird die Netzhaut präpariert, ein Flachpräperat davon erzeugt und dieses mit Isolectin B4 Antikörper gefärbt. Die Quantifizierung der neugewachsenen Gefäße wird unter Verwendung eines Zeiss ApoTome durchgeführt.

## C) Ausführungsbeispiele für pharmazeutische Zusammensetzungen

Die erfindungsgemäßen Substanzen können folgendermaßen in pharmazeutische Zubereitungen überführt werden:

### Tablette:

Zusammensetzung:

100 mg der Verbindung des Beispiels 1, 50 mg Lactose (Monohydrat), 50 mg Maisstärke, 10 mg Polyvinylpyrolidon (PVP 25) (Fa. BASF, Deutschland) und 2 mg Magnesiumstearat.
Tablettengewicht 212 mg. Durchmesser 8 mm, Wölbungsradius 12 mm.

Herstellung:

Die Mischung aus der Verbindung des Beispiels 1, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben).

### Orale Suspension:

Zusammensetzung:

1000 mg der Verbindung des Beispiels 1, 1000 mg Ethanol (96%), 400 mg Rhodigel (Xanthan gum) (Fa. FMC, USA) und 99 g Wasser.
Einer Einzeldosis von 100 mg der erfindungsgemäßen Verbindung entsprechen 10 ml orale Suspension.

Herstellung:

Das Rhodigel wird in Ethanol suspendiert, die Verbindung des Beispiels 1 wird der Suspension zugefügt. Unter Rühren erfolgt die Zugabe des Wassers. Bis zum Abschluss der Quellung des Rhodigels wird ca. 6h gerührt.

### Lösung oder Suspension zur topischen Anwendung am Auge (Augentropfen):

Eine sterile pharmazeutische Zubereitung zur topischen Anwendung am Auge kann durch Rekonstitution eines

Lyophilisats der erfindungsgemäßen Verbindung in steriler Kochsalz-Lösung hergestellt werden. Als Konservierungsmittel für eine solche Lösung oder Suspension ist beispielsweise Benzalkoniumchlorid, Thiomersal oder Phenylquecksilbernitrat in einem Konzentrationsbereich von 0.001 bis 1 Gewichtsprozent geeignet.

**Patentansprüche**

**1.** Verbindung der Formel

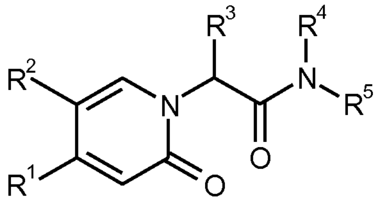 (I),

in welcher

$R^1$ für eine Gruppe der Formel

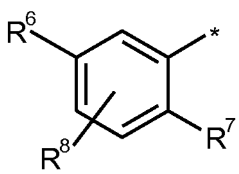

steht,
wobei * die Anknüpfstelle an den Oxopyridinring ist,

$R^6$ für Brom, Chlor, Fluor, Methyl, Difluormethyl, Trifluormethyl, Methoxy, Difluormethoxy oder Trifluormethoxy steht,
$R^7$ für Brom, Chlor, Fluor, Cyano, Nitro, Hydroxy, Methyl, Difluormethyl, Trifluormethyl, Methoxy, Ethoxy, Difluormethoxy, Trifluormethoxy, Ethinyl, 3,3,3-Trifluorprop-1-in-1-yl oder Cyclopropyl steht,
$R^8$ für Wasserstoff, Chlor oder Fluor steht,

$R^2$ für Chlor, Cyano, Methoxy, Ethoxy oder Difluormethoxy steht,
$R^3$ für Wasserstoff, $C_1$-$C_5$-Alkyl, $C_1$-$C_4$-Alkoxy, Difluormethyl, Trifluormethyl, 1,1-Difluorethyl, 1,1,2,2,2-Pentadeuteroethyl, 3,3,3-Trifluor-2-hydroxyprop-1-yl, 3,3,3-Trifluor-2-methoxyprop-1 -yl, 3,3,3 -Trifluor-2-ethoxyprop-1-yl, Prop-2-in-1-yl, Cyclopropyloxy oder Cyclobutyloxy steht,
wobei Alkyl substituiert sein kann mit einem Substituenten ausgewählt aus der Gruppe bestehend aus Fluor, Cyano, Hydroxy, Difluormethyl, Trifluormethyl, Methoxy, Ethoxy, Difluormethoxy, Trifluormethoxy, $C_3$-$C_6$-Cycloalkyl, 4- bis 6-gliedriges Oxo- Heterocyclyl, 4- bis 6-gliedriges Thio-Heterocyclyl, 1,4-Dioxanyl, Phenyl und Pyridyl,

worin Cycloalkyl substituiert sein kann mit 1 bis 2 Substituenten unabhängig voneinander ausgewählt aus der Gruppe bestehend aus Fluor, Hydroxy, Methyl, Ethyl, Methoxy, Ethoxy, Difluormethyl, Trifluormethyl, Difluormethoxy und Trifluormethoxy,
und
worin Oxo-Heterocyclyl und Thio-Heterocyclyl substituiert sein können mit 1 bis 2 Substituenten unabhängig voneinander ausgewählt aus der Gruppe bestehend aus Oxo, Fluor, Methyl, Ethyl, Difluormethyl und Trifluormethyl,

$R^4$ für Wasserstoff steht,
$R^5$ für eine Gruppe der Formel

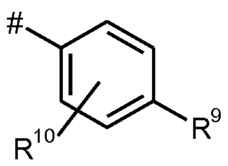 oder 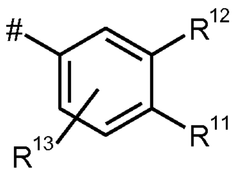 oder 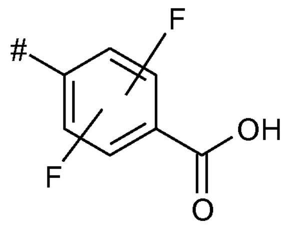

steht,
wobei # die Anknüpfstelle an das Stickstoffatom ist,

$R^9$ für Hydroxycarbonyl oder 5-gliedriges Heterocyclyl steht,
wobei Heterocyclyl substituiert sein kann mit 1 bis 2 Substituenten unabhängig voneinander ausgewählt aus der Gruppe bestehend aus Oxo, Hydroxy, Thioxo, Sulfanyl, Methyl, Difluormethyl, Trifluormethyl, 2-Hydroxycarbonyl-1,1,2,2-tetrafluorethyl und 2-Methoxycarbonyl-1,1,2,2-tetrafluorethyl,
worin Methyl substituiert sein kann mit einem Substituenten Methoxy,

$R^{10}$ für Wasserstoff, Chlor, Fluor oder Methyl steht,
$R^{11}$ und $R^{12}$ zusammen mit den Kohlenstoffatomen an die sie gebunden sind einen 5-gliedrigen Heterocyclus bilden,
wobei der Heterocyclus substituiert sein kann mit 1 bis 2 Substituenten unabhängig voneinander ausgewählt aus der Gruppe bestehend aus Oxo, Chlor, Hydroxy, Hydroxycarbonyl, Methyl, Difluormethyl, Trifluormethyl, 1,1,2,2,2-Pentafluorethyl, 2-Hydroxycarbonyl-1,1,2,2-tetrafluorethyl und 2-Methoxy-carbonyl-1,1,2,2-tetrafluorethyl,
$R^{13}$ für Wasserstoff, Chlor, Fluor, Methyl oder Methoxy steht,

oder eines ihrer Salze, ihrer Solvate oder der Solvate ihrer Salze.

**2.** Verbindung nach Anspruch 1, **dadurch gekennzeichnet, dass**

$R^1$ für eine Gruppe der Formel

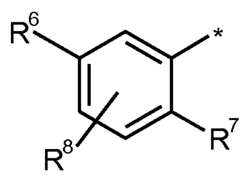

steht,
wobei * die Anknüpfstelle an den Oxopyridinring ist,

$R^6$ für Chlor steht,
$R^7$ für Brom, Chlor, Cyano, Nitro, Methyl, Difluormethyl, Trifluormethyl, Difluormethoxy, Trifluormethoxy, Ethinyl oder Cyclopropyl steht,
$R^8$ für Wasserstoff steht,

$R^2$ für Chlor, Cyano, Methoxy, Ethoxy oder Difluormethoxy steht,
$R^3$ für Wasserstoff, $C_1$-$C_5$-Alkyl, Ethoxy, 1,1,2,2,2-Pentadeuteroethyl oder Prop-2-in-1-yl steht,
wobei $C_1$-Alkyl substituiert sein kann mit einem Substituenten ausgewählt aus der Gruppe bestehend aus Difluormethyl, Trifluormethyl, Cyclopropyl, Cyclobutyl, Cyclohexyl, Oxetanyl, Tetrahydrofuranyl, Tetrahydro-2H-pyranyl, Tetrahydro-2H-thiopyranyl, 1,4-Dioxanyl, Phenyl und Pyridyl,

worin Cyclopropyl, Cyclobutyl, Cyclohexyl und Oxetanyl substituiert sein können mit 1 bis 2 Substituenten unabhängig voneinander ausgewählt aus der Gruppe bestehend aus Fluor, Hydroxy, Methyl, Ethyl, Methoxy und Trifluormethyl,
und

worin Tetrahydrofuranyl, Tetrahydro-2H-pyranyl und Tetrahydro-2H-thiopyranyl substituiert sein können mit 1 bis 2 Substituenten unabhängig voneinander ausgewählt aus der Gruppe bestehend aus Oxo, Methyl und Ethyl,

und
wobei $C_2$-$C_4$-Alkyl substituiert sein kann mit einem Substituenten ausgewählt aus der Gruppe bestehend aus Fluor, Hydroxy, Trifluormethyl, Methoxy und Trifluormethoxy,
$R^4$ für Wasserstoff steht,
$R^5$ für eine Gruppe der Formel

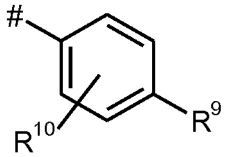
oder
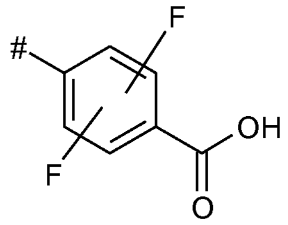

steht,
wobei # die Anknüpfstelle an das Stickstoffatom ist,
$R^9$ für Hydroxycarbonyl, Oxazolyl, Oxadiazolyl, Thiadiazolyl, Pyrazolyl, Imidazolyl, Triazolyl, Tetrazolyl oder Dihydrooxazolyl steht,
wobei Oxazolyl, Oxadiazolyl, Thiadiazolyl, Pyrazolyl, Imidazolyl, Triazolyl und Dihydrooxazolyl substituiert sein können mit 1 bis 2 Substituenten unabhängig voneinander ausgewählt aus der Gruppe bestehend aus Oxo, Hydroxy, Thioxo, Sulfanyl, Methyl, Trifluormethyl und 2-Hydroxycarbonyl-1,1,2,2-tetrafluorethyl,
worin Methyl substituiert sein kann mit einem Substituenten Methoxy,
$R^{10}$ für Wasserstoff, Chlor, Fluor oder Methyl steht,

oder

$R^5$ für 2,3-Dihydro-1H-indazol-6-yl, 1H-Benzimidazol-6-yl, Indol-6-yl, 2,3-Dihydro-1H-indazol-5-yl, 2,3-Dihydro-1H-benzimidazol-5-yl, Indol-5-yl, 1H-Indazol-6-yl oder 1H-Indazol-5-yl steht,
wobei der 5-gliedrige Heterocyclus in 2,3-Dihydro-1H-indazol-6-yl, 1H-Benzimidazol-6-yl, Indol-6-yl, 2,3-Dihydro-1H-indazol-5-yl, 2,3-Dihydro-1H-benzimidazol-5-yl, Indol-5-yl, 1H-Indazol-6-yl und 1H-Indazol-5-yl substituiert sein kann mit 1 bis 2 Substituenten unabhängig voneinander ausgewählt aus der Gruppe bestehend aus Oxo, Chlor, Hydroxycarbonyl, Methyl und Trifluormethyl,
und
wobei der Benzylring in 2,3-Dihydro-1H-indazol-6-yl, 1H-Benzimidazol-6-yl, Indol-6-yl, 2,3-Dihydro-1H-indazol-5-yl, 2,3-Dihydro-1H-benzimidazol-5-yl, Indol-5-yl, 1H-Indazol-6-yl und 1H-Indazol-5-yl substituiert sein kann mit einem Substituenten ausgewählt aus der Gruppe bestehend aus Fluor und Methoxy,

oder eines ihrer Salze, ihrer Solvate oder der Solvate ihrer Salze.

**3.** Verbindung nach einem der Ansprüche 1 oder 2, **dadurch gekennzeichnet, dass**

$R^1$ für eine Gruppe der Formel

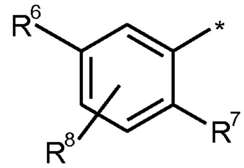

steht,
wobei * die Anknüpfstelle an den Oxopyridinring ist,

$R^6$ für Chlor steht,
$R^7$ für Cyano oder Difluormethoxy steht,
$R^8$ für Wasserstoff steht,

$R^2$ für Chlor, Cyano, Methoxy, Ethoxy oder Difluormethoxy steht,
$R^3$ für Methyl, Ethyl, n-Propyl, 2-Methyl-prop-1-yl oder n-Butyl steht,
wobei Methyl substituiert sein kann mit einem Substituenten ausgewählt aus der Gruppe bestehend aus Difluormethyl, Trifluormethyl, Cyclopropyl, Cyclobutyl, Cyclohexyl, Tetrahydrofuranyl, Tetrahydro-2H-pyranyl, 1,4-Dioxanyl, Phenyl und Pyridyl,
worin Cyclopropyl, Cyclobutyl und Cyclohexyl substituiert sein können mit 1 bis 2 Substituenten unabhängig voneinander ausgewählt aus der Gruppe bestehend aus Fluor, Hydroxy, Methyl, Methoxy und Trifluormethyl,
und
wobei Ethyl, n-Propyl und n-Butyl substituiert sein können mit einem Substituenten ausgewählt aus der Gruppe bestehend aus Fluor, Methoxy und Trifluormethoxy,
$R^4$ für Wasserstoff steht,
$R^5$ für eine Gruppe der Formel

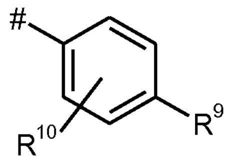

steht,
wobei # die Anknüpfstelle an das Stickstoffatom ist,

$R^9$ für Hydroxycarbonyl, Oxadiazolyl, Pyrazolyl, Triazolyl oder Tetrazolyl steht,

wobei Oxadiazolyl und Pyrazolyl substituiert sein können mit 1 bis 2 Substituenten unabhängig voneinander ausgewählt aus der Gruppe bestehend aus Oxo, Hydroxy und Trifluormethyl,
und
wobei Triazolyl substituiert sein kann mit einem Substituenten ausgewählt aus der Gruppe bestehend aus Trifluormethyl und 2-Hydroxycarbonyl-1,1,2,2-tetrafluorethyl,

$R^{10}$ für Wasserstoff oder Fluor steht,

oder
$R^5$ für 2,3-Dihydro-1H-indazol-6-yl, 1H-Benzimidazol-6-yl, 2,3-Dihydro-1H-benzimidazol-5-yl oder 1H-Indazol-5-yl steht,
wobei der 5-gliedrige Heterocyclus in 2,3-Dihydro-1H-indazol-6-yl substituiert sein kann mit 1 bis 2 Substituenten unabhängig voneinander ausgewählt aus der Gruppe bestehend aus Oxo und Methyl,
und
wobei der Benzylring in 2,3-Dihydro-1H-indazol-6-yl substituiert sein kann mit einem Substituenten Fluor,
und
wobei der 5-gliedrige Heterocyclus in 1H-Benzimidazol-6-yl substituiert sein kann mit einem Substituenten Hydroxycarbonyl,
und
wobei der 5-gliedrige Heterocyclus in 2,3-Dihydro-1H-benzimidazol-5-yl substituiert sein kann mit einem Substituenten Oxo,
und
wobei der 5-gliedrige Heterocyclus in 1H-Indazol-5-yl substituiert sein kann mit einem Substituenten Chlor,

oder eines ihrer Salze, ihrer Solvate oder der Solvate ihrer Salze.

4. Verfahren zur Herstellung einer Verbindung der Formel (I) oder eines ihrer Salze, ihrer Solvate oder der Solvate ihrer Salze nach Anspruch 1, **dadurch gekennzeichnet, dass** entweder

[A] eine Verbindung der Formel

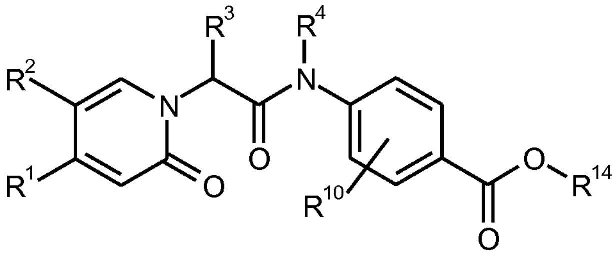

(IIa),

in welcher

$R^1$, $R^2$, $R^3$, $R^4$ und $R^{10}$ die in Anpruch 1 angegebene Bedeutung haben, und
$R^{14}$ für tert-Butyl steht,

mit einer Säure zu einer Verbindung der Formel

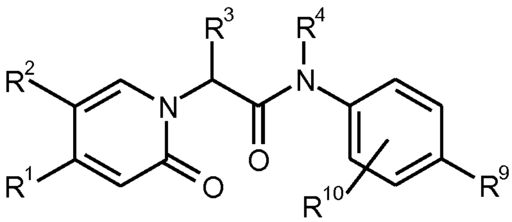

(Ib),

in welcher

$R^1$, $R^2$, $R^3$, $R^4$ und $R^{10}$ die in Anpruch 1 angegebene Bedeutung haben, und
$R^9$ für Hydroxycarbonyl steht,

umgesetzt wird,
oder
[B] eine Verbindung der Formel

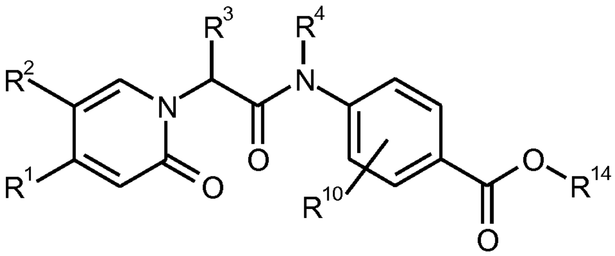

(IIb),

in welcher

$R^1$, $R^2$, $R^3$, $R^4$ und $R^{10}$ die in Anpruch 1 angegebene Bedeutung haben, und
$R^{14}$ für Methyl oder Ethyl steht,

mit einer Base zu einer Verbindung der Formel

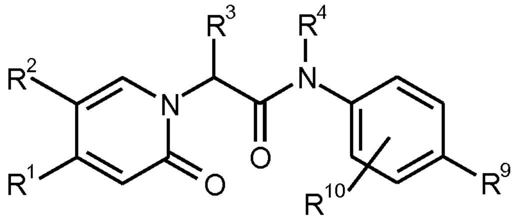 (Ib),

in welcher

$R^1$, $R^2$, $R^3$, $R^4$ und $R^{10}$ die in Anpruch 1 angegebene Bedeutung haben, und
$R^9$ für Hydroxycarbonyl steht,

umgesetzt wird,
oder
[C] eine Verbindung der Formel

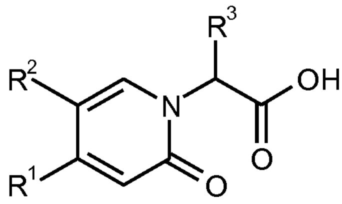 (III),

in welcher
$R^1$, $R^2$ und $R^3$ die in Anpruch 1 angegebene Bedeutung haben,
mit einer Verbindung der Formel

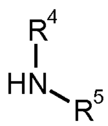 (IV),

in welcher
$R^4$ und $R^5$ die in Anpruch 1 angegebene Bedeutung haben,
in Gegenwart eines Dehydratisierungsreagenzes zu einer Verbindung der Formel (I) umgesetzt wird,
oder
[D] eine Verbindung der Formel

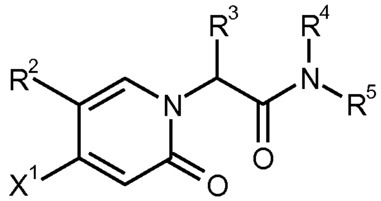 (V),

in welcher

$R^2$, $R^3$, $R^4$ und $R^5$ die in Anpruch 1 angegebene Bedeutung haben, und
$X^1$ für Chlor, Brom oder Iod steht,

mit einer Verbindung der Formel

$R^1$-Q (VI),

in welcher

$R^1$ die in Anpruch 1 angegebene Bedeutung hat, und
Q für $-B(OH)_2$, einen Boronsäure-Ester, bevorzugt Boronsäurepinakolester, oder $-BF_3^-K^+$ steht,

unter Suzuki-Kupplungsbedingungen zu einer Verbindung der Formel (I) umgesetzt wird.

**5.** Verbindung nach einem der Ansprüche 1 bis 3 zur Behandlung und/oder Prophylaxe von Krankheiten.

**6.** Verwendung einer Verbindung nach einem der Ansprüche 1 bis 3 zur Herstellung eines Arzneimittels zur Behandlung und/oder Prophylaxe von Krankheiten.

**7.** Verwendung einer Verbindung nach einem der Ansprüche 1 bis 3 zur Herstellung eines Arzneimittels zur Behandlung und/oder Prophylaxe von thrombotischen beziehungsweise thromboembolischen Erkrankungen.

**8.** Verwendung einer Verbindung nach einem der Ansprüche 1 bis 3 zur Herstellung eines Arzneimittels zur Behandlung und/oder Prophylaxe von ophthalmologischen Erkrankungen.

**9.** Arzneimittel enthaltend eine Verbindung nach einem der Ansprüche 1 bis 3 in Kombination mit einem inerten, nichttoxischen, pharmazeutisch geeigneten Hilfsstoff.

**10.** Arzneimittel nach Anspruch 9 zur Behandlung und/oder Prophylaxe von thrombotischen beziehungsweise thromboembolischen Erkrankungen.

**11.** Arzneimittel nach Anspruch 9 zur Behandlung und/oder Prophylaxe von ophthalmologischen Erkrankungen.

**12.** ({2-[4-(5-Chlor-2-cyanophenyl)-5-methoxy-2-oxopyridin-1(2*H*)-yl]-3-[tetrahydro-2*H*-pyran-2-yl]propanoyl}amino)benzoesäure (Gemisch racemischer Diastereomere) nach Anspruch 1 mit der folgenden Formel

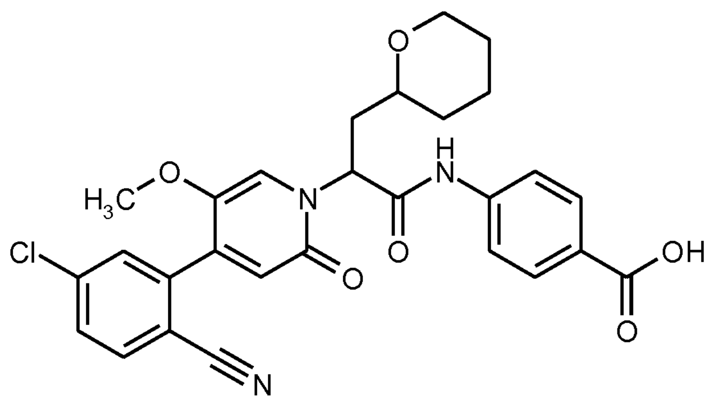

oder eines ihrer Salze, ihrer Solvate oder der Solvate ihrer Salze.

**13.** ({2-[4-(5-Chlor-2-cyanophenyl)-5-methoxy-2-oxopyridin-1(2*H*)-yl]-3-[tetrahydro-2*H*-pyran-2-yl]propanoyl}amino)benzoesäure (Enantiomer 1 des 2. Diastereomers) nach Anspruch 1 mit der folgenden Formel

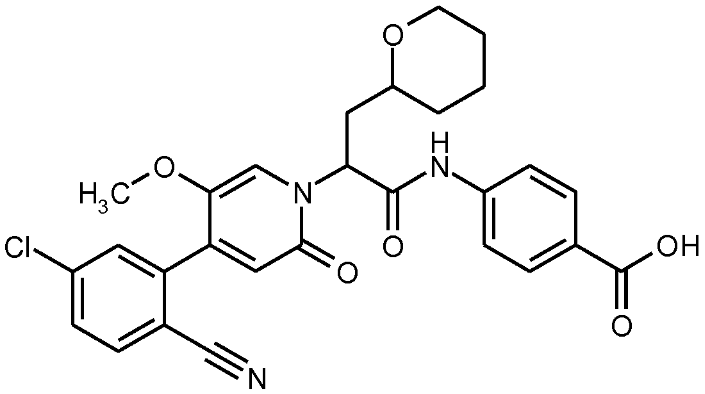

oder eines ihrer Salze, ihrer Solvate oder der Solvate ihrer Salze.

## Claims

**1.** Compound of the formula

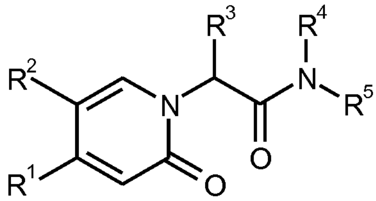

(I),

in which

$R^1$ represents a group of the formula

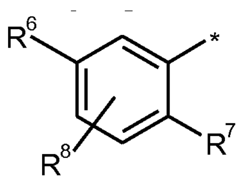

where * is the point of attachment to the oxopyridine ring,

$R^6$ represents bromine, chlorine, fluorine, methyl, difluoromethyl, trifluoromethyl, methoxy, difluoromethoxy or trifluoromethoxy,
$R^7$ represents bromine, chlorine, fluorine, cyano, nitro, hydroxy, methyl, difluoromethyl, trifluoromethyl, methoxy, ethoxy, difluoromethoxy, trifluoromethoxy, ethynyl, 3,3,3-trifluoroprop-1-yn-1-yl or cyclopropyl,
$R^2$ $R^8$ represents hydrogen, chlorine or fluorine, represents chlorine, cyano, methoxy, ethoxy or difluoromethoxy,
$R^3$ represents hydrogen, $C_1$-$C_5$-alkyl, $C_1$-$C_4$-alkoxy, difluoromethyl, trifluoromethyl, 1,1-difluoroethyl, 1,1,2,2,2-pentadeuteroethyl, 3,3,3-trifluoro-2-hydroxyprop-1-yl, 3,3,3-trifluoro-2-methoxyprop-1-yl, 3,3,3-trifluoro-2-ethoxyprop-1-yl, prop-2-yn-1-yl, cyclopropyloxy or cyclobutyloxy,
where alkyl may be substituted by a substituent selected from the group consisting of fluorine, cyano, hydroxy, difluoromethyl, trifluoromethyl, methoxy, ethoxy, difluoromethoxy, trifluoromethoxy, $C_3$-$C_6$-cycloalkyl, 4- to 6-membered oxoheterocyclyl, 4- to 6-membered thioheterocyclyl, 1,4-dioxanyl, phenyl and pyridyl,

where cycloalkyl may be substituted by 1 to 2 substituents independently of one another selected from

the group consisting of fluorine, hydroxy, methyl, ethyl, methoxy, ethoxy, difluoromethyl, trifluoromethyl, difluoromethoxy and trifluoromethoxy,
and
where oxoheterocyclyl and thioheterocyclyl may be substituted by 1 to 2 substituents independently of one another selected from the group consisting of oxo, fluoro, methyl, ethyl, difluoromethyl and trifluoromethyl,

$R^4$ represents hydrogen,
$R^5$ represents a group of the formula

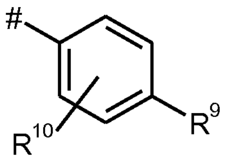
or
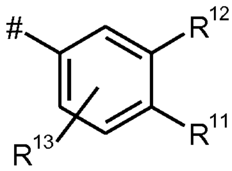
or
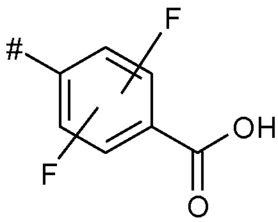

where # is the point of attachment to the nitrogen atom,

$R^9$ represents hydroxycarbonyl or 5-membered heterocyclyl, where heterocyclyl may be substituted by 1 to 2 substituents independently of one another selected from the group consisting of oxo, hydroxy, thioxo, sulphanyl, methyl, difluoromethyl, trifluoromethyl, 2-hydroxycarbonyl-1,1,2,2-tetrafluoroethyl and 2-methoxycarbonyl-1,1,2,2-tetrafluoroethyl,
where methyl may be substituted by a methoxy substituent,
$R^{10}$ represents hydrogen, chlorine, fluorine or methyl,
$R^{11}$ and $R^{12}$ together with the carbon atoms to which they are attached form a 5-membered heterocycle, where the heterocycle may be substituted by 1 to 2 substituents independently of one another selected from the group consisting of oxo, chlorine, hydroxy, hydroxycarbonyl, methyl, difluoromethyl, trifluoromethyl, 1,1,2,2,2-pentafluoroethyl, 2-hydroxycarbonyl-1,1,2,2-tetrafluoroethyl and 2-methoxycarbonyl-1,1,2,2-tetrafluoroethyl,
$R^{13}$ represents hydrogen, chlorine, fluorine, methyl or methoxy,

or one of the salts thereof, solvates thereof or solvates of the salts thereof.

**2.** Compound according to Claim 1, **characterized in that**

$R^1$ represents a group of the formula

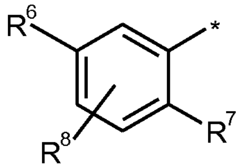

where * is the point of attachment to the oxopyridine ring,

$R^6$ represents chlorine,
$R^7$ represents bromine, chlorine, cyano, nitro, methyl, difluoromethyl, trifluoromethyl, difluoromethoxy, tri-

fluoromethoxy, ethynyl or cyclopropyl,
$R^8$ represents hydrogen,

$R^2$ represents chlorine, cyano, methoxy, ethoxy or difluoromethoxy,
$R^3$ represents hydrogen, $C_1$-$C_5$-alkyl, ethoxy, 1,1,2,2,2-pentadeuteroethyl or prop-2-yn-1-yl, where $C_1$-alkyl may be substituted by a substituent selected from the group consisting of difluoromethyl, trifluoromethyl, cyclopropyl, cyclobutyl, cyclohexyl, oxetanyl, tetrahydrofuranyl, tetrahydro-2H-pyranyl, tetrahydro-2H-thiopyranyl, 1,4-dioxanyl, phenyl and pyridyl,

where cyclopropyl, cyclobutyl, cyclohexyl and oxetanyl may be substituted by 1 to 2 substituents independently of one another selected from the group consisting of fluorine, hydroxy, methyl, ethyl, methoxy and trifluoromethyl,
and
where tetrahydrofuranyl, tetrahydro-2H-pyranyl and tetrahydro-2H-thiopyranyl may be substituted by 1 to 2 substituents independently of one another selected from the group consisting of oxo, methyl and ethyl,

and
where $C_2$-$C_4$-alkyl may be substituted by a substituent selected from the group consisting of fluorine, hydroxy, trifluoromethyl, methoxy and trifluoromethoxy,
$R^4$ represents hydrogen,
$R^5$ represents a group of the formula

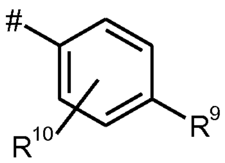

or

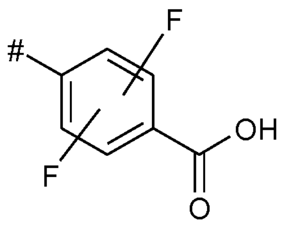

where # is the point of attachment to the nitrogen atom,

$R^9$ represents hydroxycarbonyl, oxazolyl, oxadiazolyl, thiadiazolyl, pyrazolyl, imidazolyl, triazolyl, tetrazolyl or dihydrooxazolyl,
where oxazolyl, oxadiazolyl, thiadiazolyl, pyrazolyl, imidazolyl, triazolyl and dihydrooxazolyl may be substituted by 1 to 2 substituents independently of one another selected from the group consisting of oxo, hydroxy, thioxo, sulphanyl, methyl, trifluoromethyl and 2-hydroxycarbonyl-1,1,2,2-tetrafluoroethyl,
where methyl may be substituted by a methoxy substituent,
$R^{10}$ represents hydrogen, chlorine, fluorine or methyl,

or
$R^5$ represents 2,3-dihydro-1H-indazol-6-yl, 1H-benzimidazol-6-yl, indol-6-yl, 2,3-dihydro-1H-indazol-5-yl, 2,3-dihydro-1H-benzimidazol-5-yl, indol-5-yl, 1H-indazol-6-yl or 1H-indazol-5-yl, where the 5-membered heterocycle in 2,3-dihydro-1H-indazol-6-yl, 1H-benzimidazol-6-yl, indol-6-yl, 2,3-dihydro-1H-indazol-5-yl, 2,3-dihydro-1H-benzimidazol-5-yl, indol-5-yl, 1H-indazol-6-yl and 1H-indazol-5-yl may be substituted by 1 to 2 substituents independently of one another selected from the group consisting of oxo, chlorine, hydroxycarbonyl, methyl and trifluoromethyl,
and
where the benzyl ring in 2,3-dihydro-1H-indazol-6-yl, 1H-benzimidazol-6-yl, indol-6-yl, 2,3-dihydro-1H-indazol-5-yl, 2,3-dihydro-1H-benzimidazol-5-yl, indol-5-yl, 1H-indazol-6-yl and 1H-indazol-5-yl may be substituted by a substituent selected from the group consisting of fluorine and methoxy,

or one of the salts thereof, solvates thereof or solvates of the salts thereof.

3. Compound according to either of Claims 1 and 2, **characterized in that**

$R^1$ represents a group of the formula

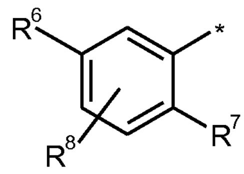

where * is the point of attachment to the oxopyridine ring,

$R^6$ represents chlorine, $R^7$ represents cyano or difluoromethoxy,
$R^2$ $R^8$ represents hydrogen, represents chlorine, cyano, methoxy, ethoxy or difluoromethoxy,
$R^3$ represents methyl, ethyl, n-propyl, 2-methylprop-1-yl or n-butyl, where methyl may be substituted by a substituent selected from the group consisting of difluoromethyl, trifluoromethyl, cyclopropyl, cyclobutyl, cyclohexyl, tetrahydrofuranyl, tetrahydro-2H-pyranyl, 1,4-dioxanyl, phenyl and pyridyl,
where cyclopropyl, cyclobutyl and cyclohexyl may be substituted by 1 to 2 substituents independently of one another selected from the group consisting of fluorine, hydroxy, methyl, methoxy and trifluoromethyl,
and
where ethyl, n-propyl and n-butyl may be substituted by a substituent selected from the group consisting of fluorine, methoxy and trifluoromethoxy,

$R^4$ represents hydrogen,
$R^5$ represents a group of the formula

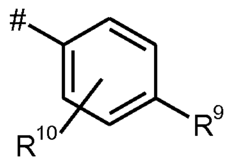

where # is the point of attachment to the nitrogen atom,

$R^9$ represents hydroxycarbonyl, oxadiazolyl, pyrazolyl, triazolyl or tetrazolyl,
where oxadiazolyl and pyrazolyl may be substituted by 1 to 2 substituents independently of one another selected from the group consisting of oxo, hydroxy and trifluoromethyl,
and
where triazolyl may be substituted by a substituent selected from the group consisting of trifluoromethyl and 2-hydroxycarbonyl-1,1,2,2-tetrafluoroethyl,
$R^{10}$ represents hydrogen or fluorine,

or
$R^5$ represents 2,3-dihydro-1H-indazol-6-yl, 1H-benzimidazol-6-yl, 2,3-dihydro-1H-benzimidazol-5-yl or 1H-indazol-5-yl, where the 5-membered heterocycle in 2,3-dihydro-1H-indazol-6-yl may be substituted by 1 to 2 substituents independently of one another selected from the group consisting of oxo and methyl,
and
where the benzyl ring in 2,3-dihydro-1H-indazol-6-yl may be substituted by a fluorine substituent,
and
where the 5-membered heterocycle in 1H-benzimidazol-6-yl may be substituted by a hydroxycarbonyl substituent,
and
where the 5-membered heterocycle in 2,3-dihydro-1H-benzimidazol-5-yl may be substituted by an oxo substituent,
and
where the 5-membered heterocycle in 1H-indazol-5-yl may be substituted by a chlorine substituent,

or one of the salts thereof, solvates thereof or solvates of the salts thereof.

4. Process for preparing a compound of the formula (I) or one of the salts thereof, solvates thereof or solvates of the

salts thereof according to Claim 1, **characterized in that** either

[A] a compound of the formula

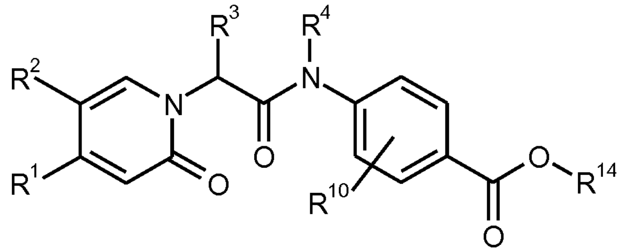

(IIa),

in which

$R^1$, $R^2$, $R^3$, $R^4$ and $R^{10}$ have the meaning given in Claim 1, and
$R^{14}$ represents tert-butyl,

is reacted with an acid to give a compound of the formula

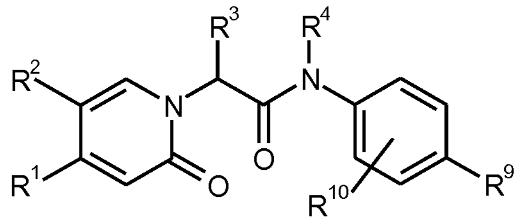

(Ib),

in which

$R^1$, $R^2$, $R^3$, $R^4$ and $R^{10}$ have the meaning given in Claim 1, and
$R^9$ represents hydroxycarbonyl,

or
[B] a compound of the formula

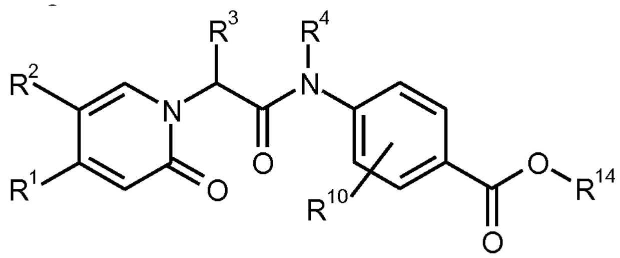

(IIb),

in which

$R^1$, $R^2$, $R^3$, $R^4$ and $R^{10}$ have the meaning given in Claim 1, and
$R^{14}$ represents methyl or ethyl,

is reacted with a base to give a compound of the formula

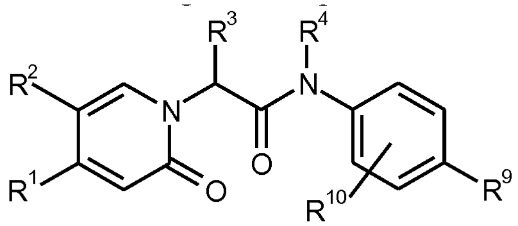

(Ib),

in which

$R^1$, $R^2$, $R^3$, $R^4$ and $R^{10}$ have the meaning given in Claim 1, and
$R^9$ represents hydroxycarbonyl,

or
[C] a compound of the formula

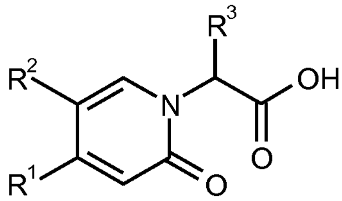

(III),

in which
$R^1$, $R^2$ and $R^3$ have the meaning given in Claim 1,
is reacted with a compound of the formula

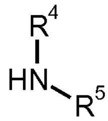

(IV),

in which
$R^4$ and $R^5$ have the meaning given in Claim 1,
in the presence of a dehydrating agent to give a compound of the formula (I),
or
[D] a compound of the formula

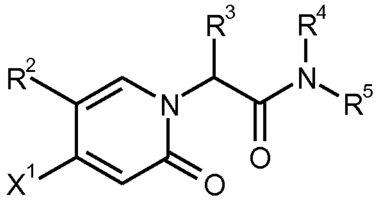

(V),

in which

$R^2$, $R^3$, $R^4$ and $R^5$ have the meaning given in Claim 1, and
$X^1$ represents chlorine, bromine or iodine,

is reacted with a compound of the formula

$R^1$-Q (VI),

in which

$R^1$ has the meaning given in Claim 1, and
Q represents $-B(OH)_2$, a boronic ester, preferably boronic acid pinacol ester, or $-BF_3^-K^+$,

under Suzuki coupling conditions to give a compound of the formula (I).

**5.** Compound according to any of Claims 1 to 3 for the treatment and/or prophylaxis of diseases.

**6.** Use of a compound according to any of Claims 1 to 3 for producing a medicament for the treatment and/or prophylaxis of diseases.

**7.** Use of a compound according to any of Claims 1 to 3 for producing a medicament for the treatment and/or prophylaxis of thrombotic or thromboembolic disorders.

**8.** Use of a compound according to any of Claims 1 to 3 for producing a medicament for the treatment and/or prophylaxis of ophthalmic disorders.

**9.** Medicament comprising a compound according to any of Claims 1 to 3 in combination with an inert, nontoxic, pharmaceutically suitable excipient.

**10.** Medicament according to Claim 9 for the treatment and/or prophylaxis of thrombotic or thromboembolic disorders.

**11.** Medicament according to Claim 9 for the treatment and/or prophylaxis of ophthalmic disorders.

**12.** ({2-[4-(5-Chloro-2-cyanophenyl)-5-methoxy-2-oxopyridin-1(2*H*)-yl]-3-[tetrahydro-2*H*-pyran-2-yl]propanoyl}amino)benzoic acid (mixture of racemic diastereomers) according to Claim 1 having the following formula

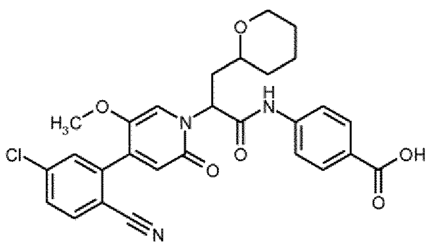

or one of the salts thereof, solvates thereof or solvates of the salts thereof.

**13.** ({2-[4-(5-Chloro-2-cyanophenyl)-5-methoxy-2-oxopyridin-1(2*H*)-yl]-3-[tetrahydro-2*H*-pyran-2-yl]propanoyl}amino)benzoic acid (enantiomer 1 of the 2nd diastereomer) according to Claim 1 having the following formula

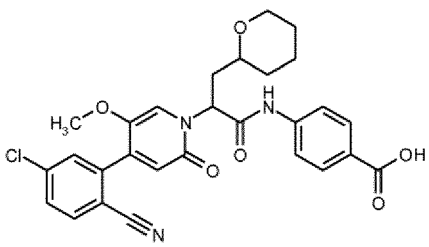

or one of the salts thereof, solvates thereof or solvates of the salts thereof.

**Revendications**

1. Composé de formule

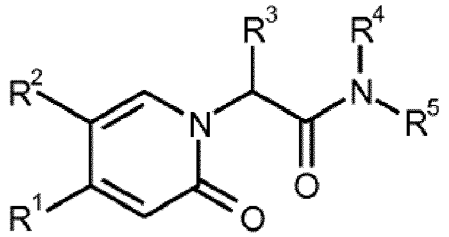

(I),

dans laquelle

$R^1$ représente un groupe de formule

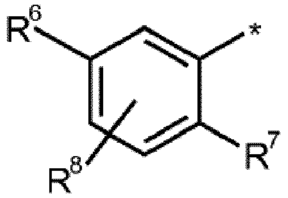

dans laquelle * est l'emplacement de liaison au cycle oxopyridine,
$R^6$ représente brome, chlore, fluor, méthyle, difluorométhyle, trifluorométhyle, méthoxy, difluorométhoxy ou trifluorométhoxy,
$R^7$ représente brome, chlore, fluor, cyano, nitro, hydroxy, méthyle, difluorométhyle, trifluorométhyle, méthoxy, éthoxy, difluorométhoxy, trifluorométhoxy, éthynyle, 3,3,3-trifluoroprop-1-yn-1-yle ou cyclopropyle,
$R^8$ représente hydrogène, chlore ou fluor,

$R^2$ représente chlore, cyano, méthoxy, éthoxy ou difluorométhoxy,
$R^3$ représente hydrogène, alkyle en $C_1$-$C_5$, alcoxy en $C_1$-$C_4$, difluorométhyle, trifluorométhyle, 1,1-difluoroéthyle, 1,1,2,2,2-pentadeutéroéthyle, 3,3,3-trifluoro-2-hydroxyprop-1-yle, 3,3,3-trifluoro-2-méthoxyprop-1-yle, 3,3,3-trifluoro-2-éthoxyprop-1-yle, prop-2-yn-1-yle, cyclopropyloxy ou cyclobutyloxy,
l'alkyle pouvant être substitué avec un substituant choisi dans le groupe constitué par fluor, cyano, hydroxy, difluorométhyle, trifluorométhyle, méthoxy, éthoxy, difluorométhoxy, trifluorométhoxy, cycloalkyle en $C_3$-$C_6$, oxo-hétérocyclyle de 4 à 6 chaînons, thio-hétérocyclyle de 4 à 6 chaînons, 1,4-dioxanyle, phényle et pyridyle,
le cycloalkyle pouvant être substitué avec 1 à 2 substituants choisis indépendamment l'un de l'autre dans le groupe constitué par fluor, hydroxy, méthyle, éthyle, méthoxy, éthoxy, difluorométhyle, trifluorométhyle, difluorométhoxy et trifluorométhoxy,
et
l'oxo-hétérocyclyle et le thio-hétérocyclyle pouvant être substitués avec 1 à 2 substituants choisis indépendamment l'un de l'autre dans le groupe constitué par oxo, fluor, méthyle, éthyle, difluorométhyle et trifluorométhyle,
$R^4$ représente hydrogène,
$R^5$ représente un groupe de formule

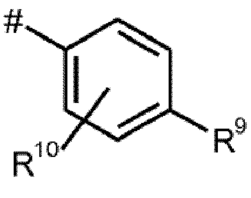

ou

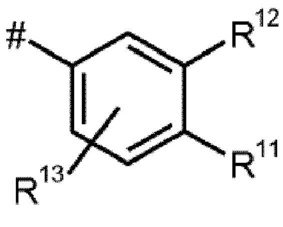

ou

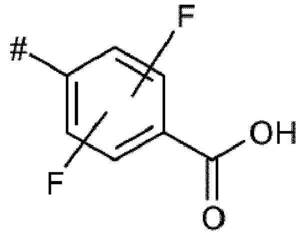

dans lesquelles # est l'emplacement de liaison à l'atome d'azote,

$R^9$ représente hydroxycarbonyle ou hétérocyclyle à 5 chaînons,
l'hétérocyclyle pouvant être substitué avec 1 à 2 substituants choisis indépendamment l'un de l'autre dans le groupe constitué par oxo, hydroxy, thioxo, sulfanyle, méthyle, difluorométhyle, trifluorométhyle, 2-hydroxycarbonyl-1,1,2,2-tétrafluoroéthyle et 2-méthoxycarbonyl-1,1,2,2-tétrafluoroéthyle,
le méthyle pouvant être substitué avec un substituant méthoxy,
$R^{10}$ représente hydrogène, chlore, fluor ou méthyle,
$R^{11}$ et $R^{12}$ forment ensemble avec les atomes de carbone auxquels ils sont reliés un hétérocycle à 5 chaînons,
l'hétérocycle pouvant être substitué avec 1 à 2 substituants choisis indépendamment l'un de l'autre dans le groupe constitué par oxo, chlore, hydroxy, hydroxycarbonyle, méthyle, difluorométhyle, trifluorométhyle, 1,1,2,2,2-pentafluoroéthyle, 2-hydroxycarbonyl-1,1,2,2-tétrafluoroéthyle et 2-méthoxy-carbonyl-1,1,2,2-tétrafluoroéthyle,
$R^{13}$ représente hydrogène, chlore, fluor, méthyle ou méthoxy,

ou un de ses sels, de ses solvates ou des solvates de ses sels.

**2.** Composé selon la revendication 1, **caractérisé en ce que**

$R^1$ représente un groupe de formule

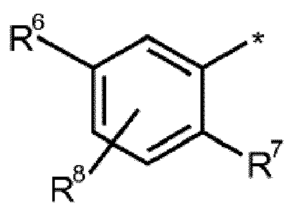

dans laquelle * est l'emplacement de liaison au cycle oxopyridine,
$R^6$ représente chlore,
$R^7$ représente brome, chlore, cyano, nitro, méthyle, difluorométhyle, trifluorométhyle, difluorométhoxy, trifluorométhoxy, éthynyle ou cyclopropyle,
$R^8$ représente hydrogène,

$R^2$ représente chlore, cyano, méthoxy, éthoxy ou difluorométhoxy,
$R^3$ représente hydrogène, alkyle en $C_1$-$C_5$, éthoxy, 1,1,2,2,2-pentadeutéroéthyle ou prop-2-yn-1-yle,
l'alkyle en $C_1$ pouvant être substitué avec un substituant choisi dans le groupe constitué par difluorométhyle, trifluorométhyle, cyclopropyle, cyclobutyle, cyclohexyle, oxétanyle, tétrahydrofuranyle, tétrahydro-2H-pyranyle, tétrahydro-2H-thiopyranyle, 1,4-dioxanyle, phényle et pyridyle,
le cyclopropyle, le cyclobutyle, le cyclohexyle et l'oxétanyle pouvant être substitués avec 1 à 2 substituants choisis indépendamment l'un de l'autre dans le groupe constitué par fluor, hydroxy, méthyle, éthyle, méthoxy et trifluorométhyle,
et
le tétrahydrofuranyle, le tétrahydro-2H-pyranyle et le tétrahydro-2H-thiopyranyle pouvant être substitués avec 1 à 2 substituants choisis indépendamment l'un de l'autre dans le groupe constitué par oxo, méthyle et éthyle,
et
l'alkyle en $C_2$-$C_4$ pouvant être substitué avec un substituant choisi dans le groupe constitué par fluor, hydroxy, trifluorométhyle, méthoxy et trifluorométhoxy,
$R^4$ représente hydrogène,
$R^5$ représente un groupe de formule

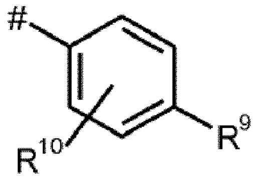

ou

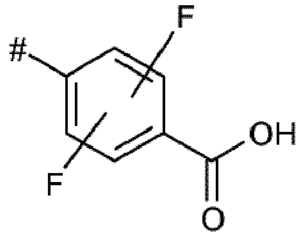

dans lesquelles # est l'emplacement de liaison à l'atome d'azote,
$R^9$ représente hydroxycarbonyle, oxazolyle, oxadiazolyle, thiadiazolyle, pyrazolyle, imidazolyle, triazolyle, tétrazolyle ou dihydrooxazolyle,
l'oxazolyle, l'oxadiazolyle, le thiadiazolyle, le pyrazolyle, l'imidazolyle, le triazolyle et le dihydrooxazolyle pouvant être substitués avec 1 à 2 substituants choisis indépendamment l'un de l'autre dans le groupe constitué par oxo, hydroxy, thioxo, sulfanyle, méthyle, trifluorométhyle et 2-hydroxycarbonyl-1,1,2,2-tétrafluoroéthyle,
le méthyle pouvant être substitué avec un substituant méthoxy,
$R^{10}$ représente hydrogène, chlore, fluor ou méthyle,
ou

$R^5$ représente 2,3-dihydro-1H-indazol-6-yle, 1H-benzimidazol-6-yle, indol-6-yle, 2,3-dihydro-1H-indazol-5-yle, 2,3-dihydro-1H-benzimidazol-5-yle, indol-5-yle, 1H-indazol-6-yle ou 1H-indazol-5-yle,
l'hétérocycle à 5 chaînons dans le 2,3-dihydro-1H-indazol-6-yle, le 1H-benzimidazol-6-yle, l'indol-6-yle, le 2,3-dihydro-1H-indazol-5-yle, le 2,3-dihydro-1H-benzimidazol-5-yle, l'indol-5-yle, le 1H-indazol-6-yle et le 1H-indazol-5-yle pouvant être substitué avec 1 à 2 substituants choisis indépendamment l'un de l'autre dans le groupe constitué par oxo, chlore, hydroxycarbonyle, méthyle et trifluorométhyle,
et
le cycle benzyle dans le 2,3-dihydro-1H-indazol-6-yle, le 1H-benzimidazol-6-yle, l'indol-6-yle, le 2,3-dihydro-1H-indazol-5-yle, le 2,3-dihydro-1H-benzimidazol-5-yle, l'indol-5-yle, le 1H-indazol-6-yle et le 1H-indazol-5-yle pouvant être substitué avec un substituant choisi dans le groupe constitué par fluor et méthoxy,

ou un de ses sels, de ses solvates ou des solvates de ses sels.

3. Composé selon l'une quelconque des revendications 1 ou 2, **caractérisé en ce que**

$R^1$ représente un groupe de formule

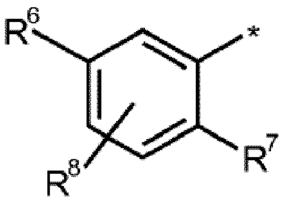

dans laquelle * est l'emplacement de liaison au cycle oxopyridine,
$R^6$ représente chlore,
$R^7$ représente cyano ou difluorométhoxy,
$R^8$ représente hydrogène,

$R^2$ représente chlore, cyano, méthoxy, éthoxy ou difluorométhoxy,
$R^3$ représente méthyle, éthyle, n-propyle, 2-méthyl-prop-1-yle ou n-butyle,
le méthyle pouvant être substitué avec un substituant choisi dans le groupe constitué par difluorométhyle, trifluorométhyle, cyclopropyle, cyclobutyle, cyclohexyle, tétrahydrofuranyle, tétrahydro-2H-pyranyle, 1,4-dioxanyle, phényle et pyridyle,
le cyclopropyle, le cyclobutyle et le cyclohexyle pouvant être substitués avec 1 à 2 substituants choisis indépendamment l'un de l'autre dans le groupe constitué par fluor, hydroxy, méthyle, méthoxy et trifluorométhyle,
et

l'éthyle, le n-propyle et n-butyle pouvant être substitués avec un substituant choisi dans le groupe constitué par fluor, méthoxy et trifluorométhoxy,
$R^4$ représente hydrogène,
$R^5$ représente un groupe de formule

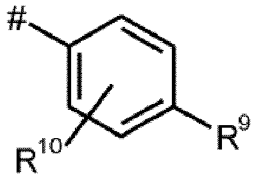

dans laquelle # est l'emplacement de liaison à l'atome d'azote,
$R^9$ représente hydroxycarbonyle, oxadiazolyle, pyrazolyle, triazolyle ou tétrazolyle,
l'oxadiazolyle et le pyrazolyle pouvant être substitués avec 1 à 2 substituants choisis indépendamment l'un de l'autre dans le groupe constitué par oxo, hydroxy et trifluorométhyle,
et
le triazolyle pouvant être substitué avec un substituant choisi dans le groupe constitué par trifluorométhyle et 2-hydroxycarbonyl-1,1,2,2-tétrafluoroéthyle,
$R^{10}$ représente hydrogène ou fluor,

ou
$R^5$ représente 2,3-dihydro-1H-indazol-6-yle, 1H-benzimidazol-6-yle, 2,3-dihydro-1H-benzimidazol-5-yle ou 1H-indazol-5-yle,
l'hétérocycle à 5 chaînons dans le 2,3-dihydro-1H-indazol-6-yle pouvant être substitué avec 1 à 2 substituants choisis indépendamment l'un de l'autre dans le groupe constitué par oxo et méthyle,
et
le cycle benzyle dans le 2,3-dihydro-1H-indazol-6-yle pouvant être substitué avec un substituant fluor,
et
l'hétérocycle à 5 chaînons dans le 1H-benzimidazol-6-yle pouvant être substitué avec un substituant hydroxycarbonyle,
et
l'hétérocycle à 5 chaînons dans le 2,3-dihydro-1H-benzimidazol-5-yle pouvant être substitué avec un substituant oxo,
et
l'hétérocycle à 5 chaînons dans le 1H-indazol-5-yle pouvant être substitué avec un substituant chlore, ou un de ses sels, de ses solvates ou des solvates de ses sels.

**4.** Procédé de fabrication d'un composé de formule (I) ou d'un de ses sels, de ses solvates ou des solvates de ses sels selon la revendication 1, **caractérisé en ce que** soit

[A] un composé de formule

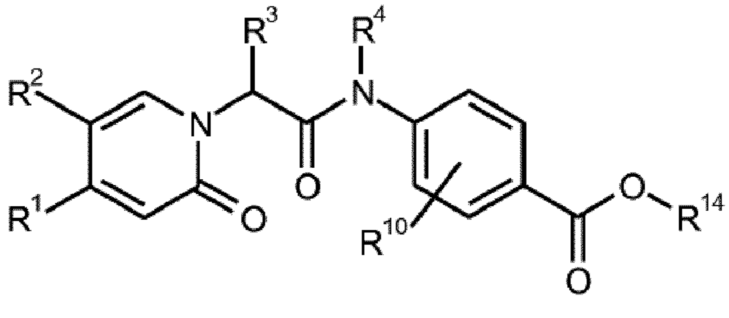 (IIa),

dans laquelle

$R^1$, $R^2$, $R^3$, $R^4$ et $R^{10}$ ont la signification indiquée dans la revendication 1, et
$R^{14}$ représente tert-butyle,

est mis en réaction avec un acide pour former un composé de formule

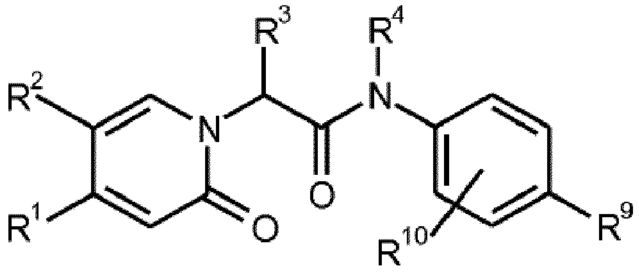

(Ib),

dans laquelle

$R^1$, $R^2$, $R^3$, $R^4$ et $R^{10}$ ont la signification indiquée dans la revendication 1, et $R^9$ représente hydroxycarbonyle,

soit
[B] un composé de formule

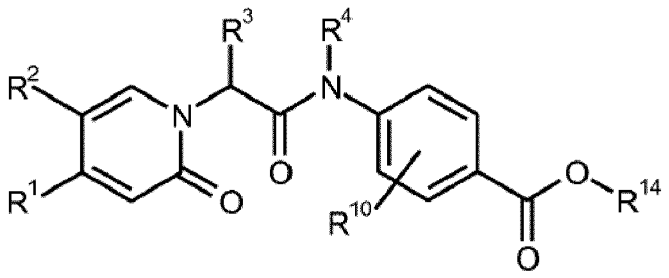

(IIb),

dans laquelle

$R^1$, $R^2$, $R^3$, $R^4$ et $R^{10}$ ont la signification indiquée dans la revendication 1, et $R^{14}$ représente méthyle ou éthyle,

est mis en réaction avec une base pour former un composé de formule

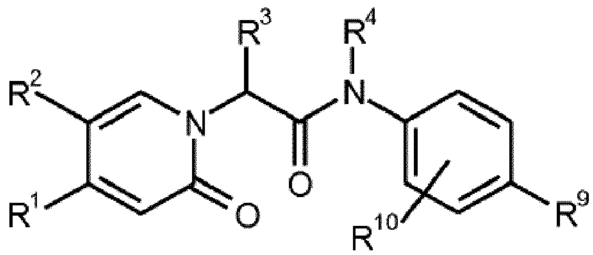

(Ib),

dans laquelle

$R^1$, $R^2$, $R^3$, $R^4$ et $R^{10}$ ont la signification indiquée dans la revendication 1, et $R^9$ représente hydroxycarbonyle,

soit
[C] un composé de formule

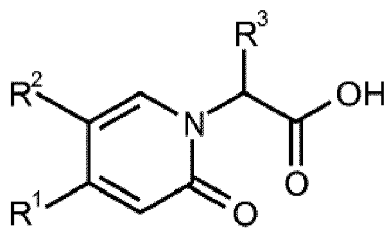 (III),

dans laquelle
$R^1$, $R^2$ et $R^3$ ont la signification indiquée dans la revendication 1,
est mis en réaction avec un composé de formule

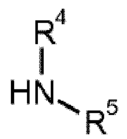 (IV),

dans laquelle
$R^4$ et $R^5$ ont la signification indiquée dans la revendication 1,
en présence d'un réactif de déshydratation pour former un composé de formule (I),
soit
[D] un composé de formule

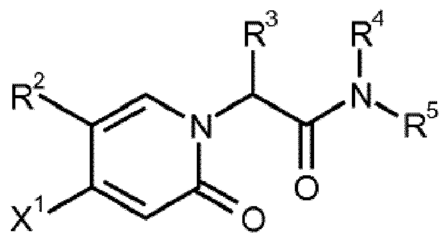 (V),

dans laquelle
$R^2$, $R^3$, $R^4$ et $R^5$ ont la signification indiquée dans la revendication 1, et
$X^1$ représente chlore, brome ou iode,
est mis en réaction avec un composé de formule

$R^1$-Q (VI)

dans laquelle

$R^1$ a la signification indiquée dans la revendication 1, et
Q représente $-B(OH)_2$, un ester de l'acide boronique, de préférence l'ester pinacolique de l'acide boronique, ou $-BF_3^-K^+$,

dans des conditions de couplage Suzuki pour former un composé de formule (I).

**5.** Composé selon l'une quelconque des revendications 1 à 3, pour le traitement et/ou la prophylaxie de maladies.

**6.** Utilisation d'un composé selon l'une quelconque des revendications 1 à 3 pour la fabrication d'un médicament pour le traitement et/ou la prophylaxie de maladies.

**7.** Utilisation d'un composé selon l'une quelconque des revendications 1 à 3 pour la fabrication d'un médicament pour le traitement et/ou la prophylaxie de maladies thrombotiques ou thromboemboliques.

**8.** Utilisation d'un composé selon l'une quelconque des revendications 1 à 3 pour la fabrication d'un médicament pour

le traitement et/ou la prophylaxie de maladies ophtalmiques.

**9.** Médicament contenant un composé selon l'une quelconque des revendications 1 à 3 en combinaison avec un adjuvant inerte, non toxique, pharmaceutiquement approprié.

**10.** Médicament selon la revendication 9 pour le traitement et/ou la prophylaxie de maladies thrombotiques ou thromboemboliques.

**11.** Médicament selon la revendication 9 pour le traitement et/ou la prophylaxie de maladies ophtalmiques.

**12.** ({2-[4-(5-Chloro-2-cyanophényl)-5-méthoxy-2-oxopyridin-1(2H)-yl]-3-[tétrahydro-2H-pyran-2-yl]propanoyl}amino)benzoïque (mélange de diastéréomères racémiques) selon la revendication 1, de formule générale

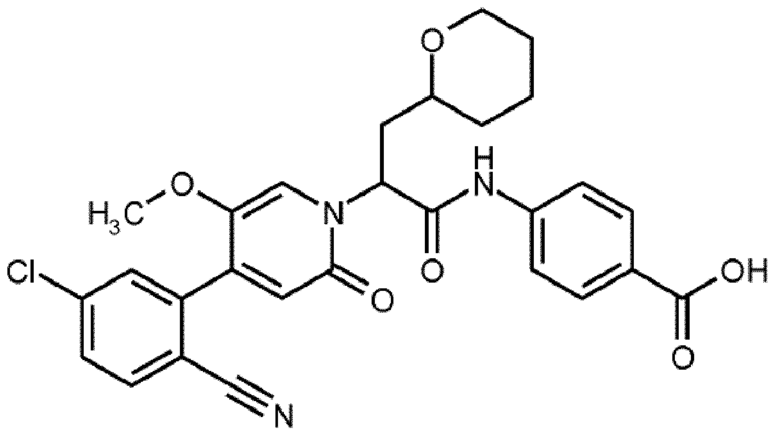

ou un de ses sels, de ses solvates ou des solvates de ses sels.

**13.** ({2-[4-(5-Chloro-2-cyanophényl)-5-méthoxy-2-oxopyridin-1(2H)-yl]-3-[tétrahydro-2H-pyran-2-yl]propanoyl}amino)benzoïque (énantiomère 1 du 2[e] diastéréomère) selon la revendication 1, de formule générale

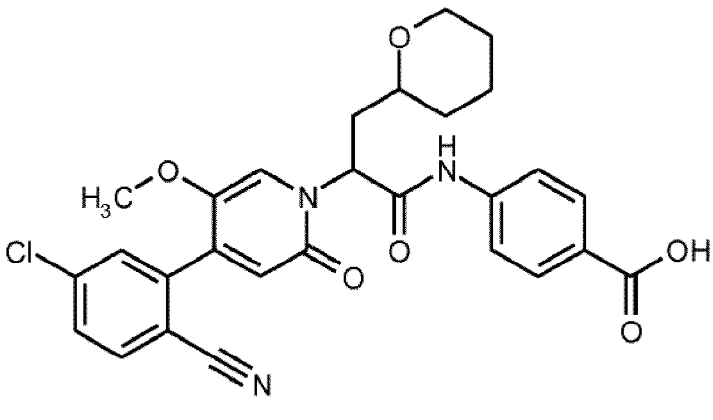

ou un de ses sels, de ses solvates ou des solvates de ses sels.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2006030032 A
- WO 2008079787 A
- WO 2007131179 A


### In der Beschreibung aufgeführte Nicht-Patentliteratur


- **EUGENE BRAUNWALD.** Heart Disease: A Textbook of Cardiovascular Medicine. W.B. Saunders Company, 1997
- Heparin. **PSCHYREMBEL.** Klinisches Wörterbuch. Walter de Gruyter Verlag, 1994, 610
- Heparin. Römpp Lexikon Chemie. Georg Thieme Verlag, 1998
- **J. HIRSH ; J. DALEN ; D.R. ANDERSON et al.** Oral anticoagulants: Mechanism of action, clinical effectiveness, and optimal therapeutic range. *Chest,* 2001, vol. 119, 8S-21S
- **J. ANSELL ; J. HIRSH ; J. DALEN et al.** Managing oral anticoagulant therapy. *Chest,* 2001, vol. 119, 22S-38S
- **P.S. WELLS ; A.M. HOLBROOK ; N.R. CROWTHER et al.** Interactions of warfarin with drugs and food. *Ann. Intern. Med.,* 1994, vol. 121, 676-683
- Crit Care Med. *ACCP/SCCM Consensus Conference Committee von 1992,* 1992, vol. 20, 864-874
- **LEVY et al.** *Crit Care Med,* 2003, vol. 31, 1250-1256
- **DELLINGER et al.** *Crit Care Med,* 2004, vol. 32, 858-873
- *J. Am. Chem. Soc.,* 2010, vol. 132, 14073-14075
- **B. A. BAKER et al.** *Org. Lett.,* 2008, vol. 10, 289-292
- *Angew. Chem. Int. Ed.,* 2013, vol. 52, 1-5
- *Journal of Fluorine Chemistry,* 2009, vol. 130, 667-670
- **J. CASTELLS et al.** *Tetrahedron,* 1994, vol. 50, 13765-13774
- **A. APONICK ; B. BIANNIC.** *Org. Lett.n,* 2011, vol. 13, 1330-1333